(19) Europäisches Patentamt
European Patent Office
Office européen des brevets

(11) EP 1 313 725 B1

(12) **EUROPEAN PATENT SPECIFICATION**

(45) Date of publication and mention of the grant of the patent:
**11.04.2007 Bulletin 2007/15**

(21) Application number: **01968188.1**

(22) Date of filing: **28.08.2001**

(51) Int Cl.:
***C07D 401/06*** (2006.01) ***C07D 401/04*** (2006.01)
***C07D 401/14*** (2006.01) ***C07D 221/16*** (2006.01)
***A61K 31/435*** (2006.01) ***A61P 35/00*** (2006.01)

(86) International application number:
**PCT/US2001/026792**

(87) International publication number:
**WO 2002/018368 (07.03.2002 Gazette 2002/10)**

(54) **TRICYCLIC ANTITUMOR COMPOUNDS BEING FARNESYL PROTEIN TRANSFERASE INHIBITORS**

TRICYCLIC ANTITUMOR KOMPONENTEN ALS FARNESYL PROTEIN TRANSFERASE INHIBITOREN

COMPOSES ANTITUMORAUX TRICYCLIQUES SOUS FORME D'INHIBITEURS DE LA FARNESYL-PROTEINE TRANSFERASE

(84) Designated Contracting States:
**AT BE CH CY DE DK ES FI FR GB GR IE IT LI LU MC NL PT SE TR**
Designated Extension States:
**RO SI**

(30) Priority: **30.08.2000 US 229183 P**

(43) Date of publication of application:
**28.05.2003 Bulletin 2003/22**

(73) Proprietors:
- **SCHERING CORPORATION**
  **Kenilworth, New Jersey 07033-0530 (US)**
- **Pharmacopeia Drug Discovery, Inc.**
  **Cranbury, New Jersey 08512 (US)**

(72) Inventors:
- **NJOROGE, F., George**
  **Warren, NJ 07059 (US)**
- **VIBULBHAN, Bancha**
  **Kenilworth, NJ 07033 (US)**
- **COOPER, Alan, B.**
  **West Caldwell, NJ 07066 (US)**
- **GUZI, Timothy**
  **Chatham, NJ 07928 (US)**
- **RANE, Dinanath, F.**
  **Morganville, NJ 07751 (US)**
- **MINOR, Keith, P.**
  **Dallas, Texas 75249 (US)**
- **DOLL, Ronald, J.**
  **Convent Station, New Jersey 07960 (US)**
- **GIRIJAVALLABHAN, Viyyoor, Moopil**
  **Parsippany, NJ 07054 (US)**
- **SANTHANAM, Bama**
  **Bridgewater, NJ 08807 (US)**
- **PINTO, Patrick, A.**
  **Morris Plains, NJ 07950 (US)**
- **ZHU, Hugh, Y.**
  **Scotch Plains, NJ 07076 (US)**
- **KEERTIKAR, Kartik, M.**
  **Rahway, NJ 07065 (US)**
- **ALVAREZ, Carmen, S.**
  **Roselle Park, NJ 07204 (US)**
- **BALDWIN, John, J.**
  **Gwynedd Valley, PA 19437 (US)**
- **LI, Ge**
  **Lujiazui Garden,**
  **200135 Shang Hai (CN)**
- **HUANG, Chia-Yu**
  **Plainsboro, NJ 08536 (US)**
- **JAMES, Ray, A.**
  **Plainsboro, NJ 08536 (US)**
- **BISHOP, Robert, W.**
  **Pompton Plains, NJ 07444 (US)**
- **WANG, James**
  **Westfield, NJ 07090 (US)**
- **DESAI, Jagdish, A.**
  **Monroe Township, NJ 08831 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

(74) Representative: **Adams, Harvey Vaughan John et al**
**Mathys & Squire**
**120 Holborn**
**London EC1N 2SQ (GB)**

(56) References cited:
**WO-A-00/37458**
**WO-A-96/30363**
**US-A- 5 089 496**
**US-A- 5 925 648**
**US-A- 6 071 907**

## Description

SUMMARY OF THE INVENTION

This invention provides compounds useful for the inhibition of farnesyl protein transferase (FPT). The compounds of this invention are represented by the formula:

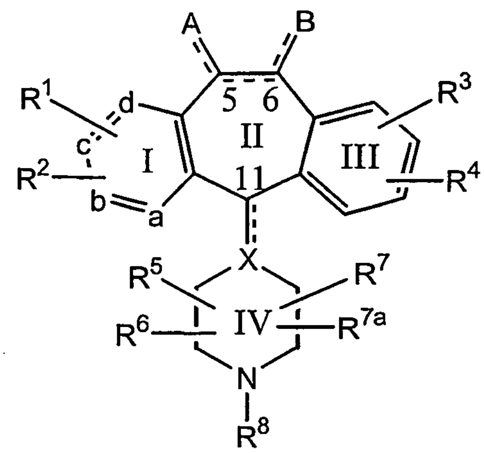

(1.0)

or a pharmaceutically acceptable salt or solvate thereof, wherein:

one of a, b, c and d represents N or $N^+O^-$, and the remaining a, b, c, and d groups represent carbon, wherein each carbon has an $R^1$ or $R^2$ group bound to said carbon; or
each of a, b, c, and d is carbon, wherein each carbon has an $R^1$ or $R^2$ group bound to said carbon;
the dotted line (---) represents optional bonds;
X represents N or CH when the optional bond (to C11) is absent, and represents C when the optional bond (to C11) is present;

When the optional bond is present between carbon atom 5 (i.e., C-5) and carbon atom 6 (i.e., C-6) (i.e., there is a double bond between C-5 and C-6) then there is only one A substituent bound to C-5 and there is only one B substituent bound to C-6 and A or B is other than H;
When the optional bond is not present between carbon atom 5 and carbon atom 6 (i.e., there is a single bond between C-5 and C-6) then there are two A substituents bound to C-5, wherein each A substituent is independently selected and two B substituents bound to C-6, wherein each B substituent is independently selected, i.e.,

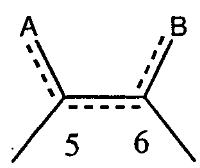

In formula 1.0 represents

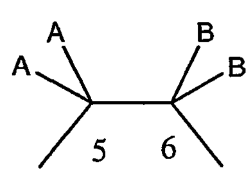

when there is a single bond between C-5 and C-6 and each A and each B are independently selected, and wherein at least one of the two A substituents or one of the two B substituents are H, and wherein at least one of the two A substituents or one of the two B substituants is other than H, (i.e., when there is a single bond between C-5 and C-6 one of the four substituents (A, A, B, and B) is H and one is other than H);

A and B is independently selected from:

(1) -H;
(2) $-R^9$;
(3) $-R^9-C(O)-R^9$;
(4) $- R^9-CO_2- R^{9a}$;
(5) $-C(O)NHR^9$;
(6) $-C(O)NH-CH_2-C(O)-NH_2$;
(7) $-C(O)NHR^{26}$;
(8) $-(CH_2)p(R^9)_2$, wherein each $R^9$ is the same or different;
(9) $-(CH_2)pC(O)R^9$;
(10) $-(CH_2)pC(O)R^{27a}$;
(11) $-(CH_2)pC(O)N(R^9)_2$, wherein each $R^9$ is the same or different;
(12) $-(CH_2)pC(O)NH(R^9)$;
(13) $-(CH_2)pNHC(O)R^{50}$;
(14) $-(CH_2)pNHC(O)_2R^{50}$;
(15) $-(CH_2)pN(C(O)R^{27a})_2$ wherein each $R^{27a}$ is the same or different;
(16) $-(CH_2)pNR^{51}C(O)R^{27}$, or $R^{51}$ and $R^{27}$ taken together with the atoms to which they are bound form a heterocycloalkyl ring consisting of 5 or 6 members, provided that when $R^{51}$ and $R^{27}$ form a ring, $R^{51}$ is not H;
(17) $-(CH_2)pNR^{51}C(O)NR^{27}$, or $R^{51}$ and $R^{27}$ taken together with the atoms to which they are bound form a heterocycloalkyl ring consisting or 5 or 6 members, provided that when $R^{51}$ and $R^{27}$ form a ring, $R^{51}$ is not H;
(18) $-(CH_2)pNR^{51}C(O)N(R^{27a})_2$, wherein each $R^{27a}$ is the same or different;
(19) $-(CH_2)pNHSO_2N(R^{51})_2$, wherein each $R^{51}$ is the same or different;
(20) $-(CH_2)pNHCO_2R^{50}$;
(21) $-(CH_2)pCO_2R^{51}$;
(22) $-NHR^9$;
(23)

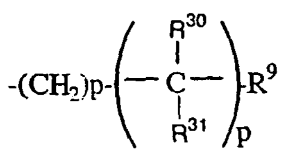

wherein $R^{30}$ and $R^{31}$ are the same or different; and
(24)

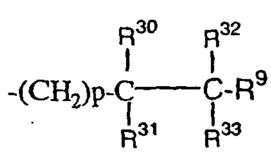
,

wherein $R^{30}$, $R^{31}$, $R^{32}$ and $R^{33}$ are the same or different;

p is 0, 1, 2, 3 or 4;

each $R^1$ and $R^2$ is independently selected from H, Halo, $-CF_3$, $-OR^{10}$, $COR^{10}$, $-SR^{10}$, $-S(O)_tR^{15}$ wherein t is 0, 1 or 2, $-N(R^{10})_2$, $-NO_2$, $-OC(O)R^{10}$, $CO_2R^{10}$, $-OCO_2R^{15}$, -CN, $-NR^{10}COOR^{15}$, $-SR^{15}C(O)OR^{15}$, $-SR^{15}N(R^{13})_2$ provided that $R^{15}$ in $-SR^{15}N(R^{13})_2$ is not $-CH_2$ and wherein each $R^{13}$ is independently selected from H or $-C(O)OR^{15}$, benzotriazol-1-yloxy, tetrazol-5-ylthio, or substituted tetrazol-5-ylthio, alkynyl, alkenyl or alkyl, said alkyl or alkenyl group optionally being substituted with halogen, $-OR^{10}$ or $-CO_2R^{10}$;

$R^3$ and $R^4$ are the same or different and each independently represent H, and any of the substituents of $R^1$ and $R^2$;

$R^5$, $R^6$, $R^7$ and $R^{7a}$ each independently represent H, $-CF_3$, $-COR^{10}$, alkyl or aryl, said alkyl or aryl optionally being substituted with $-OR^{10}$, $SR^{10}$, $-S(O)_tR^{15}$, $-NR^{10}COOR^{15}$, $-N(R^{10})_2$, $-NO_2$, $-C(O)R^{10}$,- $OCOR^{10}$, $-OCO_2R^{15}$, $-CO_2R^{10}$, $OPO_3R^{10}$, or $R^5$ is combined with $R^6$ to represent =O or =S;

$R^8$ is selected from:

H, 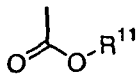 , 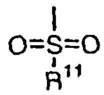 , 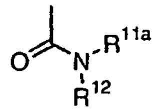 or 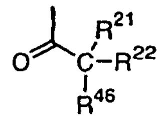

(2.0) (3.0) (4.0) (5.0)

$R^9$ is selected from:

(1) heteroaryl;
(2) substituted heteroaryl;
(3) heterocycloalkyl;
(4) substituted heterocycloalkyl;
(5) heterocycloalkylalkyl;
(6) substituted heterocycloalkylalkyl;
(7) heteroarylalkyl;
(8) substituted heteroarylalkyl;
(9) heteroarylalkenyl;
(10) substituted heteroarylalkenyl;
(11) heteroarylalkynyl and
(12) substituted heteroarylalkynyl;

wherein said substituted $R^9$ groups are substituted with one or more (e.g. 1, 2 or 3) substituents selected from:

(1) -OH;
(2) $-CO_2R^{14}$;
(3) $-CH_2OR^{14}$,
(4) halogen (e.g. Br, Cl or F),
(5) alkyl (e.g. methyl, ethyl, propyl, butyl or t-butyl);
(6) amino;
(7) trityl;
(8) heterocycloalkyl;
(9) cycloalkyl, (e.g. cyclopropyl or cyclohexyl);
(10) arylalkyl;
(11) heteroaryl;
(12) heteroarylalkyl and
(13)

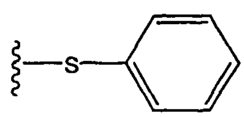 ;

wherein $R^{14}$ is independently selected from: H; alkyl; aryl, arylalkyl, heteroaryl and heteroarylalkyl;

$R^{9a}$ is selected from: alky or arylalkyl;

$R^{10}$ is selected from: H; alkyl; aryl or arylalkyl;

$R^{11}$ is selected from:

(1) alkyl;
(2) substituted alkyl;
(3) aryl;

(4) substituted aryl;
(5) cycloalkyl;
(6) substituted cycloalkyl;
(7) heteroaryl;
(8) substituted heteroaryl;
(9) heterocycloalkyl; and
(10) substituted heterocycloalkyl;

wherein said substituted $R^{11}$ groups have one or more (e.g. 1, 2 or 3) substituents selected from:

(1) -OH;
(2) halogen (e.g. Br, Cl or F) and
(3) alkyl;

$R^{11a}$ is selected from:

(1) H;
(2) OH;
(3) alkyl;
(4) substituted alkyl;
(5) aryl;
(6) substituted aryl;
(7) cycloalkyl;
(8) substituted cycloalkyl;
(9) heteroaryl;
(10) substituted heteroaryl;
(11) heterocycloalkyl; and
(12) substituted heterocycloalkyl;

wherein said substituted $R^{11a}$ groups have one or more (e.g. 1, 2 or 3) substituents selected from:

(1) -OH;
(2) -CN;
(3) $-CF_3$;
(4) halogen (e.g Br, Cl or F);
(5) alkyl;
(6) cycloalkyl;
(7) heterocycloalkyl;
(8) arylalkyl;
(9) heteroarylalkyl;
(10) alkenyl and
(11) heteroalkenyl;

$R^{12}$ is selected from: H, or alkyl;
$R^{15}$ is selected from: alkyl or aryl;
$R^{21}$, $R^{22}$ and $R^{46}$ are independently selected from:

(1) -H;
(2) alkyl (e.g., methyl, ethyl, propyl, butyl or t-butyl);
(3) aryl, (e.g. phenyl);
(4) substituted aryl,
optionally substituted with one or more substituents selected from: alkyl, halogen, $CF_3$ or OH;
(5) cycloalkyl, (e.g. cyclohexyl);
(6) substituted cycloalkyl;
optionally substituted with one or more substituents selected from: alkyl, halogen, $CF_3$ or OH;
(7) heteroaryl of the formula,

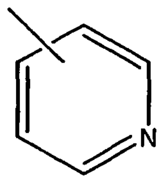

and

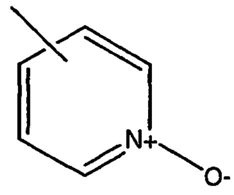

;

(8) heterocycloalkyl of the formula:

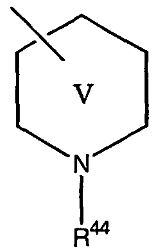

wherein $R^{44}$ is selected from:

(1) -H,
(2) alkyl, (e.g., methyl, ethyl, propyl, butyl or t-butyl);
(3) alkylcarbonyl (e.g., $CH_3C(O)$-);
(4) alkyloxy carbonyl (e.g., -C(O)O-t-$C_4H_9$, -C(O)$OC_2H_5$, and -C(O)$OCH_3$);
(5) haloalkyl (e.g., trifluoromethyl) and
(6) -C(O)NH($R^{51}$);

when $R^{21}$ $R^{22}$ or $R^{46}$ is the heterocycloalkyl of the formula above (i.e. Ring V), Ring V includes:

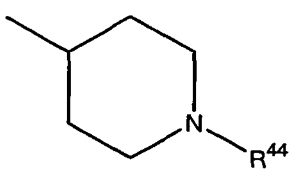

,

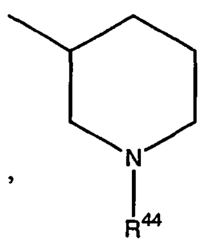

,

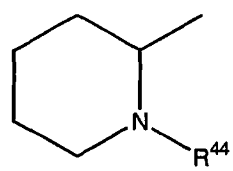

.

Examples of Ring V include:

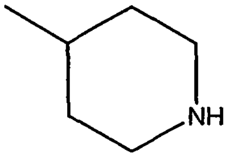
,
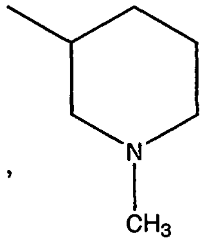
,
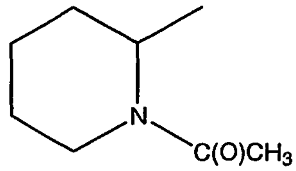
,
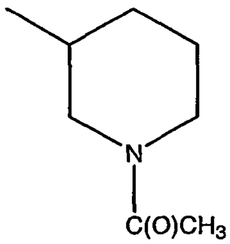
,
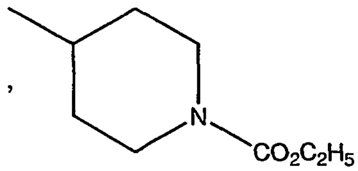
',
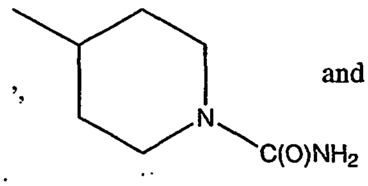
and
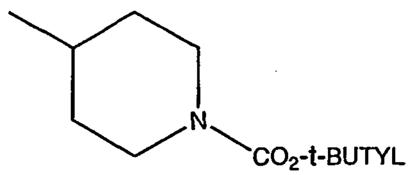

$R^{26}$ is selected from:

(1) -H;
(2) alkyl (e.g. methyl, ethyl, propyl, butyl or t-butyl);
(3) alkoxyl (e.g. methoxy, ethoxy, propoxy);
(4) $-CH_2-CN$;
(5) $R^9$;
(6) $-CH_2CO_2H$;
(7) -C(O)alkyl and
(8) $CH_2CO_2$alkyl;

$R^{27}$ is selected from:

(1) -H;
(2) -OH;
(3) alkyl (e.g. methyl, ethyl, propyl, or butyl), and
(4) alkoxy ;

$R^{27a}$ is selected from:

(1) alkyl (e.g. methyl, ethyl, propyl, or butyl), and
(2) alkoxy ;

$R^{30}$ , $R^{31}$ , $R^{32}$ and $R^{33}$ is independently selected from:

(1) -H;
(2) -OH;
(3) =O;
(4) alkyl;
(5) aryl (e.g. phenyl) and
(6) arylalkyl (e.g. benzyl);

$R^{50}$ is selected from:

(1) alkyl;
(2) heteroaryl;
(3) substituted heteroaryl and
(4) amino;

wherein said substituents on said substituted $R^{50}$ groups are independently selected from: alkyl (e.g. methyl, ethyl, propyl, or butyl); halogen (e.g. Br, Cl, or F); and -OH;
$R^{50a}$ is selected from:

(1) heteroaryl;
(2) substituted heteroaryl and
(3) amino;

$R^{51}$ is selected from: -H, or alkyl (e.g.;methyl, ethyl, propyl, butyl or t-butyl);
The compounds of this invention: (i) potently inhibit farnesyl protein transferase, but not geranylgeranyl protein transferase I, in vitro; (ii) block the phenotypic change induced by a form of transforming Ras which is a farnesyl acceptor but not by a form of transforming Ras engineered to be a geranylgeranyl acceptor; (iii) block intracellular processing of Ras which is a farnesyl acceptor but not of Ras engineered to be a geranylgeranyl acceptor; and (iv) block abnormal cell growth in culture induced by transforming Ras.
The compounds of this invention inhibit farnesyl protein transferase and the farnesylation of the oncogene protein Ras. Thus, this invention further provides a method of inhibiting farnesyl protein transferase, (e.g., ras farnesyl protein transferase) in mammals, especially humans, by the administration of an effective amount (e.g. a therapeutically effective amount) of the tricyclic compounds described above. The administration of the compounds of this invention to patients, to inhibit farnesyl protein transferase, is useful in the treatment of the cancers described below.
This invention provides a method for inhibiting or treating the abnormal growth of cells, including transformed cells, by administering an effective amount (e.g. a therapeutically effective amount) of a compound of this invention. Abnormal growth of cells refers to cell growth independent of normal regulatory mechanisms (e.g., loss of contact inhibition). This includes the abnormal growth of: (1) tumor cells (tumors) expressing an activated Ras oncogene; (2) tumor cells in which the Ras protein is activated as a result of oncogenic mutation in another gene; and (3) benign and malignant cells of other proliferative diseases in which aberrant Ras activation occurs.
This invention also provides a method for inhibiting or treating tumor growth by administering an effective amount (e.g., a therapeutically effective amount) of the tricyclic compounds, described herein, to a mammal (e.g., a human) in need of such treatment. In particular, this invention provides a method for inhibiting or treating the growth of tumors expressing an activated Ras oncogene by the administration of an effective amount (e.g. a therapeutically effective amount) of the above described compounds.
The present invention also provides a method of treating proliferative diseases, especially cancers (tumors), comprising administering an effective amount (e.g., a therapeutically effective amount) of a compound of the invention, described herein, to a mammal (e.g., a human) in need of such treatment in combination with (2) an effective amount of at least one anti-cancer agent i.e. a chemotherapeutic agent and/or radiation).
The present invention also provides a method of treating proliferative diseases, especially cancers (tumors), comprising administering an effective amount (e.g., a therapeutically effective amount) of a compound of the invention, described herein, to a mammal (e.g., a human) in need of such treatment in combination with (2) an effective amount of at least one signal transduction inhibitor.
Examples of proliferative diseases (tumors) which may be inhibited or treated include, but are not limited to, lung cancer (e.g., lung adenocarcinoma), pancreatic cancers (e.g., pancreatic carcinoma such as, for example, exocrine pancreatic carcinoma), colon cancers (e.g., colorectal carcinomas, such as, for example, colon adenocarcinoma and colon adenoma), myeloid leukemias (for example, acute myelogenous leukemia (AML)), thyroid follicular cancer, myelodysplastic syndrome (MDS), bladder carcinoma, epidermal carcinoma, melanoma, breast cancer and prostate cancer.
It is believed that this invention also provides a method for inhibiting or treating proliferative diseases, both benign and malignant, wherein Ras proteins are aberrantly activated as a result of oncogenic mutation in other genes--i.e., the Ras gene itself is not activated by mutation to an oncogenic form--with said inhibition or treatment being accomplished by the administration of an effective amount (e.g. a therapeutically effective amount) of the tricyclic compounds described herein, to a mammal (e.g., a human) in need of such treatment. For example, the benign proliferative disorder neurofibromatosis, or tumors in which Ras is activated due to mutation or overexpression of tyrosine kinase oncogenes (e.g., neu, src, abl, lck, and fyn), may be inhibited or treated by the tricyclic compounds described herein.
The tricyclic compounds useful in the methods of this invention inhibit or treat the abnormal growth of cells.

Without wishing to be bound by theory, it is believed that these compounds may function through the inhibition of G-protein function, such as Ras p21, by blocking G-protein isoprenylation, thus making them useful in the treatment of proliferative diseases such as tumor growth and cancer. Without wishing to be bound by theory, it is believed that these compounds inhibit ras farnesyl protein transferase, and thus show antiproliferative activity against ras transformed cells.

DETAILED DESCRIPTION OF THE INVENTION

As used herein, the following terms are used as defined below unless otherwise indicated:

$MH^+$-represents the molecular ion plus hydrogen of the molecule in the mass spectrum;
BOC-represents tert-butyloxycarbonyl;
CBZ-represents $-C(O)OCH_2C_6H_5$ (i.e., benzyloxycarbonyl);
$CH_2Cl_2$-represents dichloromethane;
CIMS-represents chemical ionization mass spectrum;
DBU-represents 1,8-Diazabicyclo[5.4.0]undec-7-ene;
DEAD-represents diethylazodicarboxylate;
DEC-represents EDCI which represents 1-(3-dimethyl-aminopropyl)-3-ethylcarbodiimide hydrochloride;
DMF-represents N,N-dimethylformamide;
Et-represents ethyl;
EtOAc-represents ethyl acetate;
EtOH-represents ethanol;
HOBT-represents 1-hydroxybenzotriazole hydrate;
IPA-represents isopropanol;
i-PrOH-represents isopropanol;
Me-represents methyl;
MeOH-represents methanol;
MS-represents mass spectroscopy;
FAB-represents FABMS which represents fast atom bombardment mass spectroscopy;
HRMS-represents high resolution mass spectroscopy;
NMM-represents N-methylmorpholine;
$PPh_3$-represents triphenyl phosphine;
Ph-represents phenyl;
Pr-represents propyl;
SEM-represents 2,2-(Trimethylsilyl)ethoxymethyl;
TBDMS-represents tert-butyldimethylsilyl;
$Et_3N$-represents TEA which represents triethylamine;
t-BUTYL-represents $-C-(CH_3)_3$ ;
TFA-represents trifluoroacetic acid;
THF-represents tetrahydrofuran;
Tr-represents trityl;
Tf-represents $SO_2CF_3$;
at least one- represents one or more-(e.g. 1-6), more preferrably 1-4 with 1, 2 or 3 being most preferred;
alkyl-represents straight and branched carbon chains and contains from one to twenty carbon atoms, preferably one to six carbon atoms, more preferably one to four carbon atoms; even more preferably one to two carbon atoms.
arylalkyl-represents an alkyl group, as defined above, substituted with an aryl group, as defined below, such that the bond from another substituent is to the alkyl moiety;
alkoxy-represents an alkyl moiety, alkyl as defined above, covalently bonded to an adjacent structural element through an oxygen atom, for example, methoxy, ethoxy, propoxy, butoxy and the like;
phenoxy represents an alkoxy moiety, as defined above, wherein the covalently bonded moiety is an aryl group, as defined below, for example, -O-phenyl;
alkenyl represents straight and branched carbon chains having at least one carbon to carbon double bond and containing from 2-12 carbon atoms, preferably from 2 to 6 carbon atoms and most preferably from 3 to 6 carbon atoms;
alkynyl represents straight and branched carbon chains having at least one carbon to carbon triple bond and containing from 2-12 carbon atoms, preferably from 2 to 6 carbon atoms and most preferably from 2 to 4 carbon atoms;
amino represents an $-NH_2$ moiety;
aryl-(including the aryl portion of arylalkyl and heteroarylalkyl)-represents a carbocyclic group containing from 6 to 15 carbon atoms and having at least one aromatic ring (e.g., aryl is a phenyl ring), with all available substitutable carbon atoms of the carbocyclic group being intended as possible points of attachment, said carbocyclic group

being optionally substituted with one or more (e.g., 1 to 3) of halo, alkyl, hydroxy, alkoxy, phenoxy, $CF_3$, $-C(O)N(R^{18})_2$, $-SO_2R^{18}$, $-SO_2N(R^{18})_2$, amino, alkylamino, dialkylamino, $-COOR^{23}$ or $-NO_2$, wherein $R^{18}$ represents H, alkyl, aryl, arylalkyl, heteroaryl or cycloalkyl and $R^{23}$ represents alkyl or aryl;

cycloalkyl-represents saturated carbocyclic rings of from 3 to 20 carbon atoms, preferably 3 to 7 carbon atoms, said cycloalkyl ring being optionally substituted with one or more (e.g., 1, 2 or 3) of the same or different alkyl groups (e.g., methyl or ethyl);

cycloalkylalkyl- represents an alkyl group, as defined above, substituted with a cyclo group, as defined above, such that the bond from another substituent is to the alkyl moiety;

heterocycloalkylalkyl- represents an alkyl group, as defined above, substituted with a heterocycloalkyl group, as defined below, such that the bond from another substituent is to the alkyl moiety;

halo- represents halogen i.e. fluoro, chloro, bromo and iodo;

haloalkyl- represents an alkyl group, as defined above, substituted with a halo group, as defined above, such that the bond from another substituent is to the alkyl moiety;

heteroarylalkyl- represents an alkyl group, as defined above, substituted with a heteroaryl group, as defined below, such that the bond from another substituent is to the alkyl moiety;

heteroarylalkenyl- represents an alkenyl group, as defined above, substituted with a heteroaryl group, as defined below, such that the bond from another substituent is to the alkyl moiety;

heteroalkyl- represents straight and branched carbon chains containing from one to twenty carbon atoms, preferably one to six carbon atoms interrupted by 1 to 3 heteroatoms selected from -O-, -S- and -N-;

heteroalkenyl- represents straight and branched carbon chains having at least one carbon to carbon double bond and containing from one to twenty carbon atoms, preferably one to six carbon atoms interrupted by 1 to 3 heteroatoms selected from -O-, -S- and -N-;

heteroalkynyl- represents straight and branched carbon chains having at least one carbon to carbon triple bond and containing from one to twenty carbon atoms, preferably one to six carbon atoms interrupted by 1 to 3 heteroatoms selected from -O-, -S- and -N-;

arylheteroalkyl- represents a heteroalkyl group, as defined above, substituted with an aryl group, as defined above, such that the bond from another substituent is to the alkyl moiety;

alkylcarbonyl- represents an alkyl group, as defined above, covalently bonded to a carbonyl moiety (-CO-), for example, $-COCH_3$;

alkyloxycarbonyl- represents an alkyl group, as defined above, covalently bonded to a carbonyl moiety (-CO-) through an oxygen atom, for example, $-C(O)-OC_2H_5$;

heteroaryl- represents cyclic groups, optionally substituted with $R^3$ and $R^4$, having at least one heteroatom selected from O, S or N, said heteroatom interrupting a carbocyclic ring structure and having a sufficient number of delocalized pi electrons to provide aromatic character, with the aromatic heterocyclic groups preferably containing from 2 to 14 carbon atoms, e.g., 2- or 3-furyl, 2- or 3-thienyl, 2-, 4- or 5-thiazolyl, 2-, 4- or 5-imidazolyl, 2-, 4- or 5-pyrimidinyl, 2-pyrazinyl, 3- or 4-pyridazinyl, 3-, 5- or 6-[1,2,4-triazinyl], 3- or 5-[1,2,4-thiadizolyl], 2-, 3-, 4-, 5-, 6- or 7-benzofuranyl, 2-, 3-, 4-, 5-, 6- or 7-indolyl, 3-, 4- or 5-pyrazolyl, 2-, 4- or 5-oxazolyl, triazolyl, 2-, 3- or 4-pyridyl, or 2-, 3- or 4-pyridyl N-oxide, wherein pyridyl N-oxide can be represented as:

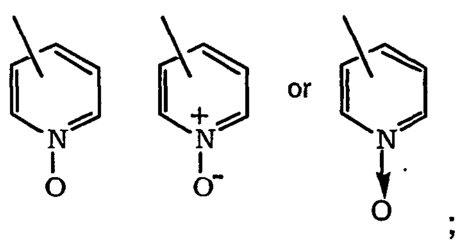

and

heterocycloalkyl- represents a saturated, branched or unbranched carbocylic ring containing from 3 to 15 carbon atoms, preferably from 4 to 6 carbon atoms, which carbocyclic ring is interrupted by 1 to 3 hetero groups selected from -O-, -S- or - $NR^{24}$, (e.g., $-NC(O)-NH_2$) wherein $R^{24}$ represents alkyl, aryl, $-C(O)N(R^{18})_2$ wherein $R^{18}$ is as above defined, suitable heterocycloalkyl groups include 2- or 3-tetrahydrofuranyl, 2- or 3- tetrahydrothienyl, 2-, 3- or 4-piperidinyl, 2- or 3-pyrrolidinyl, 1-, 2-, 3- , or 4-piperizinyl, 2- or 4-dioxanyl, morpholinyl, and

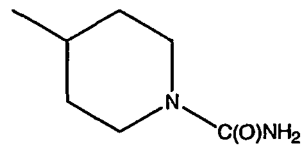

The positions in the tricyclic ring system are:

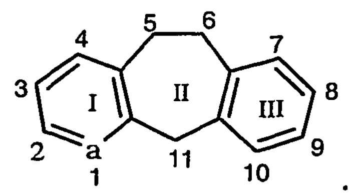

The compounds of formula 1.0 include the preferred R isomer:

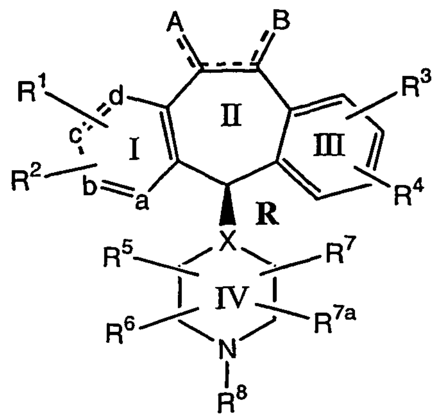

(1.0A)

X= N or CH
a =N or C

wherein the optional bond between C-5 and C-6 is present, and B is H, or the optional bond between C-5 and C-6 is absent and each B is H; and the preferred S isomer:

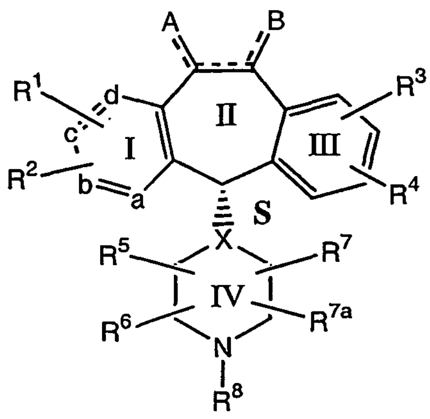

(1.0B)

X= N or CH
a=N or C

wherein the optional bond between C-5 and C-6 is present and A is H, or the optional bond between C-5 and C-6 is absent and each A is H.

Preferably, $R^1$, $R^2$, $R^3$, and $R^4$ are independently selected from H or halo, more preferably H, Br, F or Cl, and even more preferably H, or Cl. Representative compounds of formula 1.0 include dihalo (e.g., 3,8-dihalo) and monohalo (e.g., 8-halo) substituted compounds, such as, for example: (3-bromo, 8-chloro), (3,8-dichloro), (3-bromo) and (3-chloro).

Substituent a is preferably C or N with N being most preferred.

Preferably, $R^8$ is selected from:

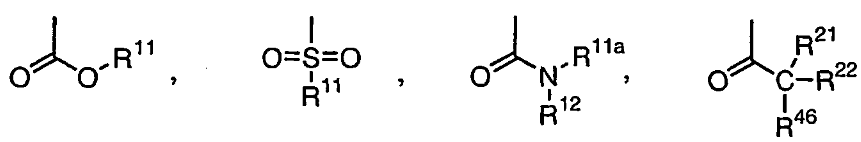

(2.0) , (3.0) , (4.0) , (5.0)

More preferably $R^8$ is 2.0 or 4.0; and most preferably $R^8$ is 4.0.

Preferably, $R^{11a}$ is selected from: alkyl, substituted alkyl, aryl, substituted aryl, heteroaryl, substituted heteroaryl, cyloalkyl or substituted cycloalkyl; wherein, said substituted aryl, heteroary, and cycloalkyl, $R^{11a}$ groups are substituted with substituents independently selected from: halo (preferably F or Cl), cyano, $-CF_3$, or alkyl; and wherein said substituted alkyl $R^{11a}$ groups substituted with substituents selected from halo, (preferably F or Cl), cyano or $CF_3$. Most preferably, $R^{11a}$ is selected from: alkyl, aryl, substituted aryl, cyloalkyl, or substituted cycloalkyl, wherein, said substituted aryl and substituted cycloalkyl groups are substituted with substituents independently selected from: halo, (preferably F or Cl), CN or $CF_3$. More preferably, $R^{11a}$ is selected from methyl, t-butyl, phenyl, cyanophenyl, chlorophenyl, fluorophenyl, or cyclohexyl. Still more preferably, $R^{11a}$ is selected from: t-butyl, cyanophenyl, chlorophenyl, fluorophenyl or cyclohexyl. Even more preferably, $R^{11a}$ is selected from cyanophenyl, with p-cyanophenyl being even still more preferred.

Preferably, $R^{11}$, is selected from alkyl, cycloalkyl, or substituted cycloalkyl, wherein said substituted cycloalkyl group is substituted with 1, 2 or 3 substituents independently selected from: halo (preferably chloro or fluoro), or alkyl, (preferably methyl or t-butyl). Examples of $R^{11}$ groups include: methyl, ethyl, propyl, t-butyl, cyclohexyl or substituted cyclohexyl. More preferably, $R^{11}$ is selected from methyl, t-butyl, cyclohexyl, chlorocyclohexyl, (preferably p-chlorocyclohexyl) or fluorocyclohexyl, (preferably p-fluorocyclohexyl). Most preferably, $R^{11}$ is selected from: methyl, t-butyl, or cyclohexyl, with t-butyl or cyclohexyl being still more preferred.

Preferably, $R^{12}$ is selected from H or methyl. Most preferably, $R^{12}$ is H.

$R^5$, $R^6$, $R^7$ and $R^{7a}$ are preferably H.

Preferably, $R^9$ is selected from:

(1) heteroaryl;

(2) substituted heteroaryl;
(3) heterocycloalkyl;
(4) substituted heterocycloalkyl;
(5) heterocycloalkylalkyl;
(6) substituted heterocycloalkylalkyl;
(7) heteroarylalkyl;
(8) substituted heteroarylalkyl;
(9) heteroarylalkenyl and
(10) substituted heteroarylalkenyl;

wherein said substituted $R^9$ groups are substituted with one or more substituents (e.g., 1, 2, or 3) independently selected from:

(1) -OH;
(2) $-CO_2R^{14}$;
wherein, $R^{14}$ is selected from: H or alkyl (e.g., methyl or ethyl), preferably alkyl,most preferably methyl or ethyl;
(3) alkyl, substituted with one or more -OH groups (e.g., 1, 2, or 3, preferably 1), for example $-(CH_2)qOH$ wherein, q is 1 -4, with q = 1 being preferred.
(4) halo (e.g., Br, F, I, or Cl);
(5) alkyl, usually $C_1$-$C_6$ alkyl, preferably $C_1$-$C_4$ alkyl (e.g. methyl, ethyl, propyl, or butyl (preferably isopropyl, or t-butyl));
(6) amino;
(7) trityl;
(8) heterocycloalkyl;
(9) arylalkyl (e.g. benzyl);
(10) heteroaryl (e.g. pyridyl) and
(11) heteroarylalkyl (piperidine-$CH_3$);

Most preferably, $R^9$ is selected from:

(1) heterocycloalkyl;
(2) substituted heterocycloalkyl;
(3) heterocycloalkylalkyl;
(4) substituted heterocycloalkylalkyl;
(5) heteroarylalkyl;
(6) substituted heteroarylalkyl;
(7) heteroarylalkenyl and
(8) substituted heteroarylalkenyl;

wherein said substituted $R^9$ groups are substituted with substituents independently selected from:

(1) -OH;
(2) $-CO_2R^{14}$;
wherein, $R^{14}$ is selected from: H or alkyl (e.g., methyl or ethyl), preferably alkyl, and most preferably methyl or ethyl;
(3) alkyl, substituted with one or more -OH groups
(e.g.,1, 2, or 3, preferably 1), for example $-(CH_2)qOH$ wherein, q is 1 - 4, with q = 1 being preferred.
(4) halo (e.g., Br or Cl);
(5) alkyl, usually C1-C6 alkyl, preferably C1-C4 alkyl
(e.g. methyl, ethyl, propyl, isopropyl, butyl or t-butyl, most preferably t-butyl);
(6) amino;
(7) trityl;
(8) heterocycloalkyl;
(9) arylalkyl;
(10) heteroaryl and
(11) heteroarylalkyl;

More preferably, $R^9$ is selected from:

(1) heterocycloalkyl;
(2) substituted heterocycloalkyl;
(3) heterocycloalkylalkyl;
(4) substituted heterocycloalkylalkyl;
(5) heteroarylalkyl;
(6) substituted heteroarylalkyl;
(7) heteroarylalkenyl and
(8) substituted heteroarylalkenyl;

wherein substituents for said substituted $R^9$ groups are each independently selected from:

(1) halo (e.g., Br, or Cl);
(2) alkyl, usually C1-C6 alkyl, preferably C1-C4 alkyl
(e.g. methyl, ethyl, propyl, isopropyl, butyl or t-butyl, most preferably t-butyl);
(3) alkyl, substituted with one or more (i.e. 1, 2, or 3, preferably 1) - OH groups, (e.g. $-(CH_2)qOH$ wherein q is 1-4, with q=1 being preferred).
(4) amino;
(5) trityl;
(6) arylalkyl, and
(7) heteroarylalkyl.

Even more preferably, $R^9$ is selected from:

(1) heterocycloalkylalkyl;
(2) substituted heterocycloalkylalkyl;
(3) heteroarylalkyl and
(4) substituted heteroarylalkyl;

wherein substituents for said substituted $R^9$ groups are each independently selected from:

(1) halo (e.g., Br, or Cl);
(2) alkyl, usually C1-C6 alkyl, preferably C1-C4 alkyl
(e.g. methyl, ethyl, propyl, isopropyl, butyl or t-butyl, most preferably t-butyl);
(3) amino and
(4) trityl.

Still more preferably, $R^9$ is selected from:

(1) heterocycloalkylalkyl;
(2) substituted heterocycloalkylalkyl;
(3) heteroarylalkyl and
(4) substituted heteroarylalkyl;

wherein substituents for said substituted $R^9$ groups are each independently selected from:

(1) halo (e.g., Br,or Cl) and
(2) alkyl, usually C1-C6 alkyl, preferably C1-C4 alkyl
(e.g. methyl, ethyl, propyl, isopropyl, butyl or t-butyl, most preferably t-butyl).

Yet even more preferably, $R^9$ is selected from:

(1) piperidinyl;
(2) piperizinyl;
(3) -(CH2)p-piperidinyl;
(4) -(CH2)p-piperizinyl;
(5) -(CH2)p-morpholinyl and
(6) -(CH2)p-imidazolyl;

wherein p is 0 to 1, and wherein the ring moiety of each $R^9$ group is optionally substituted with one, two or three substituents independently selected from:

(1) halo (e.g., Br,or Cl) and
(2) alkyl, usually C1-C6 alkyl, preferably C1-C4 alkyl
(e.g. methyl, ethyl, propyl, isopropyl, butyl or t-butyl, most preferably t-butyl).

Still more preferably, $R^9$ is selected from:

(1) -piperizinyl;
(2) -(CH2)p-piperidinyl;
(3) -(CH2)p-imidazolyl; and
(4) -(CH2)p-morpholinyl,

wherein p is 1 to 4, and the ring moiety of each $R^9$ group is optionally substituted with one, two or three substituents independently selected from: methyl, ethyl, and isopropyl.

Yet even more preferably, $R^9$ is selected from $-(CH_2)$-Imidazolyl, wherein said imidazolyl ring is optionally substituted with 1, 2, or 3 substituants, preferably 1, independently selected from methyl or ethyl.

Still even more preferably, $R^9$ is selected from $-(CH_2)$-(2-methyl)-imidazole.

Preferably, at least one of $R^{21}$, $R^{22}$ and $R^{46}$ is other than H or alkyl. More preferably, $R^{21}$ and $R^{22}$ is H and $R^{46}$ is other than H or alkyl. Most preferably, $R^{21}$ and $R^{22}$ is H and $R^{46}$ is selected from heteroaryl or heterocycloalkyl.

Preferably, said heteroaryl groups for said $R^{21}$, $R^{22}$ or $R^{46}$ is 3-pyridyl, 4-pyridyl, 3-pyridyl-N-Oxide or 4-pyridyl-N-Oxide; more preferably 4-pyridyl or 4-pyridyl- N-Oxide; most preferably 4-pyridyl- N-Oxide.

Preferably, said heterocycloalkyl groups for said $R^{21}$, $R^{22}$, or $R^{46}$ is piperidine Ring V:

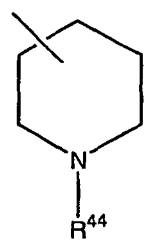

wherein $R^{44}$ is $-C(O)NHR^{51}$, and preferably $R^{51}$ is $-C(O)NH_2$. More preferably, piperidine Ring V is:

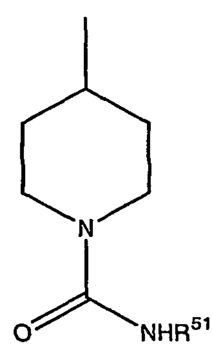

and most preferred Ring V is:

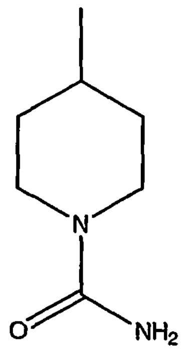

Thus, $R^{21}$, $R^{22}$ and $R^{46}$ are preferably independently selected from:

(1) H;
(2) aryl (most preferably phenyl);
(3) heteroaryl and
(4) heterocycloalkyl (i.e., Piperidine Ring V)

wherein at least one or $R^{21}$, $R^{22}$, or $R^{46}$ is other than H, and most preferably $R^{21}$ and $R^{22}$ are H and $R^{46}$ is other than H, and more preferably $R^{21}$ and $R^{22}$ are H and $R^{46}$ is selected from heteroaryl or heterocycloalkyl, and still more preferably $R^{21}$ and $R^{22}$ are H and $R^{46}$ is Piperidine Ring V; wherein the preferred definitions of heteroaryl and Piperidine Ring V are as described above.

As described above, compounds according to the invention have A and B selected from:

(1) -H;
(2) $-R^9$;
(3) $-R^9$-C(O)-$R^9$;
(4) $-R^9$-$CO_2$-$R^{9a}$;
(5) -C(O)NH$R^9$;
(6) -C(O)NH-$CH_2$-C(O)-$NH_2$;
(7) -C(O)NH$R^{26}$;
(8) -$(CH_2)$p$(R^9)_2$, wherein each $R^9$ is the same or different;
(9) -$(CH_2)$pC(O)$R^9$;
(10) -(CH2)pC(O)$R^{27a}$;
(11) -$(CH_2)$pC(O)N$(R^9)_2$, wherein each $R^9$ is the same or different;
(12) -$(CH_2)$pC(O)NH$(R^9)$;
(13) -$(CH_2)$pNHC(O)$R^{50}$;
(14) -$(CH_2)$pNHC$(O)_2R^{50}$;
(15) -$(CH_2)$pN(C(O)$R^{27a})_2$ wherein $R^{27a}$ is the same or different;
(16) -$(CH_2)$pN$R^{51}$C(O)$R^{27}$, optionally, $R^{51}$ and $R^{27}$, taken together with the atoms to which they are bound, form a heterocycloalkyl ring consisting of 5 or 6 members, provided that when $R^{51}$ and $R^{27}$ form a ring, $R^{51}$ is not H;
(17) -$(CH_2)$pN$R^{51}$C(O)N$R^{27}$, optionally, $R^{51}$ and $R^{27}$, taken together with the atoms to which they are bound, form a heterocycloalkyl ring consisting or 5 or 6 members, provided that when $R^{51}$ and $R^{27}$ form a ring, $R^{51}$ is not H;
(18) -$(CH_2)$pN$R^{51}$C(O)N$(R^{27a})_2$, wherein each $R^{27a}$ is the same or different;
(19) -$(CH_2)$pNH$SO_2$N$(R^{51})_2$, wherein each $R^{51}$ is the same or different;
(20) -$(CH_2)$pNH$CO_2R^{50}$;
(21) -$(CH_2)$p$CO_2R^{51}$;
(22) -NH$R^9$;
(23)

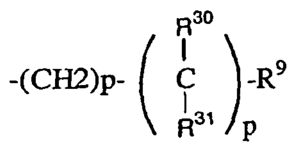

wherein $R^{30}$ and $R^{31}$ are the same or different and
(24)

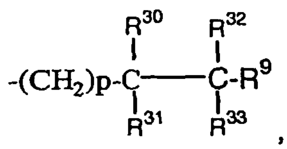

wherein $R^{30}$, $R^{31}$, $R^{32}$ and $R^{33}$ are the same or different.

Preferably in compounds of the invention, A and B are independently selected from:

(1) -H;
(2) $-R^9$;
(3) $-R^9-C(O)-R^9$;
(4) $-R^9-CO_2-R^{9a}$;
(5) $-C(O)NHR^9$;
(6) $-(CH_2)p(R^9)_2$, wherein each $R^9$ is the same or different;
(7) $-(CH_2)pC(O)R^9$;
(8) $-(CH_2)pC(O)N(R^9)_2$, wherein each $R^9$ is the same or different;
(9) $-(CH_2)pC(O)NH(R^9)$;
(10) $-(CH_2)pNR^{51}C(O)R^{27}$, optionally, $R^{51}$ and $R^{27}$, taken together with the atoms to which they are bound, form a heterocycloalkyl ring consisting of 5 or 6 members, provided that when $R^{51}$ and $R^{27}$ form a ring, $R^{51}$ is not H;
(12) $-(CH_2)pNR^{51}C(O)NR^{27}$, optionally, $R^{51}$ and $R^{27}$, taken together with the atoms to which they are bound, form a heterocycloalkyl ring consisting of 5 or 6 members, provided that when $R^{51}$ and $R^{27}$ form a ring, $R^{51}$ is not H and
(13) $-NHR^9$.

Examples of A and B include but are not limited to:

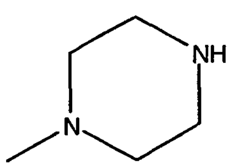 ,
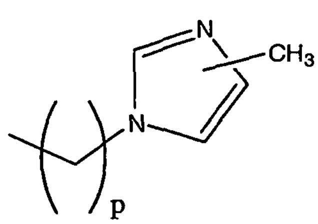 ,
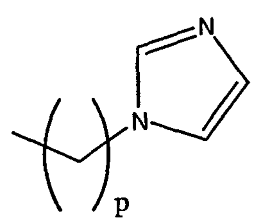 ,
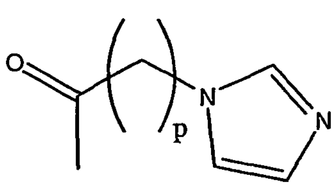 ,
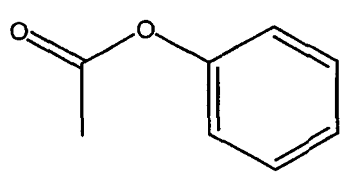 ,
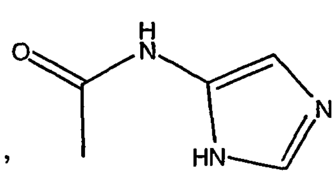 ,

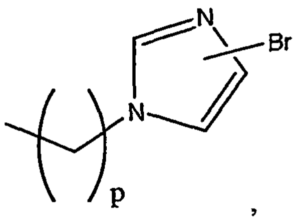, 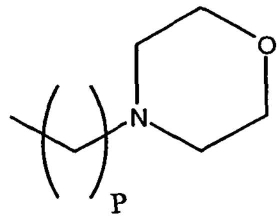, 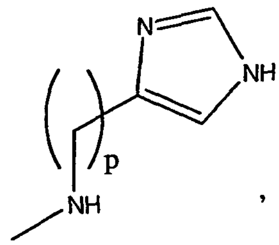,

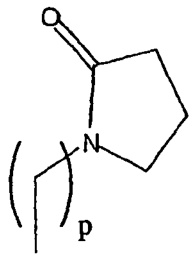, 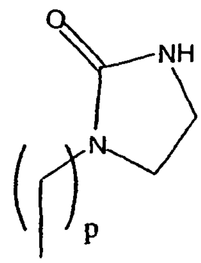, 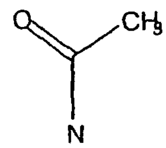,

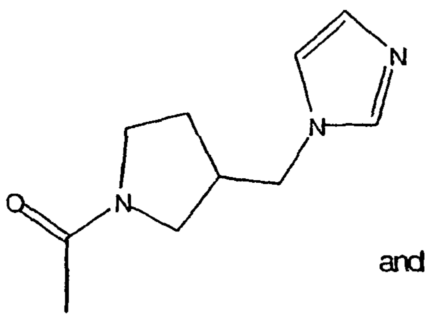 and 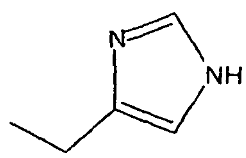

wherein p is 0, 1, 2, 3 or 4;

When the optional bond between C-5 and C-6 is present (i.e., there is a double bond between C-5 and C-6), then preferably one of A or B is H and the other is $R^9$, and preferably, $R^9$ is selected from:

(1) heteroaryl;
(2) substituted heteroaryl;
(3) heterocycloalkyl;
(4) substituted heterocycloalkyl;
(5) heterocycloalkylalkyl;
(6) substituted heterocycloalkylalkyl;
(7) heteroarylalkyl;
(8) substituted heteroarylalkyl;
(9) heteroarylalkenyl and
(10) substituted heteroarylalkenyl,

wherein the substituents for said substituted $R^9$ groups are each independently selected from:

(1) -OH;
(2) $-CO_2R^{14}$;
(3) $-CH_2OR^{14}$,
(4) halo,
(5) alkyl (e.g. methyl, ethyl, propyl, butyl or t-butyl);
(6) amino;
(7) trityl;
(8) heterocycloalkyl;

(9) arylalkyl;
(10) heteroaryl and
(11) heteroarylalkyl,

wherein $R^{14}$ is independently selected from: H; or alkyl, preferably methyl or ethyl.

More preferably, when there is a double bond between C-5 and C-6, A is H and B is $R^9$. Most preferably, when there is a double bond between C-5 and C-6, A is H and B is $R^9$ wherein $R^9$ is selected from:

(1) heterocycloalkyl;
(2) substituted heterocycloalkyl;
(3) heterocycloalkylalkyl;
(4) substituted heterocycloalkylalkyl;
(5) heteroarylalkyl;
(6) substituted heteroarylalkyl;
(7) heteroarylalkenyl and
(8) substituted heteroarylalkenyl,

wherein the substituents for said substituted $R^9$ groups are independently selected from:

(1) -OH;
(2) halo, (preferably Br);
(3) alkyl (e.g. methyl, ethyl, propyl, butyl, or t-butyl);
(4) amino and
(5) trityl.

Still more preferably, when there is a double bond between C-5 and C-6, A is H and B is $R^9$ wherein $R^9$ is selected from:

(1) heterocycloalkylalkyl;
(2) substituted heterocycloalkylalkyl;
(3) heteroarylalkyl and
(4) substituted heteroarylalkyl,

wherein said substituents for said substituted $R^9$ groups are the same or different alkyl groups (e.g., C1-C4 alkyl).

Even more preferably, when there is a double bond between C-5 and C-6, A is H and B is $R^9$ wherein $R^9$ is selected from:

(1) heteroaryl(C1-C3)alkyl and
(2) substituted heteroaryl(C1-C3)alkyl,

wherein the substituents for said substituted $R^9$ group are as defined above.

Yet still more preferably, when there is a double bond between C-5 and C-6, A is H and B is $R^9$ wherein $R^9$ is selected from:

(1) heteroaryl(C1-C3)alkyl, with heteroaryl-$CH_2$- being preferred and
(2) substituted heteroaryl(C1-C3)alkyl, with substituted heteroaryl-$CH_2$- being preferred,

wherein the substituents for said substituted $R^9$ groups are selected from one or more (e.g. 1, 2 or 3) with one being preferred, of the same or different alkyl groups (e.g.,-$CH_3$, -$C_2H_5$, -$C_3H_4$) with -$CH_3$ being preferred.

Even still more preferably, when there is a double bond between C-5 and C-6, A is H and B is $R^9$ wherein $R^9$ is selected from:

(1) -$CH_2$-imidazolyl;
(2) substituted imidazolyl-$CH_2$-;
(3) -$(CH_2)_2$-imidazolyl;
(4) substituted imidazolyl-$(CH_2)_2$-;
(5) -$(CH_2)_3$-imidazolyl;
(6) substituted imidazolyl-$(CH_2)_3$-;

(7) $-CH_2$-piperazinyl and
(8) $-CH_2$-morpholinyl;

wherein the substituents for said substituted $R^9$ groups are selected from one or more (e.g. 1, 2 or 3), with one being preferred, of the same or different alkyl groups (e.g., $-CH_3$, $-C_2H_5$, $-C_3H_4$) with $-CH_3$ being preferred; and wherein, the substituted imidazolyl groups:

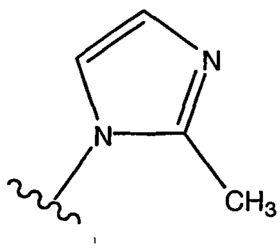
,
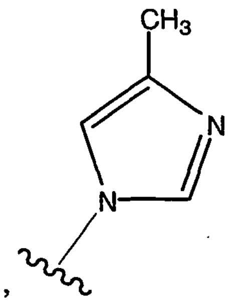
or
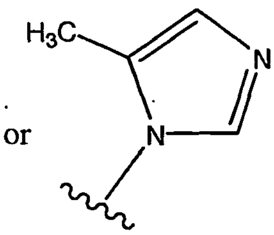

are preferred, with

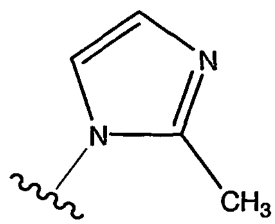

being most preferred.

Yet still more preferably, when there is a double bond between C-5 and C-6, A is H and B is $R^9$ wherein $R^9$ is substituted imidazolyl-$CH_2$-, with

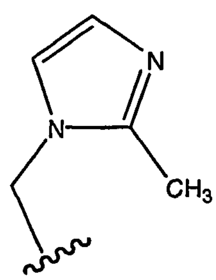

being preferred.

When B is H and A is $R^9$, and there is a double bond between C-5 and C-6, the $R^9$ groups for A are those described above for B.

When the optional bond between C-5 and C-6 is not present (i.e, there is a single bond between C-5 and C-6), each A and each B are independently selected and the definitions of A and B are the same as those described above when the optional bond is present, provided that when there is a single bond between C-5 and C-6 then one of the two A substituents or one of the two B substituents is H (i.e., when there is a single bond between C-5 and C-6 one of the four substituents (A, A, B, and B) has to be H).

Preferably, there is a double bond between C-5 and C-6.

Compounds of this invention having C-11 R- and S- stereochemistry include:

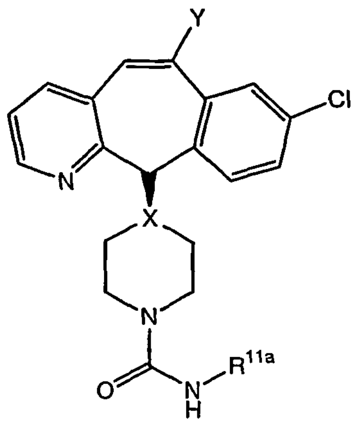
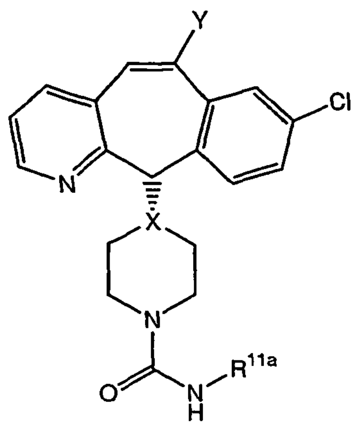

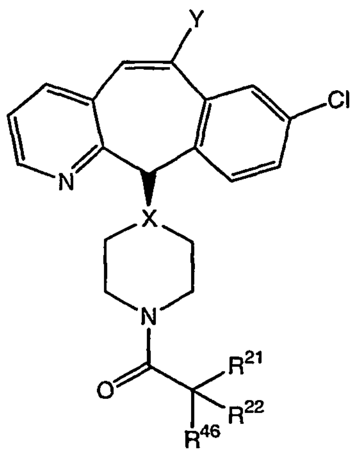
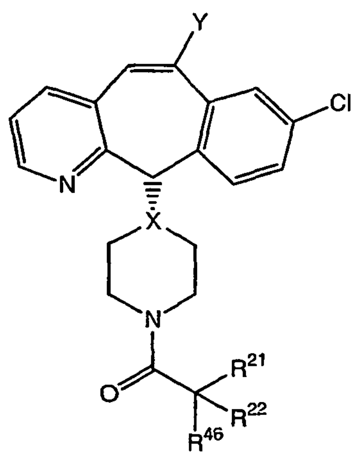

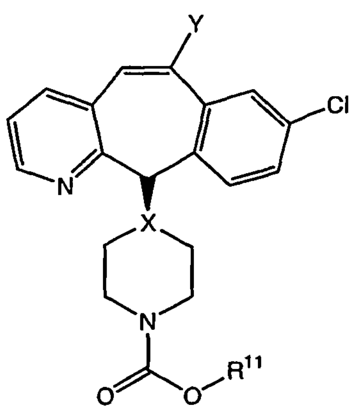
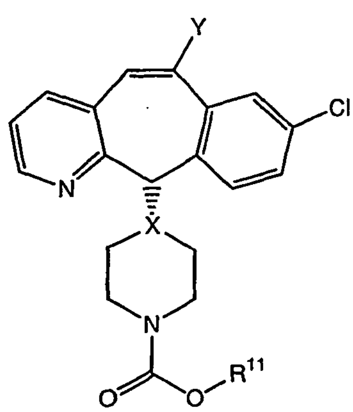

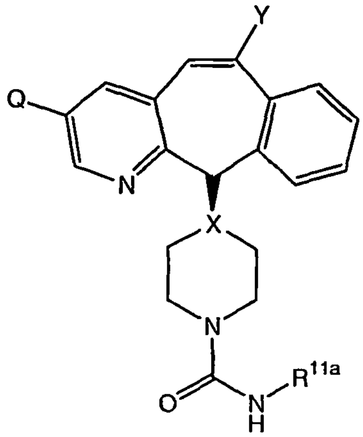

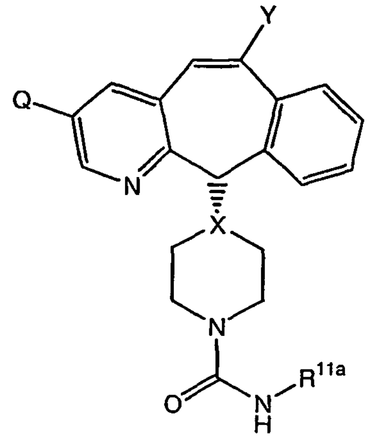

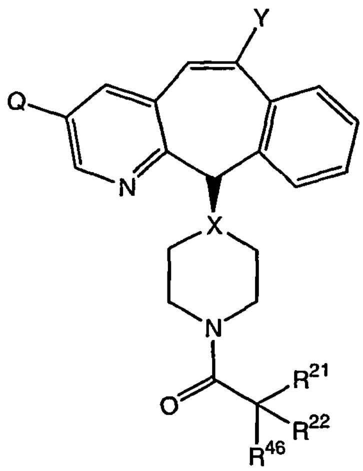

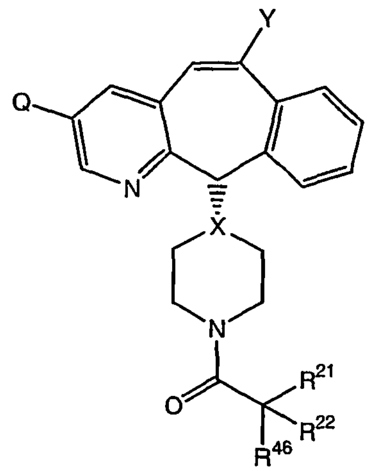

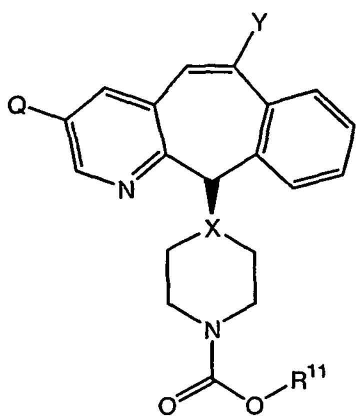

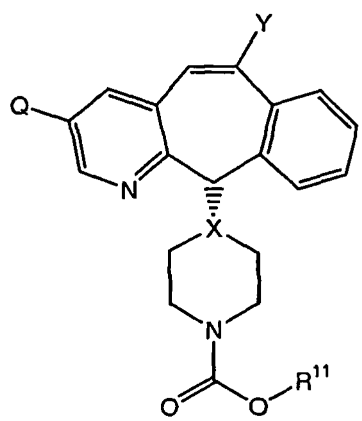

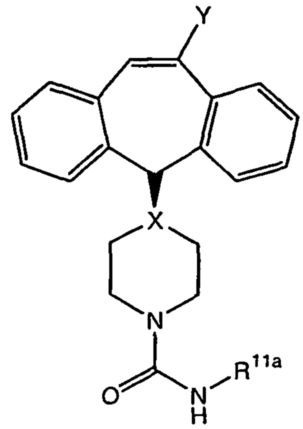

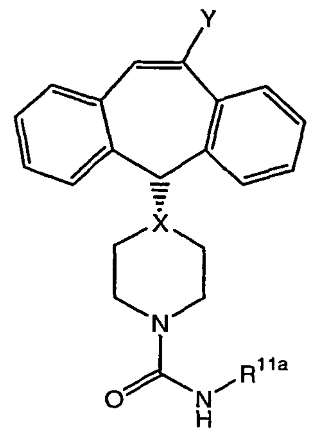

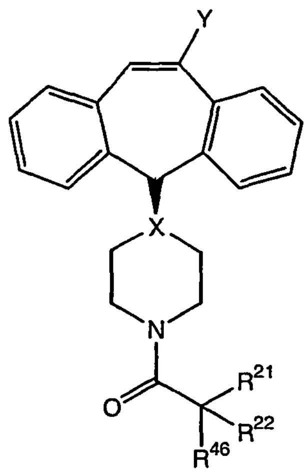

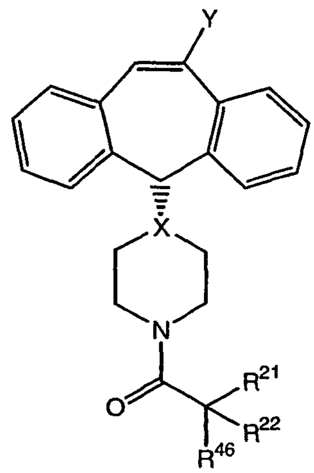

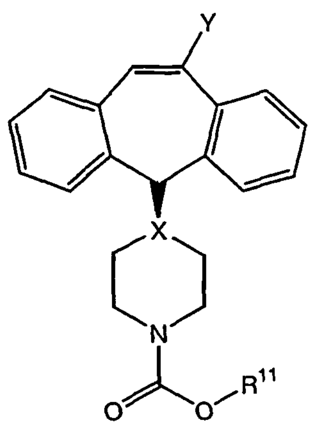

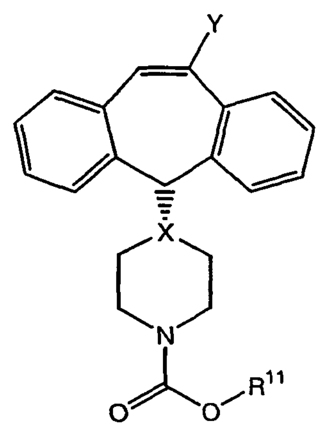

wherein X = N or C;
Q = Br or Cl;
Y = alkyl, arylalkyl, or heteroarylalkyl.

Preferred compounds of this invention are listed below:

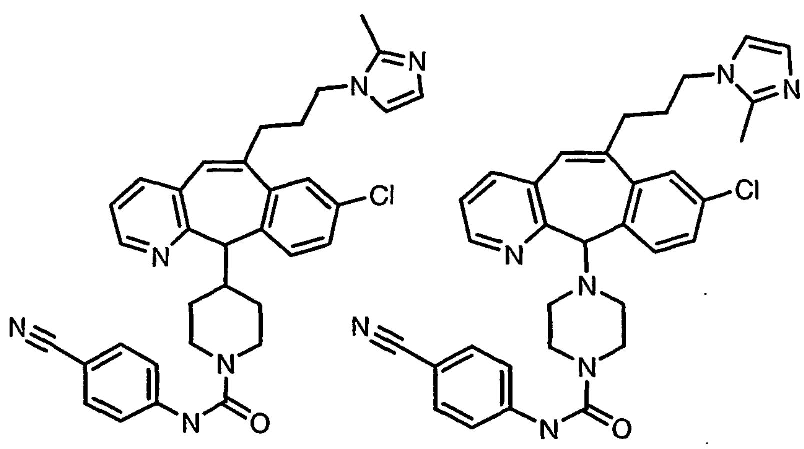

(139) (628)

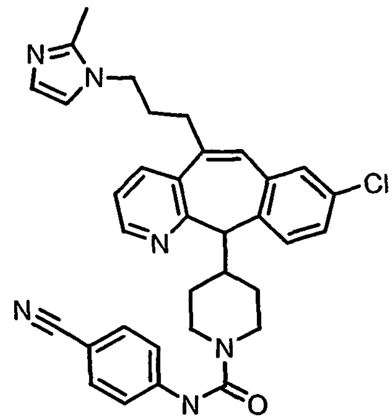

(699)

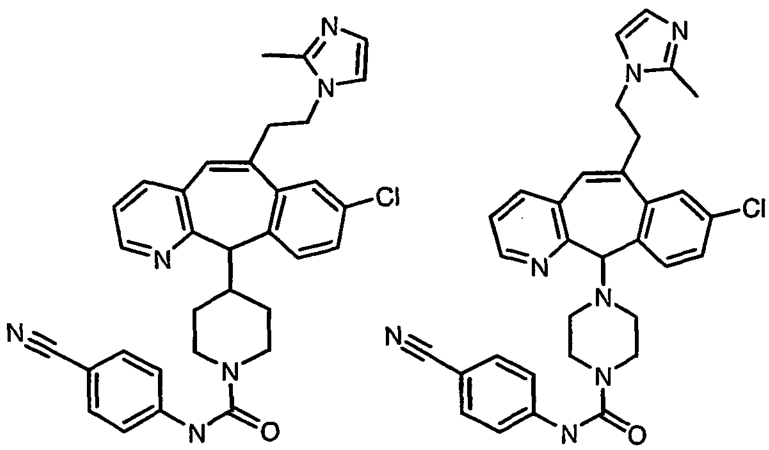

(326) (644)

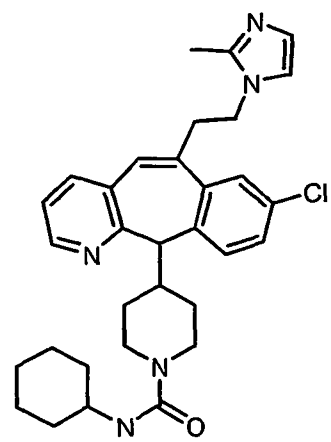

(332)

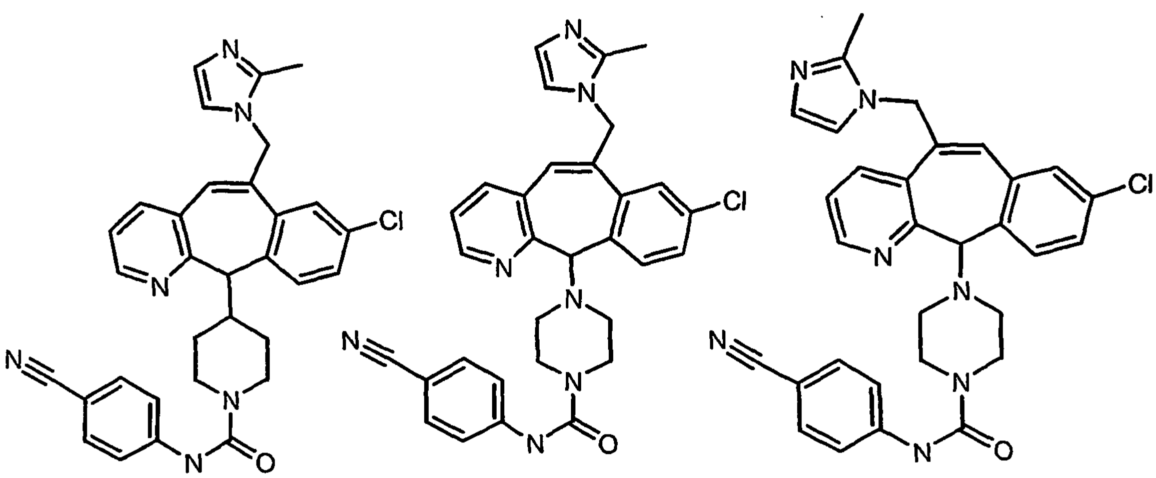

(362a) (372) (230)

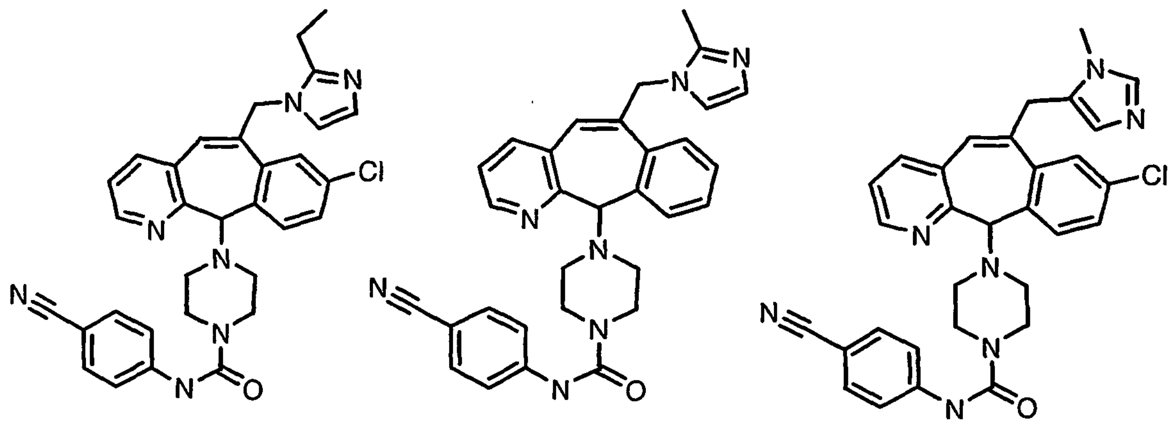

**(378)** **(690)** **(784)**

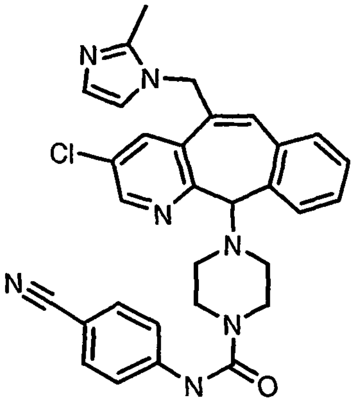

**(684)**

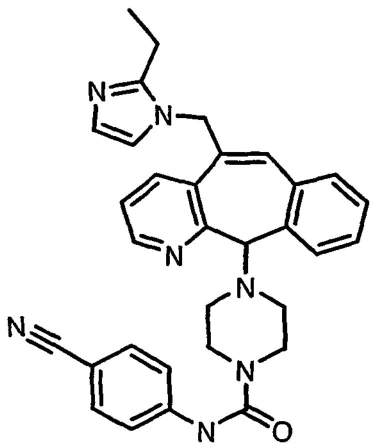

**(688)**

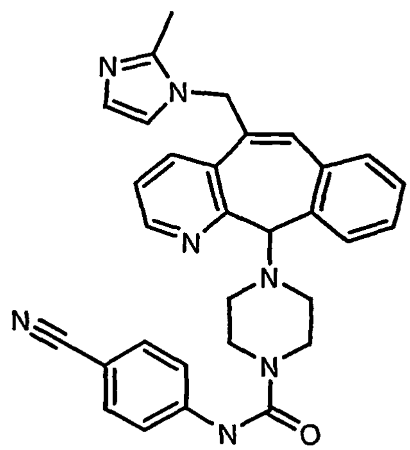

**(686)**

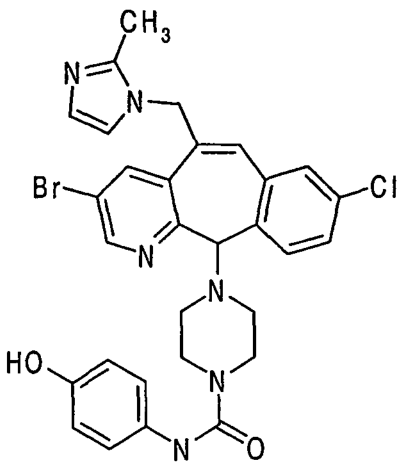

(683.2)

;

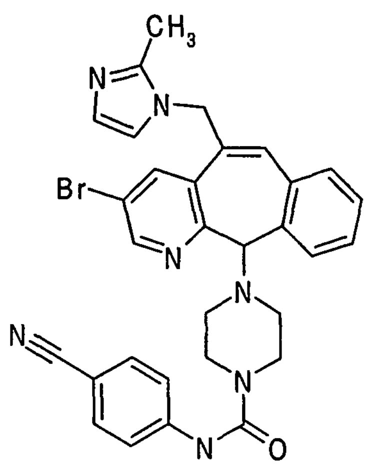

(877)

;

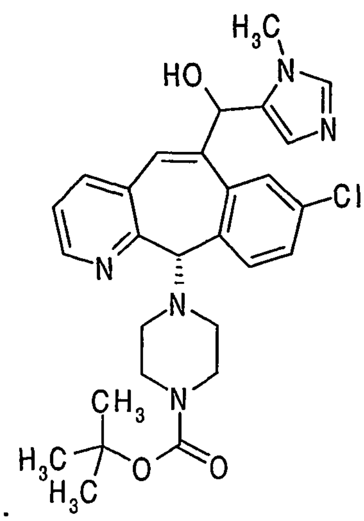

(790)

;

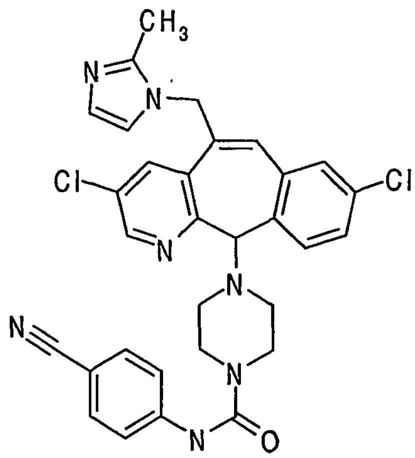

(816);

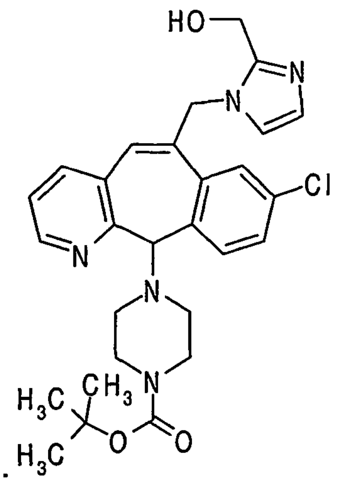

(788);

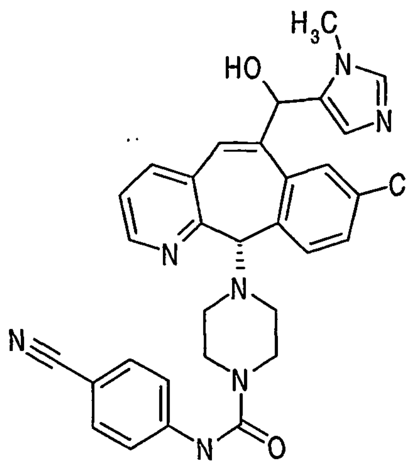

(793);

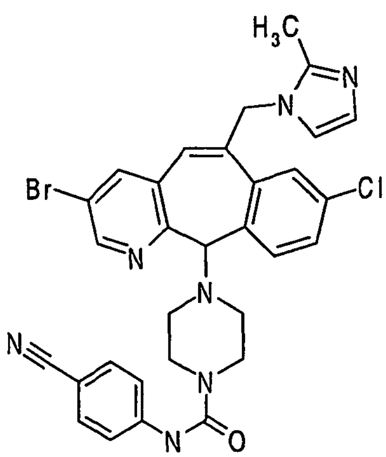

(778);

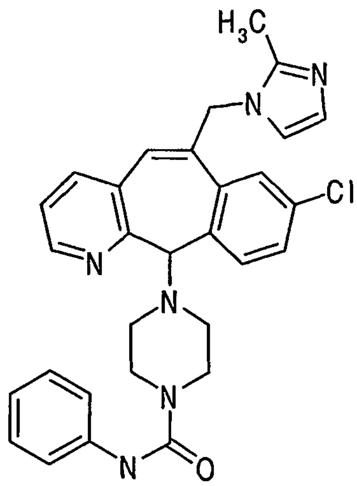

(375.1);

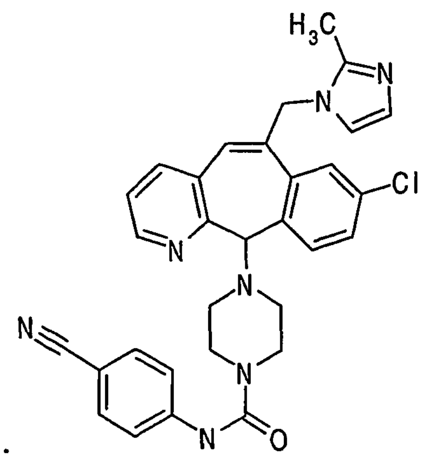

(372).

More preferred compounds of this invention are listed below:

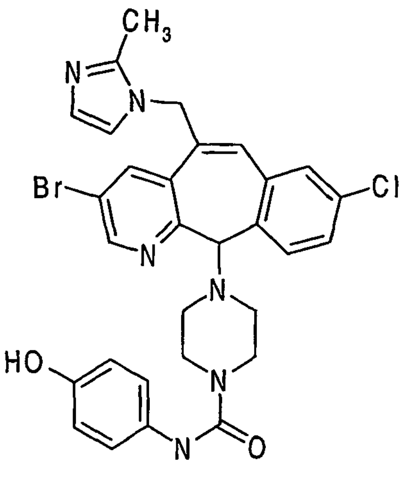

(683.2)

;

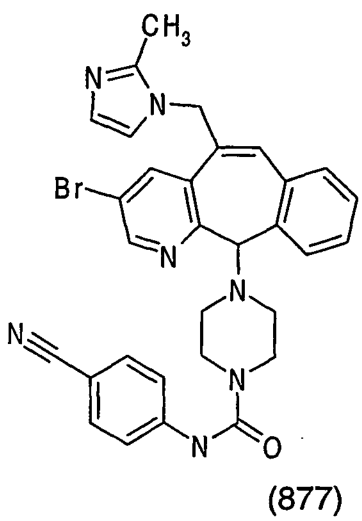

(877)

;

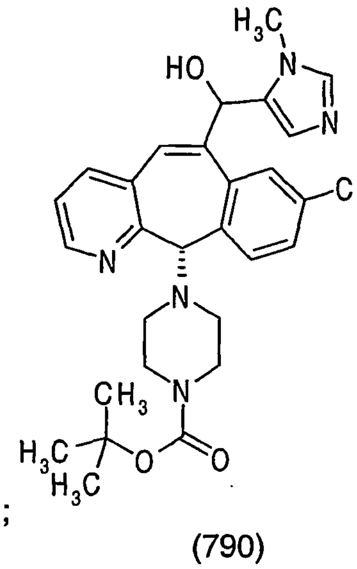

(790)

;

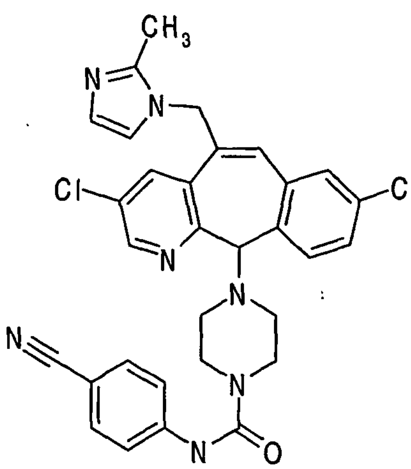

(816)

;

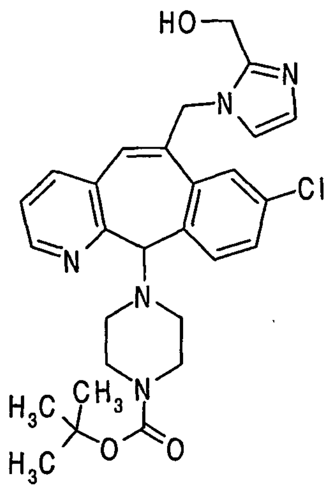

(788)

;

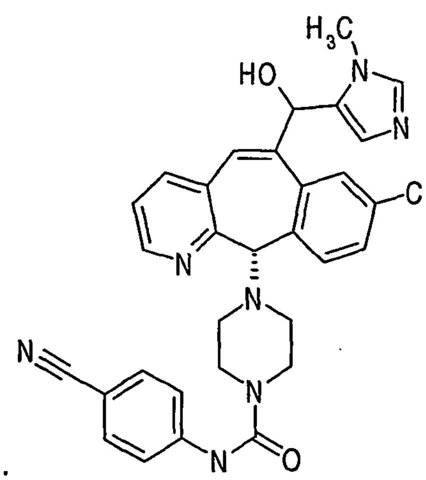

(793)

;

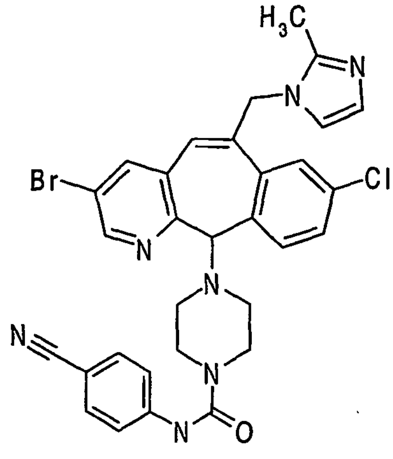

(778)

;

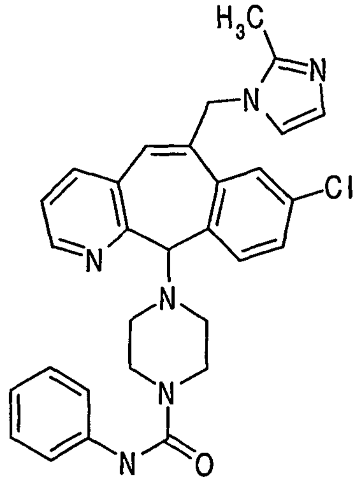

(375.1)

;

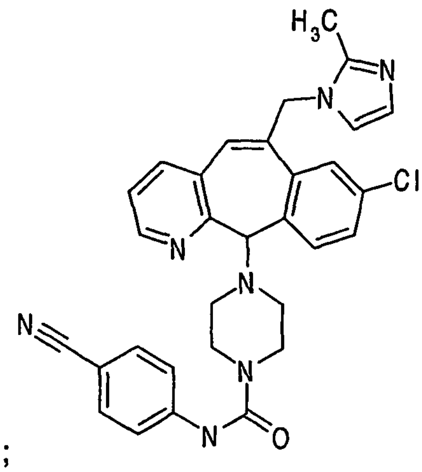

(372)

.

Most preferred compounds of this invention are listed below:

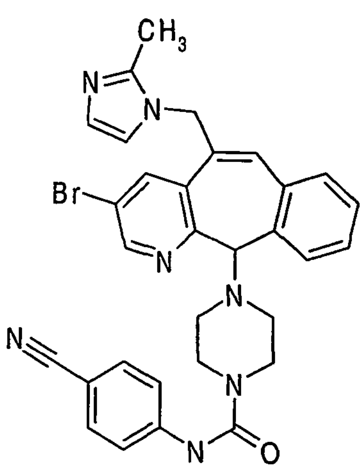
(877)
;

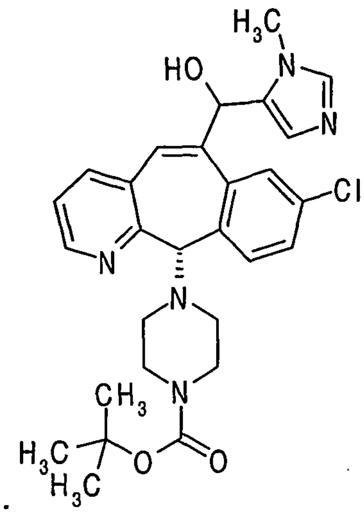
(790)
;

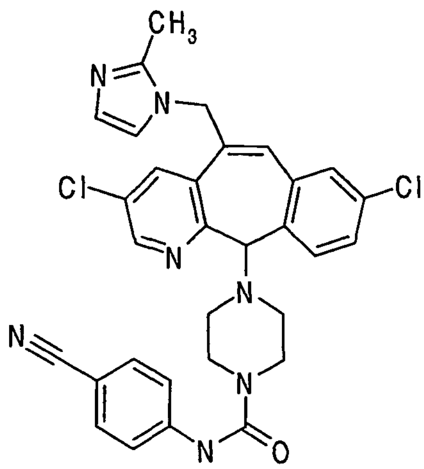
(816)
;

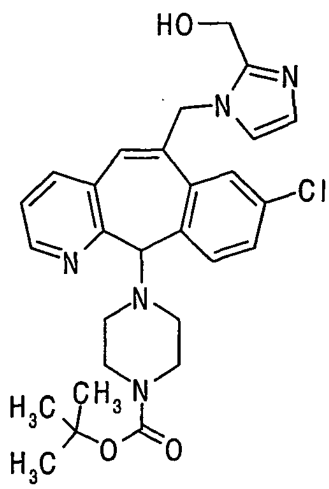
(788)
;

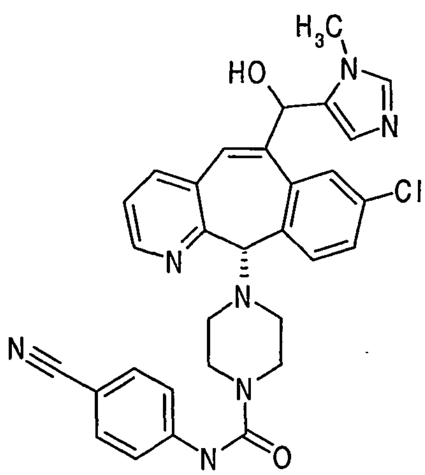
(793)
;

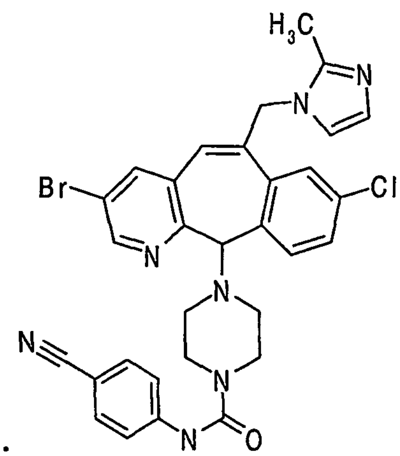
(778)
;

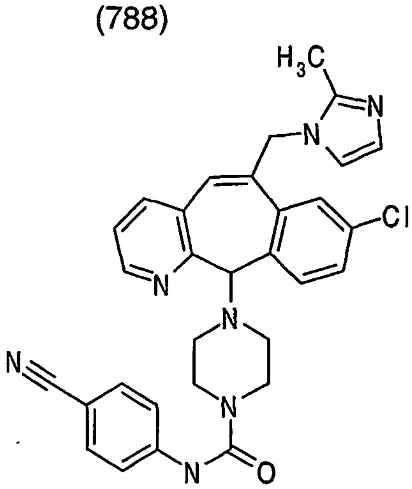
(372a).

Lines drawn into the ring systems indicate that the indicated bond may be attached to any of the substitutable

ring carbon atoms.

Certain compounds of the invention may exist in different isomeric (e.g., enantiomers, diastereoisomers, atropisomers) forms. The invention contemplates all such isomers both in pure form and in admixture, including racemic mixtures. Enol forms are also included.

Certain tricyclic compounds will be acidic in nature, e.g. those compounds which possess a carboxyl or phenolic hydroxyl group. These compounds may form pharmaceutically acceptable salts. Examples of such salts may include sodium, potassium, calcium, aluminum, gold and silver salts. Also contemplated are salts formed with pharmaceutically acceptable amines such as ammonia, alkyl amines, hydroxyalkylamines, N-methylglucamine and the like.

Certain basic tricyclic compounds also form pharmaceutically acceptable salts, e.g., acid addition salts. For example, the pyrido-nitrogen atoms may form salts with strong acid, while compounds having basic substituents such as amino groups also form salts with weaker acids. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those in the art. The salts are prepared by contacting the free base form with a sufficient amount of the desired acid to produce a salt in the conventional manner. The free base forms may be regenerated by treating the salt with a suitable dilute aqueous base solution such as dilute aqueous NaOH, potassium carbonate, ammonia and sodium bicarbonate. The free base forms differ from their respective salt forms somewhat in certain physical properties, such as solubility in polar solvents, but the acid and base salts are otherwise equivalent to their respective free base forms for purposes of the invention.

All such acid and base salts are intended to be pharmaceutically acceptable salts within the scope of the invention and all acid and base salts are considered equivalent to the free forms of the corresponding compounds for purposes of the invention.

The compounds of formula 1.0 can exist in unsolvated as well as solvated forms, including hydrated forms, e.g., hemi-hydrate. In general, the solvated forms, with pharmaceutically acceptable solvents such as water, ethanol and the like are equivalent to the unsolvated forms for purposes of the invention.

The method of treating proliferative diseases (cancer), according to this invention, includes a method for treating (inhibiting) the abnormal growth of cells, including transformed cells, in a patient in need of such treatment (e.g., a mammal such as a human), by administering, concurrently or sequentially, an effective amount of a compound of this invention and an effective amount of a chemotherapeutic agent and/or radiation. Abnormal growth of cells means cell growth independent of normal regulatory mechanisms (e.g., loss of contact inhibition), including the abnormal growth of: (1) tumor cells (tumors) expressing an activated ras oncogene; (2) tumor cells in which the ras protein is activated as a result of oncogenic mutation in another gene; and (3) benign and malignant cells of other proliferative diseases.

In preferred embodiments, the methods of the present invention include methods for treating or inhibiting tumor growth in a patient in need of such treatment (e.g., a mammal such as a human) by administering, concurrently or sequentially, (1) an effective amount of a compound of this invention and (2) an effective amount of at least one antineoplastic agent, microtubule affecting agent and/or radiation therapy. Examples of tumors which may be treated include, but are not limited to, epithelial cancers, e.g., prostate cancer, lung cancer (e.g., lung adenocarcinoma), pancreatic cancers (e.g., pancreatic carcinoma such as, for example, exocrine pancreatic carcinoma), breast cancers, colon cancers (e.g., colorectal carcinomas, such as, for example, colon adenocarcinoma and colon adenoma), ovarian cancer, and bladder carcinoma. Other cancers that can be treated include melanoma, myeloid leukemias (for example, acute myelogenous leukemia), sarcomas, thyroid follicular cancer, and myelodysplastic syndrome. In particular, the proliferative disease (tumor) that may be treated is selected from lung cancer, pancreatic cancer, prostate cancer and myeloid leukemia. Preferrably for the methods of the present invention, the disease (tumor) that may be treated is selected from lung cancer and myeloid leukemia.

The methods of treating proliferative diseases, according to this invention, also include a method for treating (inhibiting) proliferative diseases, both benign and malignant, wherein ras proteins are aberrantly activated as a result of oncogenic mutation in other genes - i.e., the ras gene itself is not activated by mutation to an oncogenic form. This method comprises administering, concurrently or sequentially, an effective amount of a compound of this invention and an effective amount of an antineoplastic agent and/or radiation therapy to a patient in need of such treatment (e.g., a mammal such as a human). Examples of such proliferative diseases which may be treated include: the benign proliferative disorder neurofibromatosis, or tumors in which ras is activated due to mutation or overexpression of tyrosine kinase oncogenes (e.g., neu, src, abl, lck, lyn, fyn).

For radiation therapy, $\gamma$-radiation is preferred.

The methods of treating proliferative diseases (cancer), according to this invention, also include a method for treating (inhibiting) the abnormal growth of cells, including transformed cells, in a patient in need of such treatment (e.g., a mammal such as a human), by administering, concurrently or sequentially, an effective amount of a compound of this invention and an effective amount of at least one signal transduction inhibitor.

Typical signal transduction inhibitors include but are not limited to:

(i) Bcr/abl kinase inhibitors such as, for example, STI 571 (Gleevec);
(ii) Epidermal growth factor (EGF) receptor inhibitor such as, for example, Kinase inhibitors (Iressa, OSI-774) and antibodies (Imclone: C225 [Goldstein et al. (1995), Clin Cancer Res. 1:1311-1318], and Abgenix: ABX-EGF) and
(iii) Her-2/neu receptor inhibitors such as, for example, Herceptin® (trastuzumab).

As used herein the following terms have the following meanings unless indicated otherwise:

antineoplastic agent - a chemotherapeutic agent effective against cancer;

concurrently - (1) simultaneously in time, or (2) at different times during the course of a common treatment schedule; and

sequentially - (1) administration of one component of the method ((a) compound of the invention, or (b) chemotherapeutic agent, signal transduction inhibitor and/or radiation therapy) followed by administration of the other component or components; after adminsitration of one component, the next component can be administered substantially immediately after the first component, or the next component can be administered after an effective time period after the first component; the effective time period is the amount of time given for realization of maximum benefit from the administration of the first component.

The term "in association with" as used herein in reference to the combination therapies of the invention meansthe agents or components are adminstered concurrently or sequentially as defined above.

CHEMOTHERAPEUTIC AGENTS

Classes of compounds that can be used as chemotherapeutic agents (antineoplastic agent/microtubule affecting agents) include but are not limited to: alkylating agents, antimetabolites, natural products and their derivatives, hormones and steroids (including synthetic analogs), and synthetics. Examples of compounds within these classes are given below.

Alkylating agents (including nitrogen mustards, ethylenimine derivatives, alkyl sulfonates, nitrosoureas and triazenes): Uracil mustard, Chlormethine, Cyclophosphamide (Cytoxan®), Ifosfamide, Melphalan, Chlorambucil, Pipobroman, Triethylene-melamine, Triethylenethiophosphoramine, Busulfan, Carmustine, Lomustine, Streptozocin, Dacarbazine, and Temozolomide.

Antimetabolites (including folic acid antagonists, pyrimidine analogs, purine analogs and adenosine deaminase inhibitors): Methotrexate, 5-Fluorouracil, Floxuridine, Cytarabine, 6-Mercaptopurine, 6-Thioguanine, Fludarabine phosphate, Pentostatine, and Gemcitabine.

Natural products and their derivatives (including vinca alkaloids, antitumor antibiotics, enzymes, lymphokines and epipodophyllotoxins): Vinblastine, Vincristine, Vindesine, Bleomycin, Dactinomycin, Daunorubicin, Doxorubicin, Epirubicin, Idarubicin, paclitaxel (paclitaxel is commercially available as Taxol® and is described in more detail below in the subsection entitled "Microtubule Affecting Agents"), paclitaxel derivatives (e.g. taxotere), Mithramycin, Deoxycoformycin, Mitomycin-C, L-Asparaginase, Interferons (especially IFN-a), Etoposide, and Teniposide.

Hormones and steroids (including synthetic analogs): 17$\alpha$-Ethinylestradiol, Diethylstilbestrol, Testosterone, Prednisone, Fluoxymesterone, Dromostanolone propionate, Testolactone, Megestrolacetate, Tamoxifen, Methylprednisolone, Methyltestosterone, Prednisolone, Triamcinolone, Chlorotrianisene, Hydroxyprogesterone, Aminoglutethimide, Estramustine, Medroxyprogesteroneacetate, Leuprolide, Flutamide, Toremifene, Zoladex.

Synthetics (including inorganic complexes such as platinum coordination complexes): Cisplatin, Carboplatin, Hydroxyurea, Amsacrine, Procarbazine, Mitotane, Mitoxantrone, Levamisole, and Hexamethylmelamine.

Particularly preferred are the antineoplastic agents selected from Cyclophasphamide, 5-Fluorouracil, Temozolomide, Vincristine, Cisplatin, Carboplatin, and Gemcitabine. Most preferrably, the antineoplastic agent is selected from Gemcitabine, Cisplatin and Carboplatin.

Methods for the safe and effective administration of most of these chemotherapeutic agents are known to those skilled in the art. In addition, their administration is described in the standard literature. For example, the administration of many of the chemotherapeutic agents is described in the "Physicians' Desk Reference" (PDR), e.g., 1996 edition (Medical Economics Company, Montvale, NJ 07645-1742, USA).

MICROTUBULE AFFECTING AGENTS

As explained above, the present invention also provides methods of treating diseased cells by contacting the cells with an FPT inhibiting compound of the invention and a microtubule affecting agent (e.g., paclitaxel, a paclitaxel

derivative or a paclitaxel-like compound). As used herein, a microtubule affecting agent is a compound that interferes with cellular mitosis, i.e., having an anti-mitotic effect, by affecting microtubule formation and/or action. Such agents can be, for instance, microtubule stabilizing agents or agents which disrupt microtubule formation.

Microtubule affecting agents useful in the invention are well known to those of skill in the art and include, but are not limited to allocolchicine (NSC 406042), Halichondrin B (NSC 609395), colchicine (NSC 757), colchicine derivatives (e.g., NSC 33410), dolastatin 10 (NSC 376128), maytansine (NSC 153858), rhizoxin (NSC 332598), paclitaxel (Taxol®, NSC 125973), paclitaxel derivatives (e.g., Taxotere, NSC 608832), thiocolchicine (NSC 361792), trityl cysteine (NSC 83265), vinblastine sulfate (NSC 49842), vincristine sulfate (NSC 67574), epothilone A, epothilone, and discodermolide (see Service, (1996) Science, 274:2009) estramustine, nocodazole, MAP4, and the like. Examples of such agents are also described in the scientific and patent literature, see, e.g., Bulinski (1997) J. Cell Sci. 110:3055-3064; Panda (1997) Proc. Natl. Acad. Sci. USA 94:10560-10564; Muhlradt (1997) Cancer Res. 57:3344-3346; Nicolaou (1997) Nature 387: 268-272; Vasquez (1997) Mol. Biol. Cell. 8:973-985; Panda (1996) J. Biol. Chem. 271:29807-29812.

Particularly preferred agents are compounds with paclitaxel-like activity. These include, but are not limited to paclitaxel and paclitaxel derivatives (paclitaxel-like compounds) and analogues. Paclitaxel and its derivatives (e.g. Taxol and Taxotere) are available commercially. In addition, methods of making paclitaxel and paclitaxel derivatives and analogues are well known to those of skill in the art (see, e.g., U.S. Patent Nos: 5,569,729; 5,565,478; 5,530,020; 5,527,924; 5,508,447; 5,489,589; 5,488,116; 5,484,809; 5,478,854; 5,478,736; 5,475,120; 5,468,769; 5,461,169; 5,440,057; 5,422,364; 5,411,984; 5,405,972; and 5,296,506).

More specifically, the term "paclitaxel" as used herein refers to the drug commercially available as Taxol® (NSC number: 125973). Taxol® inhibits eukaryotic cell replication by enhancing polymerization of tubulin moieties into stabilized microtubule bundles that are unable to reorganize into the proper structures for mitosis. Of the many available chemotherapeutic drugs, paclitaxel has generated interest because of its efficacy in clinical trials against drug-refractory tumors, including ovarian and mammary gland tumors (Hawkins (1992) Oncology, 6: 17-23, Horwitz (1992) Trends Pharmacol. Sci. 13: 134-146, Rowinsky (1990) J. Natl. Canc. Inst. 82: 1247-1259).

Additional microtubule affecting agents can be assessed using one of many such assays known in the art, e.g., a semiautomated assay which measures the tubulin-polymerizing activity of paclitaxel analogs in combination with a cellular assay to measure the potential of these compounds to block cells in mitosis (see Lopes (1997) Cancer Chemother. Pharmacol. 41:37-47).

Generally, activity of a test compound is determined by contacting a cell with that compound and determining whether or not the cell cycle is disrupted, in particular, through the inhibition of a mitotic event. Such inhibition may be mediated by disruption of the mitotic apparatus, e.g., disruption of normal spindle formation. Cells in which mitosis is interrupted may be characterized by altered morphology (e.g., microtubule compaction, increased chromosome number, etc.).

In a preferred embodiment, compounds with possible tubulin polymerization activity are screened in vitro. In a preferred embodiment, the compounds are screened against cultured WR21 cells (derived from line 69-2 wap-ras mice) for inhibition of proliferation and/or for altered cellular morphology, in particular for microtubule compaction. In vivo screening of positive-testing compounds can then be performed using nude mice bearing the WR21 tumor cells. Detailed protocols for this screening method are described by Porter (1995) Lab. Anim. Sci., 45(2):145-150.

Other methods of screening compounds for desired activity are well known to those of skill in the art. Typically such assays involve assays for inhibition of microtubule assembly and/or disassembly. Assays for microtubule assembly are described, for example, by Gaskin et al. (1974) J. Molec. Biol., 89: 737-758. U.S. Patent No. 5,569,720 also provides in vitro and in vivo assays for compounds with paclitaxel-like activity.

Methods for the safe and effective administration of the above-mentioned microtubule affecting agents are known to those skilled in the art. In addition, their administration is described in the standard literature. For example, the administration of many of the chemotherapeutic agents is described in the "Physicians' Desk Reference" (PDR), e.g., 1996 edition (Medical Economics Company, Montvale, NJ 07645-1742, USA).

US-A-6 071 907 describes pyridyl tricyclic compounds useful as FPT inhibitors.

## General Preparative Schemes

The following processes may be employed to produce compounds of the invention.

## Pyridyl Tricyclic Compounds

One skilled in the art will appreciate that the compounds of the invention represented by Formula 1, wherein one of a, b, c or d is N or $N^+$-$O^-$ can be prepared according to the following schemes:

**Scheme 1:**

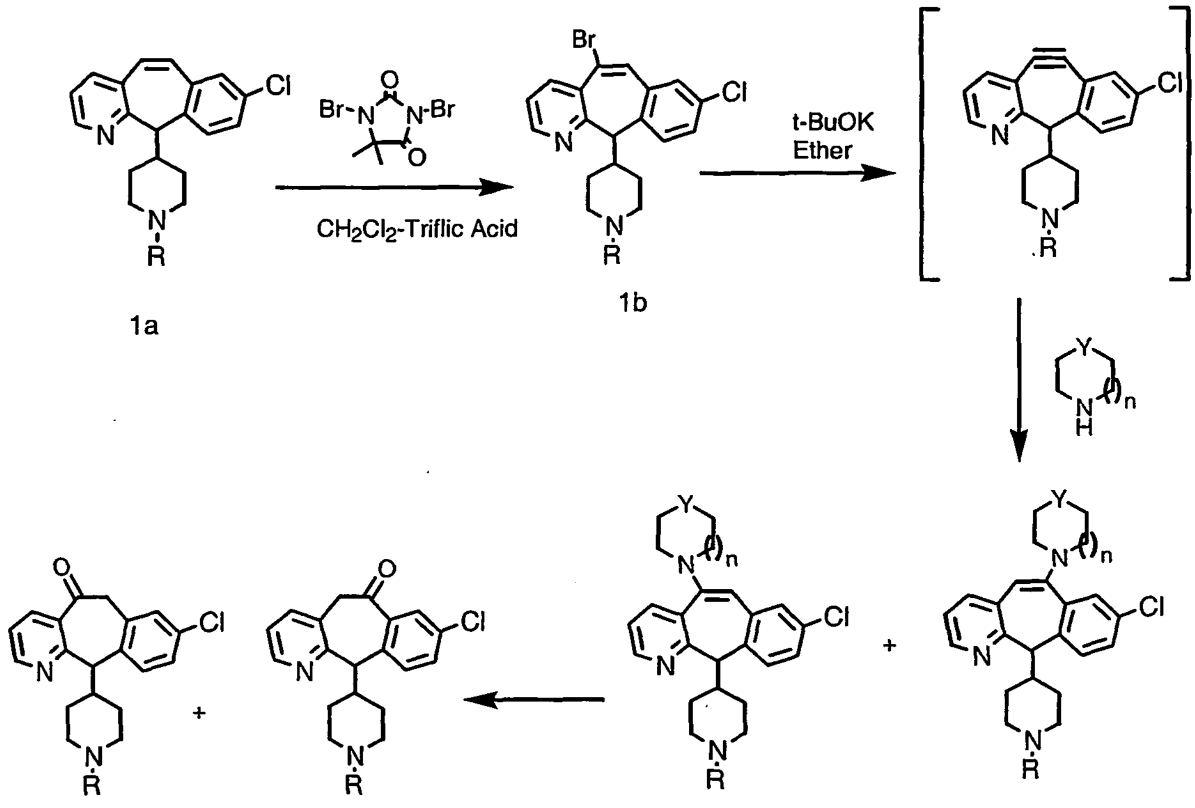


1a 1b 1c 1d 1e 1f

The synthesis of 5-bromo tricyclic compound **1b** begins with bridgehead olefin **1a** (*J. Med Chem* (1998), **41**, 1561-1567) which is treated with dibromo dimethylhydantoin in triflic acid media. Further treatment of the vinylbromide with potassium t-butoxide in the presence of the appropriate secondary amine gives the 5 and 6-substituted enamine adducts. When Y is NH (piperazine case), acylations, sulfonylations and amide formation can be carried out using standard procedures. Treatment of these amine adducts with HCl(aq) at the appropriate temperatures results in the formation of the 5 and 6 azaketones, 1f and 1e respectively.

**Scheme 2**

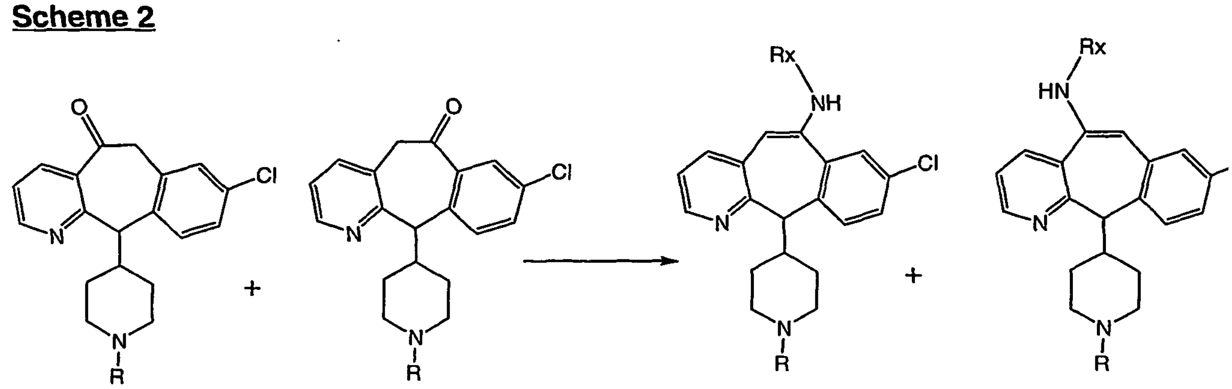

1f 1e 2a 2b

In cases where secondary enamines were required, synthesis from 1f and 1e-azaketones were utilized as outlined in scheme 2. Thus, the appropriate ketone and amine was refluxed in toluene in the presence of p-toluene sulfonic acid in a Dean Stark apparatus.

**Scheme 3:**

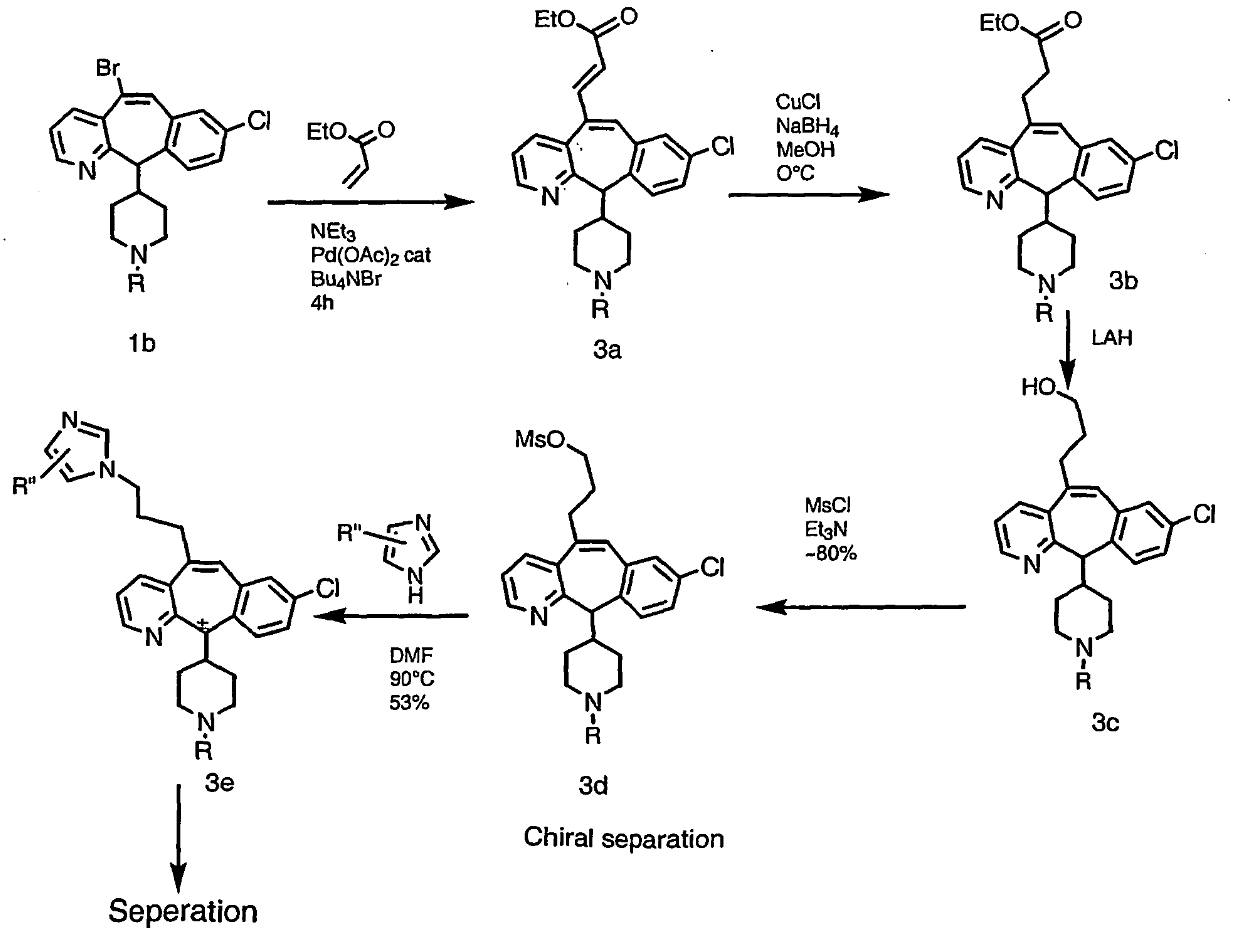


Synthesis of 3-carbon spaced analogs can be prepared as outlined in scheme 3. Thus, subjecting tricyclic vinyl bromide 1b to a *Heck type* reaction using ethyl acrylate and catalyzed by $Pd^0$ gives the α-β un-saturated ester 3a. Reduction of the conjugated double bond was carried out using copper chloride-sodium borohydride reducing reagent. The ester was further reduced to alcohol using lithium aluminum hydride. Treatment of the alcohol with methanesulfonyl chloride in an appropriate aprotic solvent, followed by displacement with an appropriate sodium salt resulted in the desired imidazole targets. In most cases, separation of isomers were effected at this point. Where the R group of 3e was a BOC group, deprotection using HCl-dioxane gave the hydrochloride salts of amines. Using standard chemistry, these amines were converted to ureas, carbamates, sulfonamides and amides.

## Scheme 4: PREPARATION OF 6-SUBSTITUTED CARBON ANLOGUES:

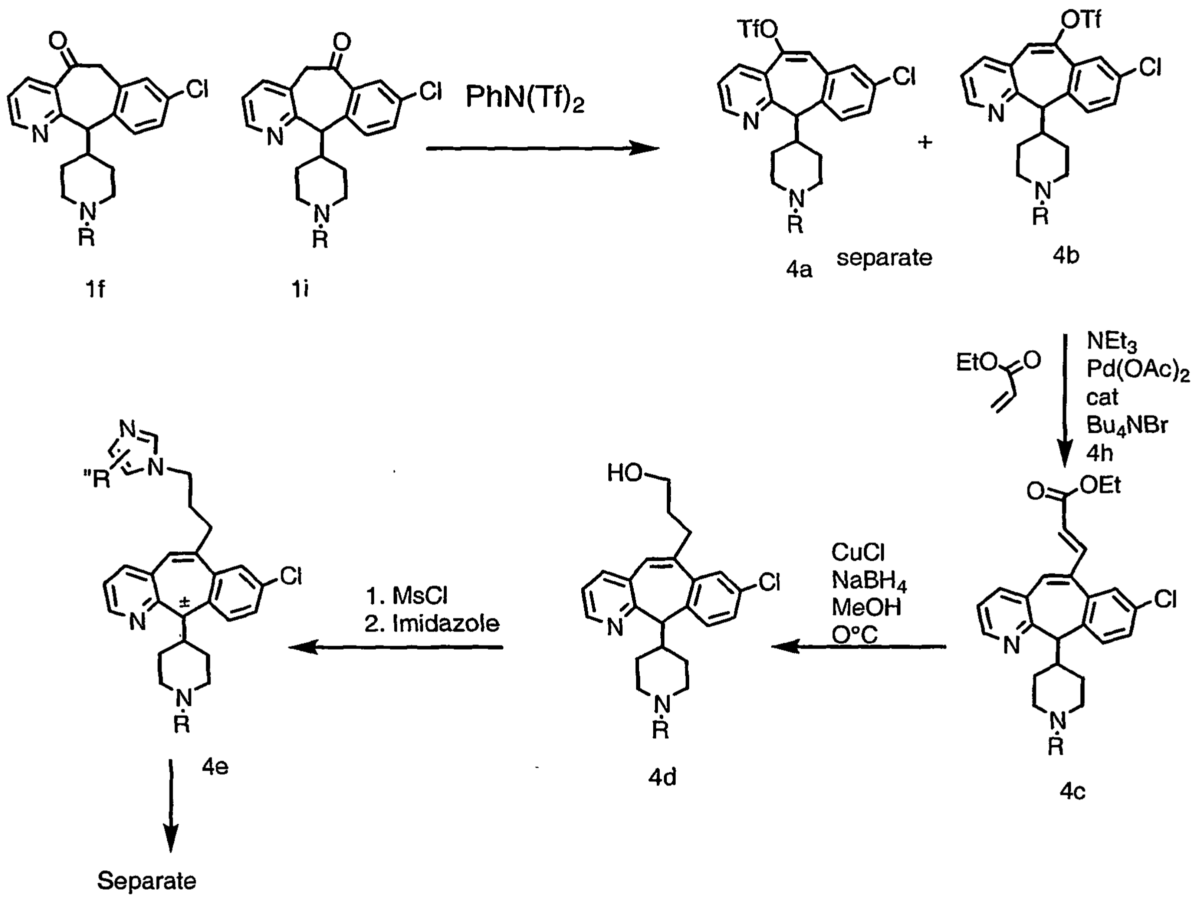


Preparation of 6-substituted 3-carbon spaced imidazole compounds was carried out as outlined in scheme 4. A mixture of ketones 1f and 1i were treated with N-phenytrifluoromethane sulfonimide to give a seperable mixture of 5 and 6-tricyclic triflate compounds. The 6-trilate adduct was converted to the desired 3-carbon spaced analogs using similar protocol as described for the 5-bromo tricyclic compounds outlined in scheme 3.

## Scheme 5: SYNTHESIS OF 2-CARBON SPACER ANALOGUES

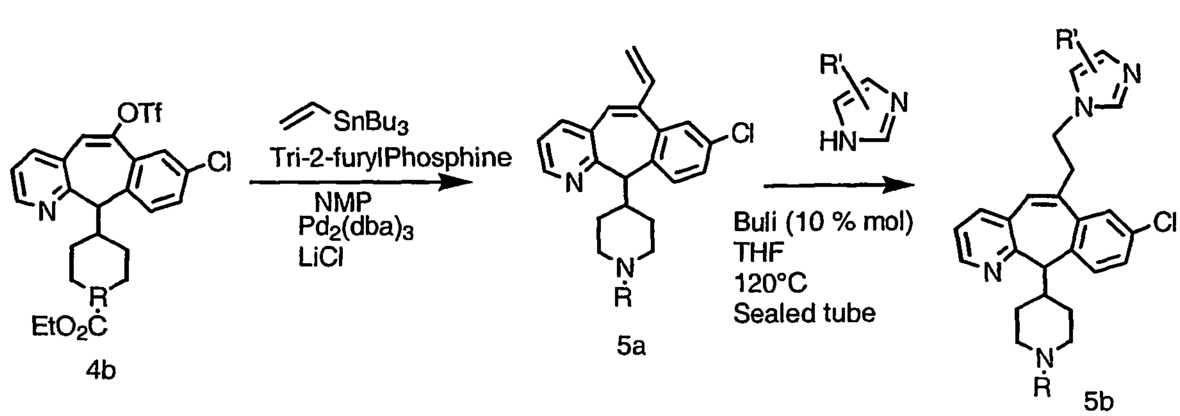

Two carbon spaced analogs were prepared as outlined in scheme 5. Thus, triflate 4b was subjected to Stille chemistry, by reacting with tributylvinyl stannate catalyzed by an appropriate Pd$^0$ to afford the tricyclic vinyl compound 5b. The 2-carbon spaced compounds were obtained by treating the tricylic compound with the appropriate imidazole that had been previously treated with Buli-THF in a sealed tube and refluxed at 120 °C. Further funtionalization was carried out as previously described. Suberane compounds were prepared in a similar way.

**Scheme 6:**

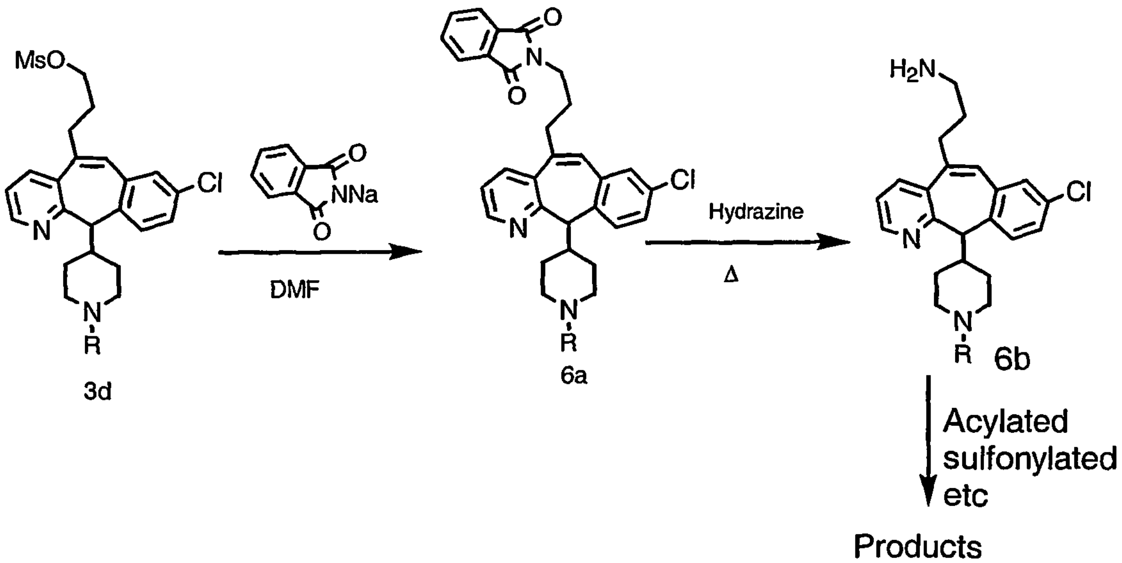


3d

6a

6b

Products

Scheme 6 illustrates method of making amine 6b through phthalimido displacement of a mesylate followed by hydazine hydrolysis of the phthalimido moiety. Amine 6b can be converted to targets that have acyl, sufonyl, carbamoyl and urea functionalities.

**Scheme 7:**

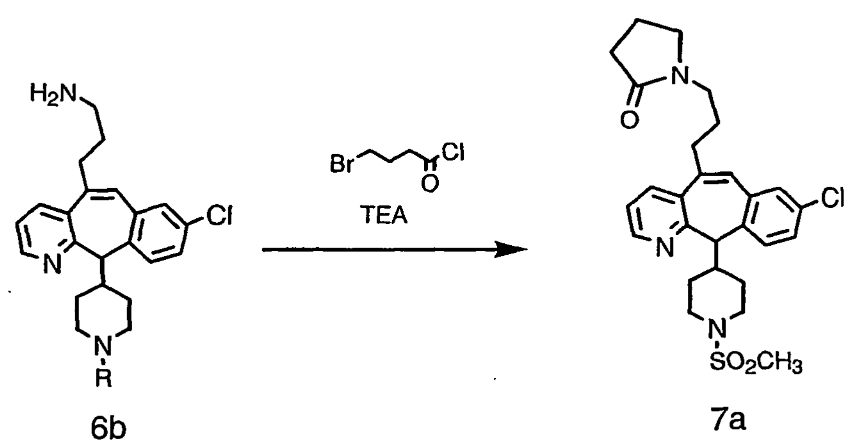


6b

7a

Lactams 7a can be prepared from amine 6b by reacting with bromo butanonyl acid chloride as outlined in scheme 7.

## Scheme 8: Preparation of cyclic ureas

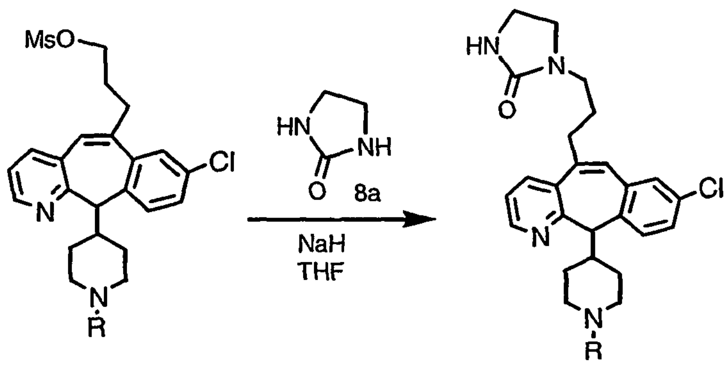


8b

Cyclic urea can be prepared from the mesylate shown above by treating with the salt of the cyclic urea 8a as outlined in scheme 8.

## Scheme 9: PREPARATION OF 5-SUSTITUTED PROPANOIC ACID DERIVATIVES

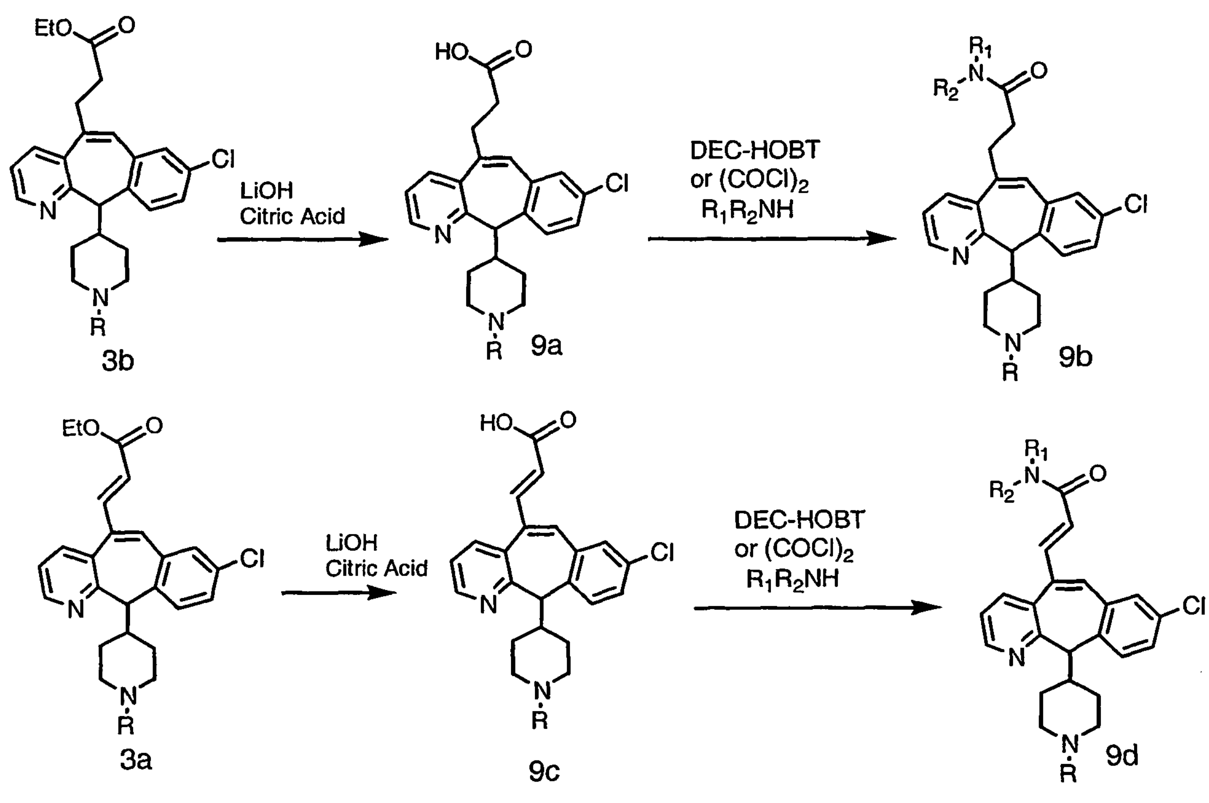


3b 9a 9b

3a 9c 9d

Amides from 3-carbon spaced carboxylic acid 9a and 9c can be prepared as outlined in scheme 10 using either DEC-HOBT mediated protocol or from the appropriate acid chloride.

**Scheme 10:**

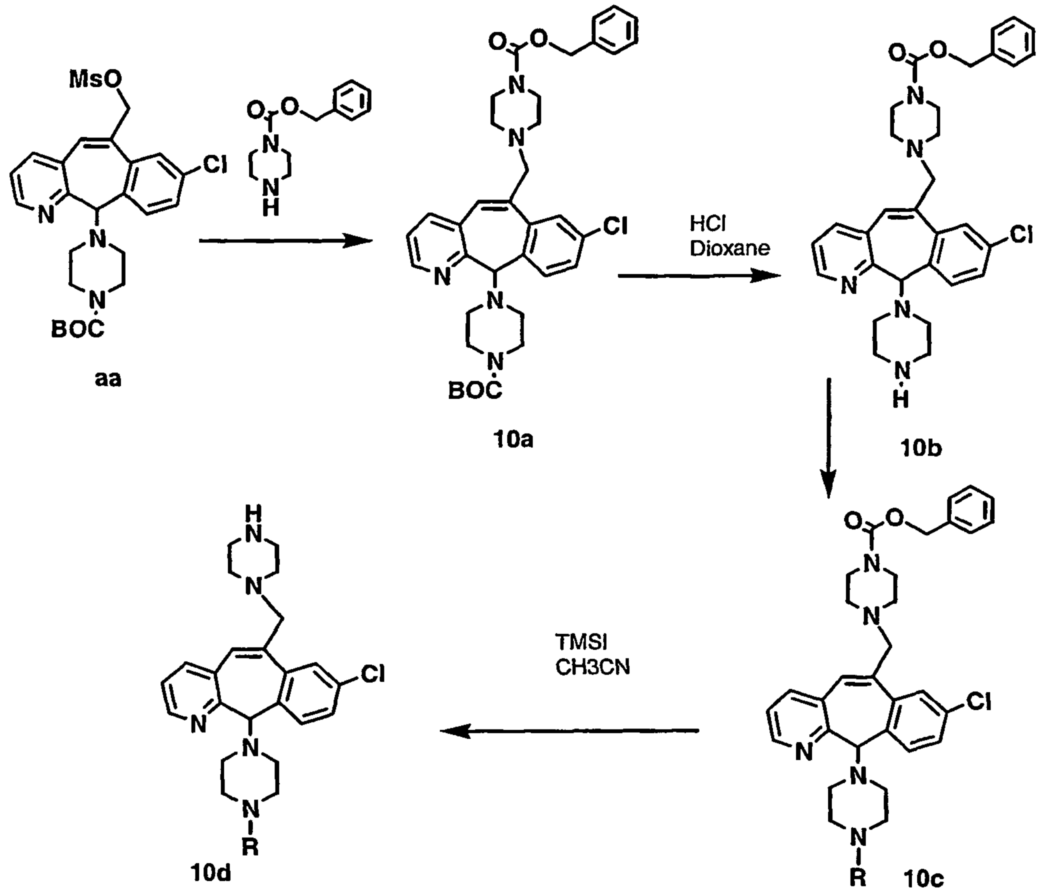


Preparation of piperazine compounds off the bridgehead starts from mesylate aa which is reacted with CBZ-protected piperazine. The BOC group is then removed and the resulting amine 10c is functionalized appropriately. Removal of CBZ group off the piperazine is effected with TMSI.

**Scheme 11: C-SUBSTITUTED IMIDAZOLE-3-METHYLENE-PIPERIDINES**

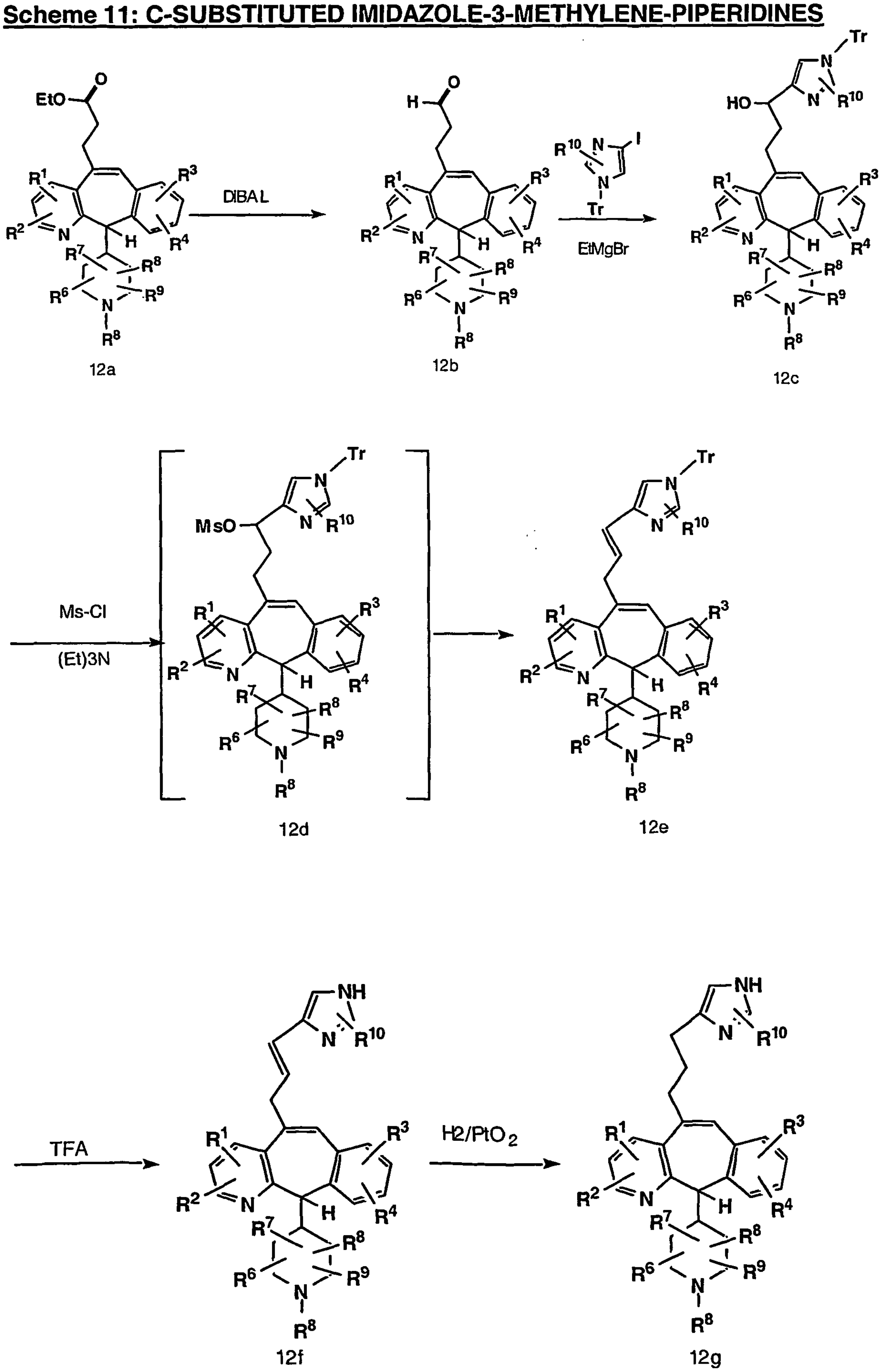


12a 12b 12c 12d 12e 12f 12g

Compound 12a is reduced with DIBAL in an inert solvent such as toluene or tetrahydrofuran to give 12b after acidic workup. Treatment of 12b with an appropriately substituted and tritylated imidazole iodide in the presence of ethylmagnesium bromide in solvents such as dichloromethane at ambient temperature yields the adduct 12c. Elimination of the hydroxyl group by converting the hydroxyl group to an appropriate leaving group such as a mesylate, tosylate, or halide, using methanesulfonyl chloride, p-toluenesulfonyl chloride, or thionyl chloride, followed by elimination using an appropriate base such as triethylamine gives 12e. Removal of the trityl group with acid such as trifluoroacetic acid or hydrochloric acid gives the double bond compound 12f which is then hydrogenated using an appropriate catalyst such as platinum oxide under from 1 to 55 psi of hydrogen in an appropriate solvent such as ethanol gave the desired product 12g.

Alternatively the ester 12a can be saponified with an appropriate base such as lithium hydroxide to obtain the acid 12h. Converting the acid 12h to the "Weinreb amide" followed by reaction with an appropriately substituted and tritylated imidazole iodide in the presence of ethylmagnesium bromide in solvents such as dichloromethane at ambient temperature yields the adduct 12c (shown in Scheme 12 below).

**Scheme 12:**

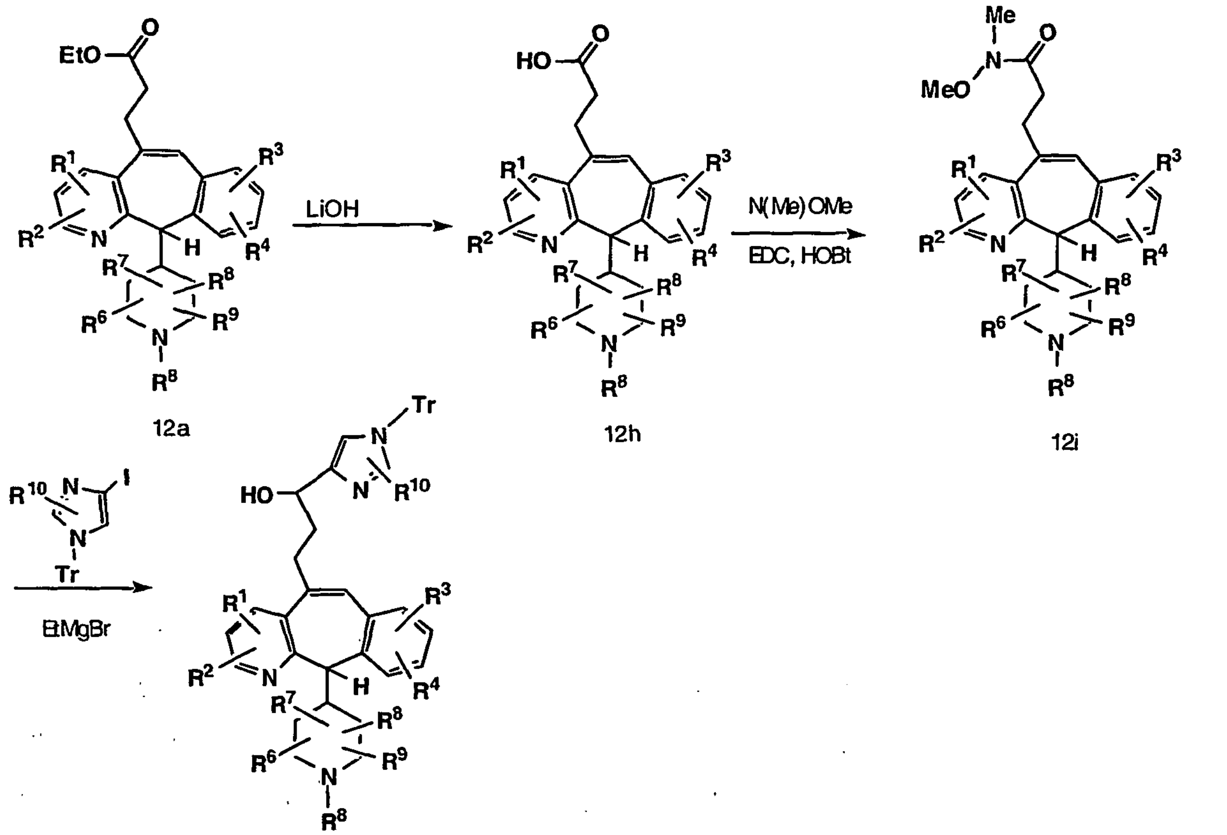


12a 12h 12i

12c

## Scheme 12a

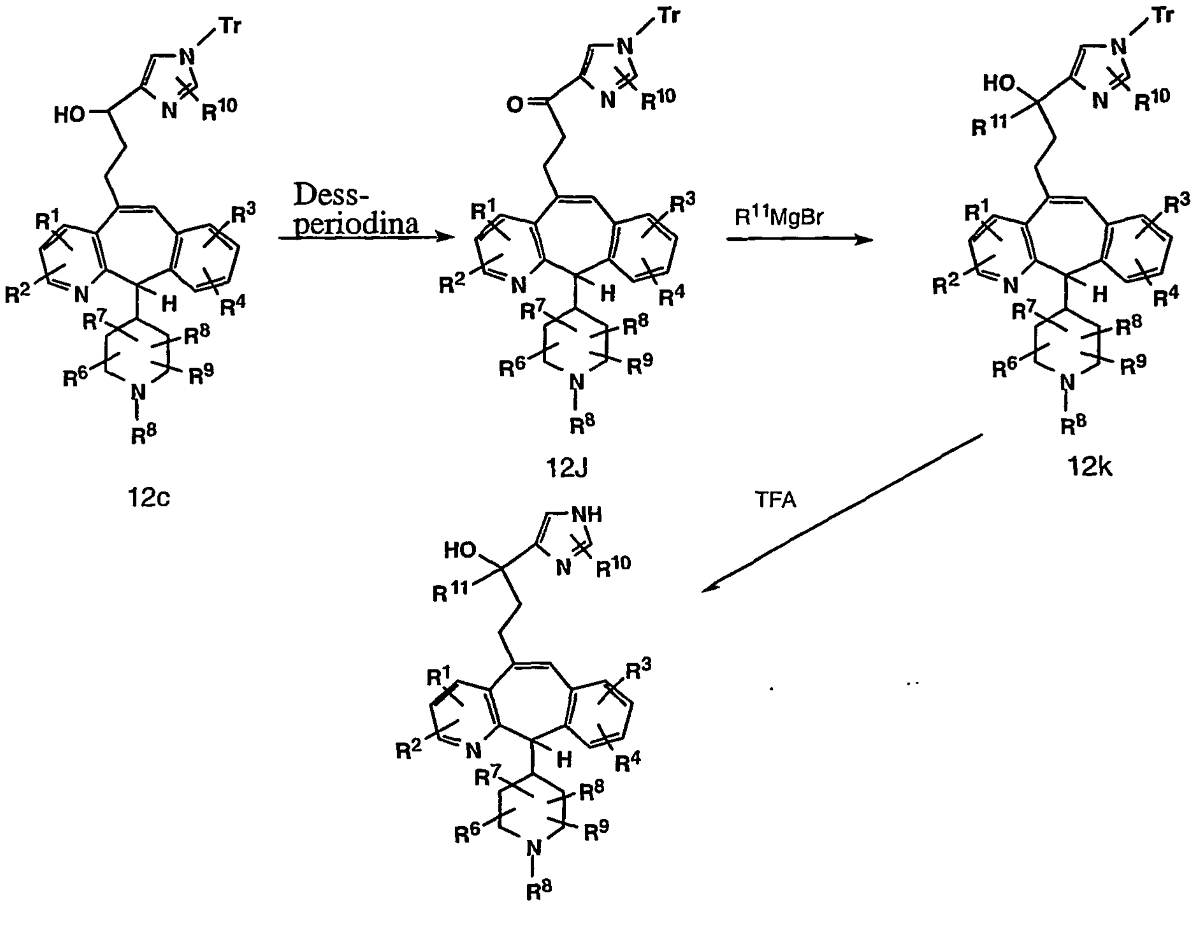


12c 12J 12k 12L

Compounds of type 12L were prepared as shown above. Oxidation of the hydroxyl compound 12c can be accomplished with the Dess Martin periodinane to obtain 12j. Reaction with a grignard reagent gave 12k. The trityl group is removed under standard conditions mentioned above to give the desired compound 12L.

## Scheme 13: *C*-Substituted Imidazole Single Methylene Bridgehead Compounds

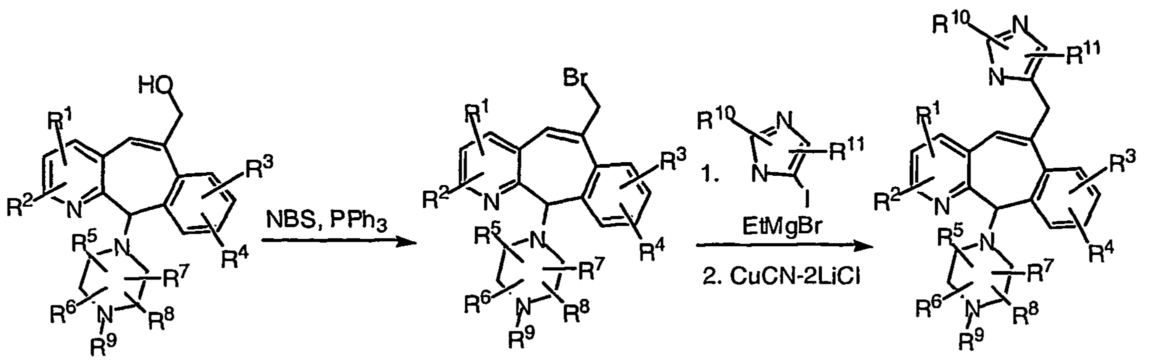


13a 13b 13c

Single methylene bridgehead *C*-imidazole derivatives (13c) were prepared as shown above. Compound 13a was first converted to bromide 13b. Treatment of compound 13b with *C*-imidazote cuprates (prepared from corresponding iodo imidazole) yielded the adduct 13c.

### Scheme 14: Preparation of one-methylene piperazines

Ketone A is brominated with brominating reagents such as NBS, with a small amount of an activator such as benzoyl peroxide, in solvents such as dichloromethane at elevated temperature, such as 80-100° C to give dibromo compound B.

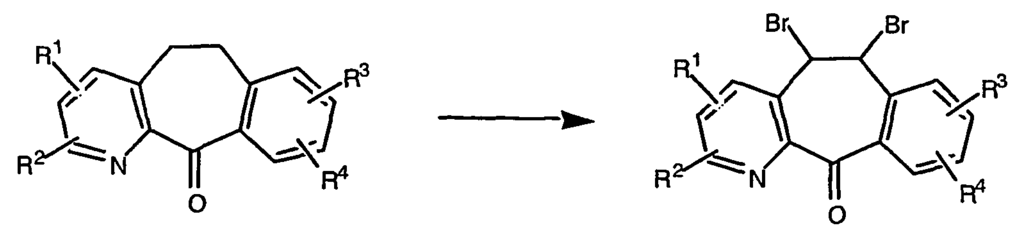

A B

Dibromo compound B is reacted with a base such as DBU in a solvent such as dichloromethane at temperatures from 0°C to room temperature to give vinylbromides C and D. These vinylbromides are separated by chromatography such as silica gel flash chromatography using solvents mixtures such as ethyl acetate and hexane. Alternatively, vinylbromides C and D can be separated by crystallization from solvents such as dichloromethane.

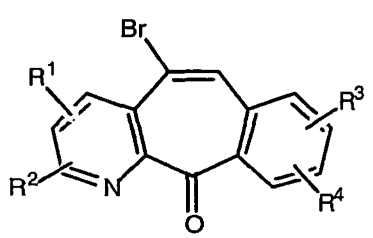

+

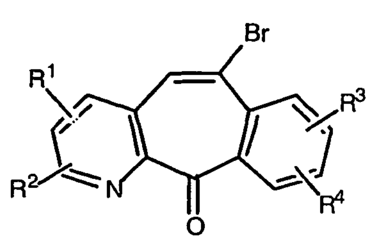

C D

The ketone groups of separated vinylbromides C and D are reduced to the corresponding alcohols E and F with a reducing agent such as $NaBH_4$ in solvents such as methanol or ethanol at temperatures of 0°C to room temperature.

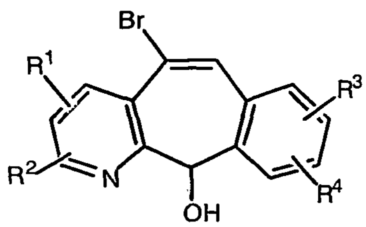

E

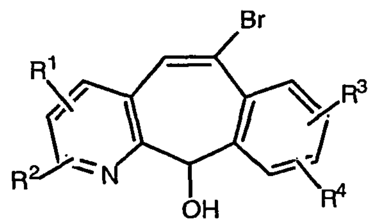

F

The resulting alcohols functions of E and F are converted to a leaving group, such as a halide, with reagents such as $SOCl_2$ in solvents such as dichloromethane containing a base such as 2,6-lutidine and running the reaction at 0°C to room temperature. The resulting intermediate halides are reacted, without purification, with piperazine or a protected piperazine, such as BOC-piperazine in a solvent such as dichloromethane at room temperature giving intermediates G and H.

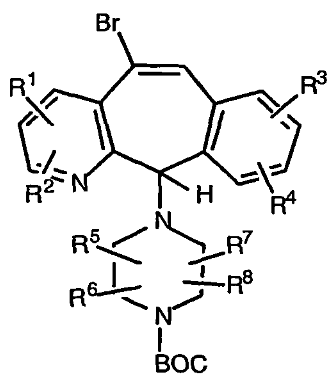

G

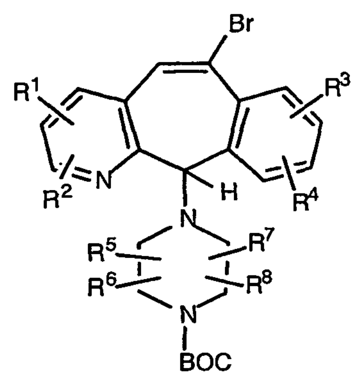

H

The vinylhalide intermediates are carbonylated with CO gas under a pressure of about 100 psi and a temperature of 80°C to 100°C using a palladium catalyst such as $PdCl_2$ and triphenyl phosphine in toluene and containing DBU and an alcohol such as methanol. If methanol is used, methyl esters I and J are obtained.

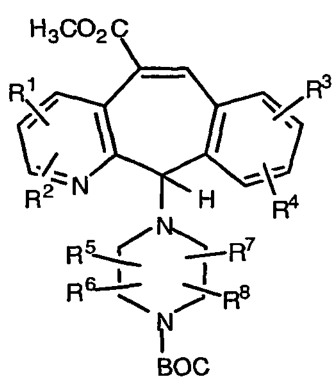

I

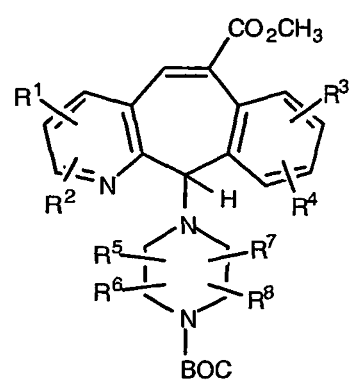

J

The ester functions are of I and J are reduced to hydroxymethyl functions of K and L. This can be done directly

by first removing the protecting BOC group with TFA or HCl-dioxane and then reducing with a reducing agent such as DIBAL-H, followed by reintroduction of the BOC group with di-tert-butyl dicarbonate. Alternatively, the ester function is hydrolyzed with LiOH and water followed by neutralization with citric acid. The resulting carboxylic acids are then converted into a function that is easily reduced, such as a mixed anhydride or an acyl imidazole. This is done by reacting the resulting carbocylic acids with a chloroformate to form the mixed anhydride or with carbonydiimidazole to form the acyl imidazole (Synlett. (1995), 839). The resulting activated carboxylic acids are reduced with $NaBH_4$ in solvents such as methanol, ethanol or aqueous THF.

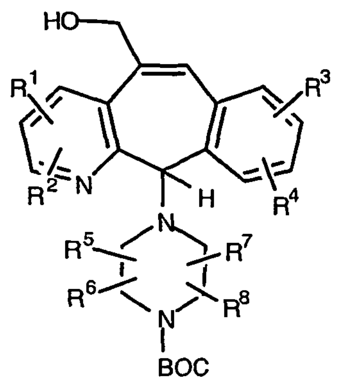

K

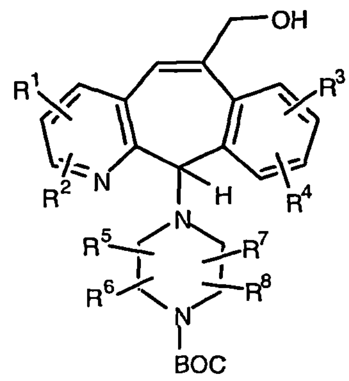

L

The hydroxy functions of K and L are converted into leaving groups such as a methanesulfonate or an arylsulfonate such as a tosylate, by reacting with the appropriate sulfonyl chloride in dichloromethane containing a base such as triethylamine. The sulfonate leaving groups can be displaced by nucleophiles such amines. The nucloephile can also be basic heterocycles such as imidazole or a substituted imidazole. In the case of an imidazole, the anion of the imidazole is first formed with NaH in DMF and then reacted with the above sulfonate. Displacement of the sulfonates with a nucleophile gives O and P, which can be converted to the compounds of this invention 1.0, by first removing the BOC protecting group and then forming the desired amide, urea, carbamate or sulfonamide on the resulting amine by methods well known in the art.

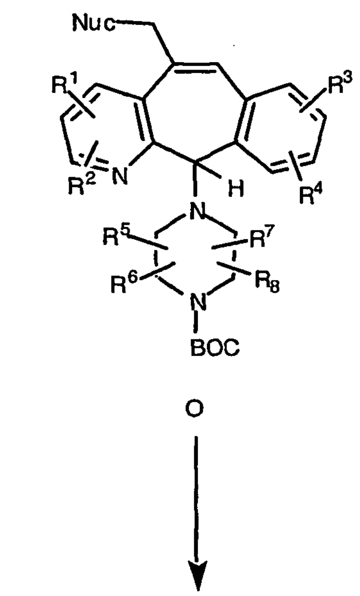

O

Formula (1.0)

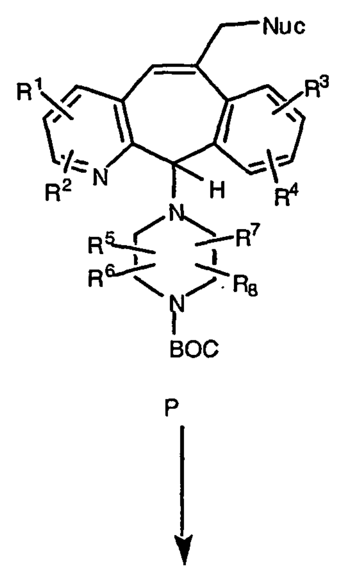

P

Formula (1.0)

## Scheme 15: Preparation of one-methylene piperidenes

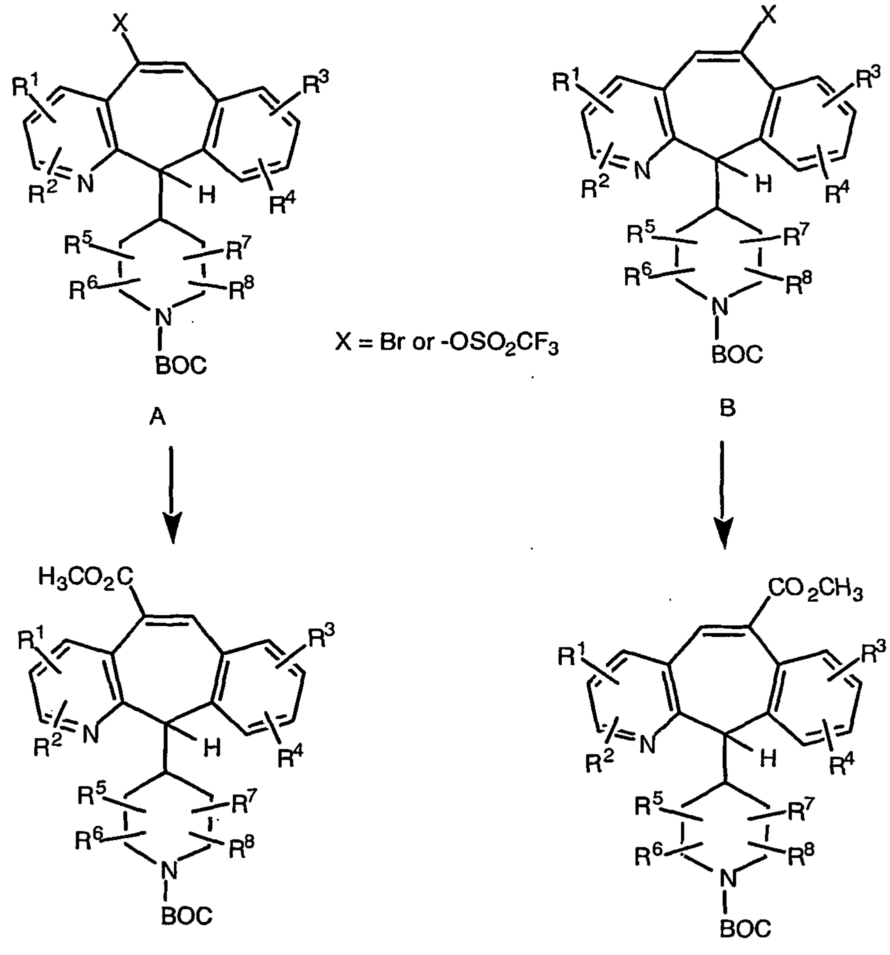

A

X = Br or $-OSO_2CF_3$

B

C

D

The vinylhalide or vinyltriflate intermediates A and B, (described in other general schemes) are carbonylated with CO gas under a pressure of about 100 psi and a temperature of 80˚C to 100˚C using a palladium catalyst such as $PdCl_2$ and triphenyl phosphine in toluene and containing DBU and an alcohol such as methanol. If methanol is used, methyl esters C and D are obtained. Intermediates C and D are reacted as are intermediates I and J in the general scheme for one methylene piperazines to yield compounds of Formula 1.0, of this invention.

## Scheme 15a:

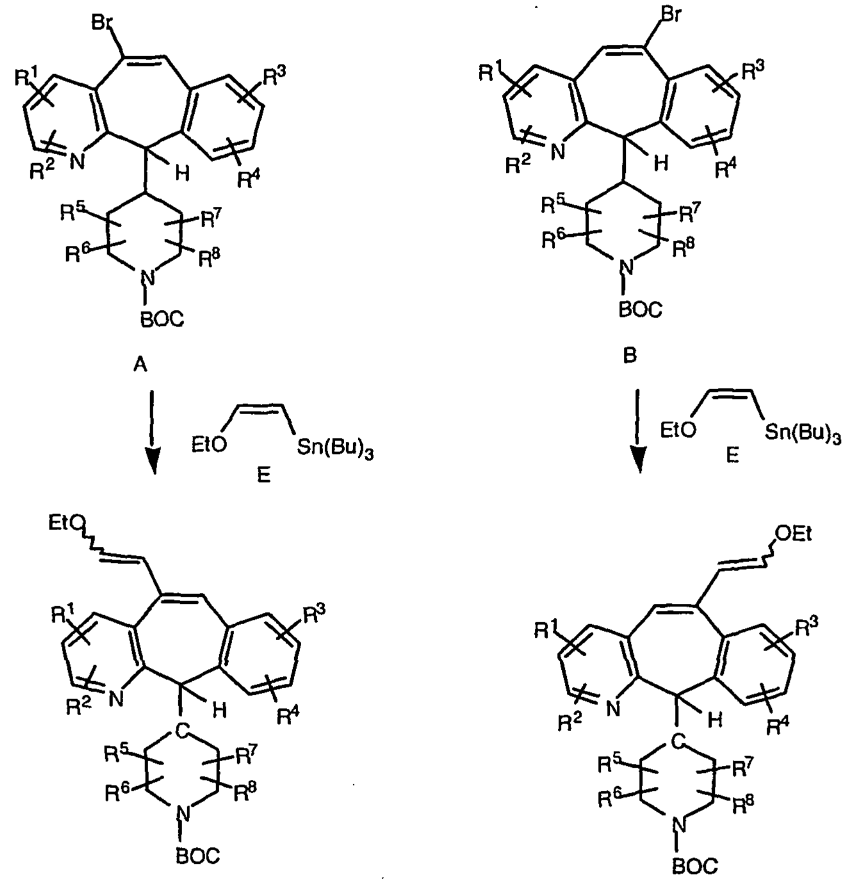

A

B

E

F

G

Alternatively, Intermediates A and B can be reacted with tin vinylether E, in the presence of $PdCl_2$, as described in Tetrahedron, (1991), 47, 1877, to yield vinylethers F and G (Scheme 15a). Allowing F and G to stand until aldehyde is visible by NMR (at least two weeks) and then reacting with $Hg(OAc)_2$, KI followed by $NaBH_4$, as described in J. Chem. Soc., Perkin Trans., (1984), 1069 and Tet. Lett., (1988), 6331, yields mixtures H, I and J, K. Intermediates H and J are separated and reacted as are intermediates K and L in the general scheme for one methylene piperazines to yield compounds of Formula 1.0, of this invention.

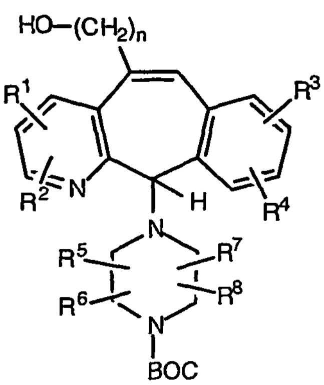

H, n = 1
I, n = 2

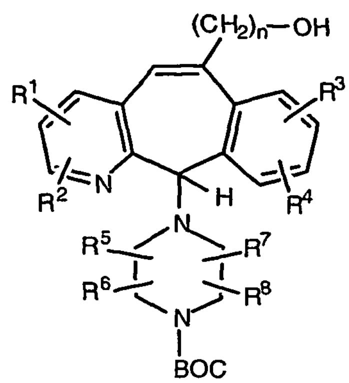

J, n = 1
K, n = 2

## Scheme 16: Branching on the methylene chain

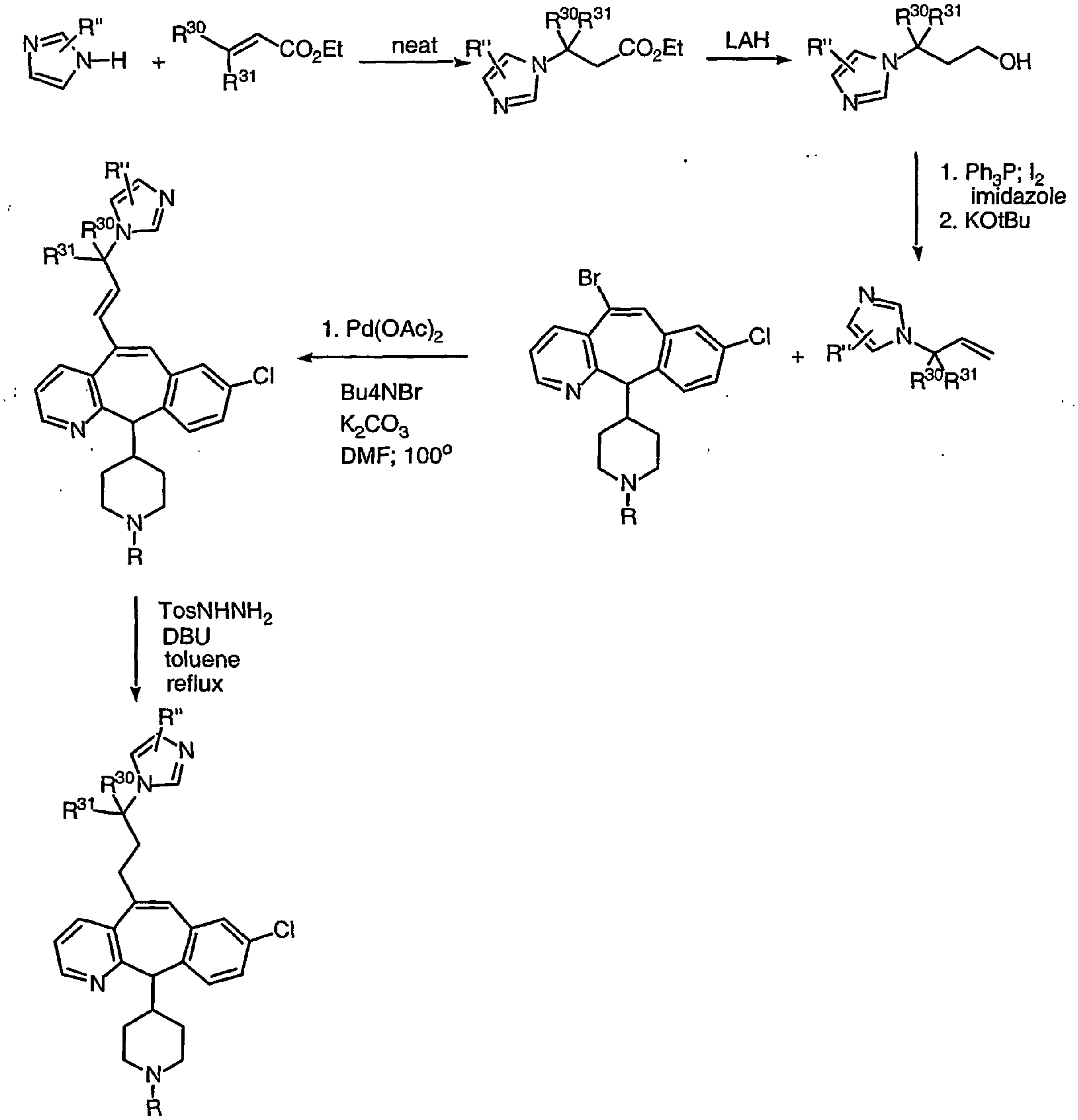


Compounds with substitution along the chain can be synthesized starting with a substituted ethyl acrylate derivative. Addition of imidazole across the olefin followed by reduction gives the terminal alkene, which can be added to the appropriately substituted vinyl bromide under Heck reaction conditions. Selective reduction of the di-substituted olefin gives the saturated derivative (Scheme 16).

## Scheme 17: C-linked imidazoles

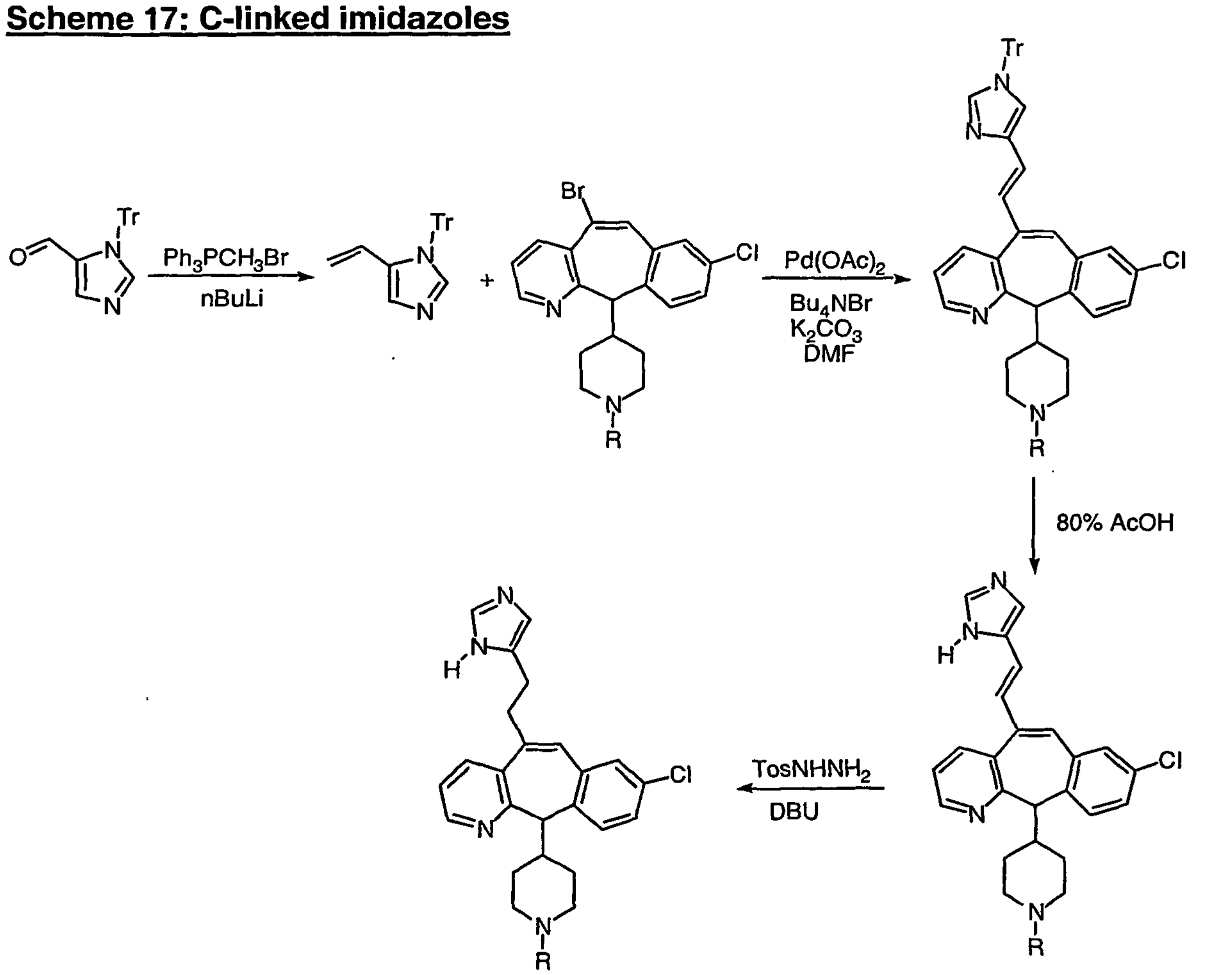


The synthesis of the C-linked imidazoles proceeds through the Heck reaction of the appropriately substituted vinyl imidazole with the appropriate vinyl bromide. Selective reduction of the resulting di-substituted olefin gives the target compound. A similar procedure can be carried out with differentially N-substituted imidazoles to give N-alkyl imidazole derivatives (Scheme 17).

### Suberyl Compounds

One skilled in the art will appreciate that the compounds of the invention represented by Formula 1.0, wherein a, b, c or d is C can be prepared according to the following schemes:

## Scheme 18: Preparation of suberyl analogues

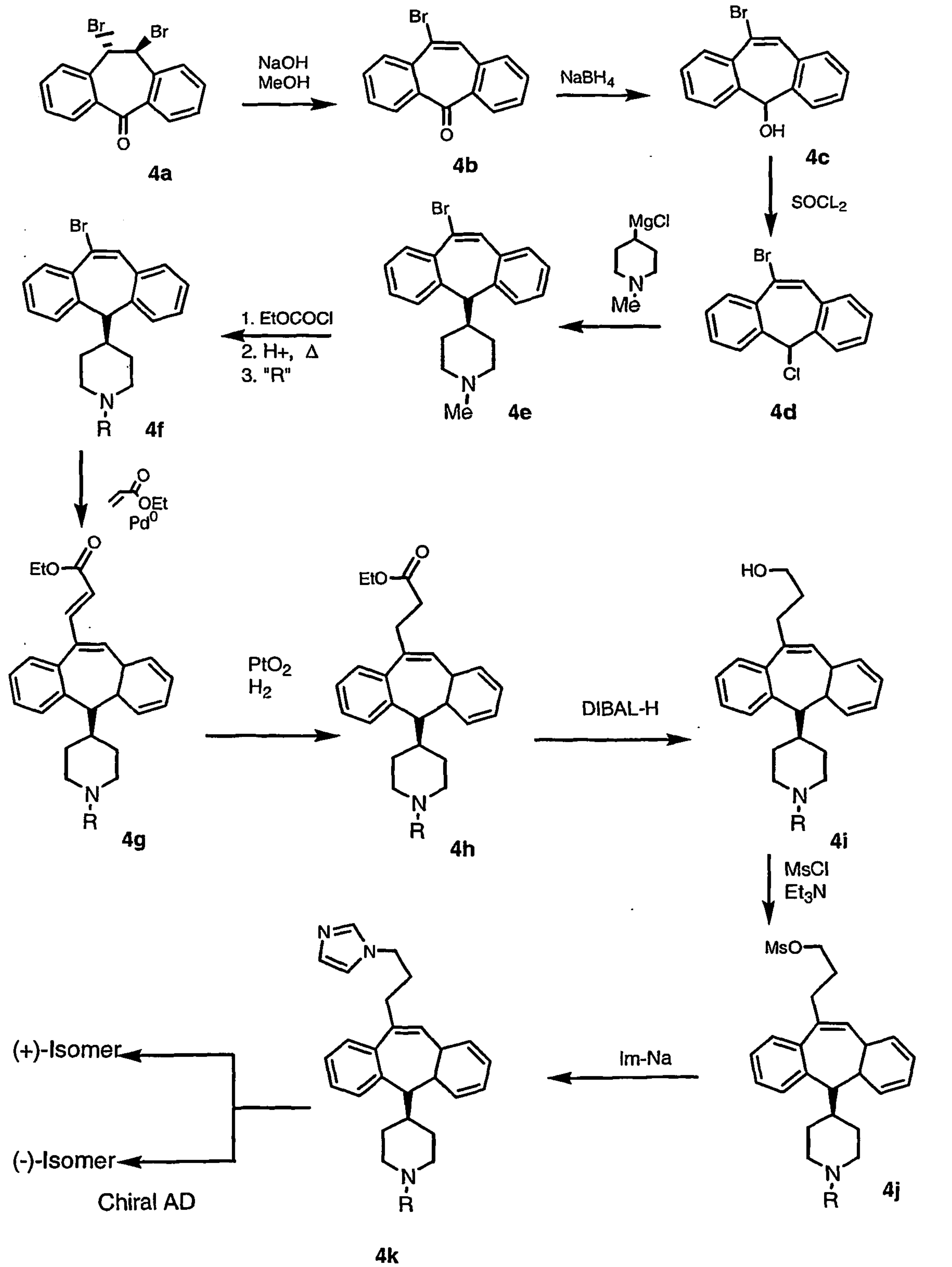


Tricyclic vinyl bromide azaketone 4b was prepared as described by Rupard et. al. (*J. Med. Chem.* **1989**, 32, 2261-2268). Reduction of ketone to alcohol 4c was carried out with $NaBH_4$. The alcohol was converted to chloride 4d and then treated with N-methylpiperidine Grignard reagent to give piperidine derivative 4e. Demethylation was effected with ethyl chloroformate followed by acid hydrolysis and subsequent derivitization (i.e sulfonylation, acylation and carbomylation etc.). Preparation of compounds with 3-carbon substituted imidazole moieties on the suberane trycyclic

bridgehead was carried out in a similar way as described in scheme 3.

**Preparation of Intermediates and Examples**

PREPARATIVE EXAMPLE 1

Step A Preparation of Compound (2).

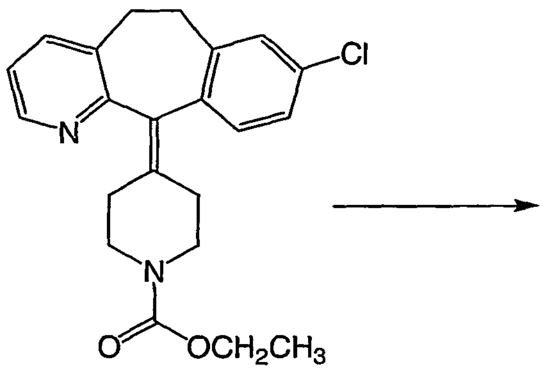

1

2

Loratadine® (448 g, 1.17 mol) was refuxed in 2 L of 70% aqueous HCl (1.4 L conc.HCl in 600 ml $H_2O$) for 12h. The reaction mixture was then cooled and poured into ice. It was then basified with 950 mL of 50% NaOH followed by extraction with $CH_2Cl_2$ (1 x 4L, and 2 x 2.5L). The organic phase was washed with brine, dried over $Na_2SO_4$ and $MgSO_4$ and then filtered. All the volatiles were then removed to give 368 g of the title compound (2). $MH^+$ = 311

Step B Preparation of Compound (3).

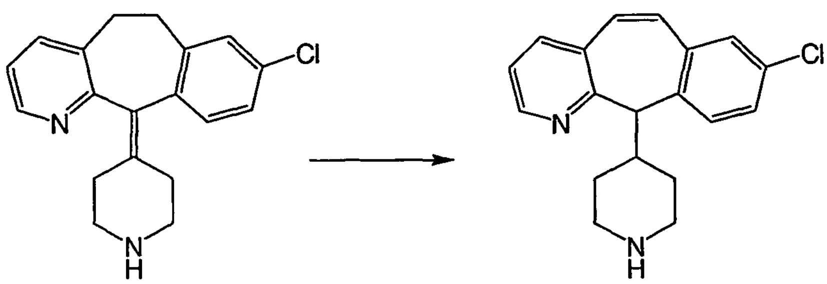

2

3

To the title compound from Preparative Example 1, Step A (363 g, 1.17 mol) was added trifuromethane sulfonic acid (1.8 Kg) under $N_2$. The reaction mixture was refluxed at 170˚C. The progress of the reaction was monitored by $^1H$ NMR. After 4 days the reaction was only 63% complete. After 8 days the reaction was found to be 80% complete according to $^1H$ NMR; thus another 130 mL of $CF_3SO_3H$ were added and refuxing continued for another 24h. It was then poured into ice and basified with 800 mL of NaOH (50%) and extracted twice with $CH_2Cl_2$(1 X 8L then 1 X 7L). The organic phase was combined, washed with $H_2O$ and filtered through celite. It was then dried over $MgSO_4$ and $Na_2SO_4$ and again filtered through celite. The filtrate was concentrated to give a black brown semi-solid that was pre adsorbed on 600 g of silica gel and then chromatographed on 2.3 Kg of silica gel eluting first with 5% $CH_3OH$-$CH_2Cl_2$ (saturated with ammonia) and then with 10% $CH_3OH$-$CH_2Cl_2$ (saturated with ammonia) to give 102 g of the title compound (3) as

a solid. mp = 73-75; MS (FAB) m/z 483 ($MH^+$).

Step C Preparation of Compound (4).

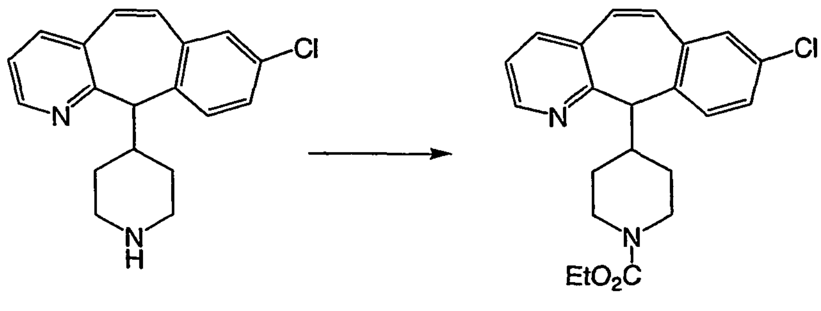

3 4

To a solution of the title compound of Preparative Example 1, Step B (145 g) in 1L of $CH_2Cl_2$ at O˚C was added ethylchloroformate (55 mL), dropwise. The reaction mixture was stirred at room temperature overnight. It was further diluted with 1L $CH_2Cl_2$ and stirred with 2L of dilute $NaHCO_3$, pH ~ 7-8. The organic layer was separated and dried over $MgSO_4$ and $Na_2SO_4$, filtered and concentrated to afford 174 g of a brown black gum. The crude compound was purified by silica gel column chromatography, eluting with 20-60% ethyl acetate-hexane to afford the title compound (4). MS (FAB) m/z 383 ($MH^+$).

D. Preparation of compounds (6) and (5).

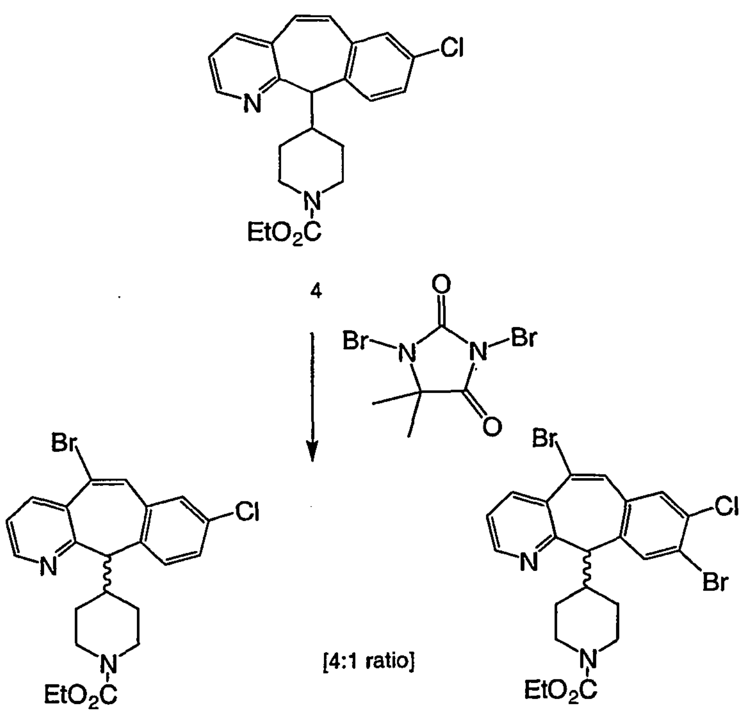
4

[4:1 ratio]

6 5

The title compound of Preparative Example 1, Step C (251 g, 0.65 mol) was dissolved in 1.65 L of $CH_2Cl_2$ and dibromo dimethylhydantoin, (132 g, 0.462 mol) was then added. The solution was stirred until the system was homogeneous. The solution was cooled to 0 °C under $N_2$ atmosphere and 174 mL of $CF_3SO_3H$ were added over 37 min. while keeping temperatures between -1 to 1°C. The reaction mixture was stirred for 3 h, cooled to -10°C and basified with 50% NaOH (170 mL), keeping the temperature below 1°C. The aqueous phase was extracted with $CH_2Cl_2$ and then dried over $MgSO_4$, dried and concentrated to give 354 g of yellow foam that was chromatographed on silica gel eluting with 10-50% of ethyl acetate-hexanes gradient to give 50 g of compound (5) (14% yield) and 147 grams of the desired title compound (6) (49% yield). Compound (6) MS m/z (rel intens) 462 ($MH^+$); Compound (5) MS m/z (rel intens) 542 ($MH^+$).

E. Mixture of compounds (7) and (8).

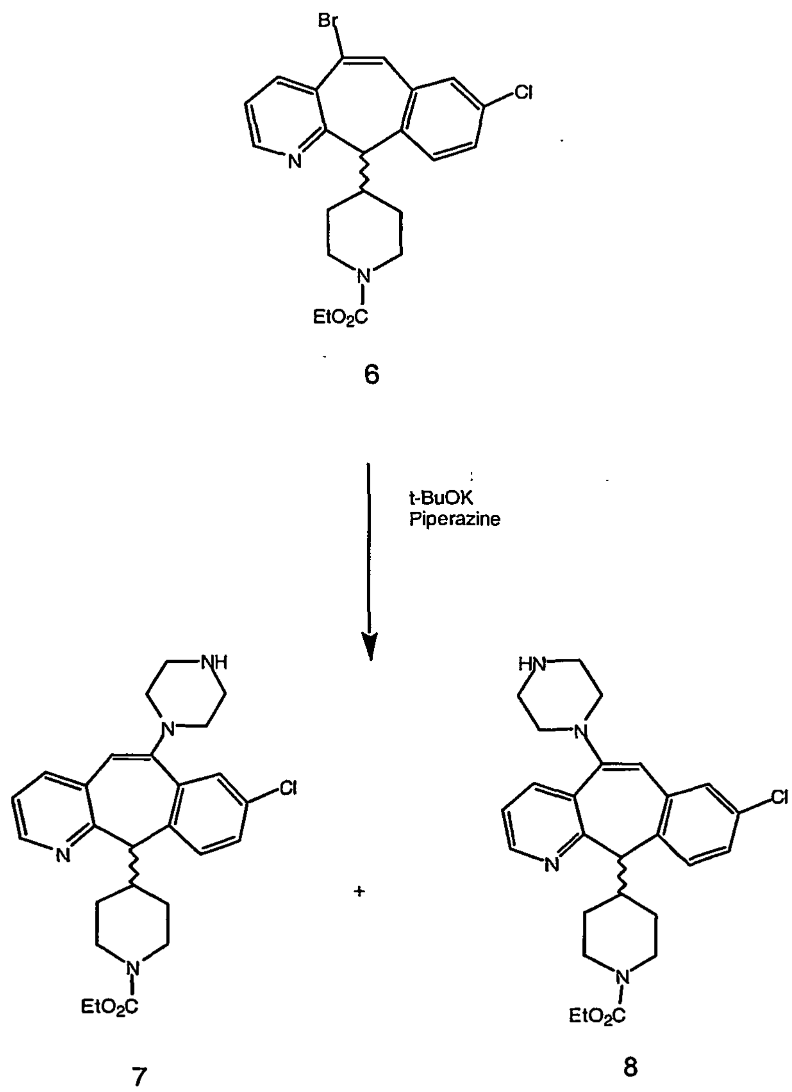


6

7 + 8

To a solution of piperazine 0.186 g (2.2 mmol, 5 equiv.) in 5 mL of THF was added 0.20 g (0.4 mmol) of compound 6 (from Preparative Example 1, Step D. The reactants stirred at room temperature until everything was in solution. To this mixture was added potassium t-butoxide (0.243 g, 2.1 mmol, 5 equivalents) in one portion. The reaction mixture was stirred at room temperature for 2 h. All of the THF was removed by rotary evaporation and the resulting crude product was purified by flash chromatography eluting with 3-4% (10% $CH_3OH$: saturated with $NH_4OH$)-$CH_2Cl_2$ to give a mixture of title compounds (7) and (8). FAB m/z 467 ($MH^+$).

F. Mixture of compounds (9) and (10).

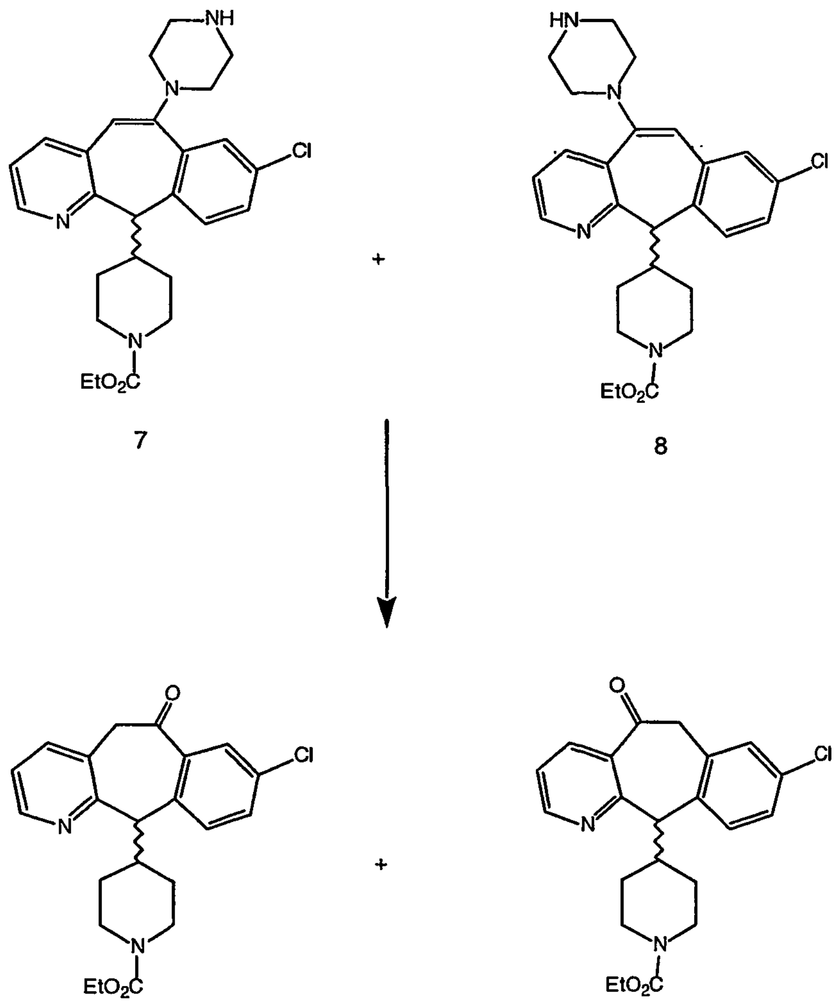

7 + 8

9 + 10

The mixture of compounds from Preparative Example 1, Step E (43.6 g) in 100 mL of conc. HCl was stirred at room temperature for 16 h. The reaction mixture was poured into ice and basified with conc. $NH_4OH$ and then extracted with $CH_2Cl_2$ to give a mixture of compounds (9) and (10). MS (FAB) m/z 399 ($MH^+$).

PREPARATIVE EXAMPLE 2

A. Compound (11).

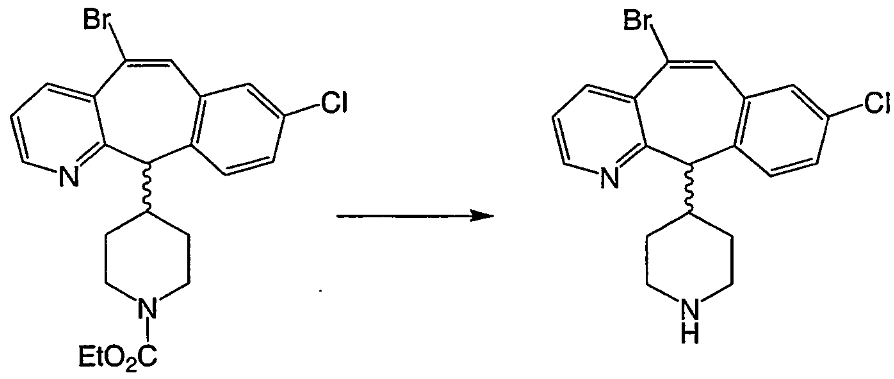

6 11

Compound 6 from Preparative Example 1, Step D (10 g, 21.7 mmol) was hydrolyzed in the same manner as described in Preparative Example 1, Step A , to give the title compound (11). MH+ = 389.

B. Compound (12).

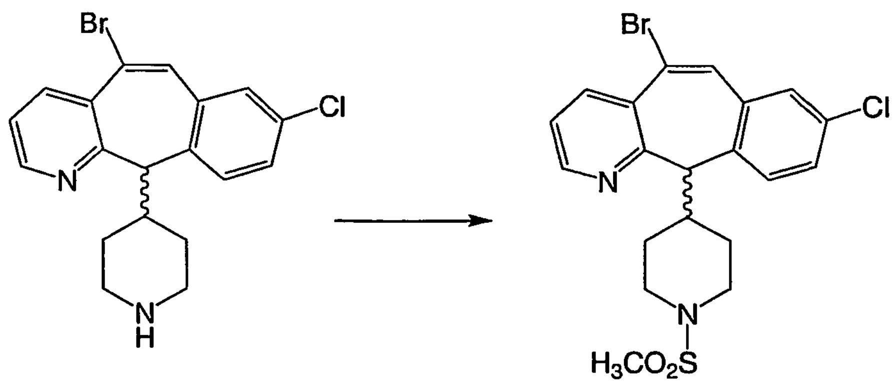

11 12

To the amine product from Preparative Example 2, Step A (20 g, 0.5 mol) and triethylamine (10.4 g, 14.4 mL, 1.02 mol) dissolved in anhydrous dichloromethane (100 mL) was added methanesulfonyl chloride (8.8 g, 6mL, 0.77 mol). After stirring at room temperature overnight, the solution was diluted with dichloromethane, washed with saturated $NaHCO_3$ and dried over anhydrous magnesium sulfate. Filtration and concentration *in vacuo* afforded the crude product that was purified by flash chromatography on a silica gel column, eluting with 1% $CH_3OH$(saturated with ammonia)-$CH_2Cl_2$ to give the title compound (12). MS (FAB) m/z 469 ($MH^+$).

Step C Preparation of Compounds (13) and (14).

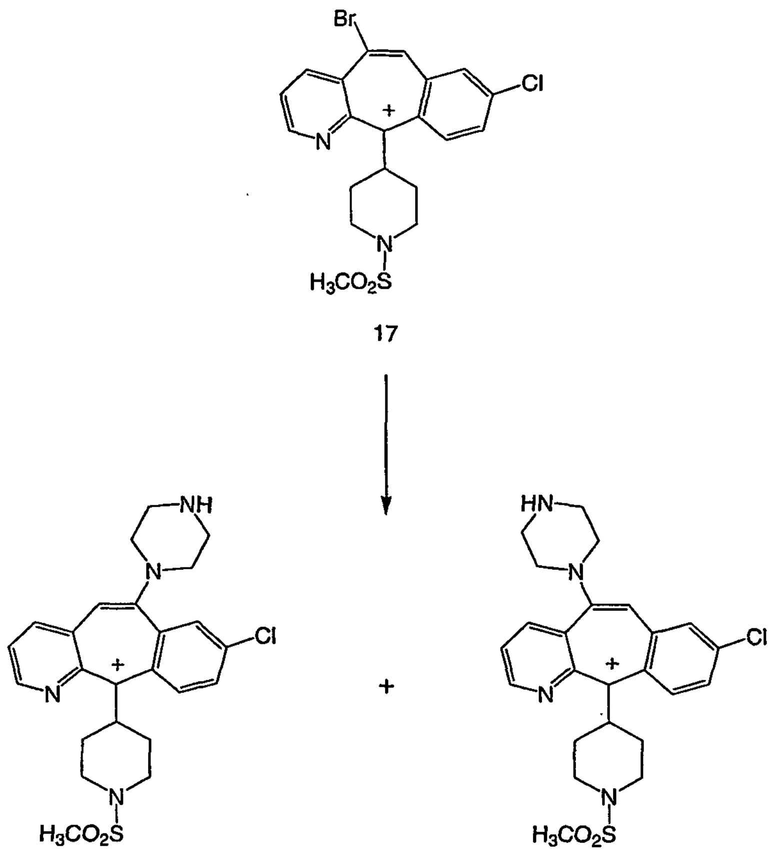

17

31 32

Product from Preparative Example 2, Step B (21.25 g, 45.3 mmol) was treated in the same manner as described in Preparative Example 1, Step E, to give 22.2 g of a mixture of compounds (13) and (14). MS (473) ($MH^+$).

D. Preparation of compounds (15) and (16).

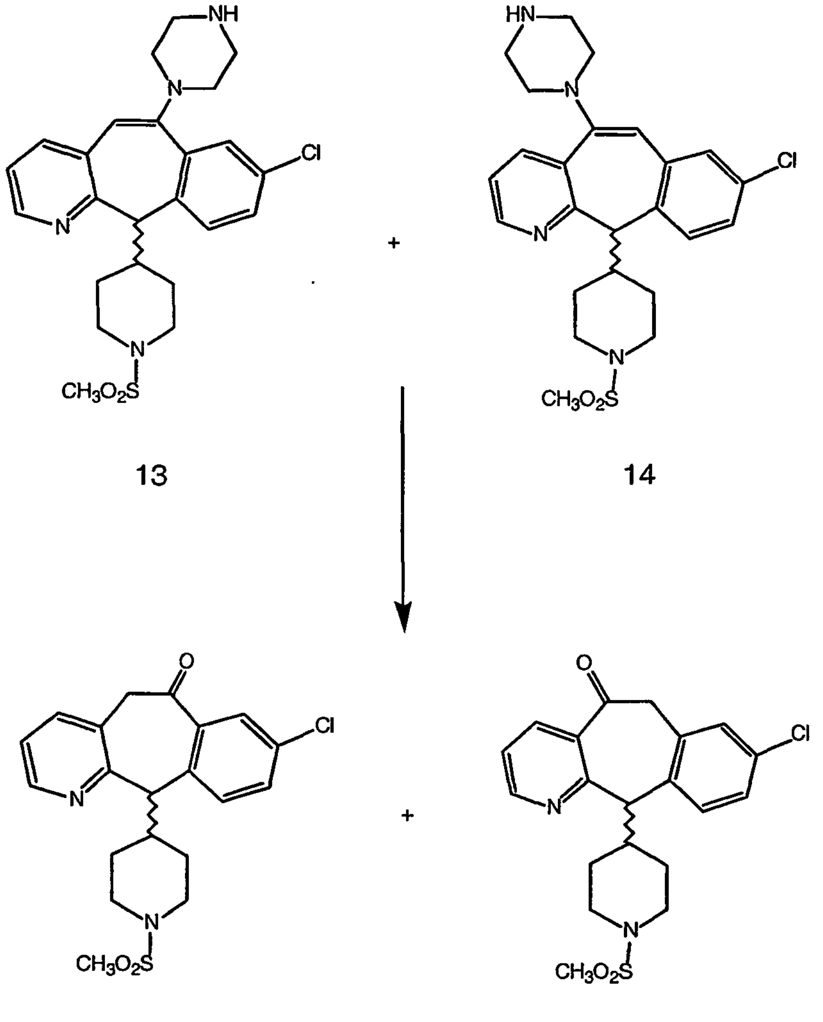

13 + 14

15 + 16

The product from Preparative Example 2, Step C (22.5 g) was dissolved in 150 mL of conc. HCl and stirred for 16 h. The reaction mixture was poured into ice, basified with conc. $NH_4OH$ and then extracted with $CH_2Cl_2$ to give a mixture of compounds (15) and (16). MS (FAB) m/z 405 ($MH^+$).

E. Preparation of compounds (17) and (18).

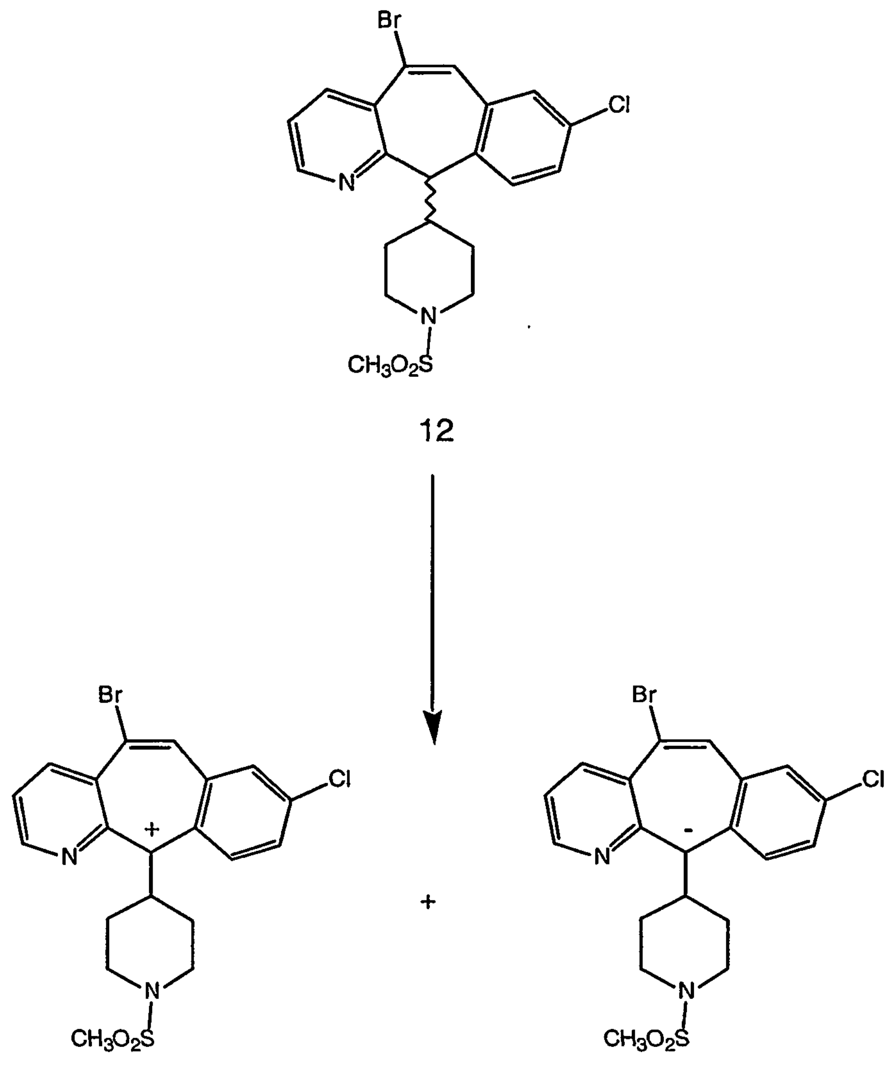

12

+

17

18

Separation of compound of Preparative Example 2 Step B by HPLC using a Chiralpack AD column eluting with 40-50% isopropanol:60-50% hexane-0.2% diethylamine gave enantiomeric amines (17) and (18).

Compound 17: mp = 118-119; $[\alpha]_D^{22}$ =+ 136.9° (9.00 mg/2mL, MeOH); MS (FAB) m/z 469 ($MH^+$).

Compound 18: mp = 119-120; $[\alpha]_D^{22}$ = -178.2° (9.90 mg/2mL, MeOH); MS (FAB) m/z 469 ($MH^+$).

<u>PREPARATIVE EXAMPLE 3</u>

A. <u>Compound (19).</u>

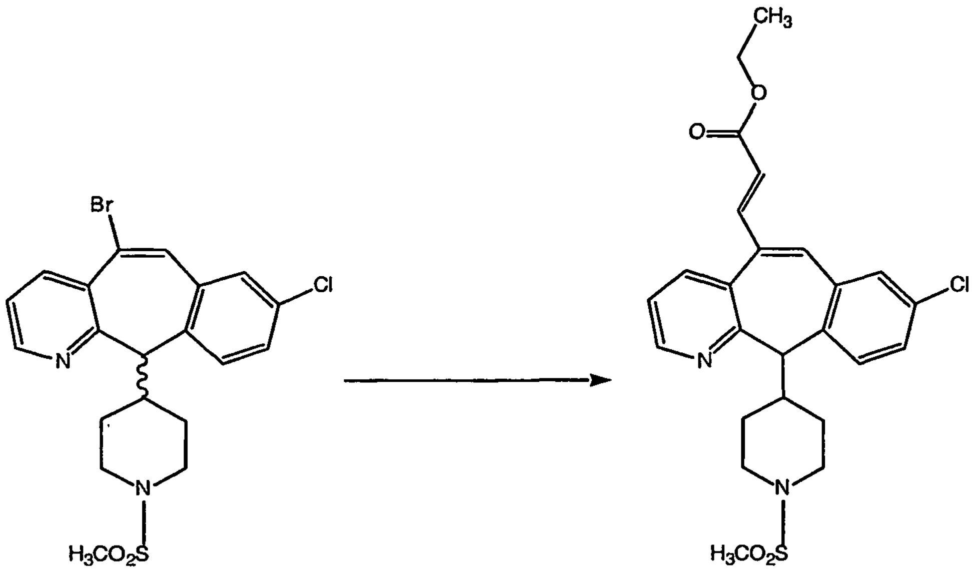

12

19

To a solution of the title compound from Preparative Example 2, Step B (2.0 g, 4.3 mmole) in DMF (50 ml) under nitrogen atmosphere, was added triethyl amine (17 ml), ethyl arcrylate (2.5 ml), potassium carbonate (3 g, 21.4 mmole), tetrabutylamonium bromide (2.8 g, 8.6 mmole) and palladium (II) acetate (0.1255 g, 0.56 mmol). The resulting mixture was heated to 100˚C, and stirred for 4 h then it was cooled to room temperature and the solvent was removed. To the residue was added $CH_2Cl_2$ and water and the mixture was then extracted with $CH_2Cl_2$. The organic layer was dried over magnesium sulfate, filtered and concentrated to dryness. The crude product was purified using pre-adsorbed flash silica column chromatography eluting with 30-50% ethyl acetate-hexane gradient to give the title compound (19). MS 487 ($MH^+$).

Step B Mlixture of Compounds (20) and (21).

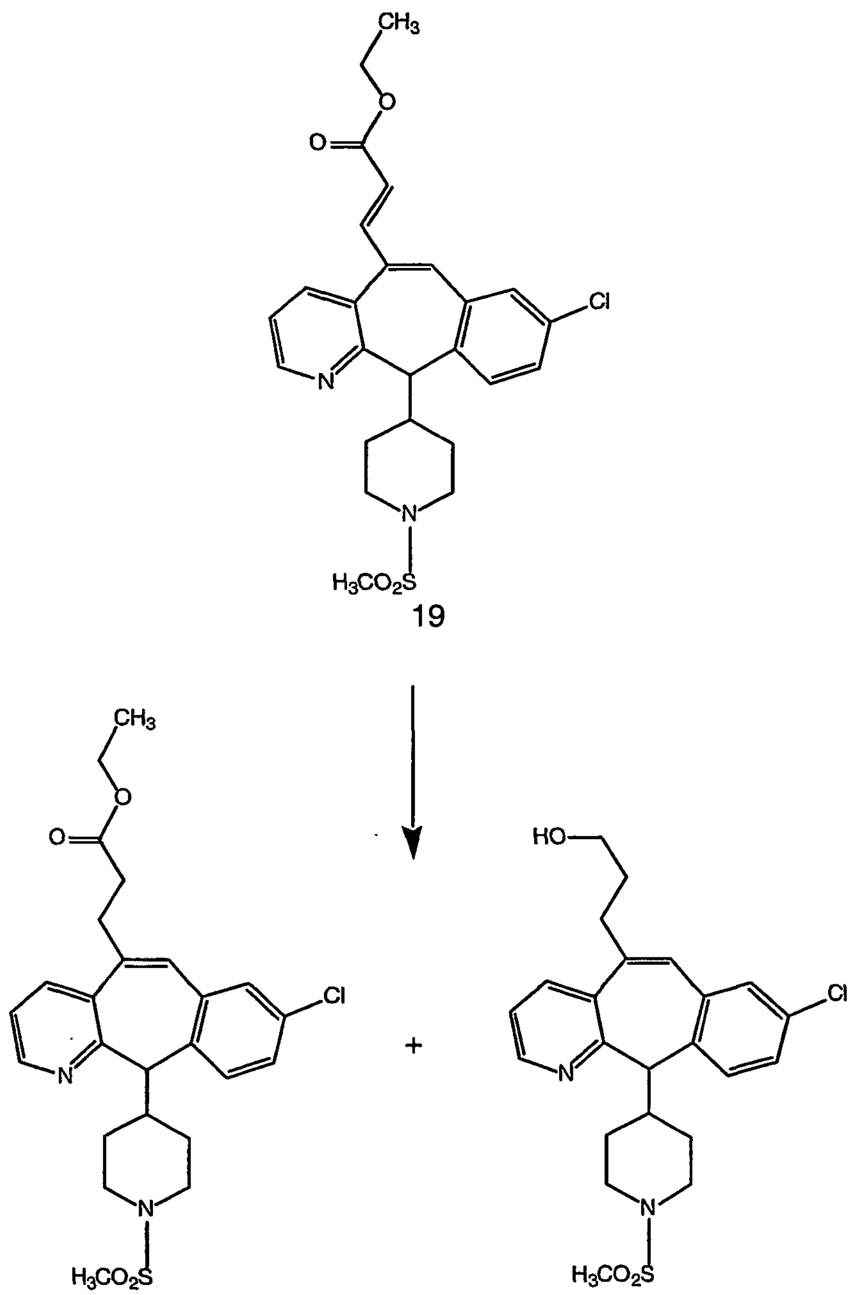

19

20 + 21

To a solution of the title compound from Preparative Example 3, Step A (6.4 g, 13 mmole) in ethanol (500 ml), was added copper chloride (0.96 g, 9.7 mmole). The reaction was cooled to 0˚C. Portionwise, added sodium borohydride (4.97 g, 131 mmole). The reaction stirred overnight at room temperature. Another portion of sodium borohydride (2.46 g, 65 mmole) was added and the reaction stirred for 2 more hours, then the solvent was removed. To the residue was added saturated sodium bicarbonate and the mixture was extracted with $CH_2Cl_2$. The organic layer was dried over sodium sulfate, filtered and concentrated to dryness to afford a mixture of the reduced ester (20) and the alcohol (21) title compounds. This crude mixture was taken on to the next step without purification.

Step C Preparation of Compound (22).

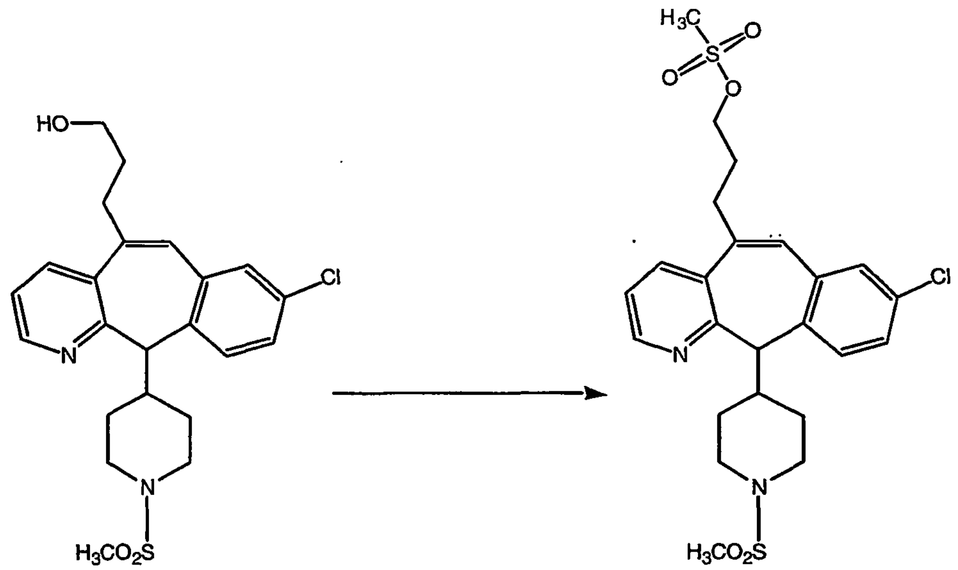

21 22

To a solution of the products from Preparative Example 3, Step B (5.74 g) in $CH_2Cl_2$ (100 ml) was added triethyl amine (2.4 ml). Slowly, methane sulfonyl chloride (0.8 ml) was added and the mixture stirred over night at room temperature. To the reaction was added saturated sodium bicarbonate and then it was extracted with $CH_2Cl_2$. The organic layer was dried over magnesium sulfate, filtered and concentrated to dryness. The crude product mixture was separated on a Biotage® column, eluting with 30% ethyl acetate-$CH_2Cl_2$, to afford the desired title compound (22). MS 525 ($MH^+$). (recovered unreacted ester (20))

PREPARATIVE EXAMPLE 4

A. Compound (23).

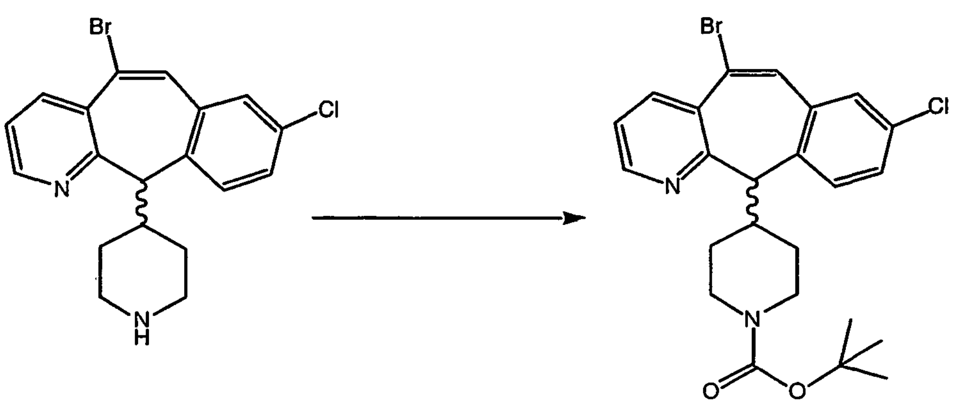

11 23

To a solution of title compound (11) from Preparative Example 2, Step A (20 g, 51.32 mmole) in $CH_3OH/H_2O$ (400 ml, 50:1) was added di-tert-butyl dicarbonate (16.8 g, 77.0 mmole). The pH was adjusted to 9 and the mixture was stirred for 4 h. The solvent was removed, then water was added. The mixture was extracted with $CH_2Cl_2$. The organic layer was dried over magnesium sulfate, filtered and concentrated to dryness affording the title compound (23). MS 491 (MH+).

B. Compound (24).

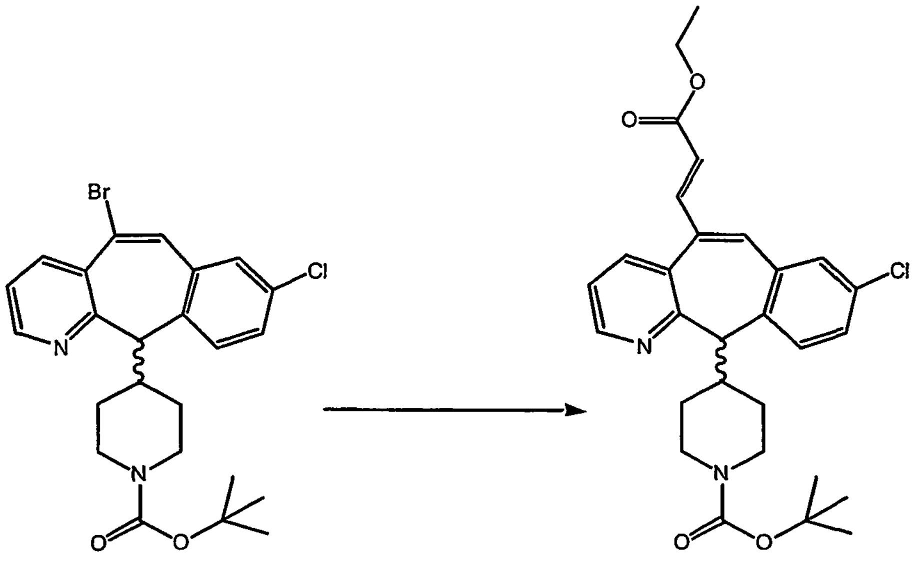

23

24

Following a similar procedure as in Preparative Example 3, Step A, the title compound (24) was prepared. MS 509 (MH+).

C. Compound (25).

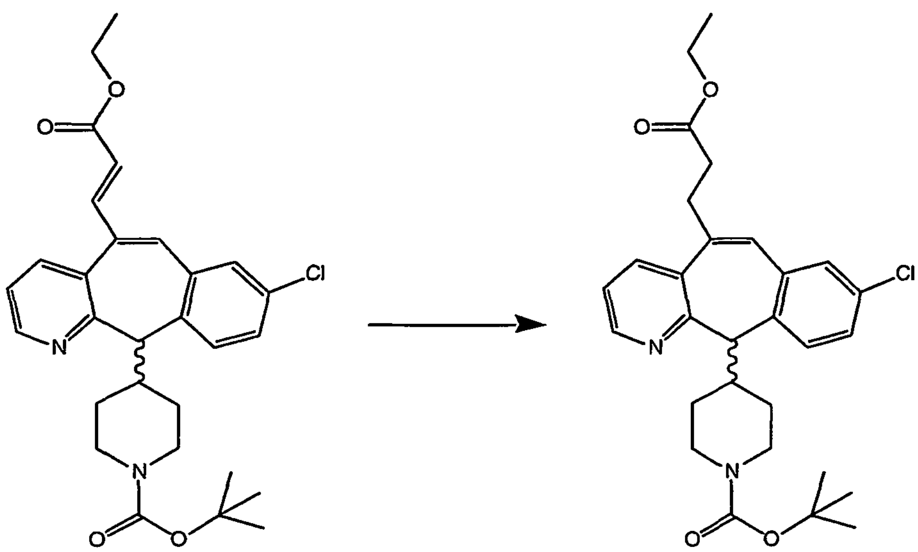

24

25

To a solution of the title compound from Preparative Example 3, Step B (19.62 g. 38.5 mmole) in ethanol (150 ml) was added platinum (IV) oxide (1.962 g). The reaction stirred over night at room temperature under $H_2$ balloon pressure atmosphere. After monitoring the reaction, an additional 2% (by weight) of platinum (IV) oxide was added and

the reaction stirred for 6 more hours, under $H_2$ balloon pressure atmosphere. The mixture was filtered through celite and concentrated to dryness to afford the title compound (25) as a white solid. MS 511 ($MH^+$).

Step D Preparation of Compound (26).

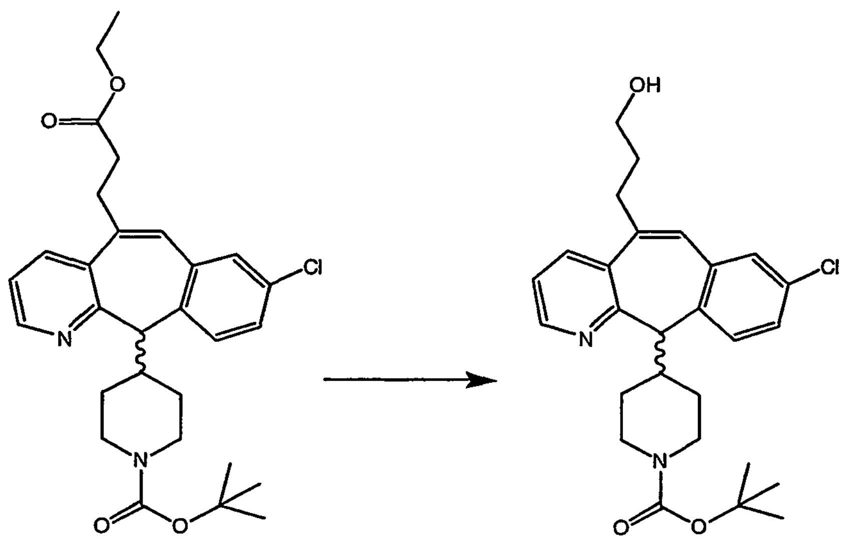

25

26

Dissolved product from Preparative Example 3, Step C (2.0 g, 3.9 mmole) in THF (30 ml) and cooled to 0˚C in an ice bath. To the reaction was added diisobutylaluminum hydride (7.8 ml, 7.8 mmole). The reaction was allowed to stir and come to room temperature over night. The reaction did not go to completion. The mixture was cooled in an ice bath (0˚C) and fresh diisobutylaluminum hydride/toluene (7.8 ml) was added. After the reaction stirred for 4 more hours, it was still not complete. The reaction mixture was cooled to 0˚C, and an additional 3.9 ml of diisobutylaluminum hydride as added. The reaction stirred for 3 more hours. The crude reaction mixture was then extracted with ethyl acetate:10% citric acid, and 1.0 N NaOH. The organic layer was dried over magnesium sulfate, filtered and concentrated to dryness to afford the desired title compound (26). MS 471 ($MH^+$).

Step E Preparation of Compound (27).

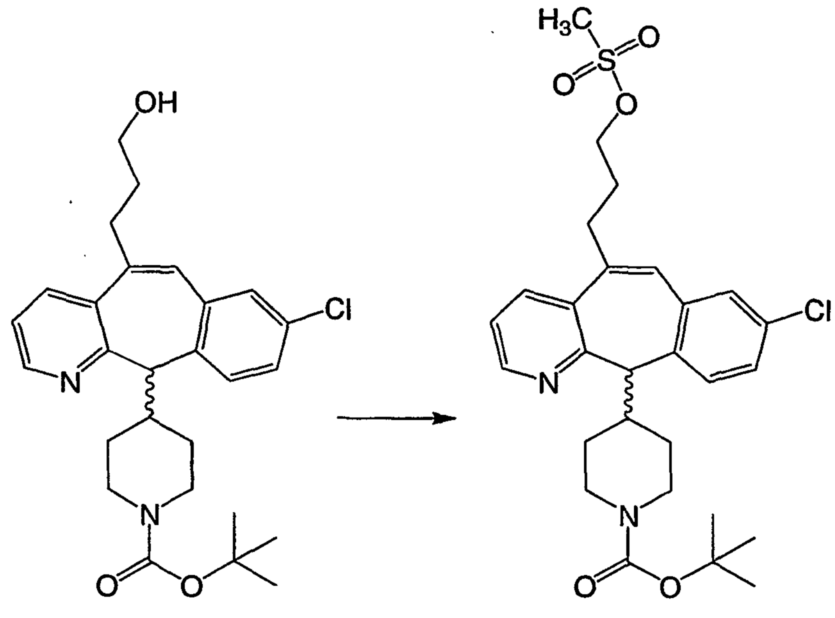

26 27

Following a similar procedure described in Preparative Example 3, Step C, the title compound (27) was prepared. MS 549 ($MH^+$).

Step F Preparation of Compound (28).

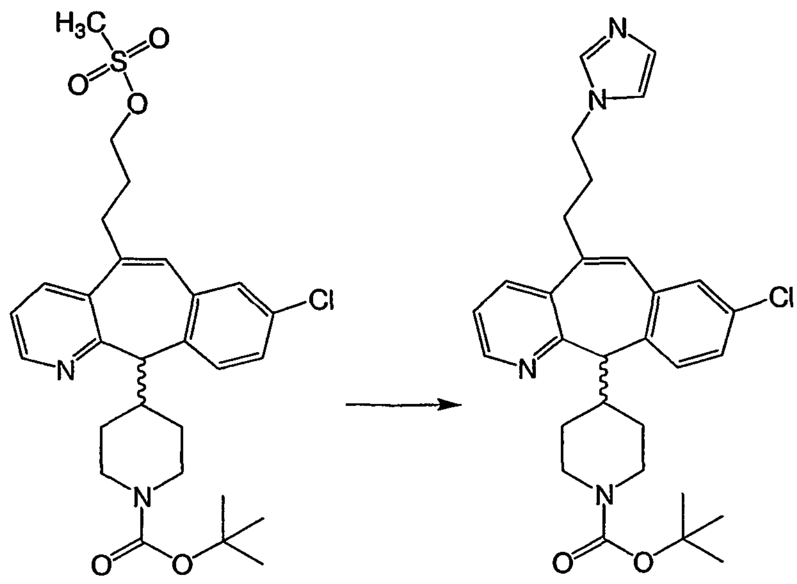

27 28

To a solution of the title compound from Preparative Example 4, Step E (1.6 g, 3.01 mmole) in DMF (50 ml) was added imidazolylsodium (Aldrich) (0.407 g, 4.52 mmole). The reaction mixture was heated to 90°C for 2 h. The reaction was cooled and the DMF was removed. Saturated sodium bicarbonate was added and the mixture was extracted with $CH_2Cl_2$. The organic layer was dried over magnesium sulfate, filtered and concentrated to dryness. The crude product was purified by column chromatography eluting with 2% $CH_3OH$: saturated with ammonia-$CH_2Cl_2$, to afford the

title compound (28). MS 519 ($MH^+$).

Step G Preparation of Compound (29).

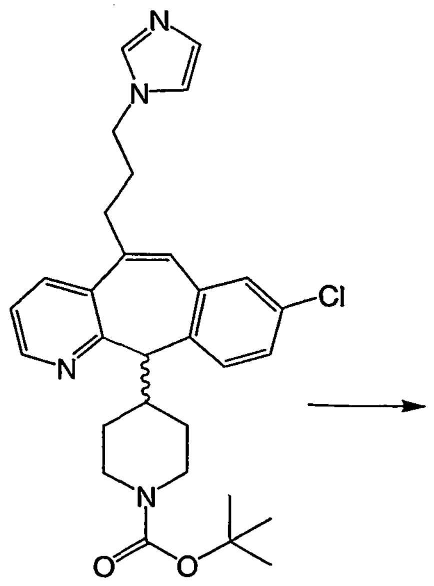

28 29

Dissolved the product from Preparative Example 4, Step F (0.55 g, 1.08 mmole) in 4 N dioxane/HCl (20 ml). The reaction mixture was stirred for 3 h at room temperature and then concentrated to dryness to afford the title compound (29) as a light yellow solid. HRMS 419 ($MH^+$).

PREPARATIVE EXAMPLE 5

A. Compound (30).

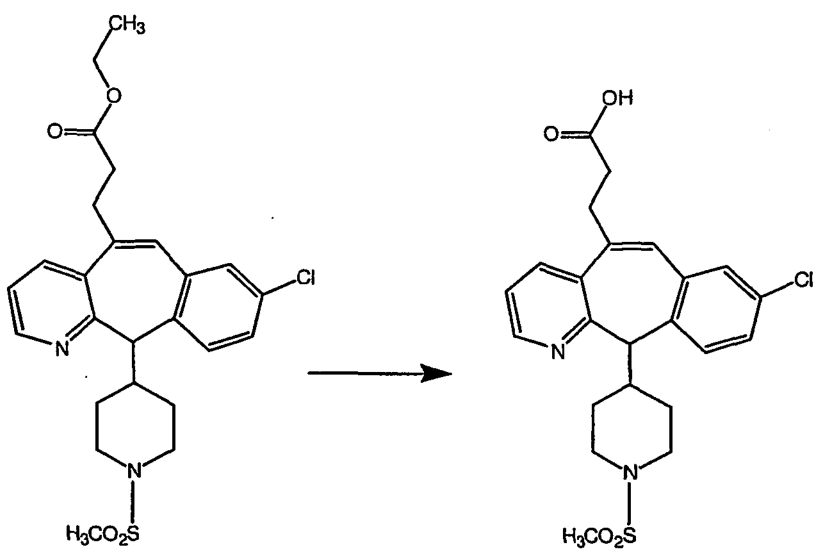

20 30

Compound (20) from Preparative Example 3, Step B (0.67 g, 1.37 mmole) was dissolved in THF (5 ml). To the mixutre was added 1N NaOH (6.9 ml) and the resulting solution stirred over night at room temperature. The reaction mixture was concentrated, acidified with 10% citric acid (w/v) and extracted with $CH_2Cl_2$. The organic layer was drived over magnesium sulfate, filtered and concentrated to dryness to afford the title compound (30) as a yellow solid. mp 122.7-123.4˚C; MS 461 ($MH^+$).

EXAMPLE 1

Preparation of compounds (31) and (32).

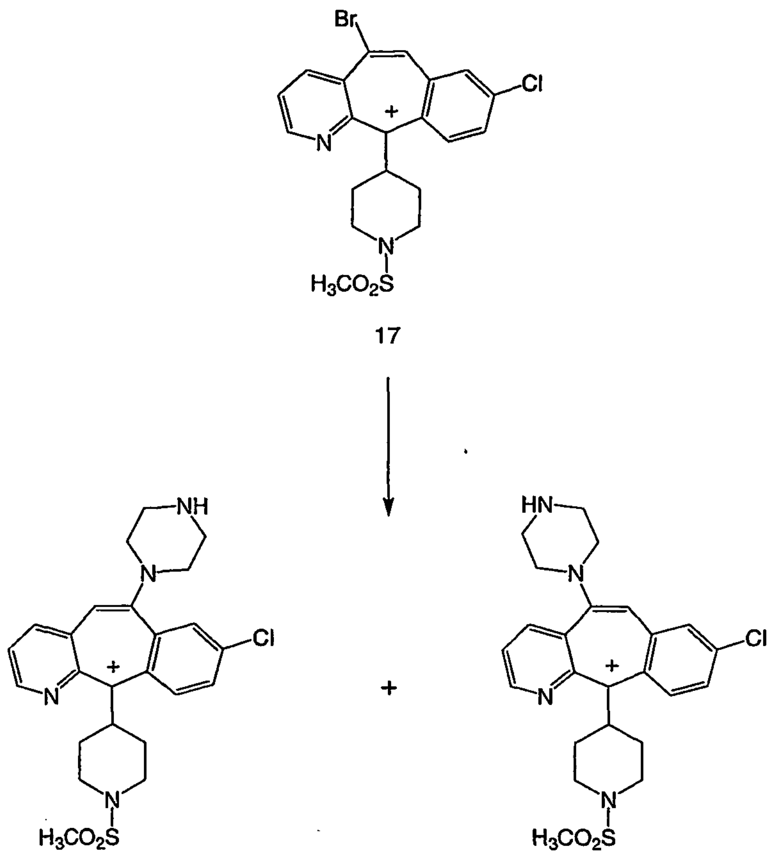

17

31 32

Compound (17) from Preparative Example 2, Step E 0.31 g (0.66 mmol) was treated in the same manner as described in Preparative Example 1, Step E to give a mixture of compounds (31) and (32) that were further separated on a HPLC Chiralpack AD column eluting with 30% isopropanol-70% hexane-0.2% diethylamine to give 0.04 g of target compound (31) and 0.07 g of target compound (32).

Compound 31: mp = 174-175; $[\alpha]_D^{22}$ =+ 96.0° (3.6 mg/2mL, $CH_2Cl_2$); MS (FAB) m/z 473 ($MH^+$).

Compound 32: mp = 173-174; $[\alpha]_D^{22}$ = + 21.7° (8.4 mg/2mL, $CH_2Cl_2$); MS (FAB) m/z 473 ($MH^+$).

EXAMPLE 2

Preparation of Compounds (33) and (34).

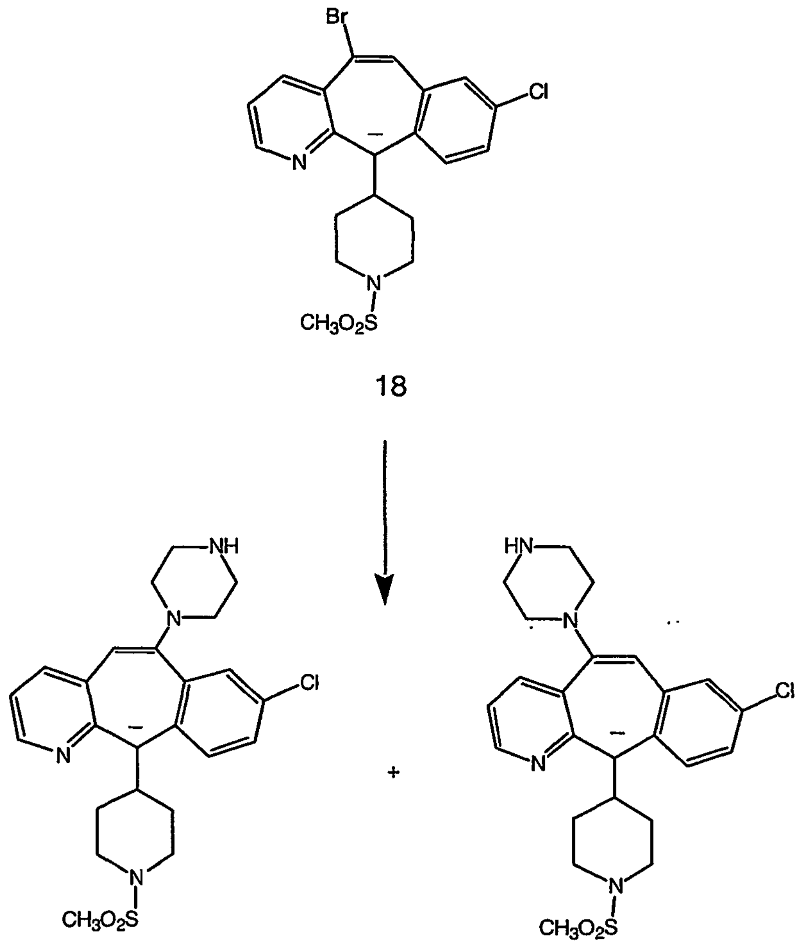

18

33 + 34

As described for preparation of Example 1 above, 0.31 g of compound (18) from Preparative Example 2 Step E was converted to a mixture of compounds (33) and (34) that were subsequently separated on a Chiralpack AD column HPLC eluting with and 30% isopropanol-70% hexane-0.2% diethylamine as eluent to give 0.12 g of target compound (33) and 0.04 g of target compound (34).

Compound 33: mp = 178-179; $[\alpha]_D^{22}$ =- 30.5° (9.5 mg/2mL, $CH_2Cl_2$); MS (FAB) m/z 473 ($MH^+$).

Compound 34: mp = 172-173; $[\alpha]_D^{22}$ = -84° (3.5 mg/2mL, $CH_2Cl_2$); MS (FAB) m/z 473 ($MH^+$).

EXAMPLE 3

Preparation of Compounds (35) and (36).

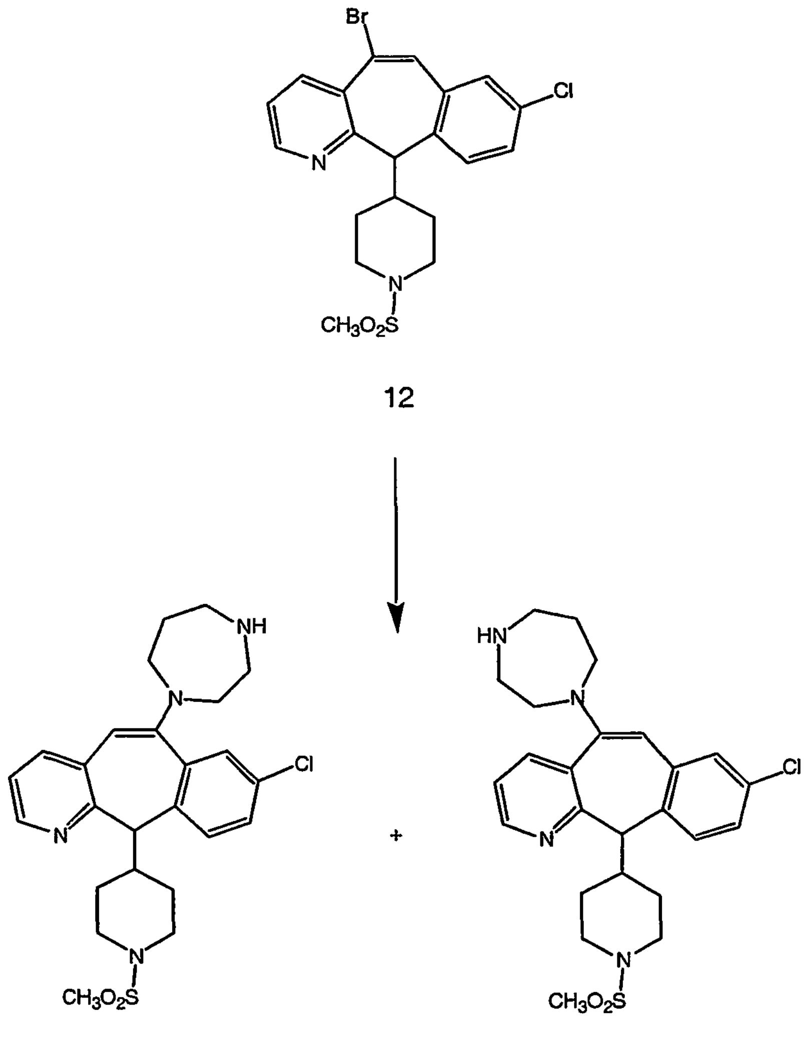

12

35 + 36

Product from Preparative Example 2, Step B (0.4 g, 0.86 mmol) was treated in the same manner as described in Preparative Example 1 Step E, substituting homopiperazine (Aldrich), to give of a mixture of compounds 35 and 36 that were further separated by flash chromatography, eluting with 10% $CH_3OH$:saturated with $NH_3/CH_2Cl_2$ as eluent to give 0.13 g of target compound (35) and 0.17 g of target compound (36).

Compound (35): mp = 116-117; MS (FAB) m/z 487 ($MH^+$).
Compound (36): mp = 111-112; MS (FAB) m/z 487 ($MH^+$).

EXAMPLE 4

Preparation of Compounds (37) and (38).

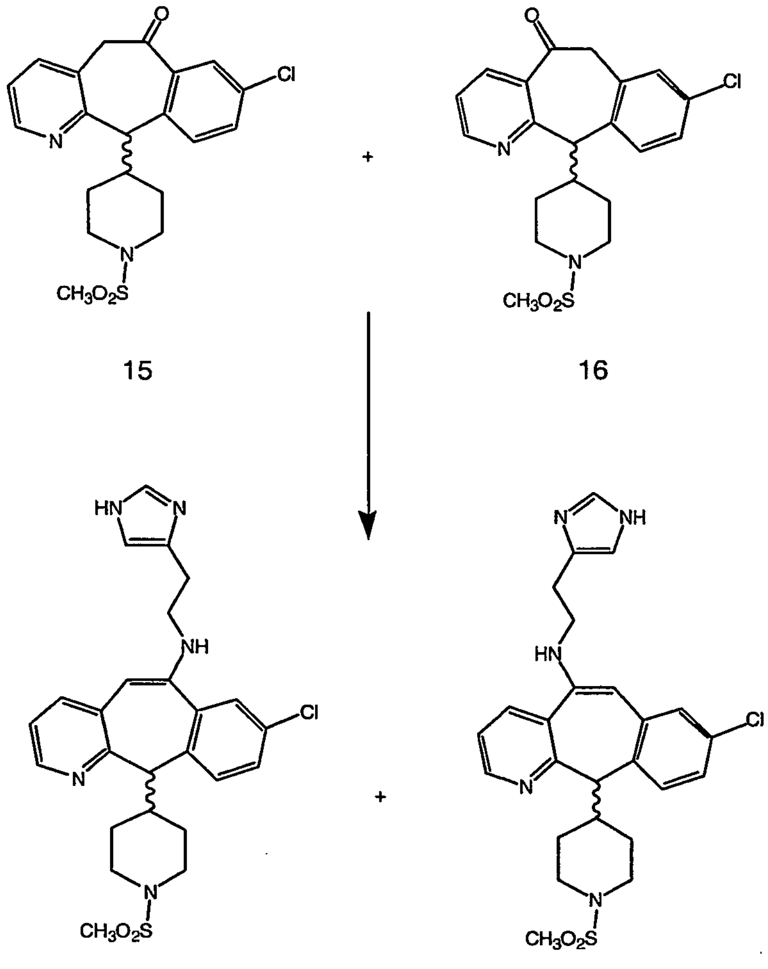

15 + 16

37 + 38

The ketones of Preparative Example 2, Step D (0.50 g, 1.23 mmol), Histamine® (0.21 g, 1.8 mmol) and p-toluene sulfonic acid (monohydrate) were dissolved in anhydrous toluene (40 mL) and refluxed in a Dean Stark trap apparatus for 24 h. The reaction mixture was then cooled, diluted with ethyl acetate and extracted with $NaHCO_3$. The organic layer was then dried over $MgSO_4$ and concentrated to dryness. Purification by flash chromatography on silica gel, eluting with 3% $CH_3OH$(saturated with $NH_3$)-$CH_2Cl_2$, afforded 0.17 g (28% yield) 5-substituted histamine adduct (38) as the first eluting product and 0.08 g (13% yield) of the 6-substituted histamine adduct (37) as the second eluting product.

Compound (37): mp = 124-125; MS (FAB) m/z 498 ($MH^+$).
Compound (38): mp =119-120; MS (FAB) m/z 498 ($MH^+$).

<u>EXAMPLES (5) AND (6).</u>

By using the same procedure as above and substituting the appropriate amines, the following mixtures of compounds were prepared:

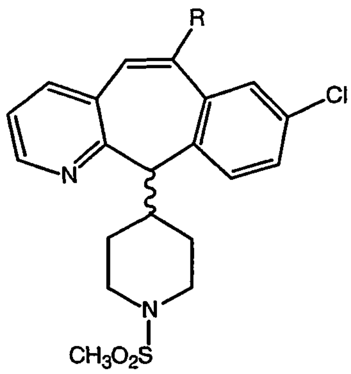

+

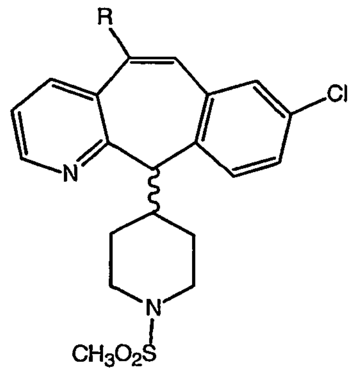

| Ex | R= | Compound #: |
|---|---|---|
| 5 | 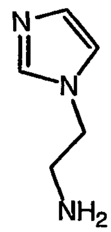 | (39) AND (40). |
| 6 | 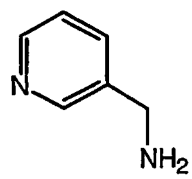 | (41) AND (42). |

EXAMPLE 7

Preparation of Compounds (43) and (44).

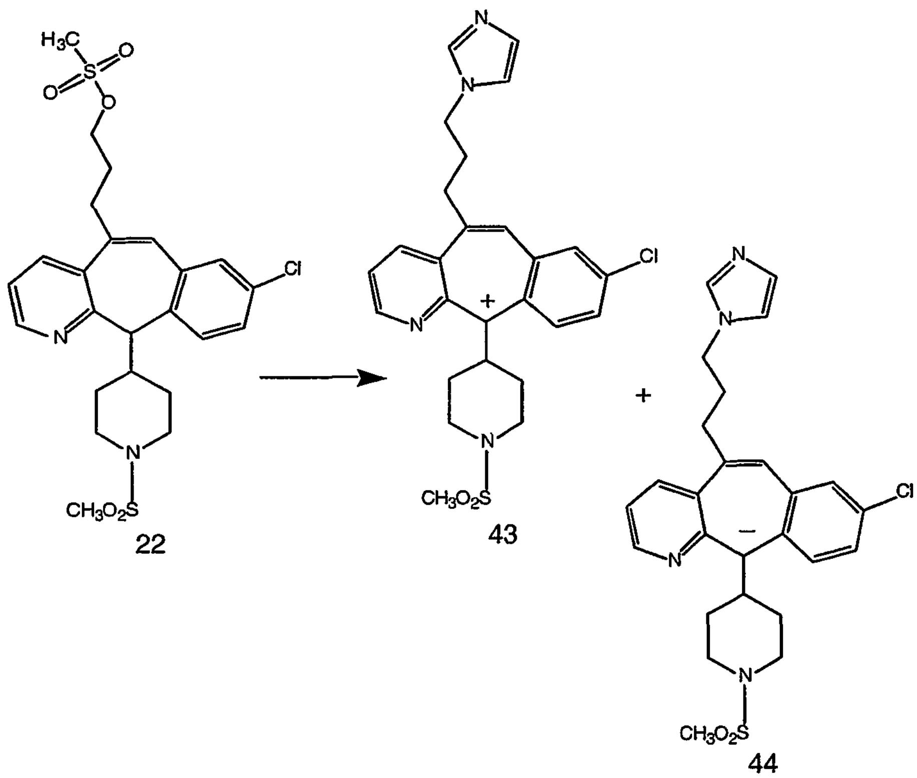

22

43

44

To a solution of the title compound (22) from Preparative Example 3, Step C (1.0 g, 2.03 mmole) in DMF (20 ml) was added imidazolylsodium (0.257 g, 2.85 mmole). The reaction mixture was heated to 90˚C for 2 h. Cooled the reaction and removed DMF. Added saturated sodium bicarbonate and extracted with $CH_2Cl_2$. Dried organic layer over magnesium sulfate, filtered and concentrated to dryness. Crude product was purified by Biotage column chromatography eluting with 3% $CH_3OH$: (saturated with ammonia)-$CH_2Cl_2$, to afford the title compound as an enantiomeric mixture. The mixture was separated into pure enantiomers on Prep HPLC Chiral AD column eluting with 35-40% Isopropanol-Hexane: 0.2% Diethyl amine, to give the title compounds (43) and (44). MS 497 ($MH^+$)

EXAMPLE 8

Step A Preparation of Compound (45).

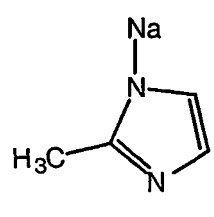

45

2-methylimidazole was dissolved in DMF (10 ml). To this was added one equivalent of NaH and the reaction was allowed to stir at room temperature for 1 h.

Step B Preparation of Compound (46).

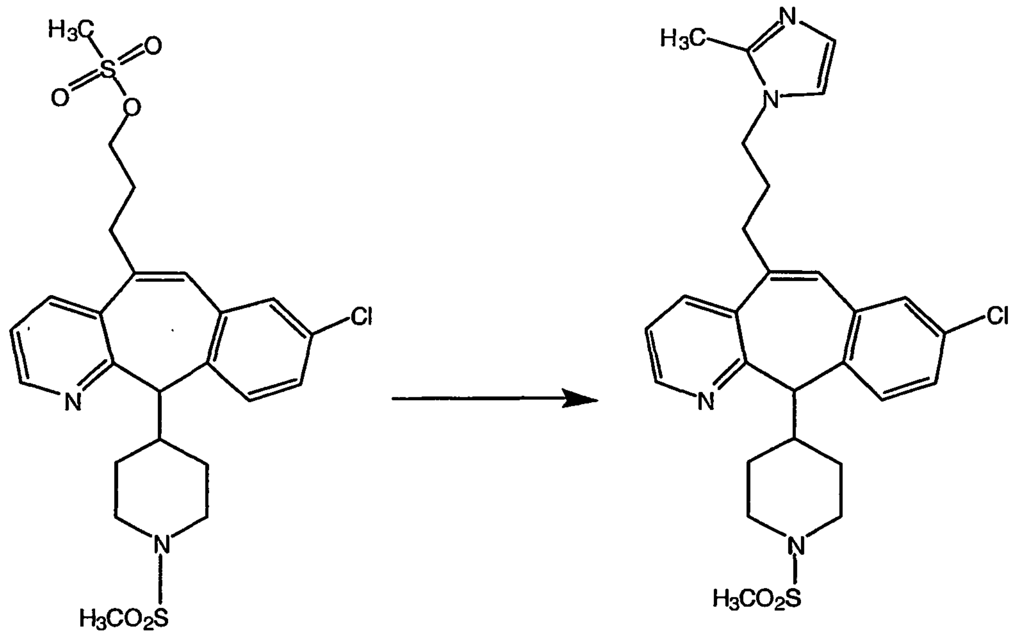

22 46

Following a similar procedure as described in Example 7, substituting 2-methyl imidazoyl sodium (45) for imidazoyl sodium, the racemic mixture of the title compound (46) was prepared. MS 511 ($MH^+$).

EXAMPLE 9

MIXTURE OF COMPOUNDS (47) AND (48).

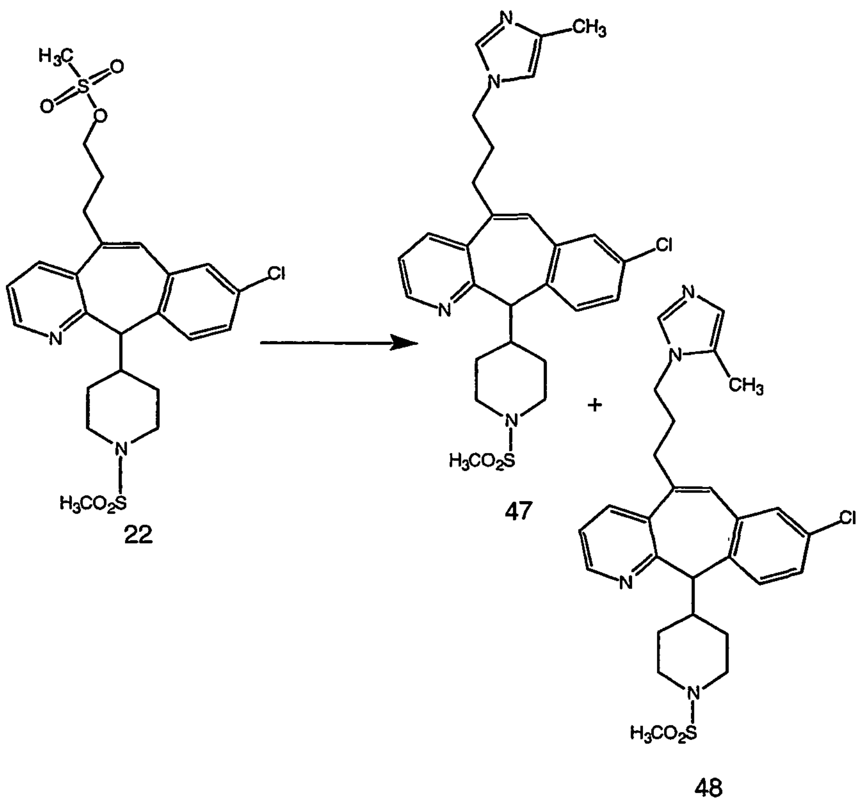

22

47

48

Compound (22) was reacted in the same the same manner as Example 8, substituting 4-methyl imidazole in Step A, affording a mixture of 4 and 5-methyl substituted imidazole derivatives (47) and (48).

EXAMPLE 10

Step A Preparation of Compound (49).

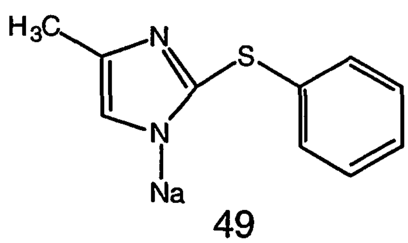

49

To SEM protected methyl imidazole (30 g, 0.141 mole) prepared according to literature procedure, Whitten, J.P., J. Org. Chem. 1986, 51, 1891-1894., in THF (250 ml) at -78˚C was added 2.5 M n-butyl lithium (74 ml, 0.184 mole) over 1 h. The solution was stirred for 1 h at -78˚C, then a solution of diphenyl disulfide (34.27 g, 0.155 mole) in THF (125 ml) was added over 1/2 h. The mixture was stirred and warmed to room temperature over night. The solvents were removed and then the residue was diluted with ethyl acetate (250 ml) and washed with 1.0 M NaOH (5 x 50 ml) and then brine (50 ml). The organic layer was dried over $Na_2SO_4$, filtered and concentrated. The crude product (45.28 g, 0.141 mole) was dissoved in ethanol (100 ml) and 5 M aqueous HCl (100 ml) and stirred for 12 h. at 60˚C. The solvent was removed and the residue was dissolved in distilled $H_2O$. 5M aqueous NaOH was added until pH=8, then the mixture was extracted with ethyl acetate. Combined organic layers and washed with brine, dried over $Na_2SO_4$, filtered and concentrated. Purified by flash chromatography eluting with 70% Hexanes:Acetone to afford the product as a white solid. The amine was further reacted with NaH (1 equivalent) in DMF for 1 h. affording the title compound (49).

Step B Preparation of Compound (50).

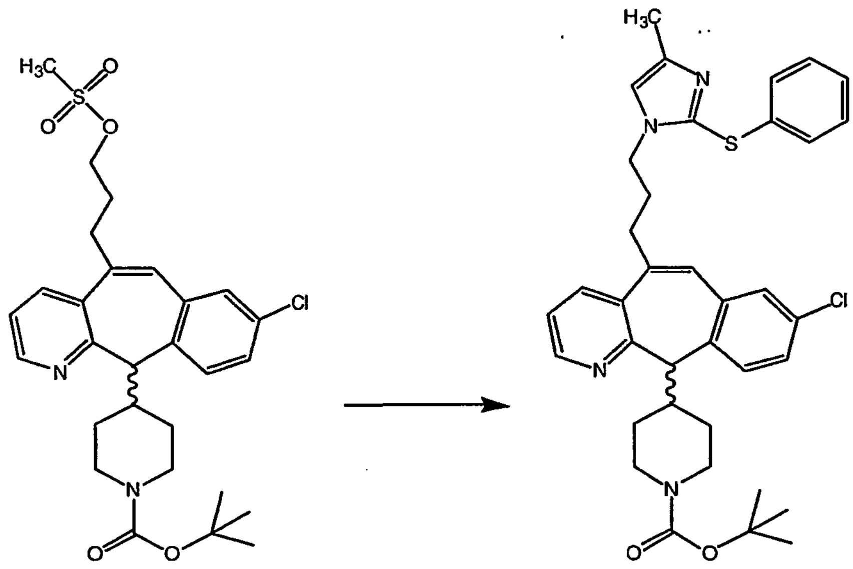

27 50

Compound (27) from PREPARATIVE EXAMPLE 4, STEP E was reacted in the same manner as EXAMPLE 8, substituting 4-methyl-2-phenylsulfanyl-1H-imidazole sodium (49), affording the title compound (50) as a light yellow solid. MS 643 ($MH^+$).

EXAMPLE 11

Step A Mixture of Compounds (51) AND (52).

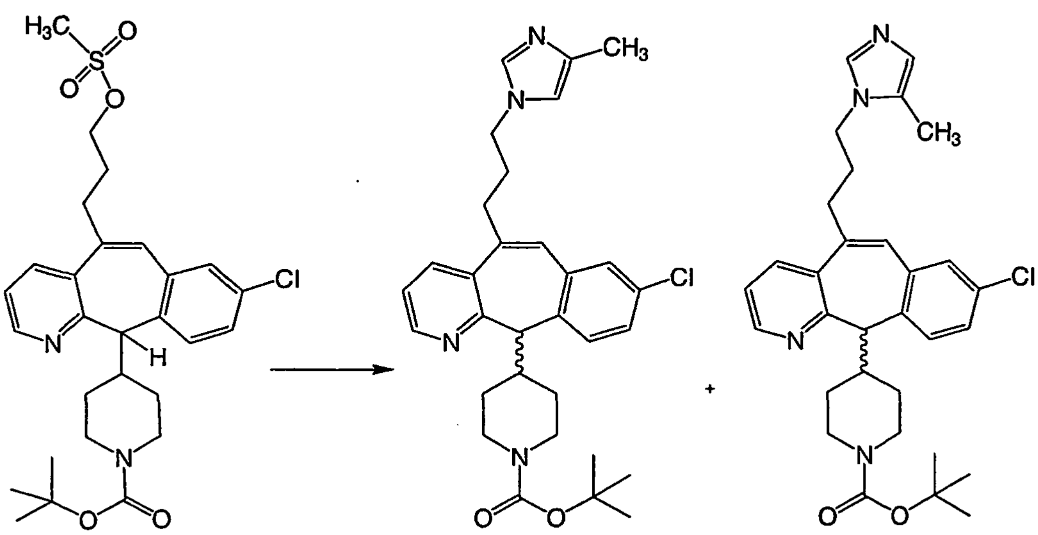

27 51 52

Compound (27) from PREPARATIVE EXAMPLE 4, STEP E, was treated in the same manner as in Example

9 above to afford a mixture of the 4 and 5-substituted imidazol title compounds (51) and (52).

Step B Preparation of pure (+,-) Compounds (53A) & (53B), and pure (+,-) (54A) & (54B).

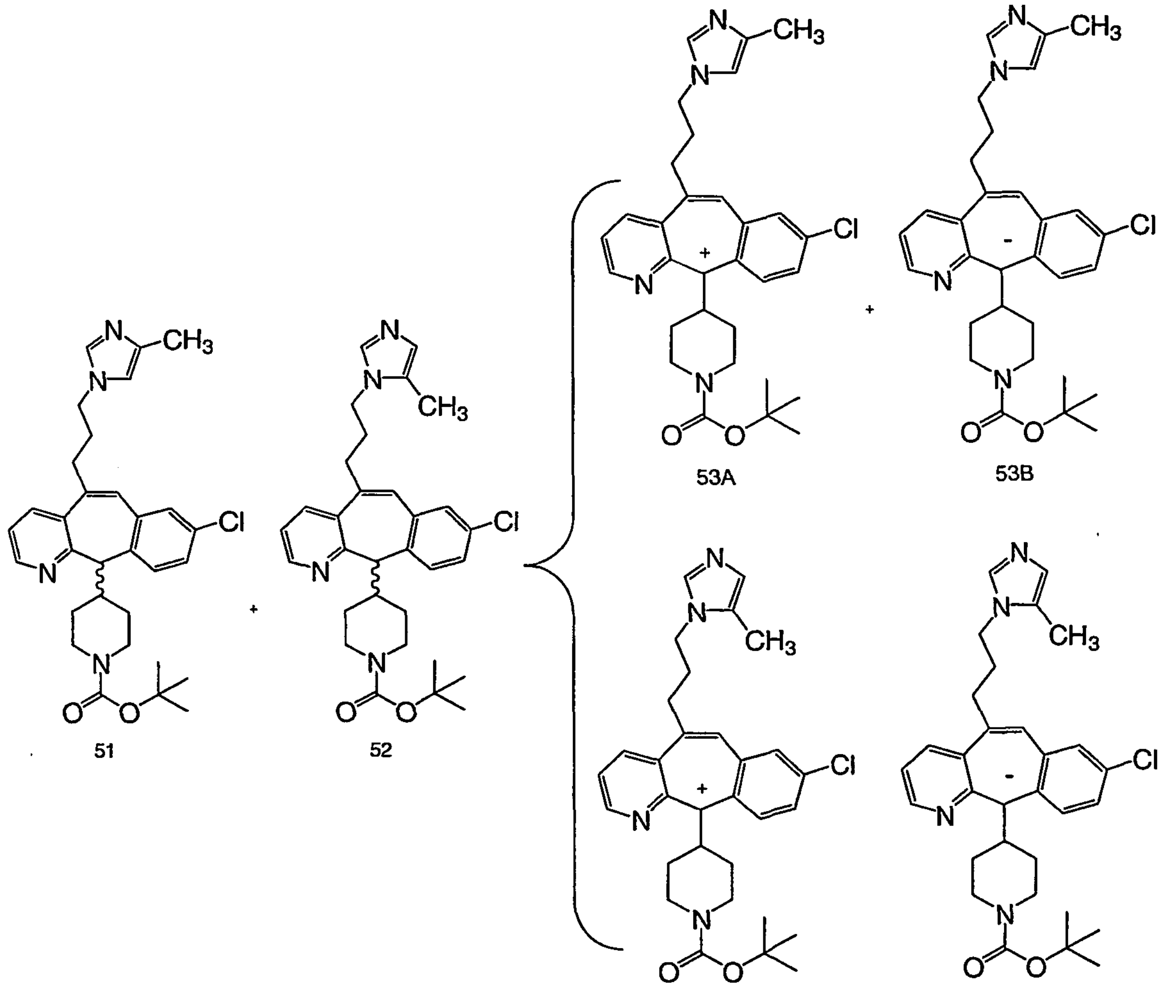

51 + 52

53A + 53B

54A 54B

The compounds from Step A above were further seperated into a mixture of (4 and 5) (+) enantiomers and (4 and 5) (-) enantiomers using preparatory HPLC Chiral AD column, eluting with 20% Isopropanol-Hexane : 0.2% Diethyl amine. MS 532 ($MH^+$). The pure (+) and (-) enantiomeric pairs were then reacted with triphenyl methyl chloride (Aldrich) in $CH_2Cl_2$ starting at 0°C and warming to room temperature over 3 h. The crude product was purified by column chromatography eluting with 50% ethyl acetate-acetone, affording the pure (+) and (-) 4-methyl substituted enantiomers (53A) and (53B); MS 533 ($MH^+$). The column was then flushed with 100% methanol, the fraction was concentrated and the residue was treated with methanol saturated with ammonia, overnight at reflux temperature. The product was purified by column chromatography eluting with 50% ethyl acetate-acetone, affording the pure (+) and (-) 5-methyl substituted enantiomers (54A) and (54B); MS 533 ($MH^+$).

EXAMPLE 12

Preparation of Compounds (55) and (56).

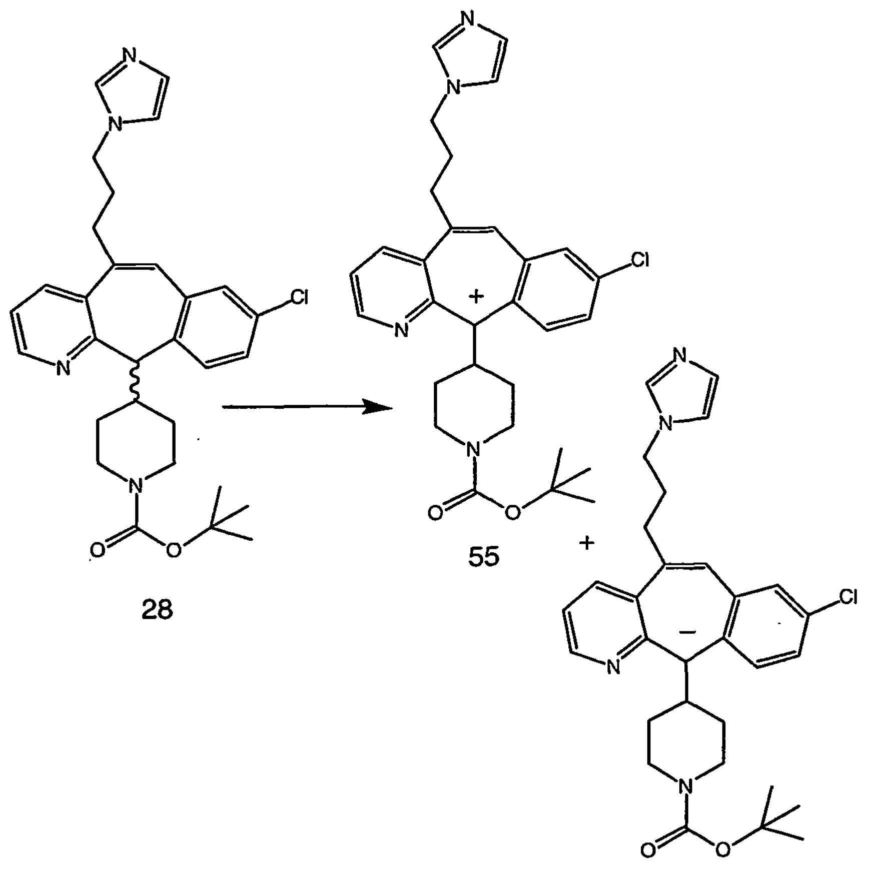

28

55

+

56

Compound (28) from PREPARATIVE EXAMPLE 4, STEP F, was separated into pure enatiomers by preparatory HPLC using a chiral AD column eluting with 20% Isopropanol:Hexane: 0.2% Diethyl amine to give pure title compounds (55) and (56). MS 519 ($MH^+$).

EXAMPLE 13

Preparation of compound (57).

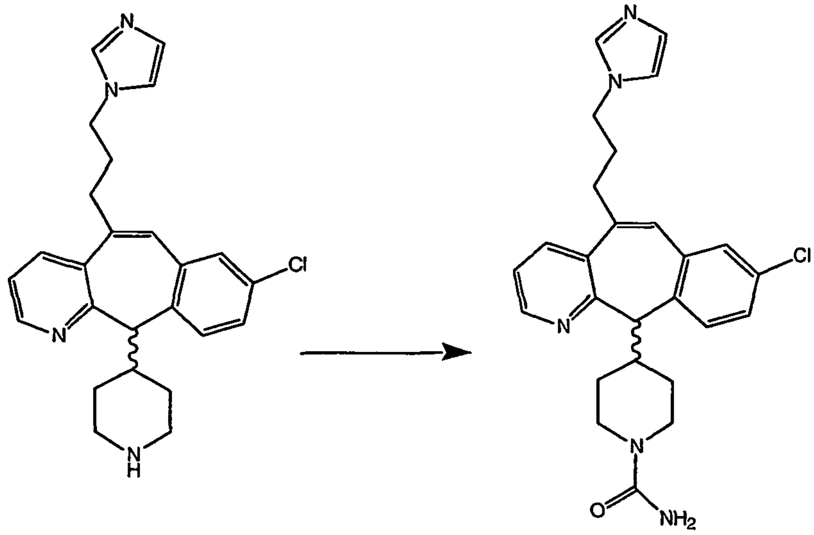

29 57

Compound (29) from PREPARATIVE EXAMPLE 4, STEP G (0.20 g, .48 mmole) was dissolved in $CH_2Cl_2$ (10 ml). Added triethyl amine (0.30 ml, 1.92 mmole) followed by trimethylsilyl isocyanate (Aldrich) (1.3 ml, 9.6 mmole) and stirred at room temperature over night. Quenched reaction with 1.0 N NaOH and extracted with $CH_2Cl_2$. Dried organic layer over $MgSO_4$, filtered and concentrated. Purified by column chromatography eluting with 3-5% Methanol saturated with Ammonia-$CH_2Cl_2$, affording the title compound (57) as a white solid. MS 464 ($MH^+$).

EXAMPLES 14 AND 15

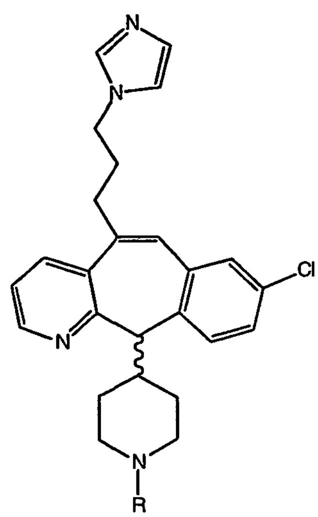

By substituting the appropriate isocyanates, and following the procedure described in EXAMPLE 13 above, the following compounds were prepared:

| Ex | R= | Compound #: |
|---|---|---|
| 14 | 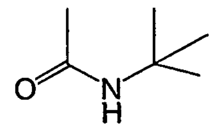 | (58). MS 518 ($MH^+$). |
| 15 | 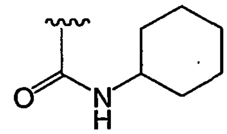 | (59). MS 544 ($MH^+$). |

EXAMPLE 16

Preparation of Compound (60).

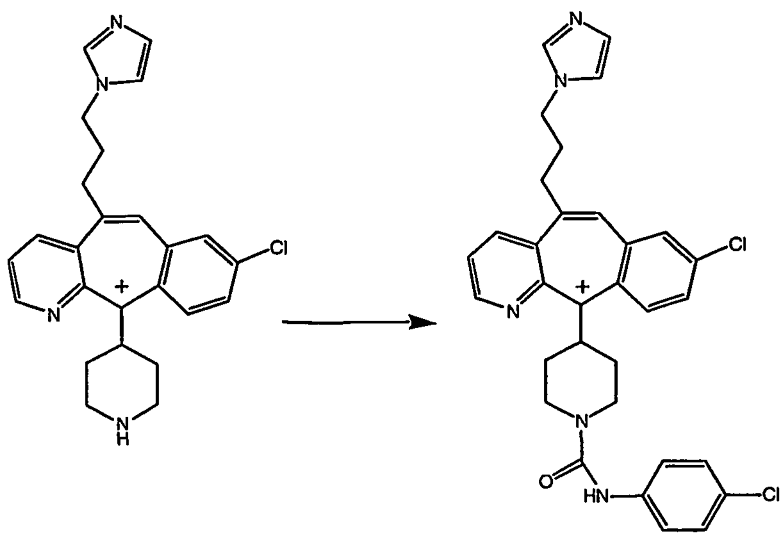

60

Compound (55) was deprotected following the procedure described in PREPARATIVE EXAMPLE 4, STEP G, to give the (+) enantiomer of the starting amine which was then reacted with 4-Chlorophenyl isocyanate (Aldrich) (0.05 g, 0.34 mmole) in the same manner as Example 13 above, affording the title compound (60) as a white solid. MS 572 ($MH^+$).

EXAMPLE 17

Preparation of Compound (61).

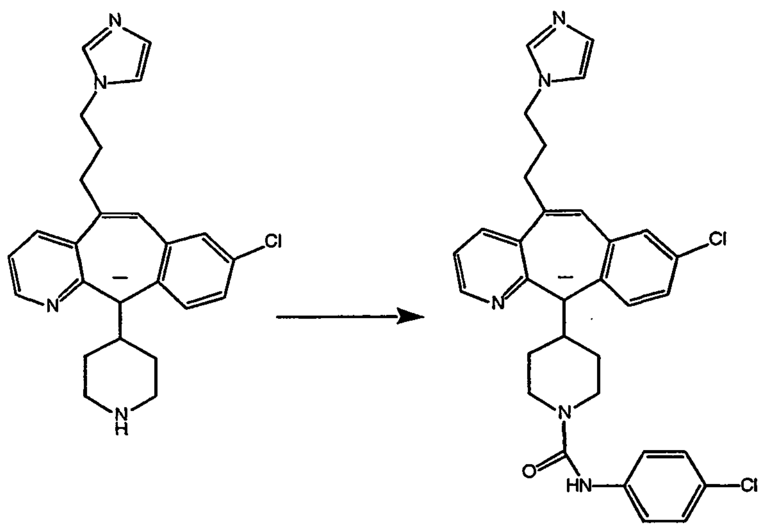

61

Compound (56) was deprotected following the procedure described in PREPARATIVE EXAMPLE 4, STEP G to give the (-) enantiomer of the starting amine. Reacting in the same fashion as Example 16 above, afforded the title compound (61) as a white solid. MS 572 ($MH^+$).

EXAMPLE 18

Preparation of Compound (62).

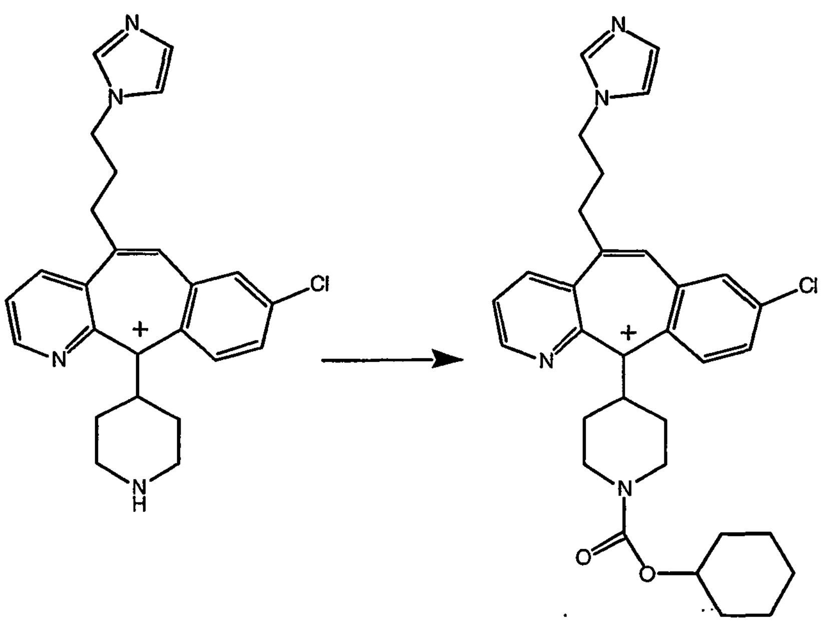

62

Following the procedure described in EXAMPLE 16, substituting cyclohexyl chloroformate (BASF) in place of the isocyanate, afforded the title compound (62) as a white solid. MS 545 ($MH^+$).

EXAMPLE 19

Preparation of Compound (63).

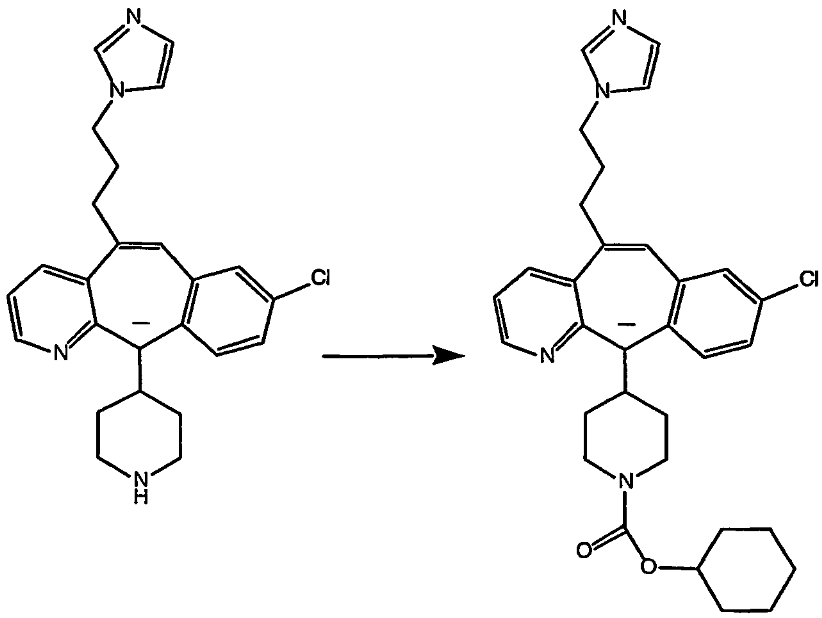

63

Following the same procedure as described in EXAMPLE 18 above, substituting the (-) enatiomer of the starting amine from EXAMPLE 17, afforded the title compound (63) as a white solid. MS 545 ($MH^+$).

PREPARATIVE EXAMPLE 6

A. PREPATION OF TRIBUTYL-(2-ETHOXY-VINYL)-STANNANE (64).

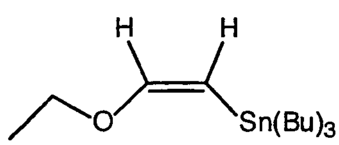

64

In a sealed tube, was added ethoxy ethyne (Fluka) followed by tributyltin hydride (Aldrich) and heated to 55°C for two days. The reaction mixture was then concentrated to a brown red liquid. Purification via distillation afforded the title compound (64) as an off-white liquid. BP range 98°-115°C, (.35 to .2 mmHg).

Step B Preparation of Compound (65).

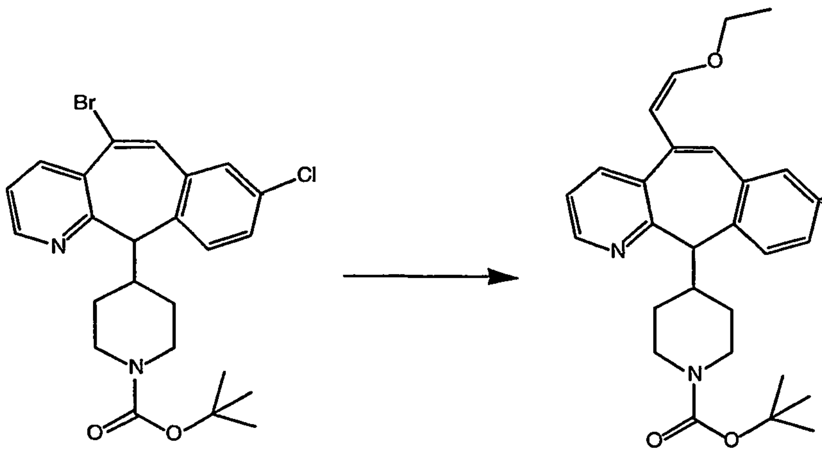

23 65

To a solution of compound (23) from Preparative Example 4, Step A (6.51 g, 13.29 mM), dichlorobis(triphenylphosphine) palladium (II) (Alrich) (0.373 g, .53 mM), and tetrabutylammonium chloride (Aldrich) (3.69 g, 13.29 mM) in DMF (50 ml) was added compound (64) from PREPARATIVE EXAMPLE 6, STEP A. The reaction stirred over night at 75-80˚C under nitrogen atmosphere. The reaction was cooled to room temperature, then a solution of KF (.93 g, 15.94 mM) in H2O (70 ml) was added. A precipitate formed upon addition. The reaction mixture was stirred for fifteen minutes then added $CH_2Cl_2$ and stirred an additional fifteen minutes. The reaction mixture was extracted with $CH_2Cl_2$, the organic layer was dried over magnesium sulfate, filtered and concentrated. Purified by silica gel column chromatography eluting with 1:3% -1:1% ethyl acetate-hexanes affording the title compound (65) as a yellow solid, mp 86-90˚C.

Step C Preparation of Compound (66).

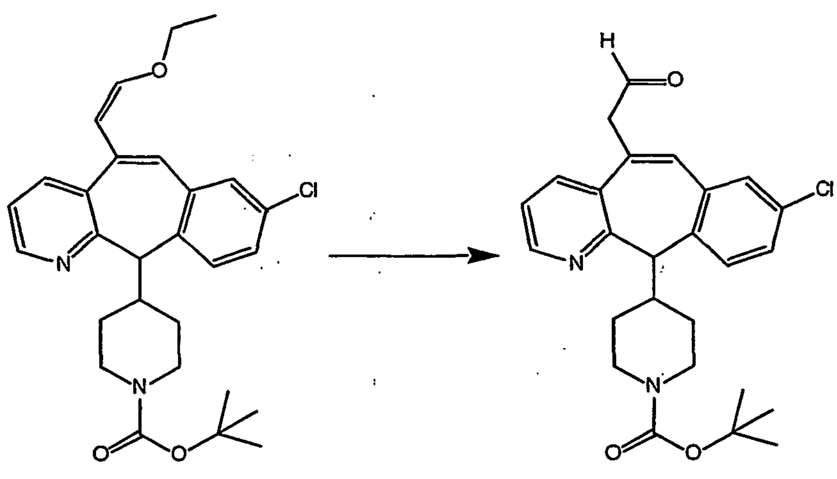

65 66

To a solution of compound (65) from Preparative Example 6, Step B (3.25 g, 6.76 mM) in THF/H2O (33.7 ml/ 7.3 ml), was added mercury (II) acetate. The reaction stirred at room temperature for fifteen minutes during which time a precipitate formed. To the mixture was then added saturated KI solution (70-80 ml) and was stirred for five minutes. Added $CH_2Cl_2$ and stirred for 1 h. The reaction was extracted with $CH_2Cl_2$ (2 x 100 ml). The organic layer was dried over magnesium sulfate, filtered and concentrated to afford the title compound (66) as a light brown solid. MS 453 ($MH^+$).

D. Preparation of Compound (67).

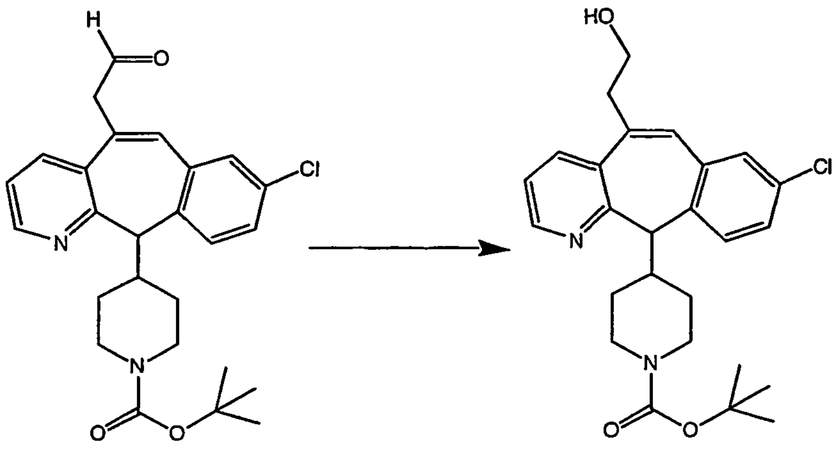

66 67

To a solution of compound (66) from Preparative Example 6, Step C (3.06 g, 6.8 mM) in ethanol (40 ml) was added sodium borohydride (0.31 g, 8.1 mM) in two portions over seven minutes. The reaction stirred for 45 minutes was then concentrated, taken up in ethyl acetate and washed with brine. Re-extracted brine layer with additional ethyl acetate and then combined organic layers, dried over magnesium sulfate, filtered and concentrated to a solid. Further purification by silica gel column chromatography eluting with 1:1-5:1 ethyl acetate-hexane afforded the title compound (67) as a white solid. MP range 120-130˚C; MS 455 ($MH^+$).

E. Preparation of Compound (68).

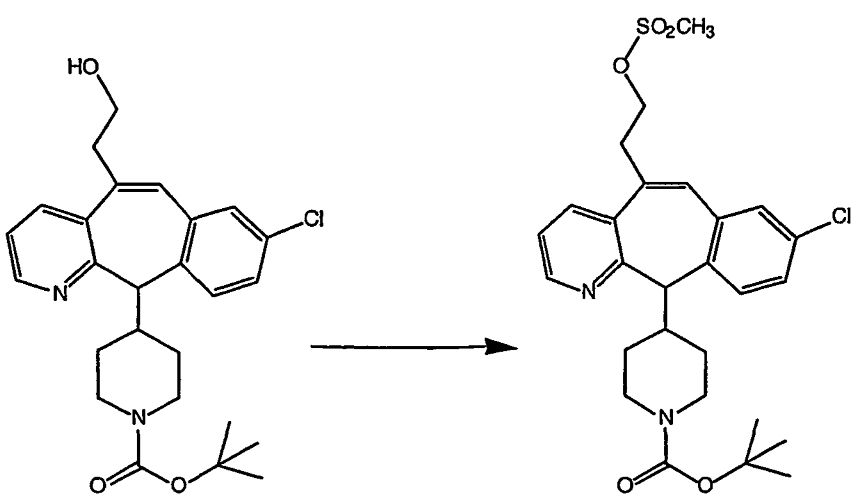

67 68

Compound (67) from Preparative Example 6, Step D was reacted in the same manner as described in Preparative Example 3, Step C, to afford the title compound (68) as a peach solid.

F. Preparation of compound (69).

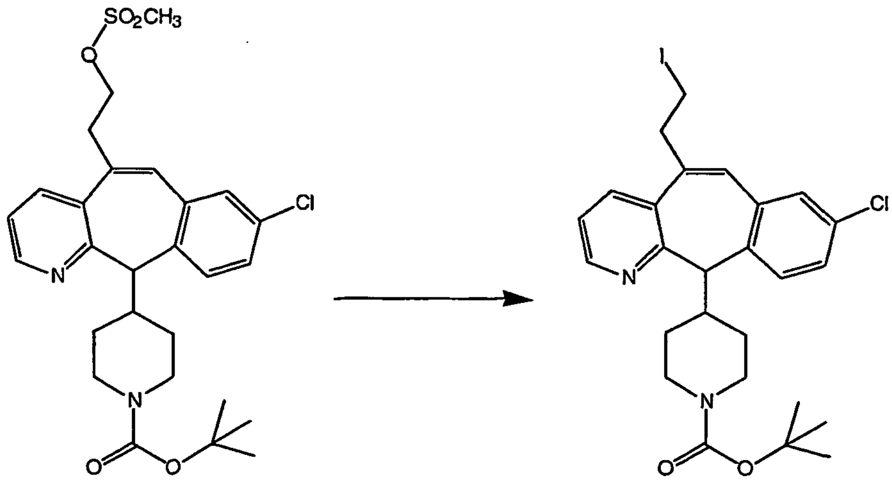

68 69

Compound (68) from Preparative Example 6, Step D (0.1g, .19 mM) was dissolved in THF (2.5 ml). To the mixture was added LiI (Aldrich) (0.064 g, .48 mM) and stirred over night at room temperature. The reaction mixture was concentrated, taken up in $CH_2Cl_2$ and washed with brine (25 ml). The organic layer was dried over magnesium sulfate, filtered and concentrated to afford the title compound (69) as a yellow-brown solid.

EXAMPLE 20

Preparation of compound (70).

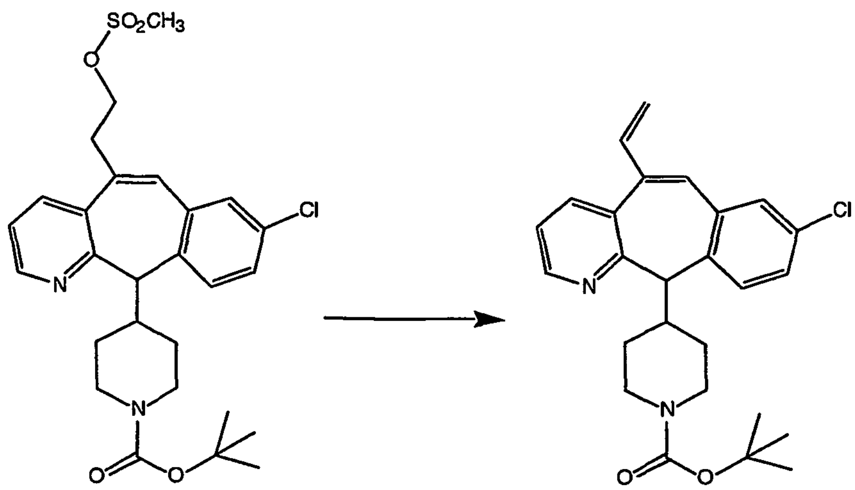

68 70

Compound (68) from Preparative Example 6, Step E, was reacted in the same manner as described in Example 8, Step B, resulting in the title compound (70) as a white solid, mp 94-101˚C.

EXAMPLE 21

Preparation of Compound (71).

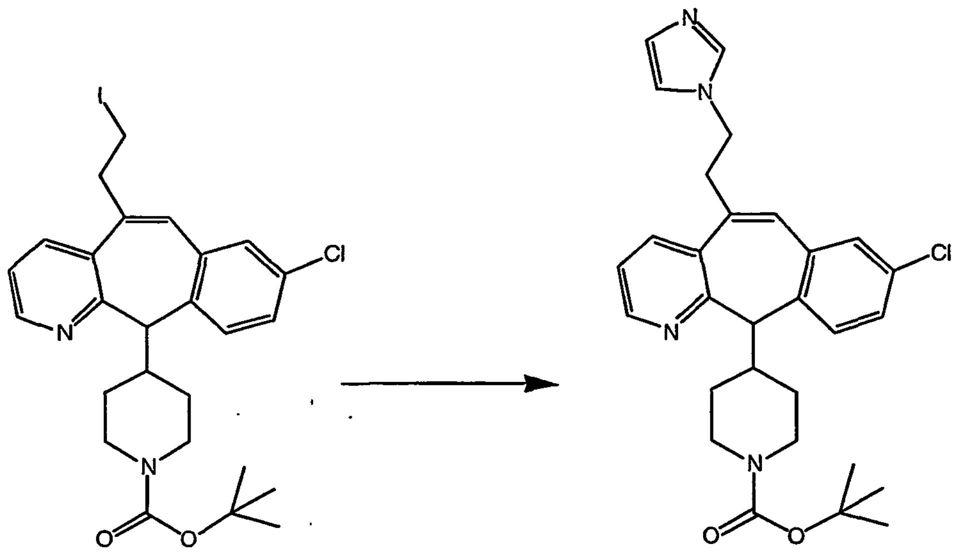

69 71

To compound (69) from Preparative Example 6, Step F (0.3 g, .05 mM) in $CH_3CN$ (1 ml) was added imidazole (Aldrich) (0.014 g, .2 mM). The reaction was heated to 52°C and stirred over night. The reaction was cooled, concentrated, then diluted with ethyl acetate and washed with brine. The organic layer was dried over magnesium sulfate, filtered and concentrated. The product was purified by silica gel column chromatography eluting with 0-5% methanol/ saturated with ammonia:$CH_2Cl_2$ to afford the title compound (71)as a white solid. mp 95-104°C; MS 505 ($MH^+$).

EXAMPLE 22

Preparation of compound (72).

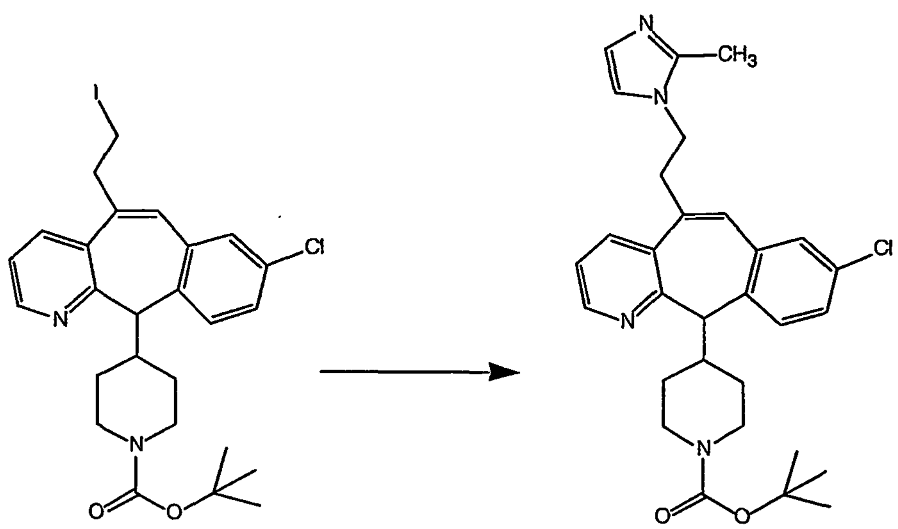

69 72

Substituting 2-methyl imidazole for imidazole and reacting in essentially the same manner as Example 21, the title compound (72) was afforded as a light tan solid. mp 93-104°C.

EXAMPLE 23

Preparation of compound (73).

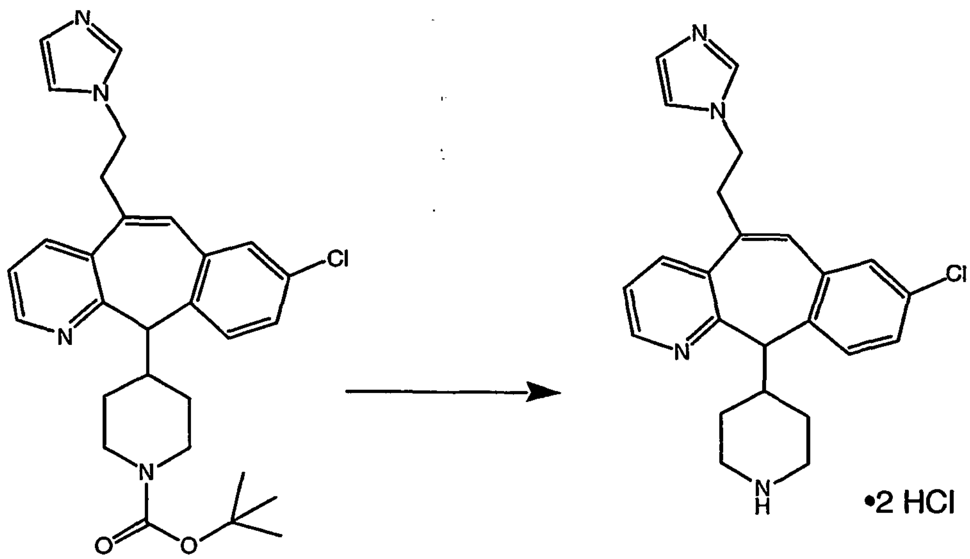

71 73

Compound (71) (0.31 g, 0.06 mM) from Example 21 was dissolved in 4M HCl/Dioxane (0.5 ml) and stirred for 1 h. Concentration of the reaction mixture afforded the title compound (73) as a light yellow solid. mp 195-205˚C.

EXAMPLE 24

Preparation of Compound (74).

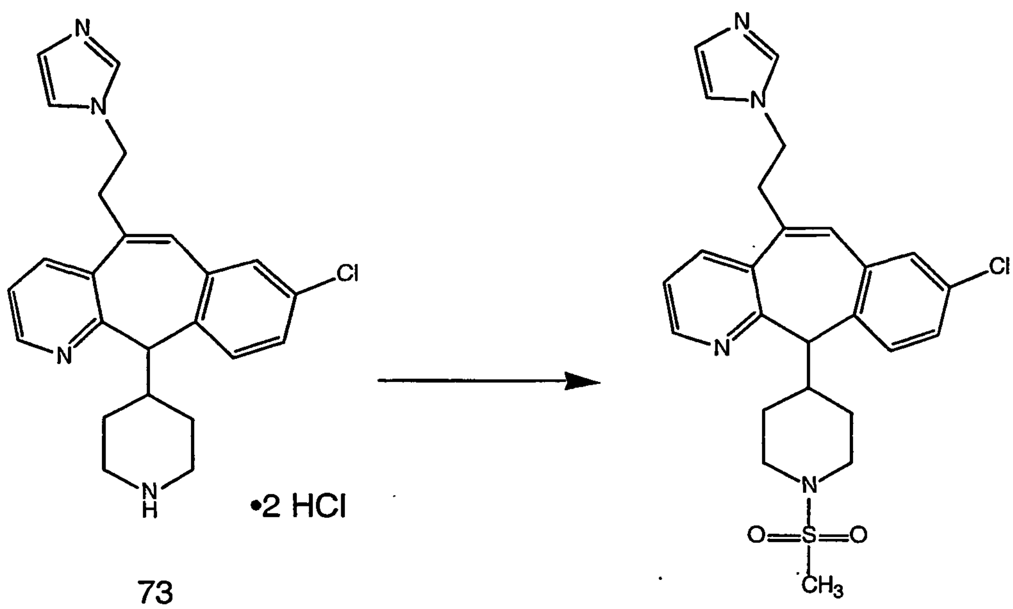

73 74

To a solution of compound (73) from Example 23 (0.026 g, 0.05 mM) in $CH_2Cl_2$, was added, triethyl amine (Aldrich) (0.046 ml, 0.33 mM) followed by methane sulfonyl chloride (Aldrich) (0.01 ml, 0.1 mM). The reaction stirred at room temperature for 36 h. The reaction was quenched with saturated sodium bicarbonate (50 ml) and extracted with ethyl acetate (2 x 75 ml). The organic layer was dried over magnesium sulfate, filtered and concentrated. The product was purified by preparatory thin layer chromatography eluting with 90:10 $CH_2Cl_2$: methanol saturated with ammonia to afford the title compound (74), mp 105-116˚C.

EXAMPLE 25

Preparation of compound (75).

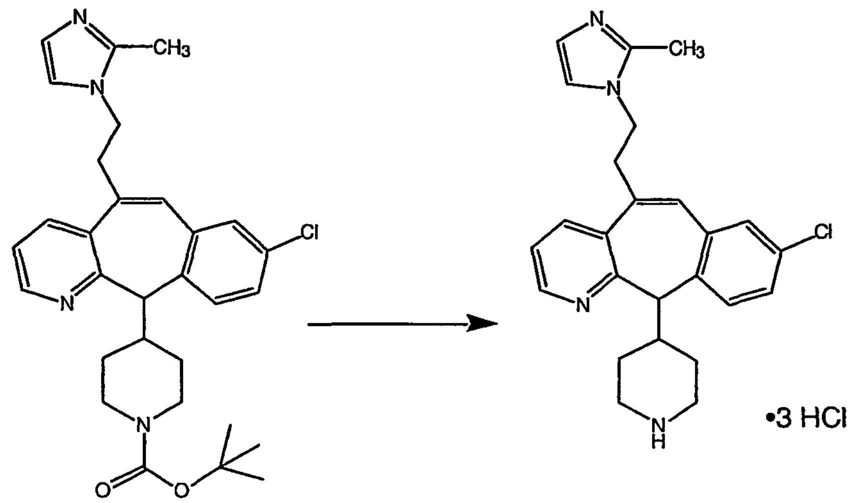

72 75

Compound (72) from Example 22 was stirred with 4M HCl/Dioxane over 2 h Concentration of reaction mixture afforded the title compound (75) as an off-white solid, mp 185-203˚C.

EXAMPLE 26-29

Reacting compound (75) from Example 25, in the same manner as described in Example 13, and substituting the appropriate isocyanate, the following compounds were prepared:

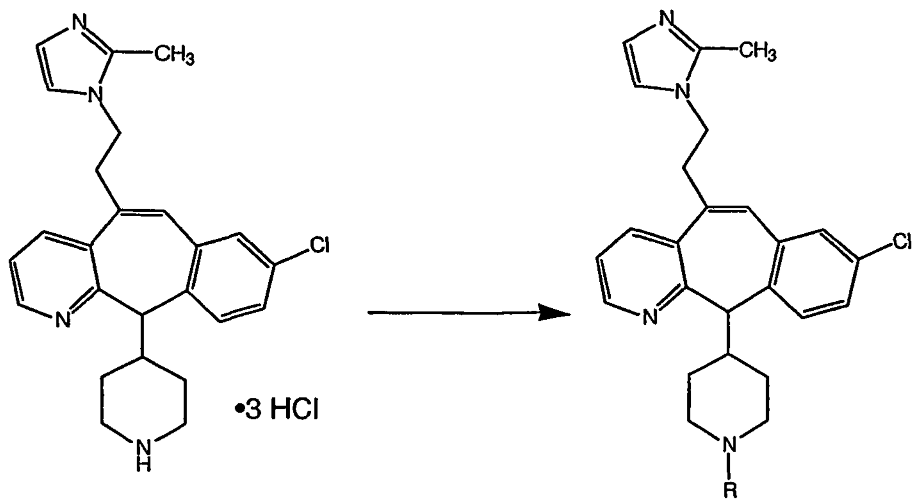


75

| Ex | R= | Compound #: |
|---|---|---|
| 26 | 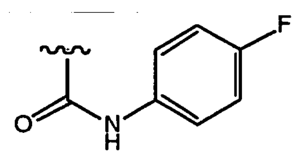 | (76).<br>mp 133-144˚C |
| 27 | 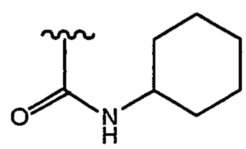 | (77).<br>mp 131-140˚C |
| 28 | 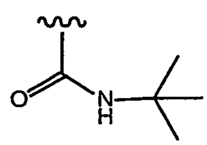 | (78).<br>mp 125-132˚C. |
| 29 | 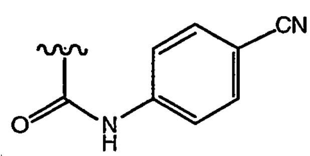 | (79).<br>mp 160-172˚C |

EXAMPLE 30

A. PREPARATION OF CYCLOHEXYL CHLOROFORMATE

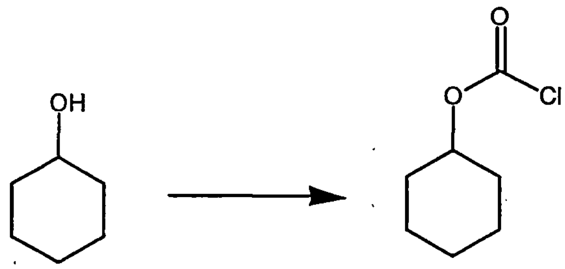

80

A solution of cyclohexanol (Aldrich) (25 ml, 0.2 mol) in $CH_2Cl_2$ (50 ml) was added dropwise over 1 h to a solution of phosgene in toluene (262 ml of a 1.93 M solution, 0.5 mol) at 0˚C. The reaction was warmed to room temperature over 3 h. and stirred over night. The volatiles were removed to afford the title compound (80) as a colorless liquid.

B. Preparation of compound (81).

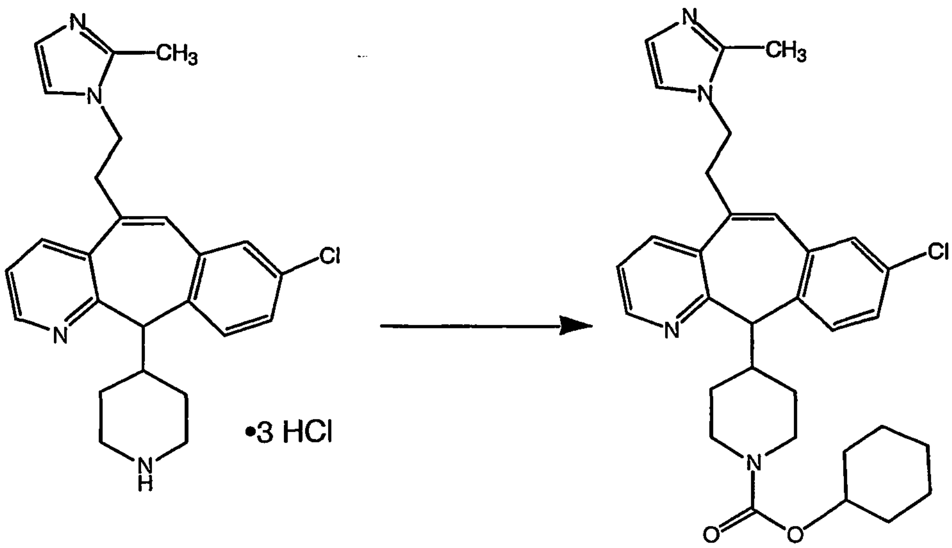

75 81

Reacting compound (75) from Example 25 in the same manner as described in Example 13, substituting the acid chloride (80) from Example 30, Step A in place of the isocyanate, afforded the title compound (81) as an off-white semi-solid.
mp 89-98˚C.

EXAMPLE 31

Preparation of compound (82).

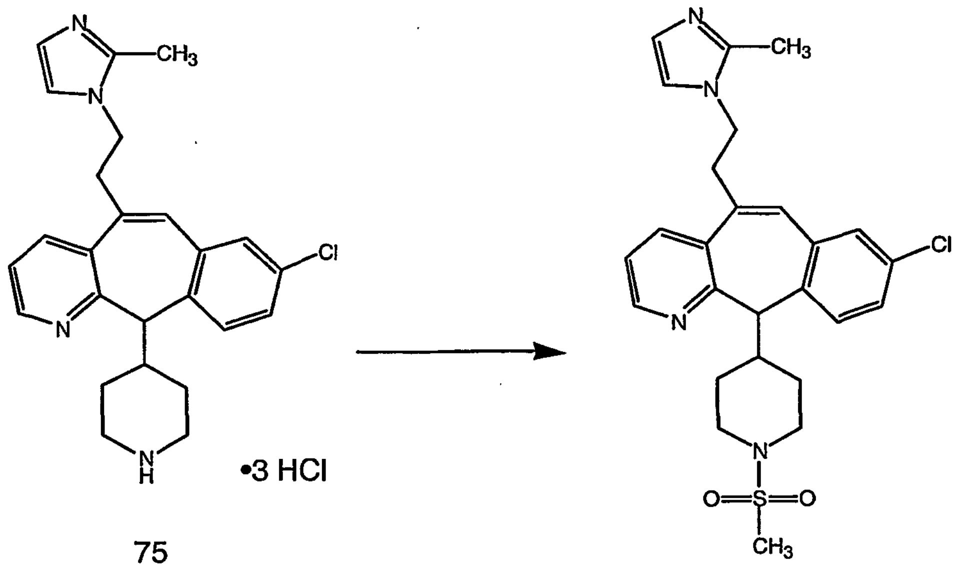


75

82

Reacting compound (75) from Example 25 in the same manner as described in Example 13 but substituting methanesulfonyl chloride in place of the isocyanate, afforded the title compound (82) as a tan semi-solid mp 120-129°C.

EXAMPLE 32

Separation of compound (75) into (+) and (-) enantiomers (83) and (84).

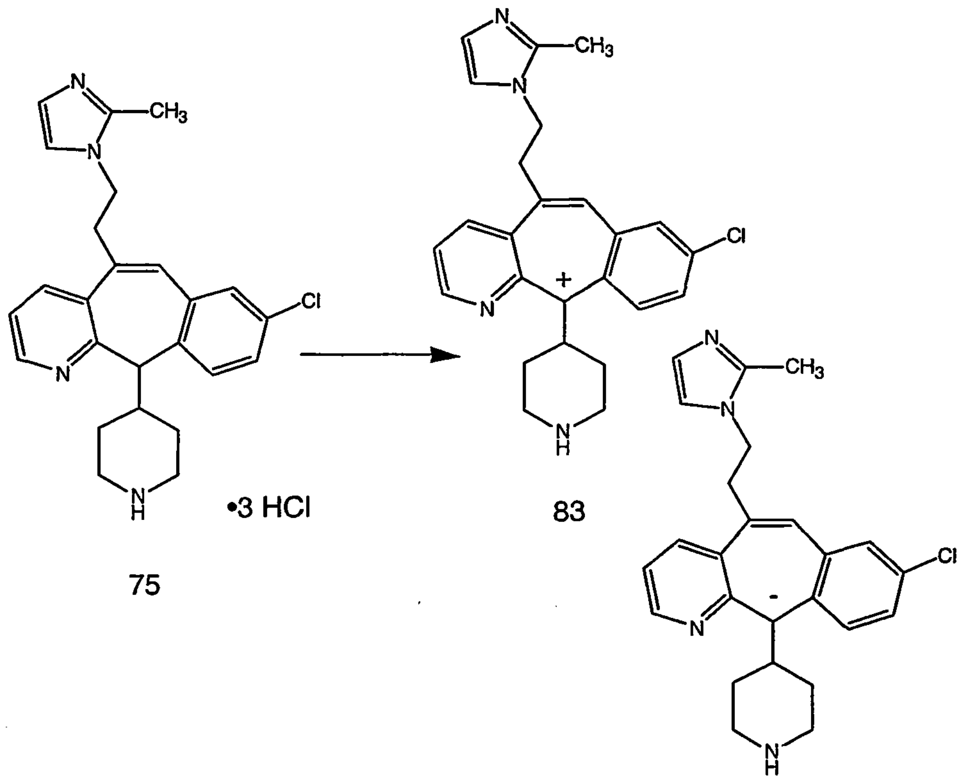

•3 HCl

75

83

84

Compound (75) was seperated into pure (+) and (-) enantiomers using preparatory chiralpak-AD column chromatography, eluting with 85:15:0.2% 2-propanol:hexane/ diethyl amine affording the title compounds (83) and (84) respectively.

EXAMPLE 33

Preparation of compound (85).

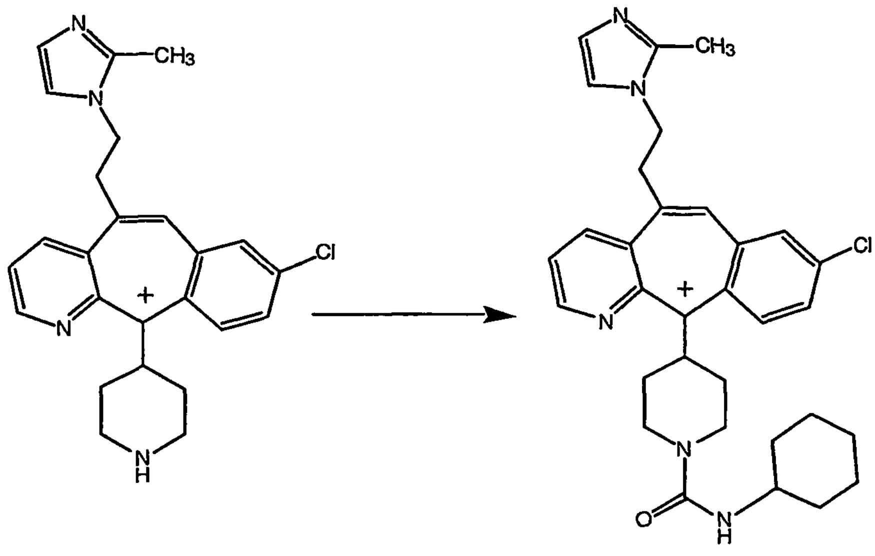

83 85

Compound (83) was reacted in the same manner as in Example 27 affording the title compound (85) as a white solid. mp 122-129˚C.

EXAMPLE 34

Preparation of compound (86).

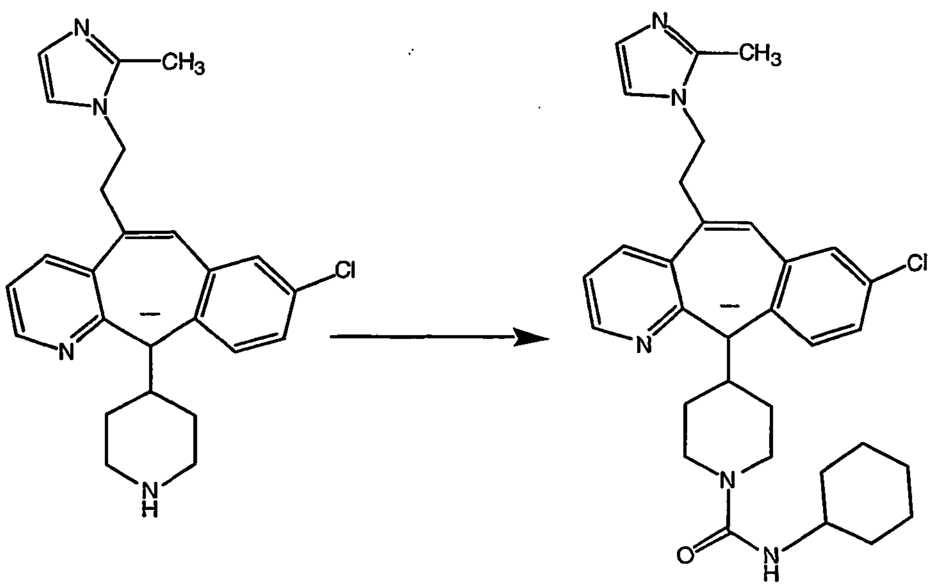

84 86

Compound (84) was reacted in the same manner as in Example 27 affording the title compound (86) as a white solid mp 118-133˚C.

EXAMPLE 35

Preparation of Compounds (87) AND (88).

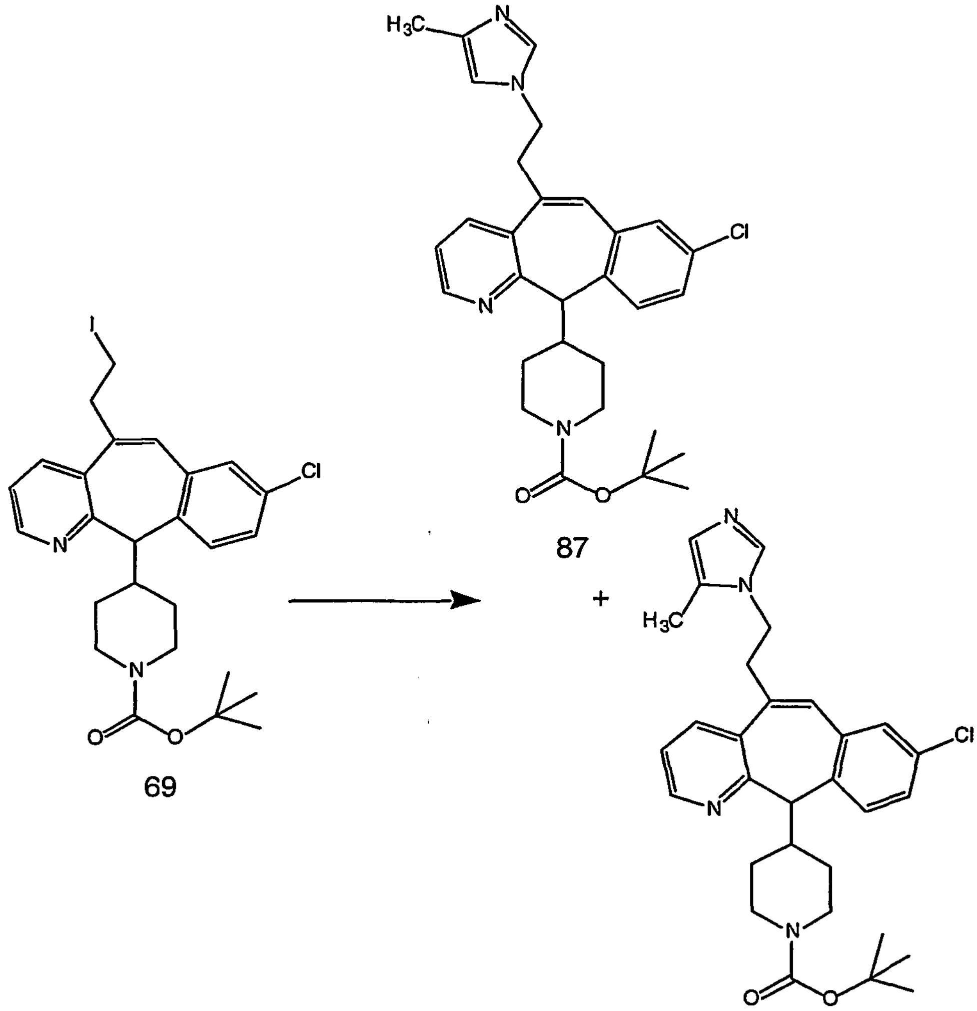

69

87

+

88

Compound (69) from Example 19 was reacted in the same manner as described in Example 21 substituting 4-methyl imidazole for imidazole, to afford a mixture of the 4 and 5 substituted imidazole derivatives. The mixture (0.234 g, 0.45 mM) was subsequently treated with trityl chloride (Aldrich) (0.047 g, 0.17 mM) and separated by preparatory thin layer chromatography, eluting with 1:6% ethyl acetate-acetone affording the pure isomers (87) and (88) mp (87) 97-107˚C (white solid).

EXAMPLE 36

Preparation of compound (89).

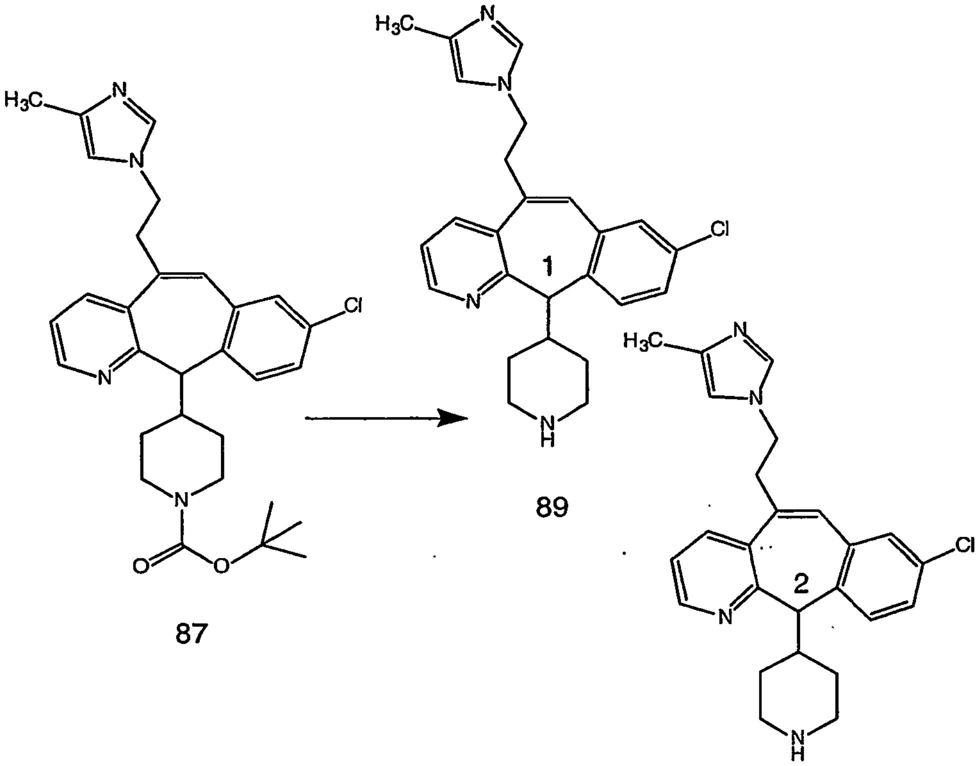

87

89

90

Compound (87) from Example 35 (0.085 g, 0.16mM) was reacted in the same manner as described in Example 25. The resulting enantiomeric mixture was then separated by Preparatory Chiralpak-AD column chromatography eluting with 15-85% Isopropanol-Hexane, 0.2% diethylamine, affording enantiomers 1 and 2 as off-white solids.

EXAMPLE 37

Preparation of compound (91).

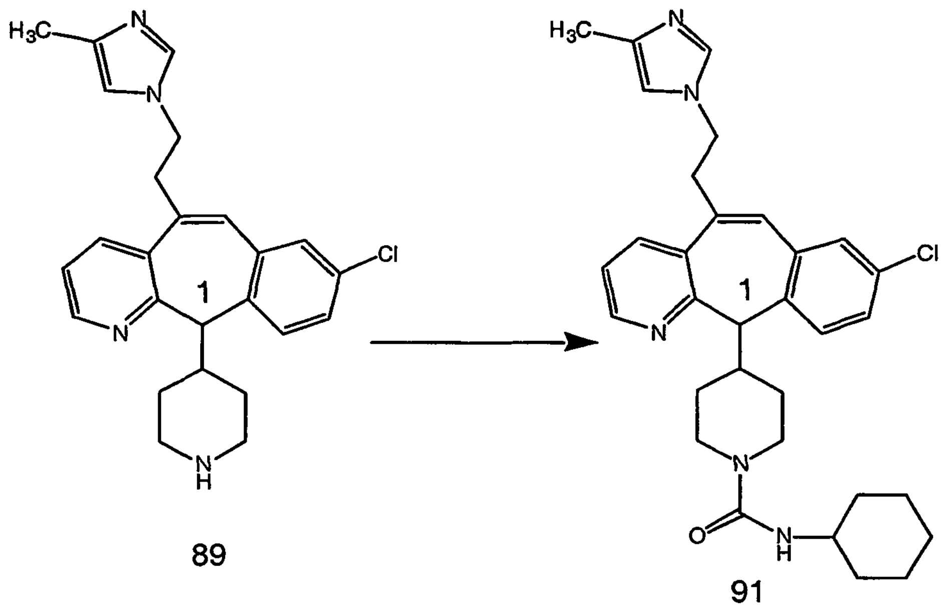

89

91

Enantiomerically pure compound (89) from Example 36 (0.02 g, 0.049 mM) was reacted in a similar manner as in Example 27 to afford the title compound (91) as a white solid. mp 130-142°C

EXAMPLE 38

Preparation of compound (92).

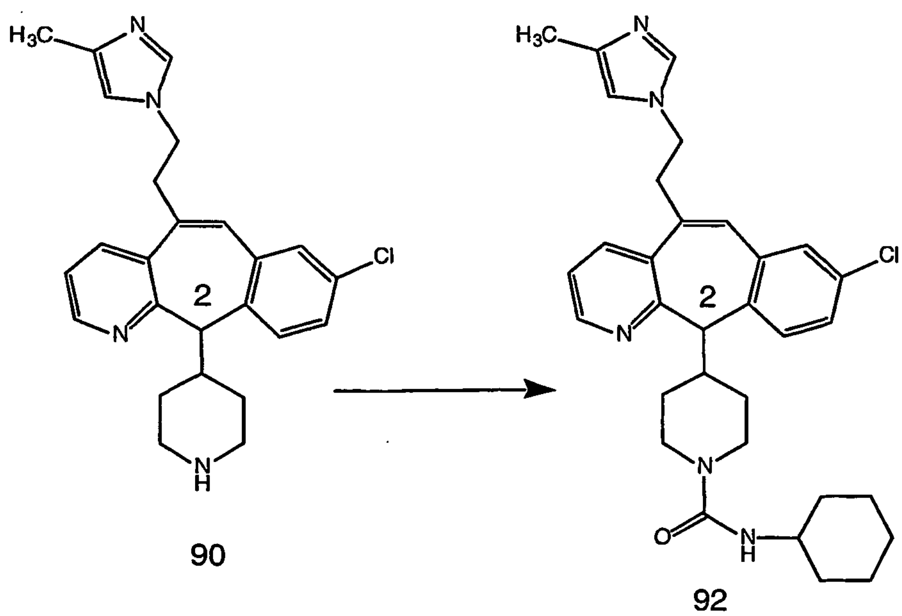

90

92

Enantiomerically pure compound (90) from Example 36 (0.023 g, 0.054 mM) was reacted in a similar manner as in Example 27 to afford the title compound (92). mp 125-135°C.

PREPARATIVE EXAMPLE 7

A. Compounds (93A & B)

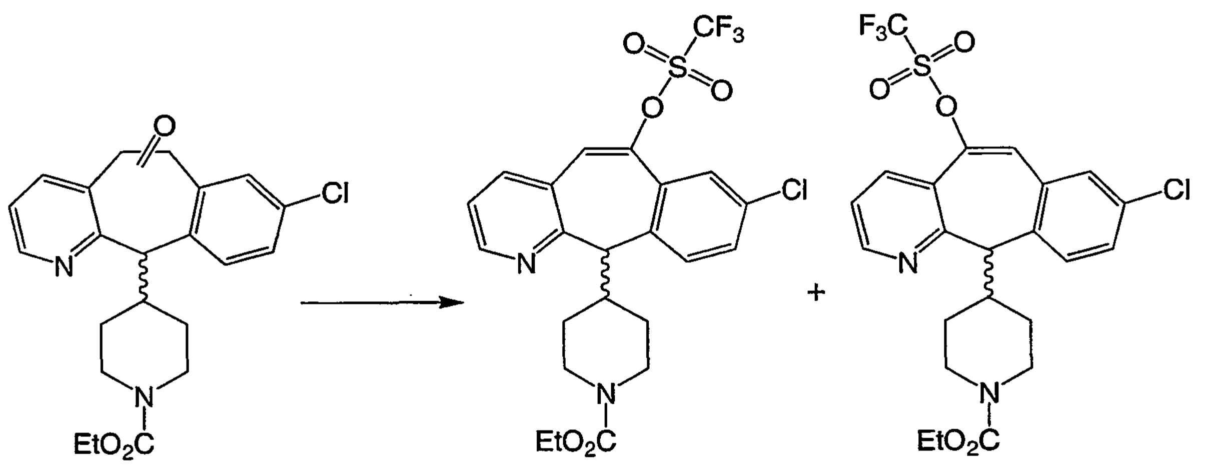

9 & 10 93A 93B

A mixture of piperizinyl compounds (9) and (10) from PREPARATIVE EXAMPLE 1, STEP F in THF at -78°C was reacted with LDA (1.1 eq.) and stirred for 1.5 h. The mixture was warmed to -20°C and then N-phenyl trifluoromethane sulfonimide (1.1 eq.) was added. Stirred over night at room temperature then extracted mixture with EtOAc and washed with $H_2O$. Dried over $Na_2SO_4$ and concentrated. Purification and separation by flash silica gel column chromatography afforded pure Compounds (93A & 93B).

B. Preparation of compound (94).

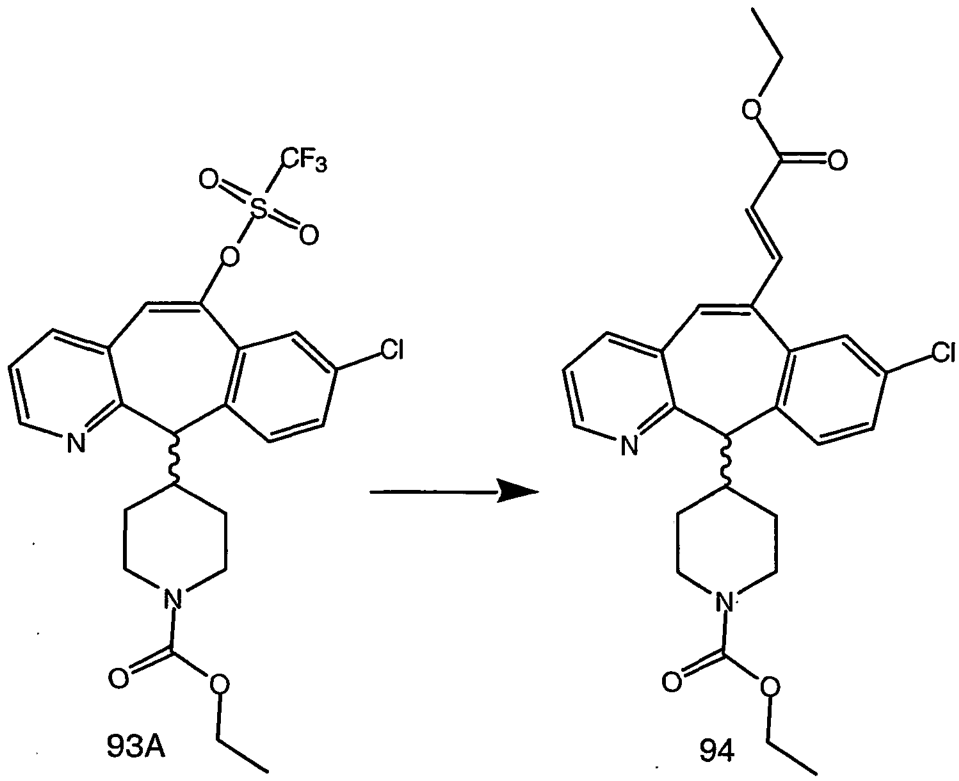

93A 94

Compound (93A) from above was dissolved in DMF. Successively added, $Et_3N$ (29 eq.), Ethyl acrylate (5.4 eq.), $K_2CO_3$ (5 eq.), $Bu_4NBr$ (2 eq.) and Palladuim (II) acetate (0.13 eq.). The mixture stirred and heated to 100°C for 4 h. After cooling, the mixture was concentrated and the residue was taken up in $CH_2Cl_2$ and extracted with $CH_2Cl_2/H_2O$. The organic layer was dried over $Na_2SO_4$ then concentrated and the residue purfied by flash silica column chromatography to afford the title compound (94).

C. Preparation of compound (95).

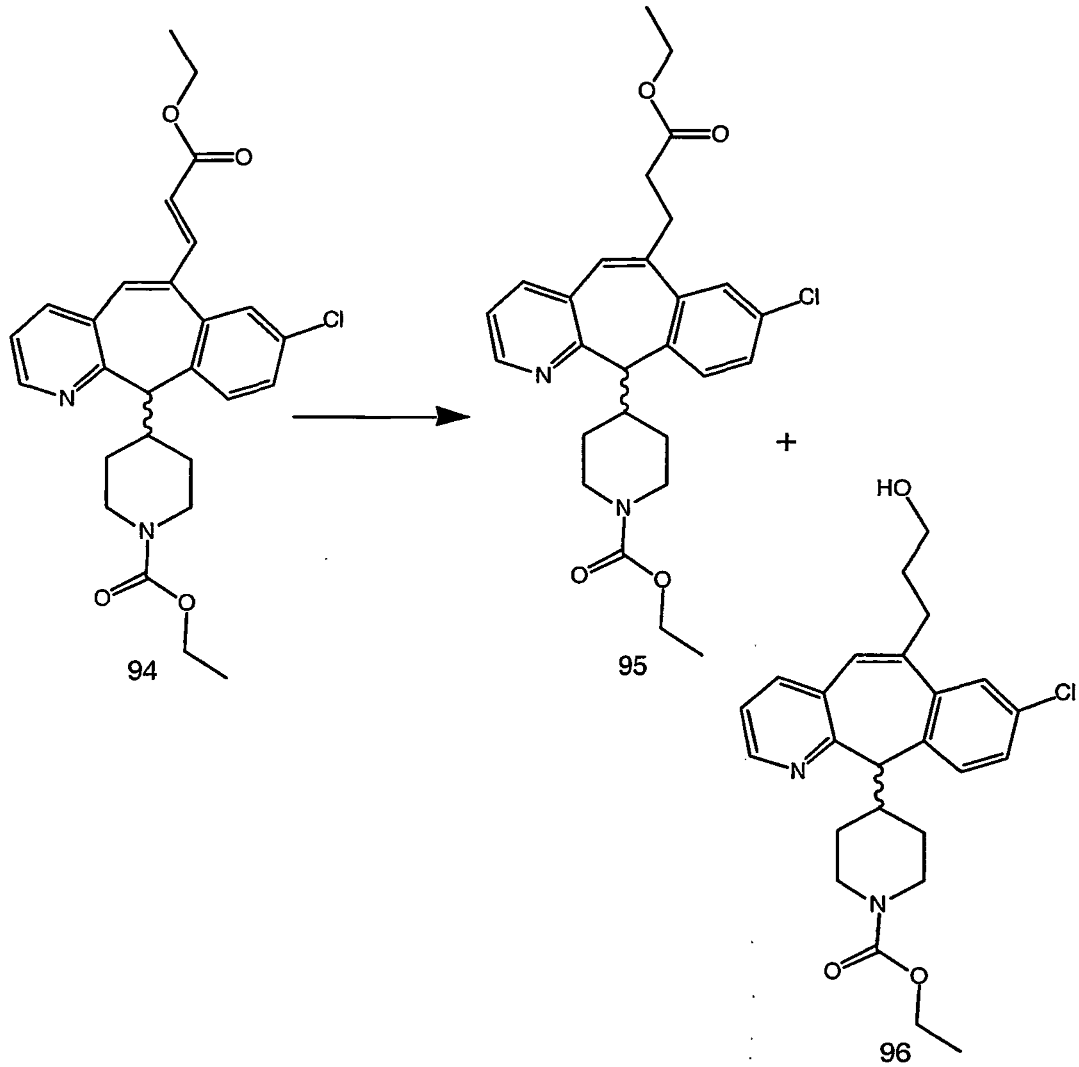

94

95

96

Compound (94) was dissolved in EtOH cooled in an ice bath and reacted with $NaBH_4$ (15 eq.) for 3 min. Then added CuCl (2 eq) and stirred for 2 h. at room temperature. The mixture was filtered, concentrated and extracted with $CH_2Cl_2$. Washed with water then brine, dried over $Na_2SO_4$ and concentrated to a mixture of the title compound (95) and the hydroxy compound (96).

D. Preparation of compound (96).

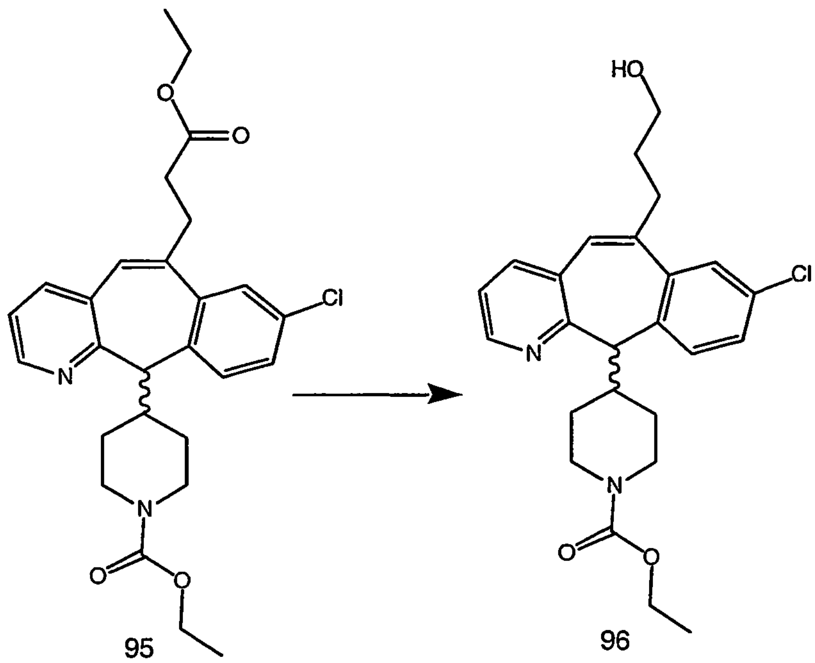

95 96

Compound (95), was then further reacted with $LiBH_4$(3 eq.) in THF at reflux temperature for 4 h. EtOAc was added and the mixture was washed with $Na_2CO_3$ then dried over $Na_2SO_4$ and concentrated to afford the title compound (96).

E. Preparation of compound (97).

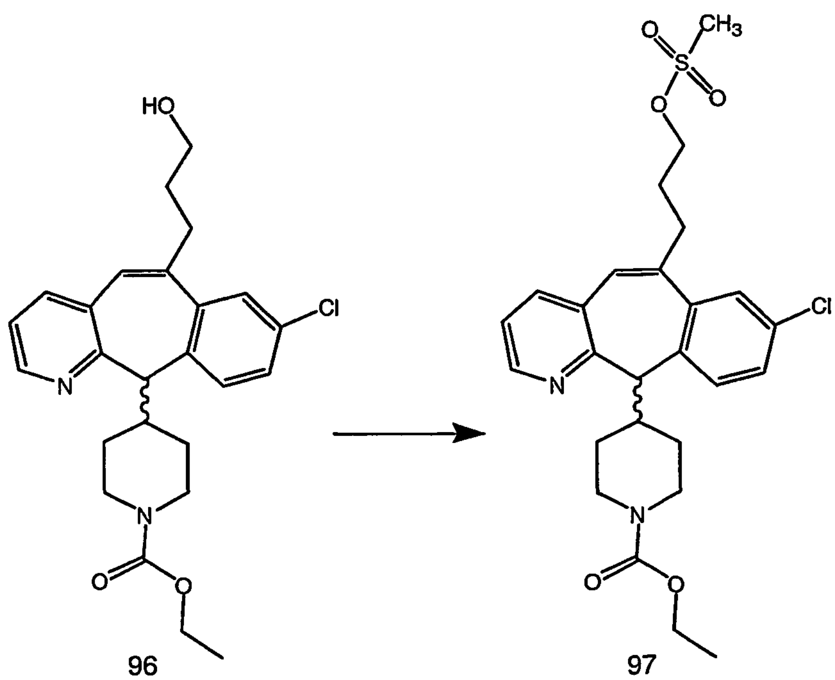

96 97

Dissolved compound (96) in $CH_2Cl_2$, added $Et_3N$ (3 eq.) followed by methane sulfonylchloride (1.5 eq.). The mixture stirred at room temperature over night then diluted with $CH_2Cl_2$ and washed with $Na_2CO_3$. Dried over $NaSO_4$

and concentrated to afford the title compound (97).

F. Compounds (98) and (99).

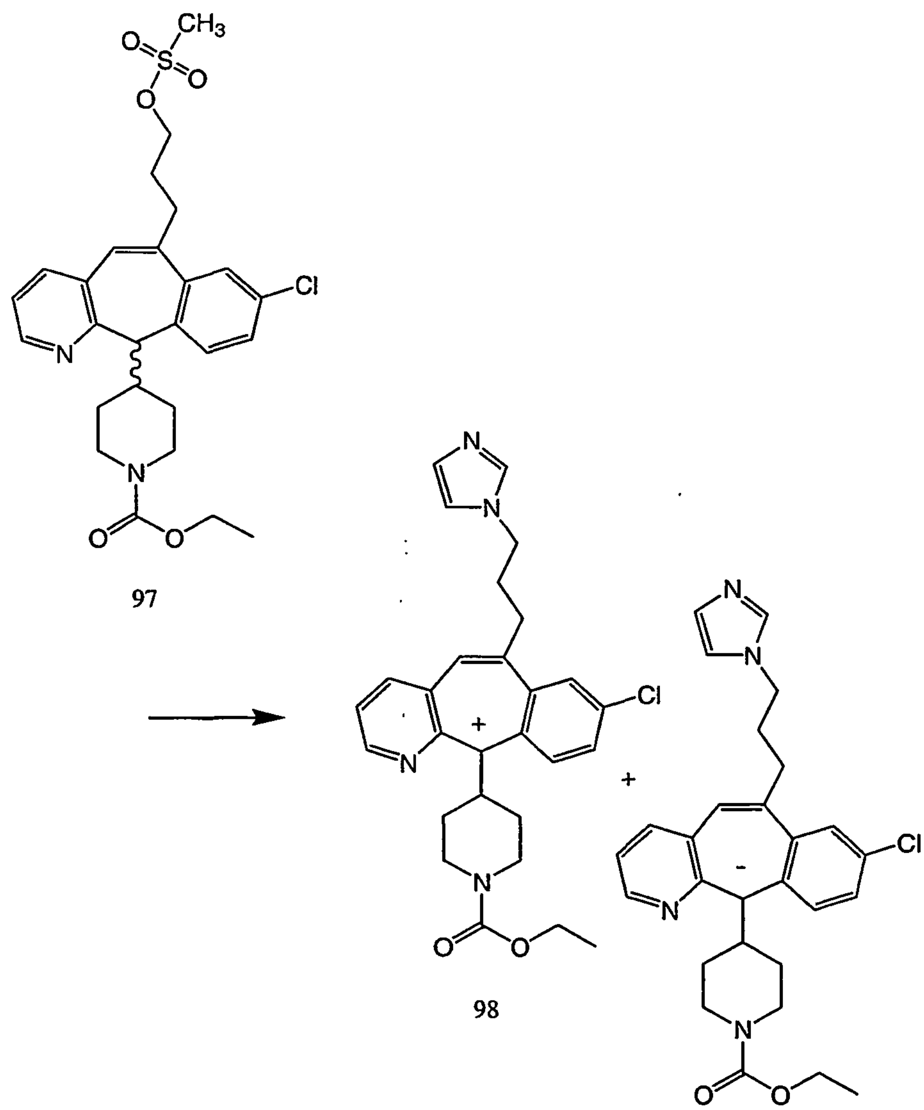

97

98

+

99

To a solution of sodium imidazole (Aldrich) in DMF was added, NaH (2 eq.). Stirred for 15 min. then added compound (97) (from above) (1 eq.) and stirred over night at room temperature. The reaction mixture was concentrated and then extracted with ethyl acetate. Washed with $Na_2CO_3$, dried over $NaSO_4$, filtered then concentrated. Crude product was purified by flash silica column chromatography. Further seperation of pure (+) enantiomers and pure (-) enantiomers was accomplished on a chiracel AD column affording the title compounds (98) and (99).

G. Compounds (100) and (101).

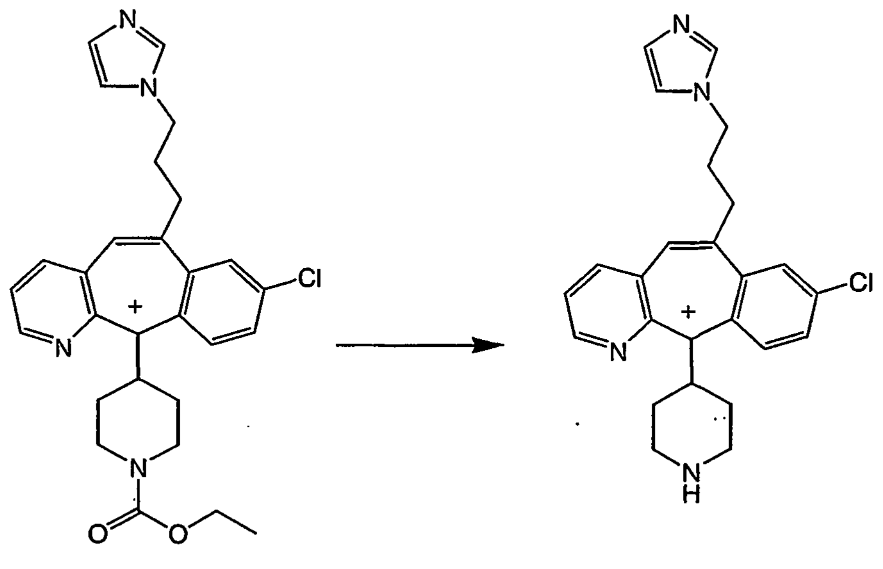

98

100

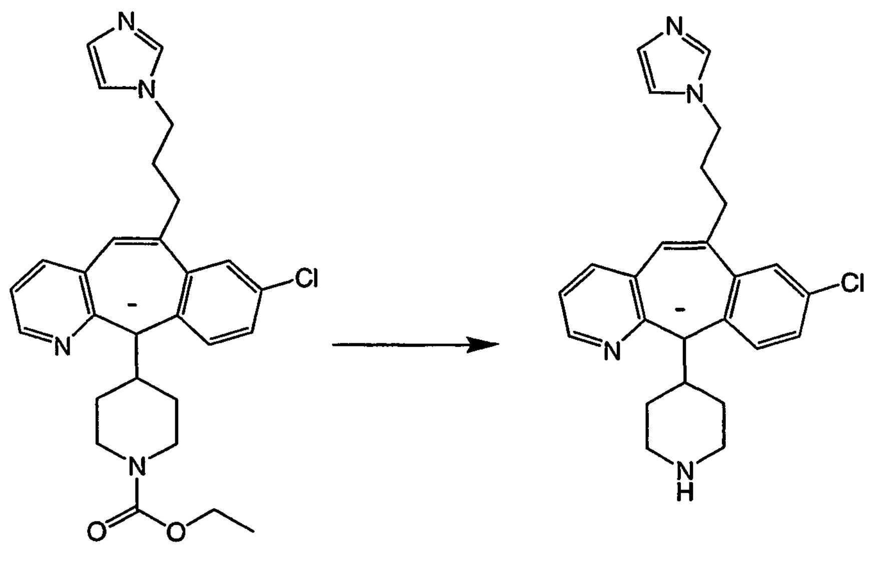

99

101

Compounds (98) and (99) were individually hydrolyzed to their free amines by refluxing in conc. HCl for 5 h. The reaction mixtures were seperately poured into ice and basified with $NH_4OH$. The solutions were then extracted with $CH_2Cl_2$, dried over $Na_2SO_4$, filtered and concentrated to afford the title compounds (100) and (101).

PREPARATIVE EXAMPLE 8

Preparation of Compounds (102) AND (103).

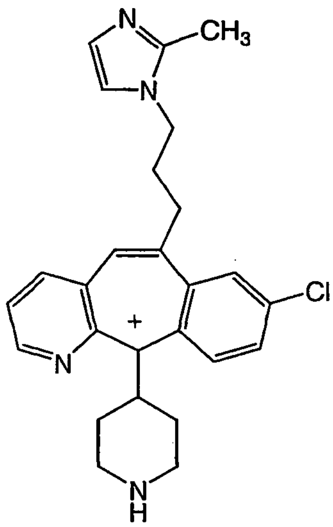

102

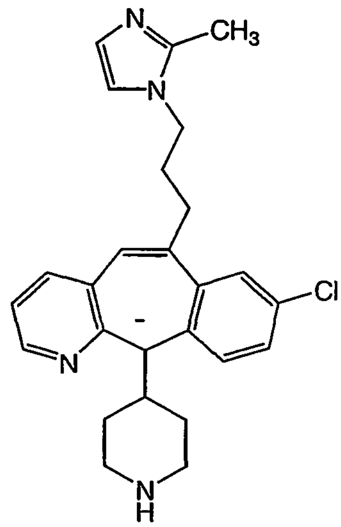

103

In a similar manner as described in Preparative Example 7, Steps A-G, substituting 2-methyl imidazole for sodium imidazole, in Step F, the title compounds (102) and (103) were prepared.

PREPARATIVE EXAMPLE 9

A. Compound (104).

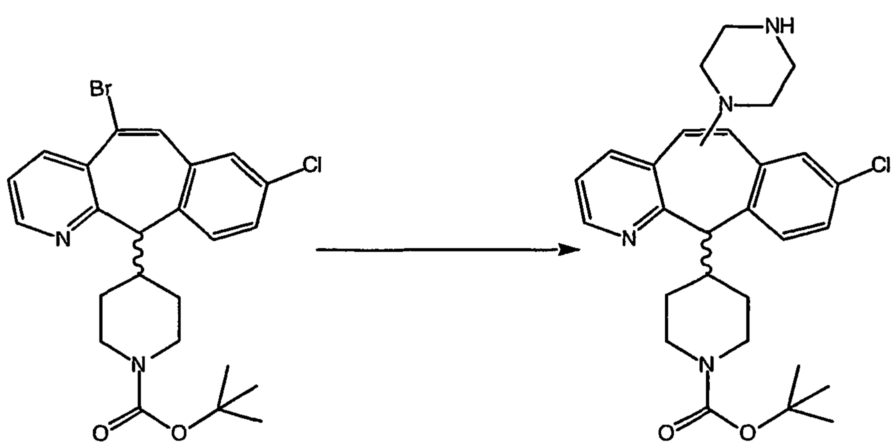

23

104

Compound (23) from Preparative Example 4 was reacted with piperazine in the same manner as described in Preparative Example 1, Step E, affording the title compound (104).

B. Preparation of compound (105).

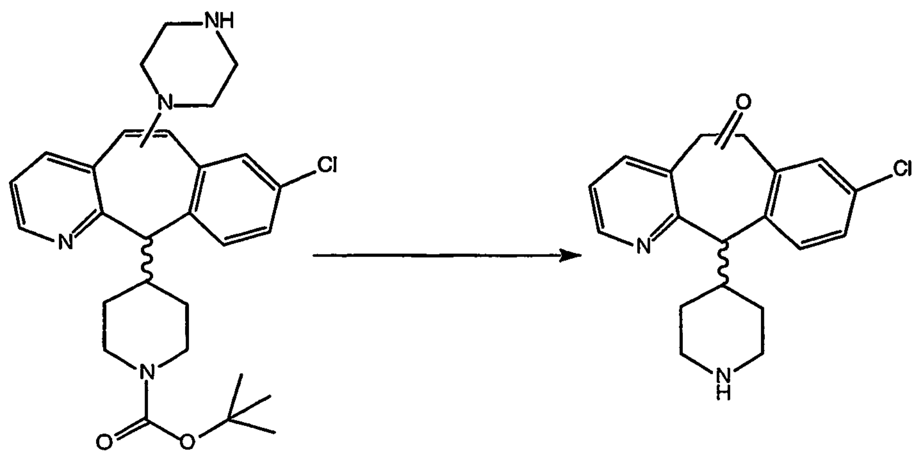

104 105

Compound (104) from above was hydrolyzed with 6N HCl over night at reflux temperature. The cooled reaction mixture was basified with 50% w/w NaOH and then extracted with 80% THF-EtOAc. The organic layer was dried over MgSO4, filtered and concentrated to dryness, affording the title compound (105).

C. Preparation of Compounds (106) and (107).

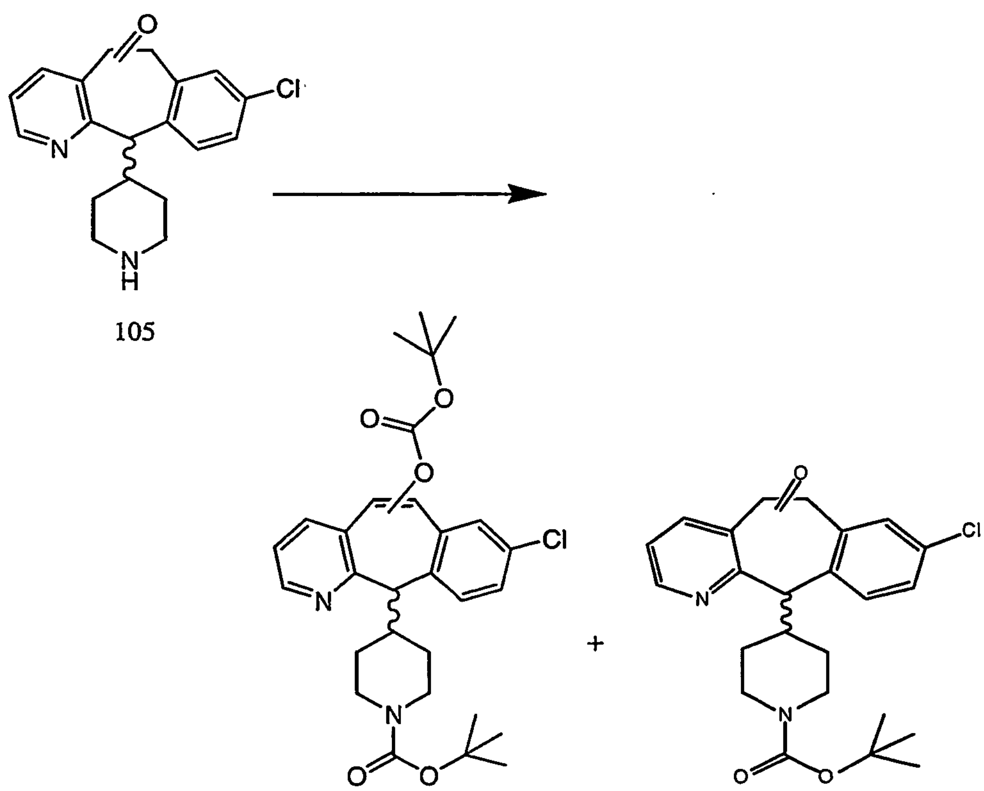

105

106 107

Compound (105) was dissolved in 50:1 MeOH:$H_2O$ then added di-tert-butyl dicarbonate (2 eq.). Adjusted pH to 9 and stirred for 4 h at room temperature. The reaction mixture was concentrated and extracted with $CH_2Cl_2$. The organic layer was washed with $Na_2CO_3$, dried, filtered and concentrated to dryness affording a mixture of title compounds

(106) and (107).

D. Preparation of compound (107).

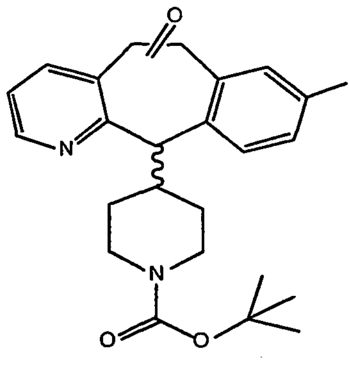

107

To the mixture of compounds (106) and (107) from Step C above, in 80% MeOH/$H_2O$ at room temperature was added, cesium carbonate (2 eq.). The reaction stirred overnight. The mixture was then concentrated, extracted with $CH_2Cl_2$, washed with $H_2O$, dried over $MgSO_4$, filtered and concentrated to dryness affording the title compound (107).

E. Preparation of Compounds (108A & B).

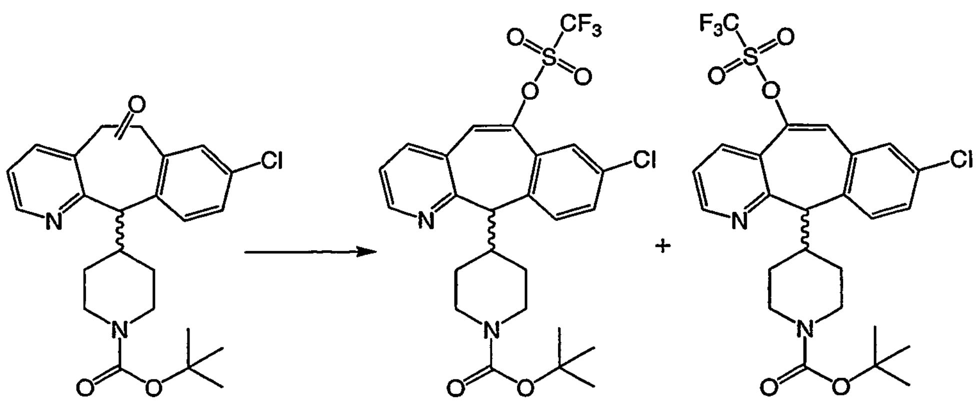

107 108A 108B

Compound (107) was reacted with N-phenyl trifluoromethane sulfonimide in a similar manner as described in Preparative Example 7, Step A, affording the title compound (108A & 108B).

F. Preparation of compound (109).

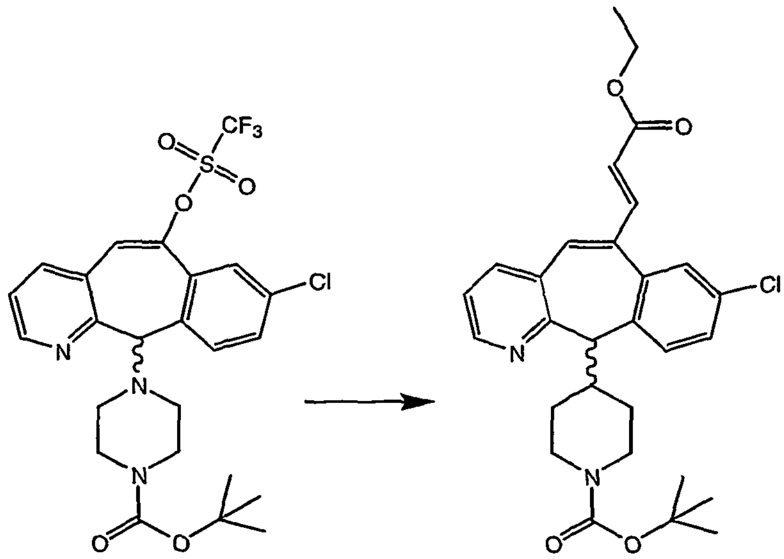

108A 109

Compound (108A) was reacted with ethyl acrylate in a similar manner as described in Preparative Example 7, Step B affording the title compound (109).

G. Preparation of compound (110).

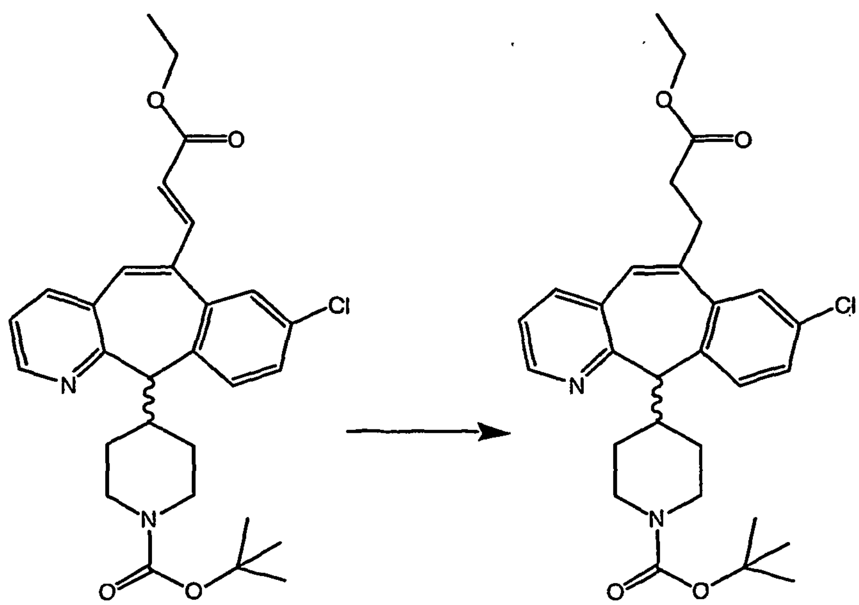

109 110

Compound (109) was reacted with $NaBH_4$ and CuCl in a similar manner as described in Preparative Example 7, Step C affording the title compound (110).

H. Preparation of Compound (111).

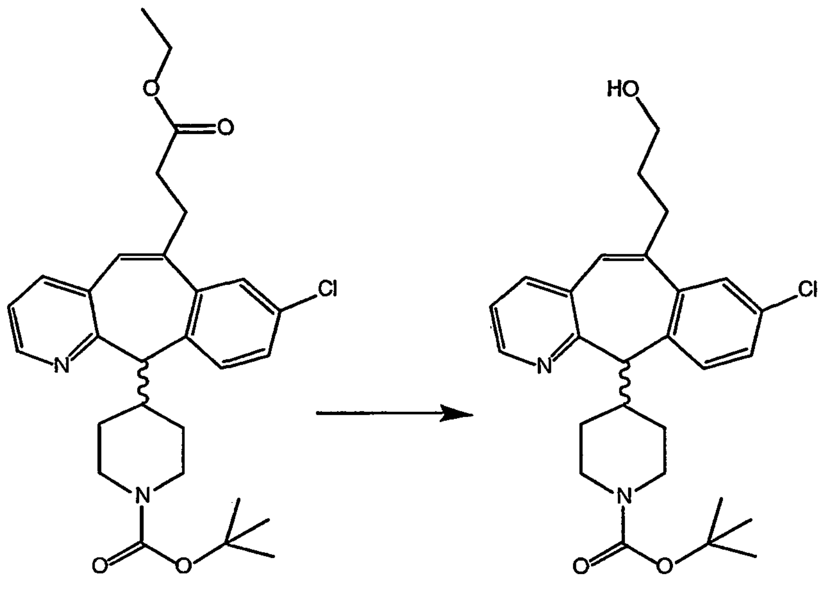

110 111

Dissolved compound (110) in THF and then added 1 M $LiAlH_4$/THF (1 eq.) and stirred for 1.5 h at room temperature. To the mixture was added $H_2O$ and 15% NaOH then extracted with EtOAc. The reaction was washed with brine, dried over $MgSO_4$, filtered and concentrated. Purification by flash silica column chromatography eluting with 20% EtOAc/$CH_2Cl_2$ afforded the hydroxy title compound (111).

I. Preparation of compound (112).

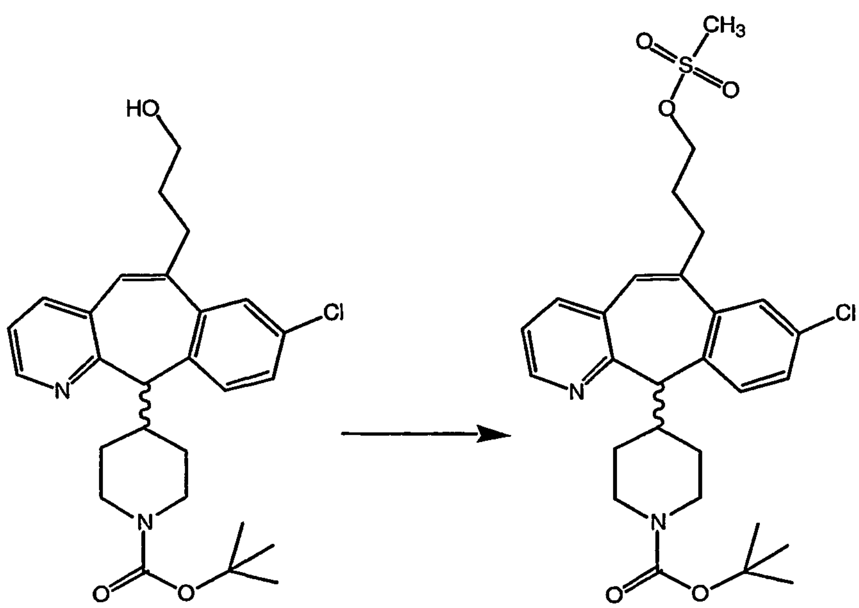

111 112

Compound (111) was reacted with methane sulfonyl chloride in a similar manner as described in Preparative Example 7, Step E affording the title compound (112).

J. Preparation of compounds (113), (114), (115) and (116).

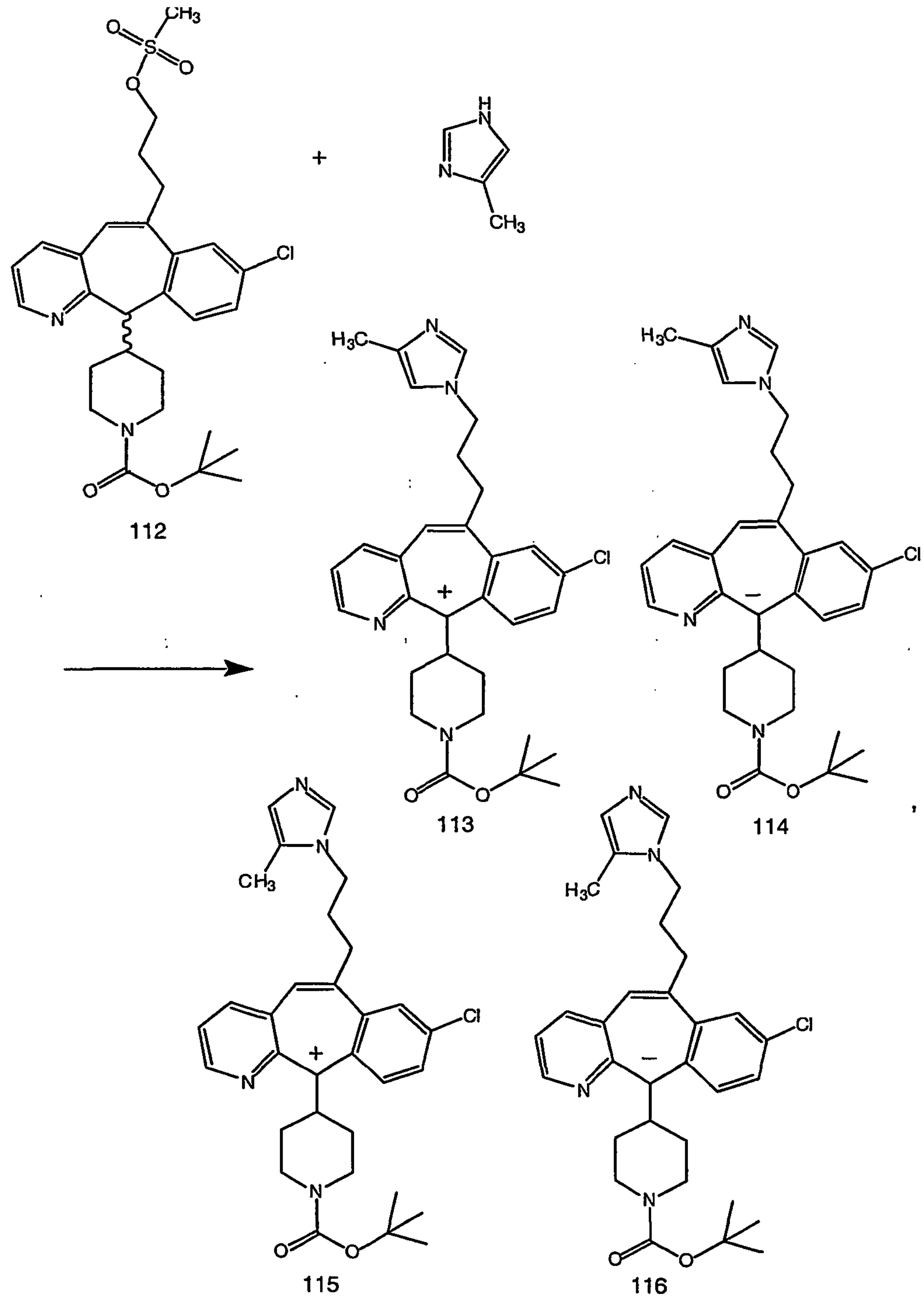

112

113

114,

115

116

Compound (112) was reacted in a similar manner as Preparative Example 7, Step F substituting 4-methylimidazole for sodium imidazole. A mixture of (+,-)4 and (+,-)5-methyl imidazoles resulted. The mixture was treated in the same manner as described in Example 11 affording pure stereoisomers (113), (114), (115) and (116).

K. Preparation of Compounds (117) and (118).

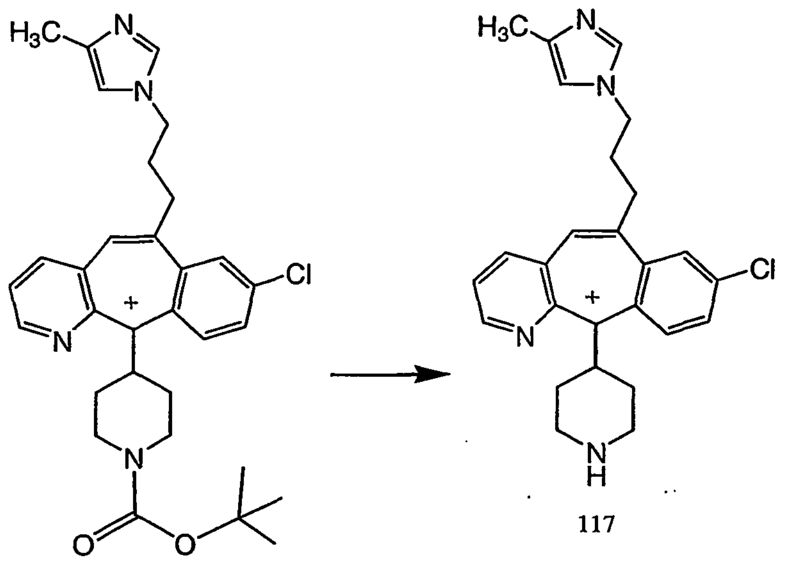

113 117

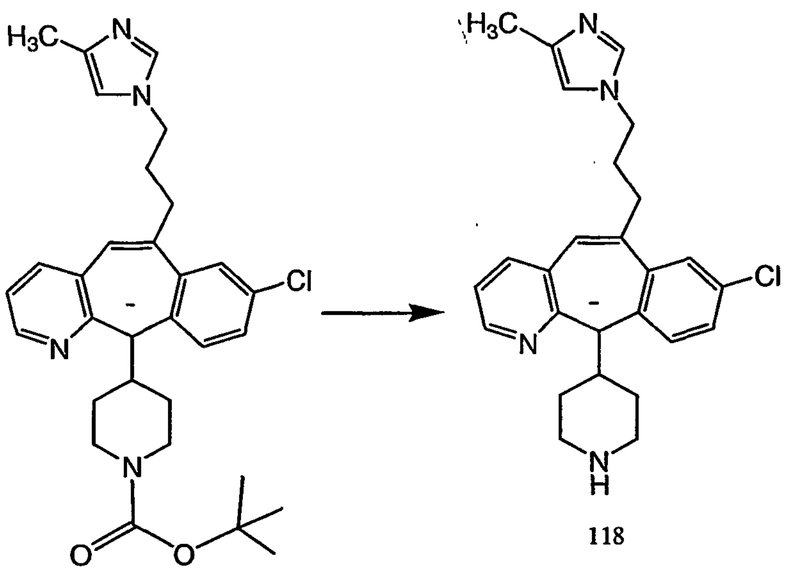

118

114

Compounds (113) and (114) were hydrolyzed to their free amines by stirring in HCl/Dioxane for 4 h. The mixtures were then concentrated to dryness affording the title compounds (117) and (118).

PREPARATIVE EXAMPLE 10

Compounds (119) AND (120).

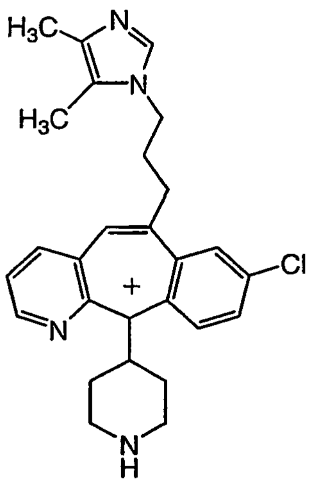

119

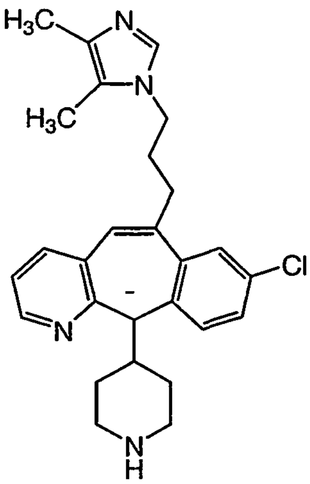

120

In a similar manner as described in Preparative Example 9, Steps A-K, substituting 4,5-dimethyl imidazole in Step J, the title compounds (119) and (120) were prepared.

<u>EXAMPLE 39-45</u>

Reacting compounds (100) or (101) from Preparative Example 7, in the same manner as described in Example 13, substituting the appropriate isocyanate or chloroformate, the following compounds were prepared:

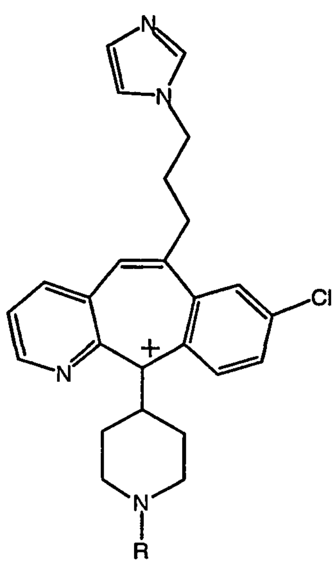

OR

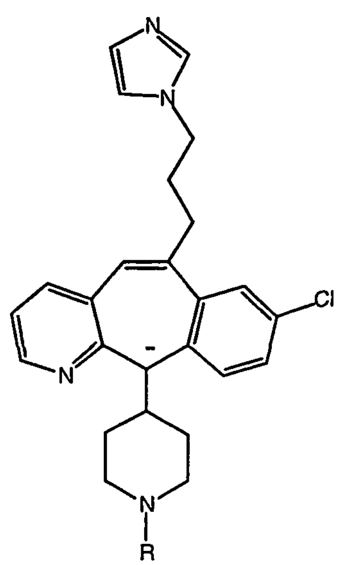

| Ex | R= | Compound #: |
|---|---|---|
| 39 | 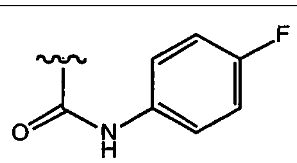 | (121) AND (122) |

(continued)

| Ex | R= | Compound #: |
|---|---|---|
| 40 | 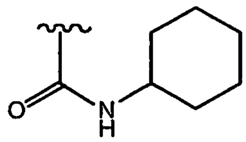 | (123) and (124) |
| 41 | 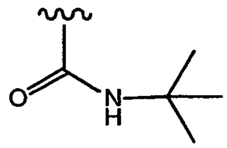 | (125) AND (126). |
| 42 | 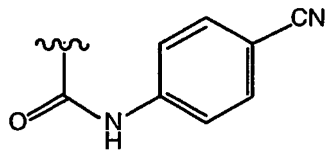 | (127) AND (128). |
| 43 | 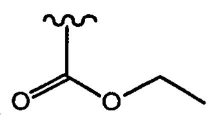 | (129) AND (130). |
| 44 | 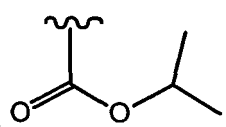 | (131) AND (132). |
| 45 | 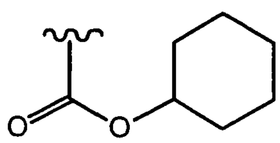 | (133) AND (134). |

EXAMPLE 46-51

Reacting compounds (102) or (103) from Preparative Example 8, in the same manner as described in Example 13, substituting the appropriate isocyanate or chloroformate, the following compounds were prepared:

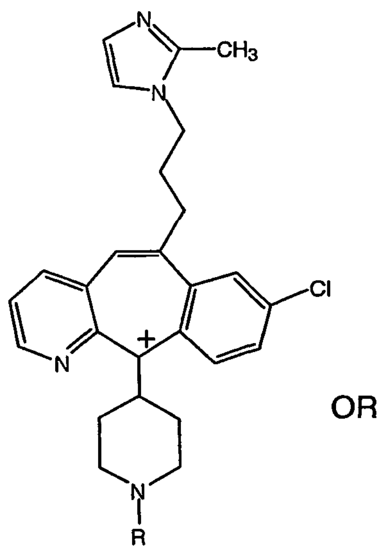

OR

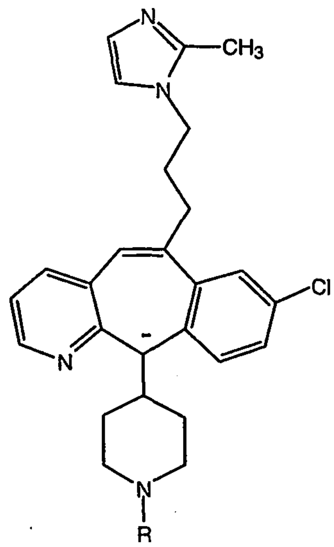

| Ex | R= | Compound #: |
|---|---|---|
| 46 | 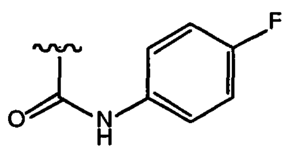 | (135) AND (136). |
| 47 | 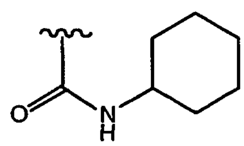 | (137) AND (138). |
| 48 | 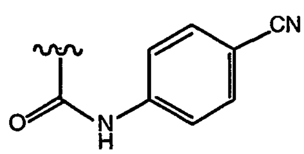 | (139) AND (140). |
| 49 | 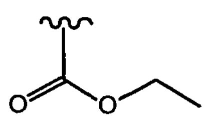 | (141) AND (142) |
| 50 | 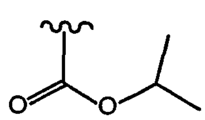 | (143) AND (144). |
| 51 | 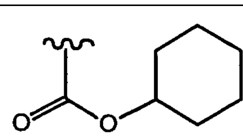 | (145) AND (146). |

<u>EXAMPLE 52-59</u>

Reacting compounds (117) or (118) from Preparative Example 9, in the same manner as described in Example 13, substituting the appropriate isocyanate, chloroformate or sulfonyl chloride, the following compounds were prepared:

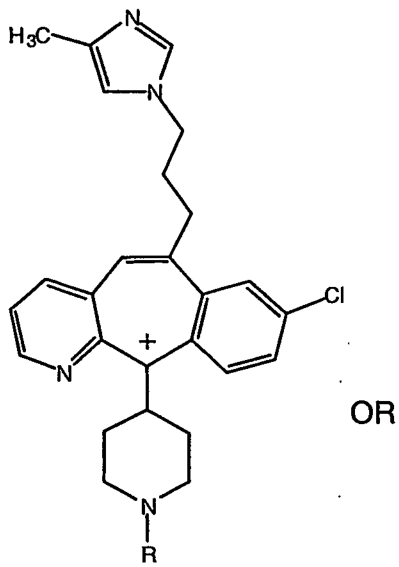

OR

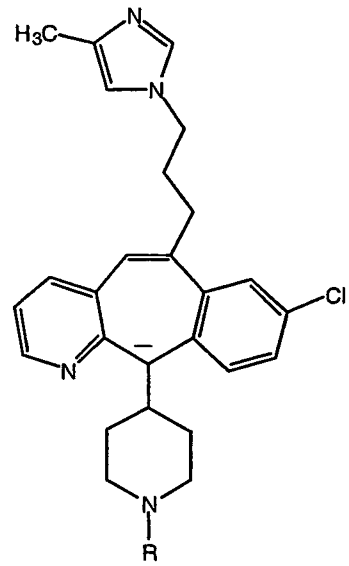

| Ex | R= | Compound #: |
|---|---|---|
| 52 | 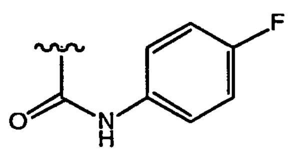 | (147) AND (148) |
| 53 | 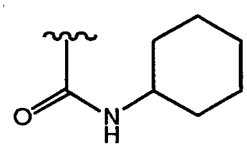 | (149) and (150) |
| 54 | 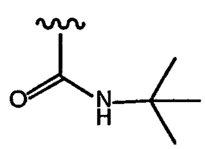 | (151) AND (152). |
| 55 | 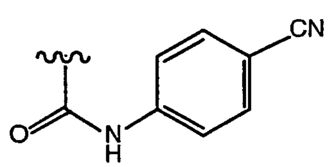 | (153) AND (154). |
| 56 | 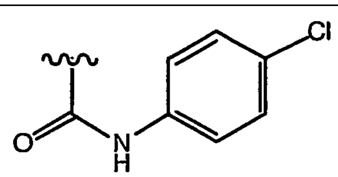 | (155) AND (156) |
| 57 | 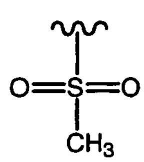 | (157) AND (158). |

(continued)

| Ex | R= | Compound #: |
|---|---|---|
| 58 | 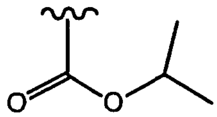 | (159) AND (160). |
| 59 | 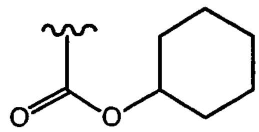 | (161) AND (162). |

EXAMPLE 60-69

Reacting compounds (119) or (120) from Preparative Example 10, in the same manner as described in Example 13, substituting the appropriate isocyanate, chloroformate or sulfonyl chloride, the following compounds were prepared:

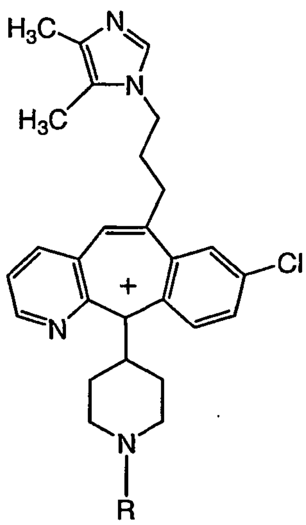

OR

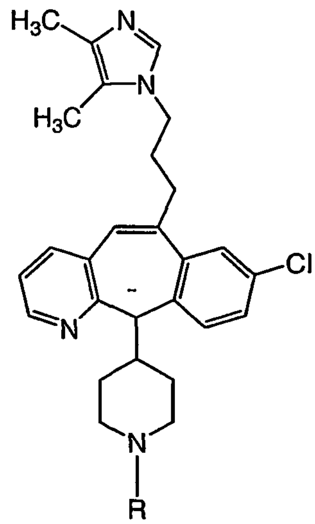

| Ex | R= | Compound #: |
|---|---|---|
| 60 | 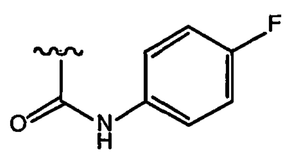 | (163) AND (164) |
| 61 | 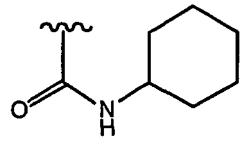 | (165) and (166) |
| 62 | 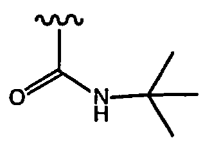 | (167) AND (168). |

(continued)

| Ex | R= | Compound #: |
|---|---|---|
| 63 | 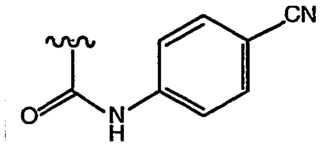 | (169) AND (170). |
| 64 | 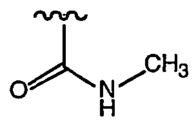 | (171) |
| 65 | 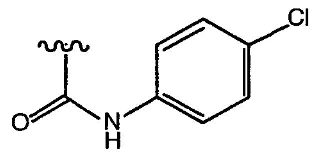 | (172) AND (173) |
| 66 | 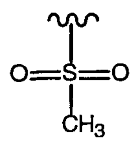 | (174) AND (175). |
| 67 | 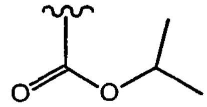 | (176) AND (177). |
| 68 | 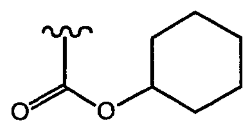 | (178) AND (179). |
| 69 | 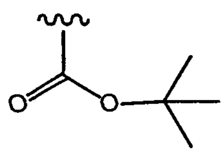 | (180) AND (181). |

PREPARATIVE EXAMPLE 11

A. PREPARATION OF COMPOUND (182).

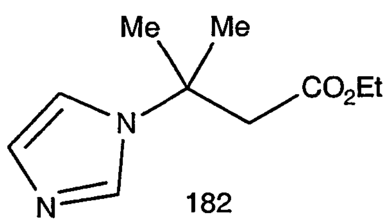

182

Ethyl 2,2-dimethyl acrylate (50.0 g, 2.0 eq.) was stirred with imidazole (13.28 g, 200 mmol) at 90° for 48 hours. The resulting solution was cooled, diluted with 300 mL $H_2O$-$CH_2Cl_2$ (1:1) and separated. The aqueous layer was extracted

with $CH_2Cl_2$ (2 x 75 mL) and the combined organic layer was dried over $Na_2SO_4$ and concentrated in vacuo. The crude mixture was purified by flash chromatography using a 10% MeOH in $CH_2Cl_2$ solution as eluent to give pure product as a clear oil. CIMS: $MH^+$= 197.

B. PREPARATION OF COMPOUND (183).

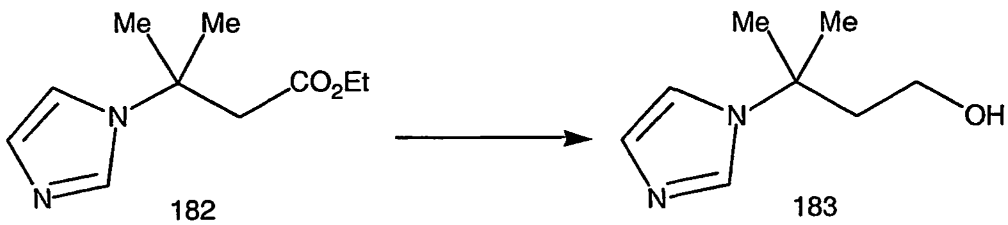

182 183

A solution of the title compound from Preparative Example 11, Step A, (10.0 g, 50.96 mmol) was treated with $LiAlH_4$ (51 mL, 1M solution in ether, 1.0 eq.). The reaction mixture was stirred one hour before quenching by the dropwise additon of saturated $Na_2SO_4$ (-3.0 mL). The resulting slurry was dried with $Na_2SO_4$ (solid), diluted with EtOAc (100 mL) and filtered through a plug of Celite. The filtrate was concentrated to give crude product which was used without further purification. CIMS: $MH^+$=155.

C. PREPARATION OF COMPOUND (184).

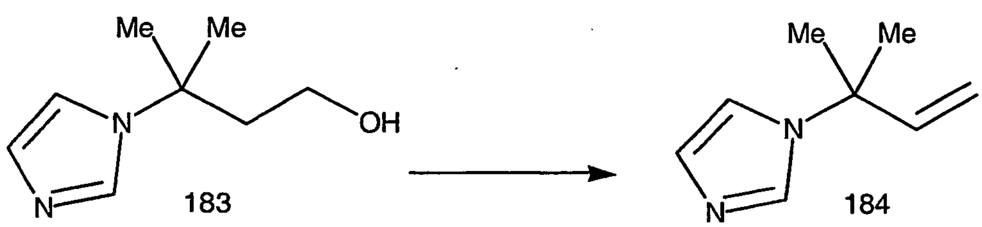

183 184

Iodine (3.83 g, 1.2 eq.) was added to a solution of $Ph_3P$ (3.95 g, 1.2 eq.) and imidazole (1.02 g, 1.2 eq.) in $CH_2Cl_2$ (30 mL) portionwise over 15 minutes followed by a solution of the title compound from Preparative Example 11, Step B, (3.83 g, 12.56 mmol) in $CH_2Cl_2$ (10 mL). The resulting solution was stirred one hour before concentrating in vacuo. The residue was dissolved in THF (100 mL), treated with KOt-Bu (4.51 g, 3.2 eq.) and stirred at room temperature over night. The reaction mixture was diluted with water (100 mL) and $CH_2Cl_2$ (100 mL), separated, and the aqueous layer extracted with $CH_2Cl_2$ (2 X 50 mL). The combined organics were dried over $Na_2SO_4$, filtered , and concentrated under reduced pressure. The product was purified by flash chromatography using neat EtOAc then 5% MeOH in EtOAc as eluent to give a pale yellow oil (184).
CIMS: $MH^+$= 137.

D. PREPARATION OF COMPOUND (185).

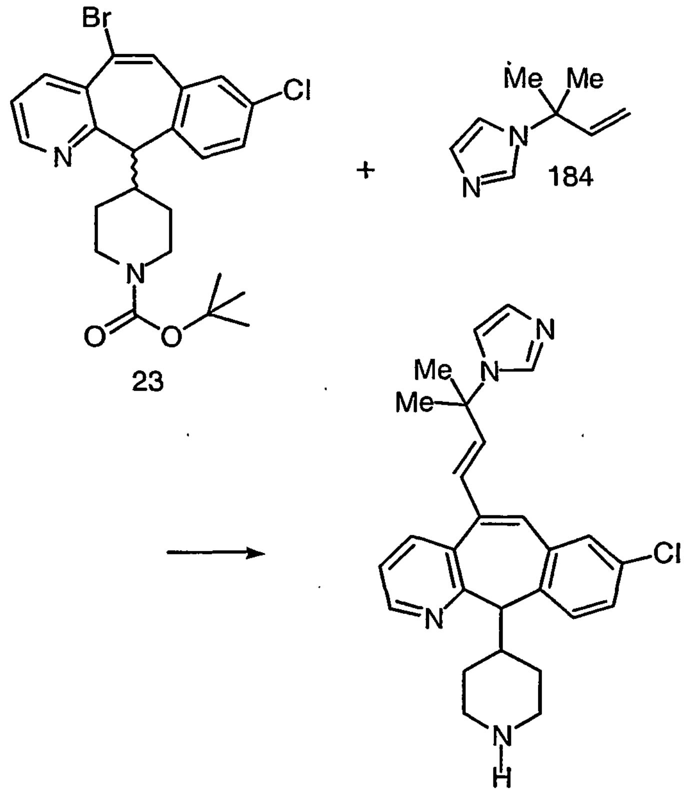

23 + 184 → 185

$Pd(OAc)_2$ (0.023 g, 10 mol%) was added to a solution of the title compound (184) from Preparative Example 11, Step C, (0.30 g, 2.0 eq.), compound (23)(0.50 g, 1.02 mmol), $Bu_4NBr$ (0.66 g, 2.0 eq.), TEA (2.84 mL, 20.eq.) and $K_2CO_3$ (0.70 g, 5.0 eq) in DMF (10 mL). The resulting solution was heated to 100˚C for 48 hours, cooled to room temperature, and concentrated under reduced pressure. The residue was diluted with water (50 mL) and $CH_2Cl_2$ (50 mL), separated, and the aqueous layer extracted with $CH_2Cl_2$ (2 X 25 mL). The combined organic layer was dried over $Na_2SO_4$, filtered, and concentrated *in vacuo.* The crude product was purified by flash column chromatography using an 8% MeOH in $CH_2Cl_2$ solution as eluent to yield a 4 : 1 mixture of the compound (184) and coupled product (185). This mixture (0.27 g) was stirred in $CH_2Cl_2$ : TFA (7.0 mL, 5 : 2) for 1.5 hours. The crude product was concentrated under reduced pressure, neutralized with NaOH (1N), and extracted with $CH_2Cl_2$ (3 X 20 mL). The combined organics were dried over $Na_2SO_4$, filtered, and concentrated *in vacuo.* The crude residue was purified by flash chromatography using a 15% (10% $NH_4OH$ in MeOH) solution in $CH_2Cl_2$ as eluent to give the title compound (185) as a tan solid. LCMS: $MH^+$= 445.

EXAMPLE 70.

Preparation of Compound (186).

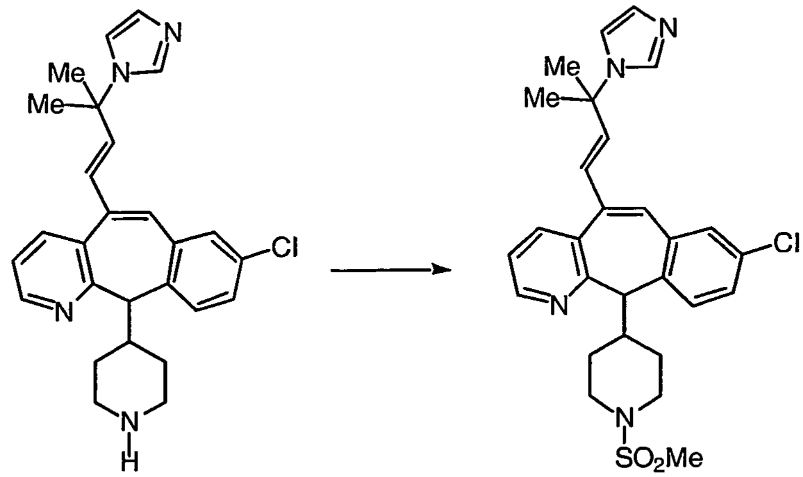

185 186

Methanesulfonyl chloride (0.005 mL, 1.3 eq) was added to a solution of Compound (185) from Preparative Example 11, Step D (0.02 g, 0.045 mmol) and TEA (0.010 mL, 1.5 eq.) in $CH_2Cl_2$ (1 mL). The resulting solution was stirred 12 hours at room temperature and diluted with saturated $NaHCO_3$ (5 mL), separated, and the aqueous layer extracted with $CH_2Cl_2$ (3 X 10 mL). The combined organic layer was dried over $Na_2SO_4$ and concentrated *in vacuo.* The crude product was purified by flash chromatography using an 8% (10% $NH_4OH$ in MeOH) solution in $CH_2Cl_2$ as eluent to give the title compound (186) as a tan solid mp 124-129 ˚C; LCMS: $MH^+$= 523.

EXAMPLE 71

Preparation of Compound (187).

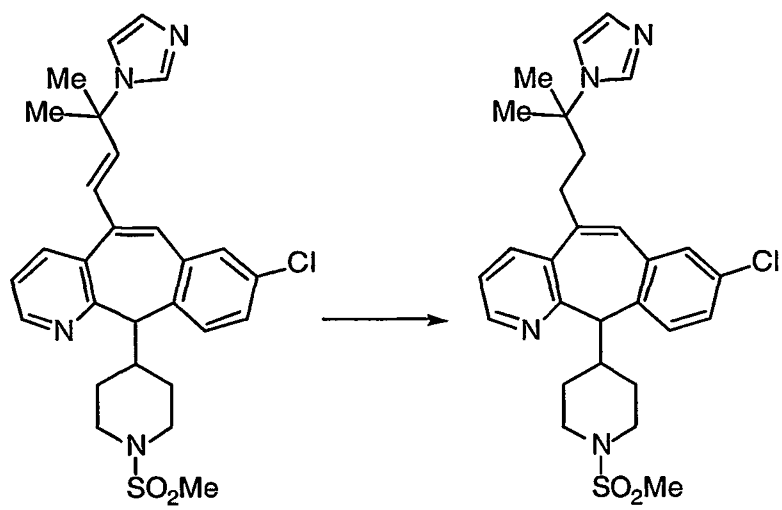

186 187

pTosNHNH$_2$ (0.085 g, 3 eq) was added to a solution of compound (186) from Example 70 (0.08 g, 0.0153 mmol) and DBU (0.11 mL, 5.0 eq.) in toluene (5 mL) and the resulting solution was heated to reflux. Subsequently, every 2 hours over 6 hours the solution was cooled and additional pTosNHNH$_2$ (3.0 eq) added and the solution heated to reflux. After heating at reflux 2 hours following the final addition the solution was cooled, diluted with $CH_2Cl_2$ (25 mL)

and washed with saturated $NaHCO_3$ (3 X 20 mL). The organic layer was dried over $Na_2SO_4$, filtered, and concentrated under reduced pressure. The crude reaction mixture was purified by flash column chromatography using a 5% (10% $NH_4OH$ in MeOH) solution in $CH_2Cl_2$ as eluent to give the title compound (187) as a tan solid. mp 112-116 ˚C; LCMS: $MH^+$= 525.

PREPARATIVE EXAMPLE 12

A. PREPARATION OF COMPOUND (188).

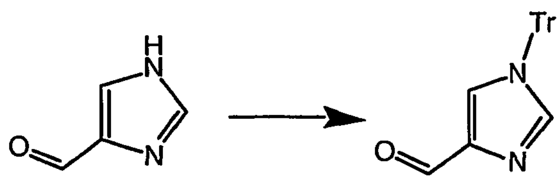

188

Literature compound 1H-imidazole-4-carbaldehyde was tritylated according to the literature procedure Kelley, et al.; J. Med. Chem 20(5), (1977), 721 affording the title compound (188).

B. PREPARATION OF COMPOUND (189).

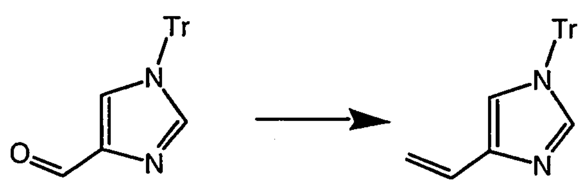

188 189

nBuLi (2.00 mL, 2.2 eq; 1.7M in hexanes) was added dropwise to $Ph_3PCH_3Br$ (1.4 g, 2.3 eq) in THF (10 mL). The resulting orange solution was stirred 30 minutes at room temperature before cooling to -78 ˚C and adding the trityl protected 1(3)H-imidazole-4-carbaldehyde (0.50 g, 1.48 mmol) in THF (7.0 mL). The resulting solution was warmed slowly to room temperature and stirred overnight. The reaction was quenched by the addition of water (20 mL) and extracted with $CH_2Cl_2$ (3 X 20 mL). The combined organics were dried over $Na_2SO_4$ and concentrated in vacuo. The crude product was purified by flash chromatography using a 45% hexanes in EtOAc solution as eluent to yield the title compound (189) as a white solid.

C. PREPARATION OF COMPOUND (190).

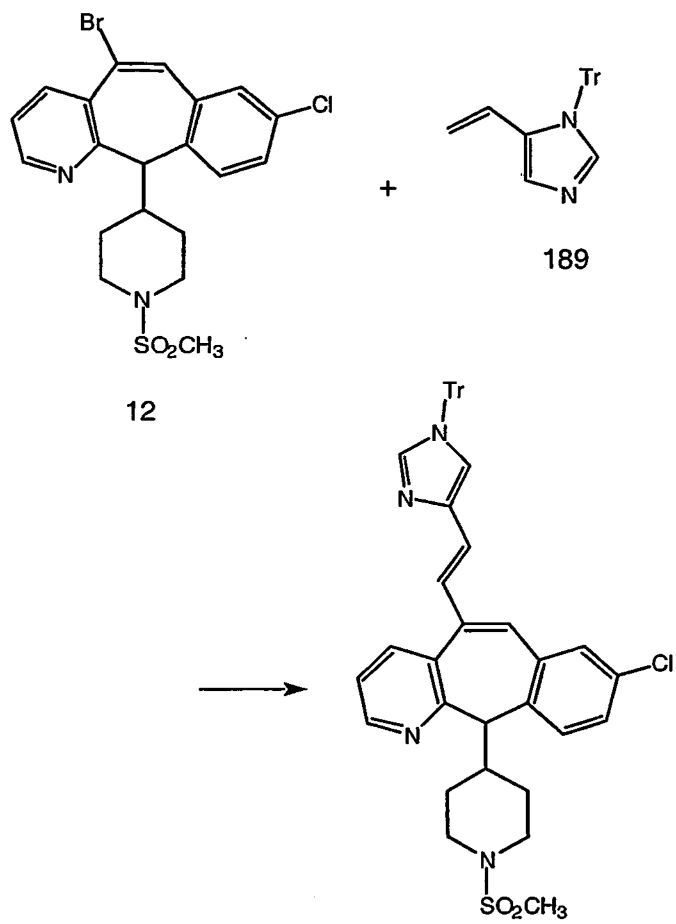

12

189

190

$Pd(OAc)_2$ (0.021 g, 0.10 eq.) was added to a solution of compound (12) from Preparative Example 2, Step B (0.44 g, 0.95 mmol), compound (189) from Preparative Example 12, Step B (0.32 g, 1.0 eq.), $Bu_4NBr$ (0.61 g, 2.0 eq.), and $K_2CO_3$ (0.66 g, 5.0 eq.) in DMF (8.0 mL). The resulting solution was heated to 100 °C over night, cooled, and concentrated under reduced pressure. The residue was diluted with water (50 mL) and $CH_2Cl_2$ (50 mL), serparated, and the aqueous layer extracted with $CH_2Cl_2$ (2 X 50 mL). The combined organics were dried over $Na_2SO_4$ and concentrated *in vacuo.* The crude product was purified by flash chromatography using 100% EtOAc as eluent. LCMS: 723 ($MH^+$).

EXAMPLE 72

Preparation of Compound (191).

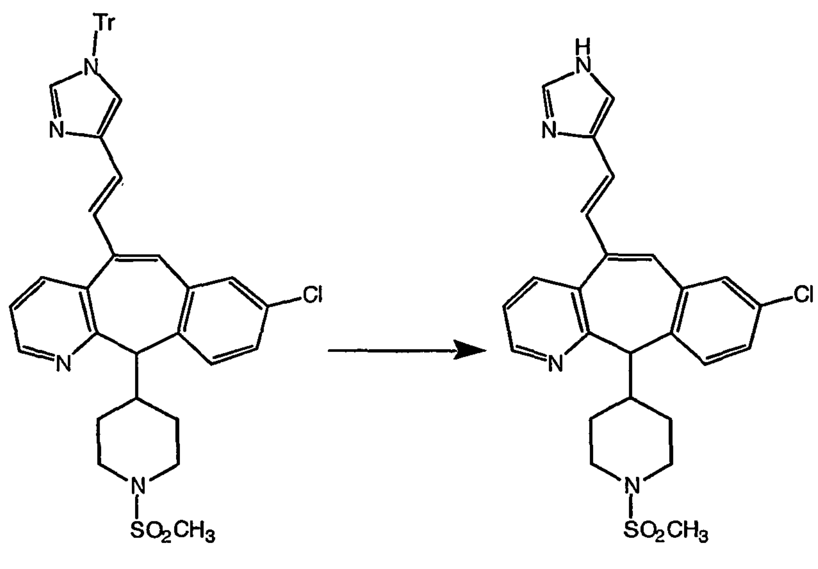

190 191

To a solution of the title compound from Preparative Example 12, Step C (1.43 g, 1.97 mmol) in water (70 mL) was added AcOH (70 mL). The resulting solution was heated at reflux two hours,cooled to room temperature and neutralized by the dropwise addition of 50% (w/w) NaOH. The solution was then extracted with $CH_2Cl_2$ (3 X 200 mL) and the combine organics were dried over $Na_2SO_4$ and concentrated under reduced pressure. The crude product was purified by flash chromatography using a 10% (10% $NH_4OH$ in MeOH) solution in $CH_2Cl_2$ as eluent. mp=190 ˚C (dec.); LCMS: $MH^+$= 483.

EXAMPLE 73

Separation of Compounds (192) AND (193).

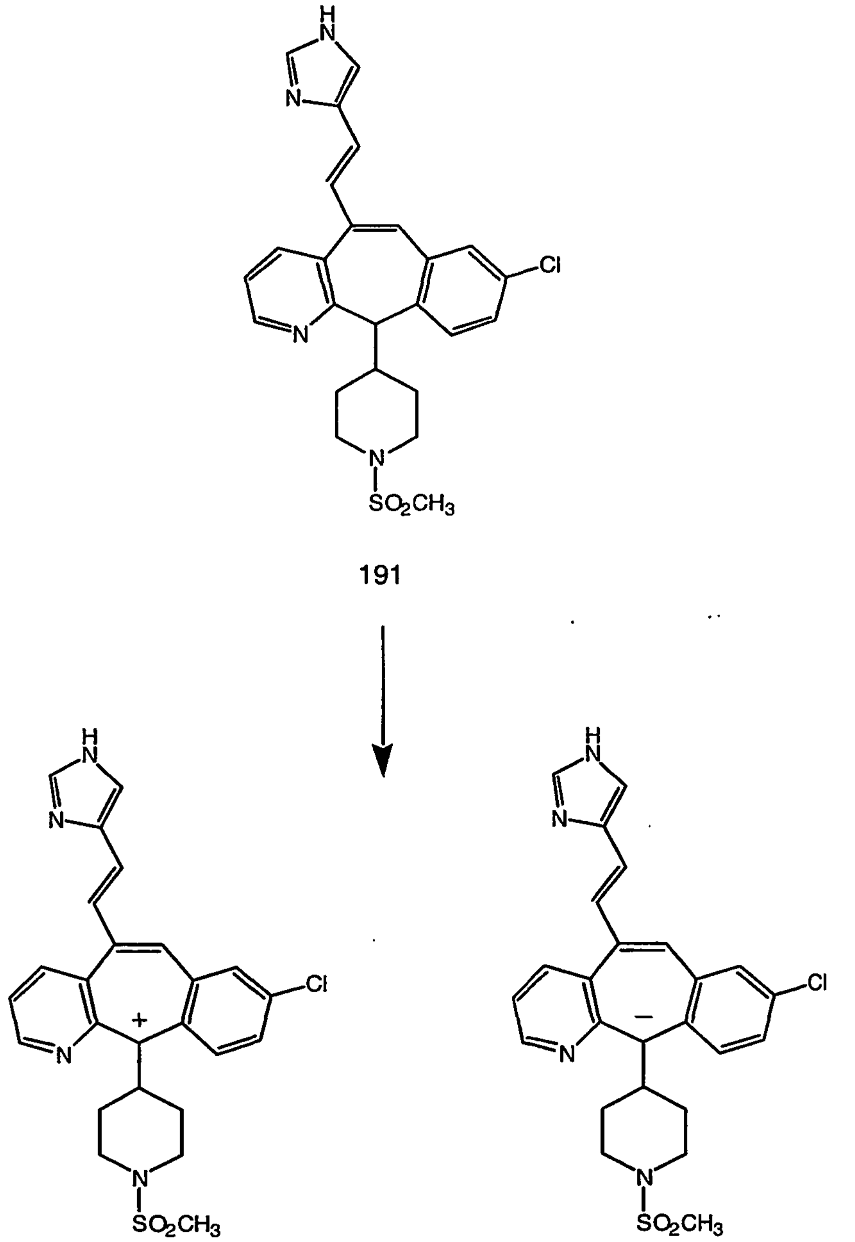

191

192

193

The title compound (191) from Example 72 was separated into individual (+)- and (-)- enantiomers by preparative HPLC using a ChiralPak AD column eluting with 70 : 30 hexanes : iPrOH containing 0.2% diethylamine as eluent.

Compound (192): FABMS: $MH^+$= 481; mp=109-112 °C; $[\alpha]^{20}_D$= +398° (2.0 mg in 2.0 mL MeOH).

Compound (193): FABMS: $MH^+$= 481; mp= 126-129 °C; $[\alpha]^{20}_D$= -367° (2.0 mg in 2.0 mL MeOH).

EXAMPLE 74

Preparation of Compound (194).

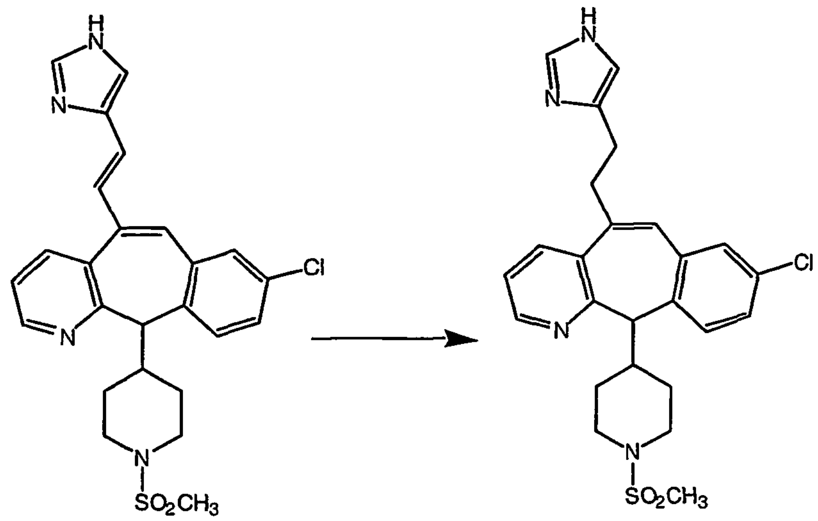

191

194

The title compound (191) from Example 72 was dissolved in toluene (50 mL) and DBU (0.26 mL, 5.0 eq.) and pTosNHNH$_2$ (0.33g, 3.3 eq.) were added. The resulting solution was heated to reflux 2.5 hours before cooling to room temperature and adding additional pTosNHNH$_2$ (0.33g, 3.3 eq.). The reaction mixture was heated at reflux for an additional 2 hours and cooling to room temperature. The resulting solution was diluted with saturated $NaHCO_3$ (100 mL) and extracted with $CH_2Cl_2$ (3 X 100 mL). The combined organics were washed with brine, dried over $Na_2SO_4$, filtered, and concentrated *in vacuo.* The crude product was purified by flash chromatography using a 5% (10% $NH_4OH$ in MeOH) solution in $CH_2Cl_2$ as eluent to give pure product (194). mp=158-162; LCMS: $MH^+$=483.

EXAMPLE 75

Separation of compounds (195) AND (196).

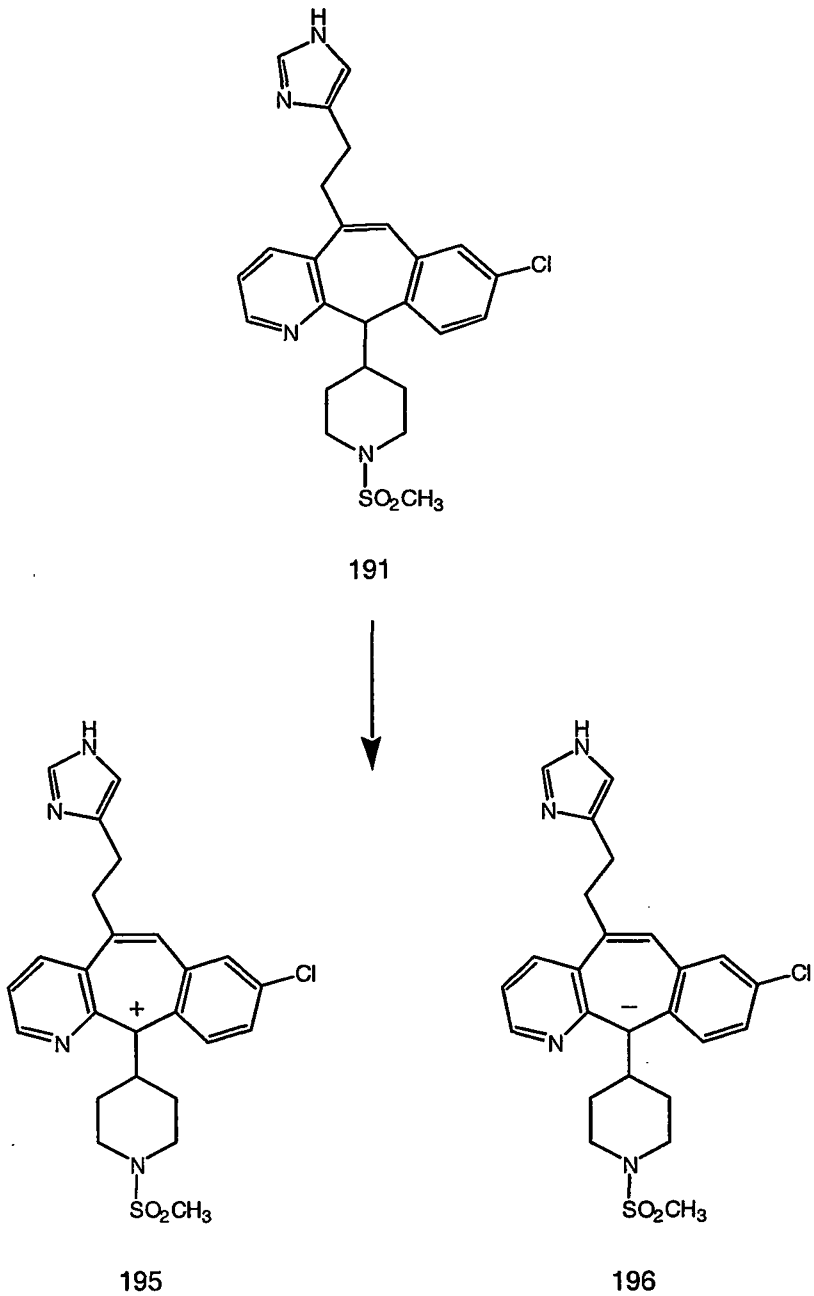

191

195

196

In a similar manner as described in Example 73 above, the following enantiomers were separated:

Compound (195): LCMS: $MH^+$= 483; mp= 129-131 ˚C; $[\alpha]^{20}_D$= +134˚ (2.0 mg in 2.0 mL MeOH).

Compound (196): LCMS: $MH^+$= 483; mp= 125-126 ˚C; $[\alpha]^{20}_D$= -105˚ (2.0 mg in 2.0 mL MeOH).

PREPARATIVE EXAMPLE 13

Preparation of Compound (197).

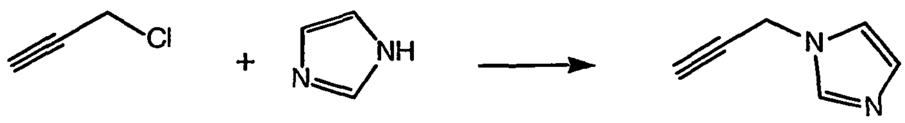

197

Imidazole (2.50g, 36.72 mmol) and basic alumina (15 g) were combined and shaken 15 minutes before adding propargyl chloride (2.66 mL, 1.0eq.). The resulting mixture was stirred 84 hours and suspended in EtOAc. The slurry was filtered and the filtrate was washed with $H_2O$ and brine and dried over $Na_2SO_4$. The solution was filtered and concentrated under reduced pressure to give a clear oil.

EXAMPLE 76

Preparation of Compound (198).

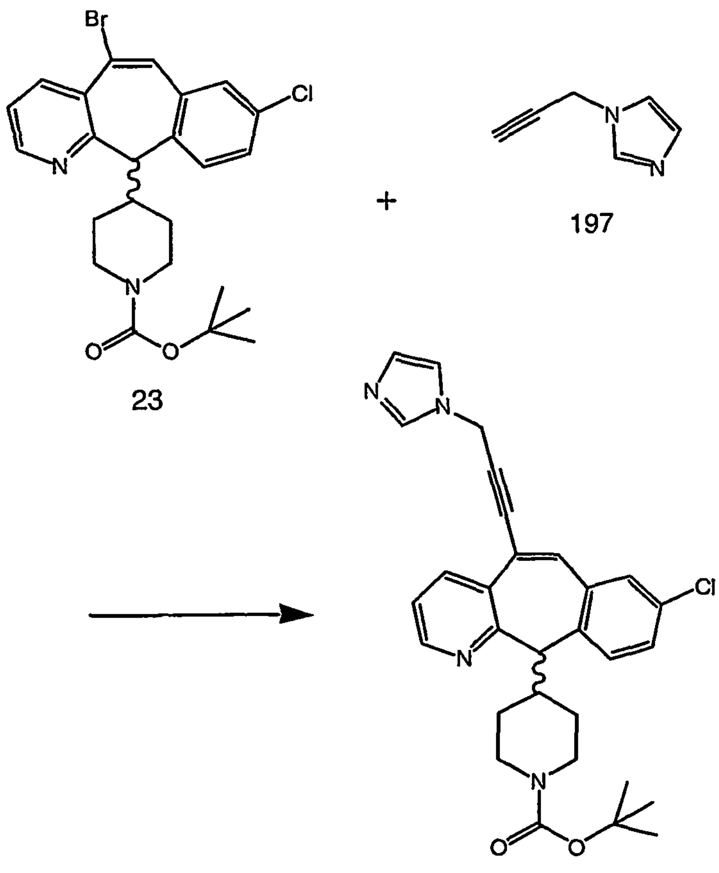

23

197

198

A solution of compound (23) (0.50g, 1.02 mmol) and compound (197) from Preparative Example 13 (0.22g, 2.0 eq.) in TEA (3.0 mL) and pyridine (0.5 mL) was deoxygenated 15 minutes before adding $PdCl_2(PPh_3)_2$ (0.018g, 2.5 mol%) and CuI (0.002g, 1.0 mol%). The resulting solution was heated for 48 hours. The reaction mixture was cooled to room temperature, diluted with $H_2O$, and extracted with $CH_2Cl_2$. The combined organic layer was dried over $Na_2SO_4$, filtered, and concentrated. The crude product was purified by flash chromatography using an 8% MeOH in $CH_2Cl_2$ solution as eluent. mp 109-112 ˚C; LCMS: 515 ($MH^+$).

PREPARATIVE EXAMPLE 14

A. Preparation of Compound (199).

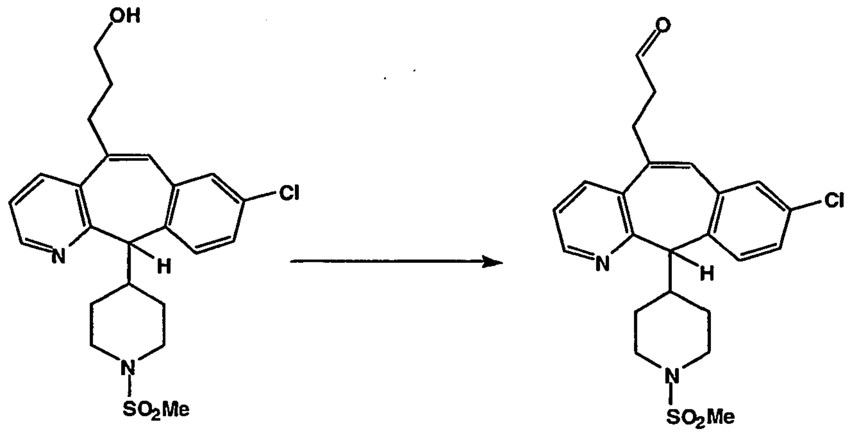

21 199

Compound (21) from Preparative Example 3, Step C, (2.83 g, 6.37 mmol) was dissolved in 120 ml of dichloromethane and 0.16 ml of de-ionized water. Dess-Martin periodinane (3.85 g, 9 mmol) was added as a solid at ambient temperature and the reaction mixture stirred for 4 hours. Then added a 20% $Na_2S_2O_3$ solution (50 ml) and stirred for 15 minutes. The layers were separated and the dichloromethane layer washed with saturated $NaHCO_3$, dried over magnesium sulfate, filtered and evaporated to obtain the title product (199). FABMS: 445 ($MH^+$).

B. Preparation of Compound (200).

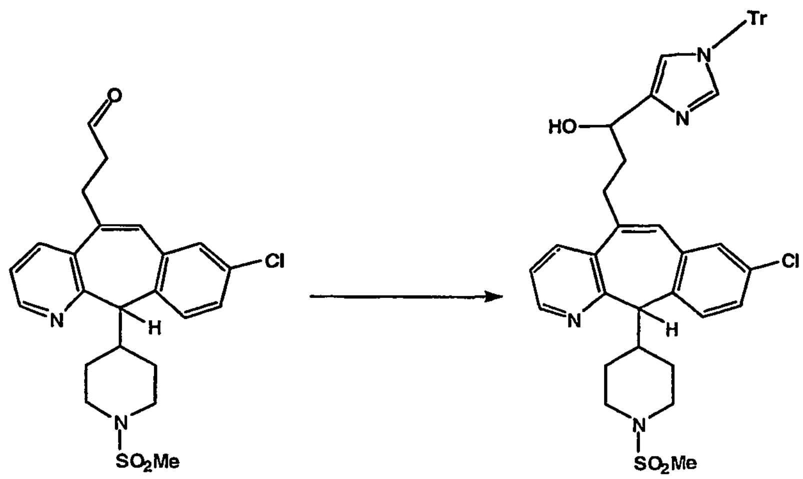

199 200

4-Iodo-1-trityl-imidazole (prepared according to the literature procedure Kirk, Kenneth L.;J. Heterocycl. Chem.; EN; 22; 1985; 57-59) (0.48 g, 1.1 mmol) was dissolved in 5 ml of dichloromethane under a dry nitrogen atmosphere. Ethylmagnesium bromide (0.36 ml) was added and the reaction mixture stirred. After 30 minutes compound (199) (0.44 g, 1 mmol) was dissolved in 5 ml of dichloromethane and added to the reaction mixture while stirring. After stirring 4 hours at ambient temperature, the mixture was washed with saturated ammonium chloride solution, dried over magnesium sulfate, filtered, and evaporated to give a solid residue. The product was chromatographed on a flash silica gel column using ethyl acetate as the eluent to obtain the title compound (200). FABMS: 756 ($MH^+$).

EXAMPLE 77.

Preparation of Compound (201).

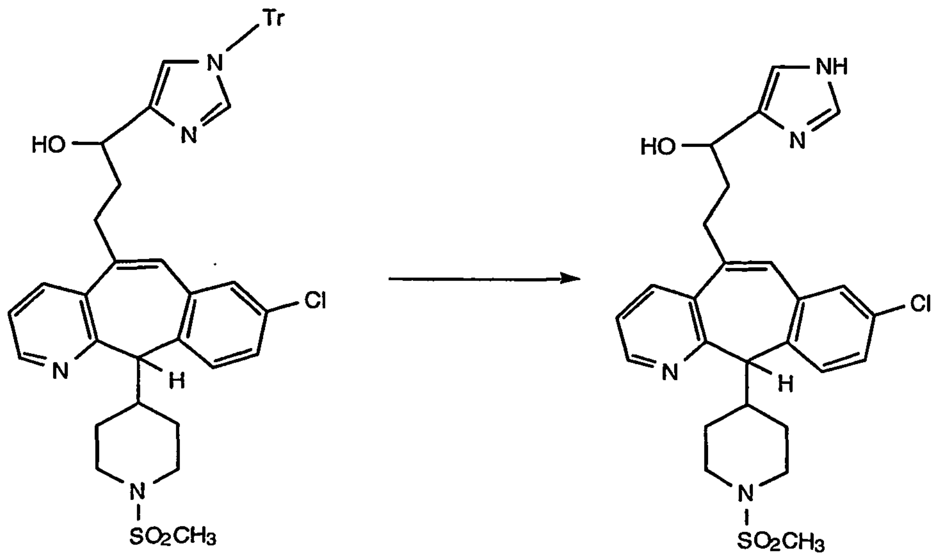

200 201

Compound (200) (0.6 gm) was dissolved in 10 ml of trifluoroacetic acid and stirred at ambient temperature. After 7 hours the reaction mixture was evaporated to dryness under vacuum and chromatographed on silica gel using 5% 2N methanol:ammonia/ dichloromethane to obtain title compound (201). FABMS: 514 ($MH^+$).

PREPARATIVE EXAMPLE 15

A. Preparation of Compounds (202).

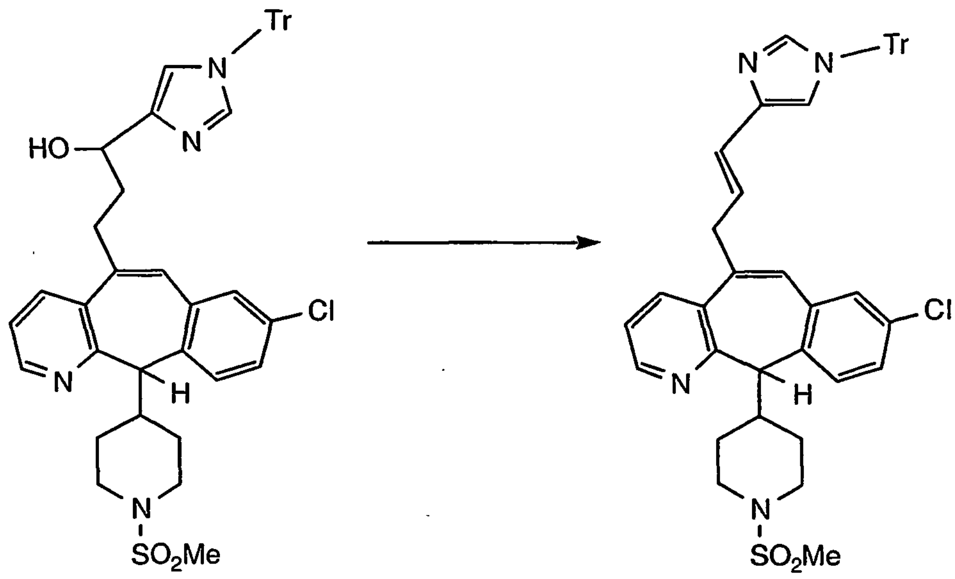

200 202

Compound (200) (0.5 g, 0.66 mmol) was dissolved in 5 ml of dichloromethane. Triethylamine (0.14 ml, 0.99 mmol) and methanesulfonyl chloride (0.062 ml, 0.79 mmol) were added and the reaction mixture stirred for 18 hours. The reaction mixture was added to brine and extracted with dichloromethane three times. Dried over magnesium sulfate, filtered and concentrated to dryness under vacuum to give a residue which was chromatographed on silica gel using ethyl acetate as the eluent to obtain the title compound (202). FABMS: 537 ($MH^+$).

B. Preparation of Compound (203)

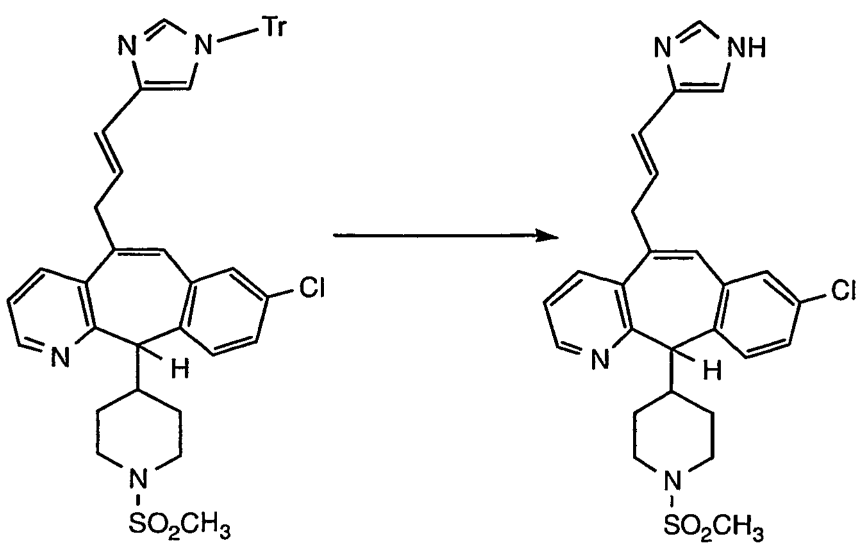

202 203

Compound (202) was detritylated in the same manner as EXAMPLE 77 affording the title compound (203). FABMS: 495 ($MH^+$).

EXAMPLE 78

Preparation of Compounds (205, 206)

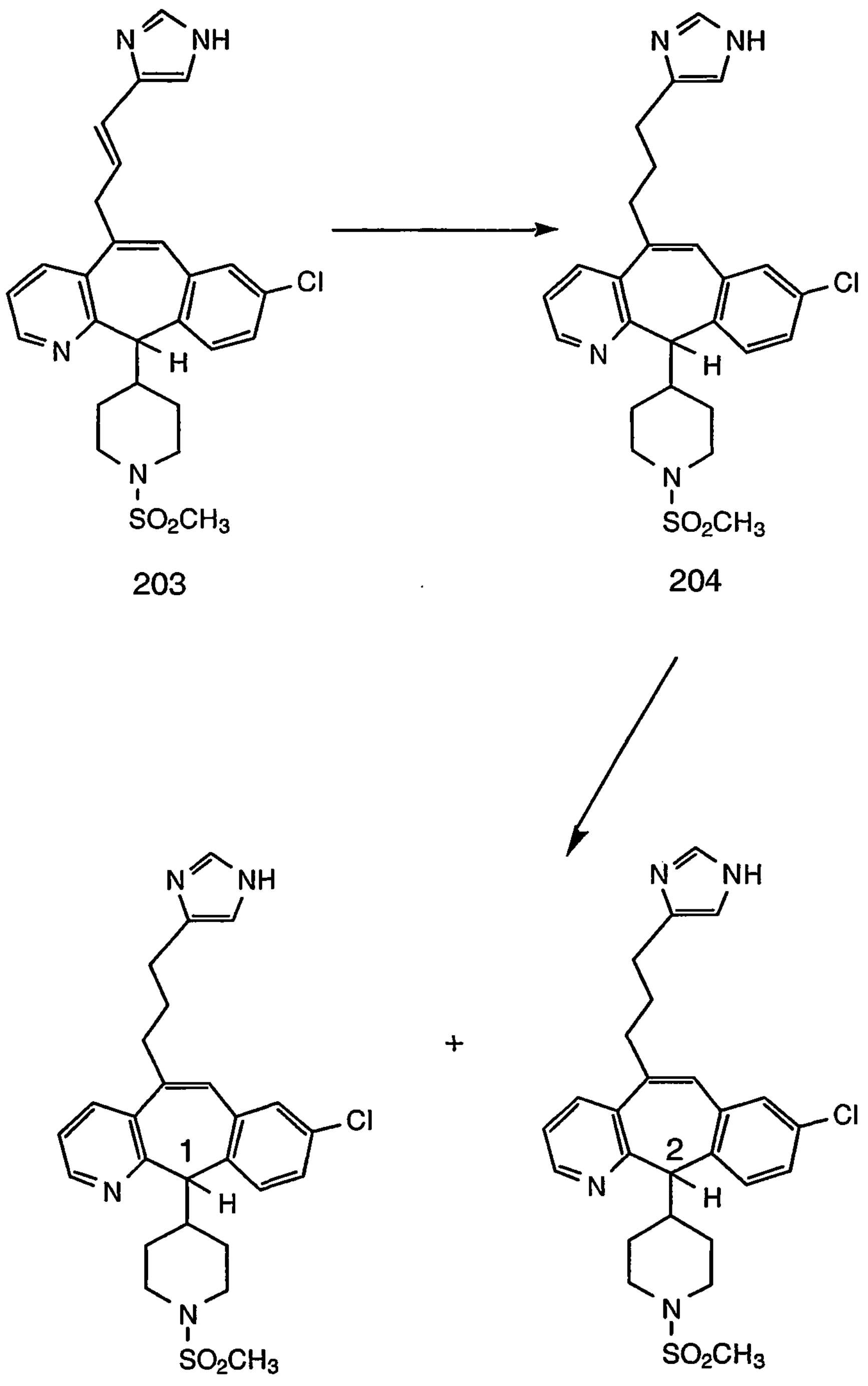

203

204

205

206

Compound (203) (77 mg) was hydrogenated over $PtO_2$ in ethanol at atmospheric hydrogen for 24 hours. After filtration of the catalyst followed by evaporation of the ethanol and chromatography on a Chiral Technologies© AD HPLC column the title product was obtained as two pure enantiomers (205) and (206). FABMS: 497 ($MH^+$).

PREPARATIVE EXAMPLE 16

Preparation of Compound (207).

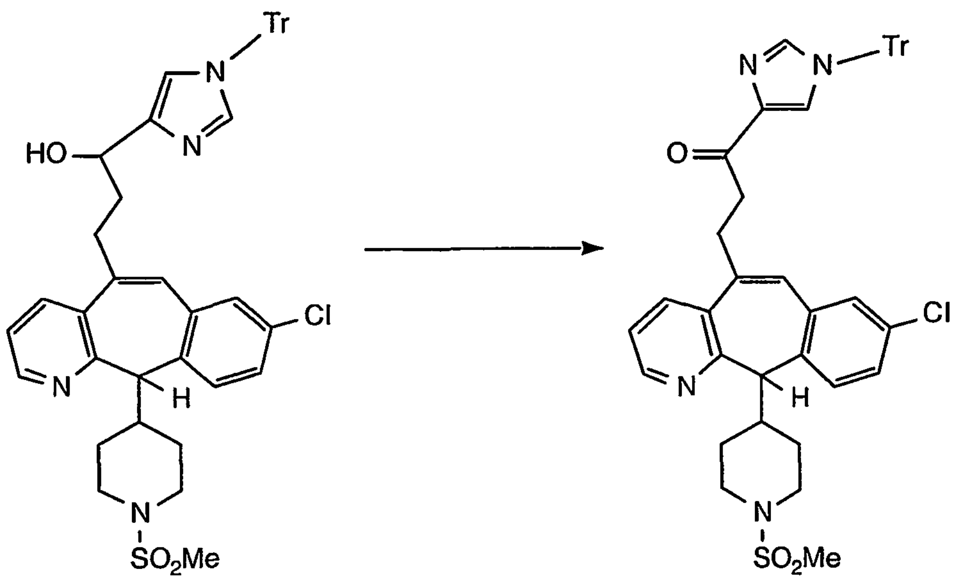

200 207

Compound (200) (0.15 g, 0.198 mmol) was dissolved in 4 ml of dichloromethane and 5 uL of de-ionized water. Dess-Martin periodinane (0.12 g, 0.3 mmol) was added and the reaction mixture stirred for 4 h. 5 ml of a 20% $Na_2S_2O_3$ solution was added and the reaction mixture stirred for another 15 minutes. The layers were separated and the dichloromethane layer was washed with saturated $NaHCO_3$, dried over magnesium sulfate, filtered and evaporated to obtain the title compound (207). FABMS: 753 ($MH^+$).

EXAMPLE 79

Preparation of Compound (208).

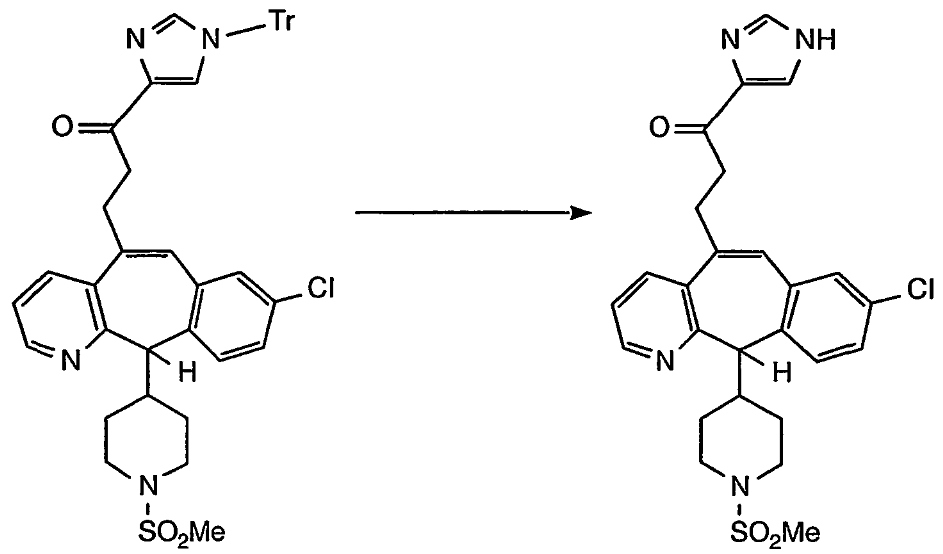

207 208

Compound (207) was detritylated in the same manner as Example 77 affording the title compound (208). FABMS: 511 ($MH^+$).

PREPARATIVE EXAMPLE 17

Preparation of Compound (209).

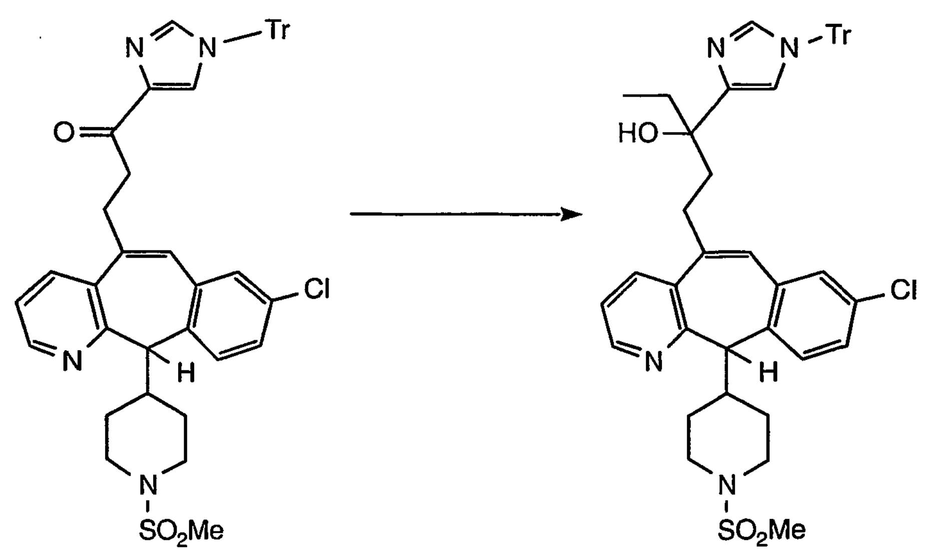

207 209

Compound (207) (0.15 g, 0.2 mmol) was dissolved in 5 ml of tetrahydrofuran. Ethylmagnesium bromide (0.1 ml, 3 M in ether) was added at ambient temperature and stirred under a dry nitrogen atmosphere. After 2 hours, added another portion of ethylmagnesium bromide (0.1 ml, 3 M in ether). After 4 hours the reaction mixture was washed with saturated ammonium chloride, dried over magnesium sulfate, filtered and evaporated to obtain the title compound (209). The product was further purified by flash silica column chromatography eluting with 50% ethylacetate/hexanes. FABMS: 783 ($MH^+$).

EXAMPLE 80

Preparation of Compound (210).

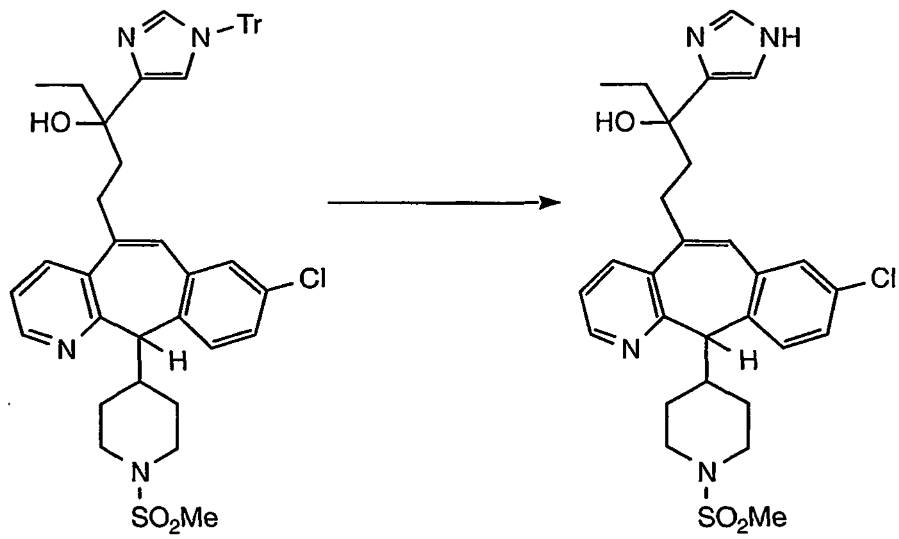

209 210

Compound (209) was detritylated in the same manner as Example 77 affording the title compound (210). FABMS: 541 ($MH^+$).

PREPARATIVE EXAMPLE 18

A. Preparation of Compound (212).

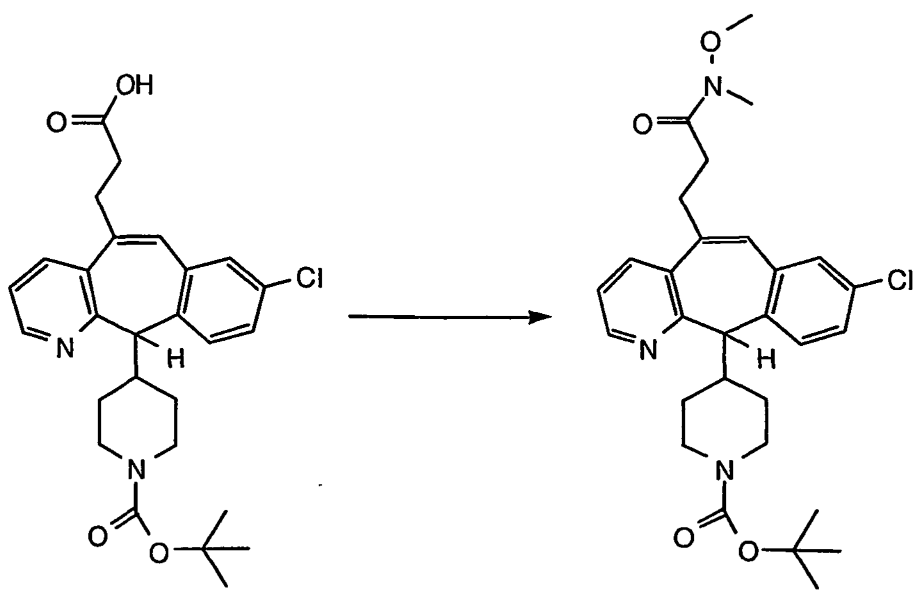

211 212

Compound (211) (14 g, 29 mmol) prepared by NaOH hydrolysis of Compound (20) from Preparative Example 3, Step B, was dissolved in 400 ml of DMF. 1-(3-dimethylamino propyl)-3-ethylcarbodiimide hydrochloride (8.3 g, 43 mmol), 1-hydroxybenzotriazole (5.9 g, 43 mmol), triethylamine (40 ml), and N,O-dimethylhydroxylamine hydrochloride (3.8 g, 40 mmol) were added and the reaction mixture stirred at room temperature under a dry nitrogen atmosphere. After 24 hours the reaction mixture was poured into brine and the product extracted with ethylacetate two times. After drying over magnesium sulfate, filtration, and chromatography on silica gel using 10% ethyl acetate/hexanes the title compound (212) was obtained.

B. Preparation of Compound (213).

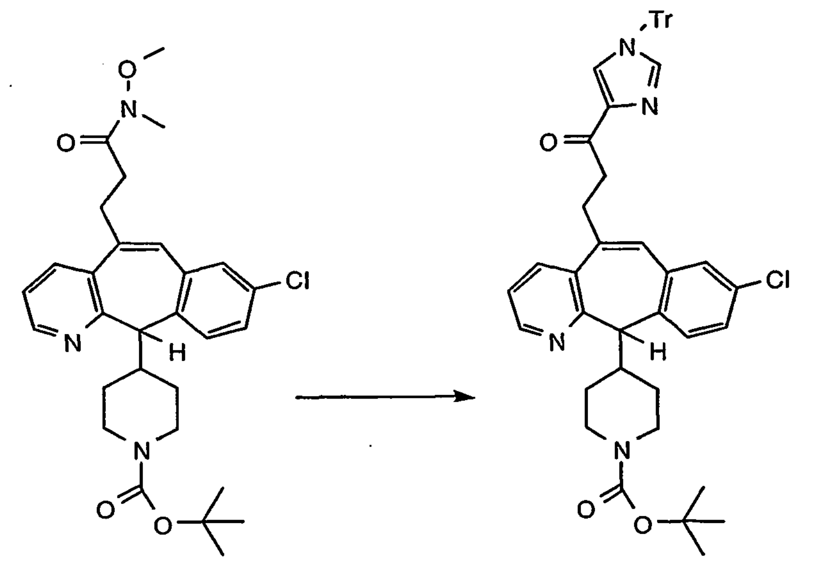

212 213

Compound (212) (0.53 g, 1.01 mmol) was treated as in PREPARATIVE Example 14, Step B to obtain the title compound (213) after silica gel chromatography.

EXAMPLE 81

Preparation of Compounds (214) and (215).

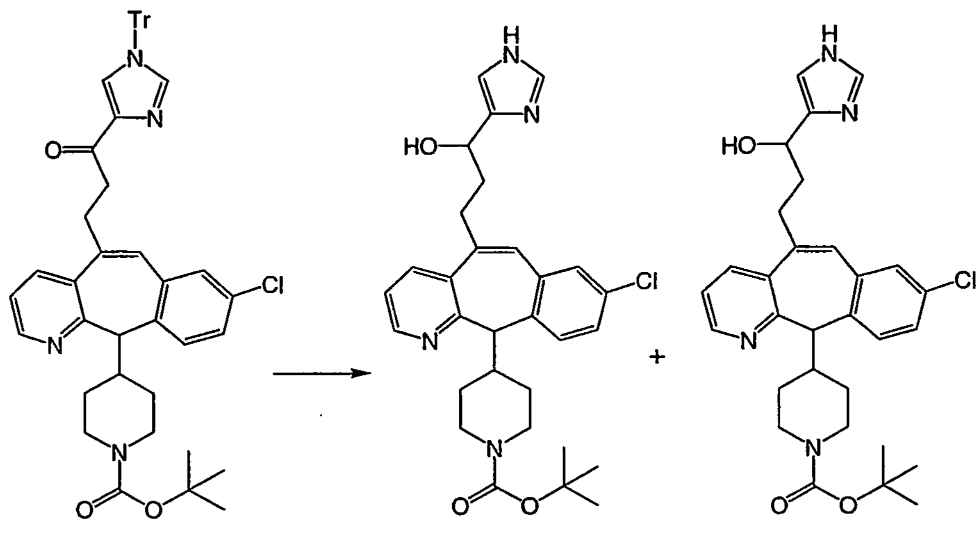

213 214 215

Compound (213) (300 mg, 0.387 mmol) was dissolved in methanol and sodium borohydride (50 mg) was added portionwise while stirring. After 1 hour the mixture was added to 1N HCl followed by the addition of 1 N NaOH and extracted with ethylacetate to obtain a crude product which was treated with neat trifluoroacetic acid for 5 hrs, and evaporated to dryness. The mixture was dissolved in methanol and reacted with di-tert.butyldicarbonate (0.2 gm) while maintaining the pH at 10 with 1N NaOH for 1 hour. The mixture was then treated with 2N Methanolic ammonia for 15 minutes followed by evaporation of the solvents and chromatography on silica gel. Further seperation of isomers was accomplished on a Chiral Technologies© AD HPLC column obtaining the pure isomers. (214) and (215). FABMS M+1=535

EXAMPLE 82

Preparation of Compounds (216)

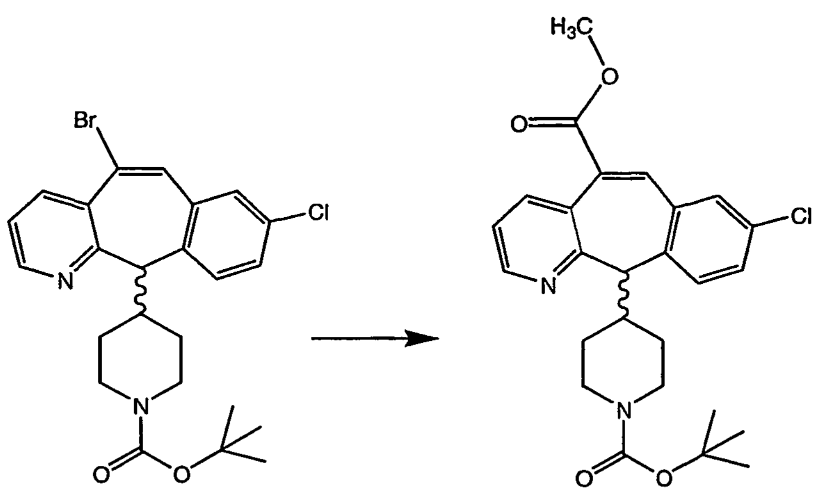

23 216

Compound (23) from Preparative Example 4, Step A (25.47 gm, 52 mmol) was dissolved in 300 ml of dry toluene and 39.5 ml of methanol. Palladium chloride (0.92 gm), triphenylphosphine (6.887 gm) and DBU (10.5 ml) were added and the reaction mixture transferred to a pressure reaction vessel. The reaction vessel was purged with carbon

monoxide and then pressurized to 100 psi with carbon monoxide and the mixture stirred at 80 ˚C for 5 hours. The reaction was cooled in an ice bath and purged with nitrogen 3-4 times. The reaction mixture was transferred to a separatory funnel and 500 ml of ethylacetate was added. The mixture was washed with water three times, dried over magnesium sulfate, filtered and evaporated to dryness under vacuum to give a dark brown gum. The gum was purified by column chromatography on silica gel using 12.5%-25% ethylacetate/hexanes to obtain 12.58 gm of pure title product (216) FABMS: 469 ($MH^+$) and 9.16 gm of a mixture of two compounds.

PREPARATIVE EXAMPLE 19

Preparation of Compound (217)

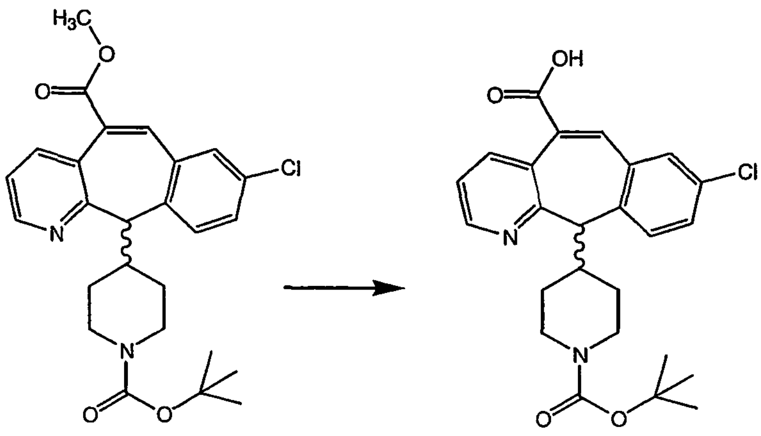

216 217

Compound (216) from Example 82 (5.16 gm, 11 mmol) was dissolved in methanol (150 ml). 10% lithium hydroxide (2.9 ml) was added along with dioxane (50 ml) and the reaction stirred for 4 hours. Added an additional portion of 10% lithium hydroxide (5.7 ml) and the reaction stirred for 18 hours. The reaction mixture was concentrated to s small volume and diluted with 50 ml of water. The mixture was acidified to pH=3 with 10% citric acid and the product extracted with dichloromethane to obtain the title compound (217). FABMS: 455 ($MH^+$)

PREPARATIVE EXAMPLE 20

A. Preparation of Compound (218)

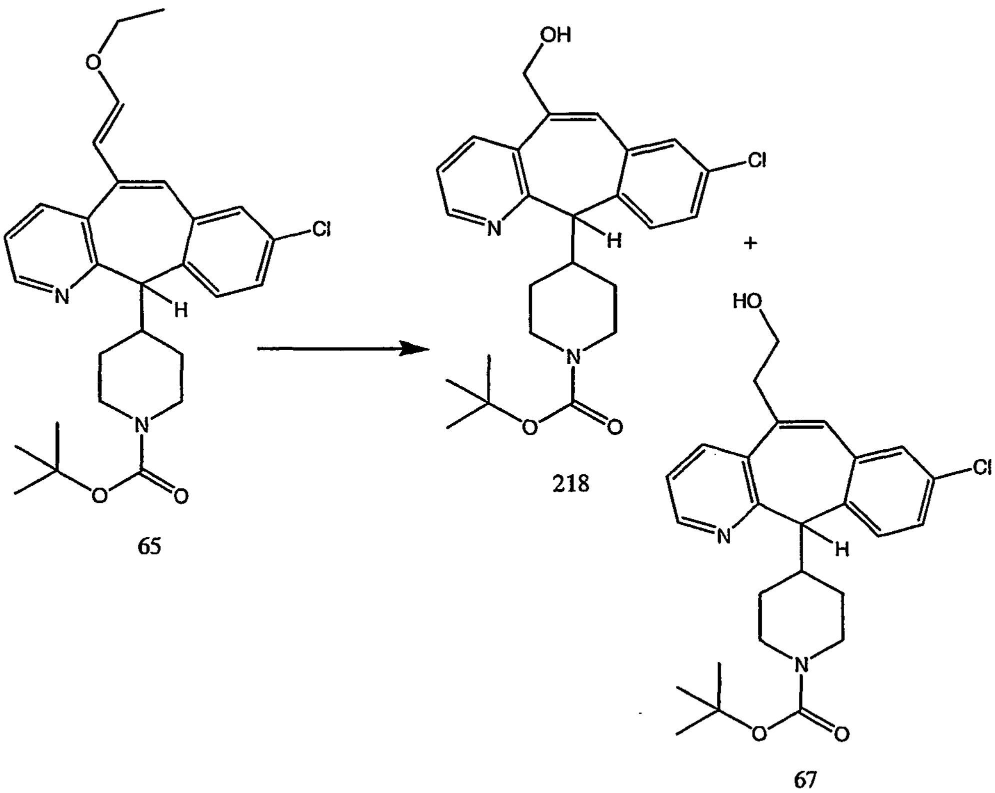

65

218

67

Compound (65) from Preparative Example (6), Step B, was let stand for approximately two weeks at room temperature, after which time the pressence of some aldehyde was observed by NMR of the crude material. This material was then treated as in Preparative Example 6, Steps C and D to afford a mixture of Compounds (218) and (67). The crude mixture was separated on flash silica column chromatography eluting with 1:1 - 3:1 ethyl acetate:hexanes to afford pure Compound (218).

B. Preparation of Compound (219)

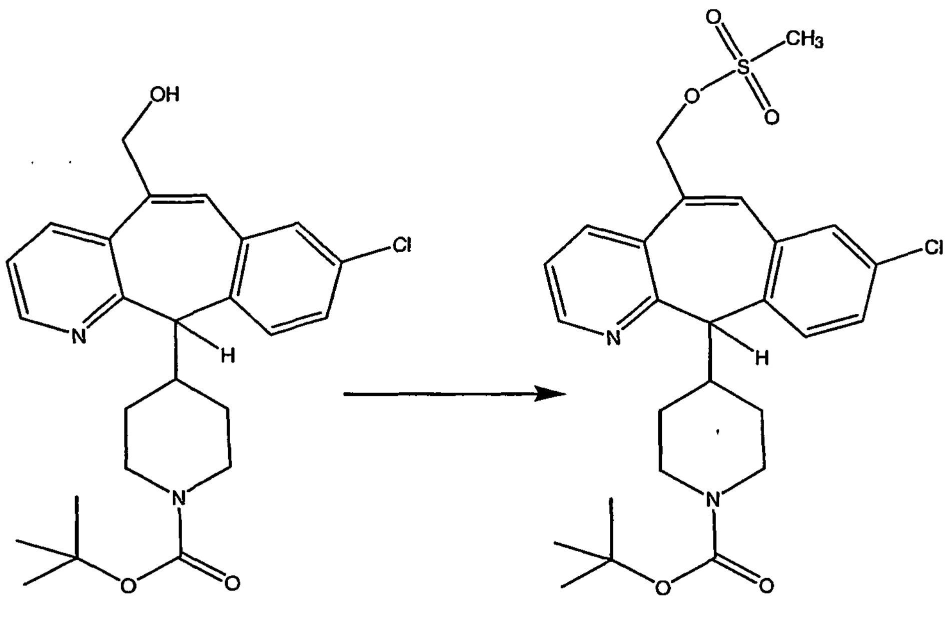

218 219

Compound (218) from Step A above, was combined with triethylamine (64.4 ml; .462 mmol) in $CH_2Cl_2$ (4 ml) treated with methyl sulfonyl chloride (17.93 ml; .231 mmol) and let stir over night at room temperature. The reaction mixture was diluted with $CH_2Cl_2$ (70 ml), quenched with brine (25 ml) and extracted. The organic layer was dried over $MgSO_4$, filtered and concentrated to give an off-white solid (219) (93 mg; 100%).

C. Preparation of Compound (220)

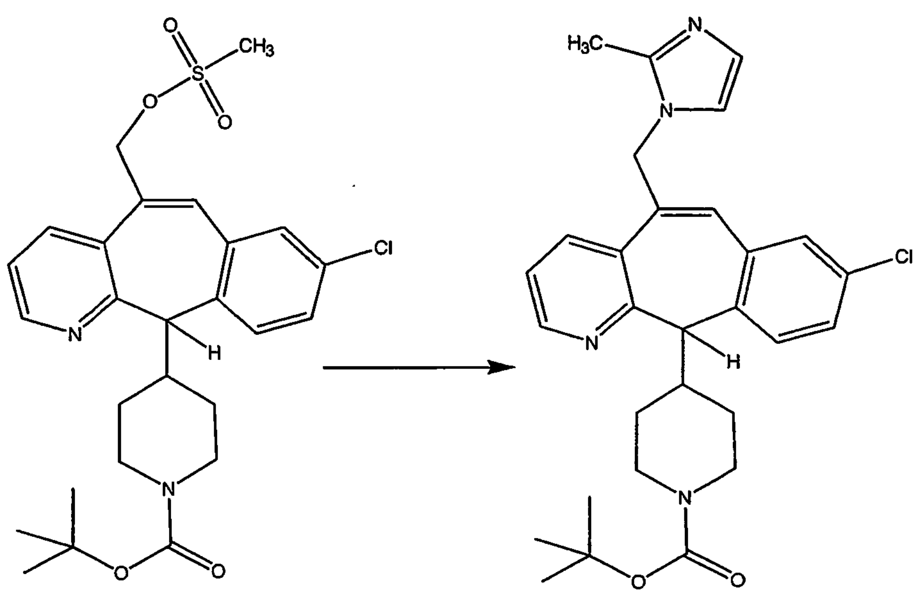

219 220

Compound (219) from Step B above, was taken up in DMF. To this solution was added a previously reacted

solution of 2-methyl imidazole (145.27 mg; 1.734 mmol) and NaH (60%) (69.4 mg; 1.734 mmol) in DMF. The reaction mixture was allowed to stir at room temperature for two hours. The DMF was removed and the residue taken up in $CH_2Cl_2$ quenched with sat. aqueous $NaHCO_3$ and extracted with 2 x 100 ml $CH_2Cl_2$. The organic layers were combined and purified by preparative TLC plates to give an off-white solid. (220)

D. Preparation of Compound (221)

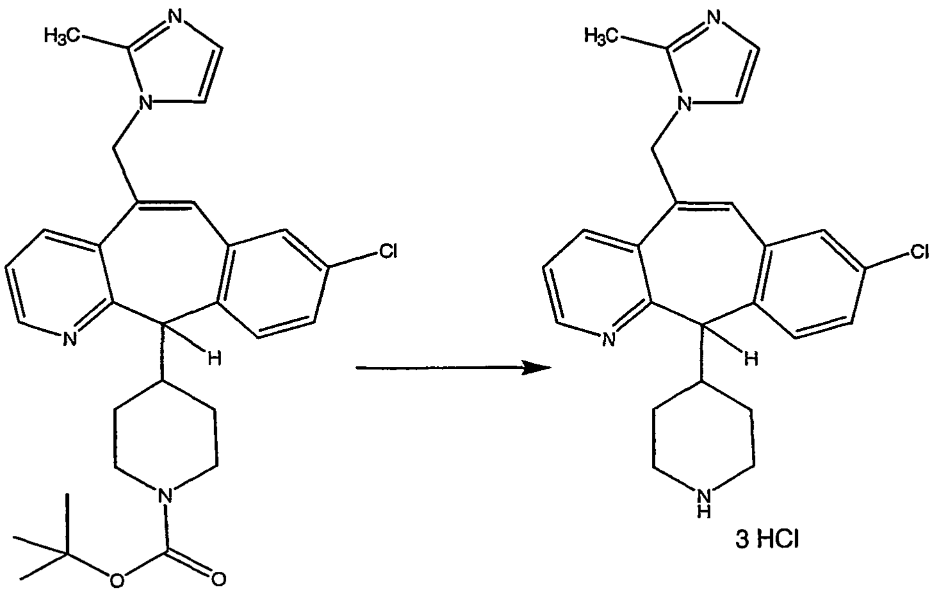

220

221

Compound (220) from Step C above, was dissolved in 1,4-Dioxane (3 ml). To this solution was then added 4M HCl in Dioxane (5 ml) and the reaction stirred for 3 hours at room temperature. The mixture was then concentrated and dried over night under high vacuum to afford the hydrochloride salt as an off-white solid. (221).

EXAMPLE 83

Preparation of Compound (222)

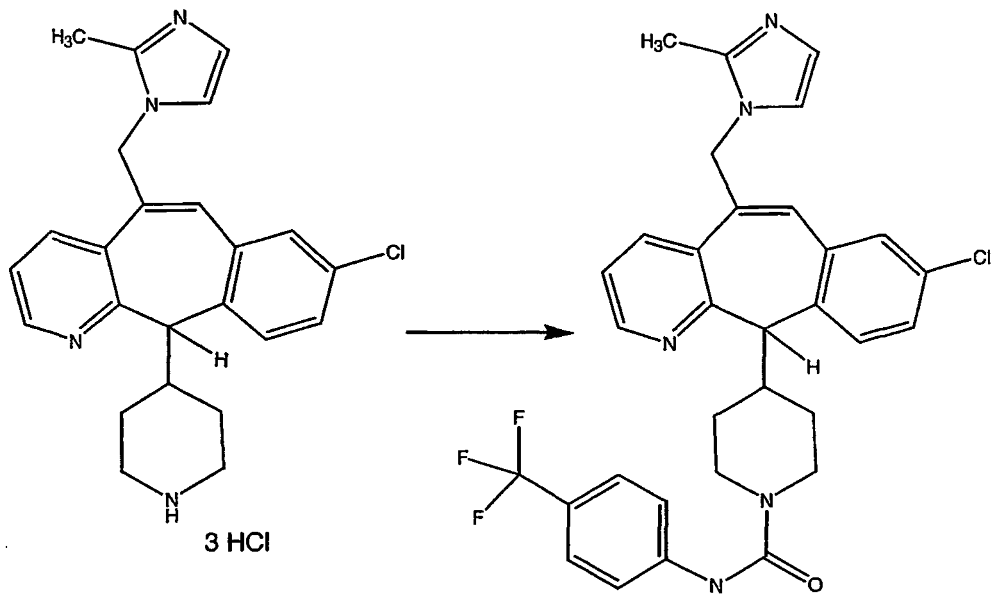

221 222

To a solution of compound (221) from Preparative Example 20, Step D (51 mg; .126 mmol) and triethylamine (61.47 ml; .441 mmol) in $CH_2Cl_2$ (2 ml) was added 4-trifluoromethylphenyl isocyanate (20.26 ml; .139 mmol) at 0 ˚C. The reaction stirred for 2-3 hours under $N_2$ atmosphere. The $CH_2Cl_2$ and excess triethylamine were removed under vacuo and the resultant product was purified by preparatory thin layer chromatography eluting with 98:2 $CH_2Cl_2$/ (sat.) MeOH/$NH_3$) affording the title compound as a white solid (222).

PREPARATIVE EXAMPLE 21

A. PREPARATION OF PIPERIDYL INTERMEDIATE

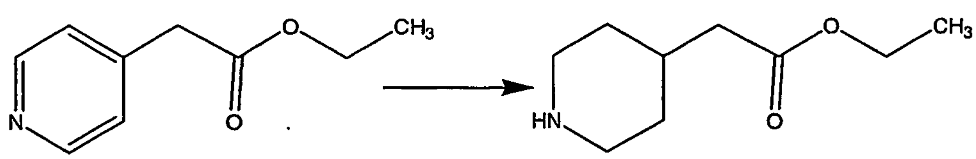

Commercially available Ethyl 4-Pyridyl Acetate (4.5g; 27.2 mmol), EtOH (70 ml) and 10% Palladium on Charcoal (catalytic) was shaken under 55 psi hydrogen at room temperature for 94 hrs. The mixture was filtered through Celite and the cake was washed with (4 x 40 ml) of EtOH. The filtrate was concentrated and purified by flash silica column chromatography eluting with 3% (10% $NH_4OH$:MeOH)/$CH_2Cl_2$.

B PREPARATION OF (1-CARBAMOYL-PIPERIDIN-4-YL)-ACETIC ACID ETHYL ESTER.

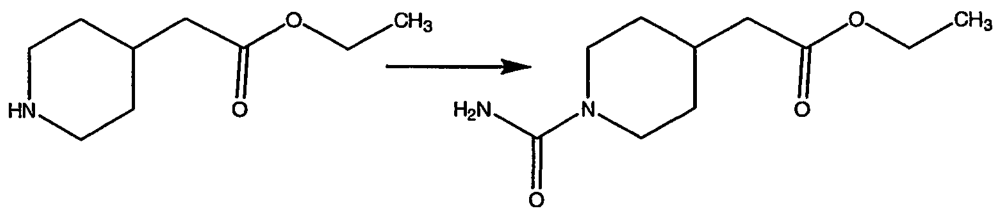

4-Pyridyl Acetic Acid (2.362 g) from Step A above, was taken up in $CH_2Cl_2$ (118 ml). To this was added trimethylsilyl isocyanate (27.87 ml). The reaction stirred for 67 hr then was diluted with $CH_2Cl_2$ (700 ml) and washed with saturated aqueous $NaHCO_3$ (150 ml). The aqueous layer was extracted with 2 x 200 ml $CH_2Cl_2$. The organic layers were combined, dried over $MgSO_4$, filtered and concentrated. The crude product was purified by flash silica column chromatography eluting with 2% (10% $NH_4OH$:MeOH)/$CH_2Cl_2$.

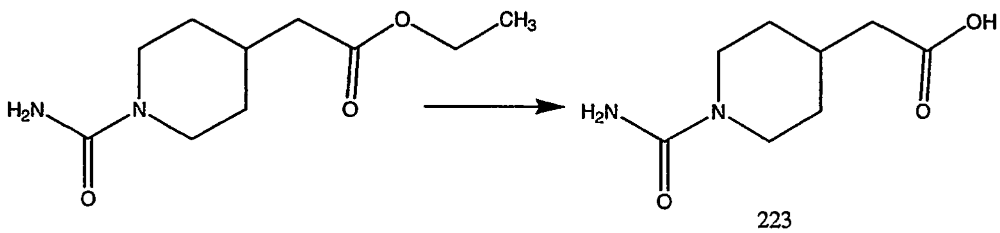

223

C. Product from Step B above (40.63 mg; 0.1896 mmol) was taken up in EtOH (2 ml) and $CH_2Cl_2$ (2 ml) and treated with 1M LiOH (.5 ml; .455 mmol). The reaction mixture was heated to 50°C and stirred for 5 hr. The reaction was cooled to room temperature treated with 1N HCl (.57 ml; .531 mmol) and stirred for 5 minutes. The resultant mixture was concentrated and dried under high vacuum for 4 days affording the title compound as a white solid. (223)

EXAMPLE 84

Preparation of Compound (224)

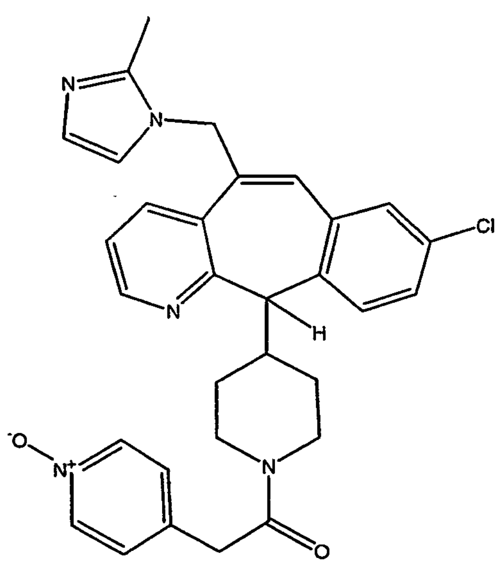

224

To a solution of Compound (221) from Preparative Example 20, Step D (51 mg; .126 mmol), 4-methylmorpholine (69.3 ml; .630 mmol), DEC (31.44 mg; .164 mmol), and HOBT (22.2 mg; .164 mmol) in DMF (2 ml) was added, 4-Pyridylacetic Acid 1-N-Oxide (disclosed in US 5,719,148; 2/17/98). The reaction stirred for 3 hours at room temperature. The reaction was diluted with $CH_2Cl_2$ and washed two times with saturated aqueous $NaHCO_3$. The organic layers were combined, concentrated and purified by preparative thin layer chromatography eluting with 95:5 $CH_2Cl_2$: sat. MeOH/$NH_3$ affording the title compound as a white solid (224).

EXAMPLE 85

Preparation of Compound (225).

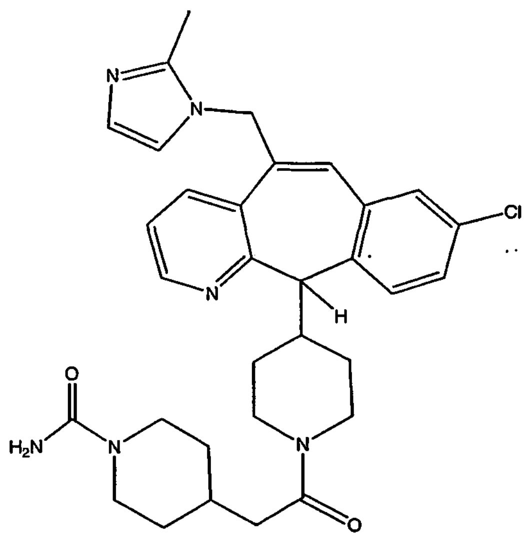

225

Compound (221) from Preparative Example 20, Step D (51 mg; .126 mmol) was combined with compound (223) from Preparative Example 21, Step C and reacted in the same manner as Example 84 to afford the title compound as a white solid. (145-155°C dec.) $MH^+$ 573.(225)

EXAMPLE 86

Preparation of Compound (226).

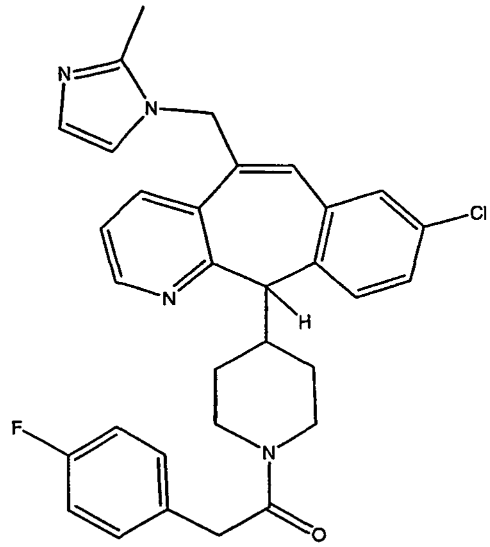

226

Compound (221) from Preparative Example 20, Step D (51 mg; .126 mmol) was combined with 4-Fluorophenylacetic acid (Acros) (29.29 mg; .190 mmol) and reacted in the same manner as Example 84 to afford the title compound as an off-white solid. (108-125˚C dec.) $MH^+$ 541.(226)

PREPARATIVE EXAMPLE 22

Preparation of Compounds (227 and 228)

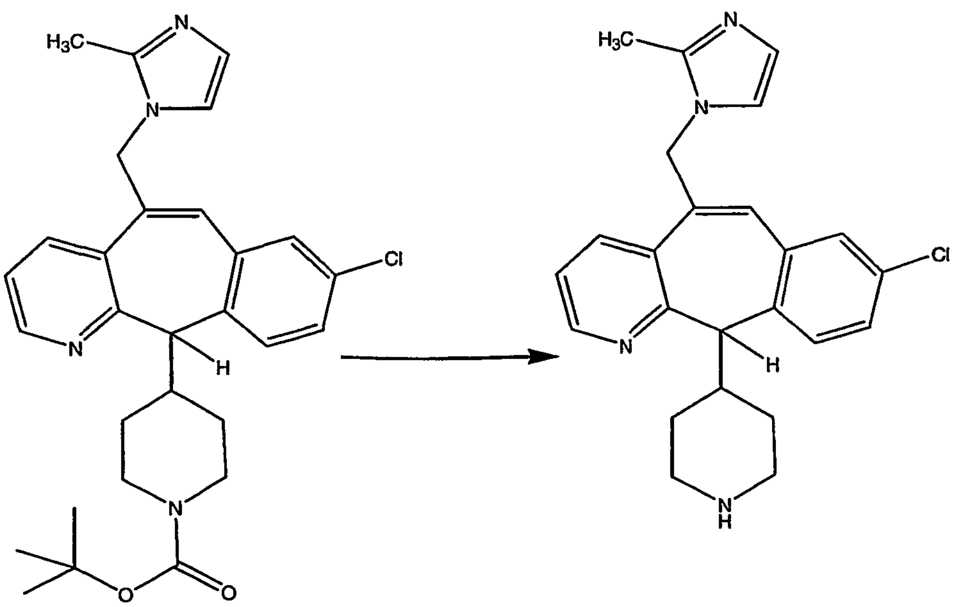

220

(+) 227
(-) 228

Compound (220) from Preparative Example 20, Step C, (150 mg; .289 mmol) was treated with 4M HCl in Dioxane and allowed to stir for 2-3 hr at room temperature under a $N_2$ atmosphere. The crude mixture was separated into pure (+) isomer (227) and (-) isomer (228) by preparative chiral HPLC using an AD column, eluting with 85:15:2 Hexanes:IPA:DEA.

EXAMPLES 87-90

The appropriate (+) compound (227) or (-) compound (228) isomer from Preparative Example 22 above, was taken up in $CH_2Cl_2$ treated with the corresponding isocyanate and stirred at room temperature over night. Crude product was purified directly by preparative thin layer chromatography to afford the following compounds (229-232):

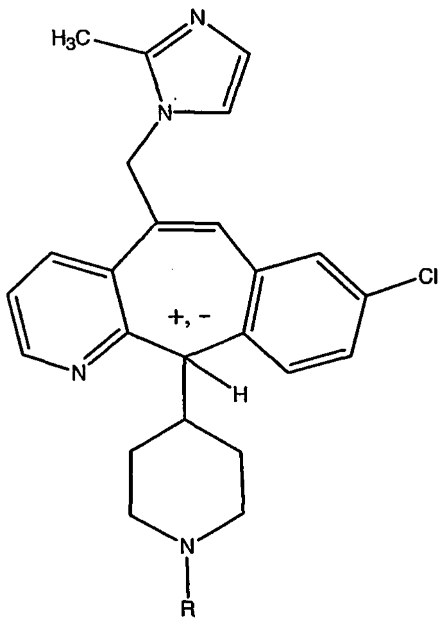

| Ex. | R | Compound # | |
|---|---|---|---|
| 87 | 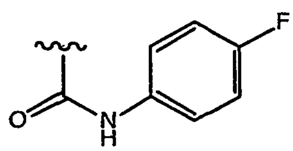 | (229) | (+)(148-156°C dec.) $MH^+$ 556. |
| 88 | 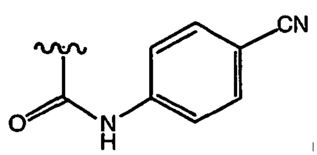 | (230) | (+)(155-166°C dec.) $MH^+$ 563. |
| 89 | 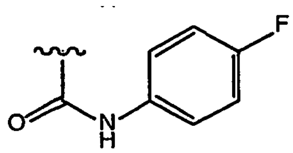 | (231) | (-)(145-153°C dec.) $MH^+$ 556. |
| 90 | 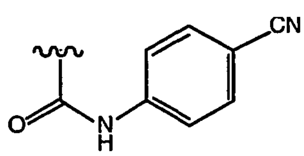 | (232) | (-)(159-168°C dec.) $MH^+$ 563. |

PREPARATIVE EXAMPLE 23

A. Preparation of Compound (233).

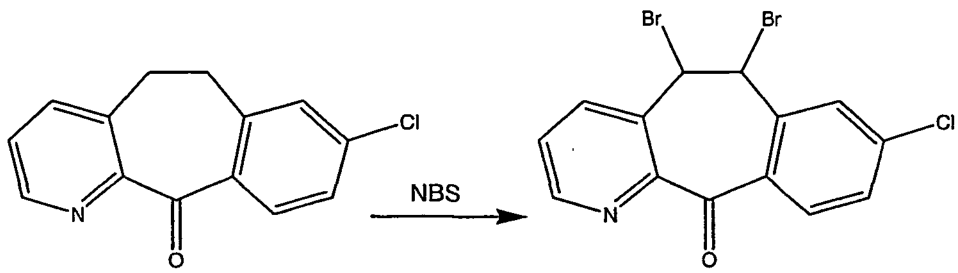


233

The tricyclic keto-compound (disclosed in US Pat. No. 5,151,423) (30.0 g; 123.2 mmol) was combined with NBS (48.2 g; 271.0 mmol) and benzoyl peroxide (0.42 g) in $CCl_4$ (210 ml). The reaction was heated to 80˚C for 10 hr. The mixture was cooled and let stand for 8 hr. The resulting precipitate was filtered. Added MeOH (200 ml) and stirred the mixture over 2 days. The solid was filtered and dried under vacuum to a constant weight.

B. Preparation of Compounds (234a) AND (234b)

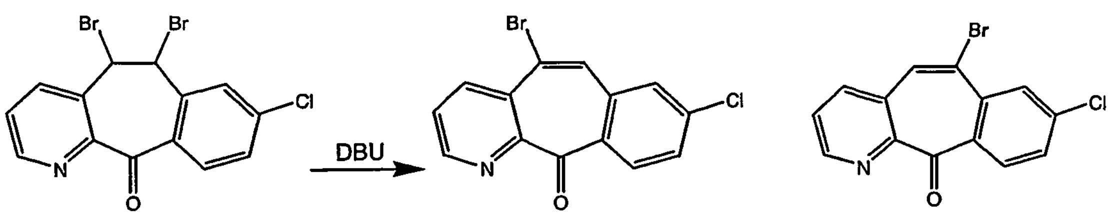


233 234a 234b

The dibromo compound (233) from Step A (35.72 g; 88.97 mmol) above was dissolved in $CH_2Cl_2$ (1.5 L) and cooled to 0˚C. Dropwise, DBU (15.96 ml) was added and the suspension stirred for 3 hr. The reaction mixture was concentrated redissolved in $CH_2Cl_2$ (1.5 L) filtered through a bed of silica gel and rinsed with 5% EtOAc/$CH_2Cl_2$ (4 L). The combined rinses were concentrated and purified by flash silica gel column chromatography into pure 5 and 6 mono-bromo substituted compounds eluting with 10-30% EtOAc/Hex then 3%EtOAc/$CH_2Cl_2$.

C. Preparation of Compound (235).

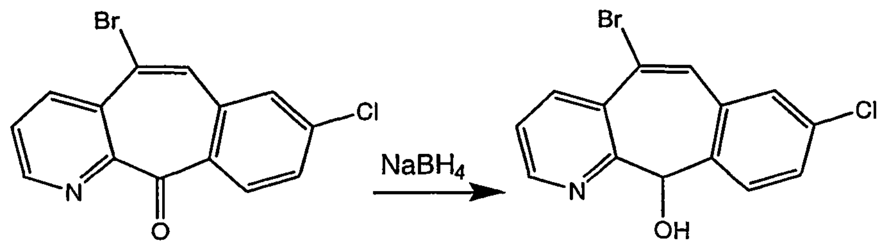


234a 235

The 5-bromo substituted compound (234a) from Step B above (4.0 g; 12.45 mmol) was taken up in MeOH and cooled to 0°C. $NaBH_4$ (916.4 mg; 24.2 mmol) was added and the reaction mixture stirred for 5.5 hr. The solvent was removed and the resulting residue was used directly.

Step D Preparation of Compound (236).

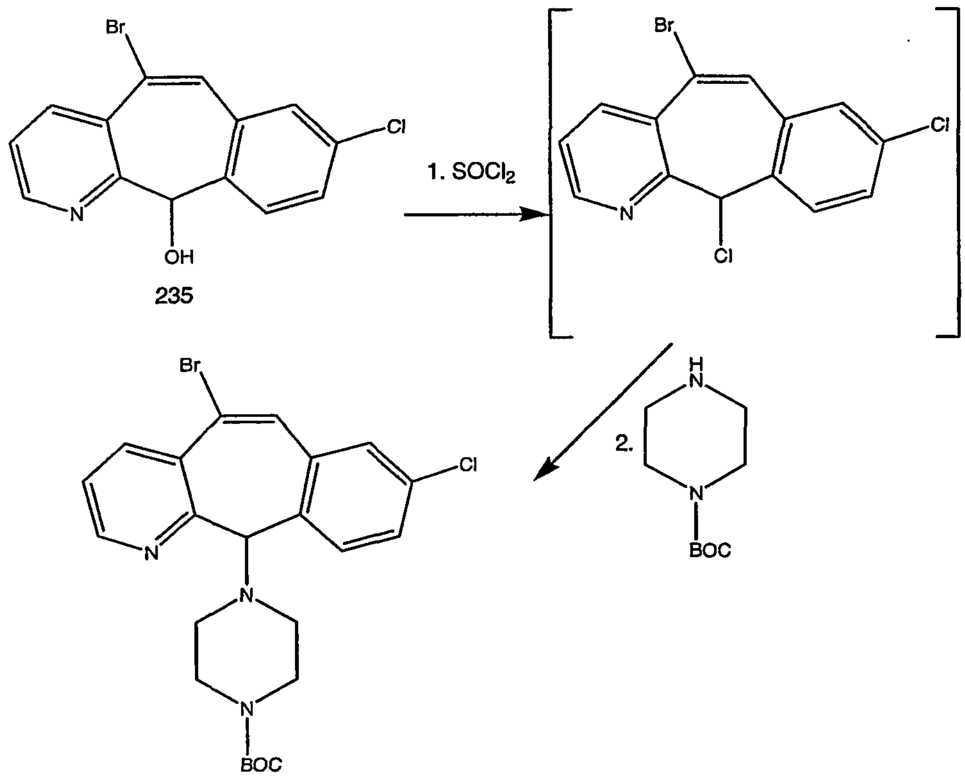


235

236

The alcohol compound (235) from Step C above (3.98 g; 12 mmol) was dissolved in $CH_2Cl_2$ cooled to 0°C and treated with 2,6-Lutidine (5.73 ml; 49 mmol). $SOCl_2$ (1.8 ml; 24.6 mmol) was added and the reaction was allowed to stir and come to room temperature over 3 hr. The reaction mixture was poured into 0.5 N NaOH (80 ml) extracted and concentrated in vacuo. The crude product was taken up in $CH_3CN$ and treated with 1,2,2,6,6-Pentamethylpiperidine (4.45 ml; 24.6 mmol) (Aldrich). The reaction was heated to 60-65°C treated with tert-butyl 1-piperazinecarboxylate (2.32 g; 12 mmol) (Aldrich) and stirred over night under $N_2$ atmosphere. The reaction mixture was concentrated to dryness, redissolved in $CH_2Cl_2$ and washed with sat. aqueous $NaCO_3$. The organic layer was dried over $Na_2SO_4$, filtered and purified by flash silica gel column chromatography eluting with 1:4-1:2 EtOAc/Hexanes to afford the product as a white solid.

Step E Preparation of Compound (237).

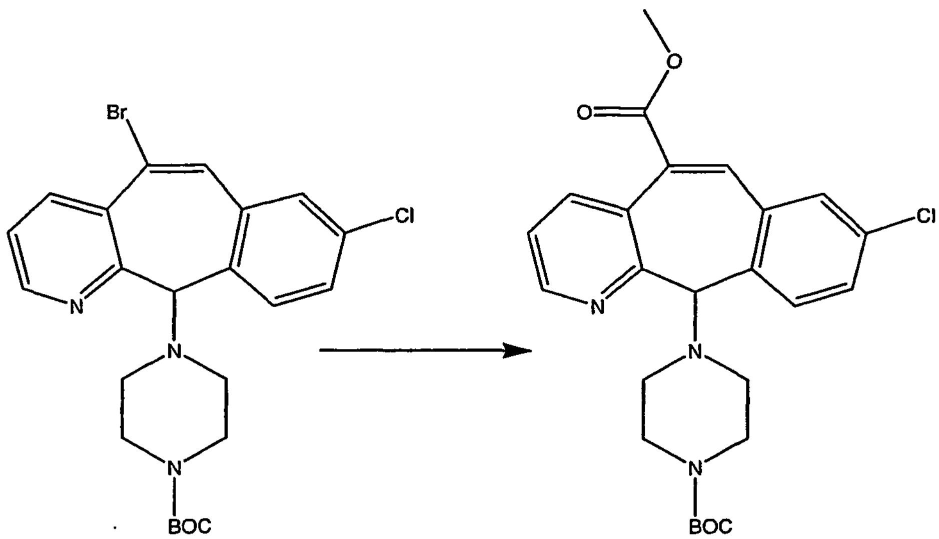

236 237

The BOC-protected bromo-compound (236) from Step D above (2 g; 4 mmol), triphenyl phosphine (.54 g; 2 mmol), and palladium chloride (.0723 g; .4 mmol) were combined in MeOH (10 ml) and toluene (30 ml). To this mixture was added DBU (.835 ml; 5.5 mmol) and the mixture was sealed in a Parr bomb. The reaction mixture was stirred and subjected to 90 psi of CO at 80˚C for 5 hr. The reaction was diluted with EtOAc (200 ml) and washed with 2 x 80 ml $H_2O$. The organic layer was dried over $MgSO_4$, filtered and purified by flash silica column chromatography eluting with 1:3 EtOAc/Hexanes.

F. Preparation of Compound (238).

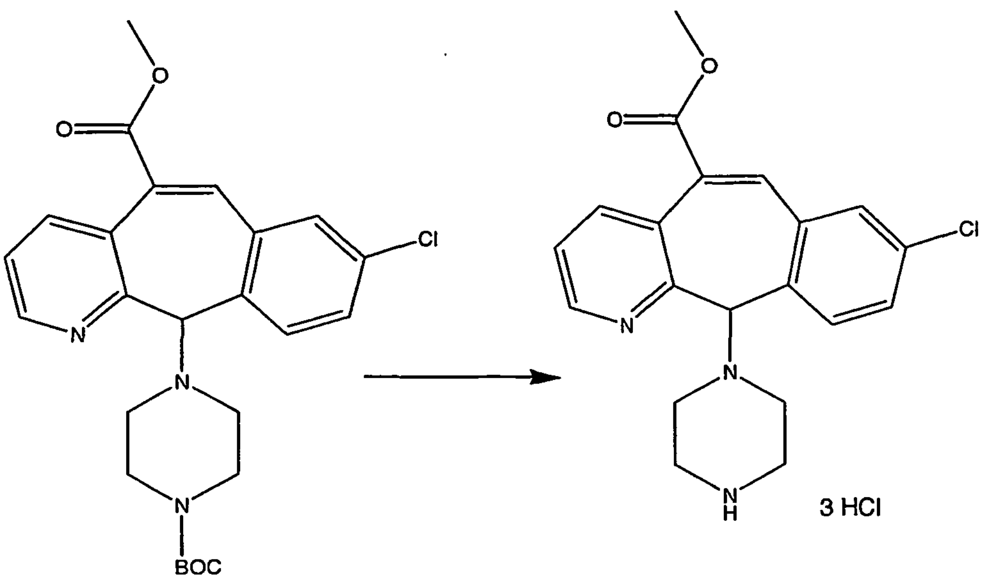

237 238

Compound (237) from Step E above (1.73g; 3.681 mmol) was treated with 4 M HCl in Dioxane (35 ml) and allowed to stir at room temperature for 3 hr. The reaction mixture was concentrated in vacuo and the resulting tan solid was further dried under high vaccuum.

G. Preparation of Compound (239).

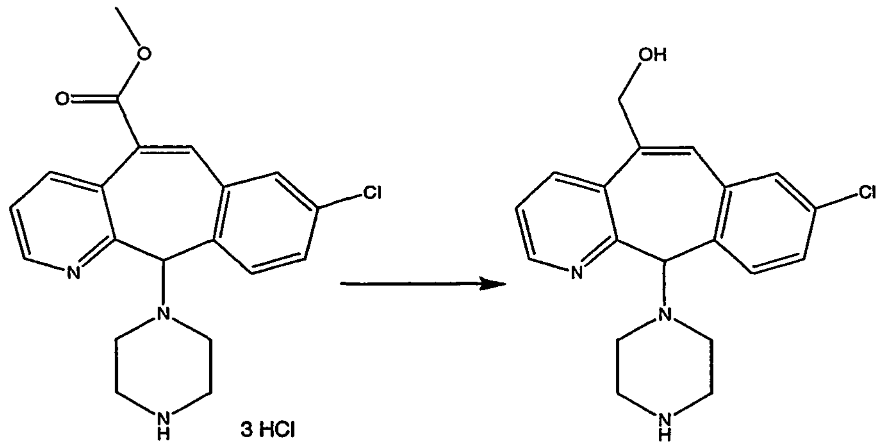

238 239

The HCl salt (238) from Step F above (1.36 g; 3.68 mmol) was dissolved in THF, cooled to 0°C, treated with 1 M DIBAL in cyclohexane (18.41 ml; 18 mmol) and stirred over night at room temperature. The mixture was concentrated to dryness and used directly in the next step.

H Preparation of Compound (240).

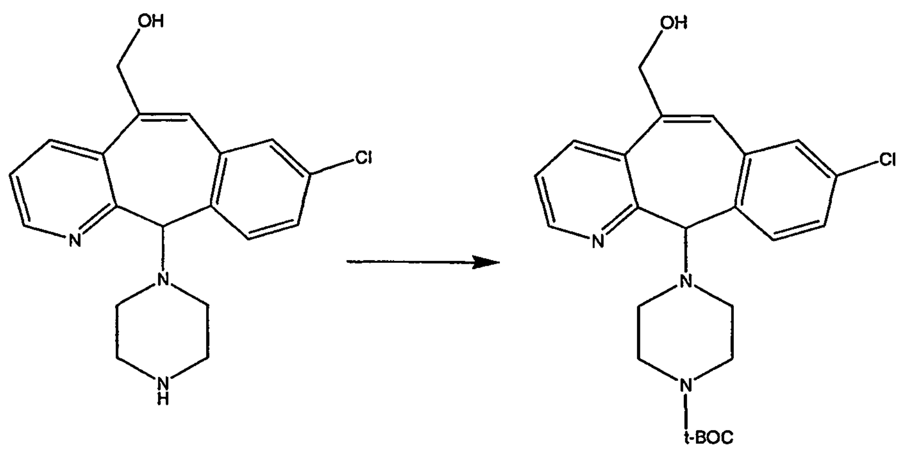

239 240

The alcohol (239) from Step G above was taken up in MeOH (50 ml) and $H_2O$ (5 ml) and treated with Boc anhydride (1.56 g; 7.14 mmol). The pH was adjusted to approximately 10 with 1N NaOH. The reaction mixture was concentrated, taken up in $CH_2Cl_2$ and washed with $H_2O$ (2 x) The organic layer was dried over $MgSO_4$, filtered and concentrated to a tan solid containing both product and an impurity.

Alternatively, compound (237) was converted to compound (240) by first preparing the acyl imidazole followed

by $NaBH_4$ reduction using the following procedure:

Compound (237) from Step E above (7.0 mmol) was dissolved in a mixture of 15 mL methanol, 60 mL dioxane and 6 mL water containing 25 mL of 10% aqueous LiOH. The mixture was heated at 60° C for 4 hr, then it was concentrated under vacuum and the pH adjusted to 5.2 with 10% aqueous citric acid. The residue was dissolved in $CH_2Cl_2$, washed with brine, dried over $MgSO_4$ and concentrated under vacuum to give the carboxylic acid. The acid was then dissolved in 20 mL THF containing 14 mmol of 1,1'-carbonyl diimidazole and heated at 38° C for 18 hr. The mixture was then concentrated under vacuum to give the acyl imidazole. The residue was dissolved in a mixture of 21.2 mL of THF and 5.3 mL water and cooled to 0° C. To the solution was added 35 mmol of $NaBH_4$ and it was stirred for 1.5 hr. 5 mL brine and 25 mL $CH_2Cl_2$ was then added The organic layer was dried over $MgSO_4$ and concentrated under vacuum to give compound (240) in essentially a quantitative yield.

I. Preparation of Compound (241).

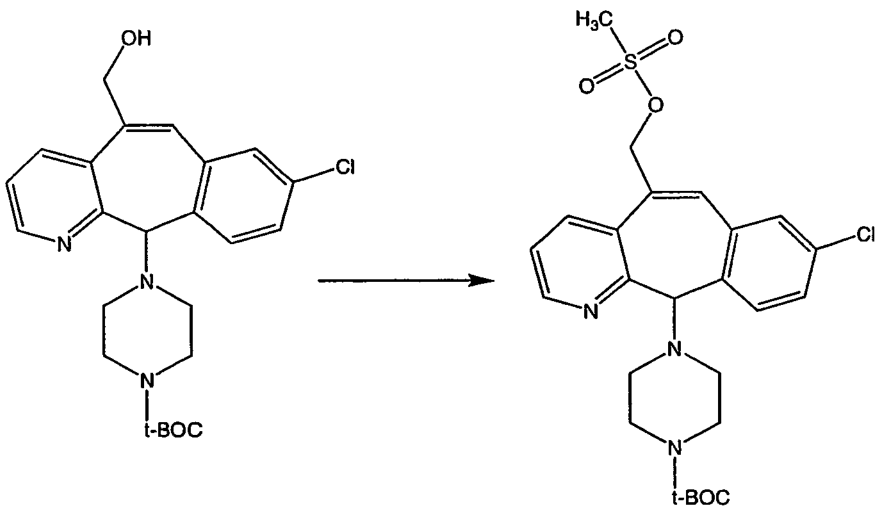

240 241

The crude product (240) from Step H above (200 mg; 0.45 mmol) was taken up in $CH_2Cl_2$ (2 ml) and treated with triethyl amine (126 ml; 0.91 mmol) followed by methanesulfonyl chloride (35 ml; 0.45 mmol). The reaction stirred over night at room temperature. The mixture was diluted with $CH_2Cl_2$ and quenched with sat. aqueous NaCl. The organic layer was dried over $MgSO_4$, filtered and concentrated to afford compound (241).

EXAMPLE 91

Preparation of Compound (242)

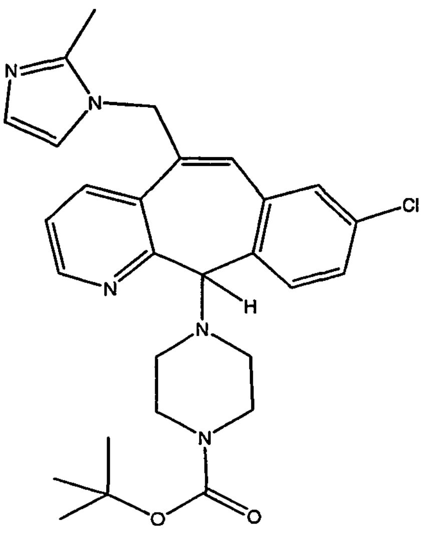

242

The mesylate compound (241) from Preparative Example 23, Step I above (230 mg; .442 mmol) was reacted in the same manner as Preparative Example 20, Step C. Purification of the crude product was accomplished by preparative TLC plates eluting with 95:5 $CH_2Cl_2$/MeOH($NH_3$) followed by 1:1 EtOAc:Hexanes to afford the title compound as a light tan solid (242) 105-116˚C (dec) $MH^+$ 506.

PREPARATIVE EXAMPLE 24

A. Preparation of Compound (243)

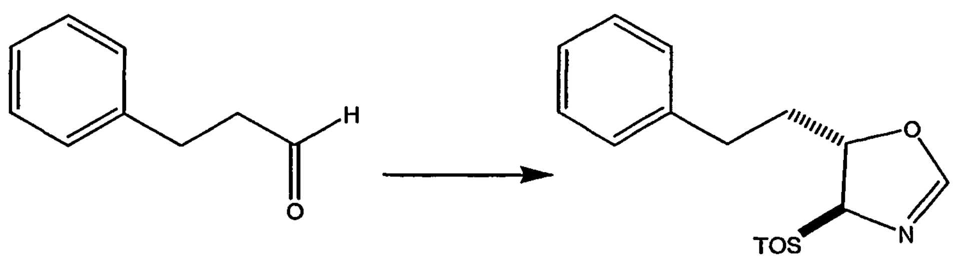

243

NaCN and 3-Phenylpropionaldehyde (ACROS) were dried overnight under vacuum. The aldehyde was then passed through activated $Al_2O_3$. Tosylmethyl isocyanide (5 g, 25.6 mmol) (ACROS) and dry 3-Phenylpropionaldehyde (3.36 g; 25.1 mmol) were combined in EtOH (42 ml) and stirred for 5 minutes. To the turbid mixture was added the dry NaCN (1.23 g; 25.1 mmol). An exothermic reaction was observed and after 5 minutes TLC showed consumption of starting material. The reaction was transferred to a sealed tube and used directly in the next experiment.

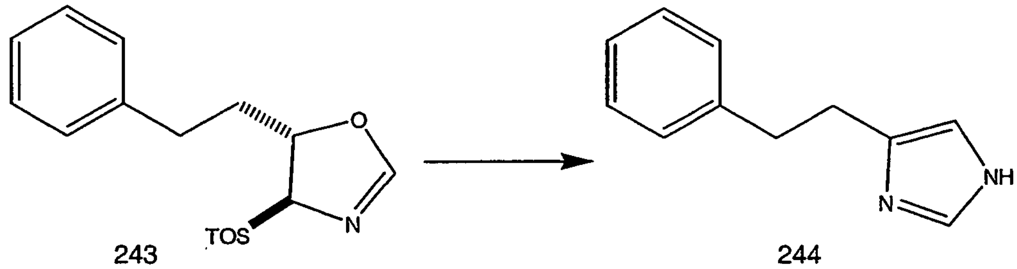

243 244

B. The crude product (243) from Step A above (25 mmol), was diluted up to 65 ml total volume with EtOH. To this mixture was added 7N $NH_3$ in MeOH (100 ml) and the reaction was heated to 90˚C over night (20 hr). The reaction was allowed to cool to room temperature and stirred for 2 hr then concentrated to dryness. The crude product was purified by flash silica column chromatoghraphy eluting with a gradient of 1-5% MeOH(sat. $NH_3$)/$CH_2Cl_2$ (244).

PREPARATIVE EXAMPLE 25

Preparation of Compound (245)

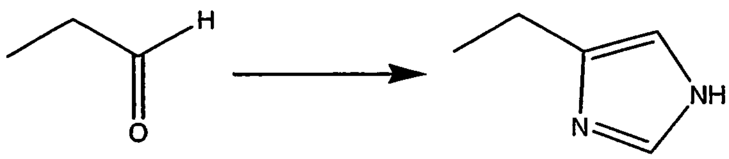

245

Propionaldehyde (1.5 g; 25.11 mmol) (ACROS) and tosylmethyl isocyanide (5 g; 25.6 mmol) were reacted in the same manner as Preparative Example 24 above to afford the title compound (245).

PREPARATIVE EXAMPLE 26

Compound (246) (+) isomer

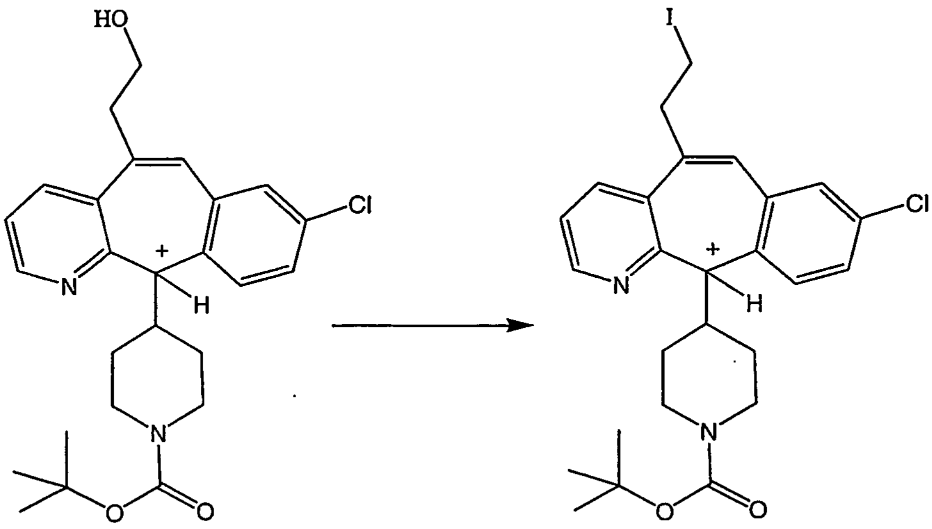

(+)67 246

The (+) isomer of compound (67) from Preparative Example 6 isolated by chiral AD column chromatography was further reacted as in Preparative Example 6 to obtain compound (246).

EXAMPLE 92 AND 93

PREPARATION OF COMPOUNDS (247) AND (248).

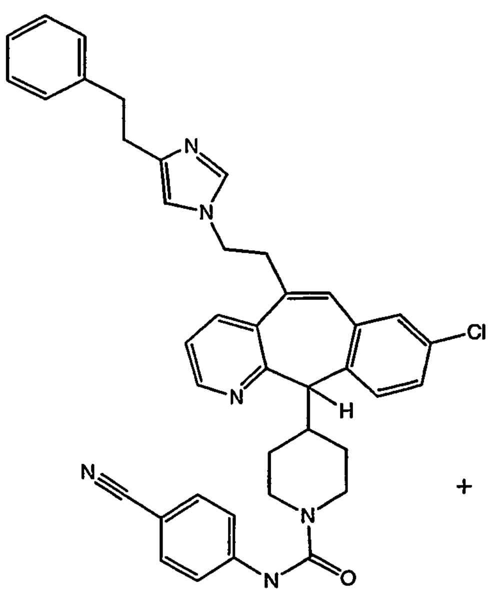

+

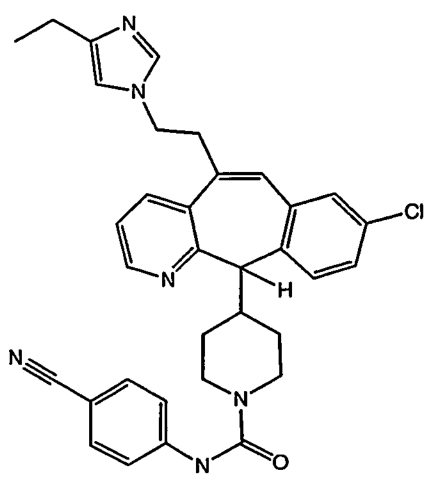

247 248

Compound (246) from Preparative Example 26 above was reacted in the same manner as Examples (22), (25) and (29) using the appropriate imidazole or isocyanate respectively to afford the title compounds (247) and (248).

EXAMPLES 94-96

Preparation of Compounds (249), (250) AND (251)

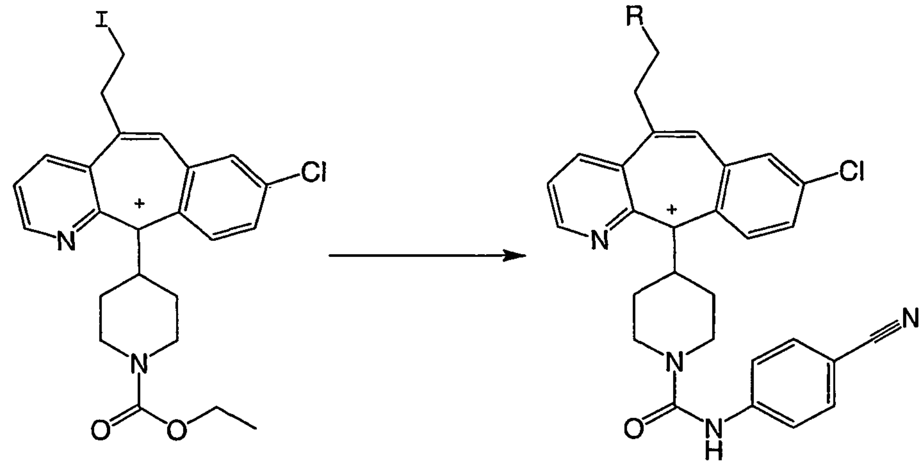

249-251

In a similar manner as Preparative Example 26 above, the (+) isomer of the carbamate was obtained and reacted in essentially the same manner as Examples 92 and 93 substituting with the appropriate imidazoles, to provide compounds (249)-(251), shown in the table below.

| Ex. # | R= | Cmp. # | Phys. Data |
|---|---|---|---|
| 94 | 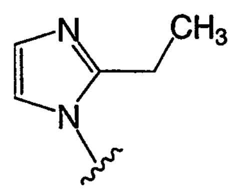 | 249 | mp 133.2-144.3°C dec. MH(+) 577.14 |
| 95 | 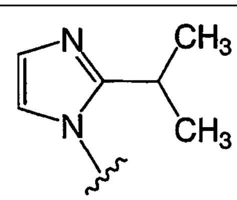 | 250 | mp 132.1-143.8°C dec. MH(+) 591.16 |
| 96 | 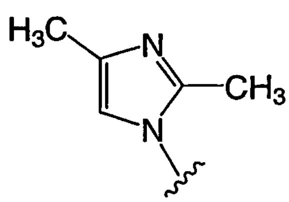 | 251 | mp 134.1-144.9°C dec. MH(+) 563.10 |

EXAMPLES 97-101

Preparation of Compounds (252), (253), (254), (255) AND (256).

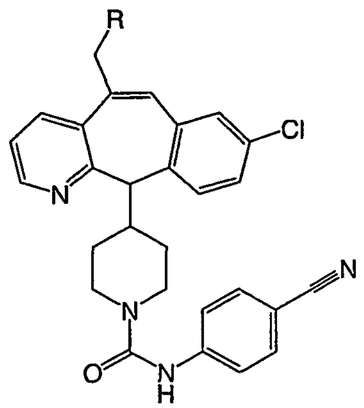

252-256

In essentially the same manner as in Preparative Example (20) and Example (29), the following compounds were prepared:

| EX. | R= | # | PHYS. DATA |
|---|---|---|---|
| 97 | 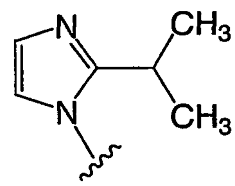 | 252 | mp 148-159°C dec. MH(+) 577. |
| 98 | 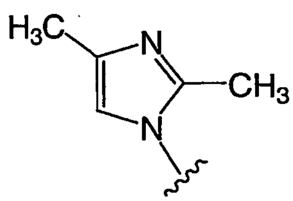 | 253 | mp 134-142°C dec. MH(+) 563. |
| 99 | 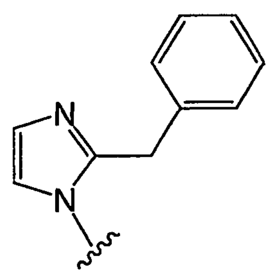 | 254 | mp 90-102°C dec. MH(+) 625. |
| 100 | 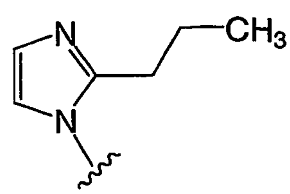 | 255 | mp 126-139°C dec. MH(+) 577. |
| 101 | 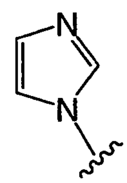 | 256 | mp 151-164°C dec. MH(+) 535. |

EXAMPLE 102

Preparation of Compound (257)

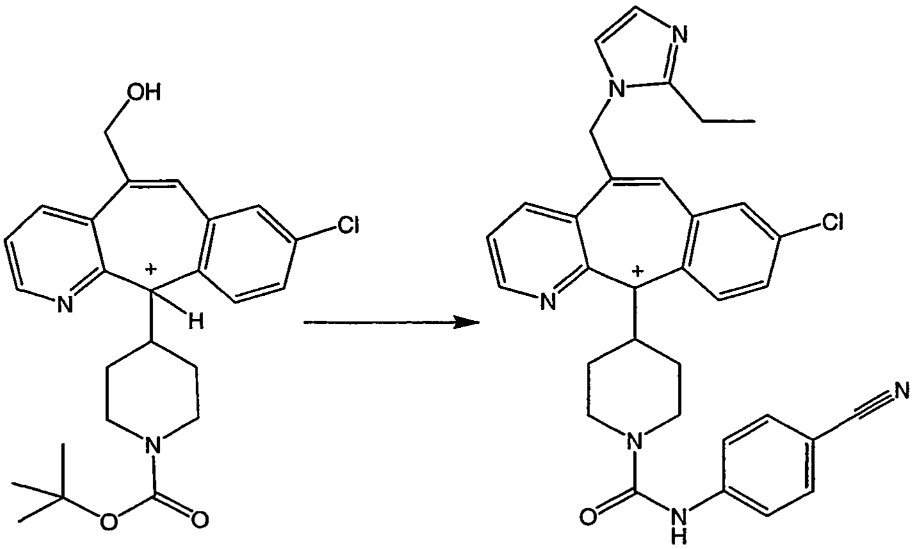

218 257

The (+) isomer of compound (218) obtained in essentially the same manner as Preparative Example (22), was further reacted in the same manner as in Preparative Example (6), Steps E and F, Examples (21), (23) and (29) sustituting with 2-Ethyl imidazole in Ex. (21) to afford the title compound (257). (146-157˚C dec.), $MH^+$ 564

PREPARATIVE EXAMPLE 27

Compounds (258A) AND (258B).

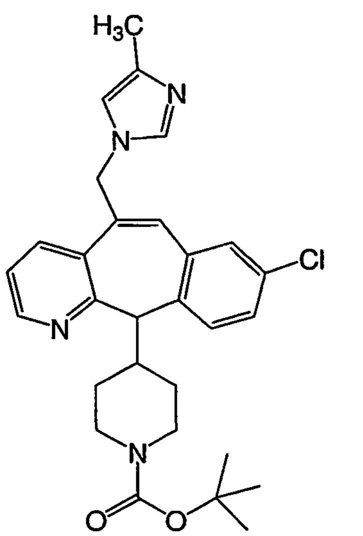

+

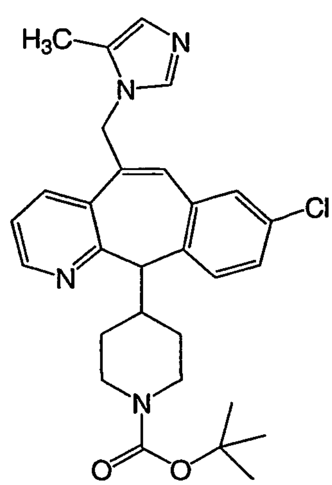

258A 258B

In essentially the same manner as Preparative Example (20), substituting 4-methylimidazole, compound (258) was prepared as a mixture of 4 and 5 substituted imidazole derivatives. This mixture was then reacted in a similar manner as Example 35 and the isomers separated (258A) and (258B).

EXAMPLE 103

Preparation of Compound (259)

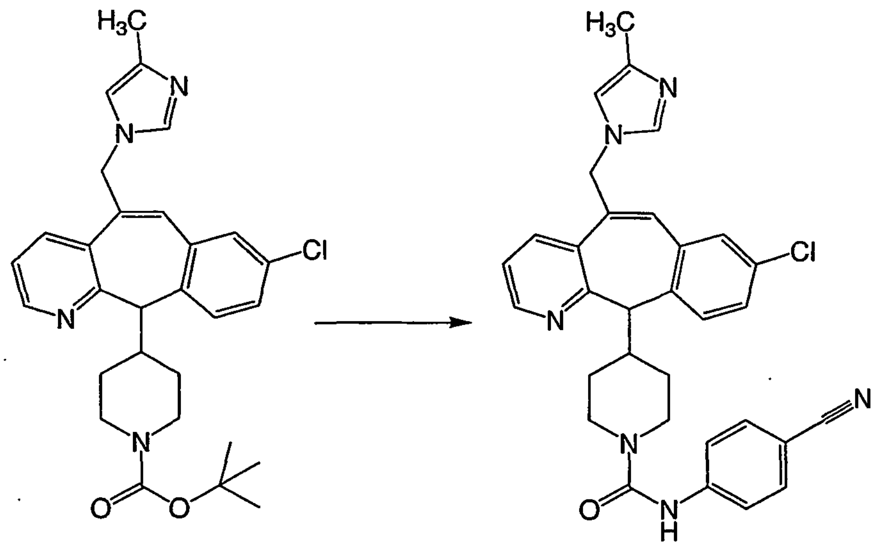

258A 259

The pure 4-methyl imidazole isomer (258A) was reacted as in Preparative Example 20, Step D, and Example (29) to afford the title compound as a white solid (259). (128-138°C dec.) $MH^+$ 549

EXAMPLE 104

Preparation of compound mixture (260a) AND

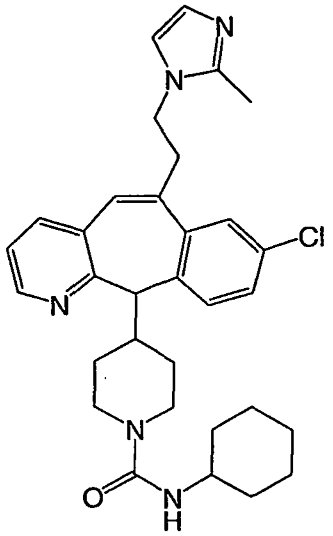

+

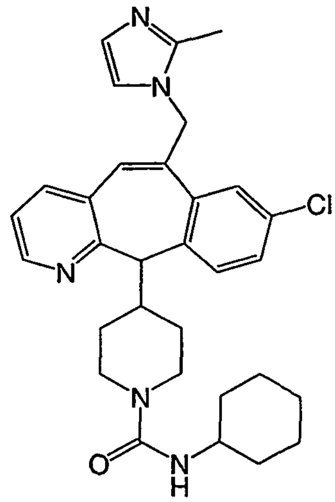

(260b).. 260a 260b

Step A Compound (108) from Preparative Example 9, Step E, was reacted with compound (64) from Preparative Example 6, Step A in essentially the same manner as in Preparative Example 6, Steps B-F, to afford a mixture of one and two methylene spaced iodo intermediates.

Step B The mixture of intermediates from Step A above was reacted in essentially the same manner as in Example 22 to afford a mixture of one and two methylene spaced imidazole derivatives.

Step C The mixture from Step B above was reacted in the same manner as Preparative Example 20, Step D, followed by a reaction with phenyl isocyante in the same manner as Example 15 to afford the title compound as a 1:1 mixture (260a) and (260b) (133-145˚C dec.); $MH^+$ 544

PREPARATIVE EXAMPLE 28

COMPOUND (261).

Step A. Ref: *Gazz. Chim. Ital.* (1972) **102**,189-195; *J. Org. Chem.* (1991) **56**,1166-1170.

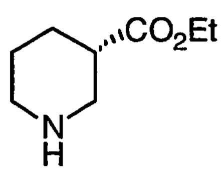

Ethyl nipecotate (70.16 g, 0.446 mmol) and D-tartaric acid (67 g, 1.0 eq) were dissolved in hot 95% EtOH (350 mL). The resulting solution was cooled to room temperature and filtered and the crystals washed with ice-cold 95% EtOH. The crystals were then recrystallized from 95% EtOH (550 mL) to give the tartrate salt (38.5g, 56% yield). The salt (38.5g) was dissolved in water (300 mL) and cooled to 0 ˚C before neutralizing with 3M NaOH. The solution was extracted with $CH_2Cl_2$ (5 X 100 mL) and the combined organics dried over $Na_2SO_4$ and concentrated under reduced pressure to give a clear oil (19.0g, 89% yield). CIMS: $MH^+$= 158.

Step B

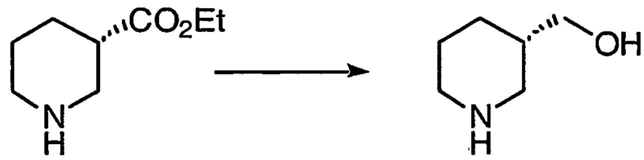

LAH (118 mL, 1.0 M in $Et_2O$, 1.0 eq.) was added to a solution of the product from Step A (18.5g, 0.125 mmol) in THF (250 mL) at 0 ˚C over 20 minutes. The resulting solution was warmed slowly to room temperature and then heated at reflux 2 hours. The reaction was cooled to room temperature and quenched by the slow addition of saturated $Na_2SO_4$. The resulting slurry was dried by the addition of $Na_2SO_4$, filtered through Celite and concentrated to give a colorless oil (13.7g, 98% crude yield). CIMS: $MH^+$=116; $[\alpha]^{20}_D$= -8.4˚ (5.0 mg in 2 mL MeOH).

Step C

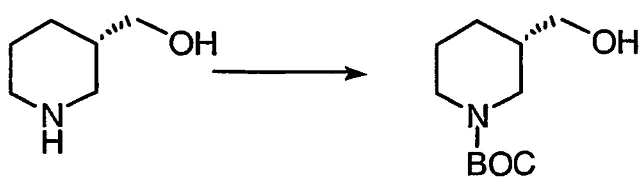

The product of Step B (13.6g, 0.104 mmol) was dissolved in MeOH (100 mL) and $H_2O$ (100 mL) di-tert-butyl dicarbonate (27.24, 1.2 eq.) was then added portionwise keeping the pH >10.5 by the addition of 50% NaOH. The reaction mixture was stirred at room temperature an additional 2.5 hours and concentrated *in vacuo.* The residue was diluted with $H_2O$ (350 mL) and extracted with $CH_2Cl_2$ (3 X 150 mL). The combined organics were dried over $Na_2SO_4$, filtered, and concentrated under reduced pressure. The crude product was purified by flash chromatography using a 50% EtOAc in hexanes solution as eluent to give a white solid (12.13g, 48% yield). FABMS: $MH^+$= 216; $[\alpha]^{20}_D$= +15.2 (5.0 mg in MeOH).

Step D

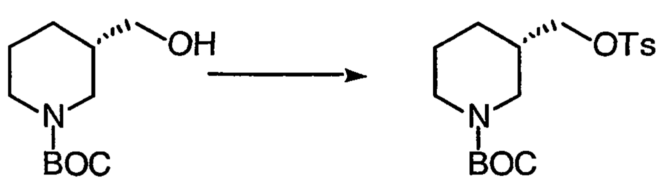

p-Toluenesulfonyl chloride (12.75g, 1.2 eq.) was added portionwise to a solution of the product from Step C (12.00g, 55.74 mmol) in pyridine (120 mL) at 0 ˚C. The resulting solution was stirred at 0 ˚C overnight. The reaction mixture was diluted with EtOAc (300 mL) and washed with cold 1N HCl (5 X 300 mL), saturated $NaHCO_3$ (2 X 150 mL), $H_2O$ (1 X 100 mL), and brine (1 X 100 mL), dried over $Na_2SO_4$ and concentrated *in vacuo* to give a pale yellow solid (21.0g, 100% crude yield). FABMS: $MH^+$= 370.

Step E

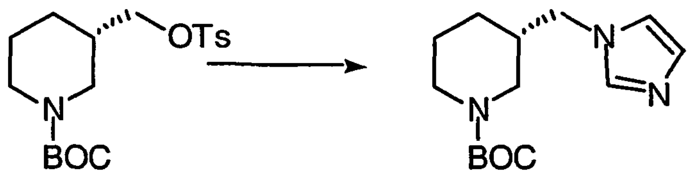

The product of Step D (21.0g, 55.74 mmol) in DMF (300 mL) was treated with sodium imidazole (8.37 g, 1.5 eq.) and the resulting solution heated at 60 °C for 2 hours. The reaction mixture was cooled to room temperature and concentrated in vacuo. The residue was diluted with $H_2O$ (300 mL) and extracted with $CH_2Cl_2$ (3 X 150 mL). The combined organic layer was dried over $Na_2SO_4$, filtered, and concentrated. The crude product was purified by flash chromatography using a 7% MeOH in $CH_2Cl_2$ solution as eluent to give a pale yellow solid (7.25 g, 49% yield). FABMS: $MH^+$= 266; $[\alpha]^{20}_D$= +8.0 (5.0 mg in MeOH).

Step F

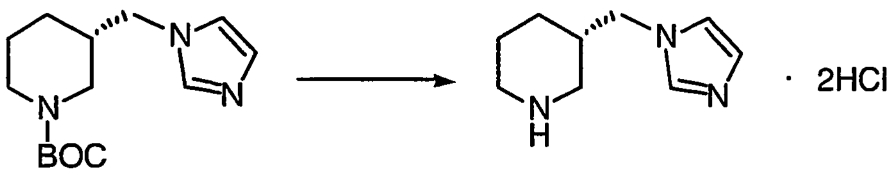

261

The product of Step E (5.50 g, 20.73 mmol) was stirred at room temperature in 4M HCl in dioxane (50 mL) overnight. The resulting solution was concentrated and the residue triturated with $Et_2O$ to give Compound (261) as a yellow solid (4.90 g, 99% yield). CIMS: $MH^+$= 166.

PREPARATIVE EXAMPLE 29

Compound (262)

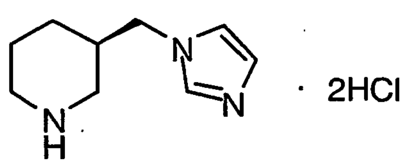

262

By essentially the same procedure set forth in Preparative Example 28 above, using L-tartaric acid instead of D-tartaric acid in Step A, Compound (262) was prepared.

PREPARATIVE EXAMPLE 30

PREPARATION OF COMPOUNDS (263) AND (264).

Step A 1N-tert-BUTOXYCARBONYL-3(R) AND 3(S) -(1H-IMIDAZOL-I-YL) METHYL) PYRROLIDINES

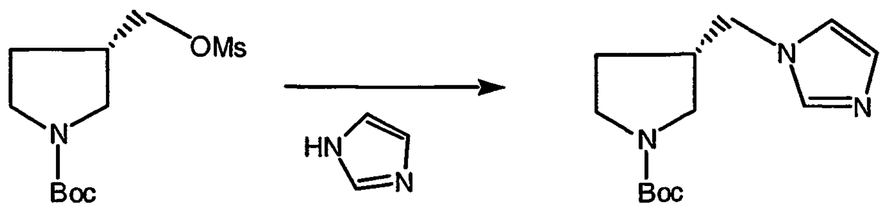

(R)

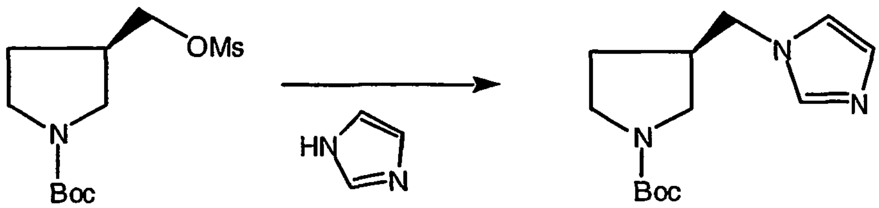

(S)

3(R)-(3-Methanesulfonyloxymethyl)pyrrolidine (J. Med. Chem. 1990, 33, 77-77) (0.993g, 3.56 mmoles) was dissolved in anhydrous DMF (25 mL) and sodium imidazole (0.6g, 10 mmoles) was added. The mixture was heated at 60° C for 2 h and then evaporated to dryness. The product was extracted with $CH_2Cl_2$ and washed with brine. The $CH_2Cl_2$ extract was evaporated to dryness to give the titled compound (263) (1.1409g, 100%), ESMS: FABMS (M+1) = 252; $^1$HNMR ($CDCl_3$) 1.45 (s, 9H), 1.5-1.7 (m, 1H), 1.9 - 2.1 (m, 1H), 2.5-2.7 (m, 1H), 3.0-3.2 (m, 1H), 3.3- 3.6 (m, 2H), 3.9 (dd, 2H), 6.9 (s, 1H), 7.1 (s, 1H), 7.45 (s, 1H)

In a similar manner, the (S) isomer was prepared from 3(S)-(3-Methanesulfonyloxymethyl)pyrrolidine (0.993g, 3.56 mmol) to give the title compound (1.14 g, 100%).

Step B 3(R) AND 3(S)-(1H-IMIDAZOL--1-YL)METHYL] PYRROLIDINES

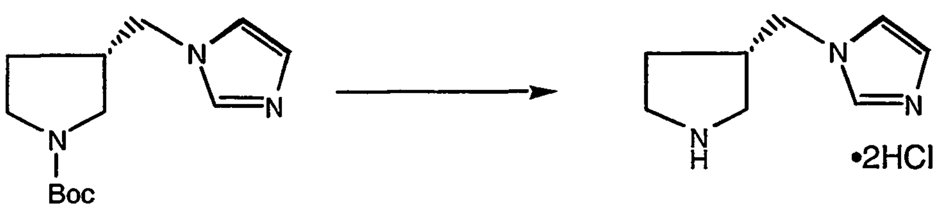

(263)

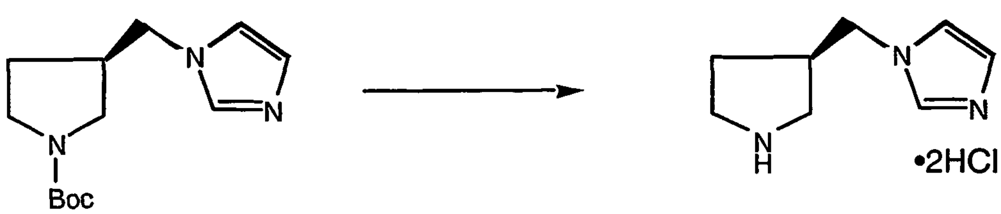

(264)

The (R) product (0.48g, 1.91 mmoles) from Step A was stirred in 4N HCl in dioxane (10 mL) for 2h and then evaporated to dryness to give the title compound (263) as the HCl salt.

In a similar manner the (S) isomer was prepared to give compound (264) as the HCl salt.

PREPARATIVE EXAMPLE 31

Compounds (265) AND (266).

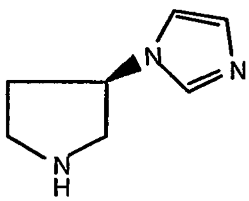

(R)

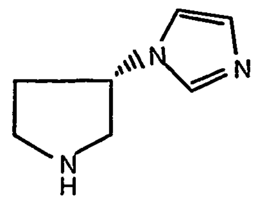

(S)

Step A 1N-BENZYL-3-(R) AND (S)-METHANESULFONYLOXY)-PYRROLIDINES

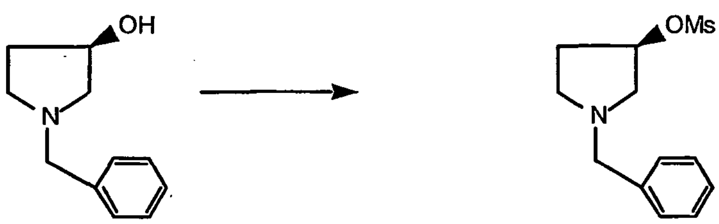

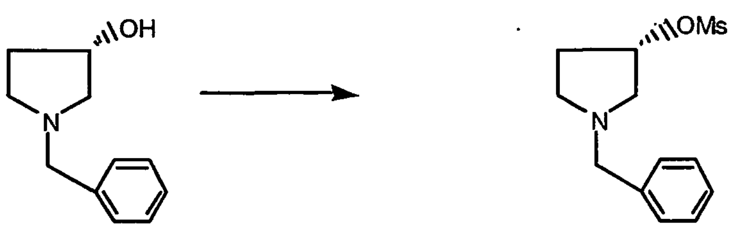

1N-Benzyl-3(R)-hydroxy-pyrrolidines (5g, 28.21 mmol) and triethylamine (7.86 mL, 56.35 mmol) were dissolved in $CH_2Cl_2$ (50 mL) and the mixture was stirred under nitrogen at 0°C. Methanesulfonylchloride (2.62 mL, 33.87 mmol) was added and the solution was stirred at room temperature for 2 h. The solution was diluted with $CH_2Cl_2$ and washed with saturated aqueous sodium bicarbonate, water and dried ($MgSO_4$), filtered and evaporated to dryness to give the (R) title compound (7.2g, 96.4 %). FABMS (M+1) = 256; $^1$HNMR ($CDCl_3$) 2.2 (m, 1H), 2.3 (m, 1H), 2.52 (m, 1H), 2.7-2.85 (m, 3H), 2.95 (s, 3H), 3.65 (q, 2H), 5.16 (m, 1H), 7.3 (s, 5H).

In a similar way the (S) isomer was prepared from 1N-Benzyl-3(S)-hydroxy-pyrrolidines (5g, 28.21 mmoles) to give the (S) title compound (7.15g, 98%).

Step B 1N-BENZYL-3-(S) AND (R)-(1H-IMIDAZOL-1-YL)-PYRROLIDINES

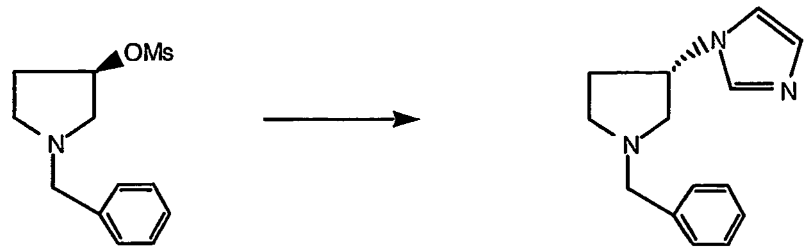

(R) (S)

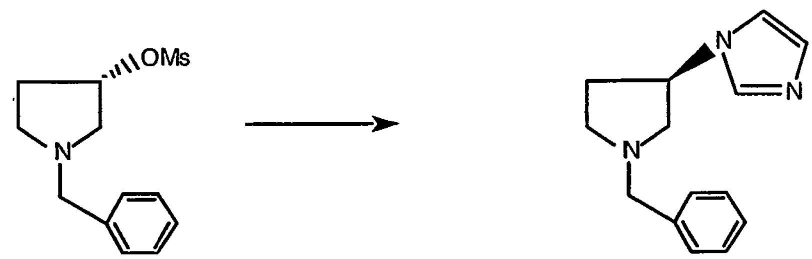

(S) (R)

A solution of the (R) product from Step A (2.0g, 7.84 mmoles) was added to a stirred solution of imidazole (1.1 g, 16.17 mmoles) in DMF (25 mL) under nitrogen atmosphere. The mixture was stirred at 60 ˚C for 16 h. DMF was evaporated *in vacuo.* The resulting crude product was extracted with $CH_2Cl_2$ and the extract was successively washed with water and brine, and the $CH_2Cl_2$ was evaporated to leave the title residue which was chromatographed on silica gel using 3% (10% conc $NH_4OH$ in methanol)- $CH_2Cl_2$ as eluant to give the title compound (0.95 g, 50.56%). FABMS (M+1) = 228.

In a similar fashion the other isomer was prepared.

Step C 3-(R) AND (S)-(1H-IMIDAZOL-1-YL)-PYRROLIDINES

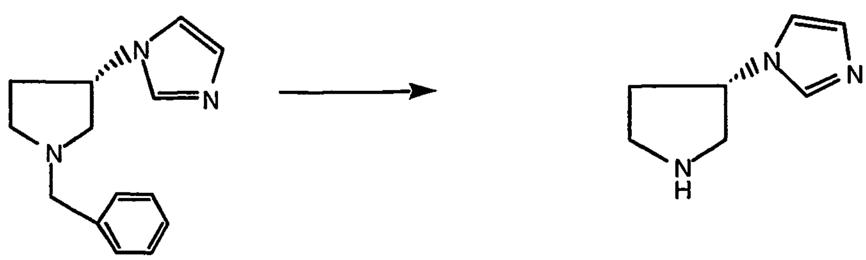

(266)

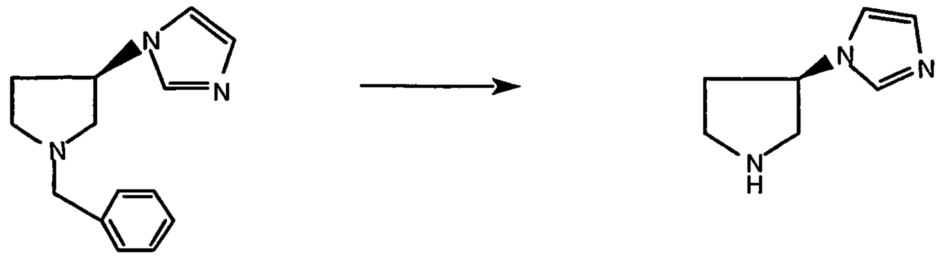

(265)

A mixture of the (S) product (0.95 g) from Step B and 10% Pd on carbon (0.5 g) in EtOH (20 mL) was shaken at 50 psi under an atmosphere of hydrogen for 24h. The catalyst was filtered and the solvent removed to give the title compound (266) (0.522 g, 99.9%).

In a similar manner the (R) isomer was prepared from 1.0 g of the starting (R) product from Step B and 10% Pd on carbon (0.6 g) to give compound (265) in 99% yield.

PREPARATIVE EXAMPLE 32

Compounds (267) AND (268)

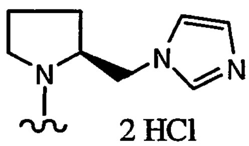

2 HCl

267

.

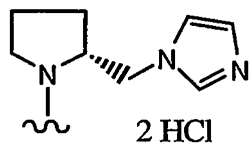

2 HCl

268

By essentially the same procedure set forth in Preparative Example 31 above, beginning with L or D-prolinol, the title compounds (267) and (268) were prepared.

EXAMPLE 105

Preparation of Compound (269).

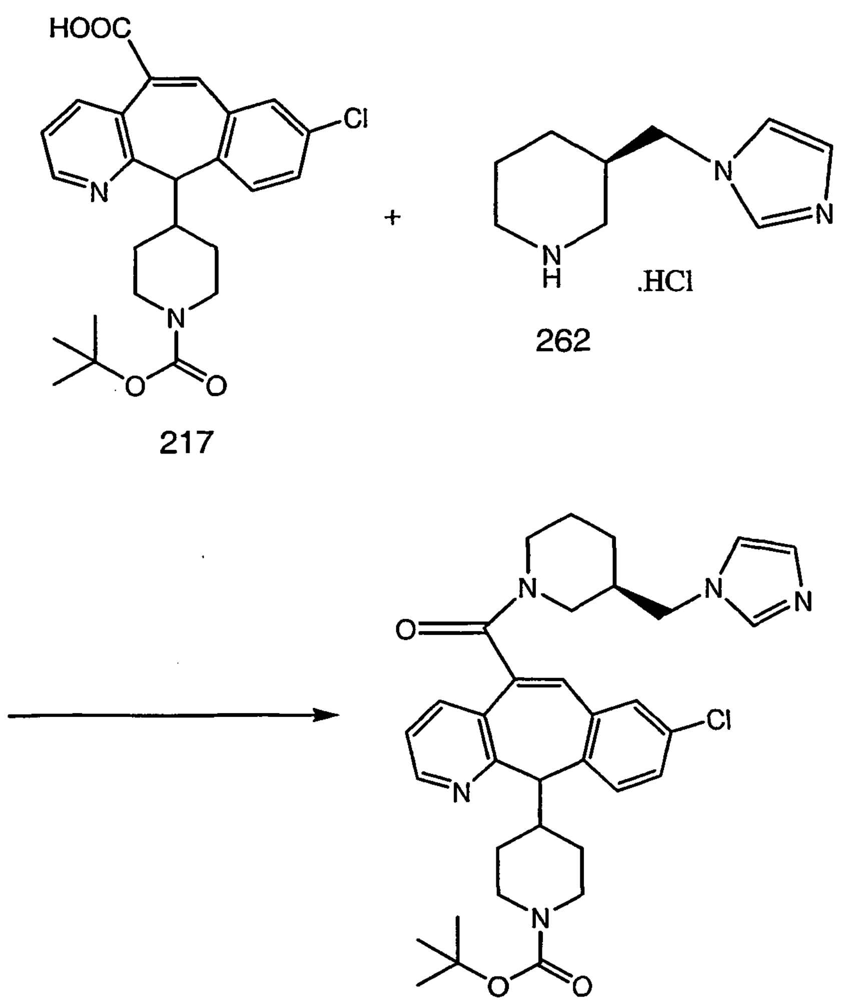

217

262

269

Compound (217) from Preparative Example 19 (0.227g, 0.499 mmol) was added to a solution of Compound (262) from Preparative Example 29 (0.131 g, 0.,649 mmol), DEC (0.249 g,1.3 mmol), HOBT (0.175 g, 1.3 mmol) and NMM (0.5 mL) in DMF (25 mL). The resulting solution was stirred at room temperature for 24 hours. The reaction mixture was diluted with $H_2O$ until precipitation ceased and the slurry was filtered. The precipitate was diluted with $CH_2Cl_2$, washed with brine, dried over $Na_2SO_4$ and concentrated. The crude product was purified by chromatography using a 5% (10% $NH_4OH$ in MeOH) solution in $CH_2Cl_2$ as eluent to give the title compound (269) (0.184 g, 62 % yield).

EXAMPLES 106-111

Preparation of Compounds (270)-(275). Using the appropriate amine from the Preparative Examples 28-32, and following essentially the same procedure as in Example 105 above, the following compounds were prepared:

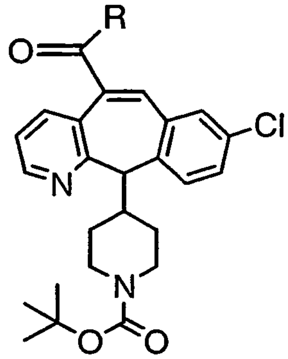

| EX. | R= | Compound # | PHYS. DATA |
|---|---|---|---|
| 106 | 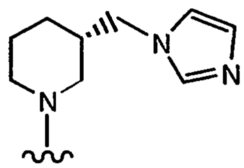 | 270 | $MH^+$=603 |
| 107 | 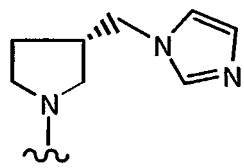 | 271 | $MH^+$=589 |
| 108 | 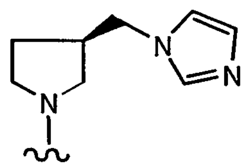 | 272 | $MH^+$=589 |
| 109 | 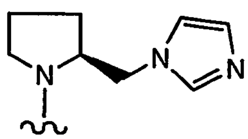 | 273 | $MH^+$=589 |
| 110 | 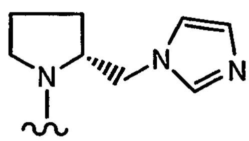 | 274 | $MH^+$=603 |
| 111 | 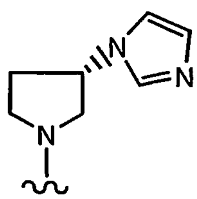 | 275 | $MH^+$=603 |

EXAMPLE 112

Preparation of Compound (276)

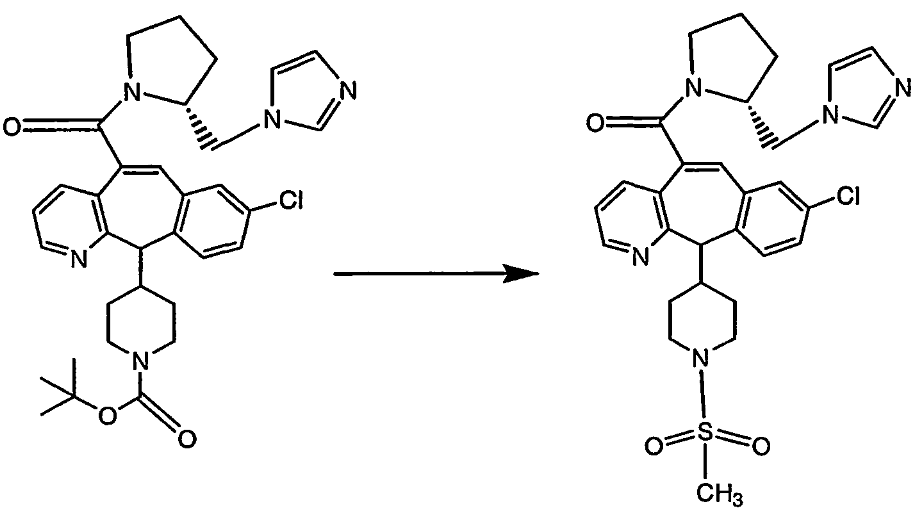

274 276

Compound (274) from Example 110 above (0.125g, 0.213 mmoles) in $CH_2Cl_2$ (50 mL) was stirred with TFA (10 mL) at room temperature overnight. The reaction mixture was evaporated to give the TFA salt (0.28g) which was redissolved in $CH_2Cl_2$ (50 mL) and cooled (ice water bath). Triethyl amine (0.1 mL) followed by methane sulfonyl chloride (0.038 g, 0.319 mmoles) were added and the reaction mixture was stirred at room temperature overnight. The reaction mixture was washed with sodium bicarbonate and water. The organic layer was dried over $MgSO_4$ and evaporated to dryness to give the title compound (276) (0.05g, MH+=567)

EXAMPLE 113

Preparation of Compound (277)

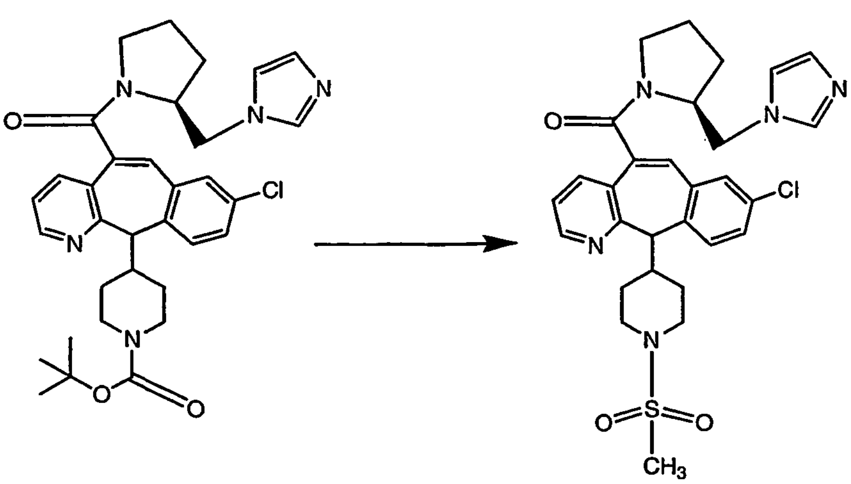

273 277

Starting with Compound (273) from Example 109 above and following essentially the same procedure as in Example 112 above, Compound (277) was prepared (MH+=567).

PREPARATIVE EXAMPLE 33

A. Compound (278)

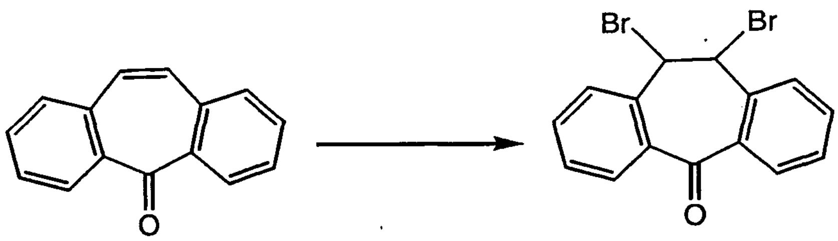

278

To a stirred solution of bromine (33.0g, 210 mmol) in $CCl_4$ (100 ml) was added a solution of dibenzosuberenone (37.0g, 179 mmol) in $CCl_4$ (200ml) at room temperature. The resulting solution was stirred at room temperature for 1.5 hours. The white crystals were collected by filtration to give the product (278) (60.12g, 92% yield, M+H=367).

B. Preparation of Compound (279)

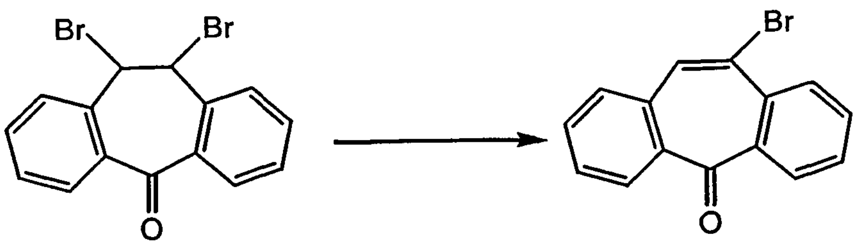

278 279

A solution of the di-bromo compound (278) from step A (60.0 g, 163 mmol) and NaOH (20.0 g, 491 mmol) in MeOH (500ml) was stirred and heated to reflux for 1.5 hours. The reaction mixture was then cooled to room temperature and stirred overnight. The mixture was evaporated to dryness then extracted with $CH_2Cl_2$-$H_2O$. The combined organic layer was dried over $MgSO_4$, filtered and evaporated to dryness to give a yellow solid (279) (46.34 g, 100% yield, M=285)

C. Preparation of Compound (280).

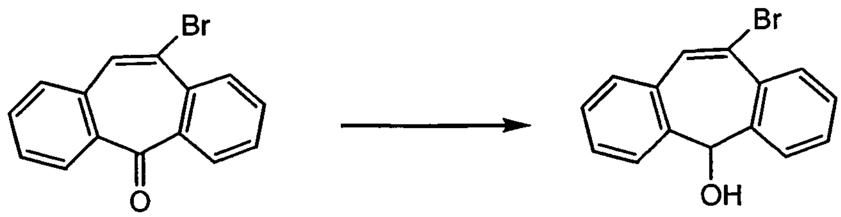

279 280

To a stirred solution of the mono-bromo compound (279) from step B (10.0 g, 35.07 mmol) in MeOH (200 ml) under nitrogen at 0 ˚C was added $NaBH_4$ (1.94 g, 51.2 mmol). The resulting solution was stirred at 0 ˚C for 1.5 hours, then evaporated, followed by extraction with $CH_2Cl_2$-$H_2O$. The combined organic layer was dried over $MgSO_4$, filtered, and evaporated to dryness to give a white solid (280) (10.3 g, 100%, M=287).

D. Preparation of Compound (281).

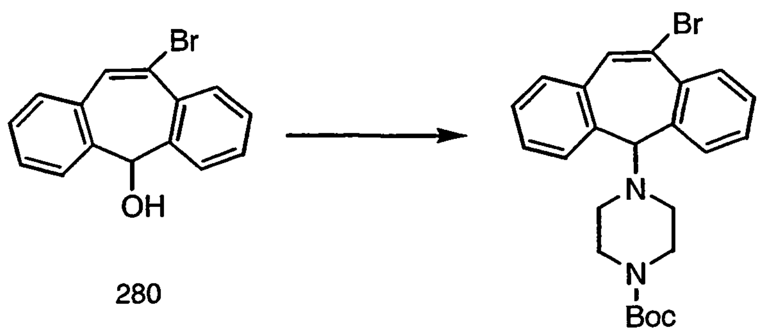

280

281

To a stirred solution of the alcohol (280) from Step C (10.0 g, 34.8 mmol) in $CH_2Cl_2$ (200 ml) at 0 ˚C was added 2,6-lutidine (14.9 g, 139.3 mmol) and thionyl chloride (8.28 g, 69.66 mmol). The resulting solution was warmed to room temperature and stirred overnight. The solution was then poured onto 0.5N NaOH solution, followed by extraction with $CH_2Cl_2$. The combined aqueous layer was dried over $Na_2SO_4$, filtered, and concentrated to dryness to give a crude brown oil (15.5 g). To a solution of this crude oil (15.5 g) in acetonitrile (200 ml) was added 2,6-Bis (dimethyl)-1-methyl piperidine (10.81 g, 69.66 mmol) and N-Boc piperidine (6.49 g, 34.83 mmol). The resulting mixture was warmed to 65 ˚C overnight. The mixture was evaporated to dryness, followed by extraction with $CH_2Cl_2$/saturated $NaHCO_3$. The combined organic layer was dried over $Na_2SO_4$, concentrated and purified by column chromatography on silica gel, eluting with 5% EtOAc/95% Hexane to give the protected N-Boc compound (281) (5.68 g, 36% yield, $MH^+$=455).

E. Preparation of Compound (282).

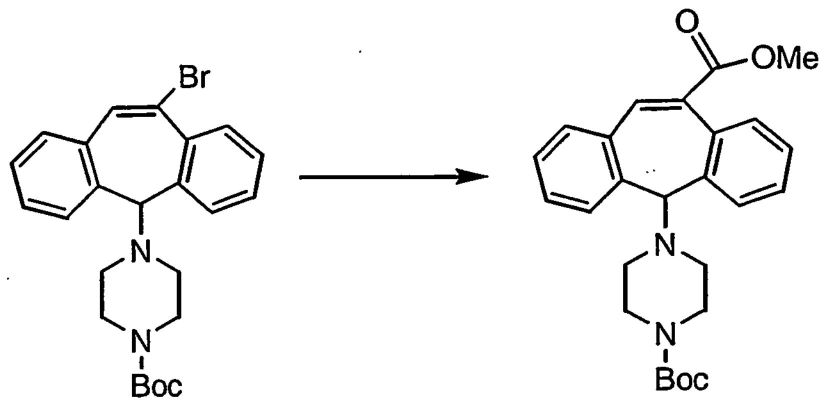

281 282

To a solution of the N-Boc compound (281) from Step D (4.0 g, 8.78 mmol) in anhydrous toluene (100 ml) and methanol (20 ml) was added triphenylphosphine (1.15 g, 4.39 mmol), DBU (1.81 g, 11.9 mmol) and palladium (II) chloride (0.15 g, 0.88 mmol). The resulting mixture was purged with carbon oxide at 80 psi to 100 psi and heated to 78 ˚C-82 ˚C for 5 hours, followed by stirring at room temperature for overnight. The solution was then extracted with EtOAc. The combined organic layer was washed with water, brine, dried over $Na_2SO4$, filtered, evaporated and the crude product was purified by column chromatography on silica gel, eluting with 10% EtOAc/90% Hexane to give the ester compound (282) (2.1 g, 55% yield, $MH^+$=435).

F. Preparation of Compound (283).

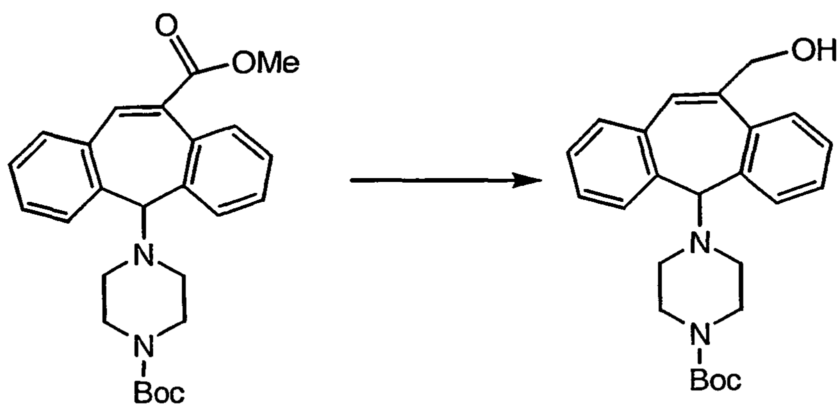

282 283

To a stirred solution of the ester compound (282) from Step E (1.2 g, 2.77 mmol) in THF (15 ml) at 0 ˚C was added a 1M solution of DIBAL (16.62 ml, 16.62 mmol). The resulting solution was stirred at room temperature for 4 hours. To the solution was then added 10% potassium sodium tartarate, followed by extraction with EtOAc. The combined organic layer was dried over $Na_2SO4$, filtered, and evaporated to give a solid (283) (1.1 g, 100% yield, $MH^+$=406).

G. Preparation of Compound (284).

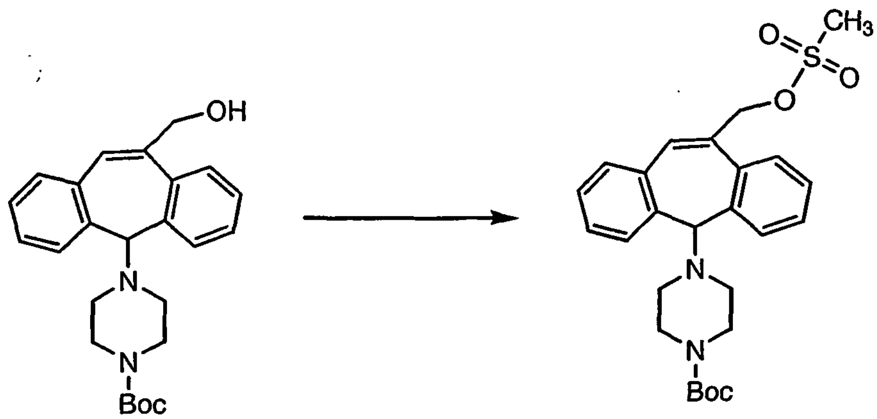

283 284

To a solution of the alcohol (283) from Step F (0.62 g, 1.52 mmol) in $CH_2Cl_2$ (15 ml) under nitrogen was added triethyl amine (0.64ml, 4.56mmol) and methane sulfonyl chloride (0.26 g, 2.29 mmol). The resulting solution was stirred at room temperature overnight. The mixture was washed with $NaHCO_3$ solution, dried over $Na_2SO4$, filtered and concentrated to dryness to give the mesylate compound (284) (0.53 g, 76% yield, M-$CH_3SO_3H$=389.1).

H. Preparation of Compound (285).

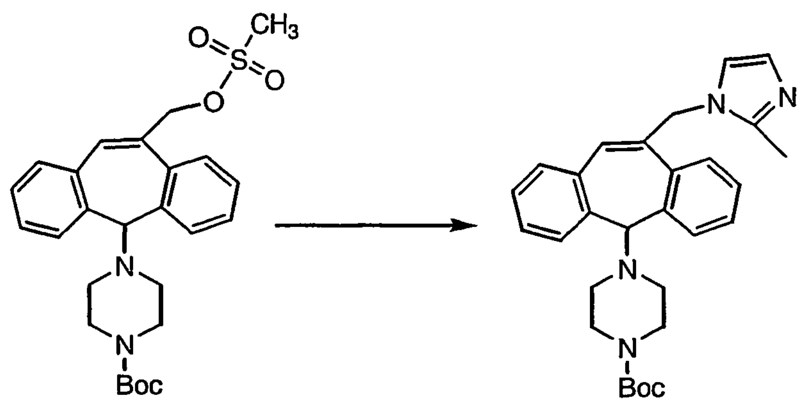

284 285

To a stirred solution of 1-methyl-imidazole (1.04 g, 12.7 mmol) in DMF (10 ml) under nitrogen, was added NaH (0.305 g, 12.7mmol). The resulting solution was stirred at room temperature for 15 minutes, followed by the addition of the mesylate compound (284) from step G (2.05 g, 4.23 mmol). The reaction mixture was stirred at room temperature overnight, then evaporated to dryness, and extracted with an EtOAc-$NaHCO_3$ solution. The combined organic layer was dried over $Na_2SO4$, concentrated and the crude product purified by silica gel column chromatography eluting with 2% MeOH/98% $NH_3$-$CH_2Cl_2$ to give the product (285) (0.775 g, 39% yield, $MH^+$=471).

I. Preparation of Compound (286).

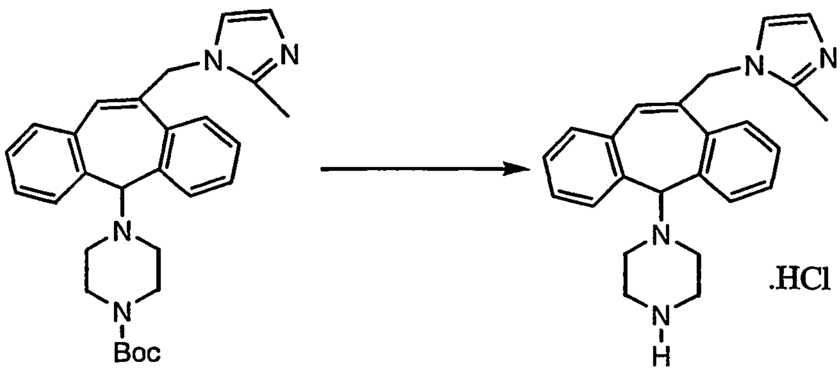

285 286

A solution of the product (285) from step H (0.3 g, 0.64 mmol) in 4M HCl in dioxane (40 ml) was stirred at room temperature for 3 hours and then concentrated to dryness to give the hydrochloride salt of the title product (286) (0.42 g, 100% yield, $MH^+$=371).

EXAMPLES 114 AND 115

Compounds (287) AND (288).

The racemic mixture of Preparative Example 33, Step H above was seperated into its pure isomers by HPLC, using a Chiral AD column eluting with 15% IPA/75% Hexane/0.2% DEA to afford the compounds in the table below:

| EX. # | PROCEDURE | R= | CMPD # | PHYS. DATA |
|---|---|---|---|---|
| 114 | Prep. Ex. 33, Steps A-H | BOC | 287 isomer 1 | MS $M^+$=471 |
| 115 | Prep. Ex. 33, Steps A-H | BOC | 288 isomer 2 | MS $M^+$=471 |

EXAMPLES 116-119.

Starting with the piperazine compound (286) from Preparative Example 33 Step I, and reacting it with the appropriate isocyanate or sulfonyl chloride, following essentially the same procedure as indicated in the table below, the following compounds were prepared:

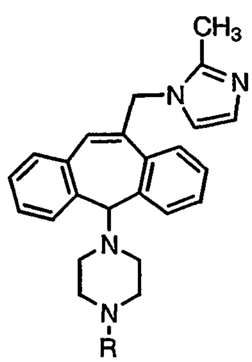

(286)

| EX. # | PROCEDURE | R= | CMPD # | PHYS. DATA |
|---|---|---|---|---|
| 116 | Example 13 | 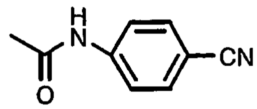 | 289 isomer 1 | MS $M^+$=515 |
| 117 | Example 13 | 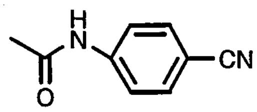 | 290 isomer 2 | MS $M^+$=515 |
| 118 | Example 24 | 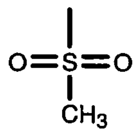 | 291 a isomer 1 | MS $M^+$=449 |
| 119 | Example 24 | 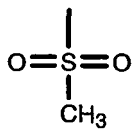 | 291 b isomer 2 | MS $M^+$=449 |

PREPARATIVE EXAMPLE 34

A. Preparation of Compound (292).

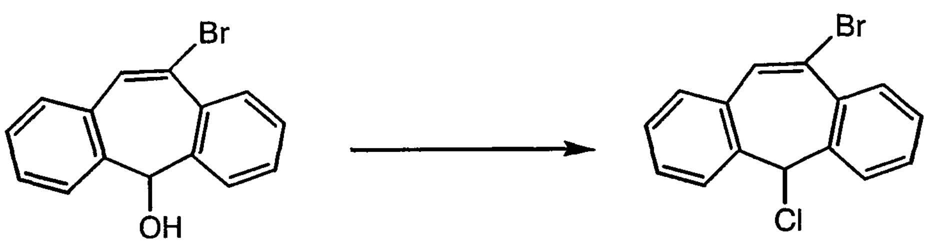

280

292

To a stirred solution of alcohol (280) from Preparative Example 33, Step C (30.0 g, 104.5 mmol) in $CH_2Cl_2$ (500 mL) under nitrogen at -20 ˚C was added thionyl chloride (106.7 mL, 1,46 mmol). The resulting solution was stirred at room temperature overnight and then evaporated to dryness. The crude mixtue was diluted with toluene (50 mL), followed by the addition of more $SOCl_2$ (106.7 mL) at room temperature. The resulting solution was heated to reflux for 2 hours until the reaction went to completion. The reaction mixture was then cooled to room temperature and concentrated to dryness to give a light brown solid (292) (35.67 g, 100% yield, M-BrCl=191).

B. Preparation of Compound (293).

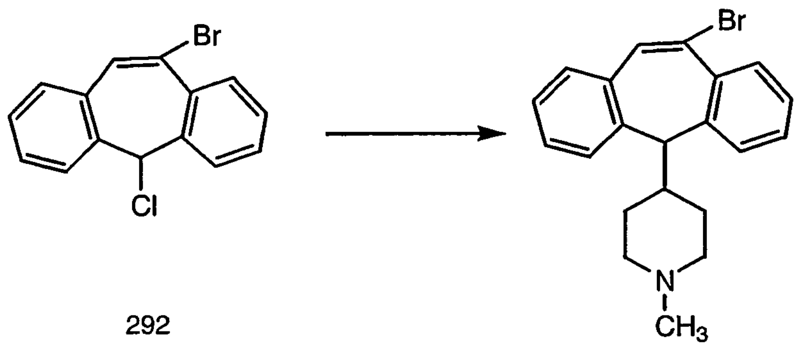

292 293

To a suspension of Mg (3.63 g) in anhydrous THF (95 mL) under nitrogen at room temperature was added 4-chloro-1-methyl piperidine (3 mL, 10% of the total amount) and one small crystal of iodine. The resulting solution was heated to reflux, followed by the addition of iodomethane (0.5 mL) and the remainder of the 4-chloro-1-methyl piperidine (27 mL). The reaction was stirred for one hour and then concentrated to dryness to give the crude Grignard reagent (0.8M).

To a stirred solution of the chloro compound (292) from Preparative Example 34, Step A (35.67 g, 116.7 mmol) in anhydrous THF (200 mL) under nitrogen at room temperature , was added dropwise the Grignard reagent (as obtained above) (0.8M, 146 mL, 116.7 mmol).The resulting solution was stirred at room temperature for 3 hours, followed by the extraction with EtOAc-$H_2O$. The combined organic layer was dried over $MgSO_4$, filtered and evaporated to dryness to give the product (293) (49.25 g, 100% yield, $MH^+$=368).

C. <u>Preparation of Compound (294).</u>

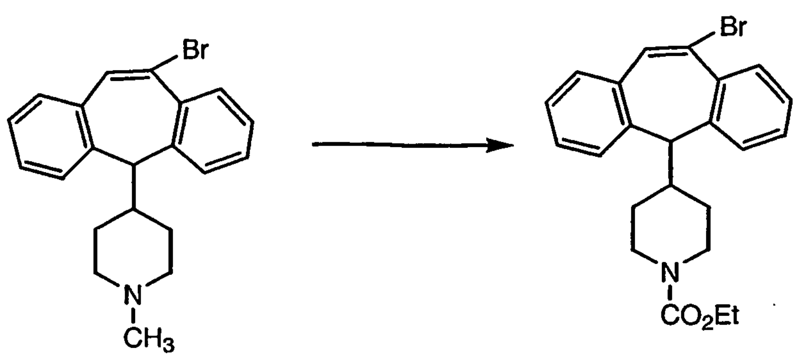

293 294

To a stirred solution of Compound (293) from Step B above (42.9 g, 116.5 mmol) in toluene (400 mL) under nitrogen was added triethylamine (49 mL, 349.5 mmol). The resulting solution was heated to refux, followed by the dropwise addition of ethyl chloroformate (126 g, 1165 mmol). Continued to heat the solution at the reflux temperature for 2 hours. The reaction was then stirred at room temperature overnight, followed by extraction with an EtOAc-1N NaOH solution. The combined organic layer was dried over $MgSO_4$, filtered, concentrated to dryness and the crude product purified by column chromatography on normal phase silica gel, eluting with 30% EtOAc/70% Hexane to give a light yellow solid (294) (2.99 g, 12% yield, $MH^+$=426.3).

D. <u>Preparation of Compounds (295a) and (295b).</u>

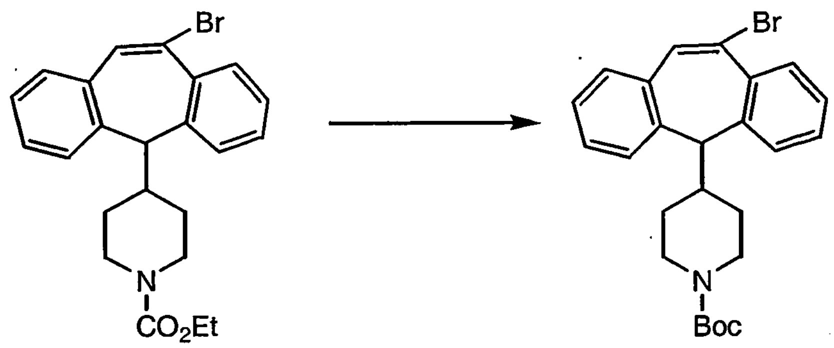

294 295a 295b

A solution of the ester (294) from step C above (3.34 g, 7.83 mmol) in 6N HCl (20 mL) was heated to reflux overnight. The reaction was cooled to room temperature and basified with $NH_4OH$ solution, followed by extraction with $CH_2Cl_2$. The combined organic layer was dried over $MgSO_4$, filtered, and evaporated to dryness to give a crude free piperidine (2.80 g, 100% yield, $MH^+$=534)

To the crude material (as obtained above) (2.77 g, 7.82 mmol) in 50% MeOH/1% $H_2O$ (200 mL) was added Di-tert-butyl dicarbonate (3.41 g, 15.64 mmol). The reaction mixture was adjusted to pH=9 and stirred at room temperature for 4 hours, evaporated to dryness then extracted with $CH_2Cl_2$-$H_2O$. The combined organic layer was dried over $MgSO_4$, filtered, concentrated to dryness and purified by HPLC, using chiral AD column, eluting with 15% IPA/75% Hexane/0.2% DEA to give the pure isomers of the N-Boc compounds (295a) and (295b) (3.42 g, 96% yield, $MH^+$=454).

E. Preparation of Compounds (296a) and (296b)

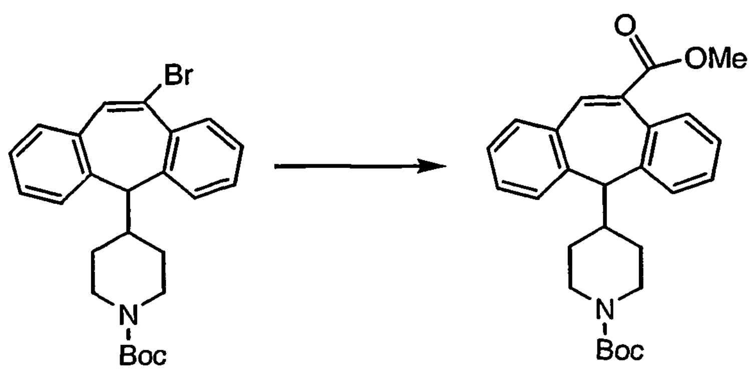

295 296

To a stirred solution of the pure (+) or (-) isomer of the N-Boc compound from Step D above (4.0 g, 8.78 mmol) in anhydrous toluene (100 mL) and methanol (20 mL) was added triphenyl phosphine (1.15 g, 4.39 mmol), DBU (1.81 g, 11.9 mmol) and palladium (II) chloride (0.15 g, 0.88 mmol). The resulting mixture was purged with carbon monooxide at 80 psi to 100 psi and heated to 78 °C-82 °C for 5 hours, followed by stirring at room temperature overnight. The solution was then extracted with EtOAc. The combined organic layer was washed with water, brine, dried over $Na_2SO4$, filtered, evaporated and purified by column chromatography on silica gel, eluting with 10% EtOAc/ 90% Hexane to give the ester (296a) or (296b) (2.1 g, 55% yield, $MH^+$=435).

F. Preparation of Compounds (297a) and (297b).

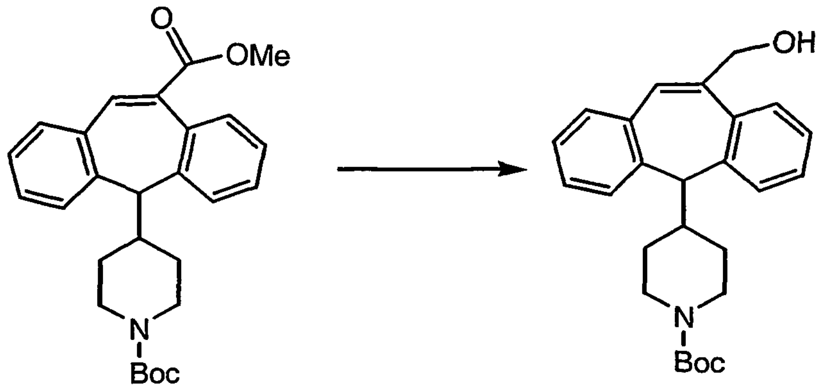

296 297

To a stirred solution of the (+) or (-) isomer of the ester from Step E above, (1.2 g, 2.77 mmol) in THF (15 mL) at 0 ˚C was added 1M solution of DIBAL (16.62 mL, 16.62 mmol). The resulting solution was stirred at room temperature for 4 hours. To the solution was then added 10% potential sodium tartarate, followed by extraction with EtOAc. The combined organic layer was dried over $Na_2SO_4$, filtered, and evaporated to give a solid (297a) or (297b) (1.1 g, 100% yield, $MH^+$=406).

G. Preparation of Compounds (298a) and (298b).

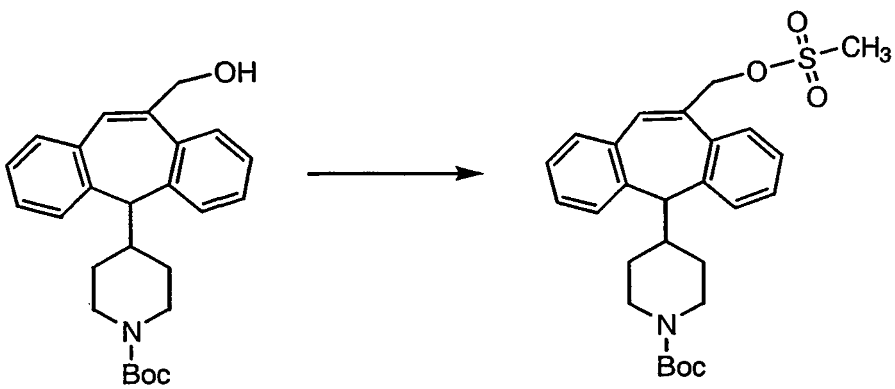

297 298

To a stirred solution of the (+) or (-) isomer of the alcohol from Step F, above (0.62 g, 1.52 mmol) in $CH_2Cl_2$ (15 mL) under nitrogen was added triethyl amine (0.64 mL, 4.56 mmol) and methane sulfonyl chloride (0.26 g, 2.29 mmol). The resulting solution was stirred at room temperature for overnight. The mixture was washed with $NaHCO_3$ solution, dried over $Na_2SO4$, filtered and concentrated to dryness to give the mesylate compound (298a) or (298b) (0.53 g, 76% yield, M-$CH_3SO_3H$=389.1).

H. Preparation of Compounds (299a) and

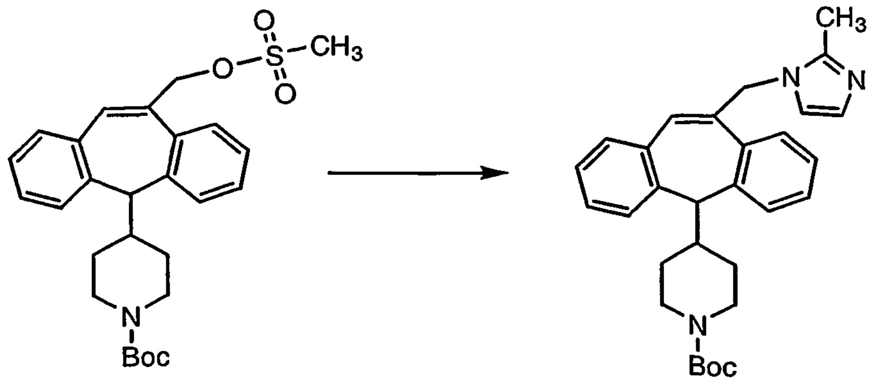

(299b). 298 299

To a stirred solution of 1-methyl-imidazole (1.04 g, 12.7 mmol) in DMF (10 mL) under nitrogen, was added NaH (0.305 g, 12.7 mmol). The resulting solution was stirred at room temperature for 15 minutes, followed by the addition of the (+) or (-) isomer of the mesylate compound (299) from Step G above (2.05 g, 4.23 mmol). The reaction mixture was stirred at room temperature overnight then evaporated to dryness, followed by extraction with an EtOAc-$NaHCO_3$ solution. The combined organic layer was dried over $Na_2SO4$, concentrated and the crude product was purified by silica gel column chromatography, eluting with 2% MeOH/98% $NH_3$-$CH_2Cl_2$ to give the product (299a) or (299b) (0.775 g, 39% yield, $MH^+$=471).

I. Preparation of Compounds (300a and 300b).

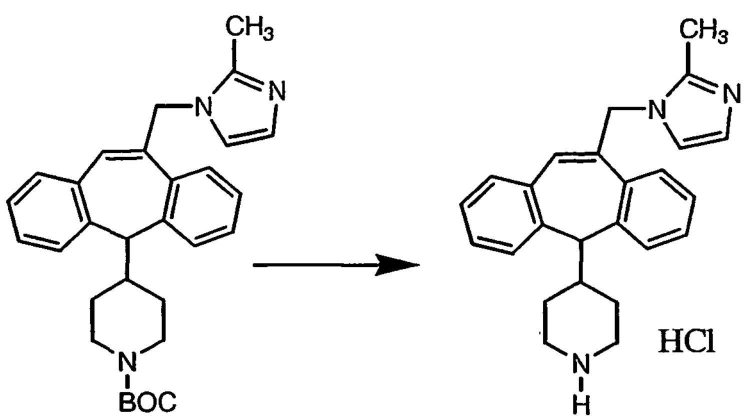

299 300

A solution of the (+) or (-) isomer of the product from Step I above (0.3 g, 0.64 mmol) in 4M HCl in dioxane (40 mL) was stirred at room temperature for 3 hours and then concentrated to dryness to give the HCl salt of the product (300a) or (300b) (0.42 g, 100% yield, $MH^+$=371).

EXAMPLES 120 AND 121

Starting with the appropriate (+) or (-) isomer of Compound (300) and reacting in a similiar manner as in Example 13 using the appropriate isocyanate, the following compounds were prepared:

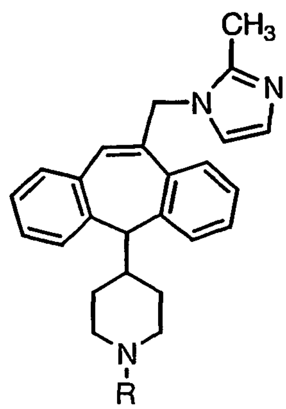

300

| EX. # | PROCEDURE | R= | CMPD # | PHYS. DATA |
|---|---|---|---|---|
| 120 | Example 13 | 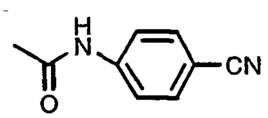 | 301 isomer 1 | MS $MH^+$=514 |
| 121 | Example 13 | 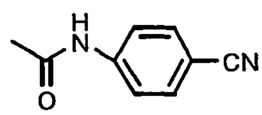 | 302 isomer 2 | MS MH+=514 |

PREPARATIVE EXAMPLE 35

A. Preparation of Compound (303a).

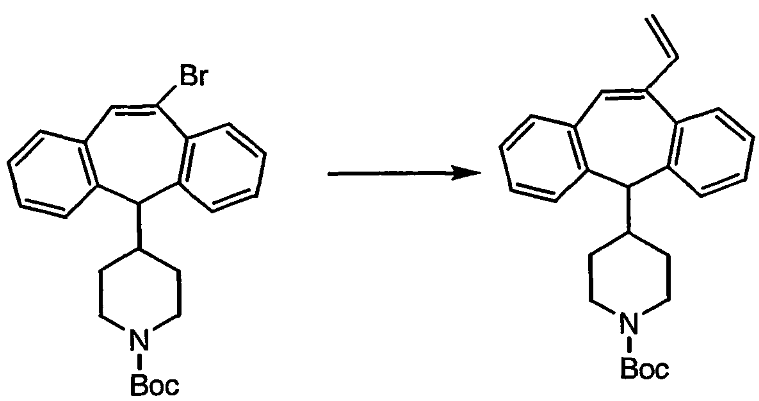

295a 303a

To a stirred solution of isomer 1 of the bomo-compound (295a) from Preparative Example 34, Step D,(0.5 g, 1.10 mmol) in 1-methyl-2-pyrrolidinone (4.3 mL) under nitrogen, was added lithium chloride (0.14 g, 3.3 mmol), tri-2-furylphosphine (0.013 g, 0.04 mmol) and tris(dibenzylideneacetone)-dipalladium(O) (0.02 g, 0.02 mmol). The resulting solution was stirred at room temperature for 5 minutes, followed by the addition of tributyl (vinyl) tin (0.39 g, 1.24 mmol). The reaction was then heated to 85˚C for 2 hours, followed by extraction with EtOAc-$H_2O$. The combined organic layer

was dried over $MgSO_4$, filtered, concentrated to dryness and purified by column chromatography on normal phase silica gel, eluted with 10% EtOAc/90% $CH_2Cl_2$ to give a light yellow liquid (303a) (0.06 g, 15% yield, $MH^+$=390).

B. Preparation of Compound (304a).

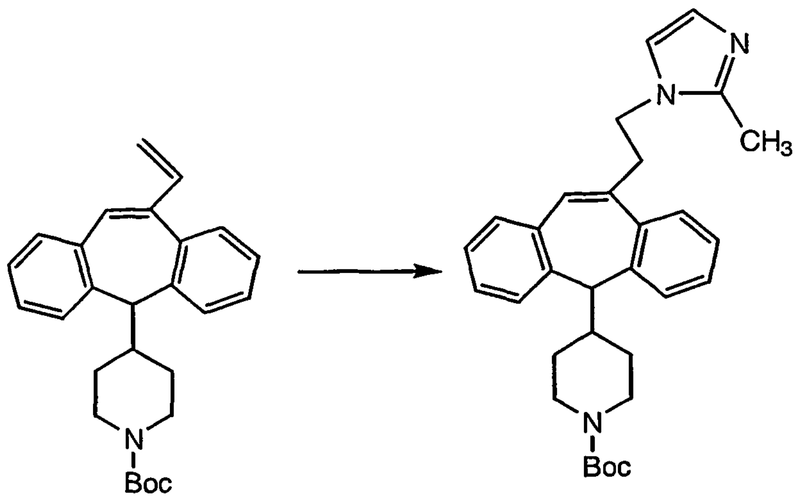

303a 304a

To a stirred solution of 1- methyl imidazole (0.377 g, 4.6 mmol) in anhydrous THF (4mL) under nitrogen at -78˚C, was added 2.5M n-BuLi/Hexane (0.33 mL). The resulting solution was stirred at -78˚C for 30 minutes and then allowed to warm at room temperature. To this stirred solution was added the alkene compound (303a) from step A above,(0.78 g, 2.1 mmol) in THF. The resulting solution was then heated to 120˚C overnight then cooled to room temperature, and extracted with EtOAc-$H_2O$. The combined organic layer was dried over $MgSO_4$, filtered, evaporated and purified by column chromatography on normal phase silica gel, eluted with 3% MeOH/97% $NH_3$-$CH_2Cl_2$ to give a light yellow solid (304a) (0.09 g, 10% yield, $MH^+$=456.1).

C. Preparation of Compound (305a).

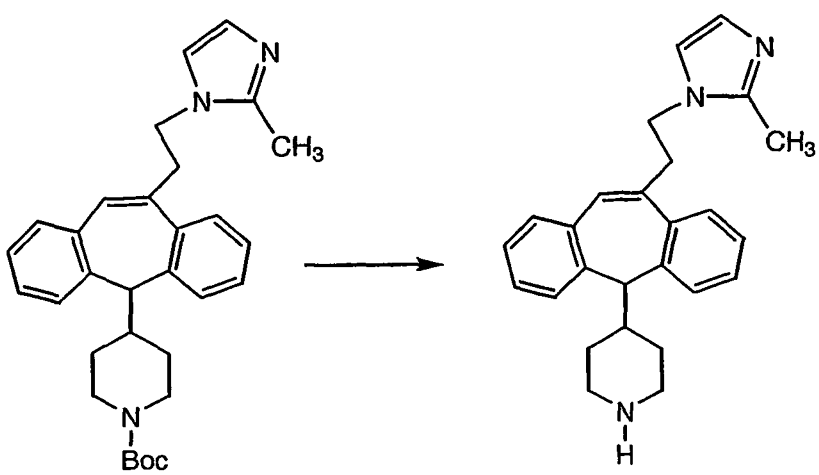

304a 305a

A solution of the product (304a) from Step B above (0.18 g, 3.72 mmol) in 4M HCl/dioxane (5 mL) was stirred at room temperature for 2 hours, then concentrated to dryness to give a crude off white solid (305a) (0.22 g, 100% yield, $MH^+$=384.2).

Using the same procedure as defined in Preparative Example 35 above starting with Isomer 2 of the Boc-protected Bromo compound (295b), Isomer 2 (305b) was prepared ($MH^+$=384.2).

EXAMPLES 122-125

Starting with the appropriate (+) or (-) isomer of Compound (305) and reacting in a similiar manner as in Example 13 using the appropriate isocyanate, the following compounds were prepared:

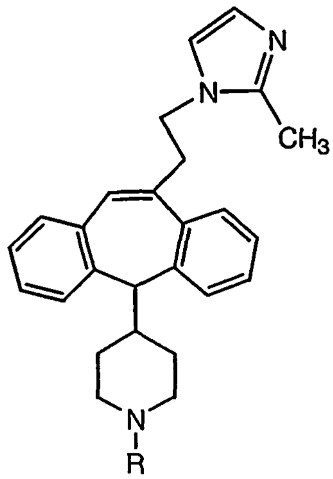

305

| EX. # | PROCEDURE | R= | CMPD # | PHYS. DATA |
|---|---|---|---|---|
| 122 | Example 13 | 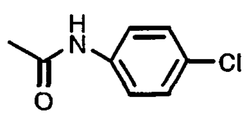 | 306 isomer 1 | MS $MH^+$=537.1 m.p.=118.1-119.0°C |
| 123 | Example 13 | 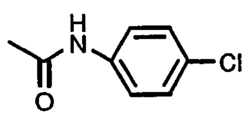 | 307 isomer 2 | MS $MH^+$=537.1 m.p.=107.8-108.4°C |
| 124 | Example 13 | 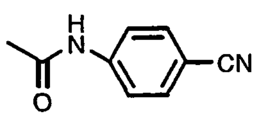 | 308 isomer 1 | MS $MH^+$=528.2 m.p.=119.6-120.2°C |
| 125 | Example 13 | 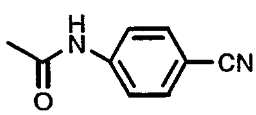 | 309 isomer 2 | MS $MH^+$=528.2 m.p.= 120.5-121.3°C |

PREPARATIVE EXAMPLE 36

A. Preparation of Compound (310)

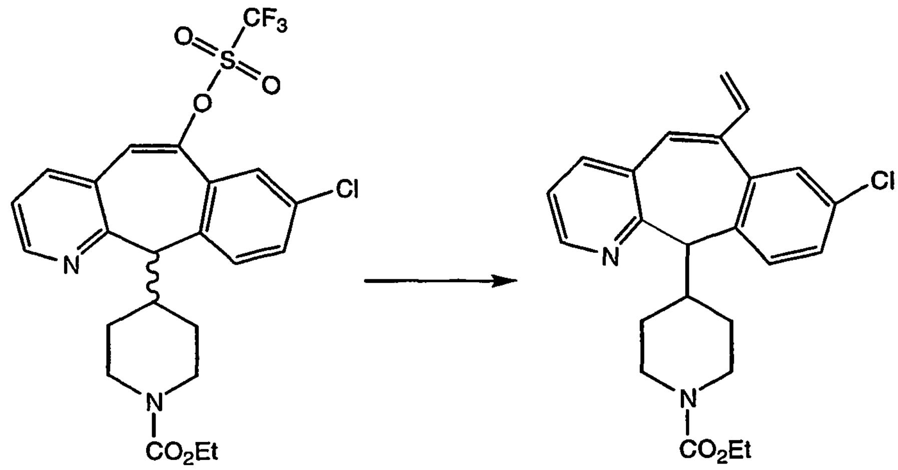

93A 310

To a solution of Compound (93A) from Example 7, Step A (5.0g, 10.02 mmol) in 1-methyl-2-pyrrolidinone (40 mL) under nitrogen at room temperature, was added LiCl (1.27g, 30.06 mmol), Tri-2-furrylphosphine (0.093g, 0.4 mmol) and tris(dibenzylidene acetone)dipalladium(0) (0.18g, 0.2 mmol).The resulting solution was stirred at room temperature for 5 minutes, followed by the addition of tributyl(vinyl) tin (3.3 mL, 11.3 mmol) and stirred overnight at 80 ˚C-85 ˚C. The solution was cooled to room temperature, followed by extraction with EtOAc-$H_2O$. The organic layer was dried over MgSO4, filtered, concentrated to dryness and purified by column chromatography on silica gel, eluted with 20% EtOAc/ 80% $CH_2Cl_2$ to give the product (310) (3.88g, 95% yield, $MH^+$=409.1)

B. Preparation of Compound (311).

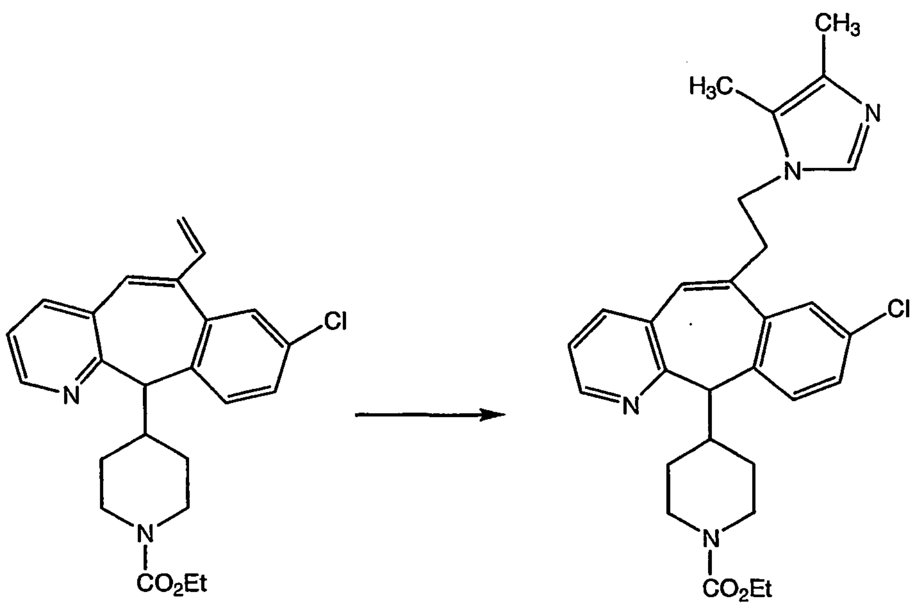

310 311

To a stirred solution of 4,5-dimethylimidazole (25.8 mg, 0.268 mmol) in anhydrous THF (0.2 mL) at -78˚C under

Argon, was added 2.5M n-BuLi (0.032 mL, 0.08 mmol). The resulting solution was warmed to room temperature, followed by the addition of the alkene compound (310) from Step A above (0.1 g, 0.24 mmol) in anhydrous THF (0.2 mL). The solution was then heated in an oil bath to 120°C for 25 hours, followed by extraction with $CH_2Cl_2$-$H_2O$. The combined organic layer was then washed with brine, dried over $Na_2SO4$, filtered and purified by column chromatography on silica gel, eluting with 5% MeOH/95% $CH_2Cl_2$ to give the product (311) (0.046 g, 100% yield, $MH^+$=505).

C. Preparation of Compounds (312a) AND (312b).

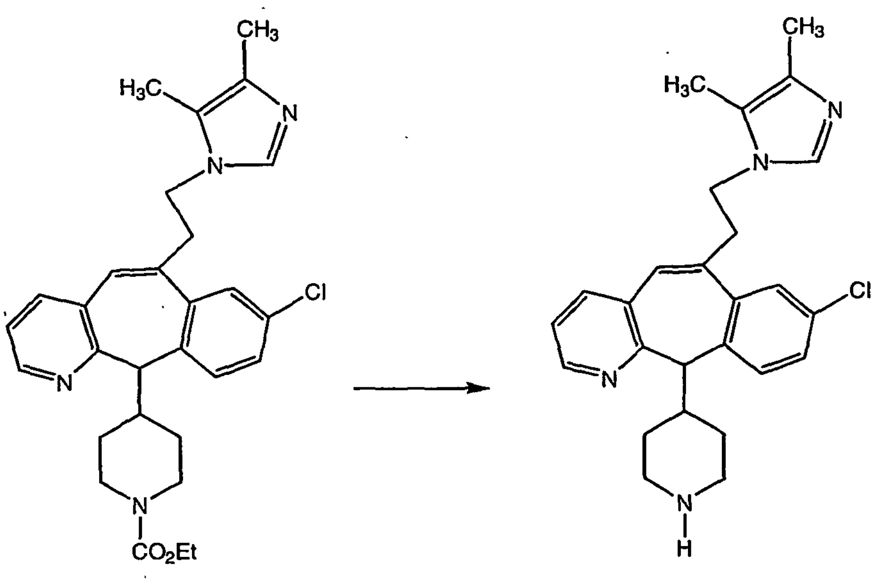

311

312a/
312b

A solution of Compound (311) from Step B above (0.57 g, 1.28 mmol) in 6N HCl (20 mL) was heated to reflux for 24 hours then concentrated to dryness. To the residue was then added saturated $NaHCO_3$ and Nacl. The solution was extracted twice with $CH_2Cl_2$, The combined organic layer was dried over $Na_2SO_4$ and concentrated to dryness to give the crude product (0.52 g, 93% yield). The crude material was then dissolved in 20% EtOH/80% Hexane/0.2% DEA and purified by HPLC on a preparative AD column, eluting with 20%-50% IPA/Hexane/0.2% DEA (UV=254nm, Attn=1024, ABS=2) to give pure isomers of the product (312a) and (312b) (0.225 g, $MH^+$=433).

EXAMPLES 126-133

Starting with the appropriate (+) or (-) isomer of Compound (312) and reacting in a similiar manner as in Example 13 using the appropriate isocyanate or sulfonyl chloride, the following compounds were prepared:

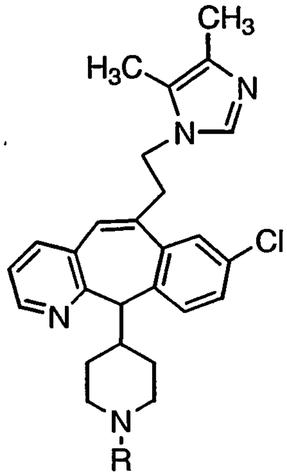

312

| EX. # | PROCEDURE | R= | CMPD # | PHYS. DATA |
|---|---|---|---|---|
| 126 | Example 13 | 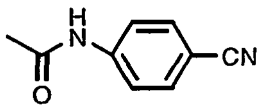 | 313 | Mass spec. $M^+$=577 |
| 127 | Example 13 | 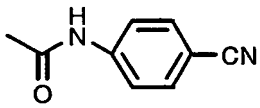 | 314 | Mass spec. $M^+$=577 |
| 128 | Example 13 | 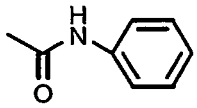 | 315 | Mass spec. $M^+$=558 |
| 129 | Example 13 | 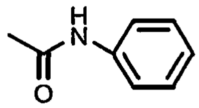 | 316 | Mass spec. $M^+$=558 |
| 130 | Example 13 | 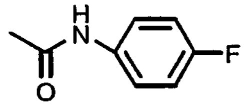 | 317 | Mass spec. $M^+$=570 |
| 131 | Example 13 | 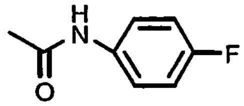 | 318 | Mass spec. $M^+$=570 |
| 132 | Example 13 | 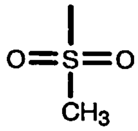 | 319 | Mass spec. $M^+$=511 |

(continued)

| EX. # | PROCEDURE | R= | CMPD # | PHYS. DATA |
|---|---|---|---|---|
| 133 | Example 13 | 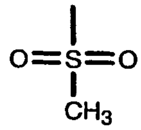 | 320 | Mass spec. $M^+$=511 |

PREPARATIVE EXAMPLE 37

A. Preparation of Compound (321)

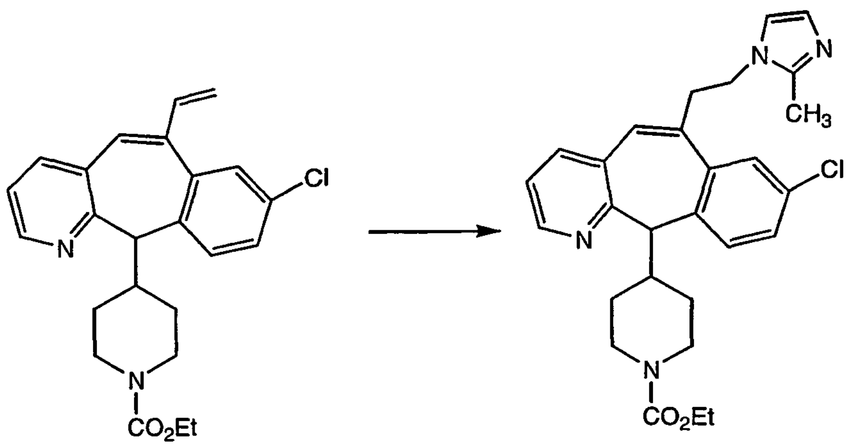

310 321

To a solution of Compound (310) from Preparative Example 36, Step A (0.66 g, 8.1 mmol) in THF (4.0 mL) under nitrogen at -78 ˚C, was added dropwise 2.5M n-BuLi/Hexane (1.5 mL). The resulting solution was stirred at -78˚C for 30 minutes, then allowed to warm to room temperature, followed by the addition of 1-methylimidazole (3.0 g, 7.3 mmol) in THF (3.0 mL). The solution was then heated to 120˚C over the weekend and then cooled down to room temperature and concentrated to dryness. The mixture was extracted with EtOAc-$H_2O$, dried over $MgSO_4$, filtered and purified by column chromatography on silica gel, eluting with 3% MeOH/97% $NH_3$-$CH_2Cl_2$ to give the product (321)(1.64 g, 46% yield, $MH^+$=491.1).

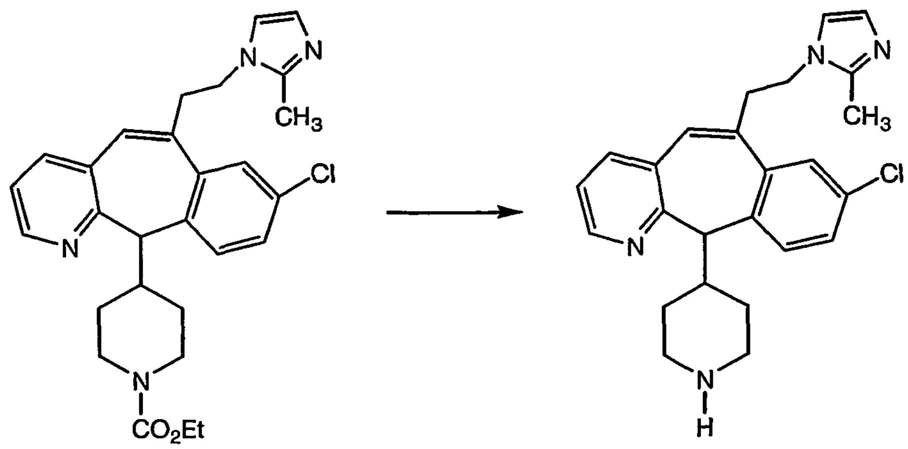

321 322

A solution of Compound (321) from Preparative Example 37, Step A above (0.6 g, 1.22 mmol) in 12N HCl (10 mL) was heated to reflux overnight then concentrated to dryness to give the residue as a gum. This residue was dissolved in saturated $NaHCO_3$, stirred for 10 minutes, saturated with NaCl and then stirred with $CH_2Cl_2$ for 10 minutes. The solid was filtered and the aqueous layer was extracted twice with $CH_2Cl_2$, and the organic layer was dried over $Na_2SO_4$, filtered and concentrated to dryness to give the Compound (322) as a light brown solid (566 mg, $MH^+$=419.1).

C. Preparation of Compounds (323a) and (323b).

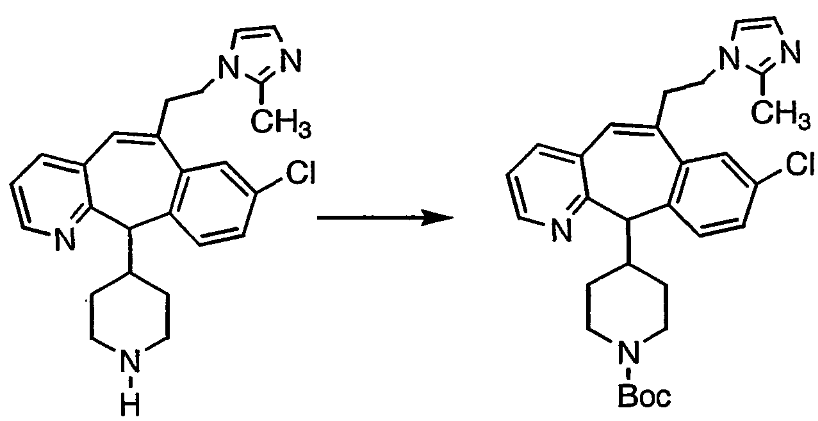

322 323a 323b

To a solution of Compound (322) from Step B above (0.566 g, 1.35 mmol) in MeOH (20 mL) and $H_2O$ (1 mL) at 0°C, was added Boc anhydride (0.44 g, 2.02 mmol). The solution was basified with 1N NaOH solution to maintain pH=8.5-9.5 and concentrated to dryness, followed by extraction with $CH_2Cl_2$-$H_2O$. The combined organic layer was washed twice with $H_2O$ then brine, dried over $Na_2SO_4$, filtered and concentrated to dryness to give a mixture of isomers 1 and 2 (0.63 g, 100% yield). The isomers were separated by HPLC on a prep AD column, eluting with 15%IPA/85%hexane/0.2%DEA (wave length=254nm, Attn=64, ABS=1) to give isomer 1 (323a) (0.28 g, $MH^+$=519.2) and isomer 2 (323b) (0.28 g, $MH^+$=519.2)

D. Preparation of Compound (322a).

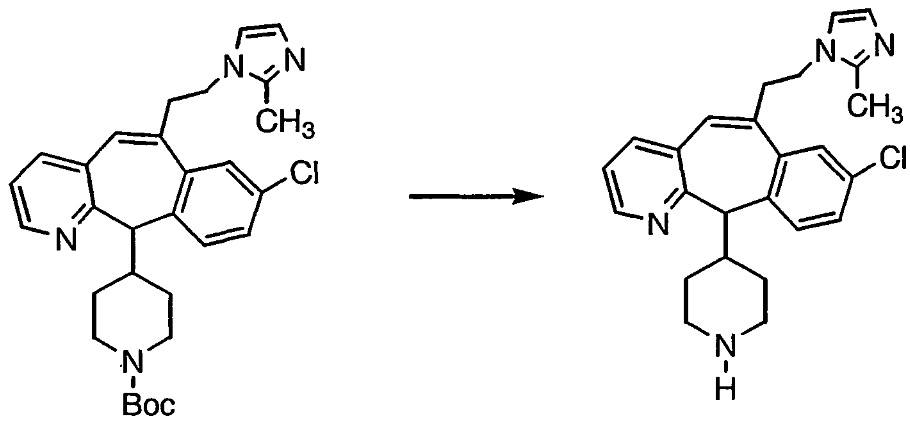

323a

322a

A solution of Compound (323a) isomer 1 from Step C above (0.24 g, 0.46 mmol) in 4N HCl/Dioxane (20 mL) was stirred at room temperature for 1hr. $CH_2Cl_2$ (7 mL) was added to the solution and the reaction continued to stir for 2 hrs before being concentrated to dryness. The solution was stirred for 5 minutes with saturated $NaHCO_3$, then saturated with NaCl and extracted three times with $CH_2Cl_2$. The combined organic layer was dried over $Na_2SO_4$, filtered and evaporated to dryness to give Compound (322a) isomer 1(0.163 g, 84% yield, $MH^+$=4.19.2).

Compound (322b) was prepared in a similar manner as in Step D above, starting with Compound (323b) to give the other isomer (0.193 g, 84% yield, $MH^+$=419.2)

EXAMPLES 134-147

Starting with compound 322a (isomer 1) or 322b (isomer 2) and reacting in a similiar manner as in Example 13 using the appropriate chloroformate, isocyanate, or sulfonyl chloride (or in the case of carboxylic acid, using DEC mediated coupling) the following compounds were prepared:

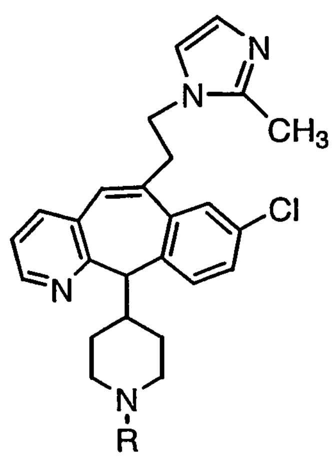

| EX. # | PROCEDURE | R= | CMPD # | PHYS. DATA |
|---|---|---|---|---|
| 134 | Example 13 | 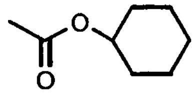 | 324 Isomer 1 | MS $M^+$=545.2 |
| 135 | Example 13 | 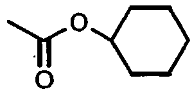 | 325 Isomer 2 | MS $M^+$=545.2 |
| 136 | Example 13 | 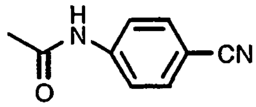 | 326 Isomer 1 | MS $M^+$=563.2 |
| 137 | Example 13 | 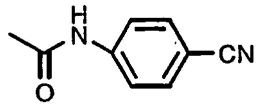 | 327 Isomer 2 | MS $M^+$=563.2 |
| 138 | Example 13 | 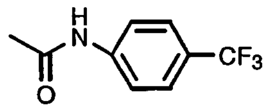 | 328 Isomer 1 | MS $M^+$=606.1 m.p.=62.7-63.0˚C |
| 139 | Example 13 | 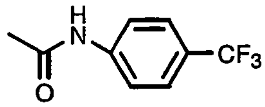 | 329 Isomer 2 | MS $M^+$=606.1 m.p.=70.1-71.0˚C |
| 140 | Example 13 | 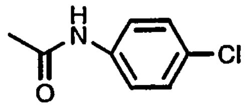 | 330 Isomer 1 | MS $M^+$=572.1 m.p.=120.1-121.4˚C |
| 141 | Example 13 | 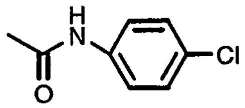 | 331 Isomer 2 | MS $M^+$=572.1 m.p.=128.0-129.0˚ |
| 142 | Example 13 | 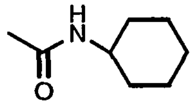 | 332 Isomer 1 | MS $M^+$=544.2 |
| 143 | Example 13 | 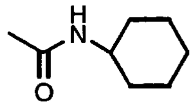 | 333 Isomer 2 | MS $M^+$=544.2 |
| 144 | Example 13 | 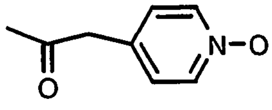 | 334 Isomer 1 | MS $M^+$=554.1 m.p.=111.9-112.0˚C |
| 145 | Example 13 | 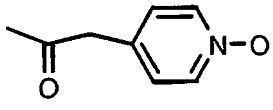 | 335 Isomer 2 | MS $M^+$=554.1 m.p.=114.3-115˚ |
| 146 | Example 13 | 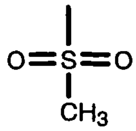 | 336 Isomer 1 | MS $M^+$=497.1 m.p.=52.4-53.3˚C |

(continued)

| EX. # | PROCEDURE | R= | CMPD # | PHYS. DATA |
|---|---|---|---|---|
| 147 | Example 13 | 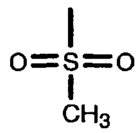 | 337<br>Isomer 2 | MS $M^+$=497.1<br>m.p.=47.1-48.0˚ |

PREPARATIVE EXAMPLE 38

A. Compounds (338) AND (339).

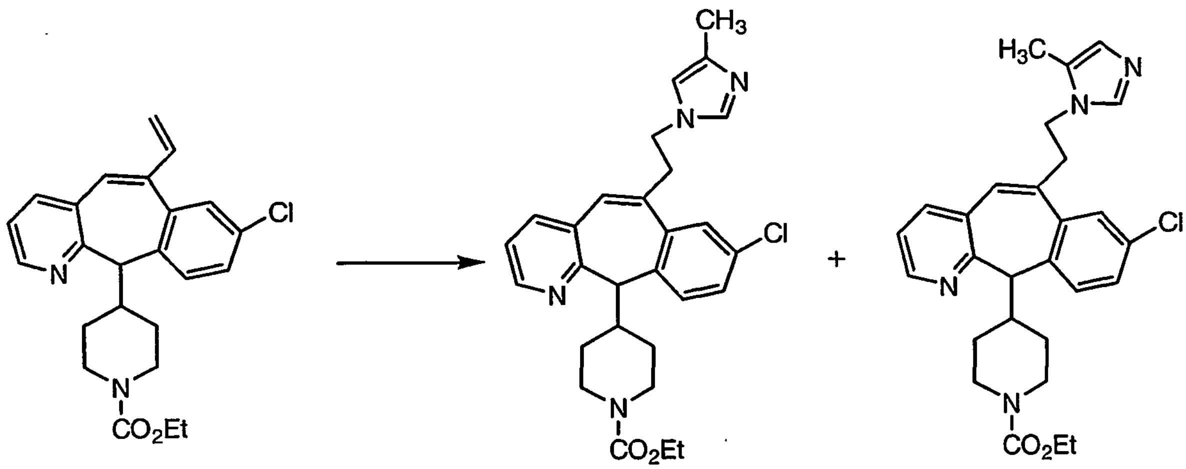

310 338 339

To a solution of Compound (310) from Preparative Example 36 Step A (3.0 g, 7.34 mmol) in THF (8 mL) under nitrogen at -78˚C, was added dropwise 2.5M n-BuLi/Hexane (0.65mL, 8.07 mmol). The resulting solution was stirred at -78˚C for 30 minutes, then allowed to warm to room temperature, followed by the addition of 4-methylimidazole (0.66 g, 8.07 mmol) in THF. The solution was heated to 120˚C over night cooled down to room temperature and concentrated to dryness The reaction mixture was extracted with EtOAc-$H_2O$, and the organic layer was dried over $MgSO_4$, filtered and concentrated to give a mixture of 4-methyl substituted (338) and 5-methyl substituted (339) products (2.76 g, 76% yield, $M^+$=491.1).

B. Separation of compounds (338a/b) and (339a/b).

In a similar manner as described in Example 11, the mixture of products from Step A, above were first seperated into a mixture of pure 4 and 5-substitured (+) enantiomers and pure 4 and 5-substituted (-) enantiomers using chiral HPLC column chromatography, then upon treatment with triphenyl methyl chloride following the procedure in Example 11, the compounds were further seperated into the pure isomers of the 4-substituted compound (338a) (MS M+=491; mp= 72.1-73.0˚C) and (338b) (MS M+=491; mp= 68.9-69.0˚C) and the 5-substituted compound (339a) and (339b).

C. Preparation of Compound (340a).

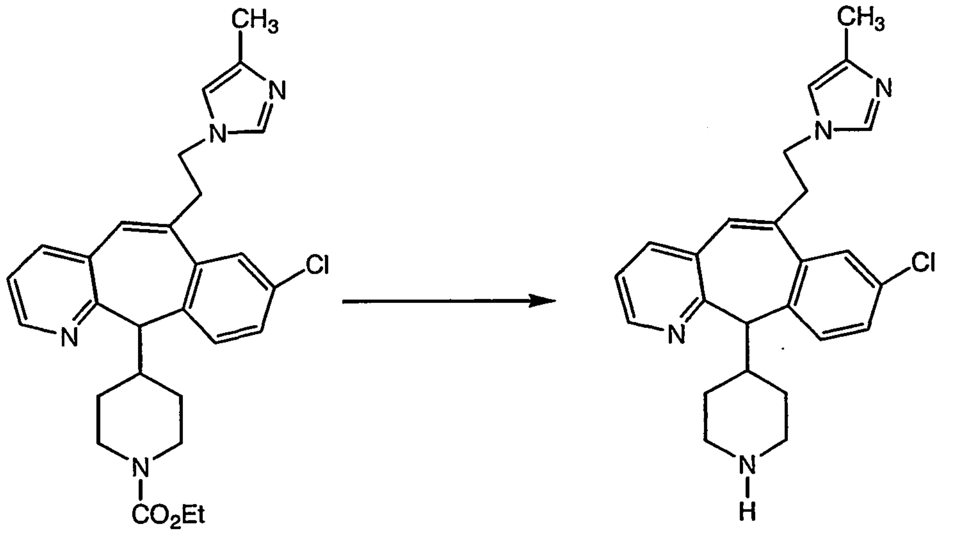

338a 340a

A solution of Compound (338a) from step B above (0.035 g, 0.071 mmol) in 6N HCl (2.0 mL) was heated to reflux overnight. The solution was cooled to room temperature, basified with $NH_4OH$ solution and extracted with $CH_2Cl_2$. The combined organic layer was dried over $MgSO_4$, filtered and concentrated to give pure isomer 1, Compound (340a) (0.0334 g, 100% yield, $MH^+$=419.1; mp= 60.3-61.0°C).

In a similar manner as above, starting with Compound (338b) (isomer 2), Compound (340b) ($MH^+$=419.1) was prepared.

EXAMPLES 148-156

Starting with the appropriate (+) or (-) isomer of Compound (340) and reacting in a similiar manner using the procedure shown in the table below, with the appropriate chloroformate, isocyanate or sulfonyl chloide, the following compounds were prepared:

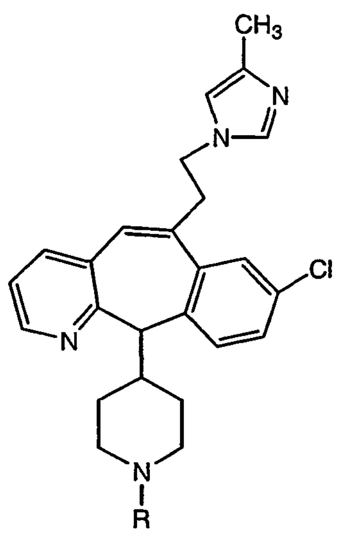

| EX.# | PROCEDURE | R= | CMPD # | PHYS. DATA |
|---|---|---|---|---|
| 148 | Preparative Ex.4; Step A | BOC | 341 | MS MH$^+$=519 m.p.=90.2-91.0°C |
| 149 | Example 13 | 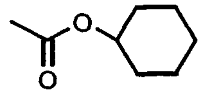 | 342 isomer 1 | MS MH$^+$=545 m.p.=58.8-59.6°C |
| 150 | Example 13 | 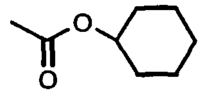 | 343 isomer 2 | MS MH$^+$=545 m.p.=60.8-61.2°C |
| 151 | Example 13 | 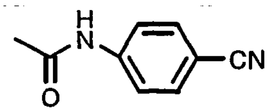 | 344 isomer 1 | MS MH$^+$=545 m.p.=98.7-99.5°C |
| 152 | Example 13 | 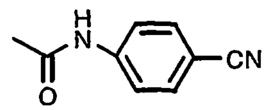 | 345 isomer 2 | MS MH$^+$=545 m.p.=111.3-112.0°C |
| 153 | Example 13 | 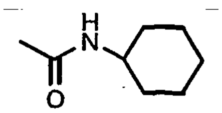 | 346 isomer 1 | MS MH$^+$=544 m.p.=77.1-77.8°C |
| 154 | Example 13 | 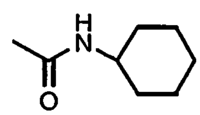 | 347 isomer 2 | MS MH$^+$=544 m.p.=78.9-79.0°C |
| 155 | Example 13 | 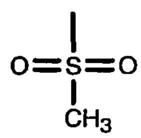 | 348 isomer 1 | MS MH$^+$=497 m.p.=87.4-88.0 °C |
| 156 | Example 13 | 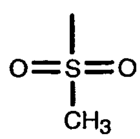 | 349 isomer 2 | MS MH$^+$=497 m.p.=88.8-89.0°C |

PREPARATIVE EXAMPLE 39

Preparation of Compound (350a).

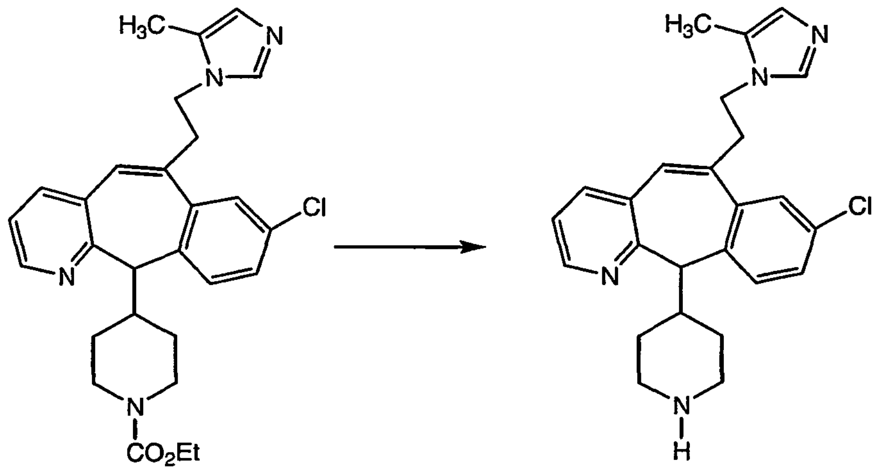

339a 350a

Compound (339a) was reacted in a similar manner as in Preparative Example 38, Step C to give Compound (350a) (isomer 1) (0.13 g, 76% yield, $MH^+$=419.3).

Compound (350b) (isomer 2) was prepared in the same manner as above.

EXAMPLES 157-160

Starting with the appropriate (+) or (-) isomer of Compound (350) and reacting in a similiar manner using the procedure indicated in the table below and the appropriate Boc or isocyanate reagent, the following compounds were prepared:

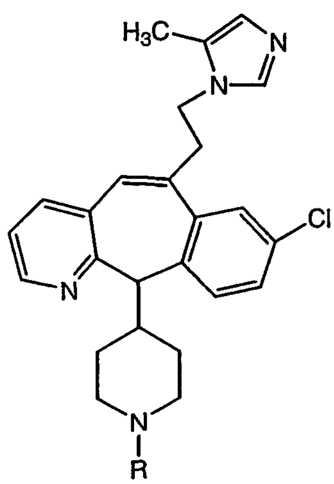

| EX. # | PROCEDURE | R= | CMPD # | PHYS. DATA |
|---|---|---|---|---|
| 157 | Preparative Ex.4; Step A | BOC | 351 isomer 1 | MS $MH^+$=519 m.p.=87.8-88.2˚C |
| 158 | Preparative Ex.4; Step A | BOC | 352 isomer 2 | MS $MH^+$=519 m.p.=89.0-89.9˚C |

(continued)

| EX. # | PROCEDURE | R= | CMPD # | PHYS. DATA |
|---|---|---|---|---|
| 159 | Example 13 | 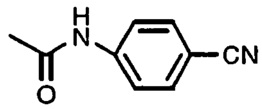 | 353 isomer 1 | MS $MH^+$=563 |
| 160 | Example 13 | 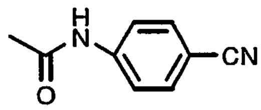 | 354 isomer 2 | MS $MH^+$=563 m.p.=130.1-131.0˚C |

PREPARATIVE EXAMPLE 40

A. Compound (355).

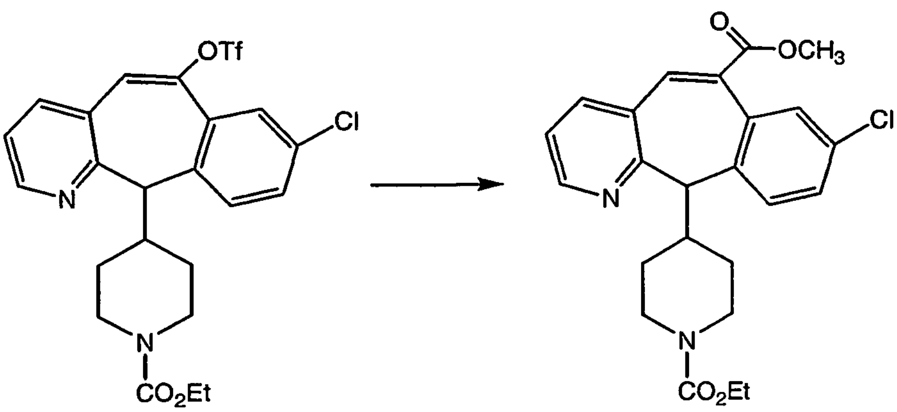

93A 355

To a solution of Compound (93A) from Preparative Example 7, Step A (2.92 g, 5.5 mmol) in anhydrous toluene (70 mL) and MeOH (10 mL) was added triphenyl phosphine (0.72 g, 2.75 mmol), DBU (1.11 mL, 7.42 mmol) and $PdCl_2$ (0.097 g, 0.55 mmol). The resulting solution was purged with CO (100psi), then heated to 80˚C for five hours. The solution was cooled to room temperature, purged with nitrogen and evaporated to dryness to give a brown oil. The product was purified by silica gel column chromatography eluting with 1% MeOH/99% $CH_2Cl_2$ to 4% MeOH/96%$CH_2Cl_2$ to give Compound (355) (2.22 g, 92.5% yield, $MH^+$=441.1).

B. Preparation of Compound (356).

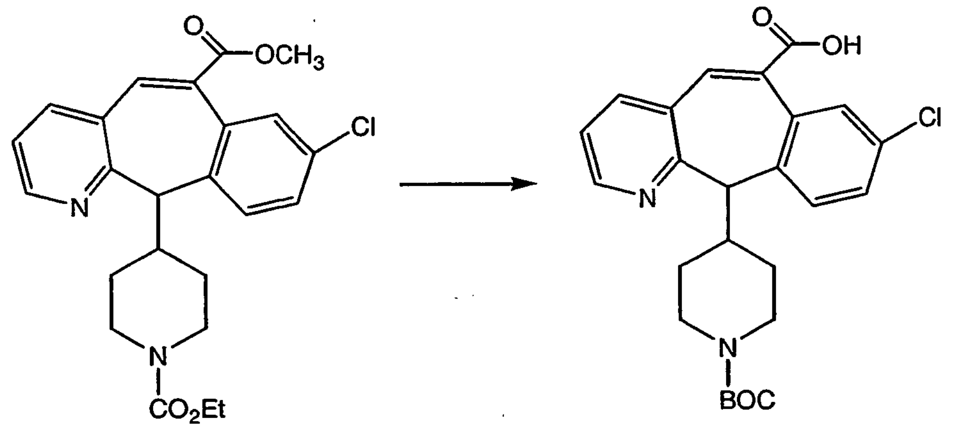

355 356

A solution of Compound (355) from Preparative Example 40, Step A (2.2 g, 4.99 mmol) in 6N HCl (50mL) was heated to 100˚C - 110˚C overnight. The solution was cooled to room temperature and evaporated to dryness to give the crude product. To a solution of the crude material in MeOH (50mL) and $H_2O$ (1 mL) at 0˚C, was added Boc anhydride (1.63 g, 7.48 mmol). The resulting solution was basified with 1N NaOH to pH=8.5 - 9.5 and stirred for two hours at 0˚C, then evaporated to dryness and extracted with EtOAc-5% Citric acid solution. The organic layer was washed with $H_2O$, then brine, dried over $Na_2SO_4$, filtered and concentrated to dryness to give Compound (356) as a yellow solid (2.29g, 100% yield, $MH^+$=455.1).

C. Preparation of Compound (357).

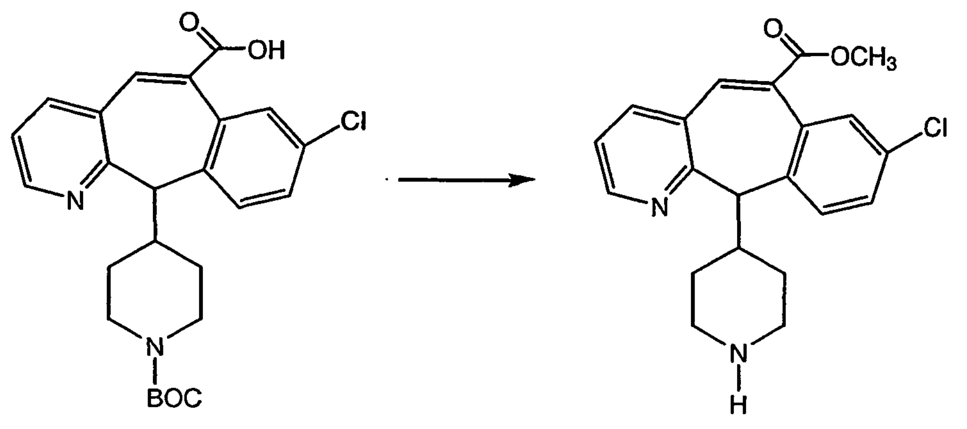

356 357

To a solution of Compound (356) from Preparative Example 40, Step B above(2.26 g, 4.97 mmol) in anhydrous benzene (18.0 mL) and MeOH (2 mL), was added, over five minutes, (trimethylsilyl)diazomethane (3 mL, 5.99 mmol) in 2M 1N Hexane. The resulting solution was stirred at room temperature for one hour then evaporated to dryness to give 2.33g of crude material ($MH^+$=369).

A solution of the crude material (obtained above) in 4N HCl in Dioxane (25 mL) was stirred at room temperature for one hour. The reaction was then evaporated to dryness and purified by flash silica gel column chromatography, eluting with 2% MeOH/98% $CH_2Cl_2$ to 6% MeOH/94% $CH_2Cl_2$ and then with 50% (10% $NH_4OH/CH_3OH$/50% $CH_2Cl_2$). The collected fractions were evaporated to dryness and diluted with $CH_2Cl_2$. The organic solution was then washed with saturated $NaHCO_3$ and brine, dried with $Na_2SO_4$, filtered and evaporated to dryness to afford Compound (357) (1.26g, 68.3% yield, $MH^+$=369).

D. Preparation of Compound (358).

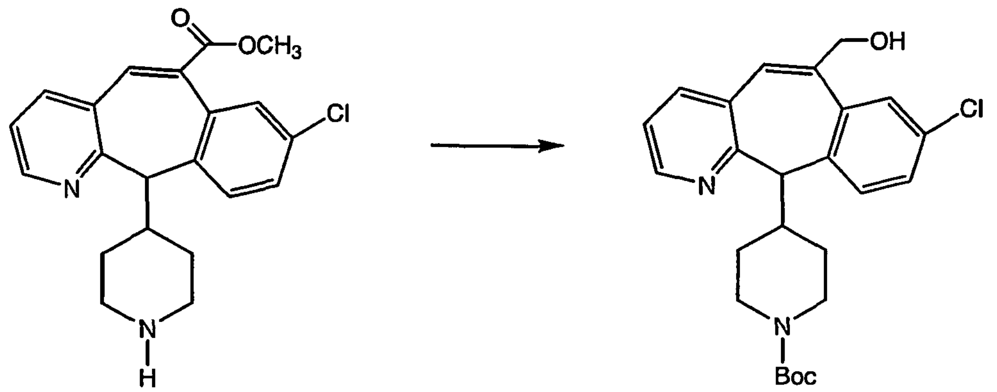

357 358

To a solution of Compound (357) from Preparative Example 40, Step C (0.6 g, 1.62 mmol) in anhydrous THF (6 mL) at 0˚C was added DIBAL (1M solution in toluene) (9.78 mL, 9.78 mmol). The resulting solution was warmed to room temperature and stirred overnight. The solution was then quenched with MeOH and evaporated to dryness to give a crude product.

To the crude material (obtained above) in MeOH at 0˚C was added Boc anhydride (1.06g, 4.9 mmol). The resulting solution was basified with 1N NaOH to pH=8.5-9.5, stirred for 1 hour and evaporated to dryness. The crude material was diluted with $CH_2Cl_2$ to give a slurry. The precipitate was then filtered through celite and the $CH_2Cl_2$ was washed with $H_2O$, brine, filtered over $Na_2SO4$ and concentrated to dryness. The crude alcohol product (358) (1.27g, 100% yield) was used in the next step without further purification.

E. Preparation of Compound (359).

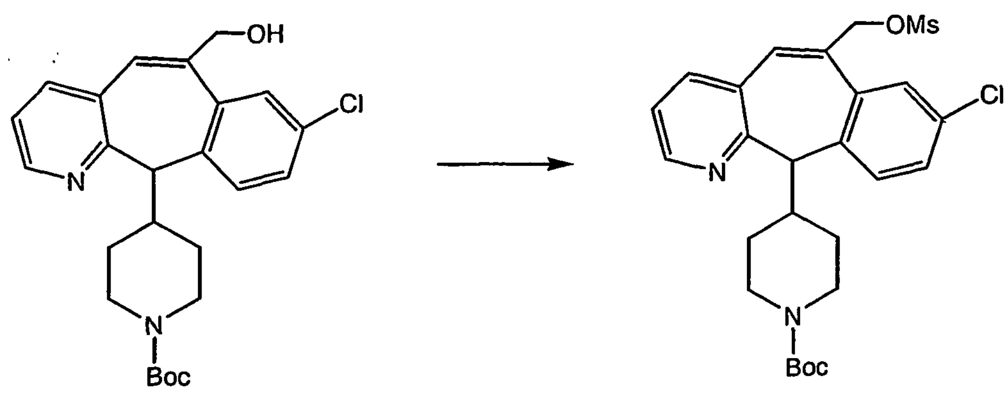

358 359

To a cooled solution of the alcohol (358) from Step D above (1.2 g, 2.73 mmol) in anhydrous $CH_2Cl_2$ (12 mL) at 0˚C was added triethyl amine (1.14 mL, 8.18 mmol) and methanesulfonyl chloride (0.3 mL, 4.1 mmol). The resulting solution was warmed to room temperature stirred overnight, then quenched with $H_2O$ and stirred for 10 minutes. The reaction was washed with water, then brine, dried over $Na_2SO_4$, filtered and evaporated to dryness to give Compound (359) (1.22 g, 86% yield).

F. Preparation of Compounds (360a) AND (360b).

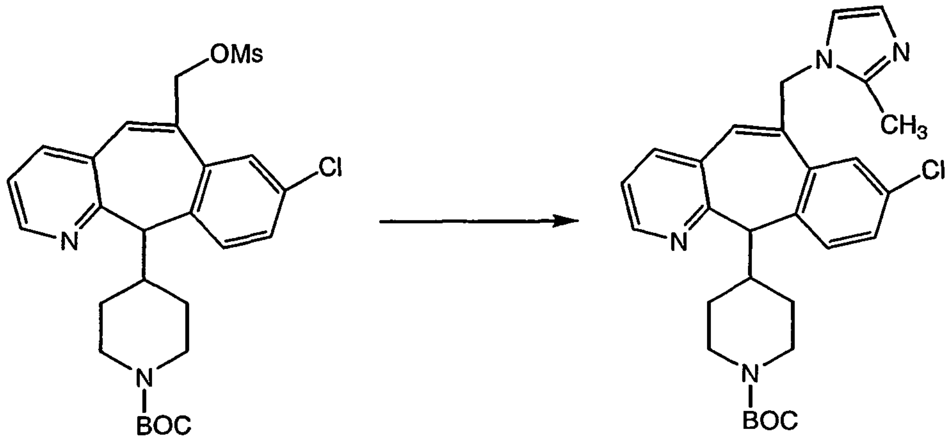

359

360a/ 360b

To a solution of anhydrous DMF (5 mL) at 0°C was added, NaH (0.19 g, 8.18 mmol) and 2-methylimidazole (0.67 g, 8.18 mmol). The resulting solution was warmed to room temperature and stirred for 20 minutes. To the reaction was added a solution of Compound (359) from Step E above (1.22 g, 2.3 mmol) in anhydrous DMF (5 mL). The resulting of solution was stirred at room temperature overnight, then diluted with EtOAc and washed with water then brine. The organic layer was dried over $Na_2SO_4$, concentrated to dryness and purified by silica gel column chromatography eluting with 1% MeOH/99% $CH_2Cl_2$ to 5%MeOH/$CH_2Cl_2$ to give the product as a mixture of isomers (1.18 g, 100% yield, $MH^+$=505.2). Separation of the product mixture by HPLC using a prep AD column, eluting with 25%IPA/75%hexane/ 0.2%DEA (isocratic 60 ml/min.) afforded pure isomer 1 (360a) (0.251 g, $MH^+$=505.1) and isomer 2 (360b) (0.251 g, $MH^+$=505.1) as light pink solids.

G. Preparation of Compounds (361a) AND (361b).

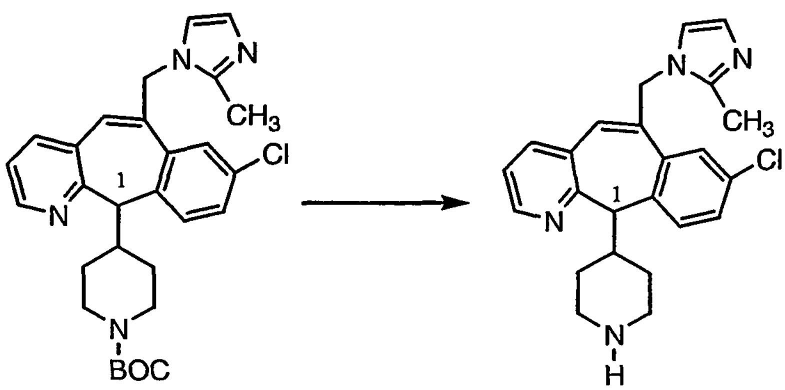

360a

361a

A solution of Compound (360a) (isomer 1) from Step F above (0.2 g, 0.4 mmol) in 4N HCl in Dioxane (10 mL) was stirred at room temperature for 2 hours and then evaporated to dryness to afford Compound (361a) (0.292 g, 100% yield).

Compound (361b) (isomer 2) was prepared in a similar manner as above beginning with Compound (360b)

from Preparative Example 40, Step F.

EXAMPLES 161-166

Starting with the appropriate (+) or (-) isomer of Compound (361) and reacting in a similiar manner as in Example 13 using the appropriate isocyanate shown in the table below, the following compounds were prepared:

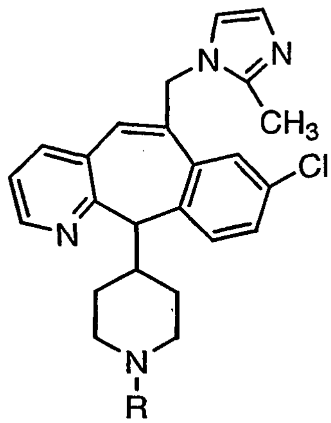

| EX. # | PROCEDURE | R= | CMPD # | PHYS. DATA |
|---|---|---|---|---|
| 161 | Example 13 | 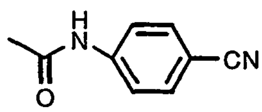 | 362a isomer 1 | MS MH+=548 |
| 162 | Example 13 | 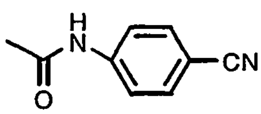 | 362b isomer 2 | MS MH+=548 |
| 163 | Example 13 | 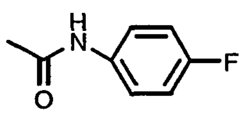 | 363a isomer 1 | MS MH+=541 |
| 164 | Example 13 | 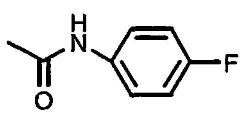 | 363b isomer 2 | MS MH+=541 |
| 165 | Example 13 | 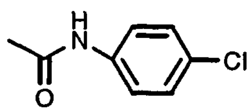 | 364a isomer 1 | MS MH+=558 |
| 166 | Example 13 | 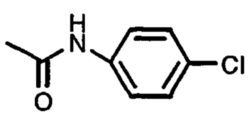 | 364b isomer 2 | MS MH+=558 |
| 166.1 | Example 13 | 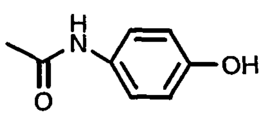 | 364c | Mp 201.5-208.3°C |

PREPARATIVE EXAMPLE 41

Compound (365).

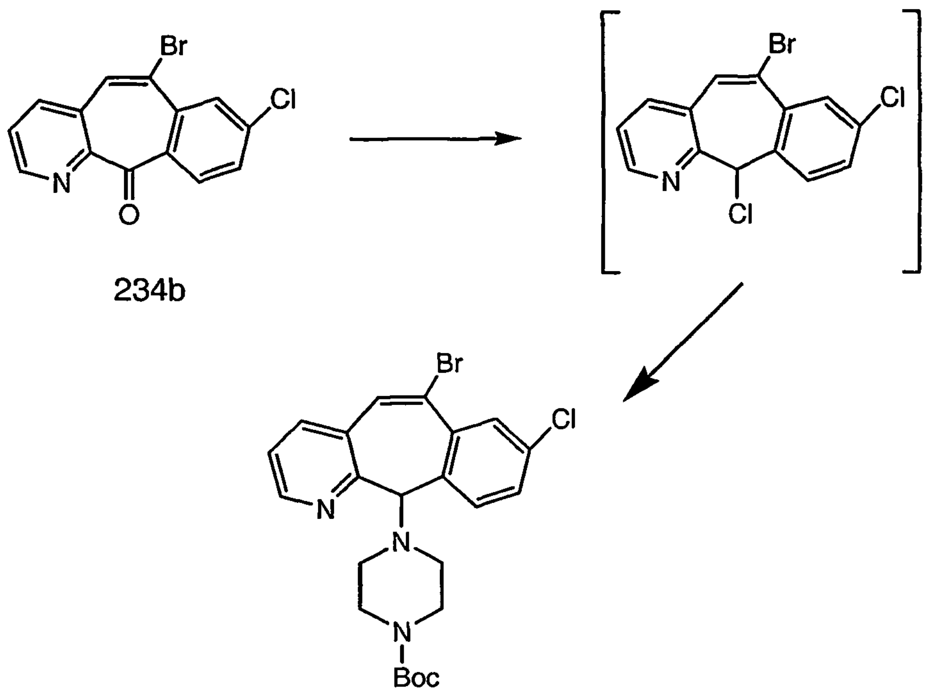

234b

365

In essentially the same manner as in Preparative Example 23, Steps A-D, using the 6-Bromo substituted product from Step B, Compound (234b), the product Compound (365) was prepared (76.6 g, 100% yield).

PREPARATIVE EXAMPLE 42

A. Preparation of Compound (366).

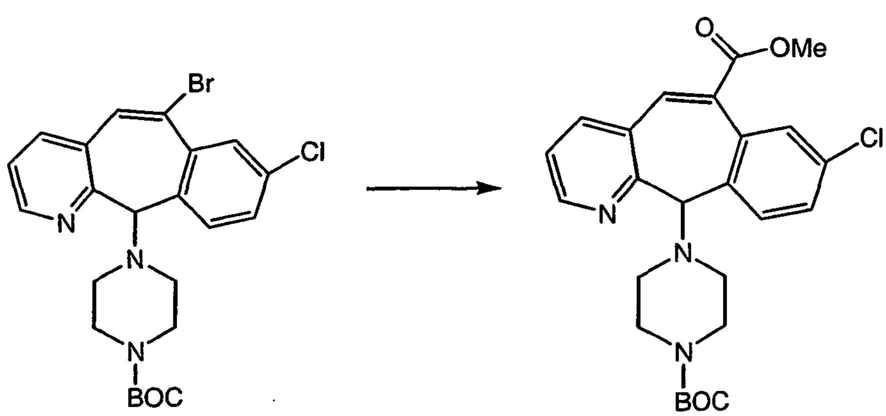

365 366

To a solution of Compound (365) from Preparative Example 41 (4.0g, 8.16 mmol) in toluene (75 mL) and MeOH (20 mL), was added triphenyl phosphine (1.099g, 4.08 mmol), DBU (1.7 g, 11.02 mmol) and palladium chloride (0.145 g, 0.82 mmol). The resulting solution was evacuated with CO at 100 psi and heated at 78˚C-82˚C for 5 hours, followed by the extraction with EtOAc-$H_2O$. The combined organic layer was then washed with brine, dried over $Na_2SO4$, concentrated to dryness and purified by column chromatography, eluting with 30% EtOAc/70% Hexane to give a Compound (366) (3.12 g, 100% yield, $MH^+$=470.1).

B. Preparation of Compound (367).

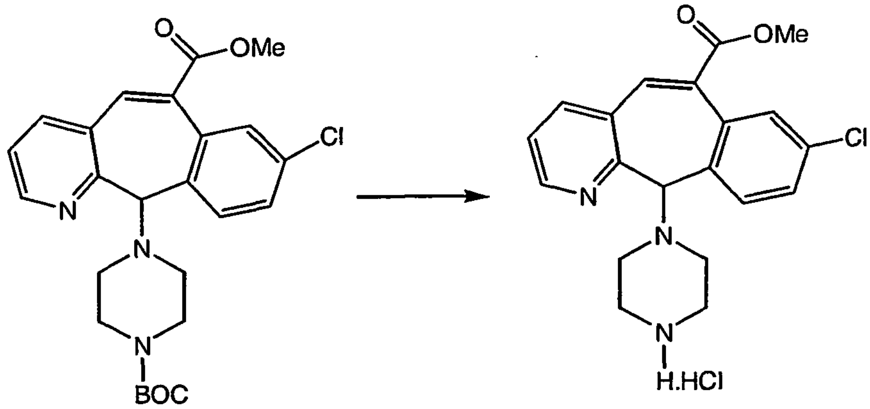

366 367

A solution of Compound (366) from Step A above (3.1 g, 6.6 mmol) in 4M HCl /Dioxane (120 mL) was stirred for 3 hours and then concentrated to dryness to give the crude salt of Compound (367) (3.89 g, 100% yield, $MH^+$=370.2)

C. Preparation of Compound (368).

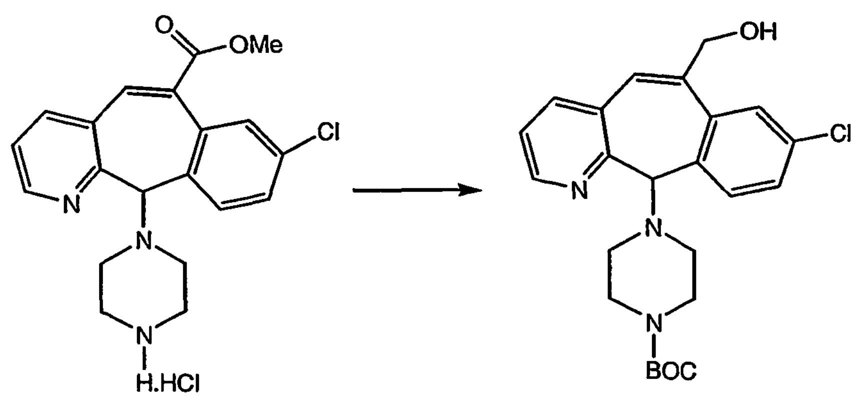

367 368

To a solution of Compound (367) from Step B above (3.43 g, 8.45 mmol) in THF (60 mL) at 0˚C, was added DIBAL (7.21 g, 50.7mmol). The resulting solution was warmed to room temperature, stirred overnight and then concentrated to dryness, followed by the addition of Boc anhydride (3.69 g, 16.9 mmol). The reaction was then extracted with $CH_2Cl_2$-$H_2O$, filtered over $Na_2SO_4$ and concentrated to dryness to afford Compound (368) (3.75g, 100% yield, $MH^+$=442.4).

C.1 Alternate Preparation of Compound (368).

A solution of compound 366 from step A above (23.46g, 50.98 mmol) in $CH_2Cl_2$ - MeOH - $H_2O$ (120mL, 600 mL, 60 mL respectively) combined with LiOH (12.0 g, 350.88 mmol) was refluxed at 40˚C overnight. Solvent was removed from the reaction mixture and the residue diluted with $CH_2Cl_2$, was acidified to pH 6 with 1N HCl. The organic layer was separated and washed with water, dried over $Na_2SO_4$ and concentrated. The product was dissolved in THF (285 mL) at 0˚C. Triethyl amine (6 mL, 42.97 mmol) and ethyl chloroformate (4.1 mL, 42.97 mmol) were added and stirred at 0˚C for 1 h. The reaction mixture was filtered and the filtrate was cooled to -70˚C. To this filtrate was added $NaBH_4$ (3.97g, 104.94 mmol) and stirred for 1 h at -70˚C after which time 40 mL of MeOH was added dropwise. The solvents were removed and the residue taken up in methylene chloride, washed with sat. (aq) $NaHCO_3$, then brine, dried over $Na_2SO_4$ and concentrated to give Compound (368) as a solid.

D. Preparation of Compound (369).

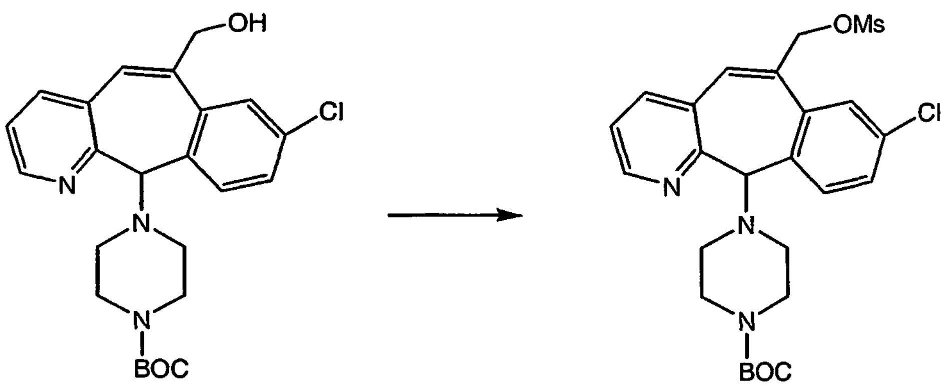

368 369

To a solution of Compound (368) from Step C above (3.74 g, 8.46 mmol) in $CH_2Cl_2$ (100 mL) was added triethyl amine (3.5 mL, 25.38 mmol) and methanesulfonyl chloride (1.45g, 2.7 mmol). The resulting solution was stirred under nitrogen at room temperature for overnight and then washed with saturated $NaHCO_3$ then brine, and dried over $Na_2SO_4$ to give the mesylate compound (369) (3.86 g, 88% yield).

E. Preparation of Compounds (370a) AND (370b)

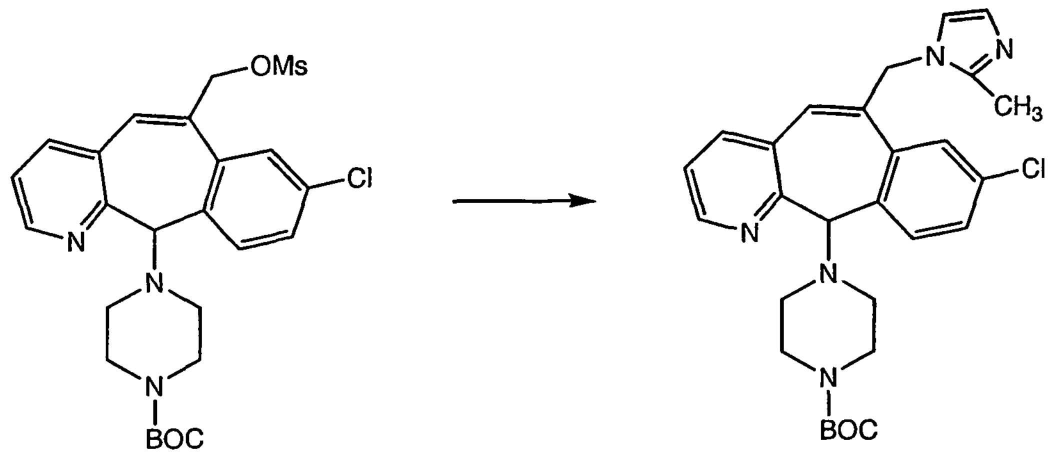

369

370a (isomer 1)
370b (isomer 2)

To a solution of 2-methylimidazole (2.43g, 29.68 mmol) in DMF (30 mL) under $N_2$ was added NaH (0.53g, 22.3 mmol) and stirred for 10 min, followed by the addition of Compound (369) from Step D above (3.86 g, 7.42 mmol). The solution was stirred over night. The solution was then concentrated to dryness and extracted with EtOAc-$NaHCO_3$, dried over $Na_2SO_4$, and concentrated. The crude product was purified by column chromatography, eluting with 2% MeOH-$NH_3$/98% $CH_2Cl_2$ to afford a mixture of isomers. Further separation was accomplished by Preparative HPLC Chiral AD Column chromatography, eluting with 25% IPA/75% hexane/0.2% DEA to give pure Compound (370a) (isomer 1) (0.160g) and Compound (370b) (isomer 2) (0.140 g) ($MH^+$=506.1)

F. Preparation of Compounds (371a) AND (371b).

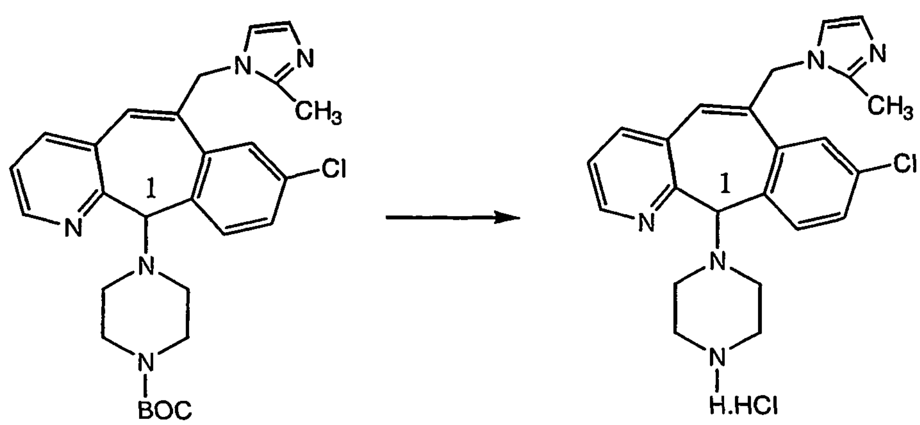

370a

371a

A solution of Compound (370a) (isomer 1) from Step E above (0.105g, 0.21 mmol) in 4M HCl/Dioxane (10 mL) was stirred at room temperature for 3 hours and concentrated to dryness to afford Compound (371 a) (0.147g, 100% yield)

Compound (370b) (isomer 2) from Step E was treated in the same manner as isomer 1 above, to afford Compound (371 b) (isomer 2).

EXAMPLE 167

Preparation of Compound (372)

To a solution of compound 371a (1.3g, 2.94 mmol) in $CH_2Cl_2$ (60 mL) was added triethyl amine (1.3 mL, 9.4 mmol) and p-cyano phenyl isocyanate (0.466g, 3.24 mmol). The resulting solution was stirred at room temperature overnight, followed by the extraction with $CH_2Cl_2$ and saturated $NaHCO_3$. The organic layer was dried over $Na_2SO_4$, evaporated and the residue purified by column chromatography, eluting with 1 % -2% MeOH-$NH_3$/98% $CH_2Cl_2$ to afford compound (372) (0.870 g , 48% yield) see table below.

EXAMPLE 168

Preparation of Compound (373)

Compound 371b (isomer 2) was reacted in a similar manner as in Example 13 with p-cyano phenyl isocyanate to afford compound (373) see table below.

EXAMPLE 169

Preparation of Compound (374)

Compound 371a (isomer 1) was reacted in a similar manner as in Example 13 with p-chloro phenyl isocyanate to afford compound (374) see table below.

EXAMPLE 170

Preparation of Compound (375)

Compound 371b (isomer 2) was reacted in a similar manner as in Example 13 with p-chloro phenyl isocyanate to afford compound (375) see table below.

Examples 167-170

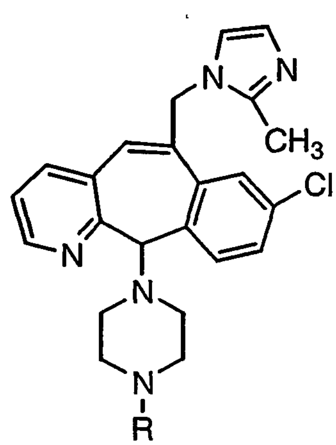

| EX. # | PROCEDURE | R= | CMPD # | PHYS. DATA |
|---|---|---|---|---|
| 167 | Example 13 | 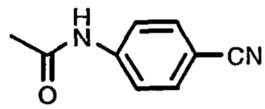 | 372 isomer 1 S-isomer | MS MH+=550 |
| 168 | Example 13 | 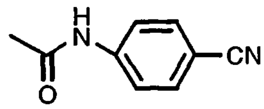 | 373 isomer 2 R-isomer | MS MH+=550 |
| 169 | Example 13 | 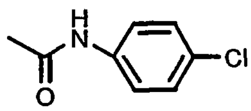 | 374 isomer 1 S-isomer | MS MH+=559 |
| 170 | Example 13 | 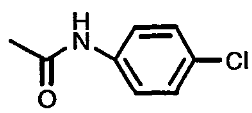 | 375 isomer 2 R-isomer | MS MH+=559 |
| 170.1 | Example 13 | 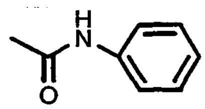 | 375.1 isomer 1 | MS MH+=525 |

PREPARATIVE EXAMPLE 43

A. Preparation of Compounds (376a) AND (376b).

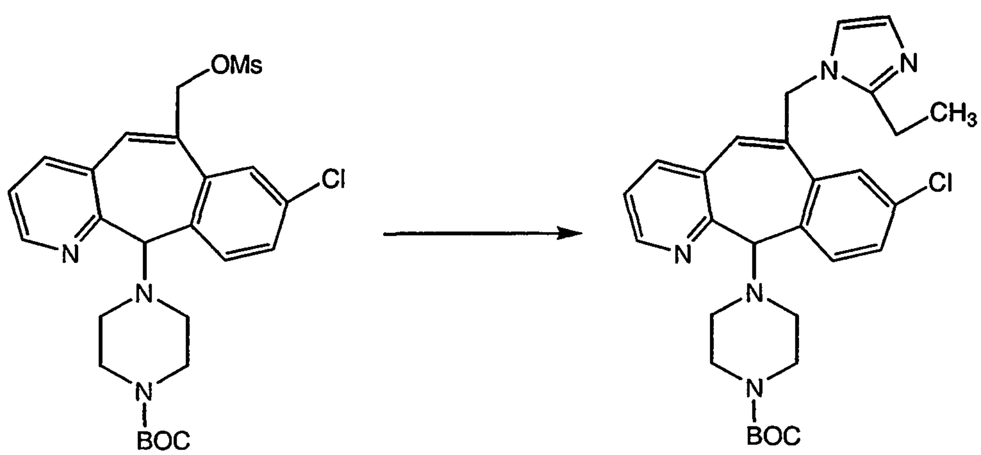

369

376a/
376b

To a solution of 1-ethylimidazole (0.33g, 3.46 mmol) in DMF (5 mL) under nitrogen was added NaH (0.083g, 3.46 mmol) and stirred for 10 minutes, followed by the addition of Compound (369) from Preparative Example 42, Step D (0.6g, 1.15 mmol) and stirred for over night. The solution was then evaporated to dryness, diluted with ethyl acetate, washed with sodium bicarbonate, dried over sodium sulfate and concentrated to dryness. The reaction mixture was purified by column chromatography on silica gel, eluted with 3% MeOH/97% $CH_2Cl_2$ to give a mixture of isomers. Further separation was accomplished using prep. HPLC with a chiral AD column to afford pure Compound (376a) (isomer 1) and Compound (376b) (isomer 2) (MH+=520.1).

B. Preparation of compounds (377a) AND (377b).

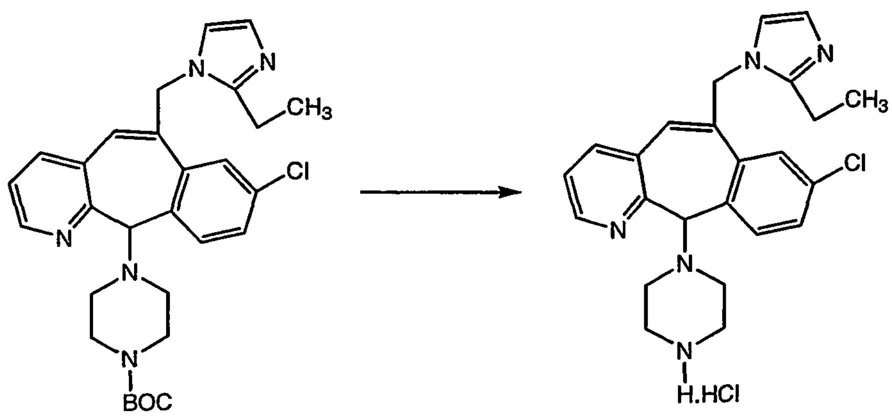

376a 377a

A solution of Compound (376a) from Step A (0.107g, 0.2 mmol) in 4M HCl in Dioxane (10 mL) was stirred for two hours at room temperature then concentrated to dryness to afford Compound (377a) (isomer 1) (0.13g, 100% yield, $MH^{+}$=420.1).

Compound (376b) was reacted in a similiar manner as above to afford Compound (377b) (isomer 2) ($MH^{+}$=420.1).

EXAMPLES 171-174

Starting with the appropriate (+) or (-) isomer of Compound (377) and reacting in a similiar manner as in Example 13 using the appropriate isocyanate as shown in the table below, the following compounds were prepared:

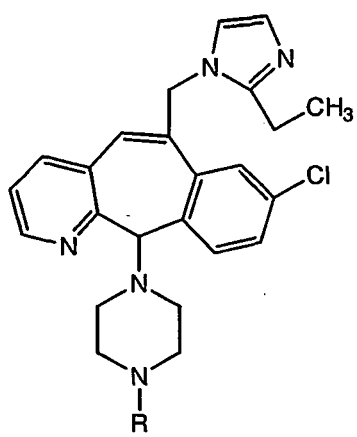

| EX. # | PROCEDURE | R= | CMPD # | PHYS. DATA |
|---|---|---|---|---|
| 171 | Example 13 | 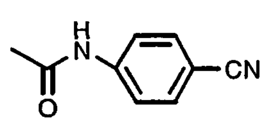 | 378 isomer 1 | MS MH+=504 |

(continued)

| EX. # | PROCEDURE | R= | CMPD # | PHYS. DATA |
|---|---|---|---|---|
| 172 | Example 13 | 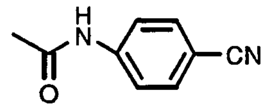 | 379 isomer 2 | MS MH+=504 |
| 173 | Example 13 | 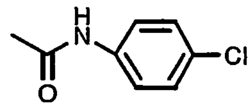 | 380 isomer 1 | MS MH+=573 |
| 174 | Example 13 | 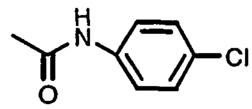 | 381 isomer 2 | MS MH+=573 |

PREPARATIVE EXAMPLE 44

Compounds (382a) AND (382b).

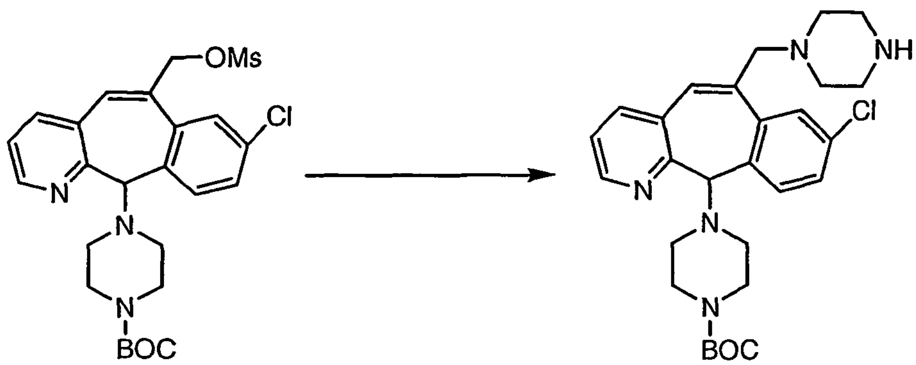

369

382a/ 382b

To a solution of Compound (369) from Preparative Example 42, Step D (0.5 g, 0.96 mmol) in $CH_3CN$ (80 mL), was added piperazine (0.25 g, 2,88 mmol) and 2,6-bis (dimethyl)-1-methylpiperidine (0.597 g, 3.84 mmol). The resulting solution was stirred at room temperature for 4hrs,concentrated to dryness and extracted with $CH_2Cl_2$-$NaHCO_3$. The combined organic layer was dried over $Na_2SO_4$ and purified by column chromatography on silica gel, eluting with 3%MeOH/ 97%$CH_2Cl_2$ to give the product of 2 isomers (0.28 g, 57% yield). These two isomers were seperated by HPLC on chiral AD column to give pure Compound (382a) (isomer 1) (0.136 g, $MH^+$=510.3) and Compound (382b) (isomer 2) (0.14 g, $MH^+$=510.3)

PREPARATIVE EXAMPLE 45

A. Compounds (383a) AND (383b).

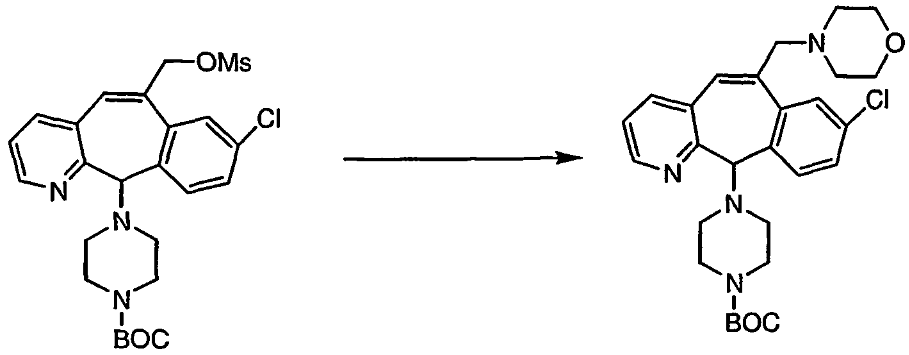

369 383a/ 383b

To a solution of Compound (369) from Preparative Example 42, Step D (1.2 g, 2.31 mmol) in $CH_3CN$ (100 mL), was added morpholine (0.8 g, 9.23 mmol) and 2,6-bis (dimethyl)-1-methylpiperidine (1.9 g, 12.24 mmol). The resulting solution was stirred at room temperature overnight and concentrated to dryness, followed by extraction with $CH_2Cl_2$-$NaHCO_3$. The combined organic layer was dried over $Na_2SO_4$ and purified by column chromatography on silica gel, eluting with 1%$NH_3$-MeOH/99%$CH_2Cl_2$ to give the product of two isomers (1.1 g, 82% yield). These two isomers were separated by HPLC on chiral AD column to give pure Compound (383a) (isomer 1) (0.24 g, $MH^+$=425.1) and Compound (383b) (isomer 2) (0.112 g, $MH^+$=425.1).

B. Preparation of Compound (384a)

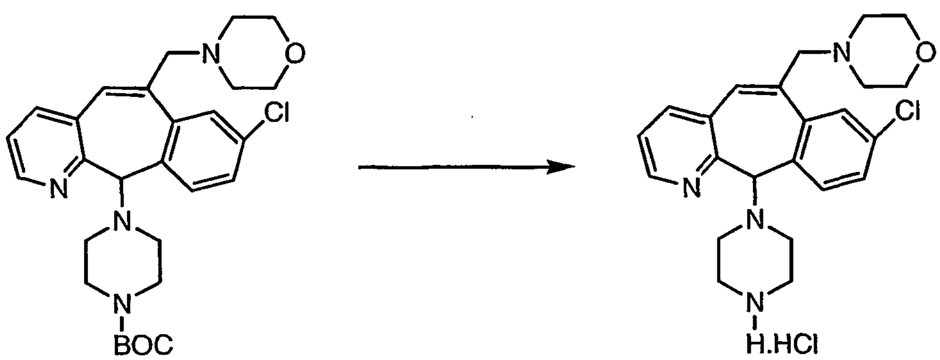

383a 384a

A solution of Compound (383a) from Step A (0.19 g, 0.37 mmol) in 4M HCl/Dioxane (25 mL) was stirred at room temperature for 2.5 hrs and concentrated to dryness to give Compound (384a) (0.194 g, $MH^+$=411.1).
Compound (384b) was prepared in a similar manner as above starting with Compound (383b) from Step A.

EXAMPLE 175

Preparation of Compounds (385a) AND (385b).

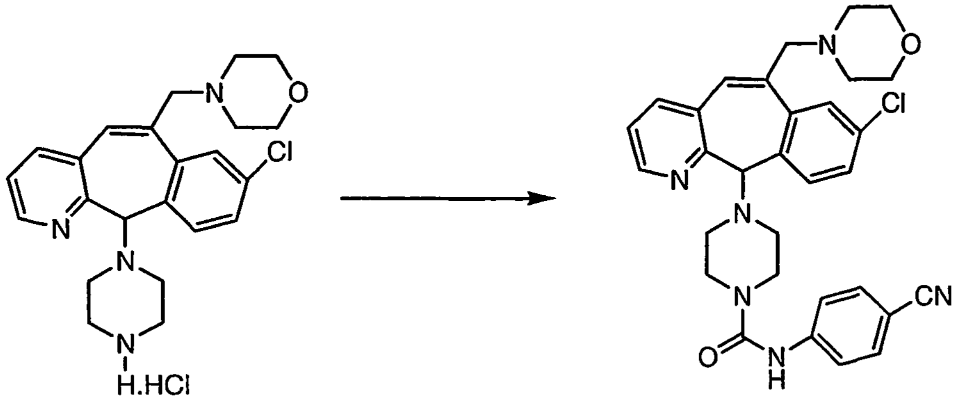

384a 385a

To a solution of Compound (384a) from Preparative Example 45, Step B above (0.05 g, 0.11 mmol) in anhydrous $CH_2Cl_2$ (5 mL) was added triethyl amine (0.036 g, 0.36 mmol) and 4-cyanophenyl isocyanate (0.018 g, 0.173 mmol). The resulting solution was stirred at room temperature for 4 hrs under nitrogen and concentrated to dryness, followed by extraction with $CH_2Cl_2$-$NaHCO_3$. The combined organic layer was dried over $Na_2SO_4$ and concentrated to dryness to give Compound (385a) (isomer 1) (0.06 g, 100% yield, $MH^+$=555.4).

Starting with Compound (384b) from Preparative Example 45, Step B and reacting it in the same manner as above, Compound (385b) (isomer 2) was prepared ($MH^+$=555.4).

PREPARATIVE EXAMPLE 46

A. Preparation of Compound (386)

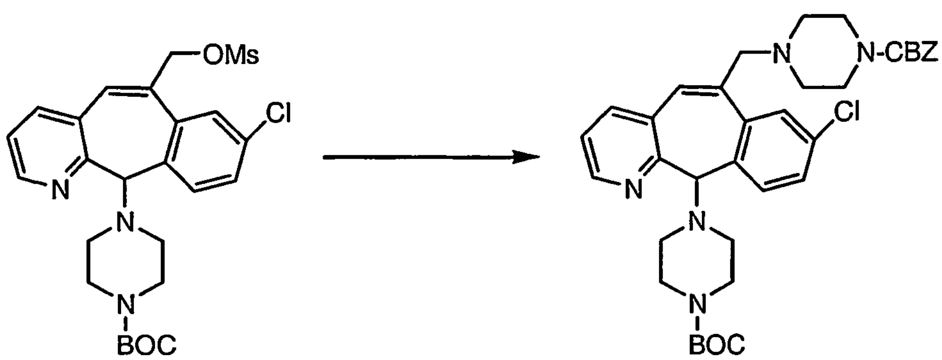

369 386

To a solution of Compound (369) from Preparative Example 42 Step D (3.0 g, 5.77 mmol) in $CH_3CN$ (150 mL) was added 2,6 -bis (dimethyl)-1 methyl piperidine (7.16 g, 16.16 mmol) and benzyl-1-piperazinecarboxylate (7.61 g, 34.62 mmol). The resulting solution was stirred overnight, concentrated to dryness, followed by extraction with $CH_2Cl_2$-$NaHCO_3$. The combined organic layer was dried over $Na_2SO_4$, concentrated to dryness and purified by column chromatography on silica gel, eluting with 1% $NH_3$-MeOH/99% $CH_2Cl_2$ and then 30%EtOAc/ 70% hexane to give the title product Compound (386) (1.24 g, 67% yield, $MH^+$=644.2)

B. Preparation of Compound (387).

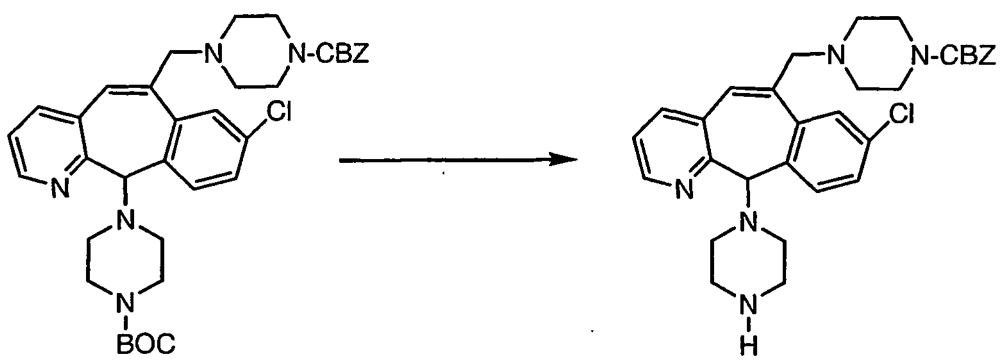

386 387

A solution of Compound (386) from Step A above (0.5 g, 0.77 mmol) in 4M HCl /Dioxane (50 mL) was stirred at room temperature for 2 hrs. The solution was then poured onto ice and basified with 1N NaOH solution, followed by extraction with $CH_2Cl_2$. The combined organic layer was dried over $Na_2SO_4$ and concentrated to dryness to give Compound (387) (0.43 g, 100% yield, $MH^+$=544.5).

C. Preparation of Compounds (388a) AND (388b).

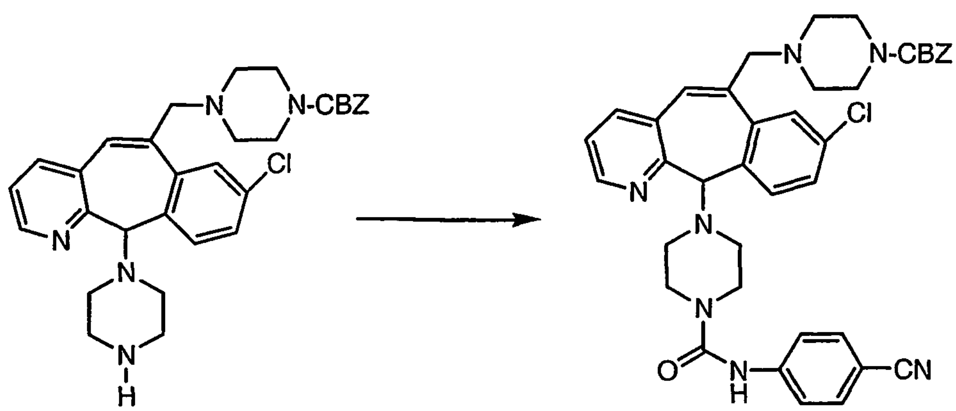

387 388a/ 388b

Compound (387) from Step B above was reacted In a similar manner to that described in Example 175 to give a mixture of 2 isomers (0.102 g, 55% yield). Further separation by HPLC, using a chiral AD column afforded pure Compound (388a) (isomer 1) (0.05 g, $MH^+$=688.2) and Compound (388b) (isomer 2) (0.048 g, $MH^+$=688.2).

EXAMPLES 176 AND 177

Reacting Compound (387) from Preparative Example 46, Step B in a similiar manner as in Example 175 using the appropriate isocyanate as shown in the table below, the following compounds were prepared:

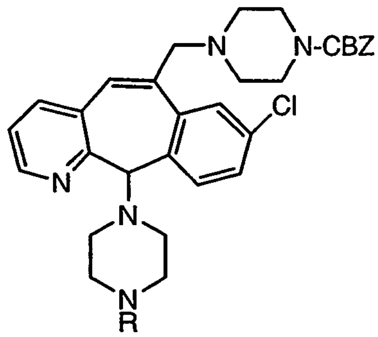

| EX. # | PROCEDURE | R= | CMPD # | PHYS. DATA |
|---|---|---|---|---|
| 176 | Example 175 | 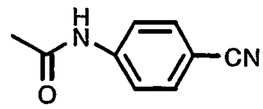 | 389 isomer 1 | MS MH+=688 |
| 177 | Example 175 | 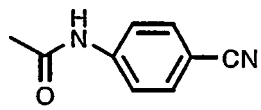 | 390 isomer 2 | MS MH+=688 |

EXAMPLE 178

Preparation of Compounds (391a) AND (391b).

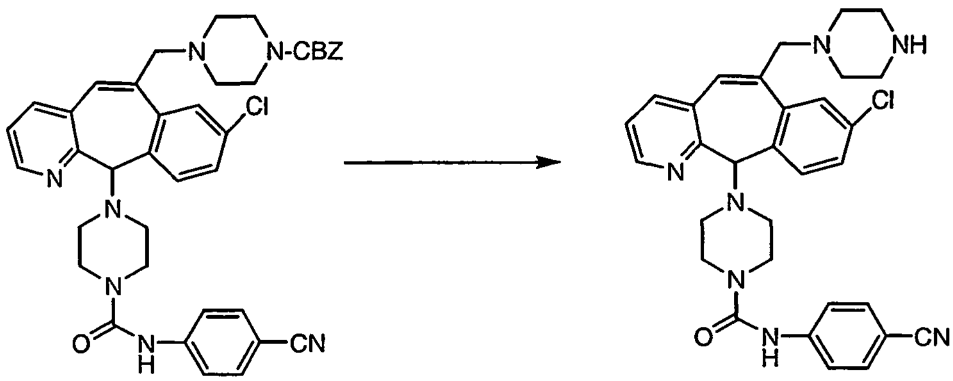

388a/
388b

391a/
391b

To a solution of Compound (388a) from Preparative Example 46, Step C (0.05 g, 0.086 mmol) in $CH_3CN$ (1 mL) at 0°C was added iodotrimethylsilane (0.05 mL, 0.343 mmol). The resulting solution was stirred at 0°C for 1 hr and concentrated to dryness. The residue was then poured onto 1N HCl solution, followed by extraction with ether. The aqueous layer was then basified with 10% $NH_4OH$ solution and then extracted with $CH_2Cl_2$. The combined organic layer was dried over $Na_2SO_4$ and concentrated to dryness affording Compound (391 a) (isomer 1) (0.02 g, 42.5% yield, $MH^+$= 554.1).

Starting with Compound (388b) from Preparative Example 46, Step C, and reacting in the same manner as above, Compound (391 b) (isomer 2) was prepared ($MH^+$= 554.1).

PREPARATIVE EXAMPLE 47

A. COMPOUND (394).

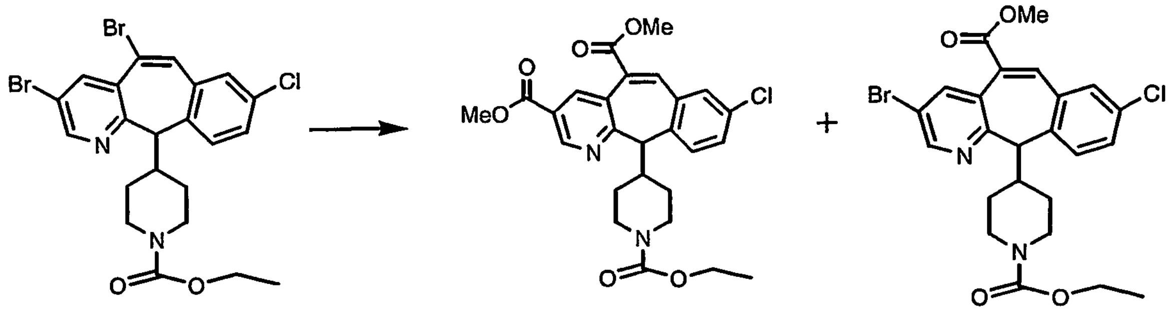

392 393 394

To a solution of Compound (392) prepared according to the procedure in, The Journal of Medicinal Chemistry (1998), 41(10), 1563 (5.0 g, 9.24 mmol) in MeOH (20 mL) and toluene (50 mL), at room temperature, was added triphenylphosphine (1.21 g, 4.62 mmol), DBU (1.90 g, 12.48 mmol) and palladium chloride (0.16 g, 0.92 mmol). The resulting solution was stirred at 80˚C for 6 hrs, then stirred at room temperature overnight. The solution was then concentrated to dryness to give two products. The desired product was purified by column chromatography on normal phase silica gel, eluting with 30% EtOAc/70%hexane to give a white solid compound (394) (2.24 g, 47% yield, $MH^+$=521.1)

B. Preparation of Compound (395).

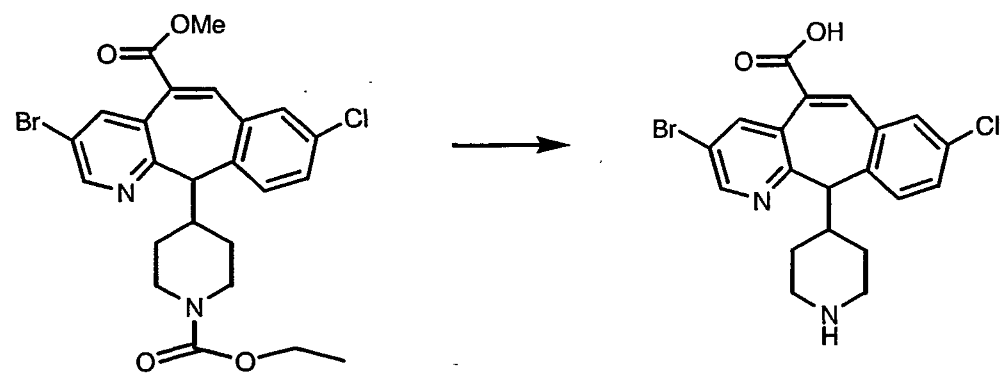

394 395

A solution of Compound (394) from Step A above (2.38 g, 4.58 mmol) in concentrated HCL (40 mL) was heated to reflux over night. The solution was then cooled down at room temperature and basified with $NH_4OH$ solution, followed by extraction with $CH_2Cl_2$. The combined organic layer was dried over $MgSO_4$, filtered and concentrated to dryness to give a white solid Compound (395) (1.03 g, 52% yield, $MH^+$=435.1).

C. Preparation of Compound (396).

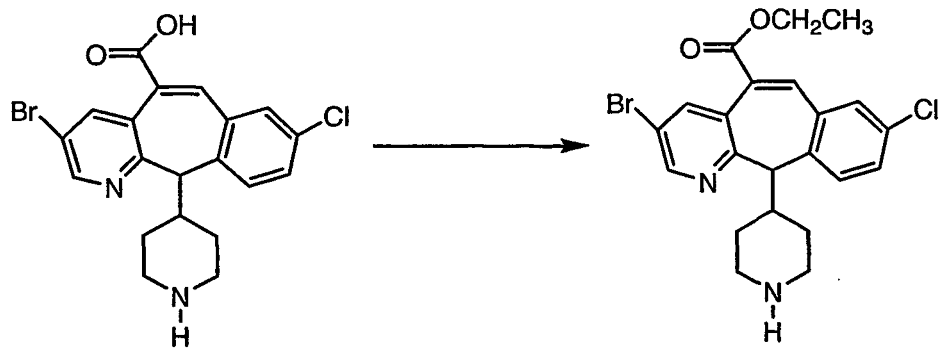

395 396

To a solution of Compound (395) from Step B (1.03 g, 2.37 mmol) in EtOH (50 mL, 200 proof) at room temperature, was bubbled in anhydrous $CH_2Cl_2$ gas for 5 minutes. The solution was then heated at 60°C for 30 minutes, cooled down to room temperature and concentrated to dryness to afford Compound (396) (1.1 g, 100% yield, MH+=463.1)

D. Preparation of Compound (397).

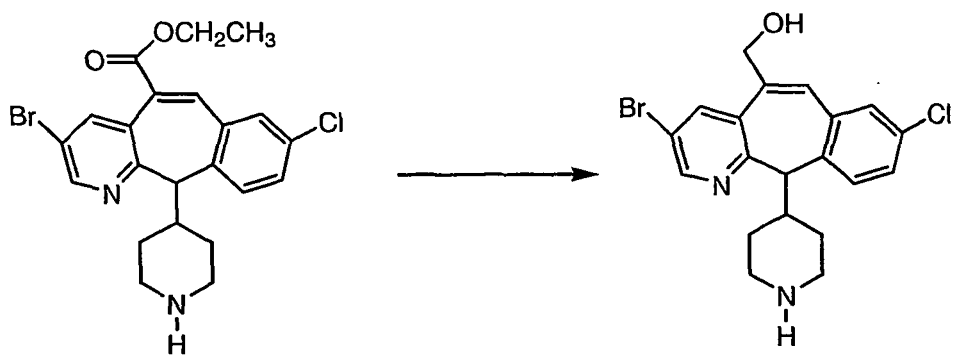

396 397

To a solution of Compound (396) from Step C (1.09 g, 2.19 mmol) in THF (10 mL) at 0°C was added dropwise DIBAL/toluene (11.0 mL, 10.95 mmol). The resulting solution was stirred overnight at room temperature, then quenched with $H_2O$ and concentrated to dryness to give a light brown solid Compound (397) (1.2 g, 100% yield, MH+=421.1).

E. Preparation of Compound (398).

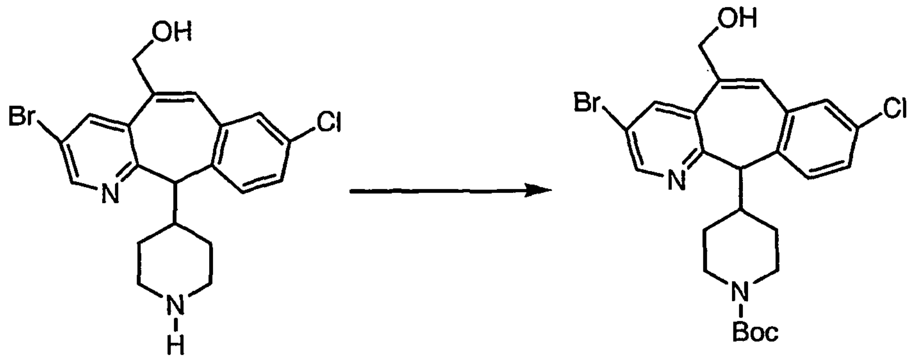

397 398

To a solution of Compound (397) from Step D (0.92 g, 2.19 mmol) in 50% MeOH/1% $H_2O$ (50 mL) at room temperature, was added Boc anhydride (0.95 g, 4.38 mmol). The resulting solution was adjusted to pH=9 and stirred at room temperature for 4 hrs and concentrated to dryness, followed by extraction with $CH_2Cl_2$-$H_2O$. The combined organic layer was dried over $MgSO_4$, filtered and concentrated to dryness to give a light brown solid Compound (398) (0.91 g, 80% yield, $MH^+$=521.1).

F Preparation of Compound (399).

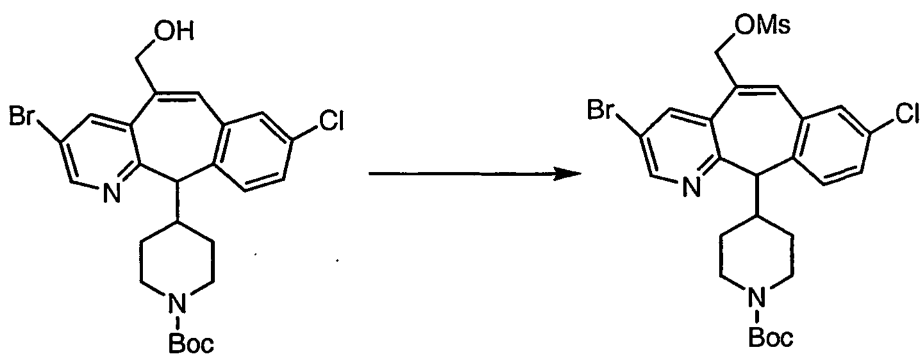

398 399

To a solution of Compound (398) from Step E (0.91 g, 1.75 mmol) in $CH_2Cl_2$ (10 mL) was added triethyl amine (0.73 mL, 5.25 mmol) and methanesulfonyl chloride (0.3 g, 2.62 mmol). The resulting solution was stirred at room temperature overnight and then washed with $NaHCO_3$ solution, dried over $Na_2SO_4$, filtered and concentrated to dryness to give the mesylate as a light yellow solid Compound (399) (0.94 g, 90% yield).

G. Preparation of Compounds (400a) and (400b).

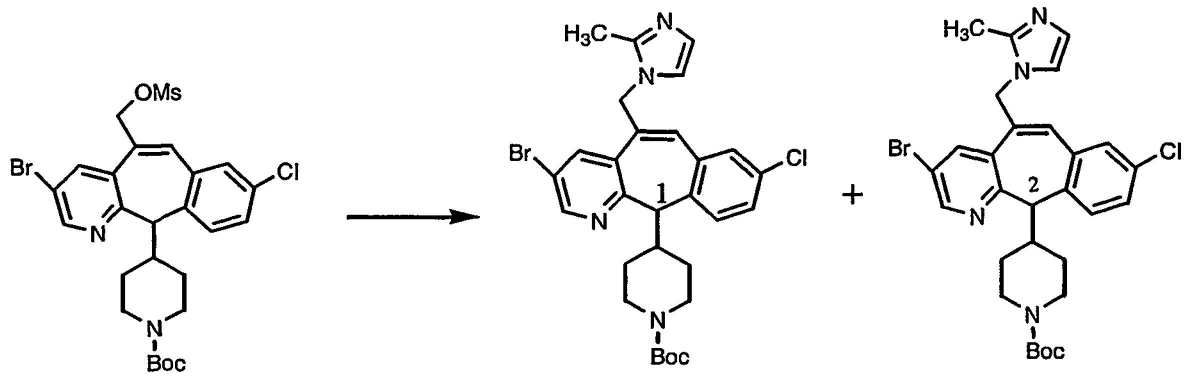

399 400a 400b

To a solution of Compound (399) from Step F (0.93 g, 1.60 mmol) in DMF (10 mL) under nitrogen, was added 2-methylimidazole (0.19 g, 2.3 mmol) and NaH (0.037g). The resulting solution was stirred at room temperature for 15 minutes, then at 90˚C for 3hrs. The solution was then cooled down to room temperature and concentrated to dryness, followed by extraction with $CH_2Cl_2$-$NaHCO_3$. The combined organic layer was dried over $MgSO_4$, filtered, concentrated and purified by column chromatography on normal phase silica gel, eluting with 5%MeOH-$NH_3$/95%$CH_2Cl_2$ to give mixture of two isomers as a light red solid (0.39 g, 42% yield, $MH^+$=585.1). The 2 isomers were separated by prep HPLC, using a chiral AD column, eluting with 15%IPA/85%hexane/0.2%DEA to give Compound (400a) (isomer 1) as a light brown solid (0.10 g, 11% yield) and Compound (400b) (isomer 2) as a white solid (0.10g, 11% yield)

H. Preparation of Compound (401).

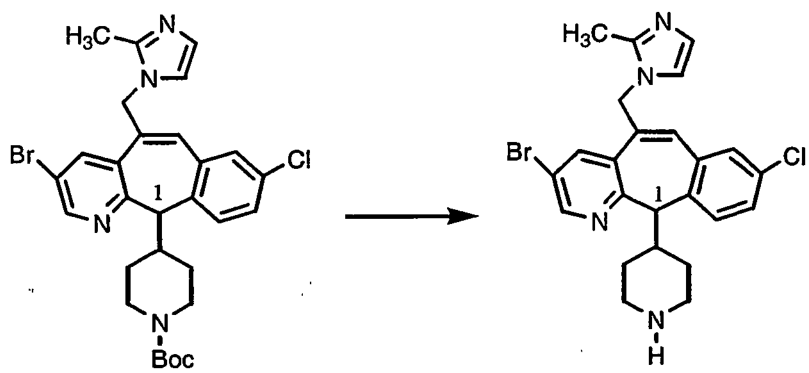

400a 401

A solution of Compound (400a) (isomer 1) from Step G above (0.07 g, 0.12 mmol) in 4M HCl/Dioxane (3 mL) was stirred at room temperature for 3 hrs then concentrated to dryness to give a white solid Compound (401) (0.06 g, 100% yield)

I. Preparation of Compound (402).

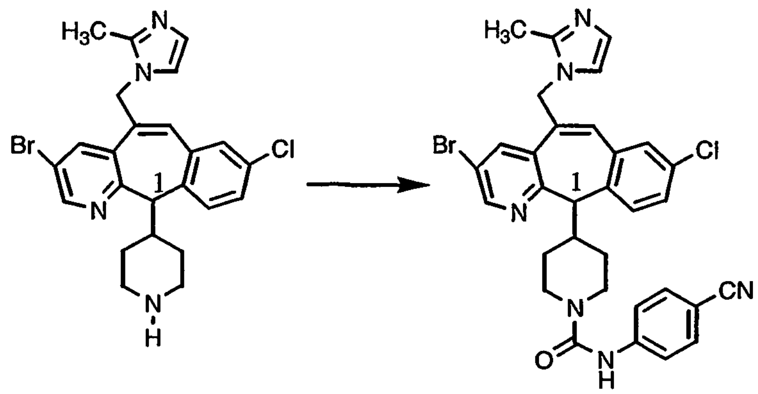

401 402

To a solution of Compound (401) from Step H above (0.057 g, 0.12 mmol) in $CH_2Cl_2$ (5 mL) under nitrogen, was added triethyl amine (0.026 g, 0.20 mmol) and 4-cyanophenyl isocyanate (0.019 g, 0.13 mmol). The resulting solution was stirred at room temperature overnight and then extracted with $CH_2Cl_2$-$NaHCO_3$. The combined organic layer was dried over $Na_2SO_4$, filtered, concentrated to dryness to afford Compound (402) (isomer 1) as a white solid (0.053 g, 70% yield, $MH^+$=629.3)

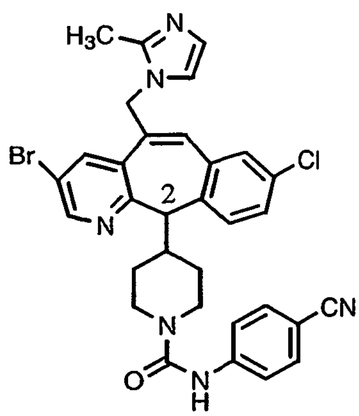

403

Compound (400b) was reacted in a similar manner as in Steps H and I above to afford Compound (403) (isomer 2) (0.059 g, 79% yield, MH+=629.3)

PREPARATIVE EXAMPLE 48

Compound (404)

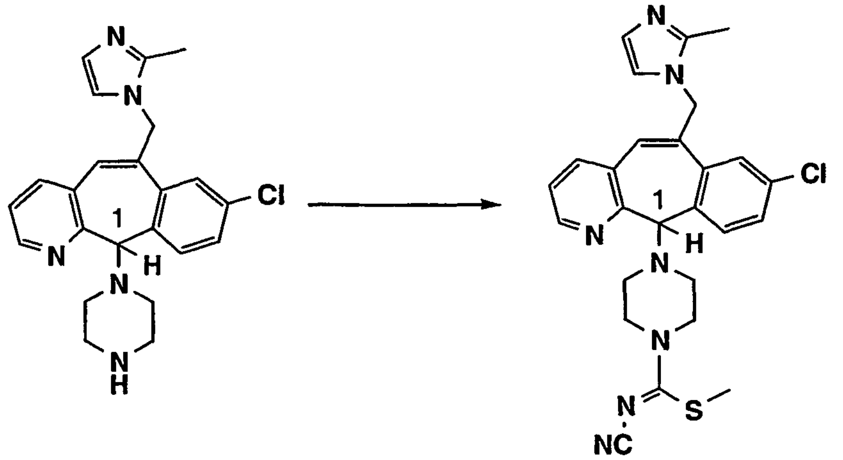

371a 404

Compound (371a) (isomer 1) from Preparative Example 42, Step F (70 mg, 0.17 mmol) was dissolved in 1 mL of ethanol and 50 uL of triethylamine. Dimethyl-N-cyanimidothiocarbonate (45 mg, 0.29 mmol) was added and the reaction mixture and stirred at 85 ˚C for 24 hours. The ethanol was evaporated under reduced pressure and the product chromatographed on silica gel using 5% methanolic-ammonia dichloromethane to obtain 47 mg of title product Compound (404) (FABMS M+1=504).

<u>EXAMPLE 179</u>

<u>Preparation of Compound (405).</u>

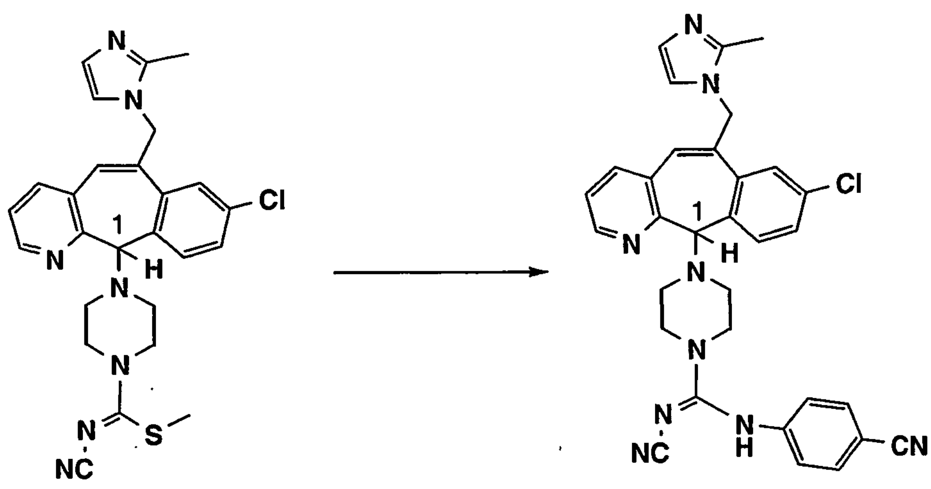

404 405

To a solution of para-cyanoanaline (53 mg, 0.45 mmol) in1 ml N,N-dimethylformamide was added sodium hydride (18 mg, 0.45 mmol). After stirring under a dry nitrogen atmosphere for 1/2 hour, Compound (404) (isomer 1) from Preparative Example 48 above (40 mg, 0.08 mmol) was added and the reaction mixture stirred at 55 ˚C for 4 hours. The reaction mixture was cooled to ambient temperature and added to brine. The crude product was extracted with dichloromethane 3 times. The extracts were concentrated and the crude product chromatographed on silica gel using 5% methanolic-ammonia/dichloromethane to otain 17.6 mg of title product.(405) FABMS M+1=574.1

EXAMPLES 180 AND 181

Preparation of Compounds (407) AND

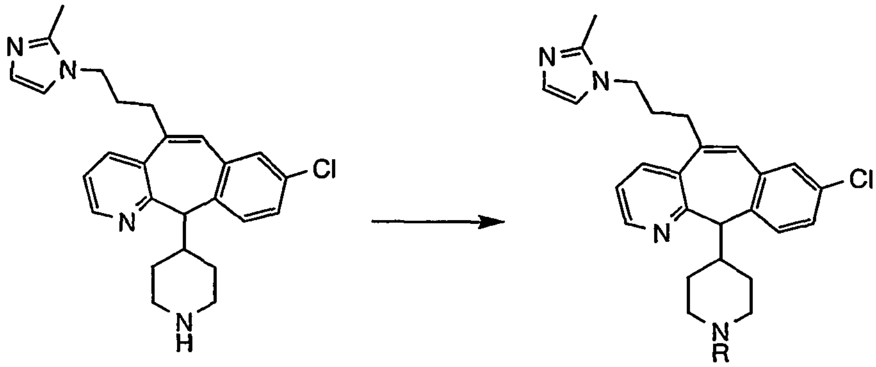

(408). 696a

Compound (696a) from Preparative Example 59, Step B, was reacted in the same manner as in Preparative Example 48 and Example 179 substituting the appropriate R reagent to afford the following compounds:

| EX. # | R= | CMPD # | PHYS. DATA |
|---|---|---|---|
| 180 | 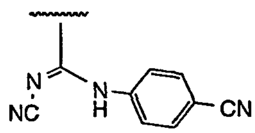 | 407 | FABMS MH+=601.1 |
| 181 | 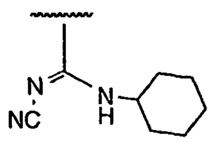 | 408 | FABMS MH+=531.1 |

PREPARATIVE EXAMPLE 49

COMPOUNDS (51a) AND (52a)

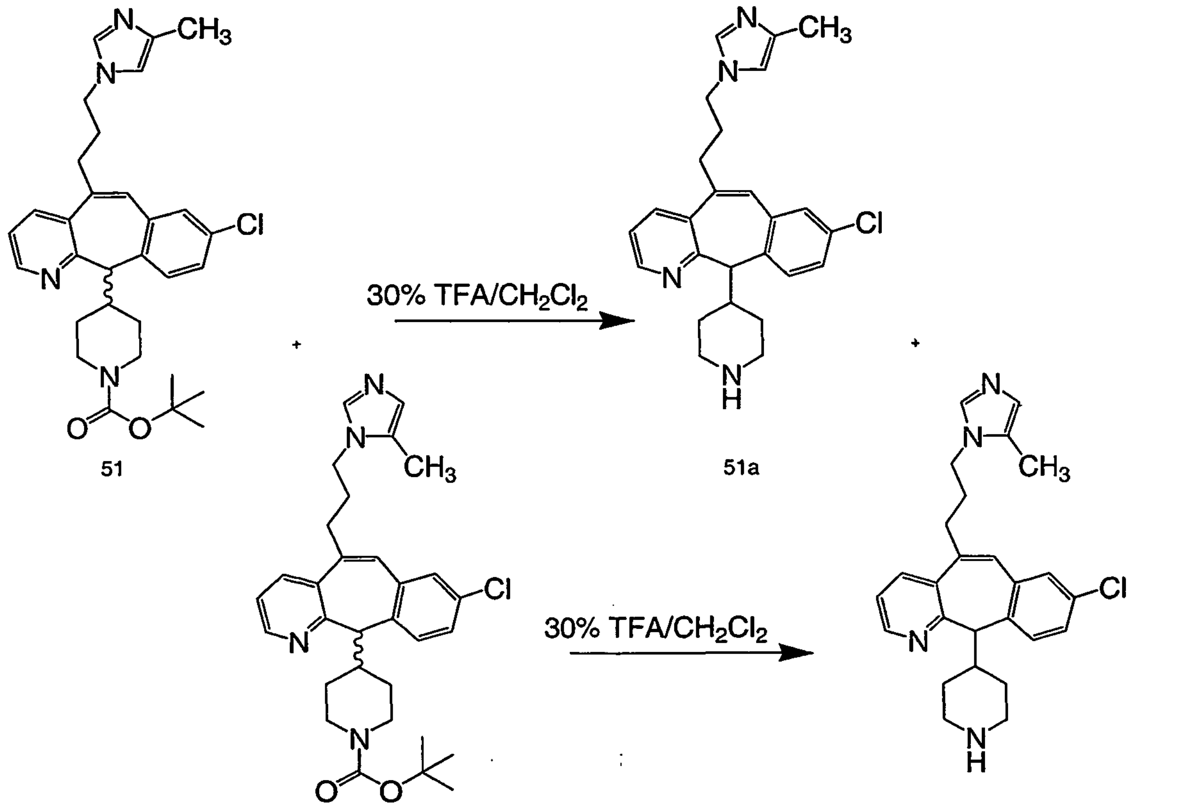


51 + 52 → 51a + 52a

Compounds (51) and (52) from Example 11, Step A, were reacted with TFA in $CH_2Cl_2$ to afford compounds (51a) and (52a).

<u>Library Preparation</u>

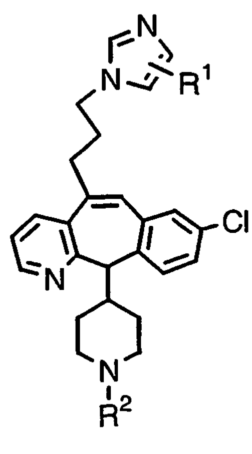

Figure 1

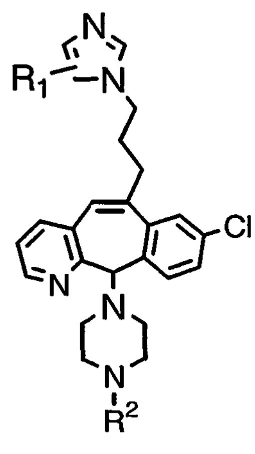

Figure 2

A library of compounds was prepared by solution phase parallel synthesis. A generic structure of these compounds is shown in Figure 1 above. The $R^1$ group on the imidazole ring can be H or $CH_3$, the $R^2$ on N-1 of the piperidine is varied in the library.

Library compounds were prepared using compound (29) from Preparative Example 4 or Compounds (51a) or (52a) from Preparative Example 49 above as templates as shown in Scheme A. Synthesis is initiated in test tubes by reacting compound (29), (51a) or (52a) with multiple equivalents of a variety of isocyanates, amines, acids, acid chlorides, sulfonyl chlorides and chloroformates in dichloromethane or chloroform. When urea is the desired product, the reaction can be carried out using isocyanates directly, or alternatively, treating an amine with CDI for several hours, then subject the templates to this solution overnight. When acids are used, the reaction is carried out in the presence of a coupling reagent such as PyBrop and a base such as DIEA overnight. When acid chlorides, sulfonyl chlorides or chloroformates are used, the reaction is typically conducted in the presence of triethylamine. After reaction, an excess amount of polystyrene aminomethyl resin is added to the reaction test tubes, and the reaction allowed to stand overnight. At which time each test tube is filtered through a Bio-Rad Poly-Prep chromatography column into another test tube, and the resin is washed with dichloromethane and MeOH. The combined filtrate solution is concentrated by rotovap evaporation. The residue in each test tube is then dissolved in $H_2O/CH_3CN$ (50/50, containing 1% TFA) and purified by Gilson 215 liquid Handling-HPLC system to give pure product. The product was identified by mass spectroscopy. Library compounds prepared in this fashion are shown in Table 1 and Table 2.

**Scheme A**

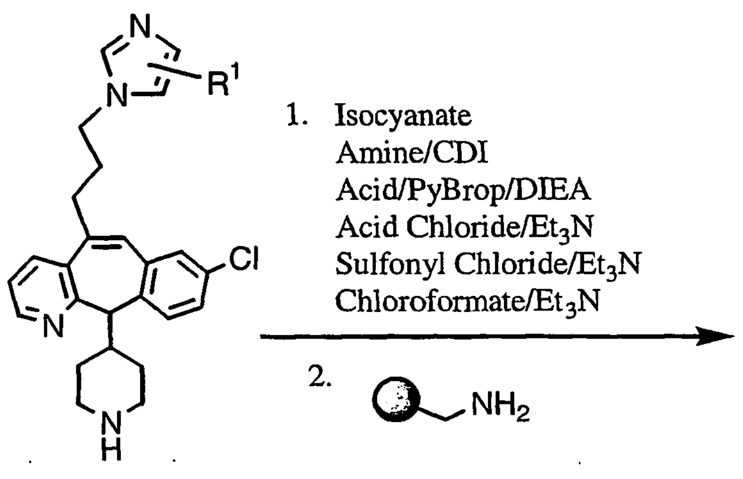


$R^1$ = H or $CH_3$ $R^1$ = H or $CH_3$

**Further purified by**
**Gilson Auto-Separation System**
**Biological Assay**

EXAMPLES 182-283

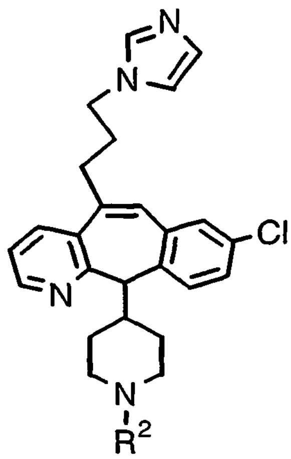

TABLE 1

| EXAMPLE #. | $R^2$ | COMPOUND # | PHYSICAL DATA |
|---|---|---|---|
| 182 | 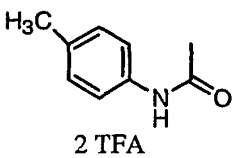 2 TFA | 409 | Mass spec. $MH^+$=552 |
| 183 | 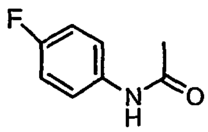 2 TFA | 410 | Mass spec. $MH^+$=556 |
| 184 | 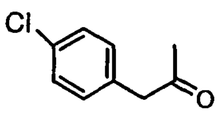 2 TFA | 411 | Mass spec. $MH^+$=571 |
| 185 | 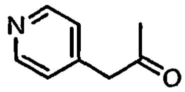 2 TFA | 412 | Mass spec. $MH^+$=538 |
| 186 | 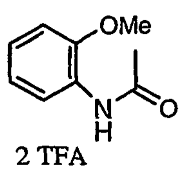 2 TFA | 413 | Mass spec. $MH^+$=568 |
| 187 | 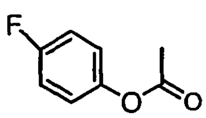 2 TFA | 414 | Mass spec. $MH^+$=557 |

(continued)

| EXAMPLE #. | $R^2$ | COMPOUND # | PHYSICAL DATA |
|---|---|---|---|
| 188 | 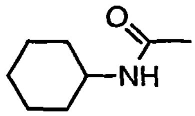 2 TFA | 415 | Mass spec. $MH^+$=544 |
| 189 | 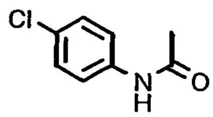 2 TFA | 416 | Mass spec. $MH^+$=572 |
| 190 | 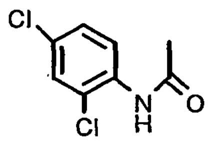 2 TFA | 417 | Mass spec. $MH^+$=606 |
| 191 | 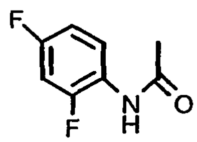 2 TFA | 418 | Mass spec. $MH^+$=574 |
| 192 | 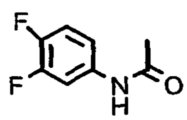 2 TFA | 419 | Mass spec. $MH^+$=574 |
| 193 | 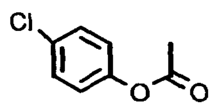 2 TFA | 420 | Mass spec. $MH^+$=573 |
| 194 | 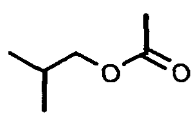 | 421 | Mass spec. $MH^+$=519 |
| 195 | 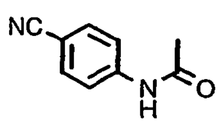 2 TFA | 422 | Mass spec. $MH^+$=563 |
| 196 | 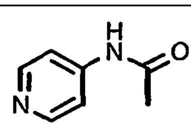 3 TFA | 423 | Mass spec. $MH^+$=539 |

(continued)

| EXAMPLE #. | $R^2$ | COMPOUND # | PHYSICAL DATA |
|---|---|---|---|
| 197 | 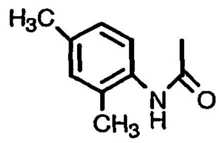 2 TFA | 424 | Mass spec. $MH^+$=566 |
| 198 | 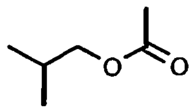 2 TFA | 425 | Mass spec. $MH^+$=505 |
| 199 | 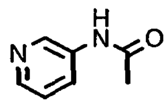 3 TFA | 426 | Mass spec. $MH^+$=539 |
| 200 | 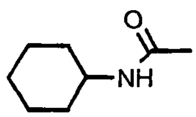 | 427 | Mass spec. $MH^+$=544 |
| 201 | 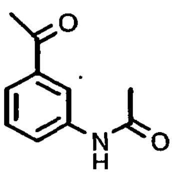 | 428 | Mass spec. $MH^+$=580 |
| 202 | 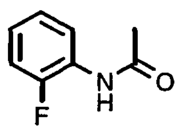 2 TFA | 429 | Mass spec. $MH^+$=556 |
| 203 | 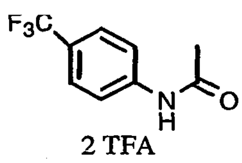 2 TFA | 430 | Mass spec. $MH^+$=606 |
| 204 | 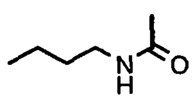 2 TFA | 431 | Mass spec. $MH^+$=518 |
| 205 | 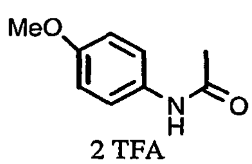 2 TFA | 432 | Mass spec. $MH^+$=568 |

(continued)

| EXAMPLE #. | $R^2$ | COMPOUND # | PHYSICAL DATA |
|---|---|---|---|
| 206 | 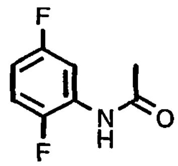 2 TFA | 433 | Mass spec. $MH^+$=574 |
| 207 | 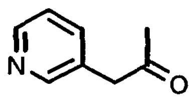 3 TFA | 434 | Mass spec. $MH^+$=538 |
| 208 | 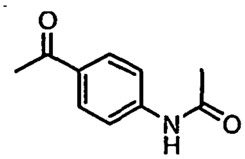 | 435 | Mass spec. $MH^+$=580 |
| 209 | 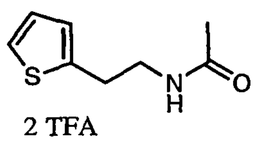 2 TFA | 436 | Mass spec. $MH^+$=572 |
| 210 | 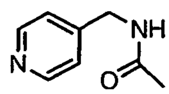 2 TFA | 437 | Mass spec. $MH^+$=553 |
| 211 | 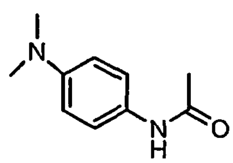 2 TFA | 438 | Mass spec. $MH^+$=581 |
| 212 | 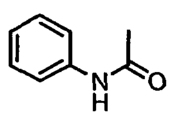 2 TFA | 439 | Mass spec. $MH^+$=538 |
| 213 | 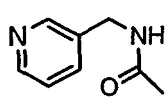 | 440 | Mass spec. $MH^+$=553 |
| 214 | 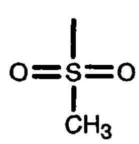 | 441 | Mass spec. $MH^+$=497 |

(continued)

| EXAMPLE #. | $R^2$ | COMPOUND # | PHYSICAL DATA |
|---|---|---|---|
| 215 | 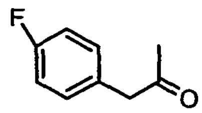 2 TFA | 442 | Mass spec. $MH^+$=555 |
| 216 | 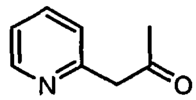 2 TFA | 443 | Mass spec. $MH^+$=538 |
| 217 | 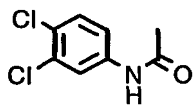 2 TFA | 444 | Mass spec. $MH^+$=606 |
| 218 | 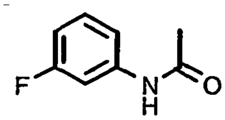 2 TFA | 445 | Mass spec. $MH^+$=556 |
| 219 | 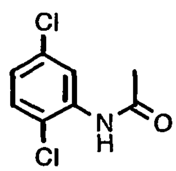 2 TFA | 446 | Mass spec. $MH^+$=606 |
| 220 | 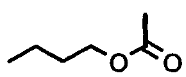 | 447 | Mass spec. $MH^+$=519 |
| 221 | 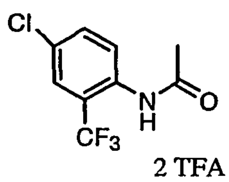 2 TFA | 448 | Mass spec. $MH^+$=640 |
| 222 | 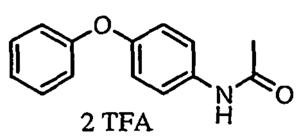 2 TFA | 449 | Mass spec. $MH^+$=630 |
| 223 | 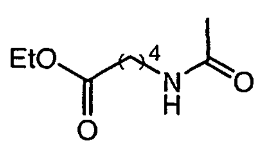 | 450 | Mass spec. $MH^+$=604 |
| 224 | 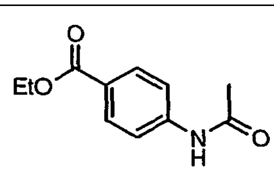 | 451 | Mass spec. $MH^+$=610 |

(continued)

| EXAMPLE #. | $R^2$ | COMPOUND # | PHYSICAL DATA |
|---|---|---|---|
| 225 | 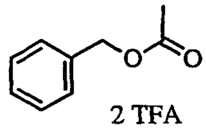 2 TFA | 452 | Mass spec. $MH^+$=553 |
| 226 | 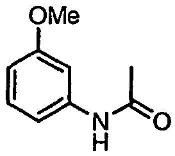 | 453 | Mass spec. $MH^+$=568 |
| 227 | 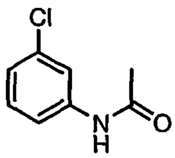 | 454 | Mass spec. $M^+$=572 |
| 228 | 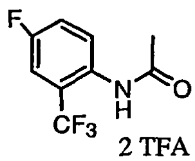 2 TFA | 455 | Mass spec. $MH^+$=624 |
| 229 | 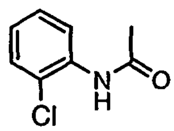 | 456 | Mass spec. $MH^+$=572 |
| 230 | 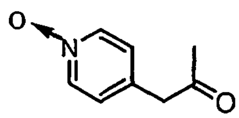 | 457 | Mass spec. $MH^+$=554 |
| 231 | 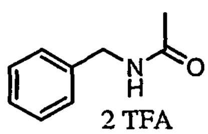 2 TFA | 458 | Mass spec. $MH^+$=552 |
| 232 | 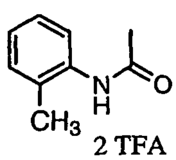 2 TFA | 459 | Mass spec. $MH^+$=552 |
| 233 | 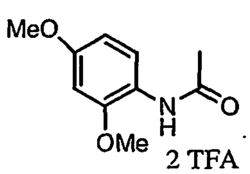 2 TFA | 460 | Mass spec. $MH^+$=598 |
| 234 | 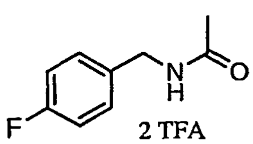 2 TFA | 461 | Mass spec. $MH^+$=570 |

(continued)

| EXAMPLE #. | $R^2$ | COMPOUND # | PHYSICAL DATA |
|---|---|---|---|
| 235 | 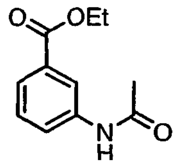 | 462 | Mass spec. $MH^+$=610 |
| 236 | 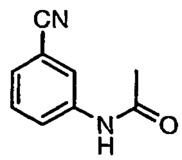 | 463 | Mass spec. $MH^+$=563 |
| 237 | 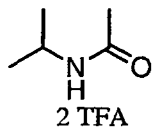 2 TFA | 464 | Mass spec. $MH^+$=504 |
| 238 | 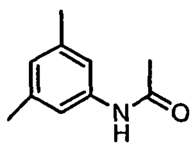 2 TFA | 465 | Mass spec. $MH^+$=566 |
| 239 | 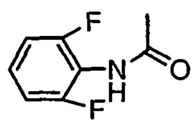 2 TFA | 466 | Mass spec.$MH^+$=574 |
| 240 | 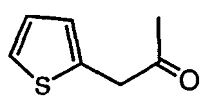 | 467 | Mass spec. $MH^+$=543 |
| 241 | 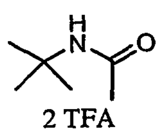 2 TFA | 468 | Mass spec. $MH^+$=518 |
| 242 | 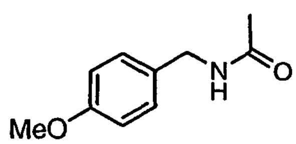 | 469 | Mass spec. $MH^+$=582 |
| 243 | 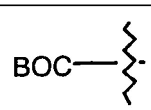 | 470 | Mass spec. $MH^+$=519 |
| 244 | 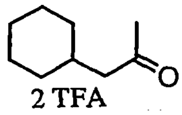 2 TFA | 471 | Mass spec. $MH^+$=543 |

(continued)

| EXAMPLE #. | $R^2$ | COMPOUND # | PHYSICAL DATA |
|---|---|---|---|
| 245 | 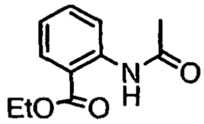 | 472 | Mass spec. $MH^+$=610 |
| 246 | 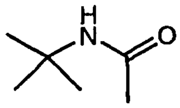 | 473 | Mass spec. $MH^+$=518 |
| 247 | 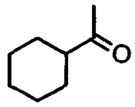 2 TFA | 474 | Mass spec. $MH^+$=529 |
| 248 | 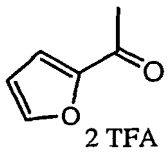 2 TFA | 475 | Mass spec. $MH^+$=513 |
| 249 | 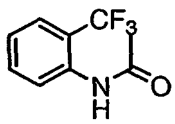 | 476 | Mass spec. $MH^+$=606 |
| 250 | 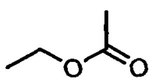 2 TFA | 477 | Mass spec. $MH^+$=491 |
| 251 | 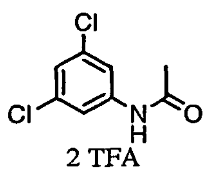 2 TFA | 478 | Mass spec. $MH^+$=606 |
| 252 | 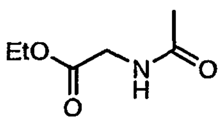 | 479 | Mass spec. $MH^+$=548 |
| 253 | 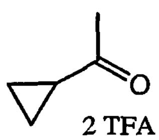 2 TFA | 480 | Mass spec. $MH^+$=487 |
| 254 | 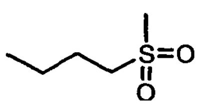 | 481 | Mass spec. $MH^+$=539 |
| 255 | 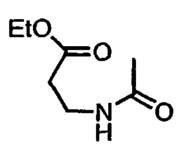 | 482 | Mass spec. $MH^+$=562 |

(continued)

| EXAMPLE #. | $R^2$ | COMPOUND # | PHYSICAL DATA |
|---|---|---|---|
| 256 | 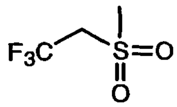 | 483 | Mass spec. $MH^+$=565 |
| 257 | 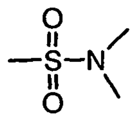 | 484 | Mass spec. $MH^+$=526 |
| 258 | 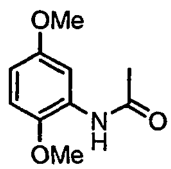 2 TFA | 485 | Mass spec. $MH^+$=598 |
| 259 | 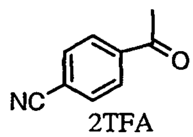 2TFA | 486 | Mass spec. $MH^+$=548 |
| 260 | 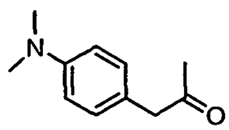 2 TFA | 487 | Mass spec. $MH^+$=580 |
| 261 | 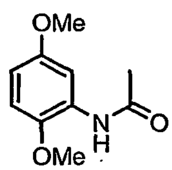 2 TFA | 488 | Mass spec. $MH^+$=598 |
| 262 | 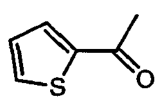 2 TFA | 489 | Mass spec. $MH^+$=529 |
| 263 | 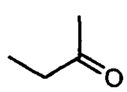 2 TFA | 490 | Mass spec. $MH^+$=475 |
| 264 | 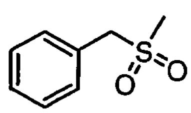 | 491 | Mass spec. $MH^+$=573 |
| 265 | 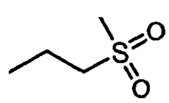 2 TFA | 492 | Mass spec. $MH^+$=525 |

(continued)

| EXAMPLE #. | $R^2$ | COMPOUND # | PHYSICAL DATA |
|---|---|---|---|
| 266 | 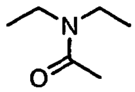 2 TFA | 493 | Mass spec. $MH^+$=518 |
| 267 | 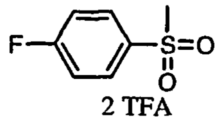 2 TFA | 494 | Mass spec. $MH^+$=577 |
| 268 | 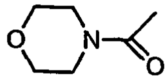 2 TFA | 495 | Mass spec. $MH^+$=532 |
| 269 | 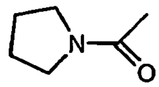 2 TFA | 496 | Mass spec. $MH^+$=516 |
| 270 | 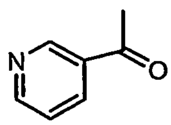 3 TFA | 497 | Mass spec. $MH^+$=524 |
| 271 | 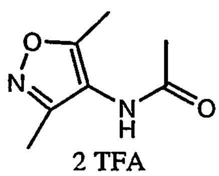 2 TFA | 498 | Mass spec. $MH^+$=557 |
| 272 | 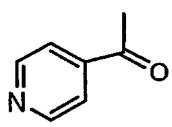 3 TFA | 499 | Mass spec. $MH^+$=524 |
| 273 | 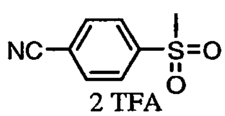 2 TFA | 500 | Mass spec. $MH^+$=584 |
| 274 | 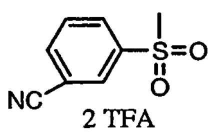 2 TFA | 501 | Mass spec. $MH^+$=584 |
| 275 | 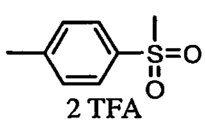 2 TFA | 502 | Mass spec. $MH^+$=573 |

(continued)

| EXAMPLE #. | $R^2$ | COMPOUND # | PHYSICAL DATA |
|---|---|---|---|
| 276 | 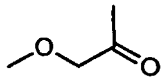 2 TFA | 503 | Mass spec. $MH^+$=491 |
| 277 | 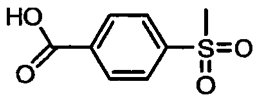 2 TFA | 504 | Mass spec. $MH^+$=603 |
| 278 | 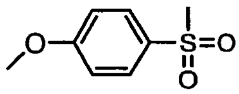 | 505 | Mass spec. $MH^+$=589 |
| 279 | 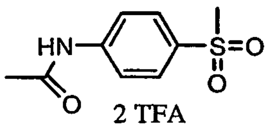 2 TFA | 506 | Mass spec. $MH^+$=616 |
| 280 | 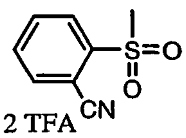 2 TFA | 507 | Mass spec. $MH^+$=584 |
| 281 | 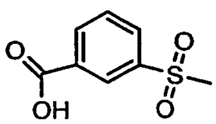 2 TFA | 508 | Mass spec. $MH^+$=603 |
| 282 | 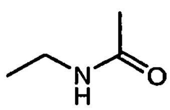 | 509 | Mass spec. $MH^+$=490 |
| 283 | 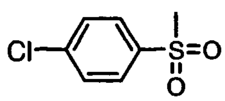 2 TFA | 510 | Mass spec. $MH^+$=593 |

EXAMPLES 284-377

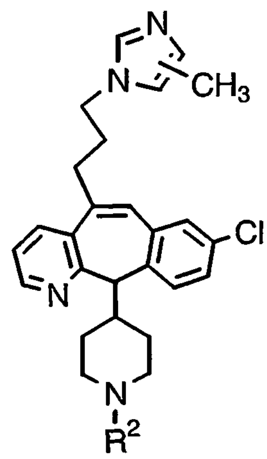

**TABLE 2**

| EXAMPLE # | $R^2$ | COMPOUND # | $MH^+$ |
|---|---|---|---|
| 284 | 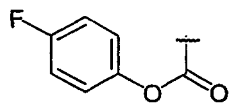 2 TFA | 511 | 571 |
| 285 | 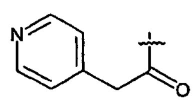 3 TFA | 512 | 552 |
| 286 | 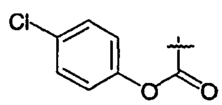 2TFA | 513 | 587 |
| 287 | 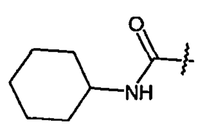 | 514 | 558 |
| 288 | 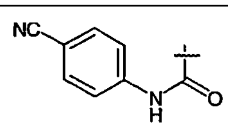 2 TFA | 515 | 577 |
| 289 | 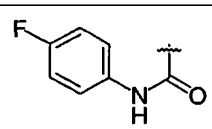 2 TFA | 516 | 570 |
| 290 | 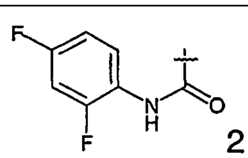 2 TFA | 517 | 588 |

(continued)

| EXAMPLE # | $R^2$ | COMPOUND # | $MH^+$ |
|---|---|---|---|
| 291 | 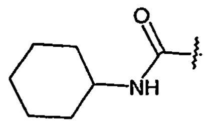 2 TFA | 518 | 558 |
| 292 | 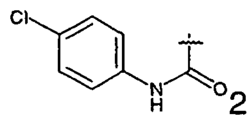 2 TFA | 519 | 586 |
| 293 | 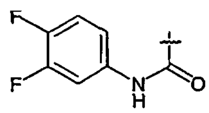 2 TFA | 520 | 588 |
| 294 | 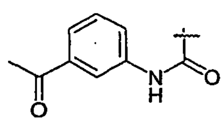 2 TFA | 521 | 594 |
| 295 | 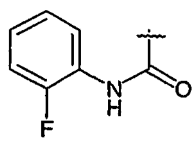 2 TFA | 522 | 570 |
| 296 | 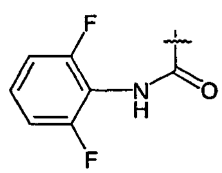 2 TFA | 523 | 588 |
| 297 | 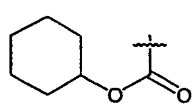 2 TFA | 524 | 559 |
| 298 | 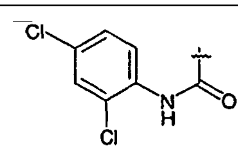 2 TFA | 525 | 620 |
| 299 | 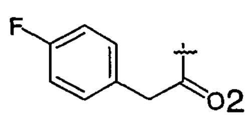 2 TFA | 526 | 569 |

(continued)

| EXAMPLE # | $R^2$ | COMPOUND # | $MH^+$ |
|---|---|---|---|
| 300 | 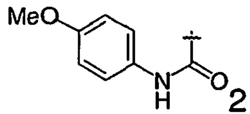 2 TFA | 527 | 582 |
| 301 | 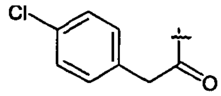 2 TFA | 528 | 585 |
| 302 | 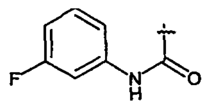 2 TFA | 529 | 570 |
| 303 | 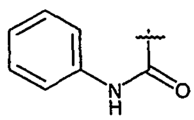 2 TFA | 530 | 552 |
| 304 | 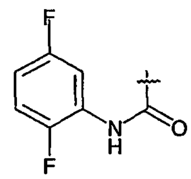 2 TFA | 531 | 588 |
| 305 | 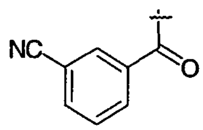 2 TFA | 532 | 562 |
| 306 | 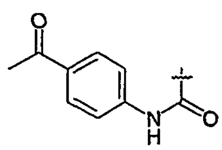 2 TFA | 533 | 594 |
| 307 | 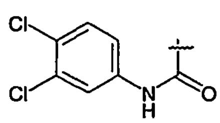 2 TFA | 534 | 620 |
| 308 | 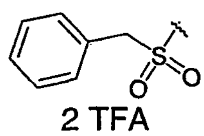 2 TFA | 535 | 587 |

(continued)

| EXAMPLE # | $R^2$ | COMPOUND # | MH+ |
|---|---|---|---|
| 309 | 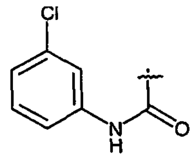 2 TFA | 536 | 586 |
| 310 | 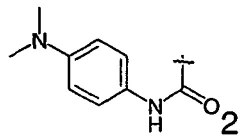 2 TFA | 537 | 595 |
| 311 | 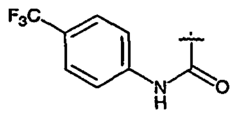 2 TFA | 538 | 620 |
| 312 | 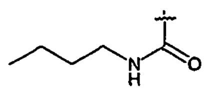 2 TFA | 539 | 532 |
| 313 | 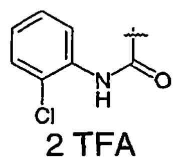 2 TFA | 540 | 586 |
| 314 | 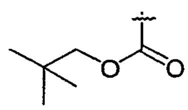 2 TFA | 541 | 547 |
| 315 | 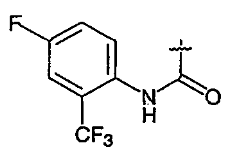 2 TFA | 542 | 638 |
| 316 | 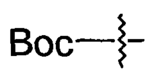 | 543 | 533 |
| 317 | 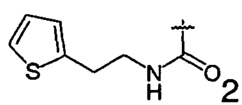 2 TFA | 544 | 586 |
| 318 | 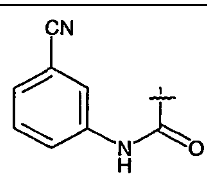 | 545 | 577 |

(continued)

| EXAMPLE # | $R^2$ | COMPOUND # | $MH^+$ |
|---|---|---|---|
| 319 | 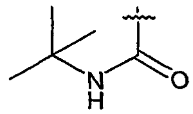 2 TFA | 546 | 532 |
| 320 | 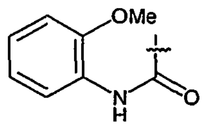 | 547 | 582 |
| 321 | 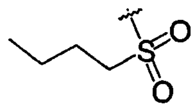 2 TFA | 548 | 553 |
| 322 | 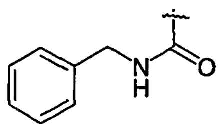 2 TFA | 549 | 566 |
| 323 | 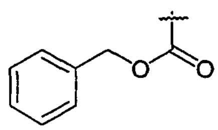 2 TFA | 550 | 567 |
| 324 | 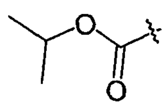 2 TFA | 551 | 519 |
| 325 | 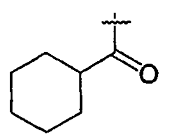 2 TFA | 552 | 543 |
| 326 | 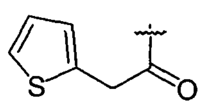 2 TFA | 553 | 557 |
| 327 | 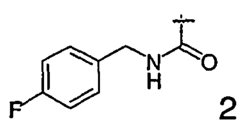 2 TFA | 554 | 584 |

(continued)

| EXAMPLE # | $R^2$ | COMPOUND # | $MH^+$ |
|---|---|---|---|
| 328 | 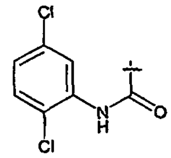 2 TFA | 555 | 620 |
| 329 | 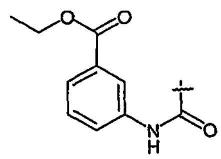 2 TFA | 556 | 624 |
| 330 | 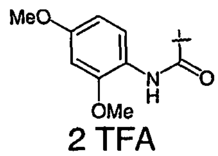 2 TFA | 557 | 612 |
| 331 | 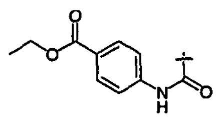 2 TFA | 558 | 624 |
| 332 | 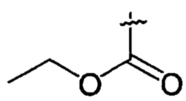 2 TFA | 559 | 505 |
| 333 | 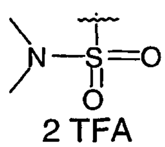 2 TFA | 560 | 540 |
| 334 | 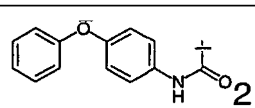 2 TFA | 561 | 644 |
| 335 | 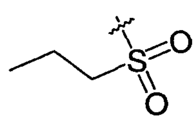 2 TFA | 562 | 539 |
| 336 | 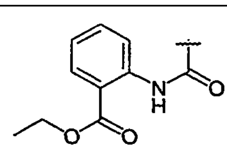 2 TFA | 563 | 624 |

(continued)

| EXAMPLE # | $R^2$ | COMPOUND # | $MH^+$ |
|---|---|---|---|
| 337 | 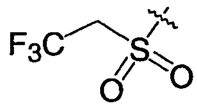 2 TFA | 564 | 579 |
| 338 | 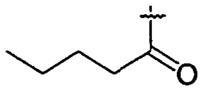 2 TFA | 565 | 517 |
| 339 | 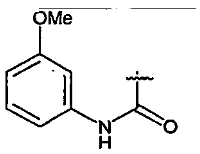 | 566 | 582 |
| 340 | 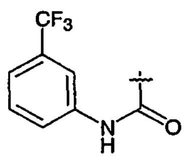 2 TFA | 567 | 620 |
| 341 | 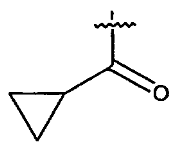 2 TFA | 568 | 501 |
| 342 | 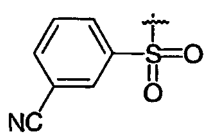 2 TFA | 569 | 598 |
| 343 | 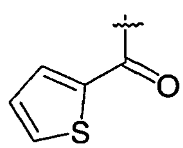 2 TFA | 570 | 543 |
| 344 | 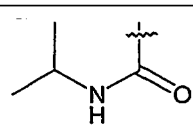 2 TFA | 571 | 518 |
| 345 | 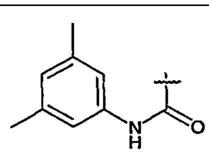 2 TFA | 572 | 580 |

(continued)

| EXAMPLE # | $R^2$ | COMPOUND # | $MH^+$ |
|---|---|---|---|
| 346 | 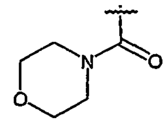 2 TFA | 573 | 546 |
| 347 | 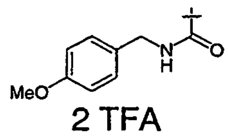 2 TFA | 574 | 596 |
| 348 | 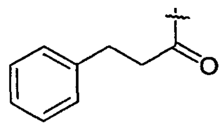 2 TFA | 575 | 565 |
| 349 | 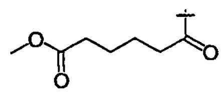 2 TFA | 576 | 575 |
| 350 | 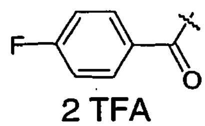 2 TFA | 577 | 555 |
| 351 | 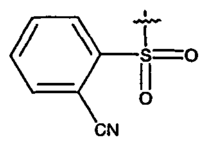 2TFA | 578 | 598 |
| 352 | 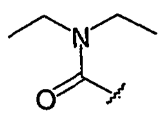 2 TFA | 579 | 532 |
| 353 | 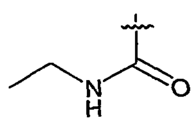 2 TFA | 580 | 504 |
| 354 | 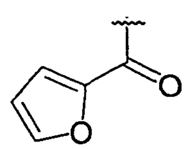 2 TFA | 581 | 527 |
| 355 | 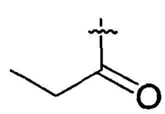 2 TFA | 582 | 489 |

(continued)

| EXAMPLE # | $R^2$ | COMPOUND # | MH+ |
|---|---|---|---|
| 356 | 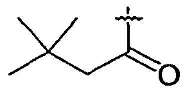 2 TFA | 583 | 531 |
| 357 | 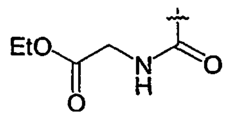 | 584 | 562 |
| 358 | 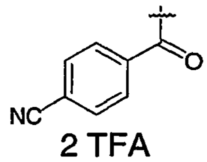 2 TFA | 585 | 562 |
| 359 | 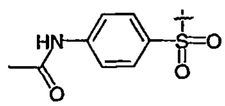 2 TFA | 586 | 630 |
| 360 | 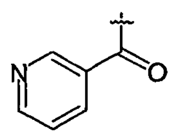 3 TFA | 587 | 538 |
| 361 | 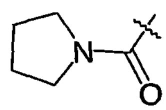 2 TFA | 588 | 530 |
| 362 | 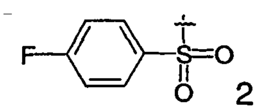 2 TFA | 589 | 591 |
| 363 | 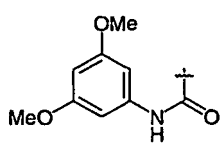 2 TFA | 590 | 612 |
| 364 | 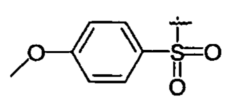 2 TFA | 591 | 603 |
| 365 | 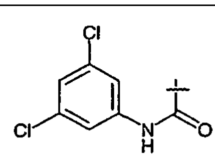 2 TFA | 592 | 620 |

(continued)

| EXAMPLE # | $R^2$ | COMPOUND # | $MH^+$ |
|---|---|---|---|
| 366 | 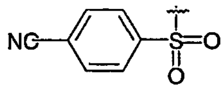 2 TFA | 593 | 598 |
| 367 | 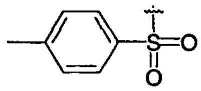 2 TFA | 594 | 587 |
| 368 | 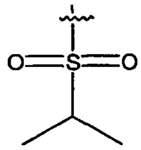 2 TFA | 595 | 539 |
| 369 | 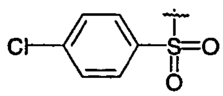 2 TFA | 596 | 607 |
| 370 | 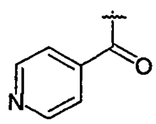 3 TFA | 597 | 538 |
| 371 | 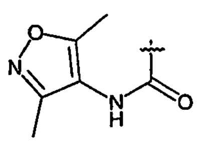 2 TFA | 598 | 571 |
| 372 | 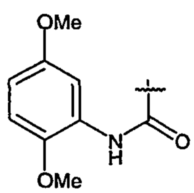 2 TFA | 599 | 612 |
| 373 | 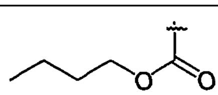 2 TFA | 600 | 533 |
| 374 | 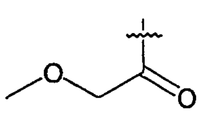 2 TFA | 601 | 505 |
| 375 | 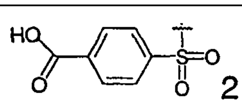 2 TFA | 602 | 617 |

(continued)

| EXAMPLE # | $R^2$ | COMPOUND # | $MH^+$ |
|---|---|---|---|
| 376 | 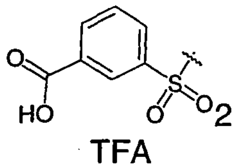 | 603 | 617 |
| 377 | 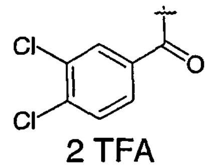 | 604 | 605 |

PREPARATIVE EXAMPLE 50

A. Compound (605), (606) AND (607)/(608).

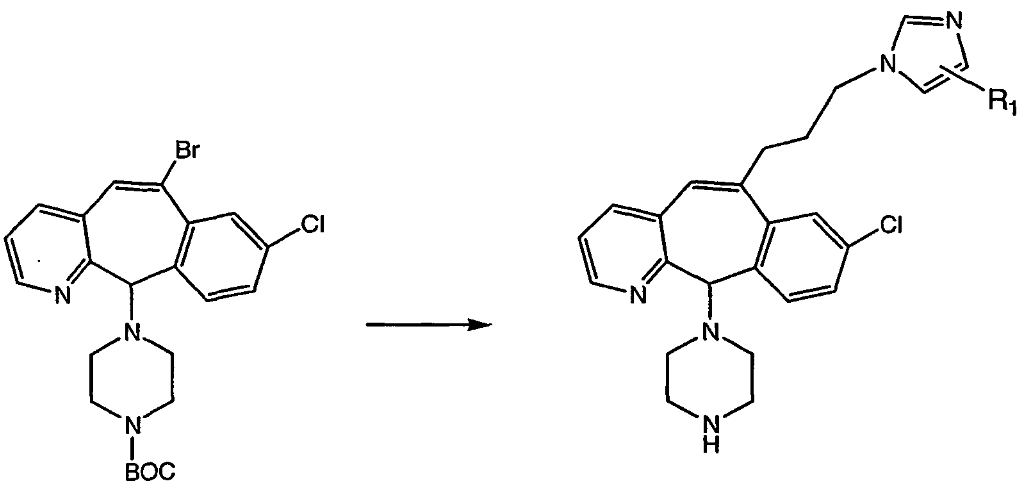

365

605 ($R^1$=H); (606) or
607/608 ($R^1$=(2 or 4 /5)-$CH_3$)

Compound (365) from Preparative Example 41 was reacted in essentially the same manner as in Preparative Example 4, substituting the appropriate imidazole to obtain Compound (605) wherein $R^1$=H or Compounds (606) and (607)/(608) wherein $R^1$=(2 or 4/5)$CH_3$.

B. Preparation of Compounds (607a)/(607b) and (608a)/(608b).

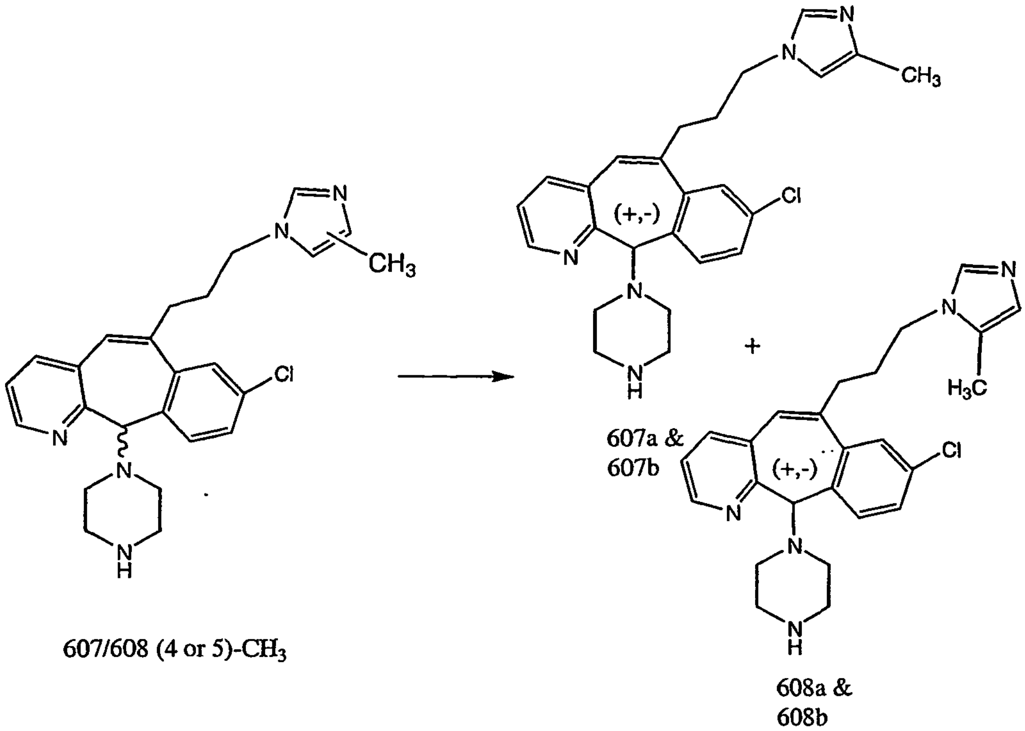

607/608 (4 or 5)-$CH_3$

607a & 607b

608a & 608b

Compounds (607) and (608) from Step A above were treated in the same manner as described in Example 11 to afford pure (+,-) 4-methyl imidazole, and pure (+,-) 5-methyl imidazole enantiomers; Compound (607a),(607b) and Compound (608a), (608b) respectively.

A library of compounds was prepared by the method described above starting with Compound (605), Compound (606), Compounds (607)/(608), (607a), (607b) or Compounds (608a) or (608b) used as the templates in Scheme 2. A generic structure of these compounds is shown in Figure 2 above. The $R^1$ group on the imidazole ring can be H or $CH_3$, the $R^2$ on N-1 of the piperazine is varied in the library. Library compounds prepared in this fashion are shown in Table 3, Table 4 and Table 5.

<u>EXAMPLES (378) - (396)</u>

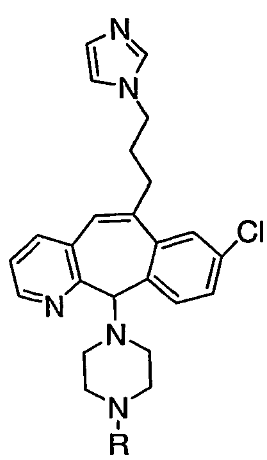

**Table 3**

| EXAMPLE # | $R^2$ | COMPOUND # | PHYSICAL DATA |
|---|---|---|---|
| 378 | 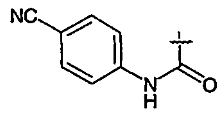 | 607 | 564 |
| 379 | 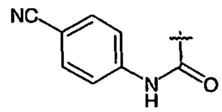 | 608 1st Enantiomer | 564 |
| 380 | 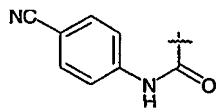 | 609 2nd Enantiomer | 564 |
| 381 | 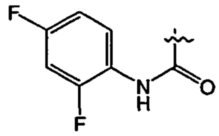 | 610 | 575 |
| 382 | 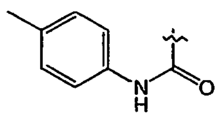 | 611 | 553 |
| 383 | 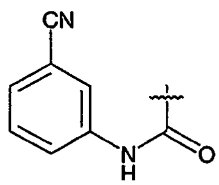 | 612 | 564 |
| 384 | 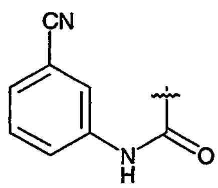 | 613 | 564 |
| 385 | 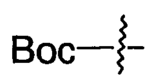 | 614 | 520 |
| 386 | 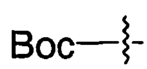 | 615 1st Isomer | 520 |
| 387 | 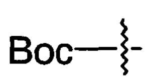 | 616 2nd Isomer | 520 |
| 388 | 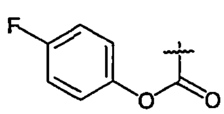 3 TFA | 617 | 558 |

(continued)

| EXAMPLE # | $R^2$ | COMPOUND # | PHYSICAL DATA |
|---|---|---|---|
| 389 | 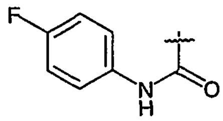 3 TFA | 618 | 557 |
| 390 | 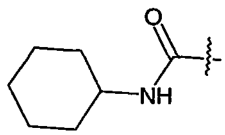 | 619 | 545 |
| 391 | 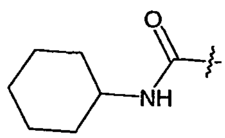 | 620 1st Isomer | 545 |
| 392 | 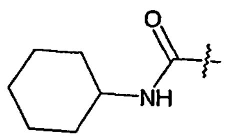 | 621 2nd Isomer | 545 |
| 393 | 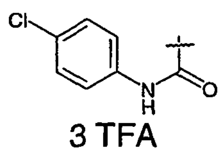 3 TFA | 622 | 573 |
| 394 | 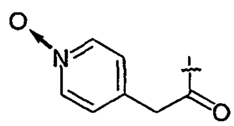 | 623 | 555 |
| 395 | 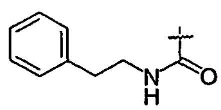 3 TFA | 624 | 567 |
| 396 | H 4 TFA | 625 | 420 |

EXAMPLES 397-401

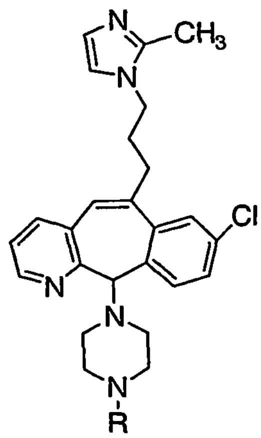

**Table 4**

| EXAMPLE # | $R^2$ | COMPOUND # | PHYSICAL DATA |
|---|---|---|---|
| 397 | 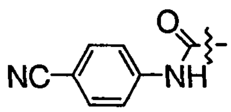 | 626<br>2 Isomers | Mass spec. MH+=578 |
| 398 | 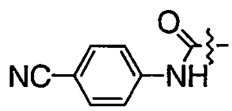<br>3 TFA | 627<br>2nd Enantiomer | Mass spec. MH+=578 |
| 399 | 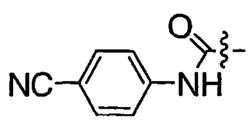 | 628<br>2nd Enantiomer | Mass spec. MH+=578 |
| 400 | 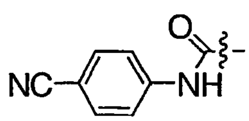 | 629<br>1st Enantiomer | Mass spec. MH+=578 |
| 401 | 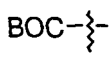 | 630<br>2 Isomers | Mass spec. MH+=534 |

EXAMPLES 402-406

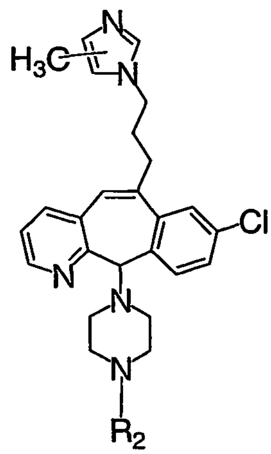

**Table 5**

| EXAMPLE # | $R^2$ | COMPOUND # | PHYSICAL DATA |
|---|---|---|---|
| 402 | 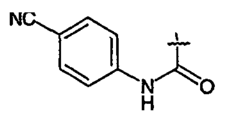 | 631<br>Mixture of 4-Me and 5-Me | Mass spec. MH+=578 |
| 403 | 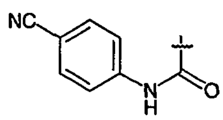 | 632<br>2nd enantiomer of 4-Me | Mass spec. MH+=578 |
| 404 | 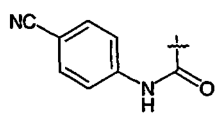 | 633<br>2nd enantiomer of 5-Me<br>1st enantiomer of 4-Me | Mass spec. MH+=578 |
| 405 | 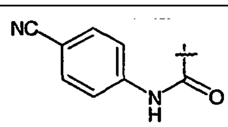 | 634<br>1st enantiomer of 5-Me | Mass spec. MH+=578 |
| 406 | 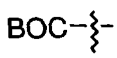 | 635<br>Mixture of 4-Me and 5-Me | Mass spec. MH+=534 |

PREPARATIVE EXAMPLE 51

Preparation of Compound (636)

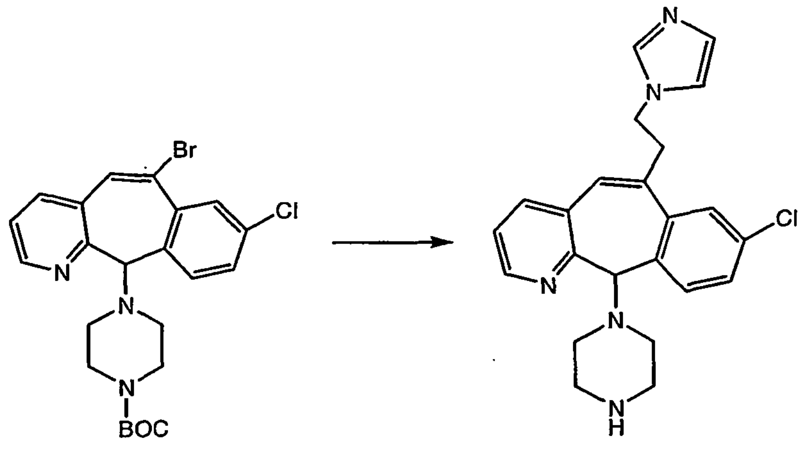

365 636

Compound (365) from Preparative Example 41, was reacted in essentially the same manner as Preparative Example 35 substituting Imidazole for 1-Methyl Imidazole in Step B to afford Compound (636) (MH+=406). Compound (636) was then reacted in the library fashion as described above following the procedure of Scheme 2 to afford the compounds in Table 6 below:

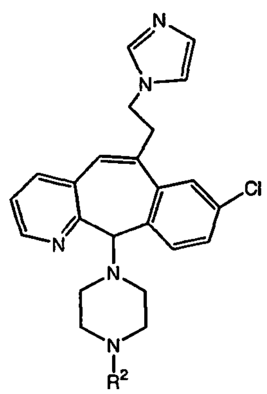

**Table 6**

| EXAMPLE # | $R^2$ | COMPOUND # | PHYSICAL DATA |
|---|---|---|---|
| 407 | 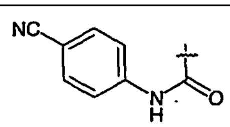 | 637 | Mass spec. MH+=550 |
| 408 | 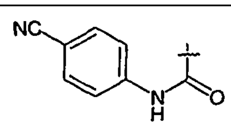 | 638<br>2nd Enantiomer | Mass spec. MH+=550 |
| 409 | 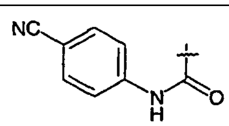 | 639<br>1ST Enantiomer | Mass spec. MH+=550 |
| 410 | 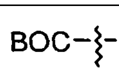 | 640 | Mass spec. MH+=506 |

PREPARATIVE EXAMPLE 52

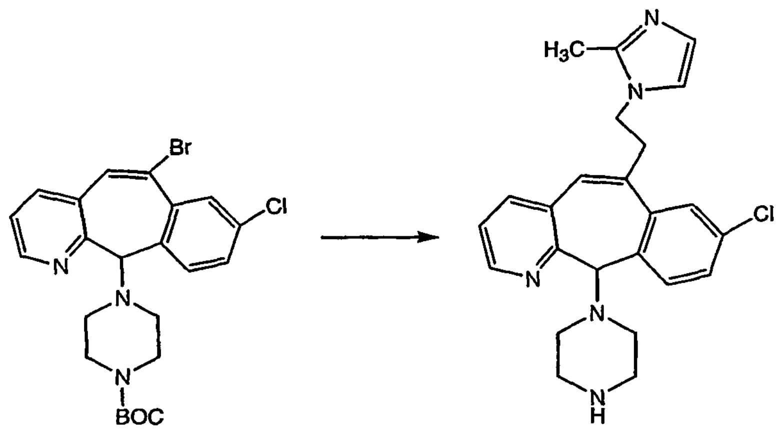

365 641

Compound (365) was reacted as above in Preparative Example 51, substituting 1-Methyl Imidazole for Imidazole to afford Compound (641) (MH+=420). Compound (641) was then further reacted in the Library fashion described above following the procedure in Scheme 2 to afford the compounds in Table 7 below:

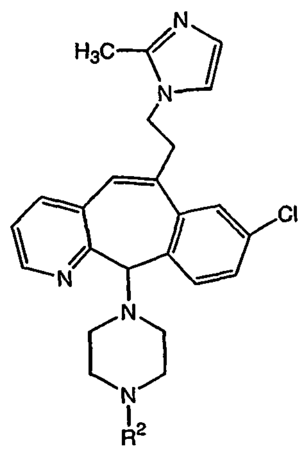

**Table 7**

| EXAMPLE # | $R^2$ | COMPOUND # | PHYSICAL DATA |
|---|---|---|---|
| 411 | 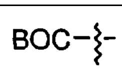 | 642 | Mass spec. $MH^+$=520 |
| 412 | 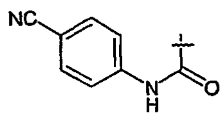 3 TFA | 643 | Mass spec. $MH^+$=564 |

(continued)

| EXAMPLE # | $R^2$ | COMPOUND # | PHYSICAL DATA |
|---|---|---|---|
| 413 | 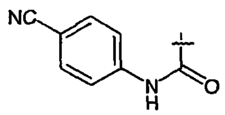 | 644<br>1st Enantiomer | Mass spec. $MH^+$=564 |
| 414 | 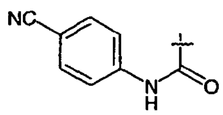 | 645<br>2nd Enantiomer | Mass spec. $MH^+$=564 |

EXAMPLE 415

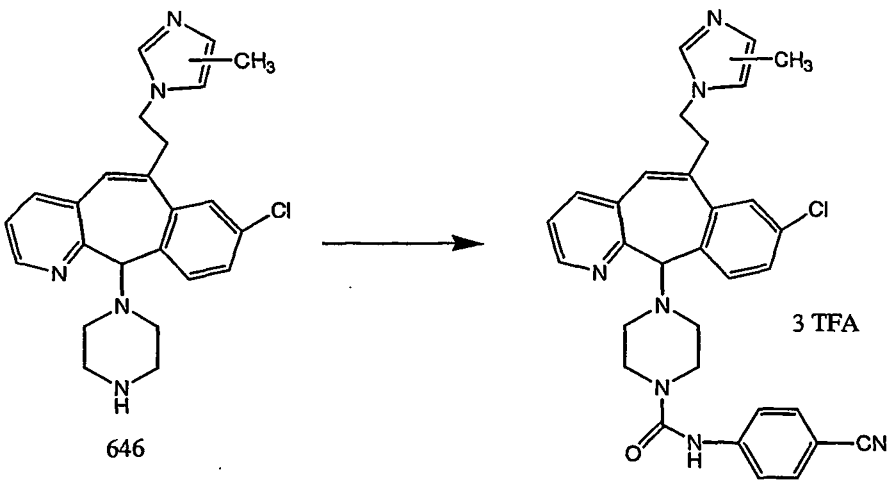


646

647

In the essentially the same manner as in Preparative Example 52 above, substituting 4-methylimidazole, the intermediate amine template was prepared Compound (646). This was then reacted in essentially the same manner as in Examples 411-414 above to afford the product Compound (647) as a mixture of 4 and 5-Methylimidazole isomers (Mass spec. $MH^+$=564).

PREPARATIVE EXAMPLE 53

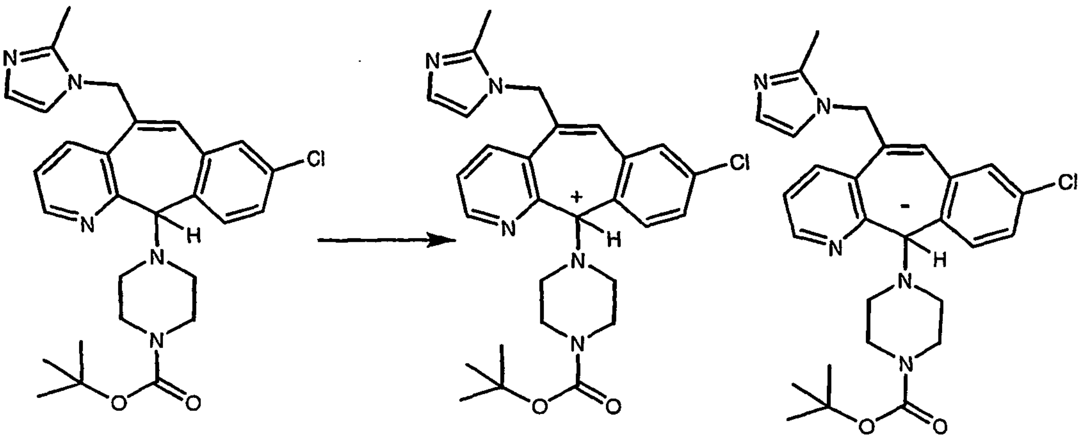

242 242a 242b

The racemic Compound (242) from Example 91 was separated by preparative chiral chromatography (Chiralpack AD, 5 cm X 50 cm column, flow rate 100 mL/min., 20% 2-propanol/hexane + 0.2% diethylamine) to afford the two enantiomers (242a) and (242b).

Compound (242a), $[\alpha]_D^{25}$ = +144.8° (3.16 mg/ 2 mL MeOH)

Compound (242b), $[\alpha]_D^{25}$= -144.8° (2.93 mg/ 2 mL MeOH)

<u>PREPARATIVE EXAMPLE 54</u>

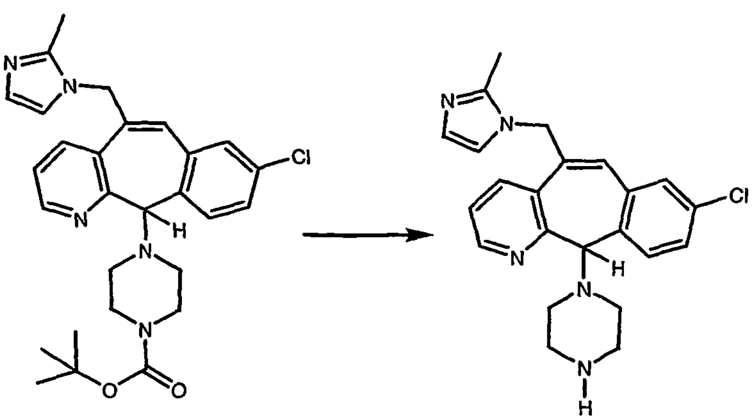

242a · 648 (+ enantiomer, A)

242b 649 (- enantiomer, B)

Compounds (242a) and (242b) from Preparative Example 53 above were reacted separately in essentially the same manner as Preparative Example 19, Step D to obtain the hydrochloride salt of compounds Compound (648) and Compound (649).

(648) (+ enantiomer, isomer A), MH+ = 406.1793

(649) (- enantiomer, isomer B), MH+ = 406.1789

PREPARATIVE EXAMPLE 55

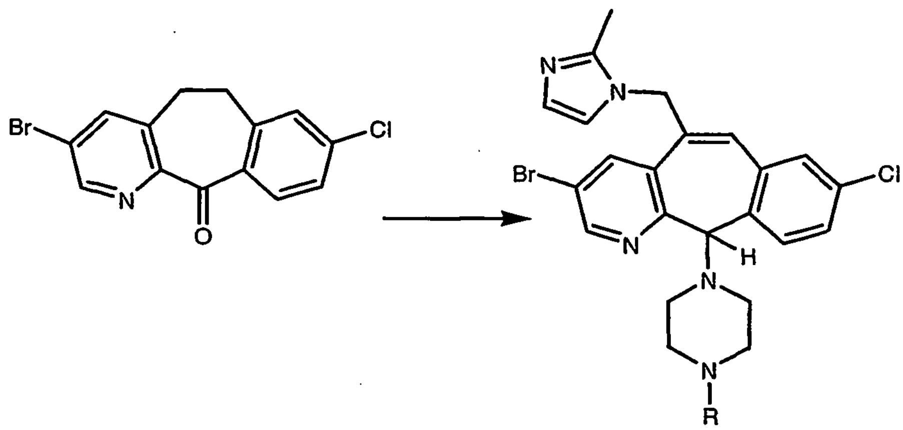

R = BOC
(650) (+ enantiomer, A)
(651) (- enantiomer, B)

R = H
(652) (+ enantiomer, A)
(653) (- enantiomer, B)

3-bromo-8-chloroazaketone (U.S. patent 5,977,128, Preparative Example 11, step A, (1999)) was reacted in essentially the same manner as in Preparative Example 23, and Example 91 to obtain the N-BOC derivatives (650) and (651). Compounds (650) and (651) were then reacted separately in essentially the same manner as in Preparative Example 19, Step D to obtain the enantiomers (652) (+ enantiomer, isomer A) and (653) (- enantiomer, isomer B).

Compound (650), BOC derivative, $[\alpha]_D^{25}$ = +69.6° (2.5 mg/ 2 mL MeOH)
Compound (651), BOC derivative, $[\alpha]_D^{25}$ = -90.0° (3.3 mg/ 2 mL MeOH)

Compound (652) (+ enantiomer, isomer A), MH+ = 485
Compound (653) (- enantiomer, isomer B), MH+ = 485

PREPARATIVE EXAMPLE 56

Step A

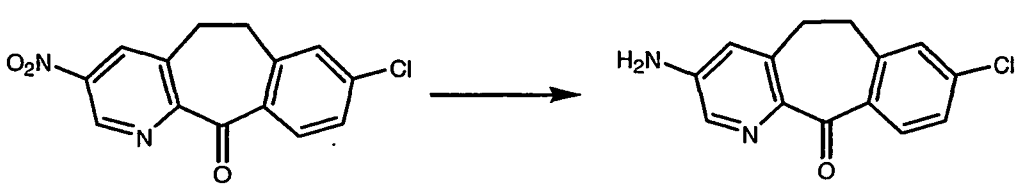

654a 654

Compound (654a) (202 g; 0.7 mole) (J. Org. Chem. 1998, 63, 445) was dissolved in ethanol (5 L). To this mixture was added 12 N HCl (80 ml) and iron powder (180 g) and the reaction was refluxed over night. Additional HCl

and iron was added to complete the reaction. The reaction mixture was filtered and the precipitate washed with hot methanol (1L). The filtrate was concentrated under vacuum to approximately 600 ml then partitioned between 4 L $CH_2CL_2$ and 1.3 L of 1.3 N NaOH. The organic layer was dried over $MgSO_4$ and filtered hot. The filtrate was concentrated under vacuum to give the aminoketone Compound (654) (184 g).

Step B

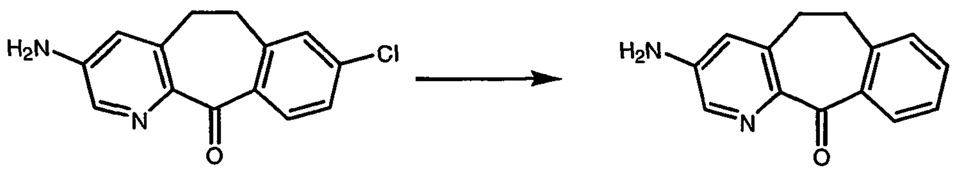

654 655

Compound (654) from Step A above (15 g; 57.98 mmol), was dissolved in 750 mL of ethanol containing 3.75 g of 5% Pd/C (50% in water) and 37.69 g (579.82 m mol) of ammonium formate. The mixture was brought to reflux for 2.5 hr then stirred at room temperature overnight. The reaction was filtered concentrated under vacuum and chromatographed on silica gel using 95:5 methylene chloride (saturated with ammonia) and methanol to give 6.15 g of the pure product Compound (655) as a yellow solid.

Step C

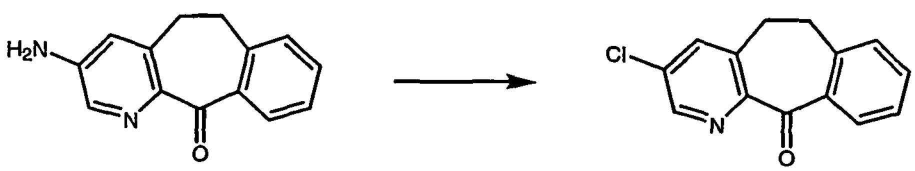

655 656

To a slurry of Compound (655) (4.79 g; 21.37 mmol) from Step A above, in 75 mL of acetonitrile cooled to 0˚C and under nitrogen, was added t-butylnitrite (10.31 g; 32.05 mmol) and $CuCl_2$ (3.45 g; 24.64 mmol). The mixture was warmed to room temp stirrd over night and then concentrated under vacuum. The residue was slurried in 30 mL of 1N HCl, then neutralized with aqueous $NH_4OH$ and extracted with 3 X 100 mL of ethyl acetate. The organic layer was dried over $Na_2SO_4$. concentrated under vacuum, and chromatographed on silica gel using hexane:ethyl acetate (70:30) to obtain the pure product Compound (656).

Step D

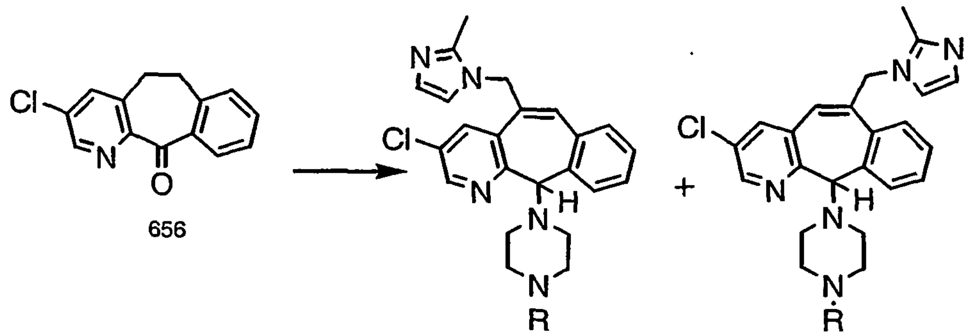

656

R = BOC
(657) (+ enantiomer, A)
(658) (- enantiomer, B)

R = BOC
(657.1) (+ enantiomer, A)
(658.1) (- enantiomer, B)

R = H
(659) (+ enantiomer, A)
(660) (- enantiomer, B)

R = H
(659.1) (+ enantiomer, A)
(660.1) (- enantiomer, B)

Compound (656) from Step B above was reacted in essentially the same manner as in Preparative Example 23, and then Example 91 to obtain the N-BOC derivatives (657), (658), (657.1) and (658.1). Compounds (657), (658), (657.1) and (658.1) were then reacted separately in essentially the same manner as in Preparative Example 19, Step D to obtain the enantiomers (659) (+ enantiomer, isomer A), (659.1) (+ enantiomer, isomer A), (660) (- enantiomer, isomer B) and (660.1) (- enantiomer, isomer B).

Compound (657), BOC derivative, $[\alpha]_D^{25}$ = +59,9° (3.3 mg/ 2 mL MeOH)
Compound (658), BOC derivative, $[\alpha]_D^{25}$ = -57.1° (3.3 mg/ 2 mL MeOH)

Compound (659), (+ enantiomer, isomer A), MH+ = 406
Compound (660), (- enantiomer, isomer B), MH+ = 406

Compound (659.1), (+ enantiomer, isomer A), MH+ = 406
Compound (660.1), (- enantiomer, isomer B), MH+ = 406

PREPARATIVE EXAMPLE 57

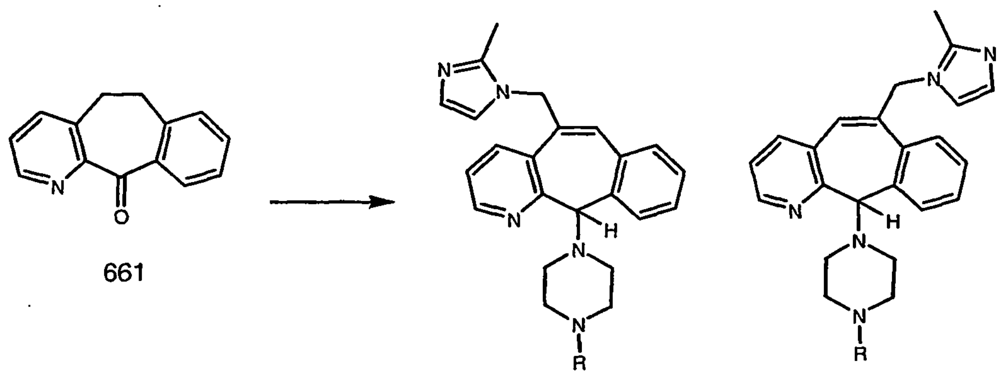

661

R = BOC
(662) (+ enantiomer, A) (663) (+ enantiomer, A)
(664) (- enantiomer, B) (665) (- enantiomer, B)

R = H
(666) (+ enantiomer, A) (667) (+ enantiomer, A)
(668) (- enantiomer, B) (669) (- enantiomer, B)

Compound (661) was reacted in essentially the same manner as in Preparative Example 23, and then Example 91 to obtain the N-BOC derivatives (662), (663), (664) and (665). Compounds (662), (663), (664) and (665) were then reacted separately in essentially the same manner as in Preparative Example 19, Step D to obtain the enantiomers (666) and (667) (+ enantiomer, isomer A) and (668) and (669)(-enantiomer, isomer B). The C5 and C-6 vinyl bromide intermediates were separated by silica gel chromatography using hexane:ethyl acetate (80:20) in essentially the same manner as was described in Preparative Example 23, Step B.

Compound (662), BOC derivative
Compound (663), BOC derivative

Compound (664), BOC derivative
Compound (665), BOC derivative

Compound (666) (+ enantiomer, isomer A), MH+ = 372
Compound (667) (+ enantiomer, isomer A), MH+ = 372

Compound (668) (- enantiomer, isomer B), MH+ = 372
Compound (669) (- enantiomer, isomer B), MH+ = 372

PREPARATIVE EXAMPLE 58

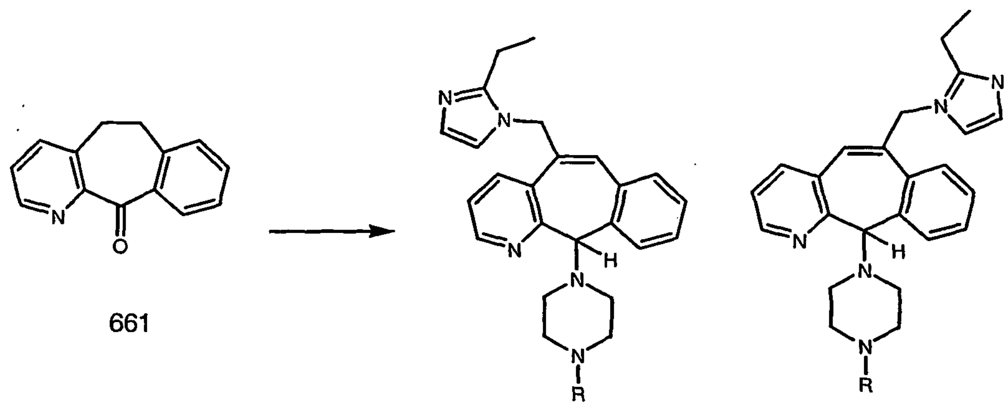

661

R = BOC
(670) (+ enantiomer, A) (671) (+ enantiomer, A)
(672) (- enantiomer, B) (673) (- enantiomer, B)

R = H
(674) (+ enantiomer, A) (675) (+ enantiomer, A)
(676) (- enantiomer, B) (677) (- enantiomer, B)

Compound (661) was reacted in essentially the same manner as in Preparative Example 23, and Example 91 substituting 2-ethylimidazole for 2-methylimidazole, to obtain the N-BOC derivatives (670), (671), (672) and (673). Compounds (670), (671), (672) and (673) were then reacted separately in essentially the same manner as in Preparative Example 19, Step D, to obtain the enantiomers (674) and (675) (+enantiomer, isomer A) and (676) and (677) (- enantiomer, isomer B). The C5 and C-6 vinyl bromide intermediates were separated by silica gel chromatography using hexane: ethyl acetate (80:20) as described in Preparative Example 23, Step B.

Compound (670), BOC derivative, (+ enantiomer, A)
Compound (671), BOC derivative, (+ enantiomer, A)

Compound (672), BOC derivative, (- enantiomer, B)
Compound (673), BOC derivative, (- enantiomer, B)

Compound (674), (+ enantiomer, isomer A), MH+ = 386
Compound (675), (+ enantiomer, isomer A), MH+ = 386

Compound (676), (- enantiomer, isomer B), MH+ = 386
Compound (677), (-enantiomer, isomer B), MH+ = 386

EXAMPLES 416-419

Compound (648)

Compound (649)

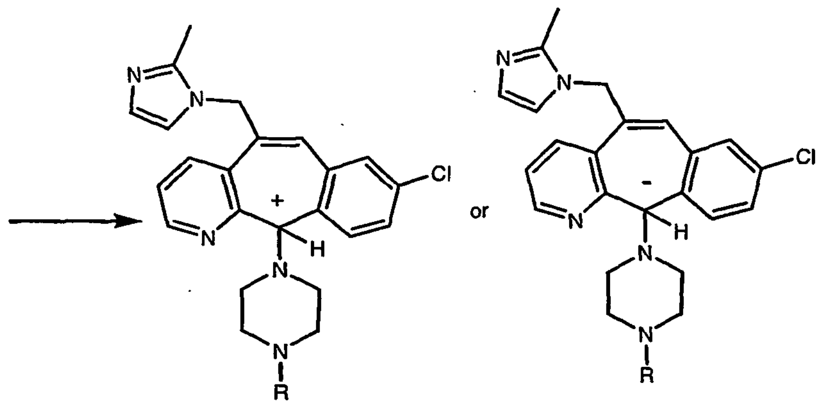

or

The appropriate (+) enantiomer (648) or (-) enantiomer (649) from Preparative Example 54 above, was taken up in $CH_2Cl_2$ treated with the corresponding isocyanate and stirred at room temperature over night. The crude product was purified directly by silica gel preparative thin layer chromatography or silica gel column chromatography to afford the following compounds in Table 8 below:

**TABLE 8**

| Example # | R | Enantiomer | Comp # | Phys. Data. |
|---|---|---|---|---|
| 416 | 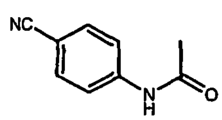 | + | 678 | Mp = 162.2-165.6°C<br>$[\alpha]_D^{25}$ = +98.2° (3 mg/ 2 mL MeOH) |
| 417 | 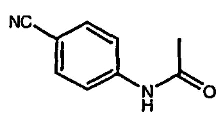 | - | 679 | Mp = 158.1-164.5°C<br>$[\alpha]_D^{25}$ = -81.2° (2.6 mg/ 2 mL MeOH) |
| 418 | 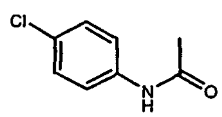 | + | 680 | Mp = 161.5-164.8°C<br>MH+ = 559.1787 |
| 419 | 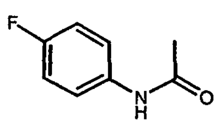 | + | 681 | Mp = 157.7-161.7°C<br>MH+ = 543.2069 |

EXAMPLES 420 AND 421

Compound (652)

Compound (653)

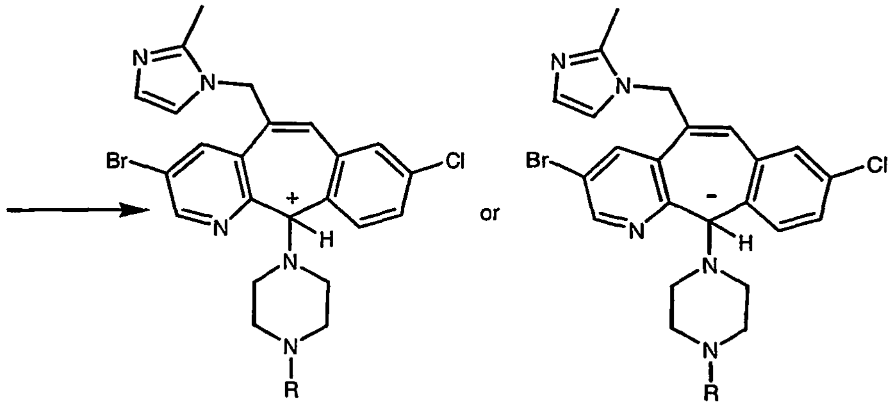

or

The appropriate (+) enantiomer (652) or (-) enantiomer (653) from Preparative Example 55 above, was taken up in $CH_2Cl_2$ treated with the corresponding isocyanate and stirred at room temperature over night. The crude product was purified directly by silica gel preparative thin layer chromatography or silica gel column chromatography to afford the following compounds in Table 9 below:

**Table 9**

| Example # | R | Enantiomer | Comp # | Phys. Data. |
|---|---|---|---|---|
| 420 | 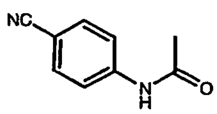 | + | 682 | Mp = 168.8-172.3°C |
| 421 | 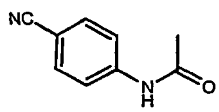 | - | 683 | Mp = 172.5-177.7°C |
| 421.1 | 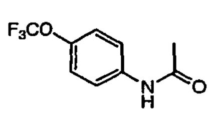 | + | 683.1 | Mp = 157.1-160.5°C (dec) |
| 421.2 | 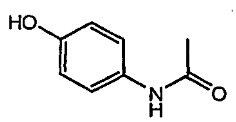 | + | 683.2 | Mp = 223.6-229.1°C (dec) |

EXAMPLES 422 AND 423

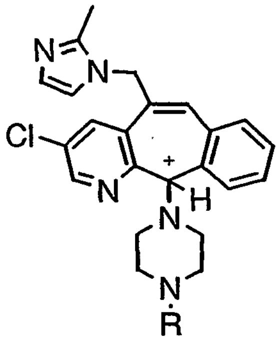,
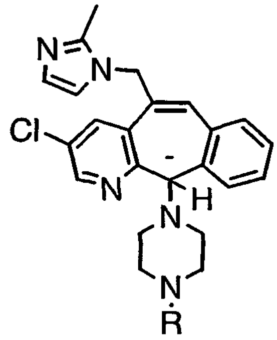
or
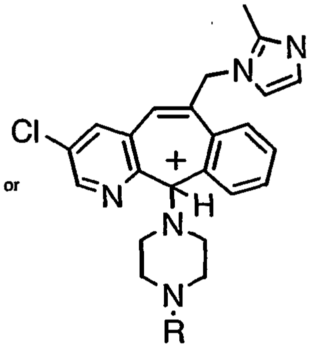

The appropriate compound (659) (+) enantiomer, (660) (-) enantiomer or (659A) (+) enantiomer from Preparative Example 56 above, was taken up in $CH_2Cl_2$ treated with the corresponding isocyanate and stirred at room temperature over night. The Crude product was purified directly by silica gel preparative thin layer chromatography or silica gel column chromatography to afford the following compounds in Table 10 below:

**Table 10**

| Example # | R | Enantiomer | Comp # | Phys. Data. |
|---|---|---|---|---|
| 422 | 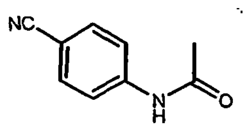 | + | 684 | Mp = 155.9-165.1 ˚C |
| 423 | 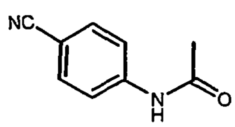 | - | 685 | Mp = 154.2-164.8˚C |
| 492 | 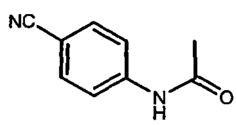 | + | 806 | Mp=157.1-160.5˚C<br>$MH^+$ = 689 |

EXAMPLES 424 AND 425

Compound (666)
Compound (668)
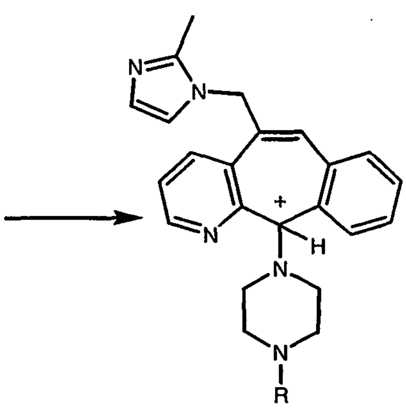
or
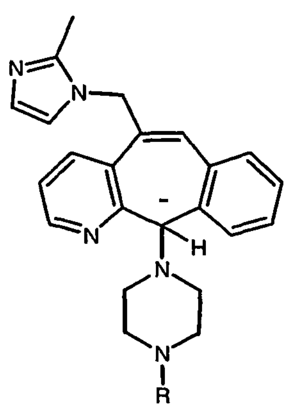

The appropriate (+) enantiomer (666) or (-) enantiomer (668) from Preparative Example 57 above, was taken up in $CH_2Cl_2$, treated with the corresponding isocyanate and stirred at room temperature over night. The crude product was purified directly by silica gel preparative thin layer chromatography or silica gel column chromatography to afford the following compounds in Table 11 below:

**Table 11**

| Example # | R | Enantiomer | Comp # | Phys. Data. |
|---|---|---|---|---|
| 424 | 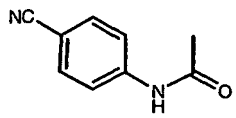 | + | 686 | Mp = 166-170°C<br>$[\alpha]_D^{25}$ = +106.8° (1.45 mg/ 2 mL MeOH) |
| 425 | 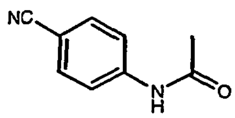 | - | 687 | Mp = 170-176°C<br>$[\alpha]_D^{25}$ = -91° (2.78 mg/ 2 mL MeOH) |

EXAMPLES 426 AND 427

Compound (674)

Compound (676)

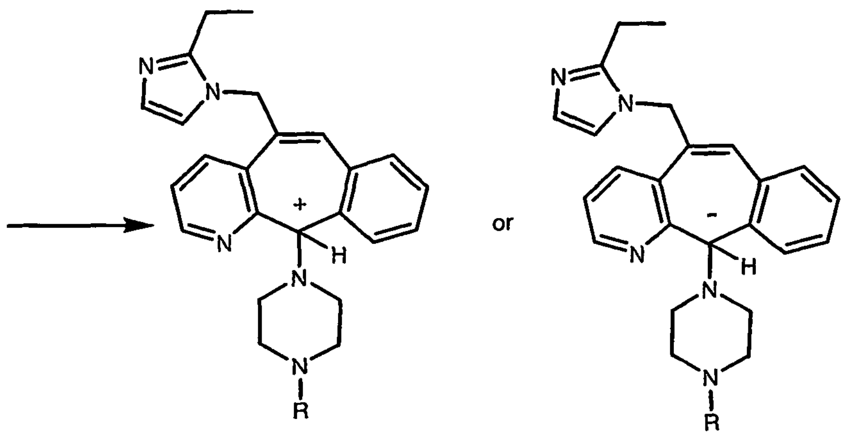

or

The appropriate (+) enantiomer (674) or (-) enantiomer (676) from Preparative Example 58 above, was taken up in $CH_2Cl_2$, treated with the corresponding isocyanate and stirred at room temperature over night. The crude product was purified directly by silica gel preparative thin layer chromatography or silica gel column chromatography to afford the following compounds in Table 12 below:

**Table 12**

| Example # | R | Enantiomer | Comp # | Phys. Data. |
|---|---|---|---|---|
| 426 | 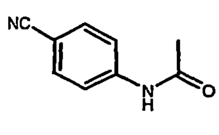 | + | 688 | Mp = 150-153°C |
| 427 | 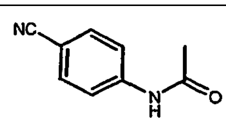 | - | 689 | Mp =154-158°C |

EXAMPLES 428 AND 429

Compound (667)

Compound (669)

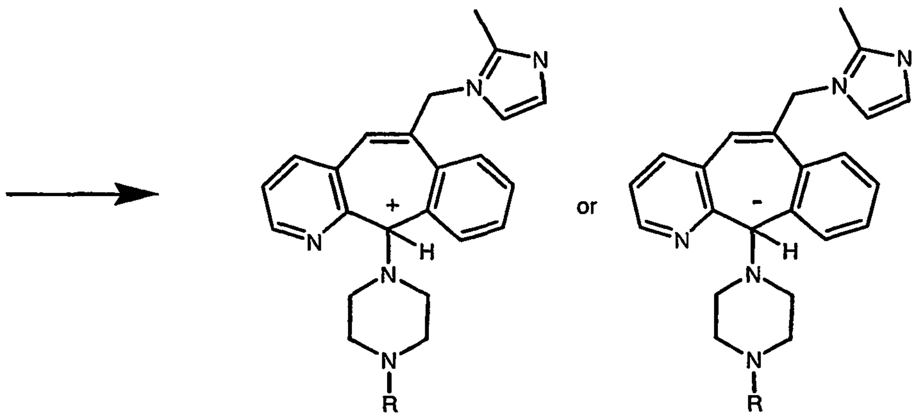

or

The appropriate (+) enantiomer (667) or (-) enantiomer (669) from Preparative Example 57 above, was taken up in $CH_2Cl_2$, treated with the corresponding isocyanate and stirred at room temperature over night. The crude product was purified directly by silica gel preparative thin layer chromatography or silica gel column chromatography to afford the following compounds in the table below:

| Example # | R | Enantiomer | Comp # | Phys. Data. |
|---|---|---|---|---|
| 428 | 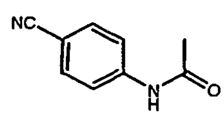 | Isomer 1 | 690 | $MH^+$= 516 |
| 429 | 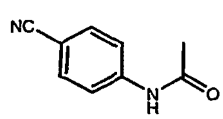 | Isomer 2 | 691 | $MH^+$= 516 |

EXAMPLES 430 AND 431

Compound (675)

Compound (677)

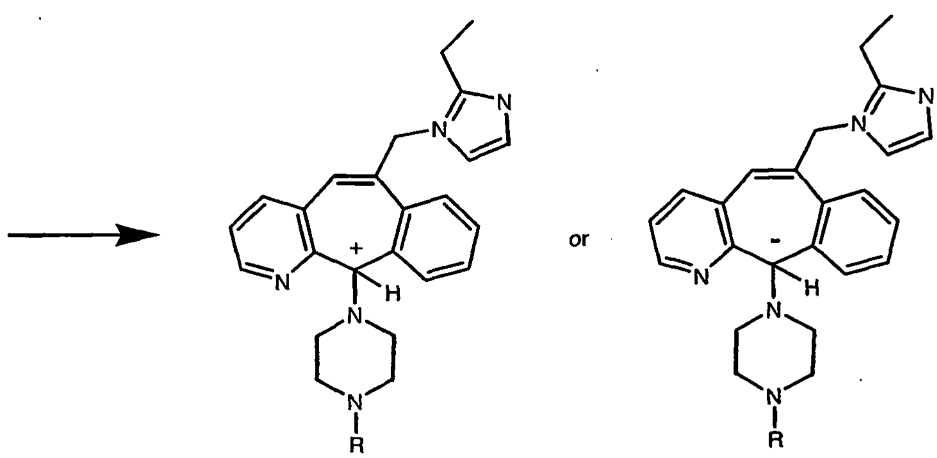

or

The appropriate (+) enantiomer (675) or (-) enantiomer (677) from Preparative Example 58 above, was taken

up in $CH_2Cl_2$, treated with the corresponding isocyanate and stirred at room temperature over night. The crude product was purified directly by silica gel preparative thin layer chromatography or silica gel column chromatography to afford the following compounds in the table below:

| Example # | R | Enantiomer | Comp # | Phys. Data. |
|---|---|---|---|---|
| 430 | 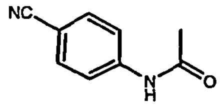 | Isomer 1 | 692 | $MH^{+}$= 530 |
| 431 | 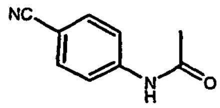 | Isomer 2 | 693 | $MH^{+}$= 530 |

PREPARATIVE EXAMPLE 59

Compounds Type A (696a), (696b), and Type B (697a), (697b)

Type A Type B

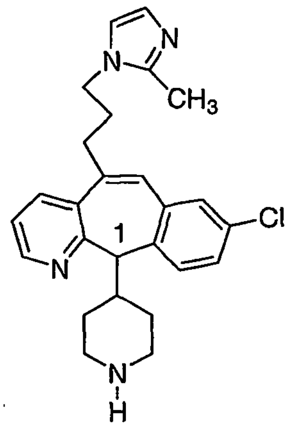

+

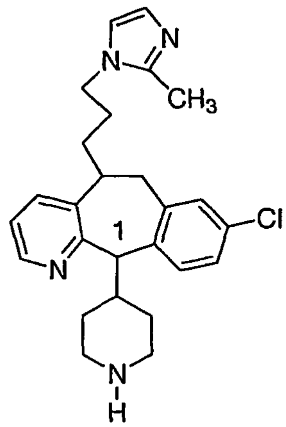

696a 697a

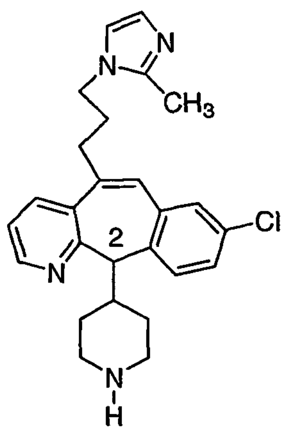

+

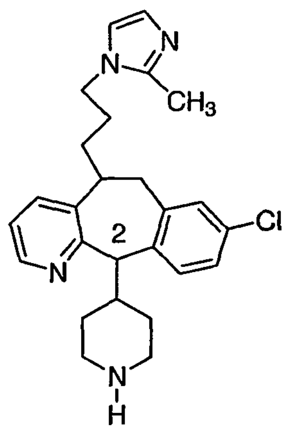

696b 697b

Step A Preparation of Compounds (694a) and (695a)

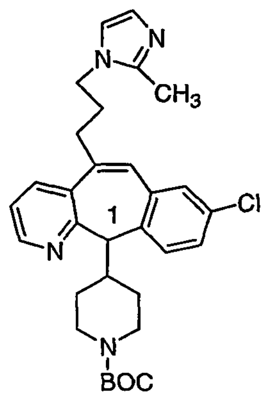

+

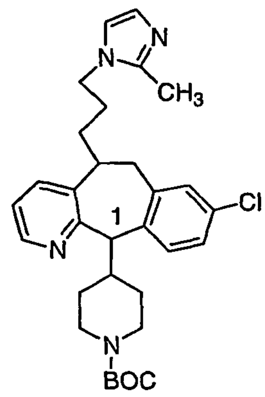

694a

695a

To a stirred solution of 2-methyl imidazole (1.80 g, 21.97 mmol) in anhydrous DMF (40 mL) at room temperature, was added NaH (5.3 g, 21.97 mmol) and Compound (27) from Preparative Example 4, Step E (4.0 g, 7.33 mmol). The resulting solution was stirred at room remperature for 1 hr and concentrated to dryness, followed by extraction with EtOAc-$NaHCO_3$. The combined organic layer was dried over $Na_2SO_4$, filtered and concentrated to dryness to give the mixture of single bond and double bond compounds. These compounds were further purified by column chromatography on silica gel, eluting with 2%MeOH/$NH_3$/98%$CH_2Cl_2$ to yield : Pure Type A Compound (694) (0.450 g) ($MH^+$=533) and a mixture of Type A (694) and Type B Compound (695) (2.55 g)($MH^+$=535).

Compounds (694) and (695) were further purified by prep HPLC, eluting with 15%IPA/85%Hexane/0.2%DEA to give : Type B Compound (695a) (isomer 1; 0.58 g, $MH^+$=535.4) and Type A Compound (694a) (isomer 1; 0.61 g, $MH^+$= 533) and a mixture of compounds (694b) and (695b) (isomer 2 products; 0.84g).

Step B Preparation of Compounds (696b) and (697b)

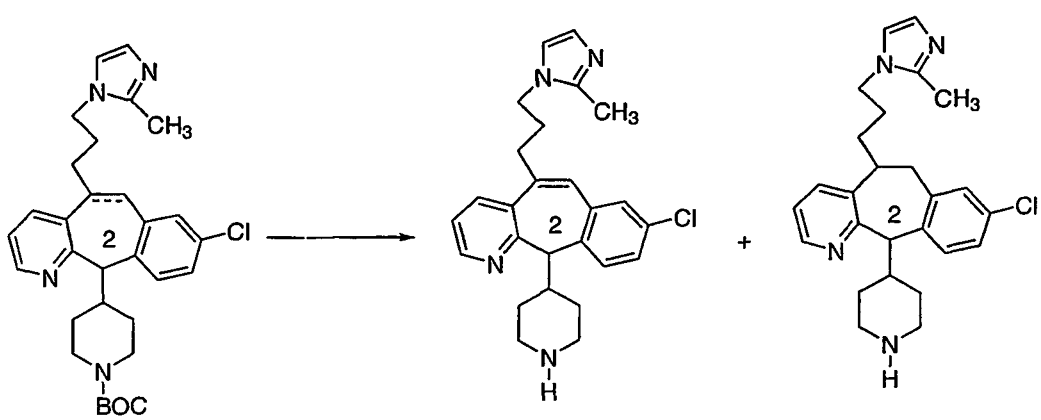

(694b)/(695b) mixture

696b

697b

The mixture of compounds (694b/695b) from Step A above (0.8 g, 1.5 mmol) in 4N HCl/Dioxane (40 mL) was stirred at room temperature for 3 hrs and concentrated to dryness to give a mixture of deprotected compounds as product. The product was further purified by HPLC, eluting with 15%IPA/85% hexane/0.2%DEA to give the pure compound (696b) Type A (isomer 2; 0.29 g) and pure Compound (697b) Type B (isomer 2, 0.19 g).

Step C Preparation of Compounds (696a) and (697a)

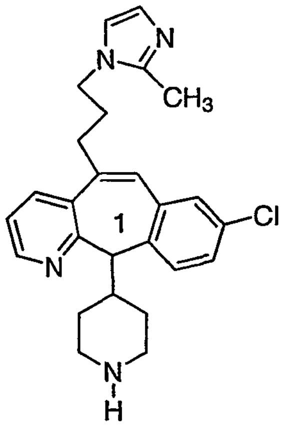

+

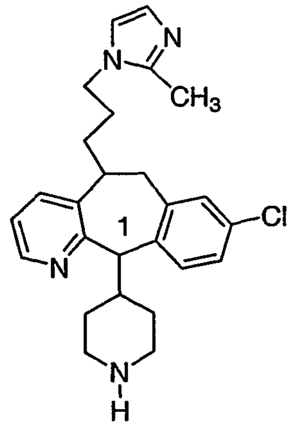

696a 697a

Compounds (694a) and (695a) (pure isomer 1) were individually deprotected using 4N HCl/Dioxane in essentially the same method as that of the isomer 2 products described above, to give the corresponding N-H products (696a) Type A (isomer 1) and (697a) Type B (isomer 1).

EXAMPLES 432-437

Reacting Compound (696a) (isomer 1) in essentially the same manner as in Example 13 with the appropriate chloroformate or isocyanate, the following compounds listed in Table 13 below, were prepared.

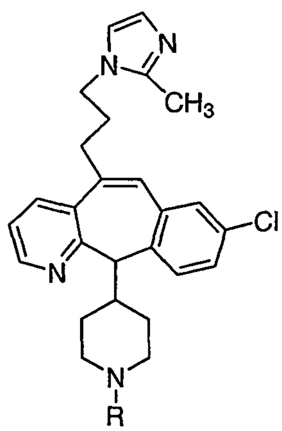

Table 13: 2-Methylpropylimidazole-5-Substituted Bridgehead Double bond Analogs

| EXAMPLE # | R | COMPOUND # | PHYSICAL DATA |
|---|---|---|---|
| 432 | 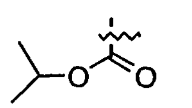 | 698 | MH+=519.1 |

(continued)

| EXAMPLE # | R | COMPOUND # | PHYSICAL DATA |
|---|---|---|---|
| 433 | 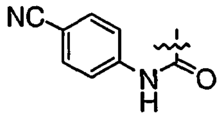 | 699 | MH+=577.1 |
| 434 | 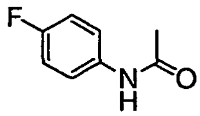 | 700 | MH+=570.1 |
| 435 | 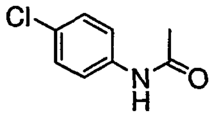 | 701 | MH+=585.1 |
| 436 | 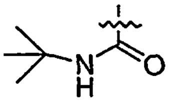 | 702 | |
| 437 | 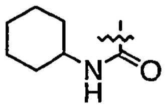 | 703 | MH+=558.1 |

EXAMPLES 438-442

Reacting Compound (697a) (isomer 1) in essentially the same manner as in Example 13 with the appropriate chloroformate or isocyanate, the following compounds listed in Table 14 below were prepared.

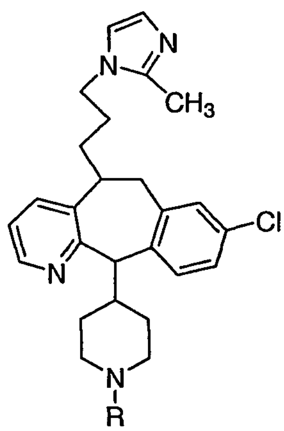

Table 14: 2-Methylpropylimidazole-5-Substituted Bridgehead Single bond Analogs

| EXAMPLE # | R | COMPOUND # | PHYSICAL DATA |
|---|---|---|---|
| 438 | 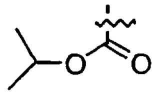 | 704 | MH+=521.1 |

(continued)

| EXAMPLE # | R | COMPOUND # | PHYSICAL DATA |
|---|---|---|---|
| 439 | 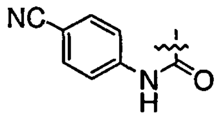 | 705 | MH+=579.1 |
| 440 | 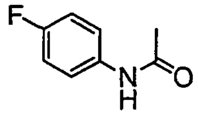 | 706 | MH+=572.1 |
| 441 | 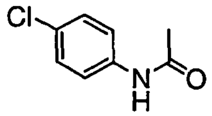 | 707 | MH+=587.1 |
| 442 | 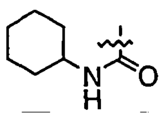 | 708 | MH+=560.1 |

PREPARATIVE EXAMPLE 60

COMPOUNDS (711a), (711b), (712a) AND (712b).

Step A Preparation of Compounds (709a), (709b), (710a) and (710b)

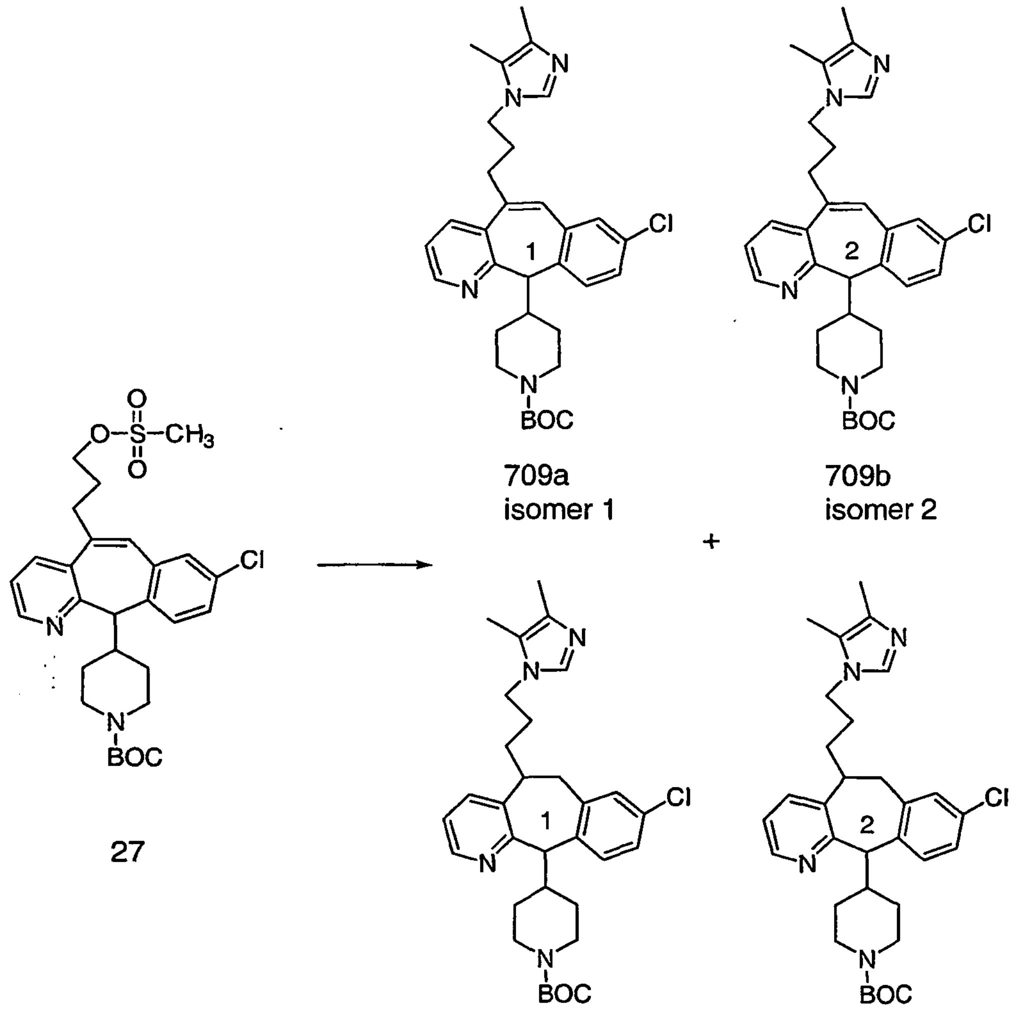

27

709a
isomer 1

709b
isomer 2

+

710a
isomer 1

710b
isomer 2

To a stirred solution of 4,5-Dimethylimidazole (1.08 g, 11.25 mmol) in anhydrous DMF (35 mL) at room temperature, was added NaH (0.27 g, 11.2 mmol) and stirred for 10 minutes, followed by the addition of Compound (27) from Preparative Example 4 Step E (4.0 g, 7.32 mmol). The resulting solution was srirred at room temperature overnight. To this solution was added the solution of 4,5-dimethylimidazole (0.35 g, 3.65 mmol) and NaH (0.088 g, 3.67 mmol) in DMF (5 mL). The resulting solution was heated at 80˚C-90˚C for 4 hrs, then cooled down to room temperature, followed by extraction with EtOAc-$H_2O$. The combined organic layer was washed with brine, dried over $Na_2SO_4$, filtered and concentrated to dryness and purified by column chromatography on silica gel, eluting with 50%EtOAc/50%hexane to 5%MeOH/$CH_2Cl_2$ to give the mixture of products Compound (709) Type A and Compound (710) Type B (1.2 g, MH+=547.3). The products were further purified by prep HPLC, using a chiral AD column, eluting with 15%IPA/85%hexane/0.2%DEA to give 4 seperate compounds:

Compound (709a) isomer 1, type A (0.291 g, MH+=547.3), Compound (710a) isomer 1, type B (0.305 g, MH+=549.3) and
Compound (709b) isomer 2, type A (0.280 g, MH+=547.3), Compound (710b) isomer 2, type B (0.2 g, MH+=549.3)

<u>Step B</u> <u>Preparation of Compounds (711a), (711b), (712a) and (712b)</u>

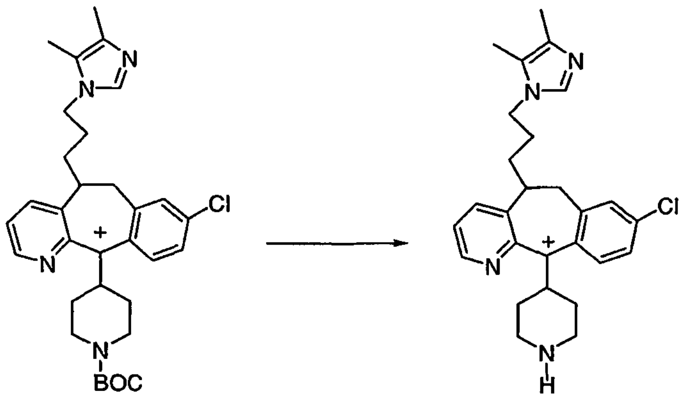

710a
isomer 1

711a
isomer 1

A solution of Compound (710a), isomer 1 type B (0.245 g, 0.45 mmol) in 4N HCl/Dioxane (2 mL) was stirred at room temperature for 3 hrs then concentrated to dryness to give Compound (711 a) isomer I, type B product (0.184 g, 98% yield) (MH+=455.1).

Compounds (711b), (isomer 2; type B); (712a) (isomer 1; type A) and (712b) (isomer 2; type A) were all prepared in a similar fashion to that of Compound (711a) isomer 1 type B in Step B above.

(711b) (0.085 g, 75% yield).
(712a) (0.141 g, 75% yield),
(712b) (0.106 g, 59% yield),

Examples 443-447

Reacting Compounds (711a) and (711b) seperately following the procedure described in Example 13 with the appropriate chloroformates or isocyanates, the following compounds listed in Table 15 below were prepared.

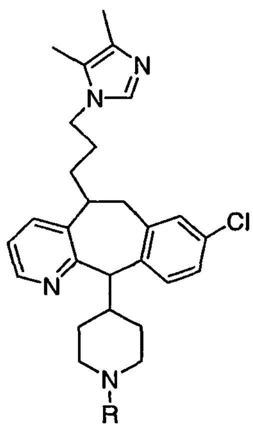

Table 15: 4,5-Dimethylpropylimidazole-5-Substituted Bridgehead Single bond Analogs

| EXAMPLE # | R | COMPOUND # | PHYSICAL DATA |
|---|---|---|---|
| 443 | 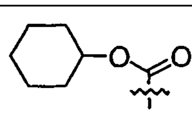 | 713 | MH+=575.1 |

(continued)

| EXAMPLE # | R | COMPOUND # | PHYSICAL DATA |
|---|---|---|---|
| 444 | 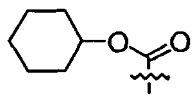 | 714 | MH+=575.1 |
| 445 | 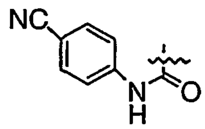 | 715 | MH+=593.2 |
| 446 | 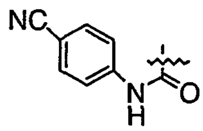 | 716 | MH+=593.2 |
| 447 | 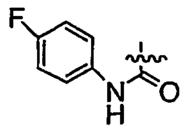 | 717 | MH+=586.1 |

Examples 448-454

Reacting Compounds (712a) and (712b) seperately following the procedure described in Example 13 with the appropriate chloroformates or isocyanates, the following compounds listed in Table 16 below were prepared.

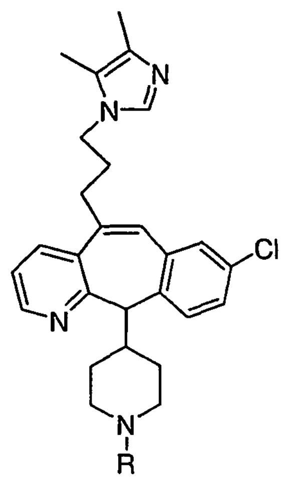

Table 16: 4,5-Dimethylpropylimidazole-5-Substituted Bridgehead Double bond Analogs

| EXAMPLE # | R | COMPOUND # | PHYSICAL DATA |
|---|---|---|---|
| 448 | 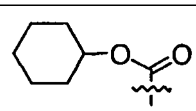 | 718 | MH+=573.1 |
| 449 | 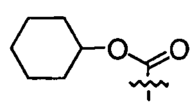 | 719 | MH+=573.1 |

(continued)

| EXAMPLE # | R | COMPOUND # | PHYSICAL DATA |
|---|---|---|---|
| 450 | 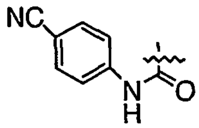 | 720 | MH+=591.1 |
| 451 | 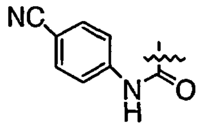 | 721 | MH+=591.1 |
| 452 | 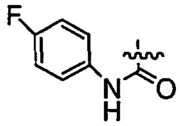 | 722 | MH+=584.1 |
| 453 | 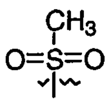 | 723 | MH+=525.1 |
| 454 | 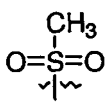 | 724 | MH+=525.1 |

PREPARATIVE EXAMPLE 61

PREPARATION OF COMPOUNDS (727a), (727b), (728a) AND (728b).

STEP A Preparation of Compounds (725a), (725b), (726a) and (726b).

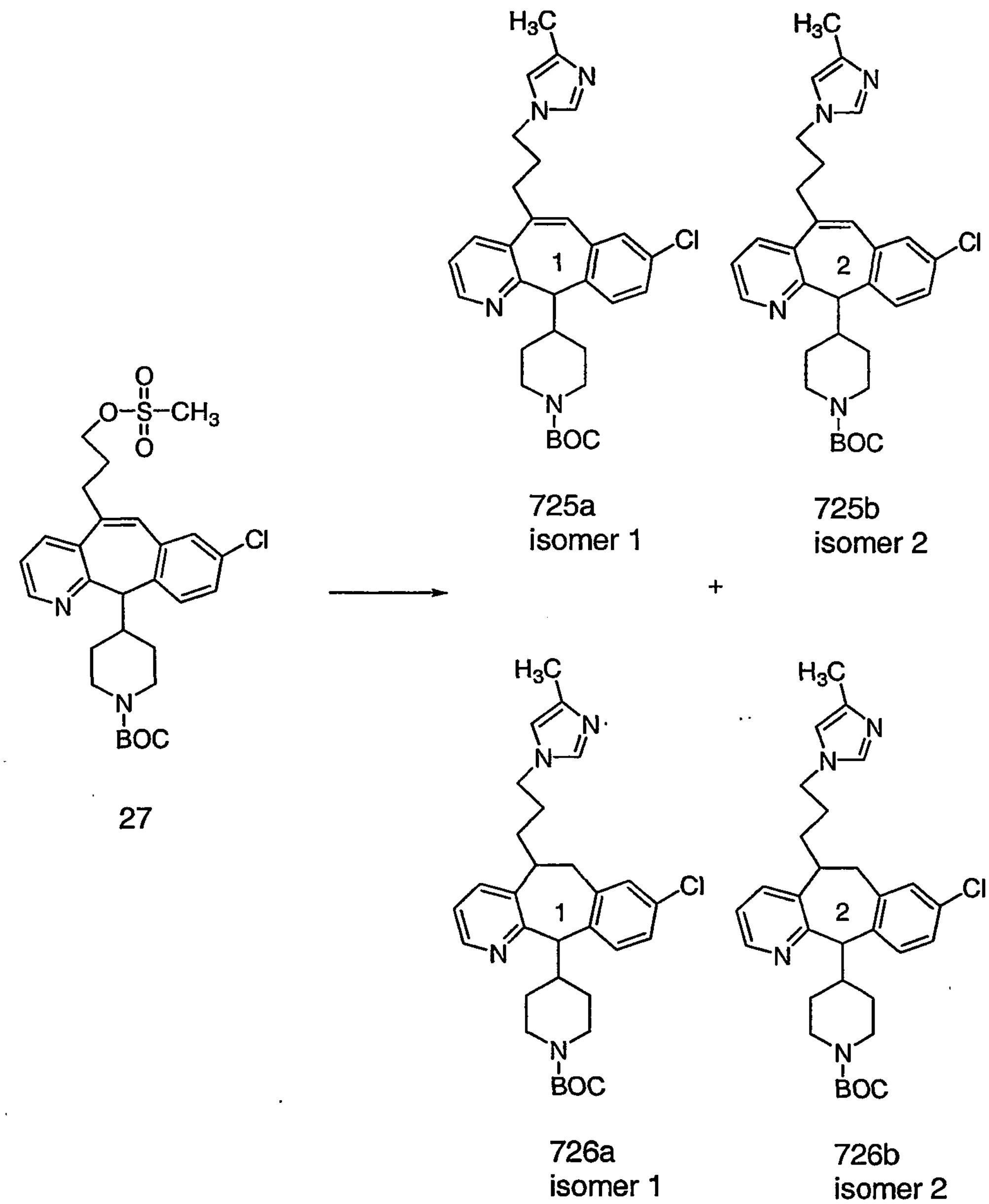

27

725a isomer 1

725b isomer 2

+

726a isomer 1

726b isomer 2

Compound (27) from Preparative Example 4, Step E was reacted in essentially the same manner as described in Preparative Example 60, Step A above substituting 4-Methylimidazole for 4,5-Dimethylimidazole to obtain four seperate compounds as products.

BOC derivatives

Compound (725a) isomer 1, type A (0.69 g, $MH^+$=533.1)
Compound (725b) isomer 2, type A (0.10 g, $MH^+$=533.1)
Compound (726a) isomer 1, type B (0.35 g, $MH^+$=535.1)
Compound (726b) isomer 2, type B, (0.22 g, $MH^+$=535.1)

STEP B Preparation of Compounds (727a) (727b), (728a), (728b)

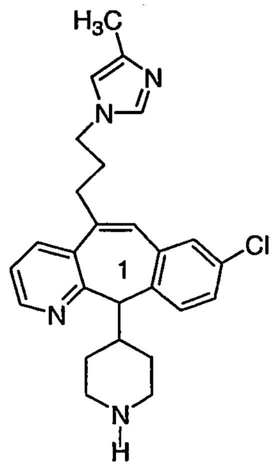

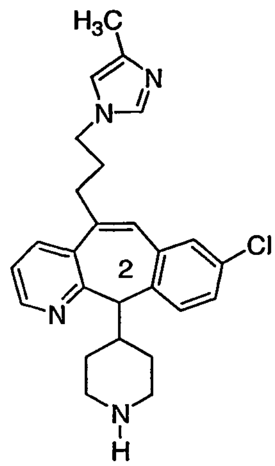

726a
isomer 1

726b
isomer 2

+

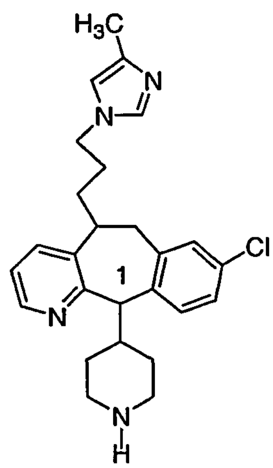

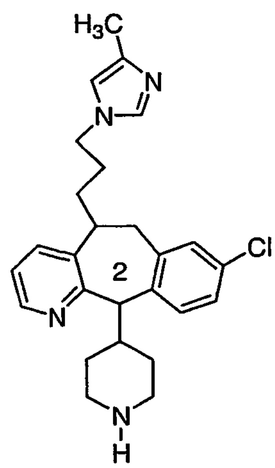

727a
isomer 1

727b
isomer 2

In essentially the same manner as described in Preparative Example 60, Step B, the -NH derivatives were prepared:

Compounds:

(727a) isomer 1 type B (0.3 g, 100% yield, MH+=435.1),
(727b) isomer 2, type B;
(728a) isomer 1, type A and
(728b) isomer 2, type A.

<u>Examples 455-459</u>

Reacting Compounds (727a) and (727b) seperately following the procedure described in Example 13 with the appropriate chloroformate or isocyanate, the following compounds listed in Table 17 below were prepared.

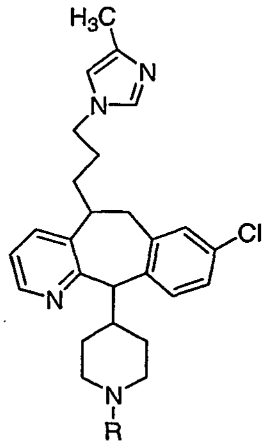

Table 17: 4-Methylpropylimidazole-5-Substituted Bridgehead Single bond Analogs

| EXAMPLE # | R | COMPOUND # | PHYSICAL DATA |
|---|---|---|---|
| 455 | 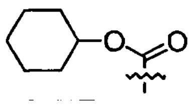 | 729 | MH+=561.1 |
| 456 | 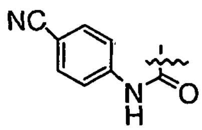 | 730 | MH+=581.1 |
| 457 | 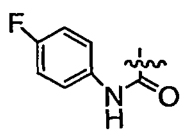 | 731 | MH+=572.1 |
| 458 | 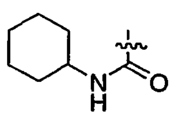 | 732 | MH+=560.1 |
| 459 | 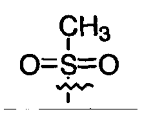 | 733 | MH+=513.1 |

Examples 460-469

Reacting Compounds (728a) and (728b) seperately following the procedure described in Example 13 with the appropriate chloroformates and isocyanates, the following compounds listed in Table 18 below were prepared.

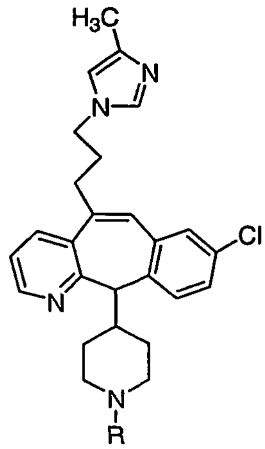

Table 18: 4-Methylpropylimidazole-5-Substituted Bridgehead Double bond Analogs

| EXAMPLE # | R | COMPOUND # | PHYSICAL DATA |
|---|---|---|---|
| 460 | 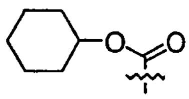 | 734 | MH+=559.1 |
| 461 | 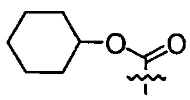 | 735 | MH+=559.1 |
| 462 | 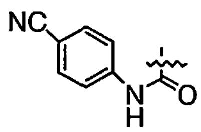 | 736 | MH+=579.1 |
| 463 | 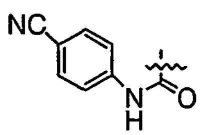 | 737 | MH+=579.1 |
| 464 | 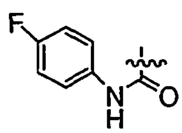 | 738 | MH+=570.1 |
| 465 | 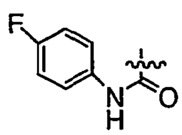 | 739 | MH+=570.1 |
| 466 | 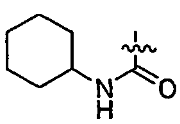 | 740 | MH+=558.1 |
| 467 | 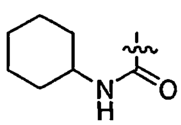 | 741 | MH+=558.1 |

(continued)

| EXAMPLE # | R | COMPOUND # | PHYSICAL DATA |
|---|---|---|---|
| 468 | 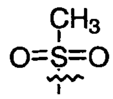 | 742 | MH+=511.1 |
| 469 | 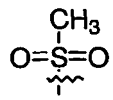 | 743 | MH+=511.1 |

EXAMPLE 470

PREPARATION OF COMPOUND (748).

Step A Preparation of compound (744).

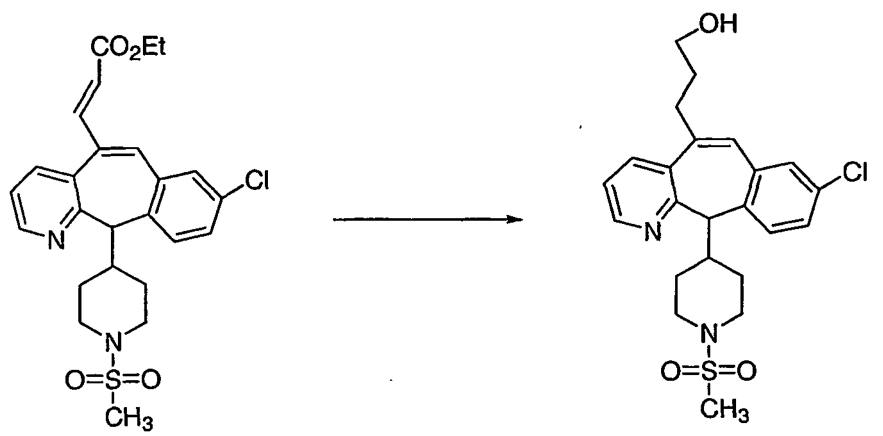

24 744

To a stirred solution of Compound (24) from Preparative Example 4, Step D (4.0 g, 8.2 mmol) under nitrogen at room temperature, was added CuCl (0.7 g, 8.2 mmol). The solution was then cooled to 0˚C, followed by portion wise addition of $NaBH_4$ (4.66 g, 123,2 mmol). The resulting solution was stirred at 0˚C for 6 h., concentrated to dryness, then extracted with $CH_2Cl_2$-sat.$NaHCO_3$. The combined organic layer was dried over $MgSO_4$, filtered, concentrated and purified by column chromatography on 200 mL of normal phase silica gel, eluting with 20%EtOAc/$CH_2Cl_2$ to give Compound (744) (3.62 g, 99% yield, $MH^+$=447).

Step B Preparation of compounds (745) AND (20).

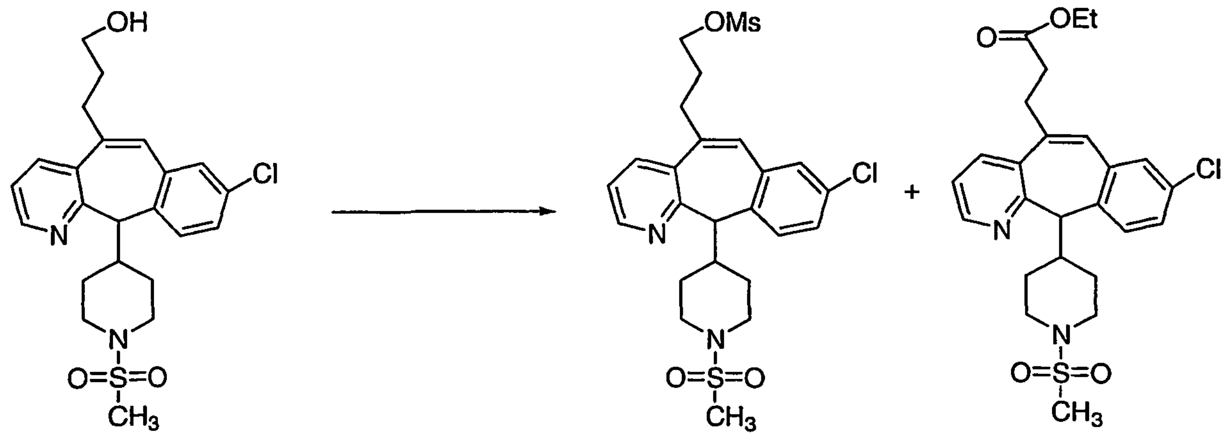

744 745 20

To a stirred solution of Compound (744) from Step A above (3.0 g, 5.7 mmol) in $CH_2Cl_2$ (100 mL) under nitrogen at room temperature, was added triethyl amine (2.4 mL, 17.1 mmol) and methanesulfonyl chloride (0.98 g, 8.7 mmol). The resulting solution was stirred at room temperature over night, then washed with saturated $NaHCO_3$. The combined organic layer was dried over $Na_2SO_4$, filtered, concentrated to dryness and purified by Biotage column chromatography, eluting with 30%EtOAc/70%$CH_2Cl_2$ to give Compound (745) as a white solid (1.19 g, $MH^+$=525.1) and Compound (20) (1.31 g, $MH^+$=489.1)

Step C Preparation of compound (746).

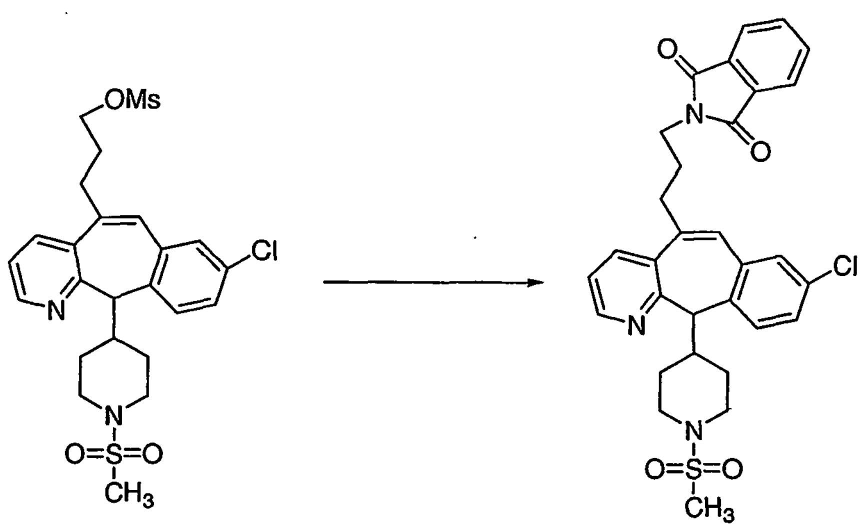

745 746

To a stirred solution of Compound (745) from Step B above (2.17 g, 4.3 mmol) in DMF (50 mL) under nitrogen at room temperature was added phthalimide potassium derivative (1.20 g, 0.5 mmol). The resulting solution was heated to 90°C for 4 h., cooled down to room temperature, concentrated to dryness and extracted with $CH_2Cl_2$-sat.$NaHCO_3$. The combined organic layer was dried over $Na_2SO_4$, filtered, concentrated to dryness and purified by column chromatography on silica gel, eluting with 50%-70%EtOAc/hexane to give Compound (746) as a white solid (1.76 g, 71 % yield, $MH^+$=577.0).

Step D Preparation of compound (747).

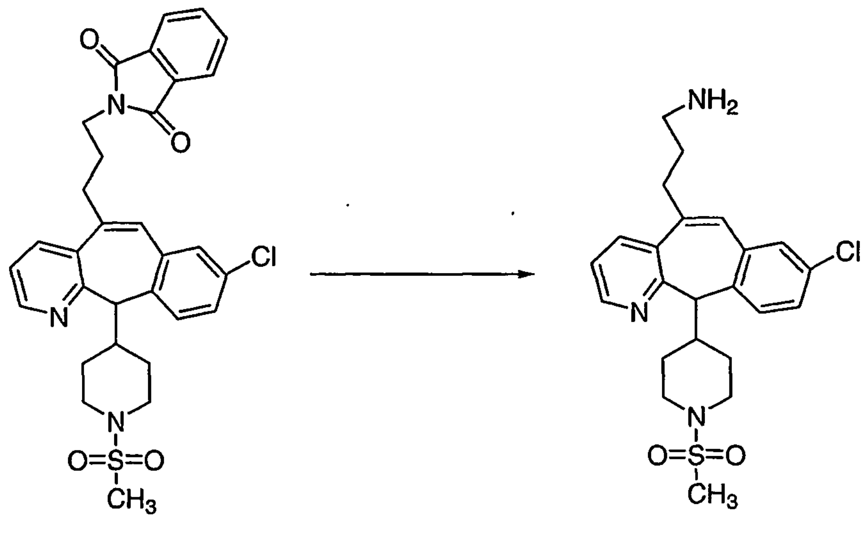

746 747

To a stirred solution of Compound (746) from Step C above (1.67 g, 2.9 mmol) in EtOH (50 mL) at room temperature, was added hydrazine monohydrate (0.29 g, 5.8 mmol). The resulting solution was heated to reflux for 4 h. cooled down to room temperature, concentrated to dryness and extracted with $CH_2Cl_2$-$H_2O$. The combined organic layer was dried over $MgSO_4$, filtered and concentrated to dryness to give Compound (747) as a white solid (1.23 g, 95% yield, $MH^+$= 446.1)

Step E Preparation of compound (748).

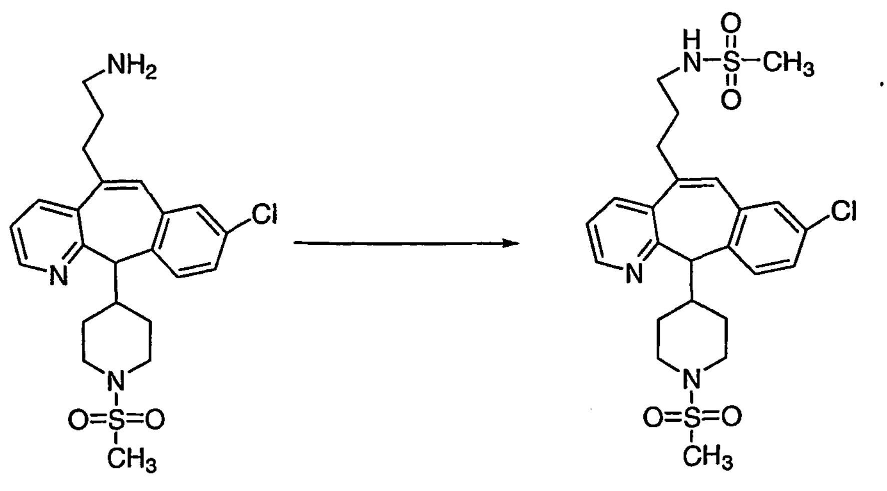

747 748

To a stirred solution of Compound (747) from Step D (0.1 g, 0.22 mmol) in $CH_2Cl_2$ (5 mL) under nitrogen at room temperature, was added TEA (0.06 mL, 0.45 mmol) and methanesulfonyl chloride (0.038 g, 0.34 mmol). The resulting solution was stirred at room temperature over night, then washed with sat. $NaHCO_3$. The combined organic

layer was dried over $Na_2SO_4$, filtered and purified by column chromatography on silica gel, eluting with 3% MeOH-$NH_3/CH_2Cl_2$ to give Compound (748) as a white solid (0.087 g, 76% yield, $MH^+$=524.0)

EXAMPLE 471

Preparation of compound (749).

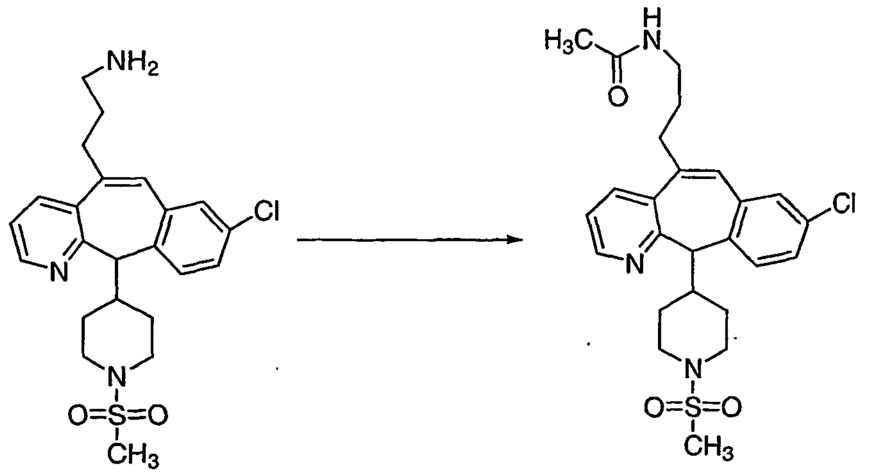

747 749

Reacting Compound (747) from Example 470 Step D above in essentially the same manner as in Step E of Example 470 substituting acetylchloride, Compound (749) was prepared.(0.048 g, 45% yield, MH+= 488.2).

EXAMPLE 472

Step A Preparation of compound (750)

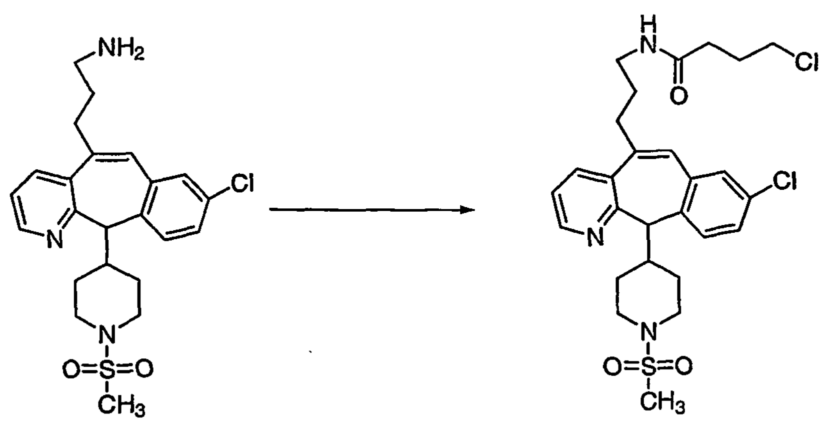

747 750

Reacting Compound (747) from Example 470 Step D above in essentially the same manner as in Step E of Example 470 substituting 4-Chlorobutyryl chloride (ACROS), Compound (750) was prepared (0.67 g, 100% yiled, $MH^+$=514.1).

Step B Preparation of compound (751).

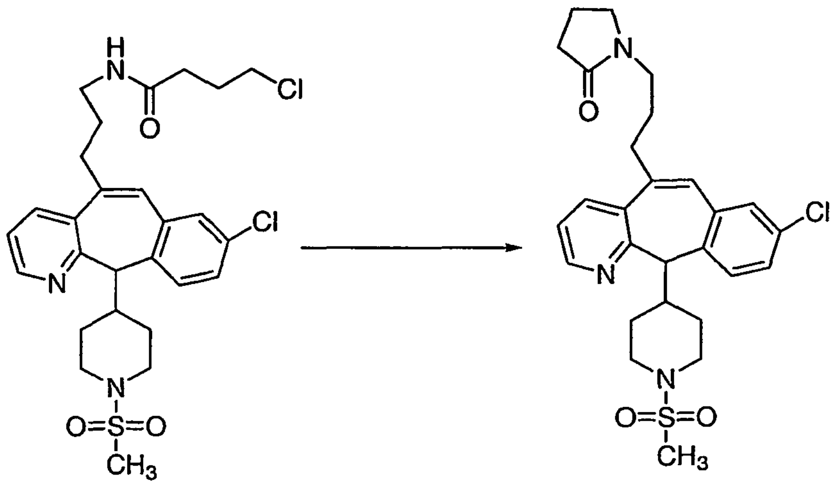

750 751

To a stirred solution of Compound (750) from Step A (0.575 g, 1.11 mmol) in toluene (15 mL) under nitrogen at room temperature, was added $K_2CO_3$ (0.55 g, 4.01 mmol). The resulting solution was stirred at room temperature over the weekend then heated to 55˚C for 7 h. The solution was then cooled down to room temperature, filtered, concentrated to dryness and purified by column chromatography, eluting with 1.5%MeOH-$NH_3$/98.5%$CH_2Cl_2$ to give Compound (751) as a white solid (0.15 g, 26% yield, $MH^+$= 524.1)

EXAMPLE 473

Step A Preparation of compound (752).

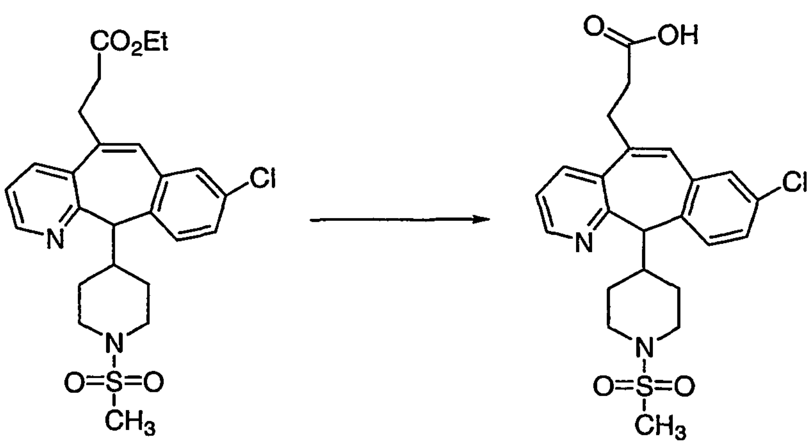

20 752

To a stirred solution of Compound (20) from Example 470, Step B (0.67 g, 1.37 mmol) in THF (5 mL), was added 1N NaOH solution (6.9 mL, 6.88 mmol). The resulting solution was stirred at room temperature overnight and concentrated to dryness. The solution was then acidified with 10% citric acid and then extracted with $CH_2Cl_2$. The combined organic layer was dried over $MgSO_4$, filtered and concentrated to dryness to give Compound (752) as a light yellow product (0.33 g, 52% yield, $MH^+$=461.1)

Step B Preparation of compound (753).

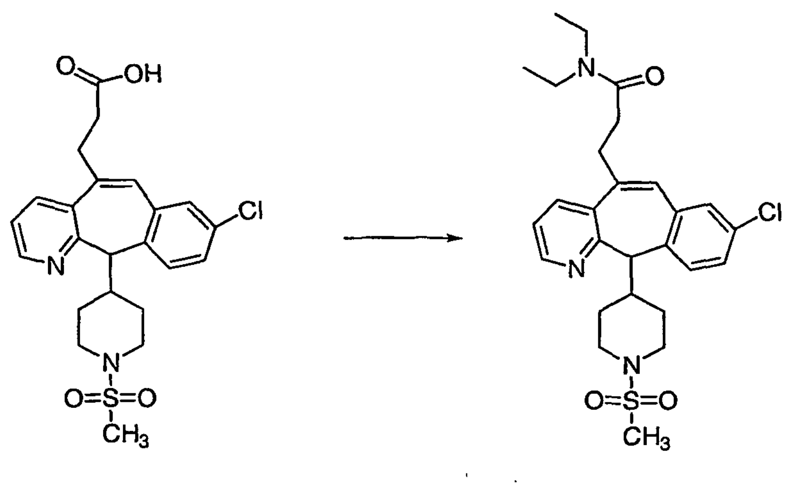

752 753

To a stirred solution of Compound (752) from Step A above (0.1 g, 0.23 mmol) in $CH_2Cl_2$ (5 mL) under nitrogen at room temperature, was added oxalyl chloride (0.97 g, 7.62 mmol) and diethyl amine (0.47 g, 6.43 mmol). The resulting solution was stirred at room temperature for 1 hr and concentrated to dryness. The crude product was then purified by column chromatography, eluting with 2%MeOH-$NH_3$/98%$CH_2Cl_2$ to give Compound (753) as a white solid (0.051 g, 49.5% yield, $MH^+$=516.1)

EXAMPLE 474

Preparation of compound (754)

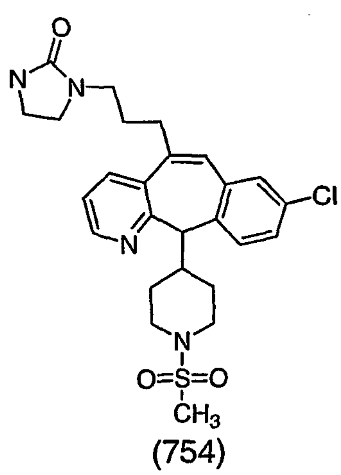

(754)

To a stirred solution of 2-imidazolidone (0.22 g, 2.0 mmol) in DMF (10 mL) was added NaH (0.28 g, 2.0 mmol). The resulting solution was stirred at room temperature for 1 hr. This solution was then added into a solution of Compound (22) from Preparative Example 3, Step C (0.67 g, 1.3 mmol) in DMF (20 mL) under nitrogen inlet at room temperature. The resulting solution was heated to 90°C for 2 hrs, concentrated to dryness, then extracted with $CH_2Cl_2$-sat.$NaHCO_3$. The combined organic layer was then dried over $MgSO_4$, filtered, concentrated to dryness and purified by column chromatography on silica gel, eluting with 3% MeOH-$NH_3$/97% $CH_2Cl_2$ to give a light yellow solid (754) (0.17 g, 25%

yield, $MH^+$=515.1).

EXAMPLE 475

PREPARATION OF COMPOUND (762)

Step A: Preparation of compound (755).

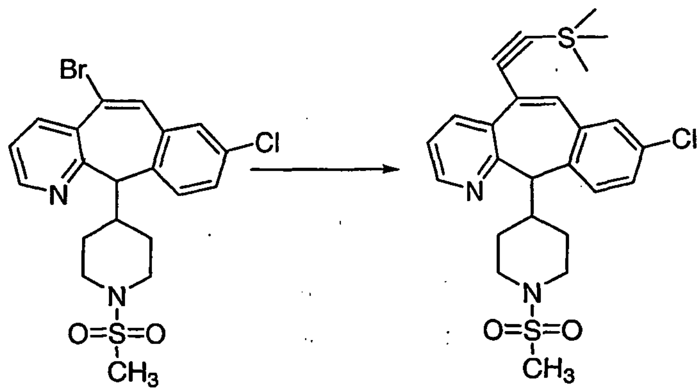

(12) (755)

To a stirred solution of Compound (12) from Preparative Example 2, Step B (15.75 g, 0.336 mmol) in DMF (200 mL) under nitrogen inlet at room temperature, was added trimethylsilylacetalene (12.14 g, 124 mmol), bis(triphenylphosphine)palladium (II)dichloride (0.47 g, 0.67 mmol), $Et_3N$ (13.1 mL, 94 mmol), CuI (0.89 g, 4.7mmol) and NaI (1.53 g, 10 mmol). The resulting solution was stirred at room temperature overnight, concentrated to dryness, then extracted with $CH_2Cl_2$-$H_2O$. The combined organic layer was dried over $MgSO_4$, filtered, concentrated to dryness and purified by column chromatography on silica gel, eluting with 20% EtOAc/80% hexane to give the product (755) (12.35 g, M=485).

Step B: Preparation of compound (756).

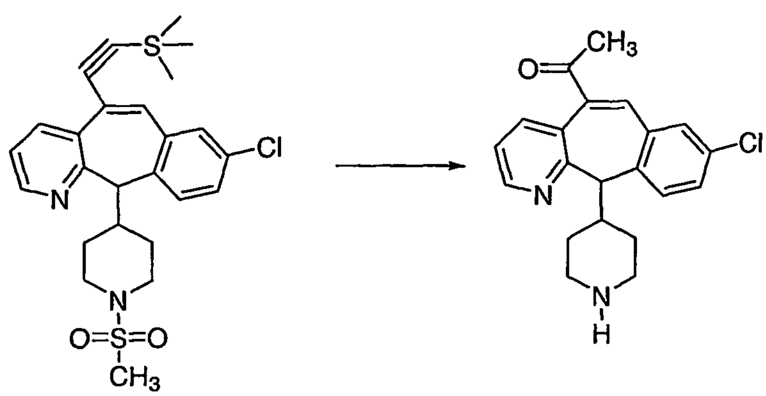

(755) (756)

A solution of Compound (755) from Step A above (4.48 g, 9.24 mmol), in concentrated HCl (100 mL) was heated to reflux overnight. The solution was then cooled down to room temperature and basified with 50% NaOH solution

(w/w) and then extracted with $CH_2Cl_2$. The combined organic layer was dried over $MgSO_4$, filtered and concentrated to dryness to give an off white solid (756) (4.40 g, 100% yield, $MH^+$=353.1).

Step C: Preparation of compound (757).

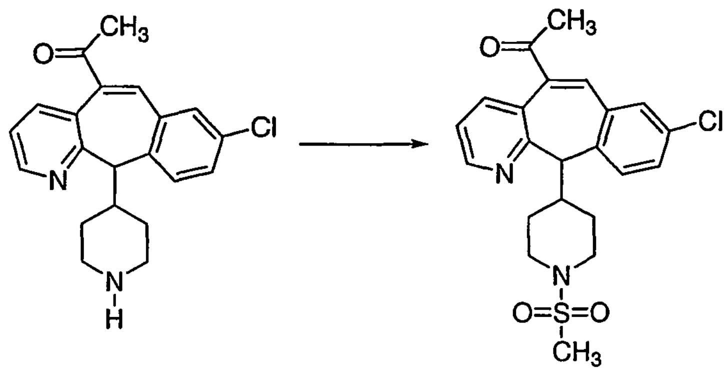

(756) (757)

To a stirred solution of Compound (756) from step B (3.15 g, 8.93 mmol) in $CH_2Cl_2$ (100 mL) was added $Et_3N$ (2.5 mL, 17.85 mmol) and methanesulfonyl chloride (0.51 g, 4.46 mmol). The resulting solution was stirred at room temperature overnight. The solution was then washed with saturated $NaHCO_3$ and the organic layer was dried over $MgSO_4$, filtered and concentrated to dryness to give a crude product (4.31 g, 100% yield, $MH^+$=431.1)

Step D: Preparation of compound (758).

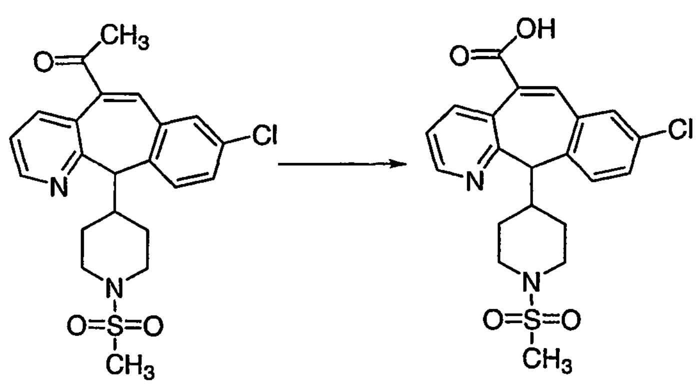

(757) (758)

The solution of Compound (757) from Step C (3.84 g, 8.91 mmol) in 4% NaClO (150 mL) and 45% NaOH solution (15 mL) was heated to reflux for 2 hrs, then cooled down to room temperature, followed by addition of saturated sodium bisulfite solution (150 mL). The solution was then adjusted to pH=6.5 and extracted with $CH_2Cl_2$. The combined organic layer was dried over $MgSO_4$, filtered and concentrated to dryness to give a light yellow solid (3.31 g, 86% yield, $MH^+$=433.1).

Step E: Preparation of compound (759).

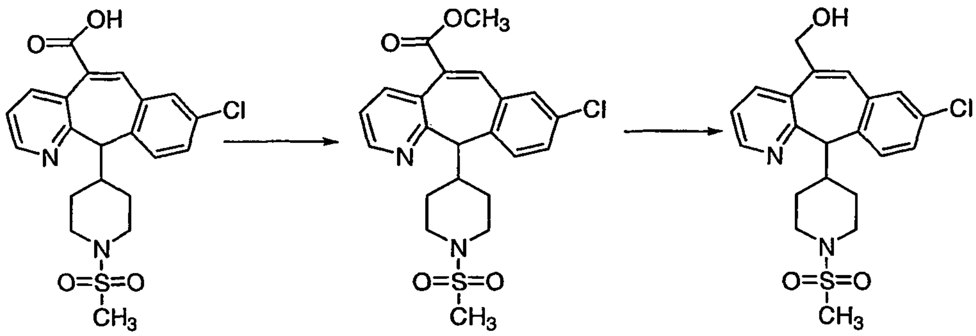

(758) (759) (760)

To a stirred solution of Compound (758) from step D (3.31 g, 7.65 mmol) in toluene (80 mL) and MeOH (50 mL) under nitrogen at room temperature, was added (trimethylsilyl)diazomethane (2.0M in hexane)(3.4 mL, 68.8 mmol) at 0˚C, until the colorless solution turned to yellow solution. The resulting solution was stirred at 0˚C for half an hour and concentrated to dryness to give a crude product (759).

To a stirred cooling solution of the crude product (759) from above, in THF (30 mL) at 0˚C was added DIBAL (15.3 mL, 15.3 mmol). The resulting solution was stirred at 0˚C for 2hrs, followed by extraction with 10% citric acid and 1N NaOH solution. The combined organic layer was dried over $MgSO_4$, filtered and concentrated to dryness to give a light yellow solid (760) (2.90 g, 90% yield, $MH^+$=419.1).

Step F: Preparation of compound (761).

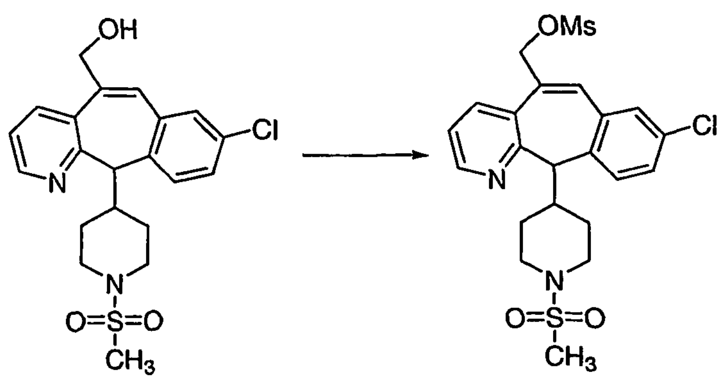

(760) (761)

Reacting Compound (760) in essentially the same manner as Step C above, Compound (761) was prepared.

Step G: Preparation of Compound (762).

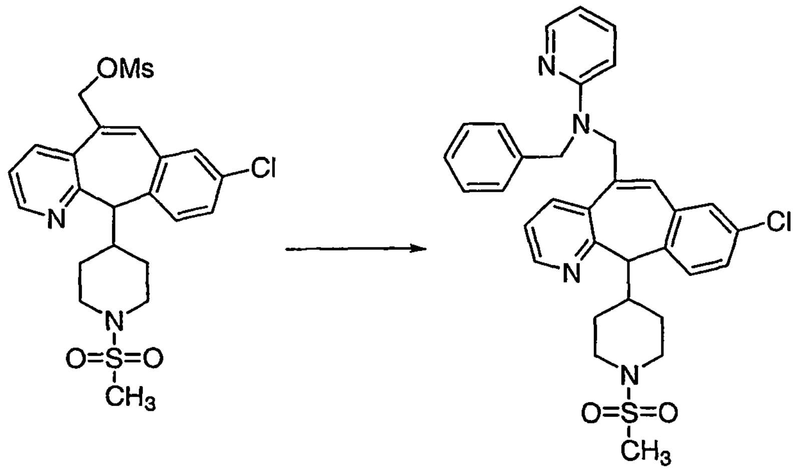

(761) (762)

To a stirred solution of 2-benzylaminopyridine (0.115 g, 0.624mmol) in DMF (10 mL) at room temperature, was added NaH (9.81 g, 0.41 mmol) and stirred for 0.5 hr. To a stirred solution of mesylate compound from step F (0.2 g, 0.41 mmol) in DMF (10 mL) under nitrogen inlet, was added the solution of 2-benzylaminopyridine in DMF above. The resulting solution was heated to 90°C for 3hrs, concentrated to dryness followed by extraction with $CH_2Cl_2$-sat.$NaHCO_3$, then dried over $MgSO_4$, filtered, concentrated to dryness and purified by column chromatography on silica gel, eluting with 5% MeOH-$NH_3$/$CH_2Cl_2$ to give a light yellow solid (762) (0.03 g, 13% yield, $MH^+$=585.1).

EXAMPLE 476

PREPARATION OF COMPOUND (768)

Step A: Preparation of Compound (763).

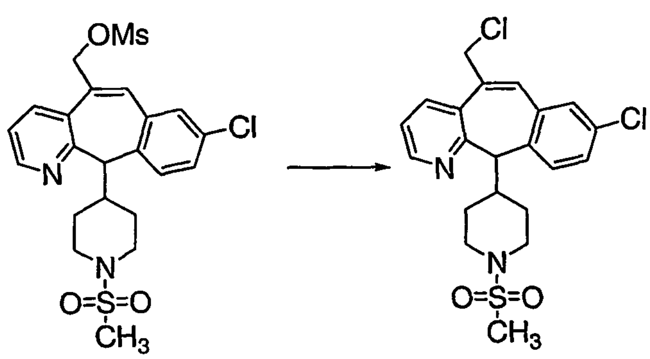

(761) (763)

In essentially the same manner as Example 475, Step E, Compound (763) was prepared.

Step B: Preparation of Compound (764).

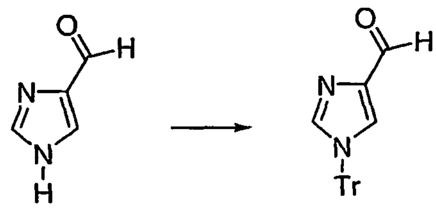

(764)

To a stirred solution of 4(5)-imidazolecarboxaldehyde (20.0 g, 0.208 mmol) in $CH_2Cl_2$ (200 mL), was added $Et_3N$ (29.0 mL, 0.208 mmol). The solution was then cooled down at 0˚C, followed by addition of triphenylmethylchloride (52.8 g, 0.18 mmol) at 0˚C. The resulting solution was stirred at room temperature overnight and then washed it with brine, water and concentrated to dryness to give a white solid (63.0 g, 98% yield, $MH^+$=339.1)

Step C: Preparation of Compound (765).

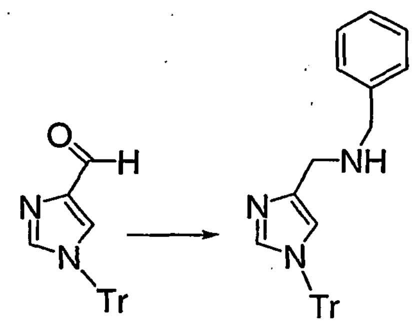

(765)

To a stirred solution of starting material benzyl amine (0.99 g, 8.87 mmol) in MeOH (50 mL) under nitrogen inlet at room temperature, was added sodium acetate (0.73 g, 8.87 mmol), 3˚A molecular sieves (3.0 g) and aldehyde (3.0 g, 8.87 mmol). The resulting solution was stirred at room temperature overnight, followed by addition of $NaBH_4$ (0.67 g, 17.74 mmol), then stirred for 4 hrs and concentrated to dryness, followed by extraction with $CH_2Cl_2$-1N NaOH. The combined organic layer was dried over $MgSO_4$, filtered, concentrated to dryness and purified by column chromatography on silica gel, eluting with 2%MeOH-$NH_3$/98%$CH_2Cl_2$ to give light yellow oil (3.75 g, 98% yield, $MH^+$=430.2)

Step D: Preparation of Compound (767).

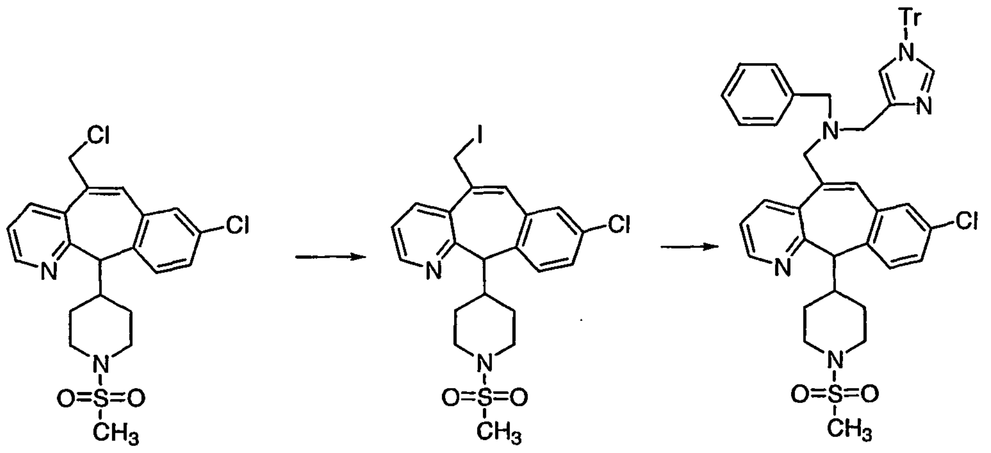

763 766 767

To a stirred solution of Compound (764) from step B (0.41 g, 1.14 mmol) in DMF (10 mL) under nitrogen at room temperature, was added NaH (0.02 g, 0.84 mmol). The resulting solution was stirred at room temperature for 1 hr.

To a stirred solution of Compound (763) from step A (0.4 g, 0.84 mmol) in acetone (30 mL) under nitrogen inlet at room temperature, was added NaI (0.12 g, 0.84 mmol). The resulting solution was heated to reflux for 1 hour and then concentrated to dryness to afford Compound (766). To crude Compound (766) was added, DMF (10 mL) and the solution of Compound (764) from above and NaH (0.02 g, 0.84 mmol). The resulting solution was heated to 90˚C for overnight, then concentrated to dryness and purified by column chromatography on silica gel, eluting with 2% MeOH-$NH_3$/98% $CH_2Cl_2$ to give Compound (767) as a yellow solid (0.23 g, 33% yield, $MH^+$=830.4)

<u>Step E:</u> <u>Preparation of Compound (768).</u>

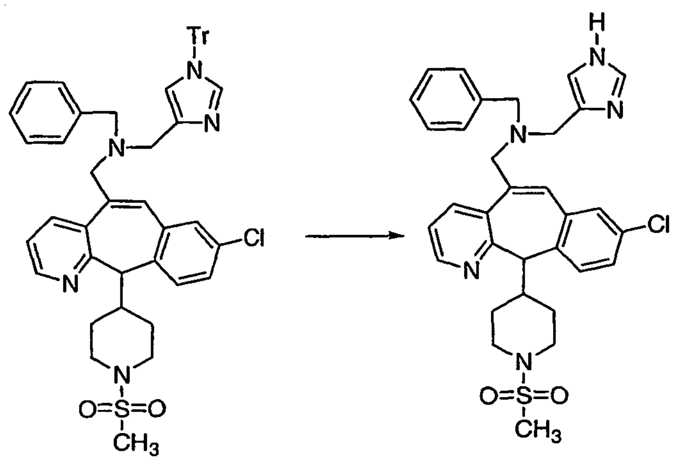

(767) (768)

A solution of Compound (767) from step C (0.238 g, 0.29 mmol) in 80% acetic acid in $H_2O$ was heated to reflux for 2 hrs and then concentrated to dryness, followed by extraction with $CH_2Cl_2$-1N NaOH. The combined organic layer was dried over $MgSO_4$, filtered, concentrated to dryness and purified by column chromatography on silica gel, eluting with 3% MeOH-$NH_3$/97%$CH_2Cl_2$ to give white solid (0.10 g, 62% yield, M=588.2).

PREPARATIVE EXAMPLE 62

Step A 1N-tert-BUTOXYCARBONYL-3(R) AND 3(S)-(1H-IMIDAZOL-1-YL) METHYL) PYRROLIDINES.

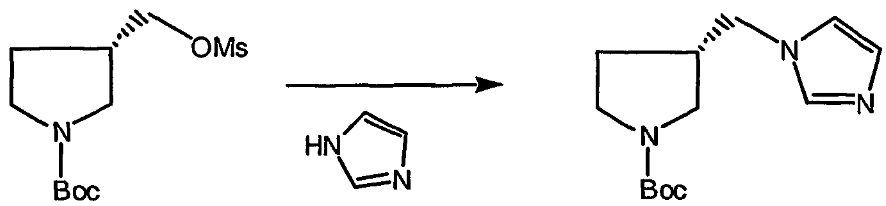

(R)

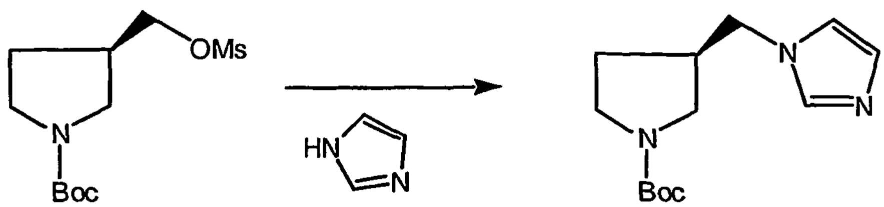

(S)

3(R)-(3-Methanesulfonyloxymethyl)pyrrolidine (J. Med. Chem. 1990, 33, 77-77) (0.993g, 3.56 mmoles) was dissolved in anhydrous DMF (25 mL) and sodium imidazole (0.6g, 10 mmoles) was added. The mixture was heated at 60° C for 2h and then evaporated to dryness. The product was extracted with $CH_2Cl_2$ and washed with brine. $CH_2Cl_2$ extract was evaporated to dryness to give the titled compound (1.1409g, 100%), ESMS: FABMS (M+1) = 252; $\delta_H$ ($CDCl_3$) 1.45 (s, 9H), 1.5-1.7 (m, 1H), 1.9 - 2.1 (m, 1H), 2.5-2.7 (m, 1H), 3.0-3.2 (m, 1H), 3.3- 3.6 (m, 2H), 3.9 (dd, 2H), 6.9 (s, 1H), 7.1 (s, 1H), 7.45 (s, 1H)

In a similar manner, (S) isomer was prepared from 3(S)-(3-Methanesulfonyloxymethyl)pyrrolidine (0.993g, 3.56 mmoles to give the title compound (1.1409g, 100%).

Step B 3(R) AND 3(S)-(1H-IMIDAZOL--1-YL)METHYL]PYRROLIDINES

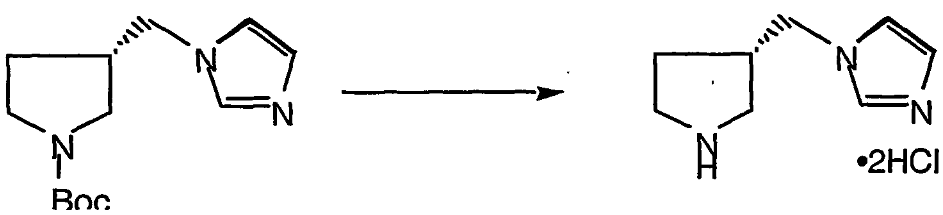

(760a)

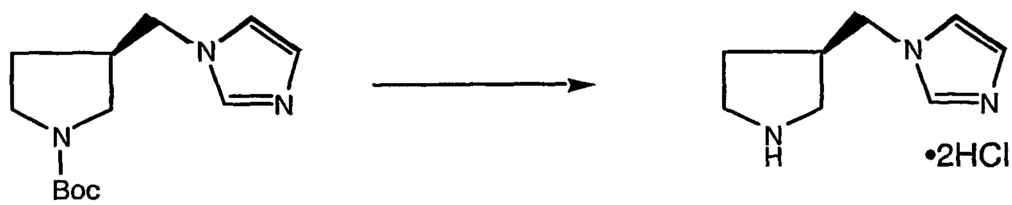

(760b)

The title compound(0.48g, 1.91 mmoles) from Step A was stirred in 4N HCl in dioxane (10 mL) for 2h and then evaporated to dryness to give the title compound which was used to couple with the tricylic acid.

In a similar manner (S) isomer was prepared.

EXAMPLE 477

PREPARATION OF COMPOUND (771)

Step A: Preparation of Compound (769).

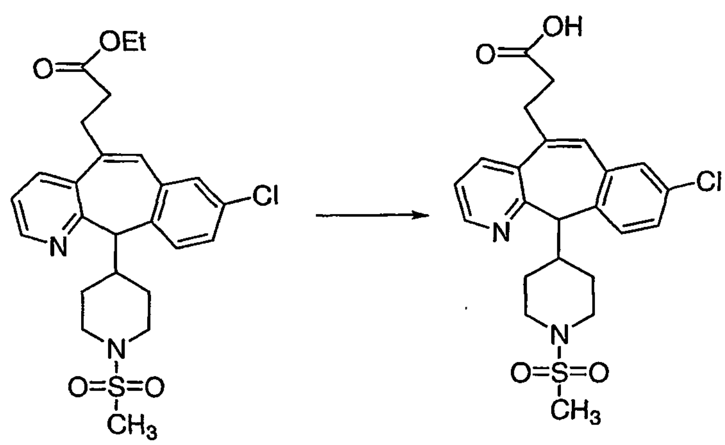

(20) (769)

To a stirred solution of Compound (20) from preparative example 3 step B (4.86 g, 9.94 mmol) in EtOH (100mL), was added 1N LiOH (80 mL). The resulting solution was then stirred at room temperature overnight and concentrated to dryness, followed by dissolving in $CH_2Cl_2$. The solution was then adjusted to pH=6.5-7.0 with 1N HCl. The aqueous layer was then separated and concentrated to dryness , then dissolved in THF to give the lithium salt (4.86 g, 100 %yield, M+Li=467.1)

Step B: Preparation of Compound (771).

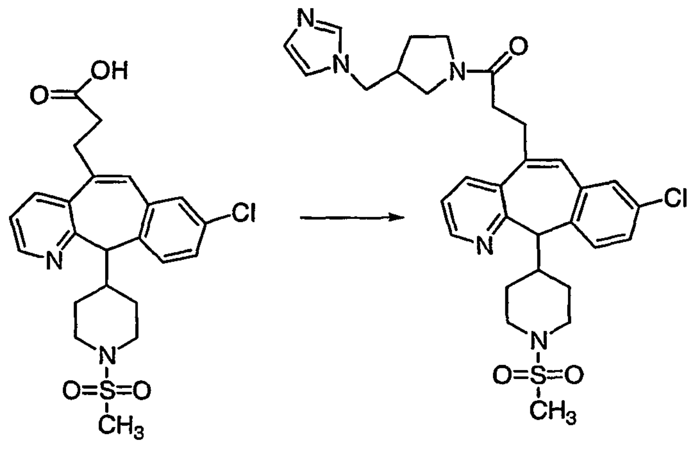

(769) (771)

To a stirred solution of Compound (769) from step A above (0.38 g, 0.84 mmol) in DMF (10mL) under nitrogen inlet at room temperature, was added Compound (770) from Preparative Example 62 (0.163 g, 1.09mmol), benzotriazoyl-N-oxtris (dimethyl - amino)phosphoniumhexafluro phosphate (0.44 g, 1.01 mmol) and $Et_3N$ (0.5 mL, 3.36 mmol). The resulting solution was stirred at room temperature overnight and concentrated to dryness, followed by extraction with $CH_2Cl_2$-10% Citric acid. The combined organic layer was then washed with saturated $NaHCO_3$, brine, dried over $MgSO_4$, filtered, concentrated to dryness and purified by column chromatography on silica gel, eluting with 3% MeOH-$NH_3$/$CH_2Cl_2$ to give a light yellow solid (0.12 g, M=594.2).

PREPARATIVE EXAMPLE 63

COMPOUND (772)

Step A 1N-tert-BUTOXYCARBONYL-4-HYDROXY-PIPERIDINE.

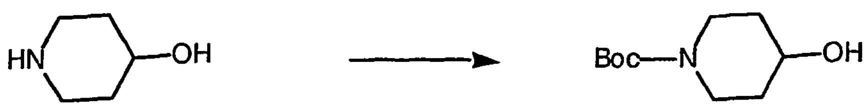

To a solution of 4-hydroxy-piperidine (2g, 19.78 mmoles) and triethylamine (4.16 mL, 29.67 mmoles) in $CH_2Cl_2$ (20mL), di-tert-butyldicarbonate (5.18g, 23.72 mmoles) was added and stirred at room temperature for 16h. The solution was diluted with $CH_2Cl_2$ and washed with water, dried($MgSO_4$) filtered and evaporated to give the title compound (3.95g, 99%). FABMS (M+1) = 202.

Step B 1N-tert-BUTOXYCARBONYL-4-METHANESULFONYLOXY-PIPERIDINE.

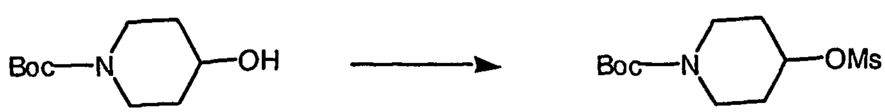

The title compound from Step A above (3.5g, 17.39 mmoles) and triethylamine (4.85mL, 34.79 mmoles) were dissolved in $CH_2Cl_2$ (30 mL) and the mixture was stirred under nitrogen at 0°C. Methanesulfonylchloride (1.62 mL, 20.88 mmoles) was added and the solution was stirred at room temperature for 2h. The solution was diluted with $CH_2Cl_2$ and washed with saturated aqueous sodium bicarbonate, water and dried ($MgSO_4$), filtered and evaporated to dryness to

give the title compound (4.68g , 96.4 %). ESMS: m/z= 280 ($MH^+$)

Step C 1N-tert-BUTOXYCARBONYL-4-(1H-IMIDAZOL-1-YL) - PIPERIDINE

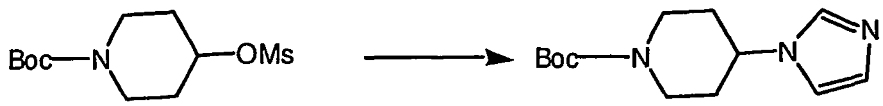

A solution of the title compound from Step B (4.0g, 14.32 mmoles) in DMF (120 mL) was added to a stirred solution of NaH (0.52g, 21.66 mmoles) and imidazole (1.46g, 21.47 mmoles) in DMF (20 mL) under nitrogen atmosphere. The mixture was stirred at 60° C for 16h. DMF was evaporated *in vacuo.* The resulting crude product was extracted with $CH_2Cl_2$ and the extract was successively washed with water and brine, and the $CH_2Cl_2$ was evaporated to leave the title residue which was chromatographed on silica gel using 3% (10% conc $NH_4OH$ in methanol)- $CH_2Cl_2$ as eluant to give the title compound (0.94 g, 26%). FABMS (M+1) = 252;•$_H$ ($CDCl_3$) 1.4 (s, 9H), 1.6-1.8 (m, 2H), 2.0 (dd, 2H), 2.8 (dt, 2H), 4.05 (m, 1H), 4.2 m, 2H), 6.9 (s, 1H), 7.0 (s, 1H), 7.65 (s,1H).

Step D 4-(1H-IMIDAZOL-1-YL)-PIPERIDINE.

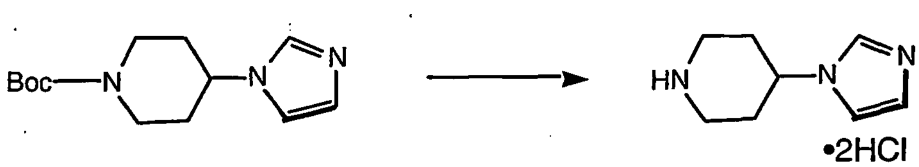

(772)

The title compound(0.21g, 0.836 mmoles) from Step C was stirred in 4N HCl in dioxane (5 mL) for 2h and then evaporated to dryness to give the title compound (772)which was used to couple with the tricylic acid.

EXAMPLE 478

PREPARATION OF COMPOUND (773)

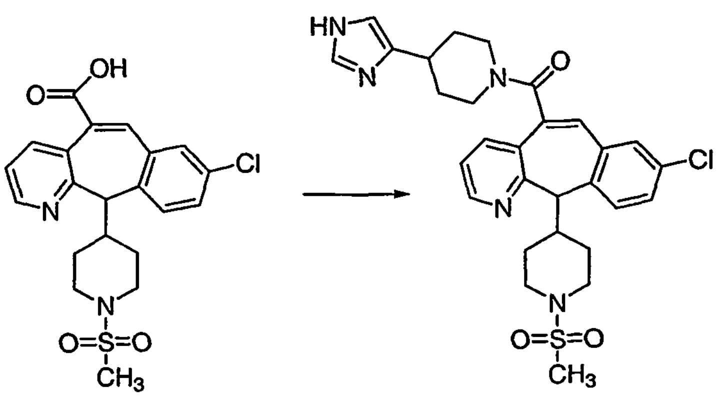

(758) (773)

To a stirred solution of Compound (758) from Example 475 step D (0.2 g, 0.46 mmol) in $CH_2Cl_2$ (5 mL) under nitrogen at room temperature, was added Compound (772) from Preparative Example 63, Step D (0.19 g, 0.55 mmol), bezotriazoyl-N-oxy-tris-(dimethylamino)phosphoniumhexaflurophosphate (0.25 g, 0.55 mmol) and $Et_3N$ (0.3 mL, 1.85 mmol). The resulting solution was stirred at room temperature overnight and concentrated to dryness, followed by extraction with $CH_2Cl_2$-10% citric acid. The combined organic layer was then washed with sat. $NaHCO_3$, brine, dried over $MgSO_4$, filtered ,concentrated to dryness and purified by column chromatography on silica gel, eluting with 3%MeOH-$NH_3$/$CH_2Cl_2$ to give a white solid (773) (0.013 g, 5% yield, M=566.2)

EXAMPLE 479

PREPARATION OF COMPOUNDS (774-777)

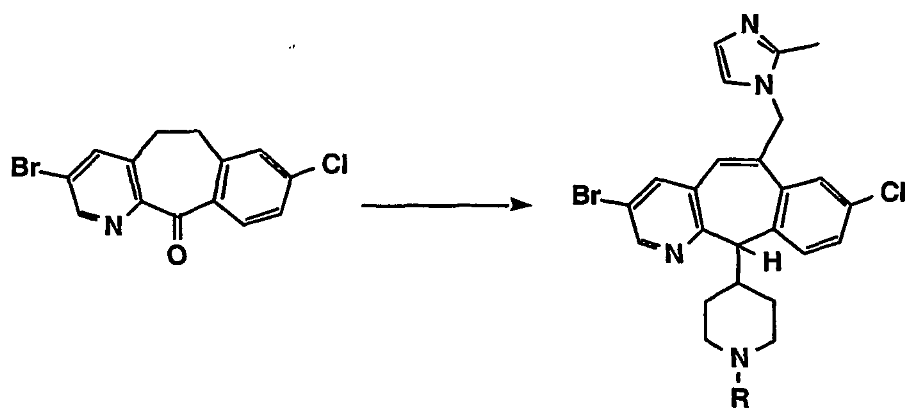

R = N-BOC
(774) (enantiomer 1), (M+1 = 584)
(775) (enantiomer 2) (M+1 = 584)

R = H
(776) (enantiomer 1)
(777) (enantiomer 2)

3-bromo-8-chloroazaketone (U.S. patent 5,977,128, Preparative Example 11, step A, (1999)) was reacted in essentially the same manner as in Preparative Example 23, and Example 91 to obtain the N-BOC derivatives (774) and (775). Compounds (774) and (775) were then reacted separately in essentially the same manner as in Preparative Example 19, Step D to obtain the enantiomers (776) and (777).

EXAMPLE 480

PREPARATION OF COMPOUNDS (778) AND (779)

In essentially the same manner as in Examples (420) and (421), Compounds (778) and (779) were prepared.

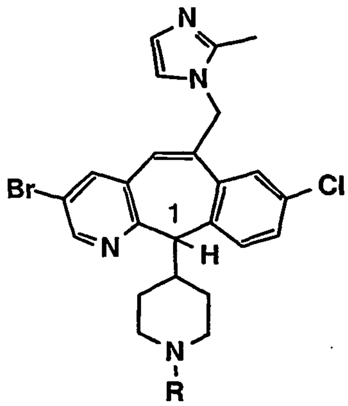

or

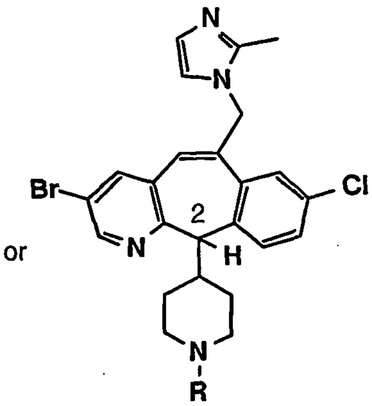

| Compound # | R= | Enantiomer | FABMS(M+1) |
|---|---|---|---|
| 778 | 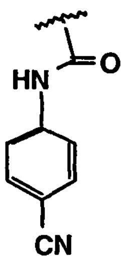 | 1 | 628 |
| 779 | 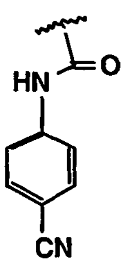 | 2 | 628 |

Phys. Data

(778): $^1$H-NMR (Varians 400 MHz, $CDCl_3$, ppm): $\delta$=8.564 (1H, d, J=2 Hz), 7.784 (1H, d, J=2 Hz), 7.624 (1H, d, J=2 Hz), 7.51-7.37 (5H, m), 7.305 (1H, s), 7.267 (1H, s), 6.870 (1H, s), 6.867 (1H, s), 6.579 (1H, s), 5.282 (1H, d, J=16 Hz), 5.031 (1H, d, J=17 Hz), 4.576 (1H, s), 3.176 (4H, br ddd, J=6, 14 and 58 Hz), 2.485 (3H, s), 1.950 (4H, dd, J=6 and 9 Hz); MS (m/e) 630 (M+H), 340, 327, 293, 263, 249; HRMS (Jeol JMS-HX110A) calcd for C31H27BrClN7O 628.1227 (M+1), found 628.1229.

EXAMPLE 481

PREPARATION OF COMPOUNDS (780) AND (781)

In essentially the same manner as in Example 70, Compounds (780) and (781) were prepared.

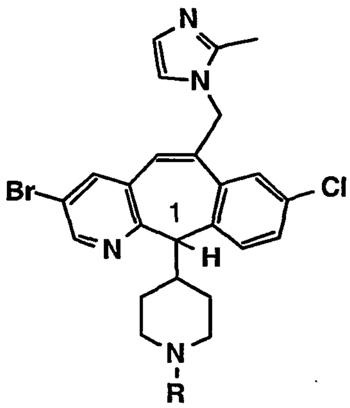

or

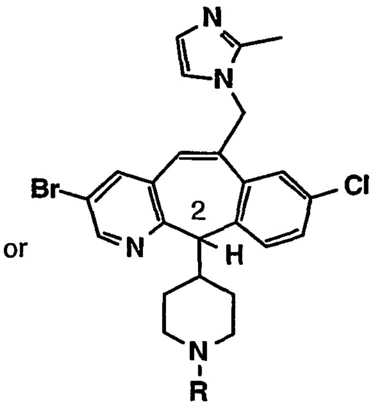

| Compound # | R= | Enantiomer | FABMS(M+1) |
|---|---|---|---|
| 780 | 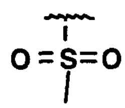 | 1 | 562 |
| 781 | 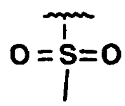 | 2 | 562 |

PREPARATIVE EXAMPLE 64

STEP A COMPOUND (782)

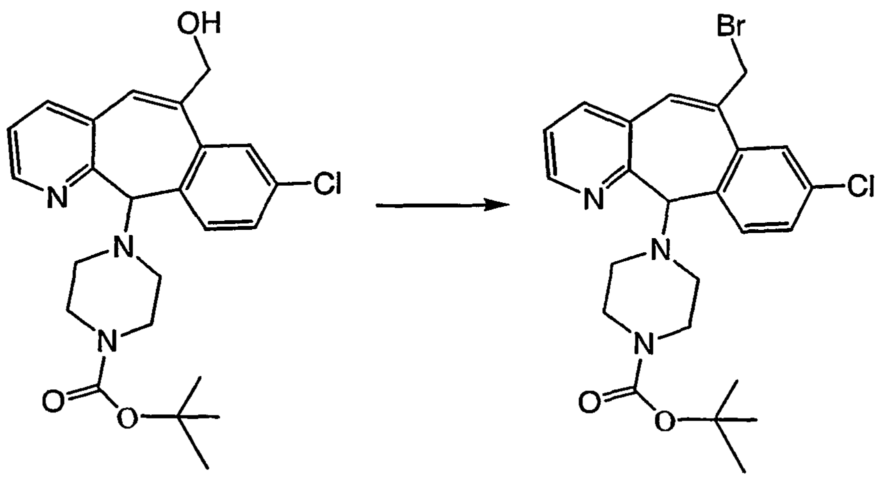

368 782

Compound (368) from Preparative Example 42, Step C (2.34g, 5.29 mmol) was dissolved in 25 mL $CH_2Cl_2$ at 0 °C. $PPh_3$ (1.66g, 6.34 mmol) and NBS (1.03g, 5.82 mmol) were added. After 90 mins, the reaction was diluted with $CH_2Cl_2$ (20 mL), washed with sat. $NaHCO_3$, brine and dried with $MgSO_4$. The crude product was purified on a silica gel column (4:1 hexanes/EtOAc to 2:1) to yield 1.8 g of Compound (782) as a light yellow solid. MS M+1 504.

Step B Compound (783)

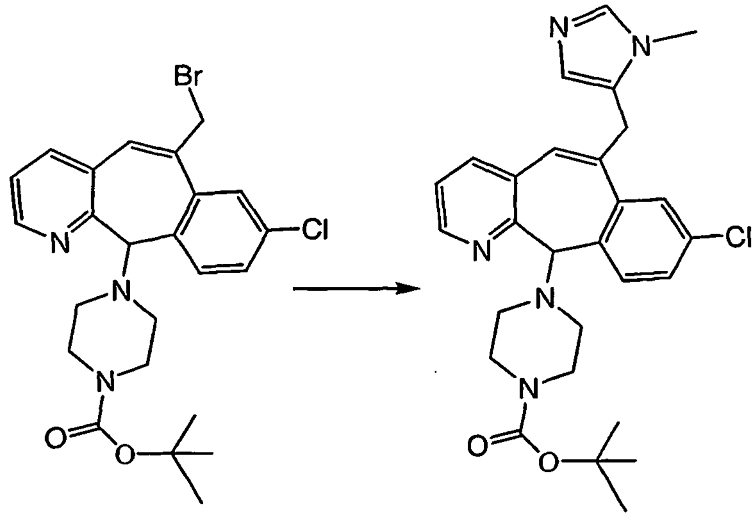

782 783

5-Iodo-1N-methyl imidazole (455 mg, 2.18 mmol) was dissolved in 10 mL THF at room temperature. EtMgBr (2.4 mL, 1.0 M in THF) was added dropwise. After 30 mins, the reaction mixture was cooled to 0˚C. 10 mL THF solution of CuCN (175 mg, 1.96 mmol) and LiCl (166 mg, 3.9 mmol) was then added. 10 mins later, Compound (782) from Step A above (989 mg, 1.96 mmol, in 10 mL THF) was added. The reaction was stirred overnight. Sat. $NH_4Cl$ solution was added to quench the reaction. The resulting emulsion was filtered through a sintered funnel and the filtrate was extracted with EtOAc twice. The organic layer was washed with $NaHCO_3$ solution and brine, dried over magnesium sulfate, filtered and evaporated *in vivo.* The resulting crude material was chromatographed on a silica gel column (using 1:1 hexanes/ EtOAc then 10:1 $CH_2Cl_2$/MeOH) to obtain 330 mg of the title product. MS M+1 = 506 The enantiomers were seperated on a chiral AD column.

EXAMPLE 482

Preparation of compound (784)

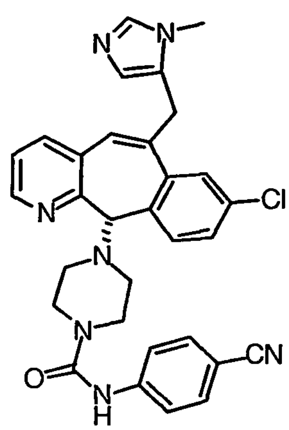

784

Compound (783) from Preparative Example 64, Step B above (40 mg) was dissolved in $CH_2Cl_2$ (5 mL) at room

temerature followed by addition of TFA (0.5 mL). After 2 hrs, the solvent was evaporated *in vivo* and coevaporated with $PhCH_3$ twice. The crude mixture was then dissolved in $CH_2Cl_2$ (4 mL) and $Et_3N$ was added dropwise till the solution became basic by PH paper. 4-Cyanophenyl isocyanate (14 mg) was added. After 5 minutes, the reaction mixture was evaporated *in vivo* to dryness. The crude material was then purified using prep TLC plate (10:1 $CH_2Cl_2$/MeOH) to get 23 mg of Compound (784) as a white solid. MS M+1 550.

EXAMPLE (483)

Preparation of compound (785)

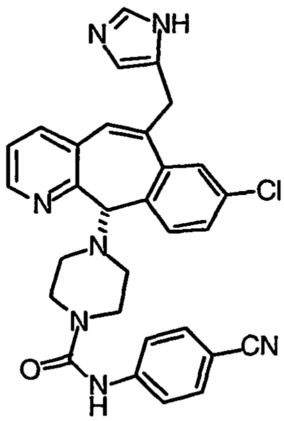

785

Compound (785) was prepared following essentially the same procedure as in Preparative Example 64 and Example 482, substituting 4-lodo-1-trityl imidazole for 5-lodo-1N-methyl imidazole.

EXAMPLE 484

Preparation of compounds (786) and (787)

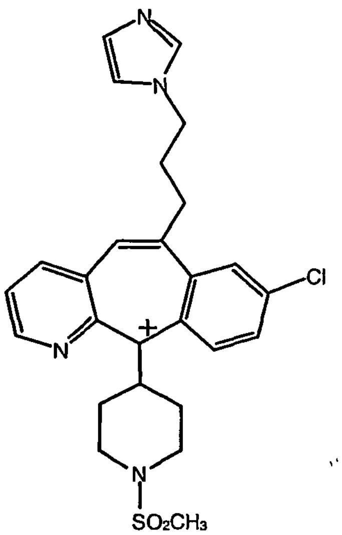

OR

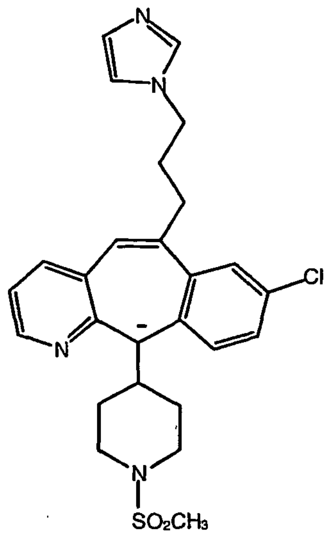

786 787

Compound (786) and (787) were prepared following essentially the same procedure as in Preparative Example 7, substituting ketones (15) and (16) from Preparative Example 2, Step D for ketones (9) and (10).

Compound (786) $MH^+$=497; $[\alpha]_D^{20}$= +15.3;
Compound (787) $MH^+$=497; $[\alpha]_D^{20}$= -13.4.

EXAMPLE 485

Preparation of compound (788)

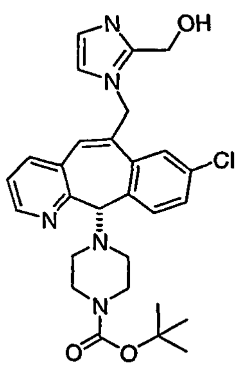

Following essentially the same procedure as in Preparative Example 33, Steps E-H, except substituting compound (365) for Compound (281) and 2-hydroxymethyl imidazole for 1-methyl imidazole, compound (788) was prepared. (788):$^1$H-NMR (Varians 400 MHz, $CDCl_3$, ppm): δ=8.5 (1H,dd), 7.34 (1H,s), 7.59 (1H, d), 7.4 (2H, m), 7.25 (2H, m), 7.04 (1H, s), 6.9 (1H, s), 6.6 (1H, s), 5.37 (2H, dd), 4.8 (2H, dd), 4.6 (1H, s), 3.2 (5H, br s), 2.0 (2H, br s), 1.9 (2H, br s), 1.4 (9H, s).

PREPARATIVE EXAMPLE 65

STEP A COMPOUND (789)

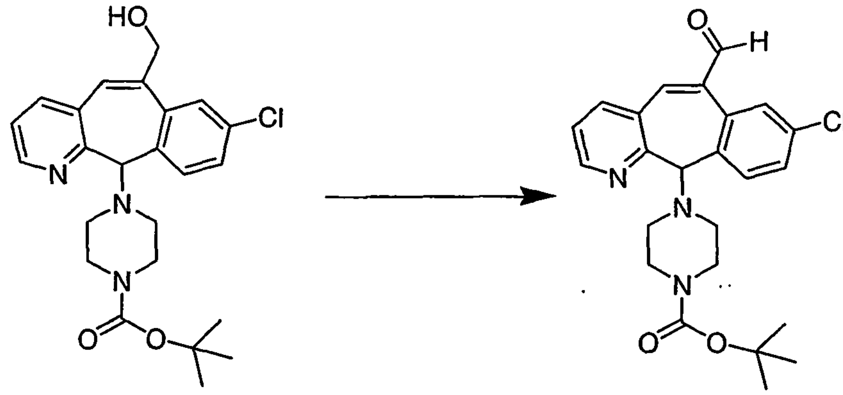

To a solution of the alcohol (3.8 g, 8.6 mmol) in $CH_2Cl_2$ (100 mL) under nirtogen was added $MnO_2$ (40 g). The resulting solution was stirred at room temperature for 4 days. The mixture was then filtered through a pad of Celite with ethyl acetate (500 mL) as the eluant. The filtrate was concentrated to yield a yellow liquid (4.0 g, MH+ 440.1). The crude material was separated into its pure isomers by HPLC, using a chiral AD column eluting with 20% IPA/80%Hexanes/0.2%DEA (isomer 1, 810 mg; isomer 2, 806 mg).

STEP B COMPOUND (790)

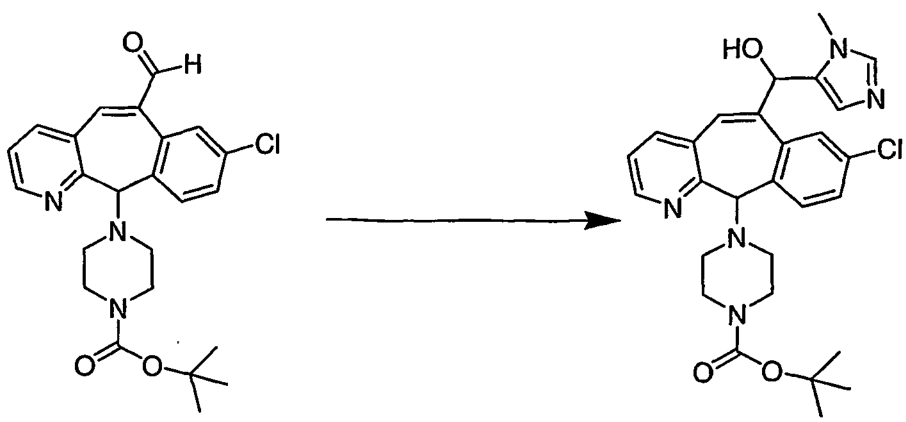

To a solution of imidazole Grignard prepared from 5-iodo-1N-methyl imidazole (312 mg, 1.5 mmol, preparative example 64 step B) was added a solution of aldehyde (791) (380 mg, 0.86 mmol) in $CH_2Cl_2$ (10 mL). After stirring at room temperature overnight, the mixture was heated to 40 ˚C for one hour. After cooling to room temperature again, saturated $NH_4Cl$ solution was added to quench the reaction. The organic layer was dried and the solvent was evaporated. The residue was then purified by silica gel column (from 2% to 10% MeOH in $CH_2Cl_2$) to give the product as a brown oil (207 mg, 46% yield, MH+= 522.1). The diastereomers were then separated by HPLC, using a chiral AD column eluting with 20%
IPA/80%Hexanes/0.2%DEA.

STEP C COMPOUND (791)

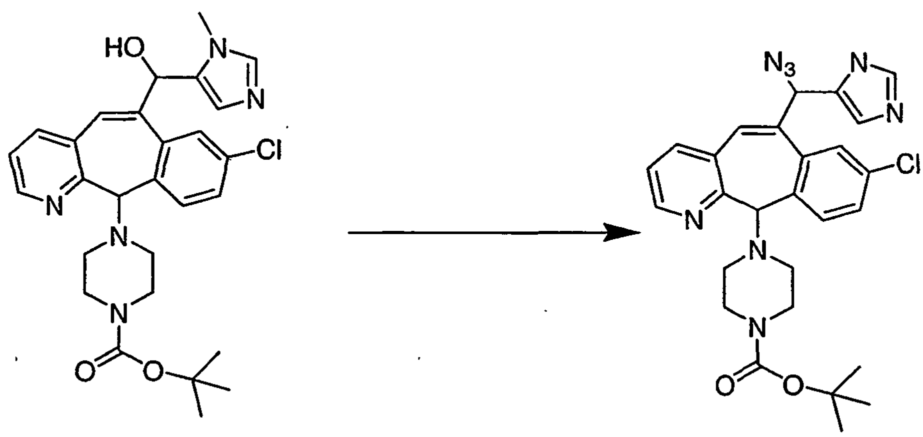

To a THF solution (5 mL) of (790) (200 mg, 0.38 mmol) at room temperature was added DPPA (210 mg, 0.76 mmol) followed by addition of DBU (120 mg, 0.76 mmol). The mixture was stirred overnight and then diluted with ethyl acetate (30 mL), washed with water twice and brine once. The organic layer was dried and the solvent was evaporated. The residue was purified by prep TLC (10% MeOH in $CH_2Cl_2$ with 0.2 % $NH_3$) to give product (791) (102.8 mg, MH+ 547.1). Starting material (790) (58 mg) was also recovered. The diastereomers of (791) were separated on a chiral AD column.

EXAMPLE 486

PREPARATION OF COMPOUND (792)

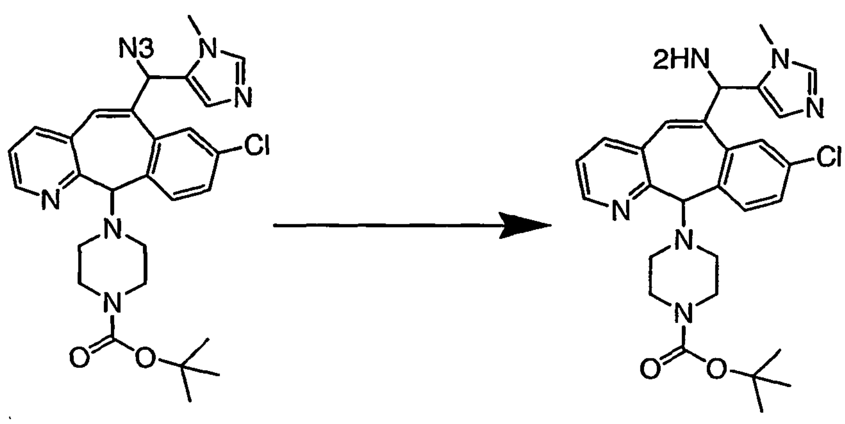

To a wet THF solution (3 mL) of (791) (48 mg, 0.09 mmol) was added $PPh_3$ (32 mg, 0.12 mmol) at room temperature. After stirring overnight, the reaction mixture was concentrated and the residue was purified with prep TLC (10% MeOH in $CH_2Cl_2$ with 0.2 % $NH_3$) to give a white solid (24.3 mg). The white solid was then redissolved in THF/$H_2O$ (5mL/0.5 ml) and the mixture was heated to reflux overnight. The reaction mixture was then partitioned between ethyl acetate and water. The organic layer was dried and concentrated. The residue was purified with prep TLC (5% MeOH in $CH_2Cl_2$ with 0.2 % $NH_3$) to yield a yellow solid (792) (8.3 mg, MH+ 521.1).

EXAMPLE 487

PREPARATION OF COMPOUND (793)

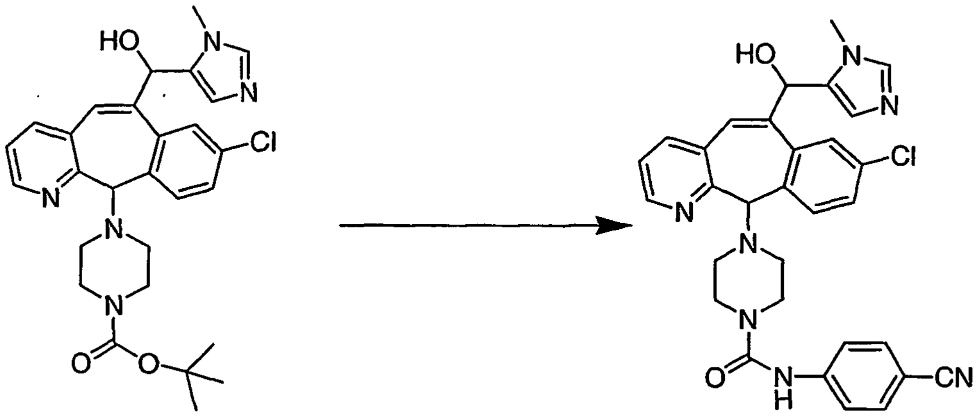

Compound (790) was converted to compound (793) following the essentially the same procedure as described in EXAMPLE 482. MS $M^{+1}$ 566.1.

EXAMPLE 488

PREPARATION OF COMPOUND (794)

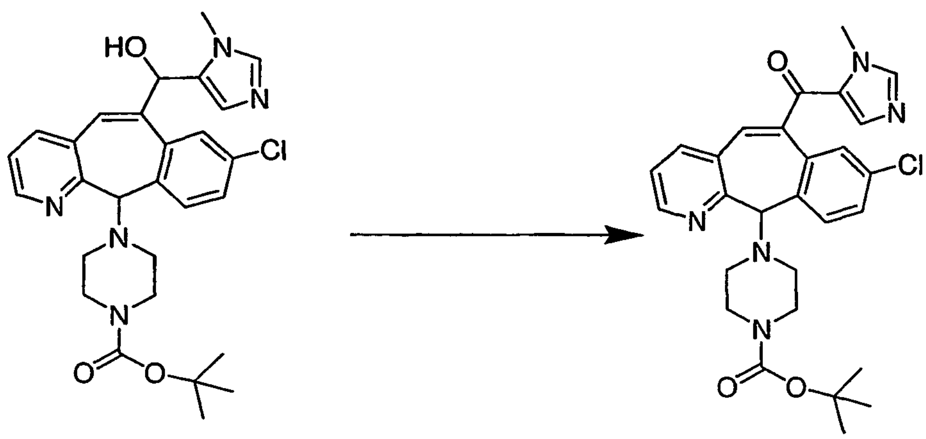

Compound (790) was converted to compound (794) following essentially the same procedure as described in PREPARATIVE EXAMPLE 65, Step A. MS $M^{+1}$ 520.1.

EXAMPLE 489

Step A. Compound (795)

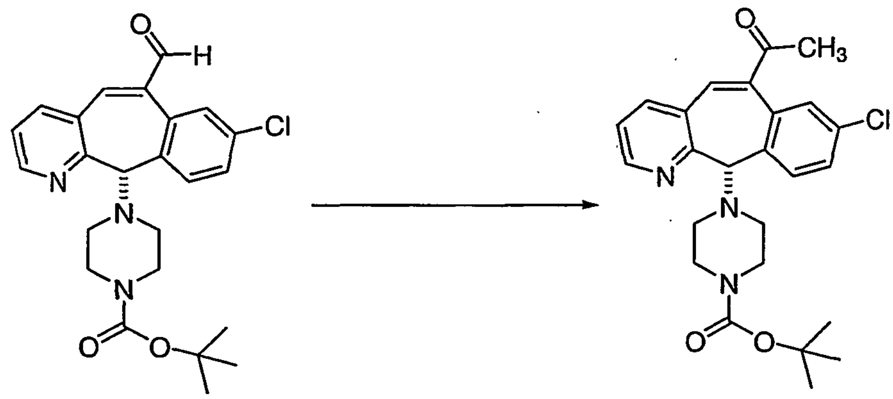

789 795

Aldehyde (789) from Preparative Example 65, Step A (150 mg, 0.34 mmol) was dissolved in THF (6 mL). To this solution was added MeMgBr (0.3 mL, 3.0M in $Et_2O$) dropwise. After stirring at room temperature for 4 hrs, the reaction mixture was quenched with sat. $NH_4Cl$ solution and extracted with ethyl acetate. The organic layer was washed with brine, dried and concentrated to give a yellow solid (150 mg). The crude product was then dissolved in $CH_2Cl_2$ (5 mL). To this solution was added Dess-Martin Periodinane (210 mg) and a drop of water. After 1 hr, aqueous $Na_2S_2O_3$ solution (4 mL, 10%) was added. The mixture was stirred for 10 min. and extracted with $CH_2Cl_2$. The organic layer was washed with $NaHCO_3$, dried and concentrated. The crude material was purified using prep TLC plates (5% methanol in $CH_2Cl_2$) to yield the methyl ketone product (795) as a yellow solid (70 mg).

Step B Compound (795.1)

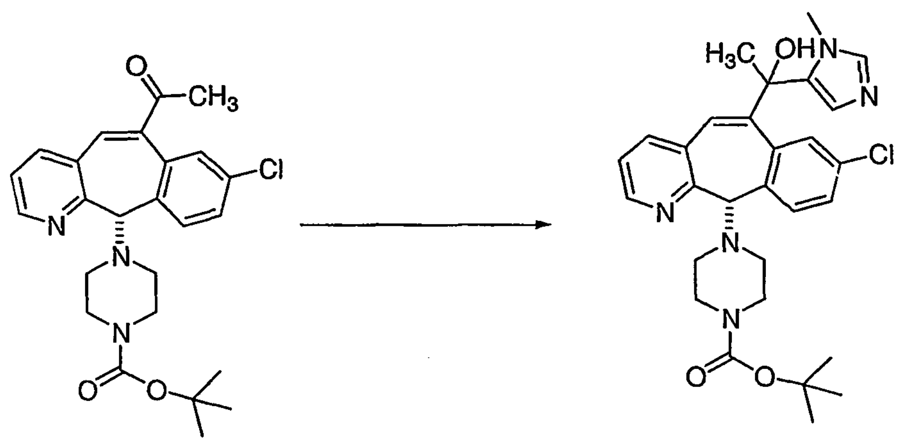

795 795.1

To a solution of imidazole Grignard prepared from 5-iodo-1N-methyl imidazole (624 mg, 3 mmol, see preparative example 64 step B using $ClCH_2CH_2Cl$ as solvent instead of THF) was added a $ClCH_2CH_2Cl$ (6 mL) solution of methyl ketone (795) (272 mg, 0.6 mmol). The mixture was heated to 60 °C for 1.5 hours. After cooling to room temperature, saturated $NH_4Cl$ solution was added to quench the reaction. The organic layer was dried and then evaporated to dryness. The residue was then purified by silica gel column (from 2% to 10% MeOH in $CH_2Cl_2$) to give the product (795.1) as a brown solid (63 mg, 10:1 diastereomeric selectivity, MH+= 536.1). Major diastereomer: ($CDCl_3$, 300 MHz) 8.47 (d, 1H), 7.66 (d, 1H), 7.57 (s, 1H), 7.54 (s, 1H), 7.34 (d, 1H), 7.25-7.22 (m, 1H), 7.05 (s, 1H), 6.89 (s, 1H), 6.82 (s, 1H), 4.61 (s, 1H), 3.84 (s, 3H), 3.24 (br s, 4H), 2.24 (m, 2H), 2.02-2.00 (m, 2H), 1.88 (s, 3H), 1.41 (s, 9H).

Step C Compound (795.2)

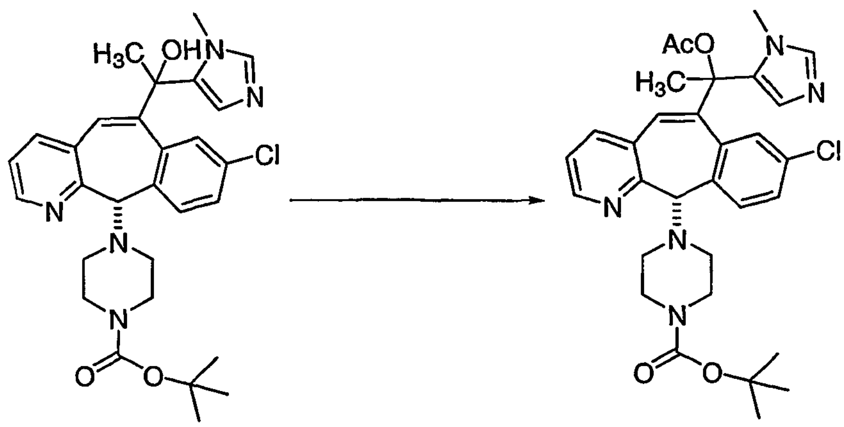

795.1 795.2

Compound (795.1) can be converted to acetate compound (795.2) by reacting it with 1 equivalent of acetic anhydride and 2 equivalents of pyridine.

Step D Compound (795.3)

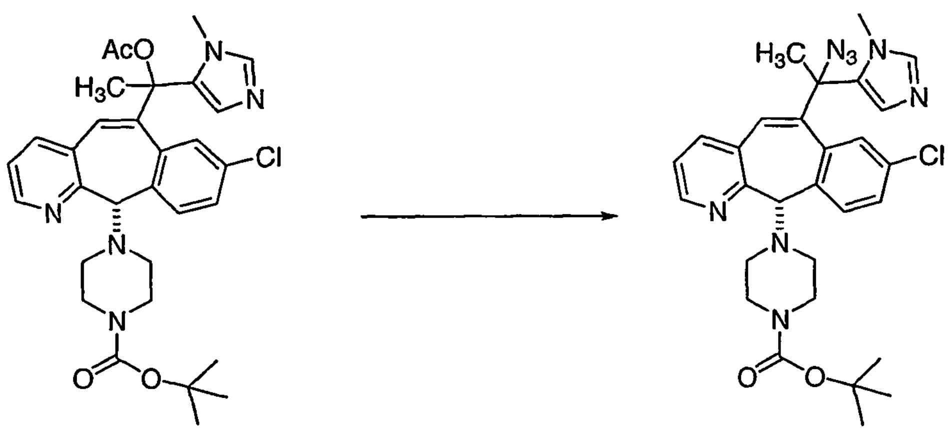

795.2 795.3

Compound (795.2) can be converted to compound (795.3) by reacting it with 1.5 equivalents of $NaN_3$, 15-crown-5, and a catalytic amount of $Pd(dba)_2/PPh_3$.

Alternatively, (795.3) can be synthesized by treating (795.1) with $NaN_3$, TFA followed by $(BOC)_2O$, and triethyl amine.

Step E Compound 795.4

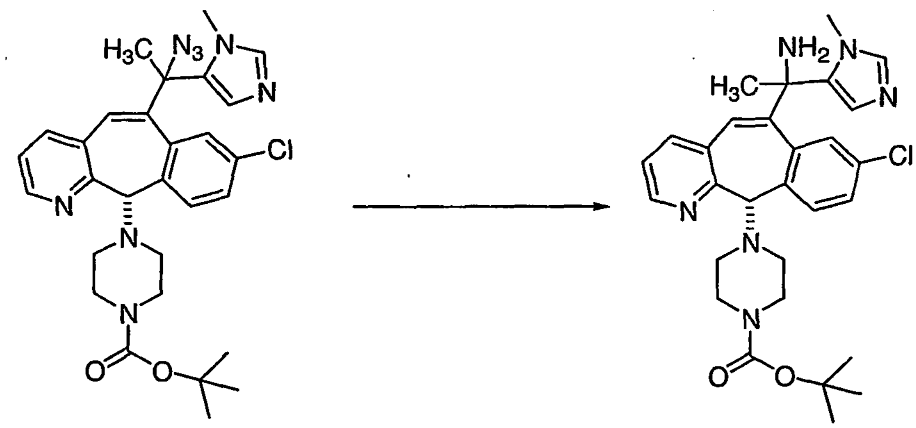

795.3 795.4

Compound (795.4) can be prepared by reacting (795.3) with $P(CH_3)_3/H_2O$.

PREPARATIVE EXAMPLE 66

Compounds (796) - (803)

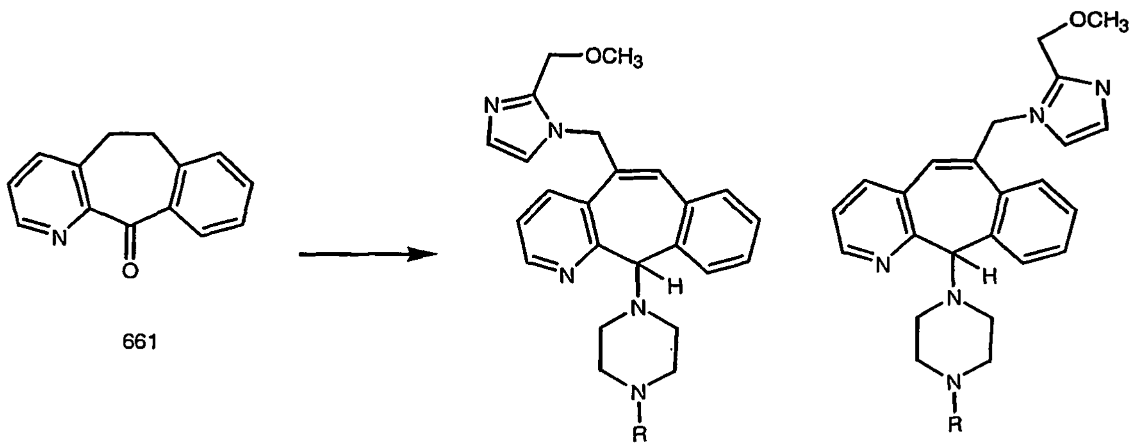

661

R = BOC
(796) (+ enantiomer, A)
(798) (- enantiomer, B)

R = BOC
(797) (+ enantiomer, A)
(799) (- enantiomer, B)

R = H
(800) (+ enantiomer, A)
(802) (- enantiomer, B)

R = H
(801) (+ enantiomer, A)
(803) (- enantiomer, B)

Compound 661 was reacted in essentially the same manner as in Preparative Example 23 and then Example 91 to obtain the N-BOC derivatives (796), (797), (798), and (799). Compounds (796), (797), (798), and (799) were then further reacted separately in essentially the same manner as in PREPARATIVE EXAMPLE 19, Step D to obtain the enantiomers (800), (801) (+ enantiomers, isomer A) and (802), (803) (-enantiomers, isomer B). The C5 and C-6 vinyl bromide intermediates were separated by silica gel chromatography using hexane:ethyl acetate (80:20) as described in PREPARATIVE EXAMPLE 23, Step B.

EXAMPLE 490-491

PREPARATION OF COMPOUNDS (804) AND (805)

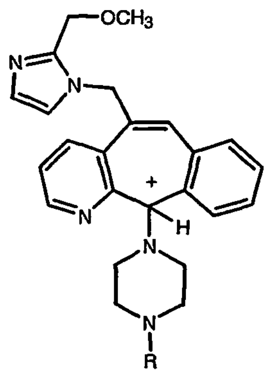

or

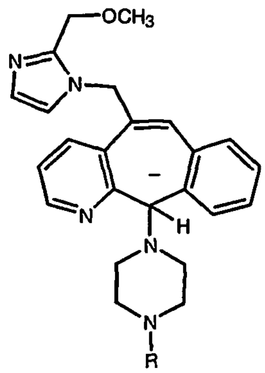

The appropriate (+) enantiomer (800) or (-) enantiomer (802) from Preparative Example 66 above, was taken up in $CH_2Cl_2$ treated with the corresponding isocyanate and stirred at room temperature over night. The crude product was purified directly by silica gel preparative thin layer chromatography or silica gel column chromatography to afford the following compounds in the table below:

| Example # | R | Enantiomer | Comp # | Phys. Data. |
|---|---|---|---|---|
| 490 | 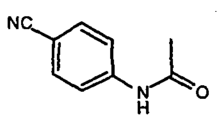 | + | (804) | Mp = 160-165°C<br>$[\alpha]_D^{25}$ = +84° (0.84 mg/ 1 mL MeOH) MH+ = 546 |
| 491 | 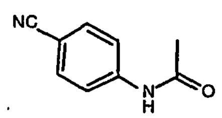 | - | (805) | Mp = 158-163°C<br>$[\alpha]_D^{25}$ = -91.6° (0.84 mg/ 1 mL MeOH) MH+ = 546 |

PREPARATIVE EXAMPLE 67

Step A COMPOUND (807)

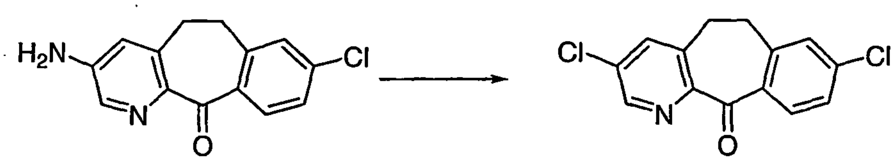

(564) (807)

15.4 g (115 mmole) of $CuCl_2$ and 17 mL (144 mmol) of t-butyl nitrite was added to 400 mL of dry $CH_3CN$. The reaction mixture was cooled to 0° C and 25 g of ketone (564) was added. The reaction was warmed to room temperature and stirred for two days. The mixture was concentrated under vacuum. Then 1N HCl was added to the residue until the pH was neutral, then $NH_4OH$ was added until the pH was basic. After extraction with ethyl acetate, the organic layer was dried over $MgSO_4$ and concentrated under vacuum to give compound (807). Alternatively, the corresponding alcohol

of 564 can be reacted as above followed by oxidation with $MnO_2$ in $CH_2Cl_2$ to give compound (807).

Step B COMPOUNDS (808) - (815)

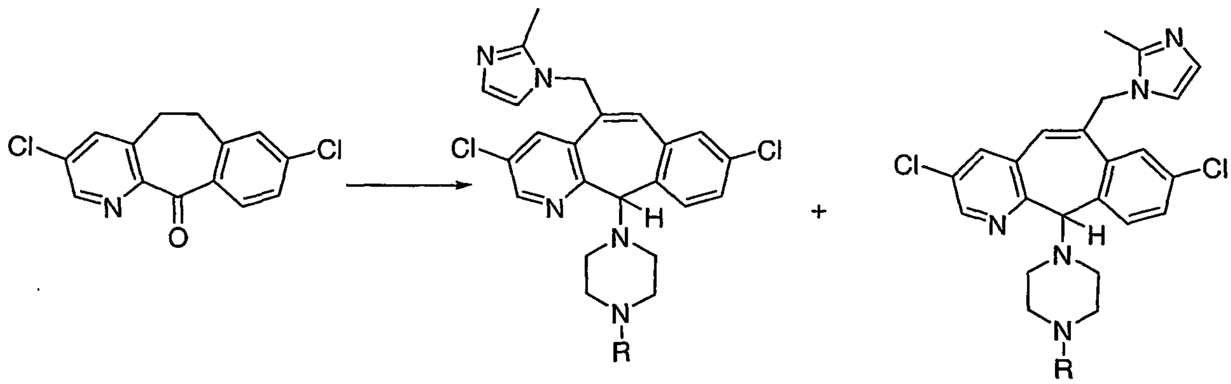

(807)

R = BOC

(808) (enantiomer 1)

(810) (enantiomer 2)

R = H

(812) (enantiomer 1)

(814) (enantiomer 2)

R = BOC

(809) (enantiomer 1)

(811) (enantiomer 2)

R = H

(813) (enantiomer 1)

(815) (enantiomer 2)

Compound (807) from step B above was reacted in essentially the same manner as in Preparative Example 23, and then Example 91 to obtain the N-BOC derivatives (808), (809), (810) and (811). These were then reacted separately in essentially the same manner as in Preparative Example 19, Step D to obtain the enantiomers (812) and (814), as well as enantiomers (813) and (815). The C5 and C-6 vinyl bromide intermediates were separated by silica gel chromatography using hexane:ethyl acetate as described in Preparative Example 23, Step B.

EXAMPLE 493

PREPARATION OF COMPOUNDS (816) AND (817)

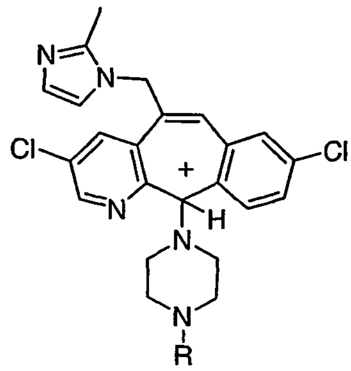

or

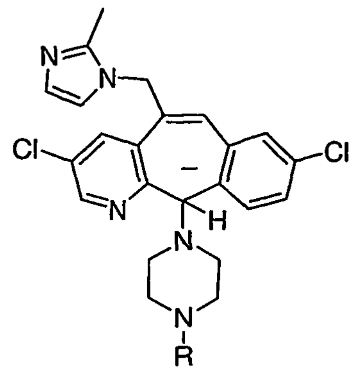

The appropriate enantiomer (812) (enantiomer 1) or (814) (enantiomer 2) from Preparative Example 67, Step B above, was taken up in $CH_2Cl_2$, treated with 4-cyanophenyl isocyanate and stirred at room temperature over night. The crude product was purified directly by silica gel preparative thin layer chromatography or silica gel column chromatography to afford the following compounds in the table below:

| Starting Cmp. # | R | Enantiomer | Comp # | Phys. Data. |
|---|---|---|---|---|
| (812) | 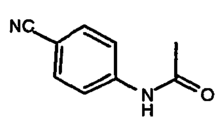 | + | 816 | Mp=175-181°C<br>$[\alpha]_D^{25}$ = +94.2° (1 mg/ 1 mL MeOH) |
| (814) | 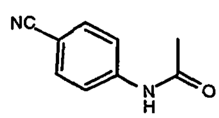 | - | (817) | Mp = 182 - 186°C<br>$[\alpha]_D^{25}$ = - 120.3° (1 mg/ 1 mL MeOH) |

EXAMPLE 494

PREPARATION OF COMPOUNDS (818) AND (819)

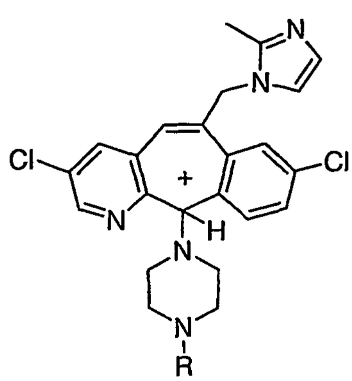

or

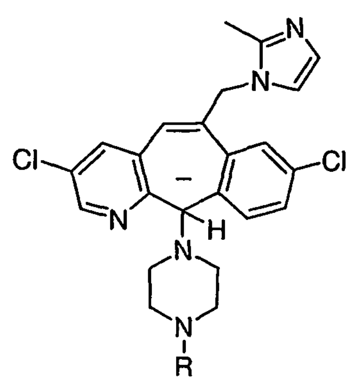

The appropriate enantiomer (813) (enantiomer 1) or (815) (enantiomer 2) from Preparative Example 67, Step B above, was taken up in $CH_2Cl_2$, treated with 4-cyanophenyl isocyanate and stirred at room temperature over night. The crude product was purified directly by silica gel preparative thin layer chromatography or silica gel column chromatography to afford the following compounds in the table below:

| Starting Cmp # | R | Enantiomer | Cmp # | Phys. Data. |
|---|---|---|---|---|
| (813) | 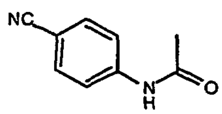 | + | (818) | Mp = 176 - 181°C<br>$[\alpha]_D^{25}$ = +46.3° (0.79 mg/ 1 mL MeOH)<br>MH+ = 584 |
| (815) | 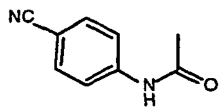 | - | (819) | Mp=174-180°C<br>$[\alpha]_D^{25}$ = -43.3° (0.94 mg/ 1 mL MeOH)<br>MH+ = 584 |

PREPARATIVE EXAMPLE 68

COMPOUNDS (820) - (827)

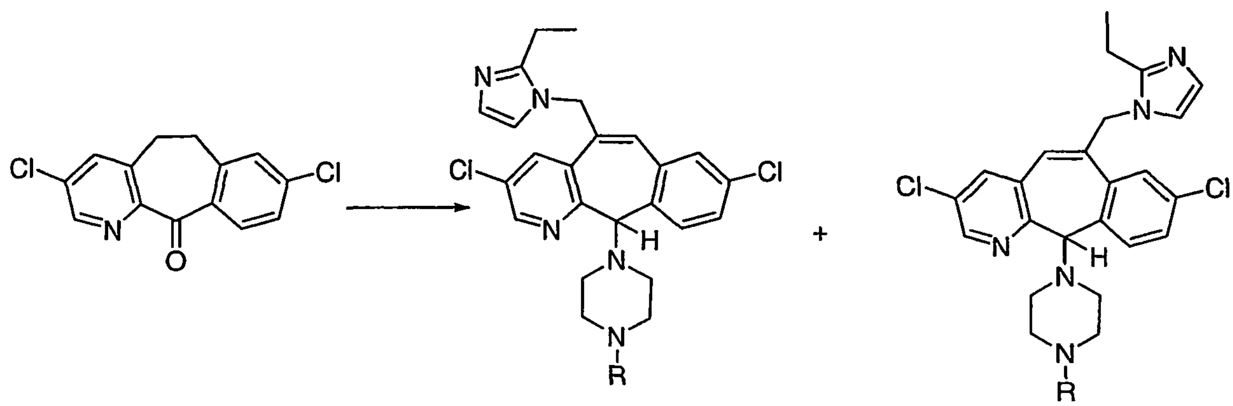


(807)

R = BOC
(820) (enantiomer 1)
(822) (enantiomer 2)

R = BOC
(821) (enantiomer 1)
(823) (enantiomer 2)

R = H
(824) (enantiomer 1)
(826) (enantiomer 2)

R = H
(825) (enantiomer 1)
(827) (enantiomer 2)

Compound (807) from Preparative Example 67, Step A above was reacted in essentially the same manner as in Preparative Example 23, and then Example 91, substituting 2-ethylimidazole for 2-methylimidazole, to obtain the N-BOC derivatives (820), (821), (822) and (823). These were then reacted seperately in essentially the same manner as in Preparative Example 19, Step D to obtain the enantiomers (824) and (826), as well as enantiomers (825) and (827). The C5 and C-6 vinyl bromide intermediates were separated by silica gel chromatography using hexane:ethyl acetate as described in Preparative Example 23, Step B.

EXAMPLE 495

PREPARATION OF COMPOUNDS (828) AND (829)

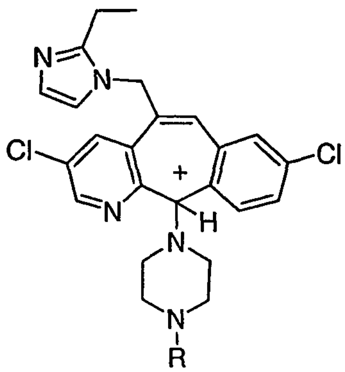

or

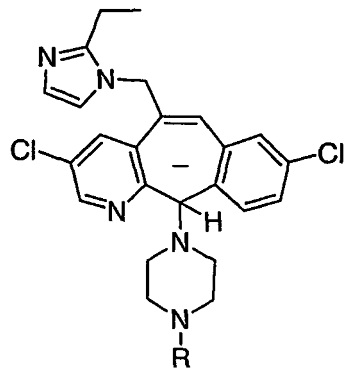

The appropriate enantiomer (824) (enantiomer 1) or (826) (enantiomer 2) from Preparative Example 68 above, was taken up in $CH_2Cl_2$, treated with 4-cyanophenyl isocyanate and stirred at room temperature over night. The crude product was purified directly by silica gel preparative thin layer chromatography or silica gel column chromatography to afford the following compounds in the table below:

| Starting Cmp # | R | Enantiomer | Comp # | Phys. Data. |
|---|---|---|---|---|
| (824) | 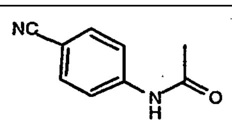 | + | (828) | Mp = 176 - 182°C<br>$[\alpha]_D^{25}$ = +84.5° (1.3 mg/1 mL MeOH)<br>MH+ = 598 |
| (826) | 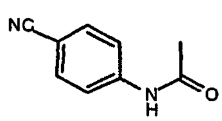 | - | (829) | Mp = 175 - 182°C<br>$[\alpha]_D^{25}$ = -88.8° (1.14 mg/ 1 mL MeOH)<br>MH+ = 598 |

EXAMPLE 496

PREPARATION OF COMPOUNDS (830) AND (831)

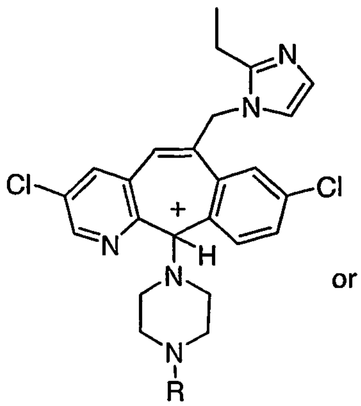

or

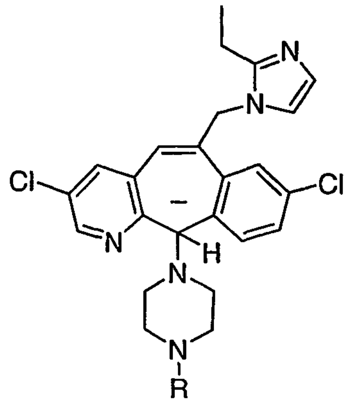

The appropriate enantiomer (825) (enantiomer 1) or (827) (enantiomer 2) from Preparative Example 68 above, was taken up in $CH_2Cl_2$, treated with 4-cyanophenyl isocyanate and stirred at room temperature over night. The crude product was purified directly by silica gel preparative thin layer chromatography or silica gel column chromatography to afford the following compounds in the table below:

| Starting Cmp # | R | Enantiomer | Comp # | Phys. Data. |
|---|---|---|---|---|
| (825) | 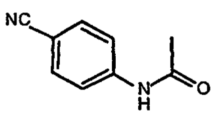 | + | (830) | Mp = 170 - 174°C<br>$[\alpha]_D^{25}$ = +39.1° (0.81 mg/ 1 mL MeOH)<br>MH+ = 598 |
| (827) | 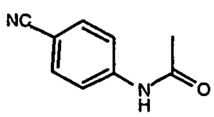 | - | (831) | Mp = 170 - 175°C<br>$[\alpha]_D^{25}$ = -36.4° (0.96 mg/ 1 mL MeOH)<br>MH+ = 598 |

PREPARATIVE EXAMPLE 69

COMPOUNDS (832) - (835)

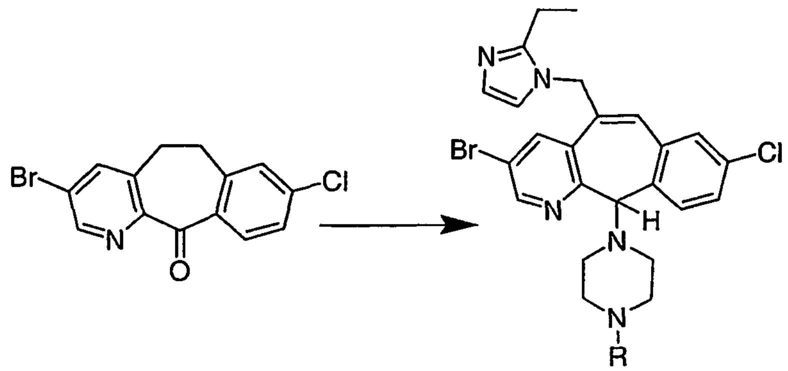

R = BOC
(832) (enantiomer, A)
(833) (enantiomer, B)

R = H
(834) (enantiomer, A)
(835) (enantiomer, B)

3-Bromo-8-chloroazaketone (U.S. Patent 5,977,128, Preparative Example 11, Step A, (1999)) was reacted in essentially the same manner as in Preparative Example 23, and then Example 91, substituting 2-ethylimidazole for 2-methylimidazole, to obtain the N-BOC derivatives (832) and (833). These were then reacted separately in essentially the same manner as in Preparative Example 19, Step D to obtain the enantiomers (834) and (835).

EXAMPLE 497

PREPARATION OF COMPOUNDS (836) AND (837)

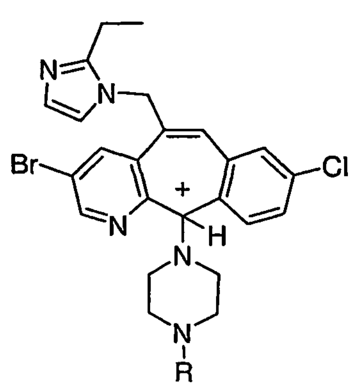

or

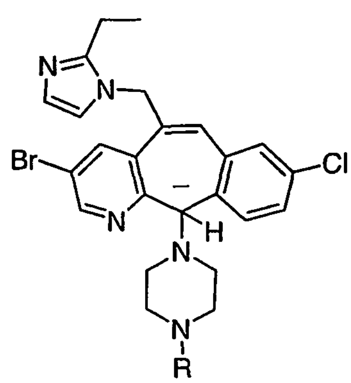

The appropriate enantiomer (834) (enantiomer 1) or (835) (enantiomer 2) from Preparative Example 69 above, was taken up in $CH_2Cl_2$, treated with 4-cyanophenyl isocyanate and stirred at room temperature over night. The crude product was purified directly by silica gel preparative thin layer chromatography or silica gel column chromatography to afford the following compounds in the table below:

| Starting Cmp # | R | Enantiomer | Comp # | Phys. Data. |
|---|---|---|---|---|
| (834) | 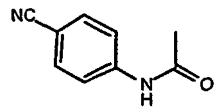 | A | (836) | Mp = 172-179°C (d)<br>MH+ = 643 |
| (835) | 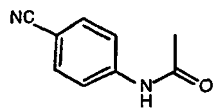 | B | (837) | Mp = 171.9 -178.3°C<br>MH+ = 643 |

PREPARATIVE EXAMPLE 70

COMPOUNDS (838) - (841)

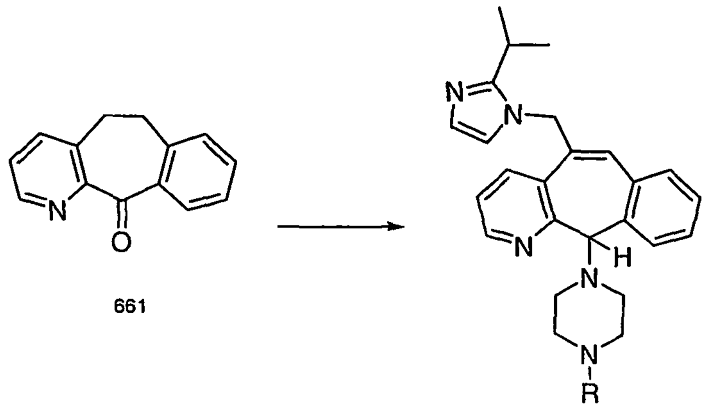

661

R = BOC

(838)

(839)

R = H

(840)

(841)

Compound 661 was reacted in essentially the same manner as in Preparative Example 23, and then Example 91, substituting 2-isopropylimidazole for 2-methylimidazole, to obtain the N-BOC derivatives (838) and (839). These were then reacted separately in essentially the same manner as in Preparative Example 19, Step D to obtain the enantiomers (840) and (841).

EXAMPLE 498

PREPARATION OF COMPOUNDS (842) AND (843)

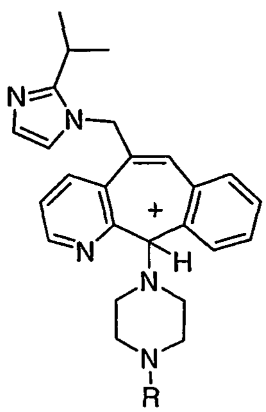

or

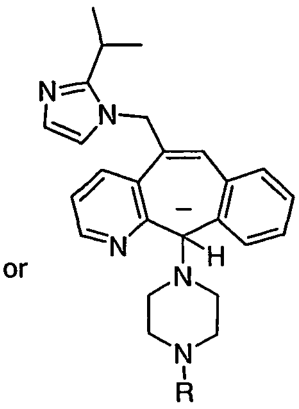

The appropriate enantiomer (840) (enantiomer 1) or (841) (enantiomer 2) from Preparative Example 70 above, was taken up in $CH_2Cl_2$, treated with 4-cyanophenyl isocyanate and stirred at room temperature over night. The crude product was purified directly by silica gel preparative thin layer chromatography or silica gel column chromatography to afford the following compounds in the table below:

| Starting Cmp # | R | Enantiomer | Comp # | Phys. Data. |
|---|---|---|---|---|
| (840) | 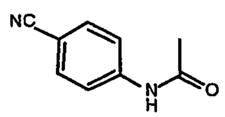 | A | (842) | Mp = 168 - 170°C (d)<br>$[\alpha]_D^{25}$ = +64.1° (0.66 mg/ 1 mL MeOH) |
| (841) | 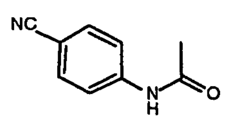 | B | (843) | Mp = 166-171°C<br>$[\alpha]_D^{25}$ = -80.9° (0.85 mg/ 1 mL MeOH) |

PREPARATIVE EXAMPLE 71

COMPOUNDS (844) - (847)

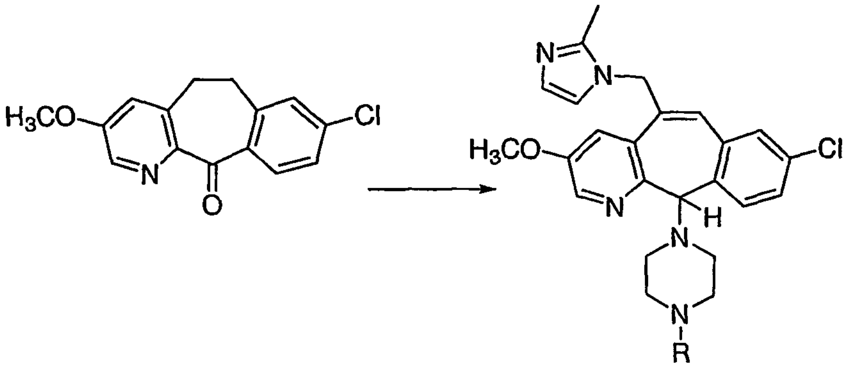

R = BOC
(844)
(845)

R = H
(846) (enantiomer A)
(847) (enantiomer B)

3-Methoxy-8-chloroazaketone (U.S. patent 5,977,128 (1999), Example 2, step D) was reacted in the same manner as in Preparative Example 23, and Example 91 to obtain the N-BOC derivatives (844) and (845). These compoounds were then reacted seperately in essentially the same manner as in Preparative Example 19, Step D to obtain the enantiomers (846) (A) and (847) (B).

EXAMPLE 499

PREPARATION OF COMPOUNDS (848) AND (849)

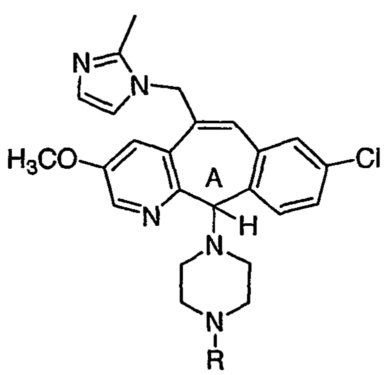

or

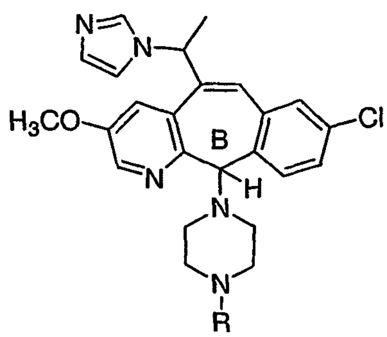

The appropriate enantiomer (846) (enantiomer A) or (847) (enantiomer B) from Preparative Example 71 above, was taken up in $CH_2Cl_2$, treated with 4-cyanophenyl isocyanate and stirred at room temperature over night. The crude product was purified directly by silica gel preparative thin layer chromatography or silica gel column chromatography to afford the following compounds in the table below:

| Starting Cmp # | R | Enantiomer | Comp # | Phys. Data. |
|---|---|---|---|---|
| (846) | 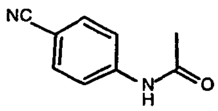 | A | (848) | Mp = 174.2 - 189.3°C (d) MH+ = 580 |
| (847) | 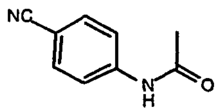 | B | (849) | Mp = 174.4 - 189.8°C MH+ = 580 |

EXAMPLE 500

PREPARATION OF COMPOUND (850)

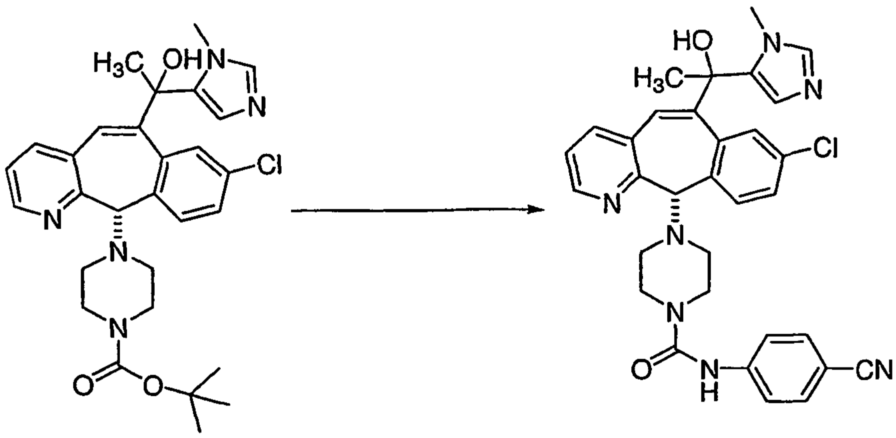

795.1

850

Compound (850) can be prepared by following essentially the same procedure as described in Example 482.

EXAMPLE 501

PREPARATION OF COMPOUND (851)

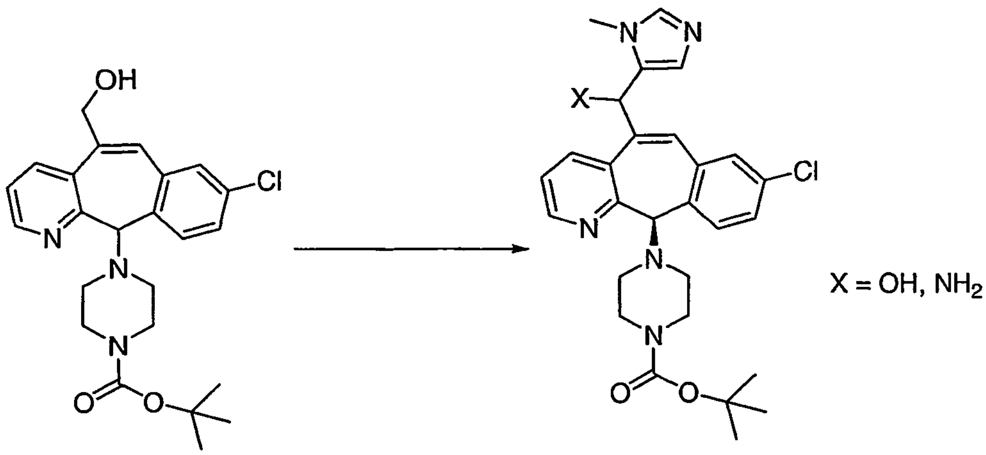


240 851

Starting with compound (240) from Preparative Example 23, Step H, compound (851) can be prepared following essentially the same procedure as described in Preparative Example 65, Steps A and B.

EXAMPLE 502

PREPARATION OF COMPOUND (852)

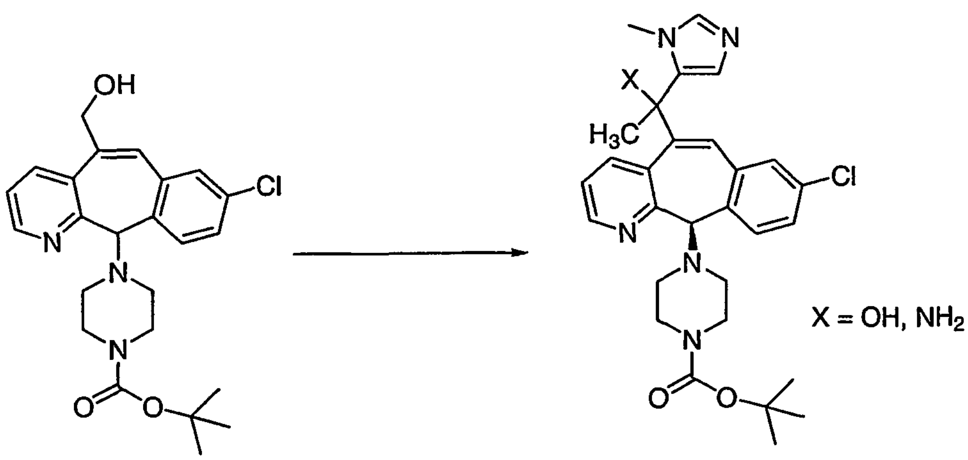


240 852

Starting with compound (240) from Preparative Example 23, Step H, compound (852) can be prepared following essentially the same procedures as described in Preparative Example 65, Step A and Example 489, Steps A-E.

PREPARATIVE EXAMPLE 72

Step A. Preparation of Compounds (853) and (854)

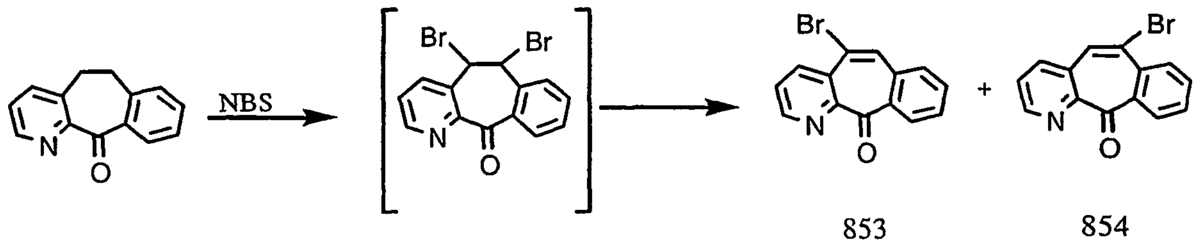


853 854

The starting tricyclic keto compound (disclosed in US Pat. No. 5,151,423) (56.5 g; 270 mmol) was combined with NBS (105 g; 590 mmol) and benzoyl peroxide (0.92 g) in $CCl_4$. The reaction was heated at 80 ˚C for 5 hr. The mixture was cooled and the resulting precipitate was filtered and treated with DBU (25.59 ml) in THF (300mL). The resulting solution was stirred at room temperature for 24 hrs, then evaporated, followed by extraction with $CH_2Cl_2$ -$H_2O$. The organic layer was dried over $MgSO_4$, filtered and evaporated to dryness to give a mixture of two compounds which were separated on a flash silica gel column eluting with Hexane-50% EtOAc to give the title compound (853) $\delta_H$ ($CDCl_3$) 8.8 (dd, 1H), 8.45 (dd, 1H), 7.99 (m, 1H), 7.92 (s, 1H), 7.59-7.64 (m, 3H), 7.23 (dd, 1H) and (854) $\delta_H$ ($CDCl_3$) 8.19 (dd, 1H), 7.99 (dd, 1H), 7.82 (dd, 1H), 7.25 -7.65(m, 4H), 7.22 (s, 1H)

Step B Preparation of Compound (855)

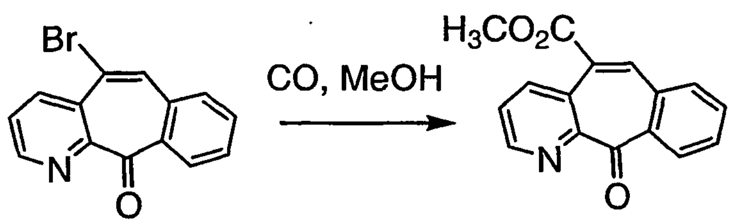


853 855

Compound (853) (25 g), triphenyl phosphine (13.75 g), and palladium chloride (1.5 g) were combine in MeOH (30 ml) and toluene (200 ml). To the mixture was added DBU (18 ml) and the mixture was sealed in a parr bomb. The mixture was stirred and subjected to 100 psi of CO at 80 ˚C for 5 hr. The reaction was diluted with EtOAc and washed with water. The organic layer was dried over $MgSO_4$, filtered and purified by flash chromatography eluting with $CH_2Cl_2$-10% EtOAc to give the title compound (855) . $\delta_H$ ($CDCl_3$) 8.8 (dd, 1H), 8.40 (dd, 1H), 8.2 (s 1H), 8.04 (dd, 1H), 7.59-7.64 (m, 4H), 3.95 (s, 3H).

Step C Preparation of Compound (856)

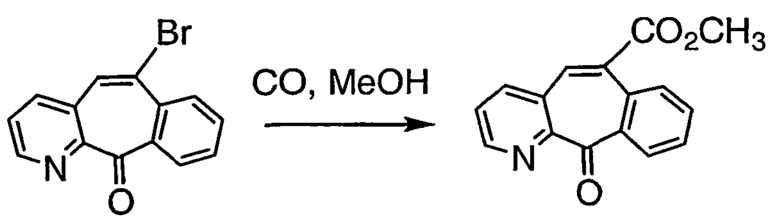


854 856

Reacting compound (854) in essentially the same manner as described in Step B above, gave the title compound (856). $\delta_H$ ($CDCl_3$) 8.85 (dd, 1H), 7.85-8.0 (m, 2H), 7.8 (s, 1H), 7.25 - 7.31 (m, 4H)

Step D Preparation of Compound (857)

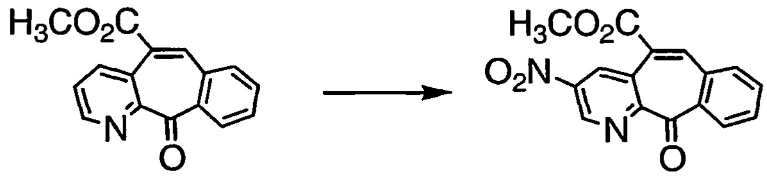

855 857

Compound (855) (19.5 g, 73.5 m mol) was dissolved in $CH_2Cl_2$ (100 mL) and cooled to 0 °C. Tetrabutyl ammonium nitrate (31.36 g, 103 n mol) and trifluoro acetic anhydride (18.52 g, 88 m mol) were added and the mixture stirred at room temperature for 5 hrs. The reaction mixture was concentrated to dryness, followed by extraction with $CH_2Cl_2$ -$NaHCO_3$. The combine organic layer was dried over $MgSO_4$ and concentrated to dryness and the residue was chromatographed on silica gel using $CH_2Cl_2$ -EtOAc (25%) to give the title compound (857) (12.4 g), $\delta_H$ ($CDCl_3$) 9.45 (dd, 1H), 9.05 (dd, 1H), 8.28 (s 1H), 8.0 (dd, 1H), 7.65 (m, 3H), 3.98 (s, 3H).

Step E Preparation of Compound (858)

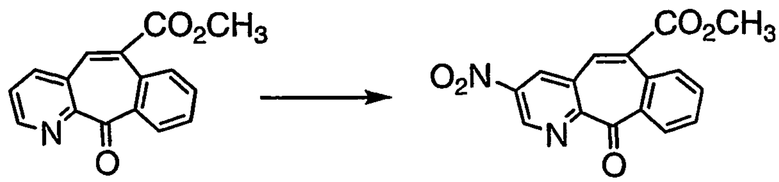

856 858

Reacting compound (856) in essentially the same manner as described in Step D above, gave the title compound (858). $MH^+$ = 311

Step F Preparation of Compound (859)

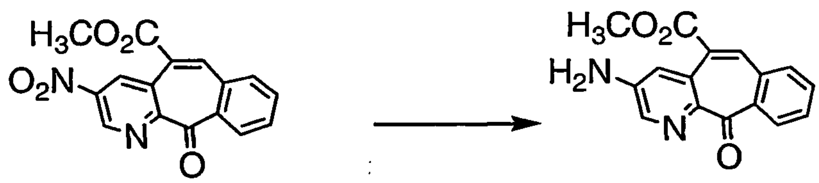

857 859

Compound (857) (6 g,) was balloon hydrogenated in MeOH (100 mL) over Raney-Ni (4.2 g) at room temperature overnight. The catalyst was filtered off and the filtrate was evaporated to dryness to give the title compound (859) (4.66 g) $MH^+$ =281

Step G. Preparation of Compound (860)

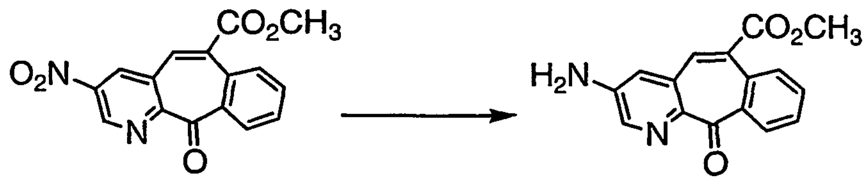

858 860

Reacting compound (858) in essentially the same manner as described in Step F above, gave the title compound (860) $MH^+$ = 281.

Step H Preparation of Compound (861)

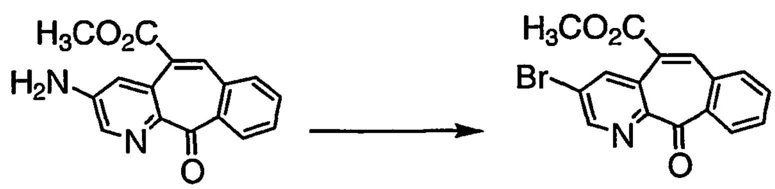

859 861

To a suspension of compound (859) (2.1 g) in 48% HBr, was added sodium nitrite (1.55 g) followed by bromine (2.11 mL) at 0 °C. The mixture was stirred at room temperature overnight. Concentrated $NH_4OH$ was then added dropwise until basic pH (to litmus paper). The reaction was extracted with $CH_2Cl_2$, washed with brine, dried over $MgSO_4$. filtered and the solvent evaporated to give the title compound (861) (1.75 g) $MH^+$ = 345.

Step I. Preparation of Compound (862)

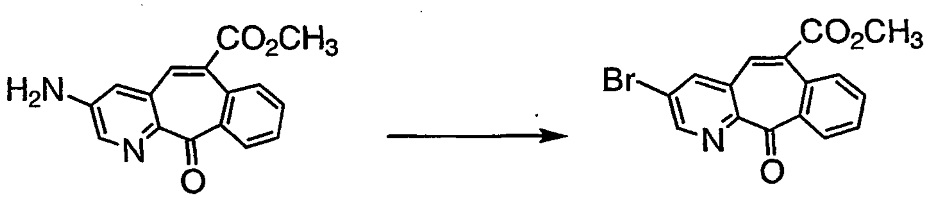

860 862

Reacting compound (861) in essentially the same manner as described in Step H above, gave the title compound (862) $MH^+$ = 345.

Step J Preparation of Compound (863)

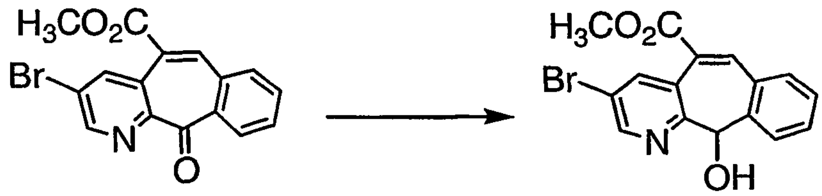

861 863

To a stirred solution of compound (861) (1.6 g, 4.64 mmole) in MeOH (30 mL) under nitrogen at 0 °C.was added $NaBH_4$ (0.3 g, 7.9 mmole). The resulting solution was stirred at room temperature for 24 hrs, then evaporated, followed by extraction with $CH_2Cl_2$-$H_2O$. The organic layer was dried over $MgSO_4$, filtered and evaporated to dryness to give the title compound (863) (1.58 g) $MH^+$= 347 .

<u>Step K</u> <u>Preparation of Compound (864)</u>

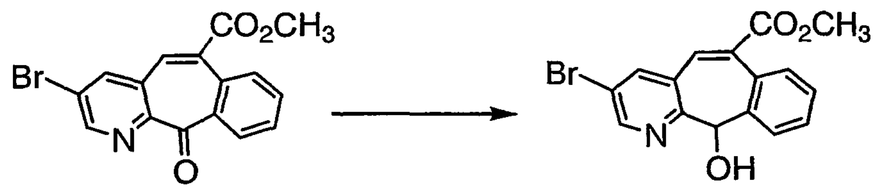

862 864

Reacting compound (862) in essentially the same manner as described in Step J above, gave the title compound (864). $MH^+$ = 347

<u>Step L</u> <u>Preparation of Compound (865)</u>

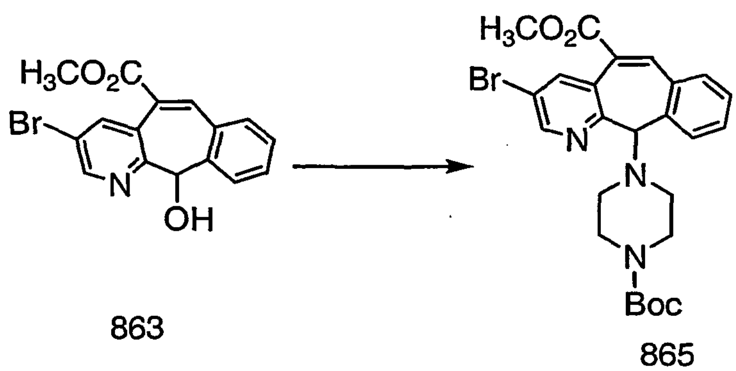

863 865

Compound 863 (1.57 g,) was stirred in thionyl chloride (10 mL) at room temperature for 4 hrs then evaporated to dryness. The resulting crude oil as taken up in acetonitrile (50 mL) and refluxed with N-Boc-piparazine (1.41 g) and triethyl amine (3.91 g) overnight. The mixture was evaporated to dryness , followed by extraction with $CH_2Cl_2$ -$NaHCO_3$. The organic layer was dried over $MgSO_4$, filtered and evaporated to dryness to give a brown gum which was purified

by column chromatography on silica gel, eluting with Hexane -20% EtOAc to give the title compound (865) (0.69g); $MH^+$ = 515.

Step M Preparation of Compound (866)

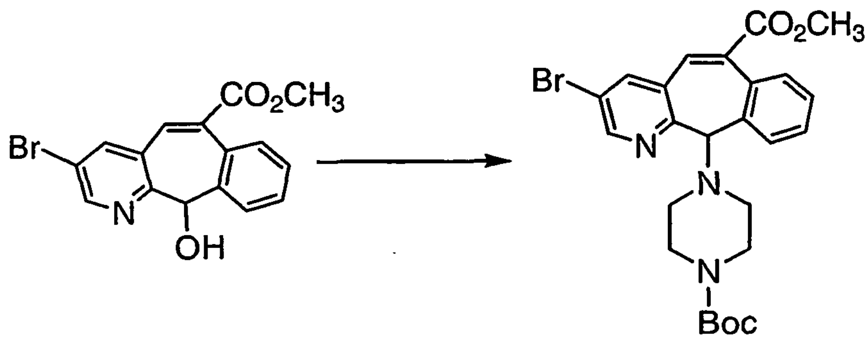

864 866

Reacting compound (864) in essentially the same manner as described in Step L above, gave the title compound (866) $MH^+$ = 515.

Step N Preparation of Compound (867)

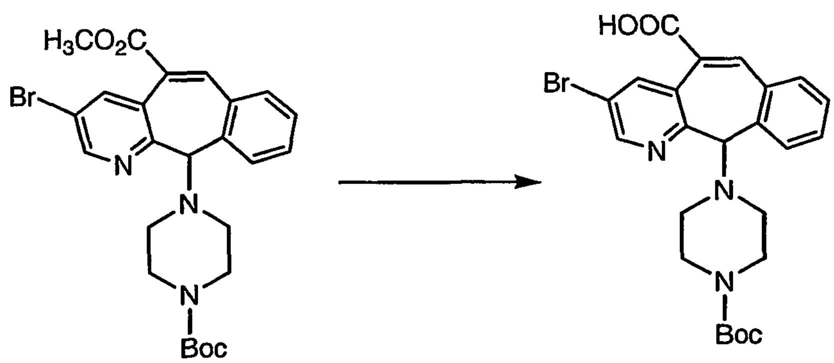

865 867

Compound (865) (0.65 g, 1.26 mmole) was refluxed with LiOH (0.45 g, , 18.79 mmole) in MeOH (15 mL) and water (1mL) for 2 hrs. 10% aq. Citric acid was added until pH = 3.5, followed by extraction with $CH_2Cl_2$ -brine . The organic layer was dried over $MgSO_4$, filtered and evaporated to dryness to give a white solid (867) (0.60 g)) $MH^+$ = 501

Step O Preparation of Compound (868)

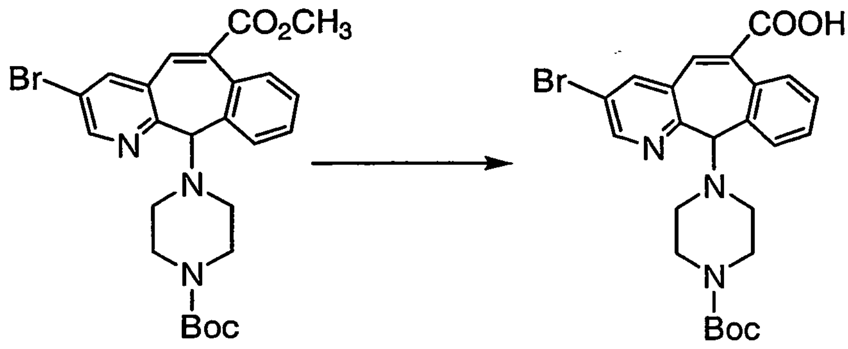

866 868

Reacting compound (866) in essentially the same manner as described in Step N above, gave the title compound (868). $MH^+$ = 501

Step P Preparation of Compound (869)

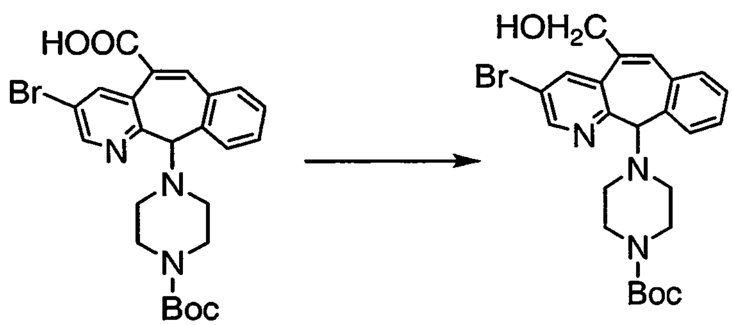

867 869

Compound (867) (0.60 g, 1.21 mmole) was stirred with carbonyl diimidazole (0.59 g, 3.63 mmole) in THF (15 mL) at at 40 ˚C overnight. The reaction mixture was cooled in an ice-bath then added $NaBH_4$ (0.28 g, 7.31 mmole) and stirred at room temperature overnight. The mixture was evaporated to dryness, followed by extraction with $CH_2Cl_2$ -water. The organic layer was dried over $MgSO_4$, filtered and evaporated to give a brown gum which was purified by column chromatography on silica gel, eluting with Hexane -50% EtOAc to give the title compound (869)(0.493g) $MH^+$ = 487.

Step Q Preparation of Compound (870)

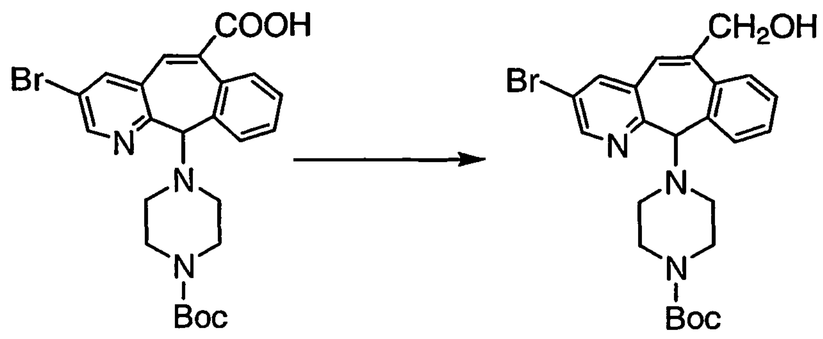

868 870

Reacting compound (868) in essentially the same manner as described in Step P above, gave the title compound (870). $MH^+ = 487$

Step R Preparation of Compound (871)

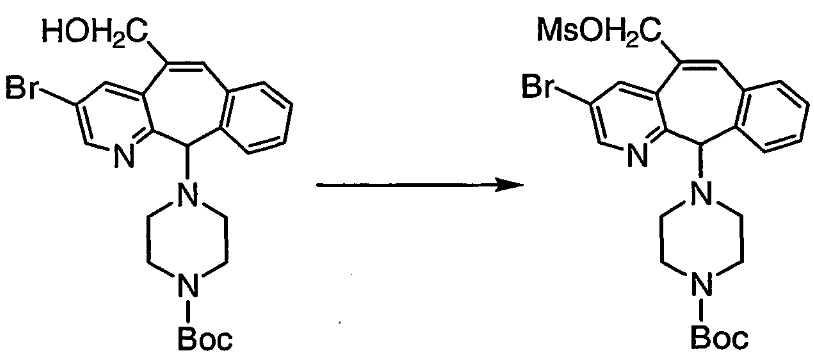

869 871

Compound (869) (0.0.38 g, 0.78 mmole) was stirred with methanesulfonylchloride (0.33 g, 1.296 mmole) and triethylamine (0.68 g, 6.72 mmole) in THF (10 mL) at room temperature overnight. The mixture was evaporated to dryness, followed by extraction with $CH_2Cl_2$ -water. The organic layer was dried over $MgSO_4$, filtered and evaporated to dryness to give the title compound (871)(0.369g). $MH^+ = 565$

Step S Preparation of Compound (872)

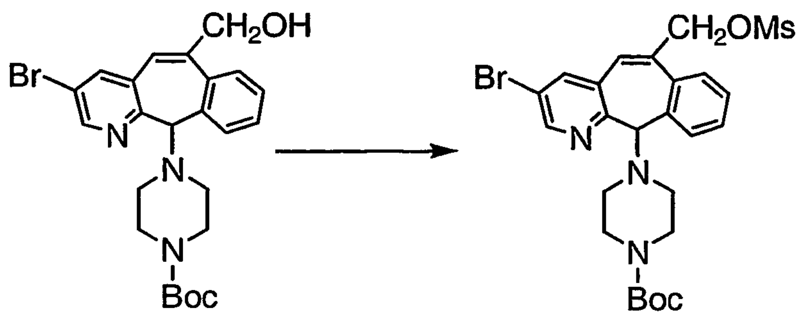

870 872

Reacting compound (870) in essentially the same manner as described in Step R above, gave the title compound (872). $MH^+$ = 565

Step T Preparation of Compounds (873) and (874)

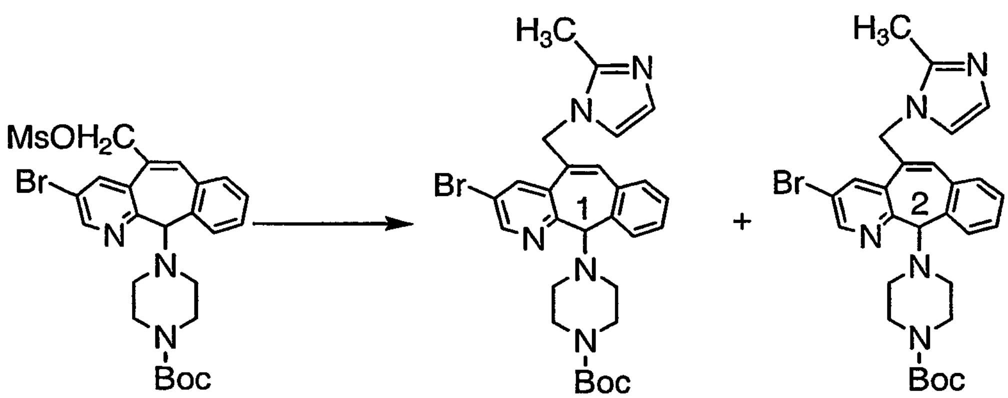

871 873 874

Compound (871) (0.0.369 g, 0.653 mmole) was stirred with 2-methylimidazole (0.188 g, 2.28 mmole) in DMF (5 mL) at room temperature overnight. The mixture was evaporated to dryness, followed by extraction with $CH_2Cl_2$ -water. The organic layer was dried over $MgSO_4$, filtered, evaporated to dryness and then purified on silica-gel prep-plate chromatography, eluting with $CH_2Cl_2$- 5% (MeOH-10% $NH_4OH$) to give the product as a mixture of isomers (1.126 g) $MH^+$=551. Separation of the product mixture by HPLC using a prep AD column, eluting with 20 % IPA/80%hexane/ 0.2%DEA (isocratic 60ml/min.) afforded pure isomer 1 (873) (0.06 g, $MH^+$= 551 and isomer 2 (874) (0.0061 g) $MH^+$=551.

Step U Preparation of Compound (875) and (876)

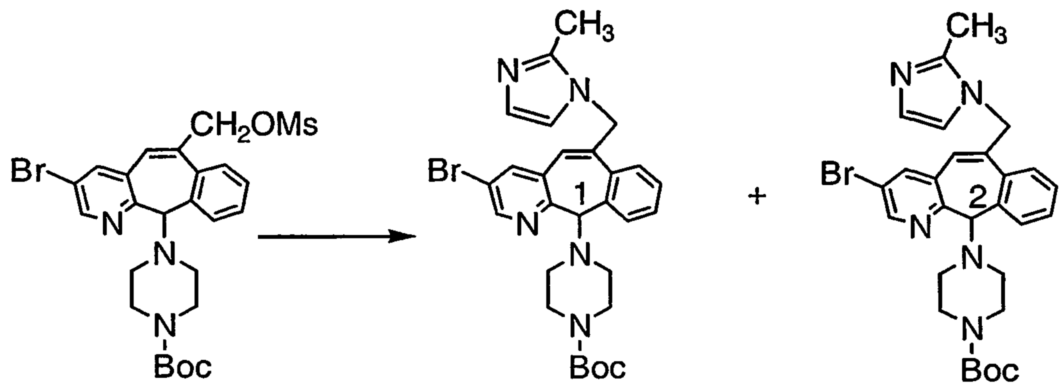

872 875 876

Reacting compound (872) in essentially the same manner as described in Step T above, gave the title compounds (875). $MH^+$ = 551, and (876) $MH^+$ = 551.

EXAMPLE 503

Compound (877)

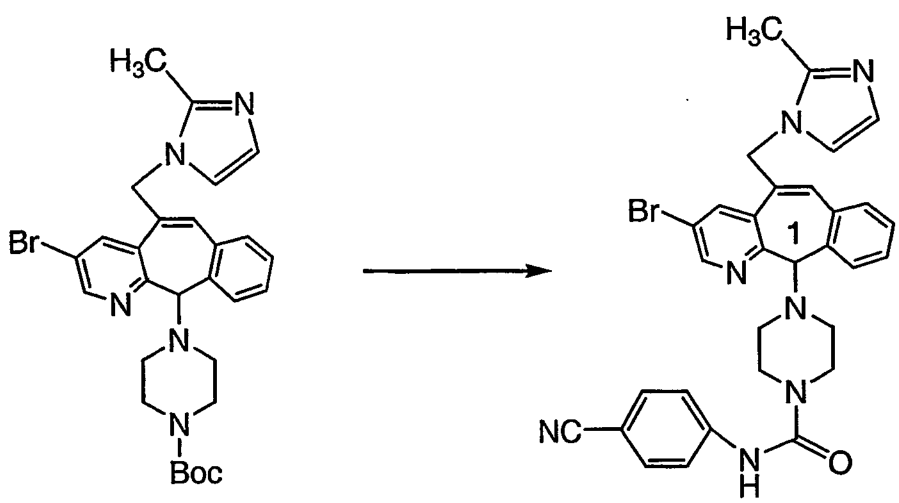

873 877

Compound (873) (0.043g, 0.078 mmole) was stirred with TFA (5 mL) in $CH_2Cl_2$ (5 mL) for 4 hrs. at room temperature. The mixture was then evaporated to dryness. To the residue was added p-cyanophenylisocyanate (0.0123 g, 0.086 mmole).and triethylamine (0.5 mL) in $CH_2Cl_2$ (5 mL) and the mixture stirred at room temperature for 2 hrs. The mixture was evaporated to dryness, followed by extraction with $CH_2Cl_2$ - brine. The organic layer was dried over $MgSO_4$, filtered and evaporated to dryness to give a brown gum which was purified by prep-plate chromatography on silica gel, eluting with $CH_2Cl_2$- 5% (MeOH-10% $NH_4OH$) to give the title compound (877) (0.0394g) . $MH^+$ = 595, $\delta_H$ ($CDCl_3$) 8.6 (1H); 8.05 (1H); 7.22-7.5 (8H); 6.99 (1H); 6.95 (1H); 6.93 (1H); 4.99 -5.25 (2H); 4.6 (1H); 3.1 - 3.25 (4H); 2.25 (3H), 1.8 - 2.05 (4H).

EXAMPLE 504

Compound (878)

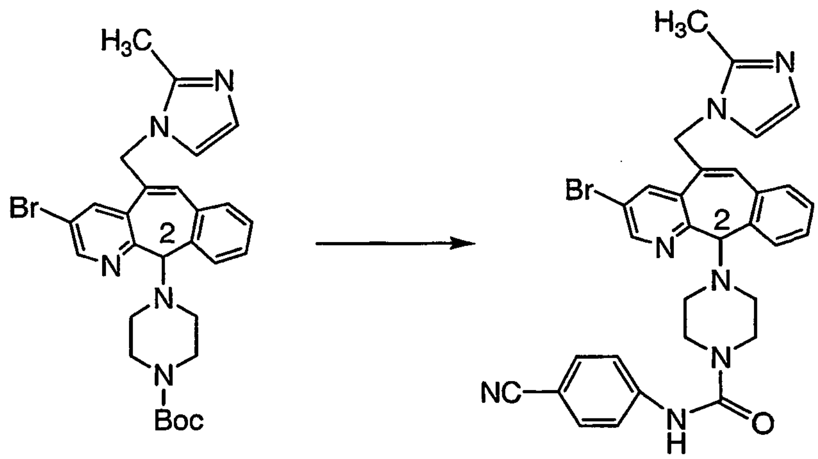

874 878

Reacting compound (874) in essentially the same manner as described in Example 503 above, gave the title compound. (878) $MH^+$ = 595, $\delta_H$ ($CDCl_3$) 8.6 (1H); 8.05 (1H); 7.22-7.5 (8H); 6.99 (1H); 6.95 (1H); 6.93 (1H); 4.99 -5.25 (2H); 4.6 (1H); 3.1 - 3.25 (4H); 2.25 (3H), 1.8 - 2.05 (4H).

EXAMPLE 505

Compound (879)

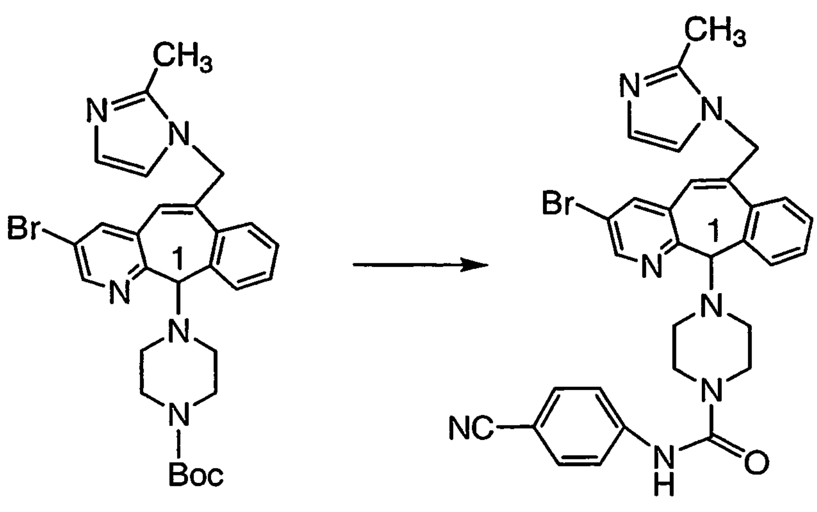

875 879

Reacting compound (875) in essentially the same manner as described in Example 503 above, gave the title compound (879). $MH^+$ = 595, $\delta_H$ ($CDCl_3$) 8.55 (1H); 7.78 (1H); 7.65 (1H);7.4 - 7.51 (6H); 6.98 (1H); 6.9 (1H); 6.85 (1H);

5.05 - 5.3 (2H); 4.6 (1H); 3.1 - 3.25 (4H); 2.5 (3H), 1.8 - 2.00 (4H).

ASSAYS

FPT activity was determined by measuring the transfer of [$^3$H] farnesyl from [$^3$H] farnesyl pyrophosphate to a biotinylated peptide derived from the C-terminus of H-ras (biotin-CVLS). The reaction mixture contains: 50 mM Tris pH7.7, 5 mM $MgCl_2$, 5 μM $Zn^{++}$, 5 mM DTT, 0.1% Triton-X, 0.05 μM peptide, 0.03 nM purified human farnesyl protein transferase, 0.180 μM [$^3$H] farnesyl pyrophosphate, plus the indicated concentration of tricyclic compound or vehicle control in a total volume of 100 μl. The reaction was incubated in a Vortemp shaking incubator at 37°C, 45 RPM for 60 minutes and stopped with 150 τl of 0.25 M EDTA containing 0.5% BSA and 1.3 mg/ml Streptavidin SPA beads. Radioactivity was measured in a Wallach 1450 Microbeta liquid scintillation counter. Percent inhibition was calculated relative to the vehicle control.

COS Cell $IC_{50}$ (Cell-Based Assay) were determined following the assay procedures described in WO 95/10516, published April 20, 1995. GGPT $IC_{50}$ (inhibition of geranylgeranyl protein transferase, in vitro enzyme assay), Cell Mat Biochemical assay and anti-tumor activity (in vivo anti-tumor studies) could be determined by the assay procedures described in WO 95/10516. The disclosure of WO 95/10516 is incorporated herein by reference thereto.

Various tumor cells (5 x $10^5$ to 8 x $10^6$) were innoculated subcutaneously into the flank of 5-6 week old athymic nu/nu female mice. Three tumor cell models were used: mouse fibroblasts transformed with H-Ras; HTB-177 human non small cell lung cancer cells or LOX human melanoma cells. Animals were treated with beta cyclodextran vehicle only or compounds in vehicle twice a day (BID) or once a day (QD) for 7 days per week for 1 (x1), 2 (x2) or 3 (x3) weeks. The percent inhibition of tumor growth relative to vehicle controls were determined by tumor measurements. The results are reported in the table below:

| Compound No. | Tumor | Dose (MPK) | Route and Schedule | Average % Tumor Inhibition |
|---|---|---|---|---|
| (372) | H-Ras fibroblasts | 40 | po, BID, x2 | 92 |
| " | H-Ras fibroblasts | 10 | po, BID, x2 | 70 |
| " | H-Ras fibroblasts | 80 | po, QD, x2 | 91 |
| " | H-Ras fibroblasts | 20 | po, QD, x2 | 55 |
| " | H-Ras fibroblasts | 60 | po, BID, x2 | 98 |
| " | H-Ras fibroblasts | 20 | po, BID, x2 | 59 |
| " | H-Ras fibroblasts | 6.6 | po, BID, x2 | 19 |
| " | HTB-177 | 60 | po, BID, x3 | 87 |
| " | HTB-177 | 20 | po, BID, x3 | 43 |
| " | HTB-177 | 120 | po, QD, x3 | 54 |
| " | HTB-177 | 40 | po, QD, x3 | 11 |
| " | HTB-177 | 80 | po, BID, x3 | 96 |
| " | HTB-177 | 40 | po, BID, x3 | 79 |
| " | HTB-177 | 20 | po, BID, x3 | 47 |
| " | LOX | 15 | po, BID, x1 | 20.9 |
| " | LOX | 30 | po, BID, x1 | 54.8 |
| " | LOX | 60 | po, BID, x1 | 90.3 |
| (The schedule "po, BID, x3", for example, means orally, twice a day for 7 days (14 times per week) for 3 weeks). | | | | |

Soft Agar Assay:

Anchorage-independent growth is a characteristic of tumorigenic cell lines. Human tumor cells can be suspended in growth medium containing 0.3% agarose and an indicated concentration of a farnesyl transferase inhibitor.

The solution can be overlayed onto growth medium solidified with 0.6% agarose containing the same concentration of farnesyl transferase inhibitor as the top layer. After the top layer is solidified, plates can be incubated for 10-16 days at 37˚C under 5% $CO_2$ to allow colony outgrowth. After incubation, the colonies can be stained by overlaying the agar with a solution of MTT (3-[4,5-dimethyl-thiazol-2-yl]-2,5-diphenyltetrazolium bromide, Thiazolyl blue) (1 mg/mL in PBS). Colonies can be counted and the $IC_{50}$'s can be determined.

Compounds of this invention have an FPT $IC_{50}$ in the range of 0.001 nM to 100 nM and a Soft Agar $IC_{50}$ in the range of 0.01 nM to 50 nM.

The preferred compounds of the invention have an FPT $IC_{50}$ range of between <0.06 nM - 0.44 nM and a Soft agar $IC_{50}$ range of between <0.05 nM - 25 nM.

The most preferred compounds have an FPT $IC_{50}$ range of between <0.05 nM - 3.0 nM and Soft agar $IC_{50}$ range of between 0.5 nM - 5 nM.

For preparing pharmaceutical compositions from the compounds described by this invention, inert, pharmaceutically acceptable carriers can be either solid or liquid. Solid form preparations include powders, tablets, dispersible granules, capsules, cachets and suppositories. The powders and tablets may be comprised of from about 5 to about 95 percent active ingredient. Suitable solid carriers are known in the art, e.g. magnesium carbonate, magnesium stearate, talc, sugar or lactose. Tablets, powders, cachets and capsules can be used as solid dosage forms suitable for oral administration. Examples of pharmaceutically acceptable carriers and methods of manufacture for various compositions may be found in A. Gennaro (ed.), Remington's Pharmaceutical Sciences, 18th Edition, (1990), Mack Publishing Co., Easton, Pennsylvania.

Liquid form preparations include solutions, suspensions and emulsions. As an example may be mentioned water or water-propylene glycol solutions for parenteral injection or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier, such as an inert compressed gas, e.g. nitrogen.

Also included are solid form preparations which are intended to be converted, shortly before use, to liquid form preparations for either oral or parenteral administration. Such liquid forms include solutions, suspensions and emulsions.

The compounds of the invention may also be deliverable transdermally. The transdermal compositions can take the form of creams, lotions, aerosols and/or emulsions and can be included in a transdermal patch of the matrix or reservoir type as are conventional in the art for this purpose.

Preferably, the pharmaceutical preparation is in a unit dosage form. In such form, the preparation is subdivided into suitably sized unit doses containing appropriate quantities of the active component, e.g., an effective amount to achieve the desired purpose.

The quantity of the compounds of the present invention in a unit dose of preparation may be varied or adjusted from about 0.01 mg to about 1000 mg, preferably from about 0.01 mg to about 750 mg, more preferably from about 0.01 mg to about 500mg, and most preferably from about 0.01 mg to about 250mg, according to the particular application.

The amount and frequency of administration of the compounds of the present invention and/or the pharmaceutically acceptable salts thereof will be regulated according to the judgment of the attending clinician considering such factors as age, condition and size of the patient as well as severity of the symptoms being treated. A typical recommended daily dosage regimen for oral administration can range from about 0.04 mg/day to about 4000 mg/day, in single or divided doses, preferably, in two to four divided doses.

The chemotherapeutic agent and/or radiation therapy can be administered in association with the compounds of the present invention according to the dosage and administration schedule listed in the product information sheet of the approved agents, in the Physicians Desk Reference (PDR) as well as therapeutic protocols well known in the art. Table 1.0 below gives ranges of dosage and dosage regimens of some exemplary chemotherapeutic agents useful in the methods of the present invention. It will be apparent to those skilled in the art that the administration of the chemotherapeutic agent and/or radiation therapy can be varied depending on the disease being treated and the known effects of the chemotherapeutic agent and/or radiation therapy on that disease. Also, in accordance with the knowledge of the skilled clinician, the therapeutic protocols (e.g., dosage amounts and times of administration) can be varied in view of the observed effects of the administered chemotherapeutic agents (i.e., antineoplastic agent or radiation) on the patient, and in view of the observed responses of the disease to the administered therapeutic agents.

In a preferred example of combination therapy in the treatment of pancreatic cancer, the compound of Formula (I) is administered orally in a range of from 50 to 400 mg/day, in two divided doses, in association with the antineoplastic agent, gemcitabine, which is administered at a dosage of from 750 to 1350 $mg/m^2$ weekly for three out of four weeks during the course of treatment.

In a preferred example of combination therapy in the treatment of lung cancer, the compound of Formula (I) is administered orally in a range of from 50 to 400 mg/day, in two divided doses, in association with the antineoplastic agent, paclitaxel, which is administered at a dosage of from 65 to 175 $mg/m^2$ once every three weeks.

In a preferred example of combination therapy in the treatment of gliomas, the compound of Formula (I) is

administered orally in a range of from 50 to 400 mg/day, in two divided doses; in association with the antineoplastic agent, temozolomide, which is administered at a dosage of from 100 to 250 mg/$m^2$.

In another example of combination therapy, the compound of Formula (I) is administered orally in a range of from 50 to 400 mg/day, in two divided doses, in association with the antineoplastic agent, cisplatin, which is administered intravenously in a range of from 50 to 100 mg/m2 once every four weeks.

In another example of combination therapy, the compound of Formula (I) is administered orally in a range of from 50 to 400 mg/day, in two divided doses, in association with the antineoplastic agent, carboplatin, which is administered intravenously in a range of from 300 - 360 mg/m2 once every four weeks

In another example of combination therapy, the compound of Formula (I) is administered orally in a range of from 50 to 400 mg/day, in two divided doses, in association with the chemotherapeutic agent, carboplatin, which is administered intravenously in a range of from 300 to 360 mg/m2 once every four weeks and the chemotherapeutic agent, paclitaxel, which is administered at a dosage of from 65 to 175 mg/$m^2$ once every three weeks.

In yet another example of combination therapy, the compound of Formula (I) is administered orally in a range of from 50 to 400 mg/day, in two divided doses, in association with the chemotherapeutic agent, Cisplatin, which is administered intravenously in a range of from 50 to 100 mg/m2 once every four weeks and the chemotherapeutic agent, Gemcitabine, which is administered at a dosage of from 65 to 175 mg/$m^2$ once every three weeks.

The signal transduction inhibition therapy can be administered according to the dosage and administration schedule listed in the product information sheet of the approved agents, in the Physicians Desk Reference (PDR) as well as therapeutic protocols well known in the art. Table (2.0) below gives ranges of dosage and dosage regimens of some exemplary signal transduction inhibitors. It will be apparent to those skilled in the art that the administration of the signal tranduction inhibitor can be varied depending on the disease being treated and the known effects of the signal transduction inhibitor therapy on that disease. Also, in accordance with the knowledge of the skilled clinician, the therapeutic protocols (e.g., dosage amounts and times of administration) can be varied in view of the observed effects of the administered signal transduction inhibitors on the patient, and in view of the observed responses of the disease to the administered therapeutic agents.

In another example of combination therapy, the compound of Formula (I) is administered orally in a range of from 50 to 400 mg/day, in two divided doses in association with the signal tranduction inhibitor, EGF receptor kinase inhibitor, Iressa (ZD1839), which is administered orally in the range of 150 - 700 mg/day.

TABLE 1.0

Examplary Chemotherapeutic Agents Dosage and Dosage Regimens

| | |
|---|---|
| Cisplatin: | 50 -100 mg/$m^2$ every 4 weeks (IV)* |
| Carboplatin: | 300 - 360 mg/$m^2$ every 4 weeks (IV) |
| Taxotere: | 60 - 100 mg/$m^2$ every 3 weeks (IV) |
| *(IV)-intravenously | |

TABLE 2.0

Examplary Signal Transduction Inhibitors Dosage and Dosage Regimens

| | |
|---|---|
| Iressa (ZD1839) - EGF receptor kinase inhibitor: | 150 - 700 mg/day (oral) |
| OSI-774 - EGF receptor kinase inhibitor: | 100 - 1000 mg/day (oral) |
| Herceptin - her-2/neu antibody: | 100 - 250 mg/$m^2$/week (IV)* |
| C225 - EGF receptor antibody: | 200 - 500 mg/$m^2$/week (IV) |
| ABX-EGF - EGF receptor antibody: | 0.2 - 2 mg/kg every 2 weeks (IV) |
| Gleevec (STI-571) - bcr/abl kinase inhibitor: | 300 - 1000 mg / day (oral) |
| *(IV)-intravenously | |

In the methods of the present invention, an FPT inhibitor compound of formula (I) is administered concurrently or sequentially with another therapeutic agent (i.e. a chemotherapeutic agent, a signal transduction inhibitor and/or radiation). Thus, it is not necessary that, for example, the therapeutic agent and the FPT inhibitor compound of formula (I) be administered simultaneously, just prior to or after one another.

Also, in general, the FPT inhibitor compound of formula (I), the chemotherapeutic agent, signal transduction inhibitor and/or radiation, do not have to be administered in the same pharmaceutical composition, and may, because of different physical and chemical characteristics, have to be administered by different routes. For example, the FPT inhibitor compound of formula (I) may be administered orally to generate and maintain good blood levels thereof, while

the chemotherapeutic agent may be administered intravenously. The determination of the mode of administration and the advisability of administration, where possible, in the same pharmaceutical composition, is well within the knowledge of the skilled clinician. The initial administration can be made according to established protocols known in the art, and then, based upon the observed effects, the dosage, modes of administration and times of administration can be modified by the skilled clinician.

The particular choice of the FPT inhibitor compound of formula (I), the chemotherapeutic agent, signal transduction inhibitor and/or radiation will depend upon the diagnosis of the attending physicians and their judgement of the condition of the patient and the appropriate treatment protocol.

The FPT inhibitor compound of formula (I), chemotherapeutic agent, signal transduction inhibitor and/or radiation may be administered concurrently (e.g., simultaneously, just prior to or after, or within the same treatment protocol) or sequentially, depending upon the nature of the proliferative disease, the condition of the patient, and the actual choice of chemotherapeutic agent, signal transduction inhibitor and/or radiation to be administered in conjunction (i.e., within a single treatment protocol) with the FPT inhibitor compound of formula (I).

If the FPT inhibitor compound of formula (I), chemotherapeutic agent, signal transduction inhibitor and/or radiation are not administered simultaneously, then the initial order of administration of the FPT inhibitor compound of formula (I), chemotherapeutic agent, signal transduction inhibitor and/or radiation, may not be important. Thus, the FPT inhibitor compound of formula (I) may be administered first followed by the administration of the chemotherapeutic agent, signal transduction inhibitor and/or radiation; or the chemotherapeutic agent, signal transduction inhibitor and/or radiation may be administered first followed by the administration of the FPT inhibitor compound of formula (1). This alternate administration may be repeated during a single treatment protocol. The determination of the order of administration, and the number of repititions of administration of each therapeutic agent during a treatment protocol, is well within the knowledge of the skilled physician after evaluation of the disease being treated and the condition of the patient. For example, the chemotherapeutic agent, signal transduction inhibitor and/or radiation may be administered first, especially if it is a cytotoxic agent, and then the treatment continued with the administration of the FPT inhibitor compound of formula (I), followed by, where determined advantageous, the administration of the chemotherapeutic agent, signal transduction inhibitor and/or radiation, and so on until the treatment protocol is complete.

Thus, in accordance with experience and knowledge, the practising physician can modify each protocol for the administration of a component (therapeutic agent-*i.e.,* FPT inhibitor compound of formula (I), chemotherapeutic agent, signal transduction inhibitor or radiation) of the treatment according to the individual patient's needs, as the treatment proceeds.

The attending clinician, in judging whether treatment is effective at the dosage administered, will consider the general well-being of the patient as well as more definite signs such as relief of disease-related symptoms, inhibition of tumor growth, actual shrinkage of the tumor, or inhibition of metastasis. Size of the tumor can be measured by standard methods such as radio-logical studies, e.g., CAT or MRI scan, and successive measure-ments can be used to judge whether or not growth of the tumor has been retarded or even reversed. Relief of disease-related symptoms such as pain, and improvement in overall condition can also be used to help judge effectiveness of treatment.

While the present invention has been described in conjunction with the specific embodiments set forth above, many alternatives, modifications and variations thereof will be apparent to those of ordinary skill in the art.

## Claims

**1.** A compound of the formula:

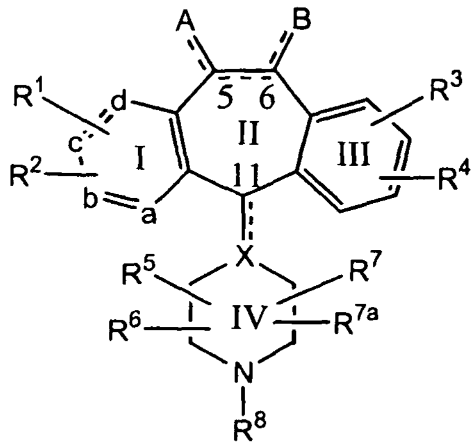

(1.0)

or a pharmaceutically acceptable salt or solvate thereof, wherein:

one of a, b, c and d represents N or $N^+O^-$, and the remaining a, b, c, and d groups represent carbon, wherein each carbon has an $R^1$ or $R^2$ group bound to said carbon; or
each of a, b, c, and d is carbon, wherein each carbon has an $R^1$ or $R^2$ group bound to said carbon;
the dotted lines (---) represent optional bonds;
X represents N or CH when the optional bond is absent, and represents C when the optional bond is present;
when the optional bond is present between carbon atom 5 and carbon atom 6 then there is only one A substituent bound to carbon atom 5 and there is only one B substituent bound to carbon atom 6 and A or B is other than H;
when the optional bond is not present between carbon atom 5 and carbon atom 6, then there are two A substituents bound to carbon atom 5 and two B
substituents bound to carbon atom 6, and wherein at least one of the two A substituents or one of the two B substituents are H, and wherein at least one of the two A substituents or one of the two B substituents is other than H, wherein each A and B substituent is independently selected from:

(1) -H;
(2) $-R^9$;
(3) $-R^9-C(O)-R^9$;
(4) - $R^9-CO_2-R^{9a}$;
(5) $-C(O)NHR^9$;
(6) $-C(O)NH-CH_2-C(O)-NH_2$;
(7) $-C(O)NHR^{26}$;
(8) $-(CH_2)p(R^9)_2$, wherein each $R^9$ is the same or different;
(9) $-(CH_2)pC(O)R^9$;
(10) $-(CH_2)pC(O)R^{27a}$;
(11) $-(CH_2)pC(O)N(R^9)_2$, wherein each $R^9$ is the same or different;
(12) $-(CH_2)pC(O)NH(R^9)$;
(13) $-(CH_2)pNHC(O)R^{50}$;
(14) $-(CH_2)pNHC(O)_2R^{50}$;
(15) $-(CH_2)pN(C(O)R^{27a})_2$ wherein each $R^{27a}$ is the same or different;
(16) $-(CH_2)pNR^{51}C(O)R^{27}$, or $R^{51}$ and $R^{27}$ taken together with the atoms to which they are bound form a heterocycloalkyl ring consisting of, 5 or 6 members, provided that when $R^{51}$ and $R^{27}$ form a ring, $R^{51}$ is not H;
(17) $-(CH_2)pNR^{51}C(O)NR^{27}$, or $R^{51}$ and $R^{27}$ taken together with the atoms to which they are bound form a heterocycloalkyl ring consisting or 5 or 6 members, provided that when $R^{51}$ and $R^{27}$ form a ring, $R^{51}$ is not H;
(18) $-(CH_2)pNR^{51}C(O)N(R^{27a})_2$, wherein each $R^{27a}$ is the same or different;
(19) $-(CH_2)pNHSO_2N(R^{51})_2$, wherein each $R^{51}$ is the same or different;
(20) $-(CH_2)pNHCO_2R^{50}$;
(21) $-(CH_2)pCO_2R^{51}$;
(22) $-NHR^9$;

(23)

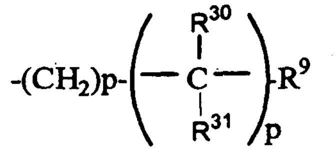

wherein $R^{30}$ and $R^{31}$ are the same or different; and
(24)

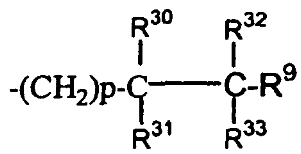
,

wherein $R^{30}$, $R^{31}$, $R^{32}$ and $R^{33}$ are the same or different;

p is 0, 1, 2, 3 or 4;
each $R^1$ and $R^2$ is independently selected from H, Halogen, $-CF_3$, $-OR^{10}$, $COR^{10}$, $-SR^{10}$, $-S(O)_tR^{15}$ wherein t is 0, 1 or 2, $-N(R^{10})_2$, $-NO_2$, $-OC(O)R^{10}$, $CO_2R^{10}$, $-OCO_2R^{15}$, -CN, $-NR^{10}COOR^{15}$, $-SR^{15}C(O)OR^{15}$, $-SR^{15}N(R^{13})_2$ provided that $R^{15}$ in $-SR^{15}N(R^{13})_2$ is not $-CH_2$, and wherein each $R^{13}$ is independently selected from H or $-C(O)OR^{15}$, benzotriazol-1-yloxy, tetrazol-5-ylthio, or substituted tetrazol-5-ylthio, alkynyl, alkenyl or alkyl, said alkyl or alkenyl group optionally being substituted with halogen, $-OR^{10}$ or $-CO_2R^{10}$;
$R^3$ and $R^4$ are the same or different and each independently represent H, or any of the substituents of $R^1$ and $R^2$;
$R^5$, $R^6$, R7 and $R^{7a}$ each independently represent H, $-CF_3$, $-COR^{10}$, alkyl or aryl, said alkyl or aryl optionally being substituted with $-OR^{10}$, $-SR^{10}$, $-S(O)_tR^{15}$, $-NR^{10}COOR^{15}$, $-N(R^{10})_2$, $-NO_2$, $-C(O)R^{10}$, $-OCOR^{10}$, $-OCO_2R^{15}$, $-CO_2R^{10}$, $OPO_3R^{10}$, or $R^5$ is combined with $R^6$ to represent =O or =S;
$R^8$ is selected from:

H, 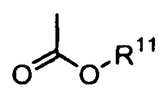 , 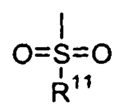 , 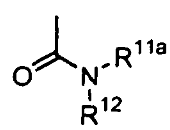 or 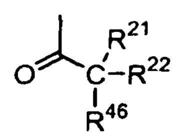

(2.0) (3.0) (4.0) (5.0)

$R^9$ is selected from:

(1) heteroaryl;
(2) substituted heteroaryl;
(3) heterocycloalkyl;
(4) substituted heterocycloalkyl;
(5) heterocycloalkylalkyl;
(6) substituted heterocycloalkylalkyl;
(7) heteroarylalkyl;
(8) substituted heteroarylalkyl;
(9) heteroarylalkenyl;
(10) substituted heteroarylalkenyl;
(11) heteroarylalkynyl; and
(12) substituted heteroarylalkynyl;

wherein said substituted $R^9$ groups are substituted with one or more substituents selected from:

(1) -OH;
(2) $-CO_2R^{14}$;
(3) $-CH_2OR^{14}$,
(4) halogen;
(5) alkyl;
(6) amino;
(7) trityl;
(8) heterocycloalkyl;
(9) cycloalkyl;
(10) arylalkyl;
(11) heteroaryl;
(12) heteroarylalkyl and
(13)

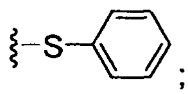

wherein $R^{14}$ is independently selected from: H; alkyl; aryl, arylalkyl, heteroaryl and heteroarylalkyl;
$R^{9a}$ is selected from: alky or arylalkyl;
$R^{10}$ is selected from: H; alkyl; aryl or arylalkyl;
$R^{11}$ is selected from:

(1) alkyl;
(2) substituted alkyl;
(3) aryl;
(4) substituted aryl;
(5) cycloalkyl;
(6) substituted cycloalkyl;
(7) heteroaryl;
(8) substituted heteroaryl;
(9) heterocycloalkyl; and
(10) substituted heterocycloalkyl;

wherein said substituted $R^{11}$ groups have 1, 2 or 3 substituents selected from:

(1) -OH;
(2) halogen and
(3) alkyl;

$R^{11a}$ is selected from:

(1) H;
(2) OH;
(3) alkyl;
(4) substituted alkyl;
(5) aryl;
(6) substituted aryl;
(7) cycloalkyl;
(8) substituted cycloalkyl;
(9) heteroaryl;
(10) substituted heteroaryl;
(11) heterocycloalkyl; and
(12) substituted heterocycloalkyl;

wherein said substituted $R^{11a}$ groups have one or more substituents selected from:

(1) -OH;
(2) -CN;
(3) $-CF_3$;
(4) halogen;
(5) alkyl;
(6) cycloalkyl;
(7) heterocycloalkyl;
(8) arylalkyl;
(9) heteroarylalkyl;
(10) alkenyl and
(11) heteroalkenyl;

$R^{12}$ is selected from: H, or alkyl;
$R^{15}$ is selected from: alkyl or aryl;
$R^{21}$, $R^{22}$ and $R^{46}$ are independently selected from:

(1) -H;
(2) alkyl;
(3) aryl;
(4) substituted aryl, optionally substituted with one or more substituents selected from: alkyl, halogen, $CF_3$ or OH;
(5) cycloalkyl;
(6) substituted cycloalkyl; optionally substituted with one or more substituents selected from: alkyl, halogen, $CF_3$ or OH;
(7) heteroaryl of the formula,

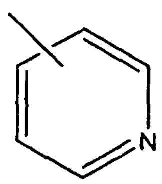

and

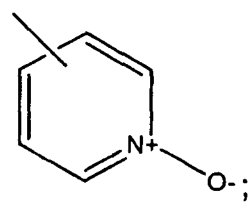

(8) heterocycloalkyl of the formula:

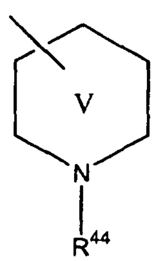

wherein $R^{44}$ is selected from:

(1) -H;
(2) alkyl;
(3) alkylcarbonyl;
(4) alkyloxy carbonyl;
(5) haloalkyl and
(6) $-C(O)NH(R^{51})$;

when $R^{21}$, $R^{22}$ or $R^{46}$ is the heterocycloalkyl of the formula above, Ring V is:

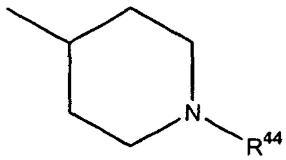 , 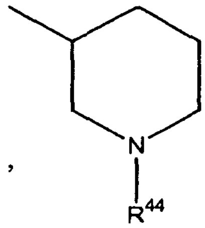 , 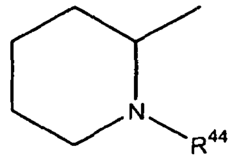

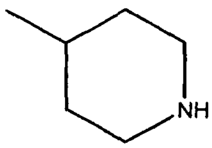 , 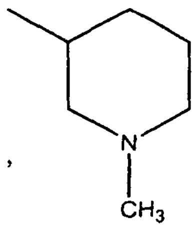 , 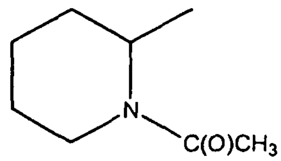 ,

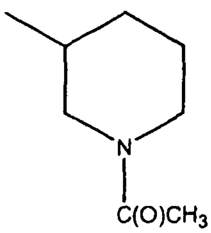 , 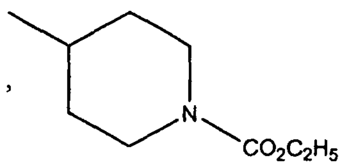 ', 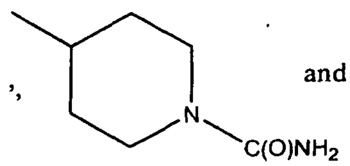 and

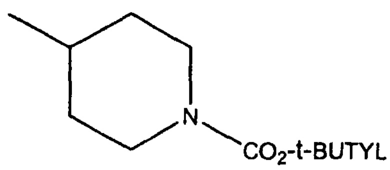

$R^{26}$ is selected from:

(1) -H;
(2) alkyl;
(3) alkoxyl;
(4) $-CH_2-CN$;
(5) $R^9$;
(6) $-CH_2CO_2H$;
(7) -C(O)alkyl and
(8) $CH_2CO_2$alkyl;

$R^{27}$ is selected from:

(1) -H;
(2) -OH;
(3) alkyl and
(4) alkoxy ;

$R^{27a}$ is selected from:

(1) alkyl or
(2) alkoxy ;

$R^{30}$ through $R^{33}$ is independently selected from:

(1) -H;
(2) -OH;
(3) =O;
(4) alkyl;
(5) aryl and
(6) arylalkyl;

$R^{50}$ is selected from:

(1) alkyl;
(2) heteroaryl;
(3) substituted heteroaryl and
(4) amino;

wherein said substituents on said substituted $R^{50}$ groups are independently selected from: alkyl; halogen; or-OH; $R^{50a}$ is selected from:

(1) heteroaryl;
(2) substituted heteroaryl and
(3) amino;

$R^{51}$ is selected from: -H, or alkyl.

**2.** A compound of Claim 1 having the structure:

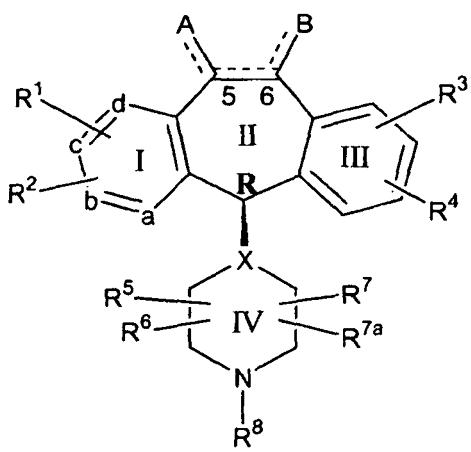

(1.0A)

wherein:

X = CH or N;
B is H when the optional bond is present between C-5 and C-6, and when the optional bond between C-5 and C-6 is absent then each B is H;
or having the structure:

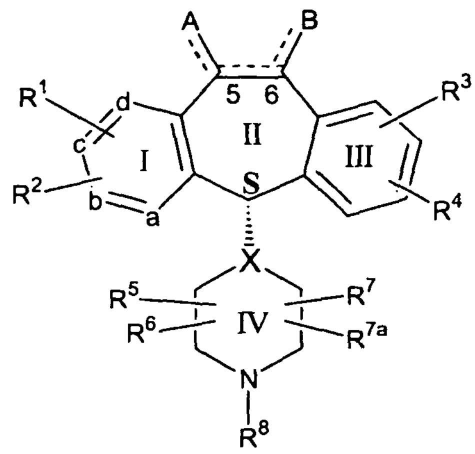

(1.0B)

wherein:

X = CH or N;
A is H when the optional bond is present between C-5 and C-6, and when the optional bond between C-5 and C-6 is absent then each A is H.

**3.** The compound of claim 1 wherein:

$R^1$ to $R^4$ are each independently selected from H or halo;
$R^5$ to $R^7$ are H;
a is N and the remaining b, c and d are carbon, or a, b, c, and d are carbon; and
$R^8$ is group 2.0, or 4.0.

**4.** The compound of claim 2 having the formula (1.0A) wherein;

a is N and the remaining b, c, and d are carbon, and
$R^1$ to $R^4$ are each independently selected from H, Br or Cl.

**5.** The compound of claim 1 wherein:

(1) $R^{11}$ is selected from: alkyl, cycloalkyl or substituted cycloalkyl, said substituted groups are substituted with halo, alkyl or amino;
(2) $R^{11a}$ is selected from: alkyl, aryl, substituted aryl, cycloalkyl or substituted cycloalkyl, said substituted groups are substituted with halo, -CN or $CF_3$;
(3) $R^{12}$, $R^{21}$, and $R^{22}$ are H; and
(4) $R^{46}$ is selected from: aryl, substituted aryl, heteroaryl of the formula:

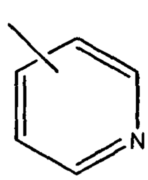

and

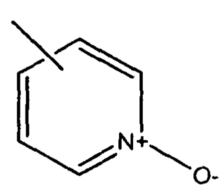

or
hetercycloalkyl of the formula:

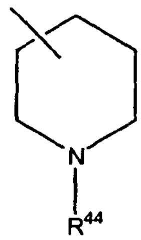

wherein, said substituted groups are substituted with alkyl, alkylcarbonyl or haloalkyl;
(5) $R^{44}$ is selected from H or $-C(O)NH_2$;
(6) $R^8$ is selected from:

(a) group 2.0 wherein $R^{11}$ is selected from:

t-butyl or cyclohexyl;

(b) group 3.0 wherein $R^{11}$ is selected from methyl or t-butyl;
(c) group 4.0 wherein, $R^{12}$ is H and $R^{11a}$ is selected from t-butyl, cyanophenyl, chlorophenyl, fluorophenyl or cyclohexyl;
(d) group 5.0 wherein $R^{21}$ and $R^{22}$ are H and $R^{46}$ is selected from:

(1) heteroaryl of the formula:

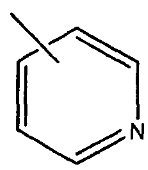

and

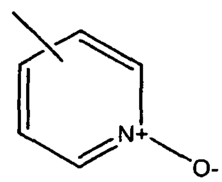

or
(2) hetercycloalkyl of the formula:

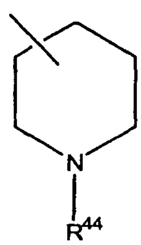

and wherein $R^{44}$ is $-C(O)NH_2$.

6. The compound of claim 5 wherein $R^8$ is group 4.0.

7. The compound of claim 1 wherein one of A and B is H and the other is $R^9$.

8. The compound of claim 1 wherein $R^9$ is selected from:

(1) heterocycloalkylalkyl of the formula $-(CH_2)n$-heterocycloalkyl;
(2) substituted heterocycloalkylalkyl of the formula $-(CH_2)n$-substituted heterocycloalkyl;
(3) heteroarylalkyl of the formula $-(CH_2)n$-heteroaryl, and
(4) substituted heteroarylalkyl of the formula $-(CH_2)n$-substituted heteroaryl.

wherein n is 1, 2, or 3 and the substituents for said substituted $R^9$ groups are each independently selected from:

(1) -OH;
(2) $-CO_2R^{14}$;
(3) $-CH_2OR^{14}$,
(4) halo,
(5) alkyl;
(6) amino;
(7) trityl;
(8) heterocycloalkyl;
(9) arylalkyl;
(10) heteroaryl and
(11) heteroarylalkyl.

wherein $R^{14}$ is independently selected from: H; or alkyl.

**9.** The compound of claim 8 wherein $R^9$ is

(1) $-(CH_2)$n-imidazolyl;
(2) -(CH2)n-substituted imidazolyl;
(3) -(CH2)n-morpholinyl;
(4) -(CH2)n-substituted morpholinyl,
(5) -(CH2)n-piperazinyl, or
(6) -(CH2)n-substituted piperazinyl,

wherein n is 1, 2, or 3.

**10.** The compound of claim 1 wherein the optional bond is present between C-5 and C-6 and A is H and B is $R^9$, or A is $R^9$ and B is H; or the optional bond between C-5 and C-6 is absent and each A is H, one B is H and the other B is $R^9$, or one A is H, the other A is $R^9$ and each B is H; $R^1$ to $R^4$ are independently H or halo; $R^5$ to $R^{7a}$ are H; a is N and the remaining b, c, an d substituents are carbon; X is N or CH and $R^8$ is group 2.0 or 4.0.

**11.** The compound of claim 10 wherein $R^9$ is selected from:

(1) heteroaryl;
(2) substituted heteroaryl;
(3) heterocycloalkyl;
(4) substituted heterocycloalkyl;
(5) heterocycloalkylalkyl;
(6) substituted heterocycloalkylalkyl;
(7) heteroarylalkyl;
(8) substituted heteroarylalkyl;
(9) heteroarylalkenyl and
(10) substituted heteroarylalkenyl,

wherein substituents for said substituted $R^9$ groups are each independently selected from:

(1) -OH;
(2) $-CO_2R^{14}$;
(3) $-CH_2OR^{14}$.
(4) halo,
(5) alkyl;
(6) amino;
(7) trityl;
(8) heterocycloalkyl;
(9) arylalkyl;
(10) heteroaryl and
(11) heteroarylalkyl,

wherein $R^{14}$ is independently selected from: H; or alkyl.

**12.** The compound of claim 11 wherein $R^9$ is selected from:

(1) heterocycloalkylalkyl of the formula $-(CH_2)n$-heterocycloalkyl;
(2) substituted heterocycloalkylalkyl of the formula $-(CH_2)n$-substituted heterocycloalkyl;
(3) heteroarylalkyl of the formula $-(CH_2)n$-heteroaryl, and
(4) substituted heteroarylalkyl of the formula $-(CH_2)n$-substituted heteroaryl.

wherein substituents for said substituted $R^9$ groups are each independently selected from:

(1) -OH;
(2) $-CO_2R^{14}$;
(3) $-CH_2OR^{14}$,
(4) halo,
(5) alkyl;
(6) amino;
(7) trityl;
(8) heterocycloalkyl;
(9) arylalkyl;
(10) heteroaryl and
(11) heteroarylalkyl.

**13.** The compound of claim 12 wherein $R^8$ is group 4.0 and wherein $R^{12}$ is H and $R^{11a}$ is selected from:

(1) alkyl;
(2) aryl;
(3) substituted aryl;
(4) cycloalkyl and
(5) substituted cycloalkyl,

wherein said substituents of said substituted groups are selected from:

(1) halo;
(2) -CN or
(3) -CF3.

**14.** The compound of claim 12 wherein $R^9$ is

(1) -(CH2)n-imidazolyl;
(2) -(CH2)n-substituted imidazolyl;
(3) -(CH2)n-morpholinyl;
(4) -(CH2)n-substituted morpholinyl;
(5) -(CH2)n-piperazinyl, or
(6) -(CH2)n-substituted piperazinyl,

wherein n is 1, 2, or 3.

**15.** The compound of claim 14 wherein the optional bond is present.

**16.** The compound of claim 15 wherein $R^8$ is 4.0 and wherein $R^{12}$ is H and $R^{11a}$ is selected from:

(1) alkyl;
(2) aryl;
(3) substituted aryl;
(4) cycloalkyl, and
(5) substituted cycloalkyl,

wherein said substituents of said substituted groups are selected from:

(1) halo;
(2) cyano, and
(3) CF3.

**17.** The compound of claim 16 wherein $R^8$ is 4.0, $R^{12}$ is H and $R^{11a}$ is substituted phenyl and wherein said substituent of said substituted group selected from:

(1) -CN or
(2) CF3.

**18.** The compound of claim 14 wherein the optional bond is absent.

**19.** The compound according to claim 1 which is selected from any one of the Examples 1-505.

**20.** The compound according to claim 1 which is selected from the group consisting of:

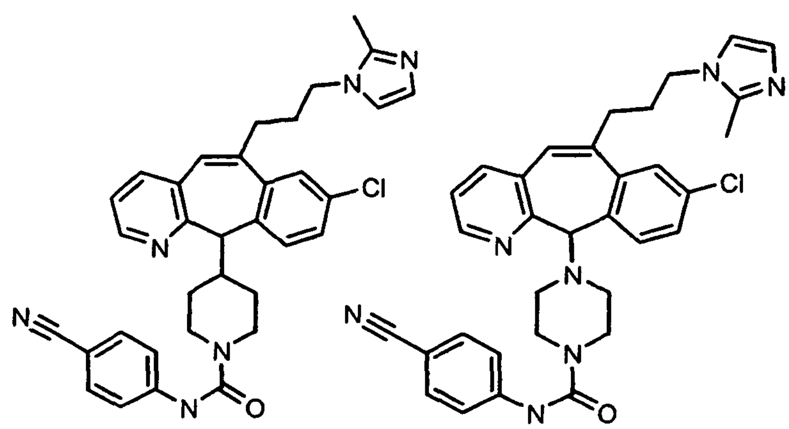

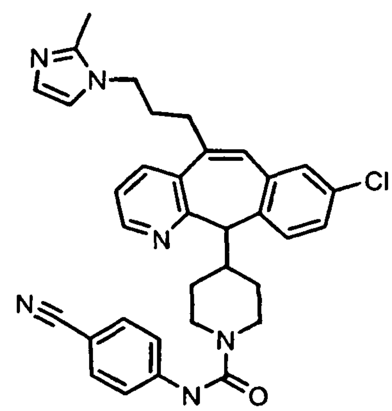

(139) (628) (699)

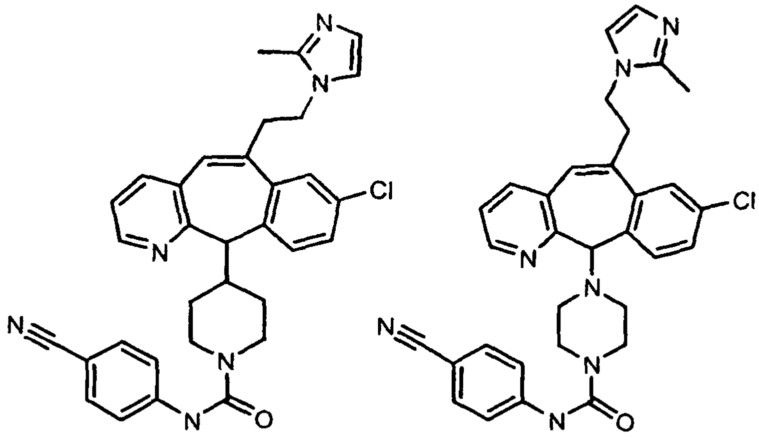

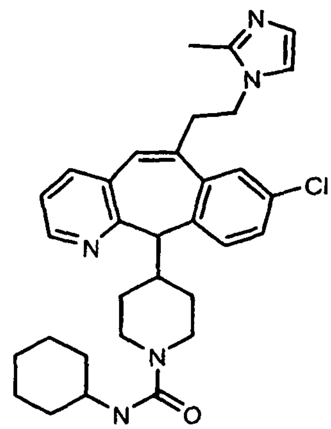

(326) (644) (332)

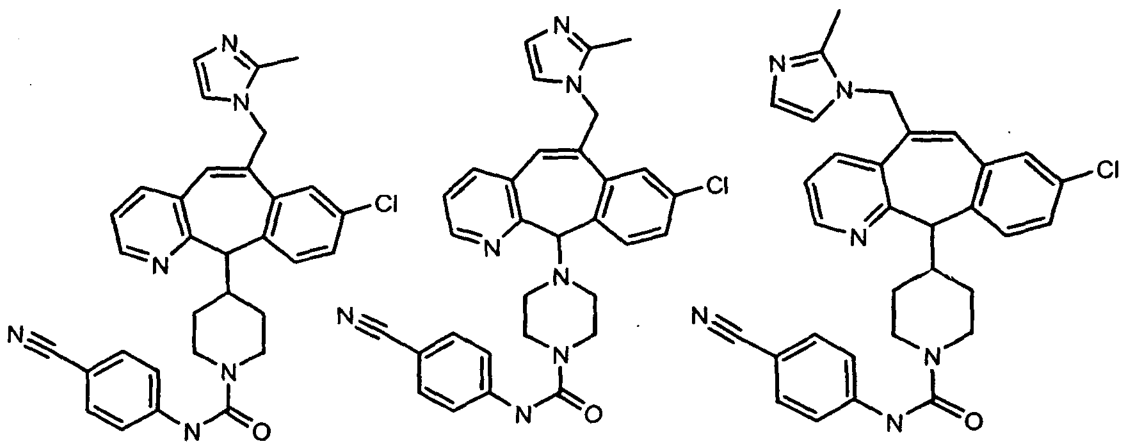

(362a) (372) (230)

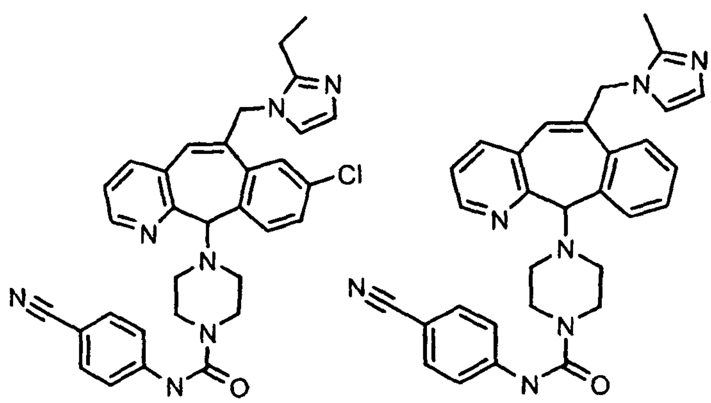

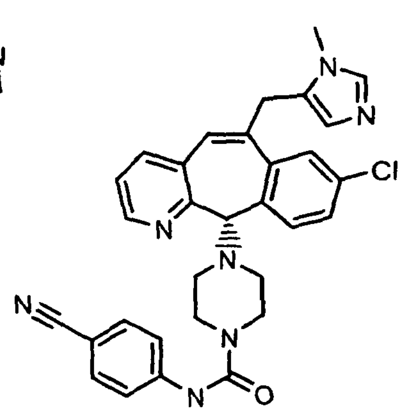

(378) (690) (784)

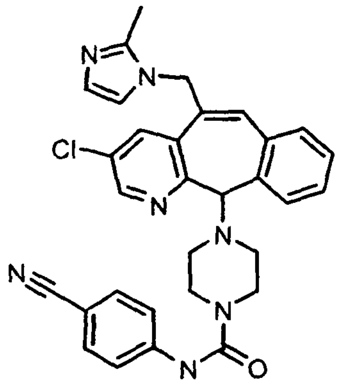

(684)

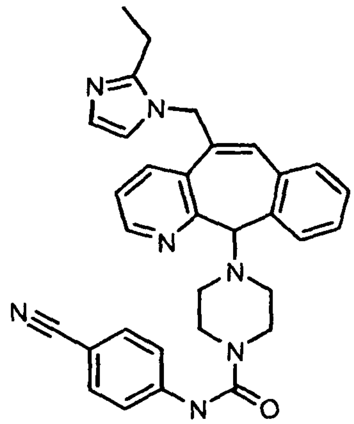

(688)

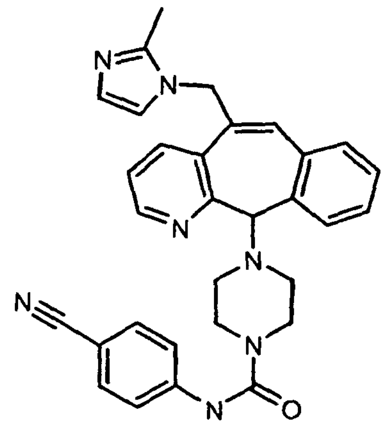

(686)

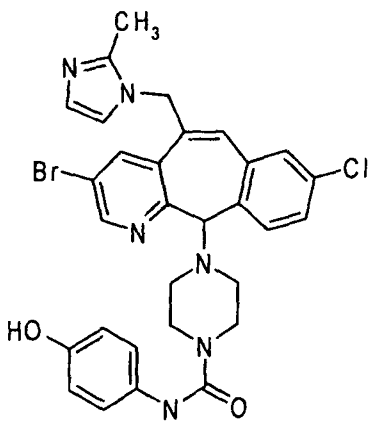

;

(683.2)

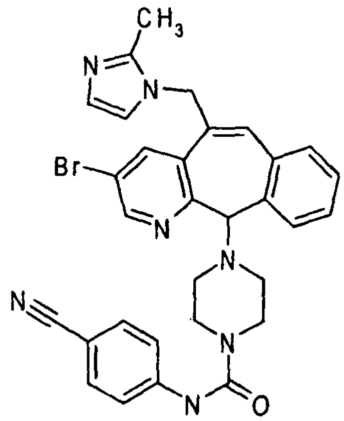

;

(877)

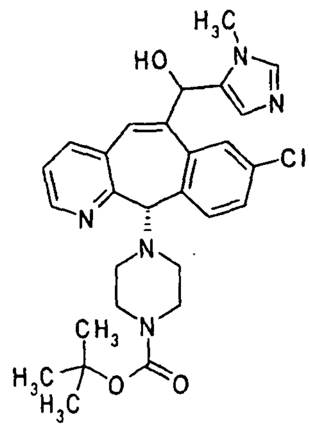

(790)

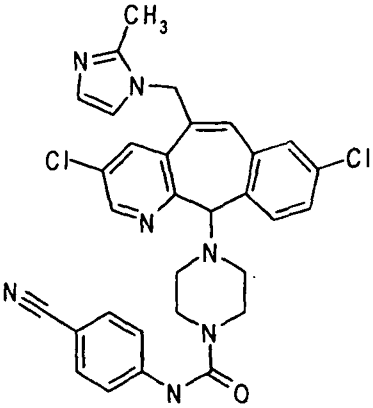

;

(816)

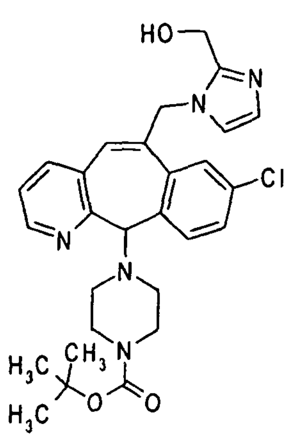

;

(788)

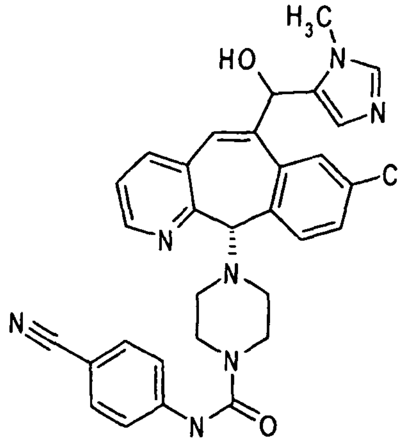

;

(793)

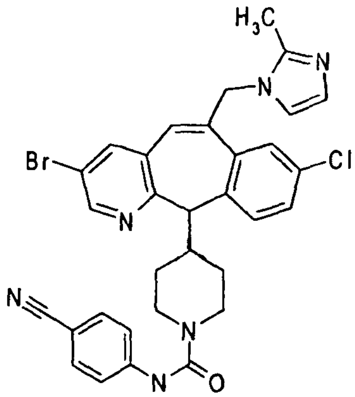 ; 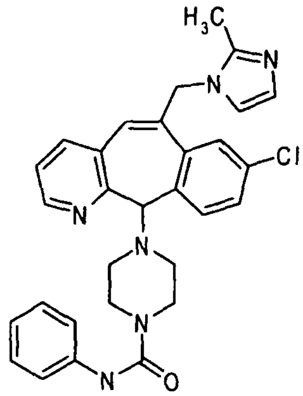 ; and 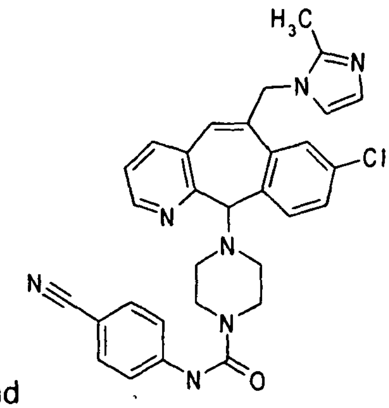 .

(778) (375.1) (372)

**21.** A pharmaceutical composition comprising an effective amount of a compound of any of claims 1 to 20 in combination with a pharmaceutically acceptable carrier.

**22.** The use of a compound of any of claims 1 to 20 for the manufacture of a medicament in a combination therapy for the treatment of a proliferative disease, wherein said compound and at least one chemotherapeutic agent are administered concurrently or sequentially.

**23.** The use of claim 22 wherein the chemotherapeutic agent is an antineoplastic agent selected from Uracil mustard, Chlormethine, Cyclophosphamide, Ifosfamide, Melphalan, Chlorambucil, Pipobroman, Triethylenemelamine, Triethylenethiophosphoramine, Busulfan, Carmustine, Lomustine, Streptozocin, Dacarbazine, Temozolomide, Methotrexate, 5-Fluorouracil, Floxuridine, Cytarabine, 6-Mercaptopurine, 6-Thioguanine, Fludarabine phosphate, Pentostatine, Gemcitabine, Vinblastine, Vincristine, Vindesine, Bleomycin, Dactinomycin, Daunorubicin, Doxorubicin, Epirubicin, Idarubicin, Paclitaxel (Taxol), Mithramycin, Deoxycoformycin, Mitomycin-C, L-Asparaginase, Interferons, Etoposide, Teniposide 17$\alpha$-Ethinylestradiol, Diethylstilbestrol, Testosterone, Prednisone, Fluoxymesterone, Dromostanolone propionate, Testolactone, Megestrolacetate, Tamoxifen, Methylprednisolone, Methyltestosterone, Prednisolone, Triamcinolone, Chlorotrianisene, Hydroxyprogesterone, Aminoglutethimide, Estramustine, Medroxyprogesteroneacetate, Leuprolide, Flutamide, Toremifene, goserelin, Cisplatin, Carboplatin, Hydroxyurea, Amsacrine, Procarbazine, Mitotane, Mitoxantrone, Levamisole, Navelbene, CPT-11, Anastrazole, Letrazole, Capecitabine, Reloxafine, Droloxafine, and Hexamethylmelamine.

**24.** The use of claim 22 wherein said chemotherapeutic agent is a microtubule affecting agent selected from allocolchicine, Halichondrin B, colchicine, colchicine derivatives, dolastatin 10, maytansine, rhizoxin, paclitaxel, paclitaxel derivatives, thiocolchicine, trityl cysteine, vinblastine sulfate, vincristine sulfate, epothilone A, epothilone, discodermolide estramustine, nocodazole and MAP4.

**25.** The use of a compound of any of claims 1 to 20 for the manufacture of a medicament in a combination thereapy for the treatment of a proliferative disease, wherein said compound and at least one signal transduction inhibitor are administered concurrently or sequentially.

**26.** The use of claim 25 wherein said signal transduction inhibitor is selected from the bcr/abl inhibitor Gleevec, the epidermal growth factor receptor inhibitors, Iressa, OSI-774, Imclone C225 and Abgenix ABX-EGF and the her-2/neu receptor inhibitor Herceptin.

**Patentansprüche**

**1.** Verbindung mit der Formel:

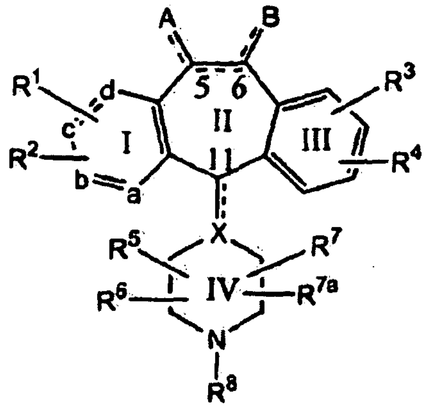

(1.0)

oder ein pharmazeutisch annehmbares Salz oder Solvat davon, worin
einer von a, b, c und d für N oder $N^+O^-$ steht, und die restlichen a-, b-, c- und d-Gruppen für Kohlenstoff stehen, wobei jeder Kohlenstoff eine an den Kohlenstoff gebundene $R^1$- oder $R^2$-Gruppe aufweist, oder
jeder von a, b, c und d Kohlenstoff ist, wobei jeder Kohlenstoff eine an den Kohlenstoff gebundene $R^1$- oder $R^2$-Gruppe aufweist;
die gestrichelten Linien (---) für optionale Bindungen stehen;
X für N oder CH steht, wenn die optionale Bindung fehlt, oder für C steht, wenn die optionale Bindung vorhanden ist;
wenn die optionale Bindung zwischen Kohlenstoffatom 5 und Kohlenstoffatom 6 vorhanden ist, dann ist nur ein A-Substituent vorhanden, der an Kohlenstoffatom 5 gebunden ist, und dann ist nur ein B-Substituent vorhanden, der an Kohlenstoffatom 6 gebunden ist, und A oder B sind von H verschieden;
wenn die optionale Bindung zwischen Kohlenstoffatom 5 und Kohlenstoffatom 6 fehlt, dann sind zwei A-Substituenten an Kohlenstoffatom 5 gebunden und zwei B-Substituenten an Kohlenstoffatom 6 gebunden, und wobei mindestens einer der zwei A-Substituenten oder einer der zwei B-Substituenten H ist, und wobei mindestens einer der zwei A-Substituenten oder einer der zwei B-Substituenten von H verschieden ist, wobei jeder A- und B-Substituent unabhängig ausgewählt ist aus:

(1) -H;
(2) $-R^9$;
(3) $-R^9-C(O)-R^9$;
(4) - $R^9-CO_2-R^{9a}$;
(5) $-C(O)NHR^9$;
(6) $-C(O)NH-CH_2-C(O)-NH_2$ ;
(7) $-C(O)NHR^{26}$;
(8) $-(CH_2)_p(R^9)_2$, wobei jedes $R^9$ gleich oder unterschiedlich ist;
(9)$-(CH_2)_pC(O)R^9$;
(10) $-(CH_2)_pC(O)R^{27a}$;
(11)$-(CH_2)_pC(O)N(R^9)_2$, wobei jedes $R^9$ gleich oder unterschiedlich ist;
(12) $-(CH_2)_pC(O)NH(R^9)$;
(13) $-(CH_2)_pNHC(O)R^{50}$;
(14) $-(CH_2)_pNHC(O)_2R^{50}$;
(15)$-(CH_2)_pN(C(O)R^{27a})_2$, wobei jedes $R^{27a}$ gleich oder unterschiedlich ist;
(16) $-(CH_2)_pNR^{51}C(O)R^{27}$, oder $R^{51}$ und $R^{21}$ zusammen mit den Atomen, an die sie gebunden sind, einen Heterocycloalkylring bilden, der aus 5 oder 6 Gliedern besteht, mit der Maßgabe, dass $R^{51}$ nicht H ist, wenn $R^{51}$ und $R^{27}$ einen Ring bilden;
(17) $-(CH_2)_pNR^{51}C(O)NR^{27}$, oder $R^{51}$ und $R^{27}$ zusammen mit den Atomen, an die sie gebunden sind, einen Heterocycloalkylring bilden, der aus 5 oder 6 Gliedern besteht, mit der Maßgabe, dass $R^{51}$ nicht H ist, wenn $R^{51}$ und $R^{27}$ einen Ring bilden;
(18) - $(CH_2)_pNR^{51}C(O)N(R^{27a})_2$, wobei jedes $R^{27a}$ gleich oder unterschiedlich ist;
(19) - $(CH_2)_pNHSO_2N(R^{51})_2$, wobei jedes $R^{51}$ gleich oder unterschiedlich ist;
(20) $-(CH_2)_pNHCO_2R^{50}$;
(21) $-(CH_2)_pCO_2R^{51}$;

(22) $-NHR^9$;

(23)

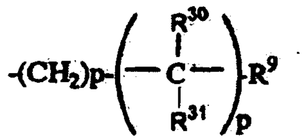

wobei $R^{30}$ und $R^{31}$ gleich oder unterschiedlich sind, und

(24)

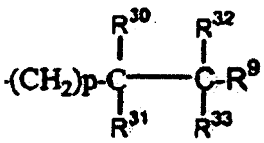

wobei $R^{30}$, $R^{31}$, $R^{32}$ und $R^{33}$ gleich oder unterschiedlich sind;

p 0, 1, 2, 3 oder 4 ist;
jedes $R^1$ und $R^2$ unabhängig ausgewählt ist aus der Gruppe bestehend aus H, Halogen, $-CF_3$, $-OR^{10}$, $COR^{10}$, $-SR^{10}$, $-S(O)_tR^{15}$ wobei t 0, 1 oder 2 ist, $-N(R^{10})_2$, $-NO_2$, $-OC(O)R^{10}$, $CO_2R^{10}$, $-OCO_2R^{15}$, -CN, $-NR^{10}COOR^{15}$, $-SR^{15}C(O)OR^{15}$, $-SR^{15}N(R^{13})_2$ mit der Maßgabe, dass $R^{15}$ in $-SR^{15}N(R^{13})_2$ nicht $-CH_2$ ist, and wobei jedes $R^{13}$ unabhängig ausgewählt ist aus H oder $-C(O)OR^{15}$, Benzotriazol-1-yloxy, Tetrazol-5-ylthio oder substituiertem Tetrazol-5-ylthio, Alkinyl, Alkenyl oder Alkyl, wobei die Alkyl- oder Alkenylgruppe gegebenenfalls mit Halogen, $-OR^{10}$ oder $-CO_2R^{10}$ substituiert ist;
$R^3$ und $R^4$ gleich oder unterschiedlich sind und jeweils unabhängig für H oder irgendwelche der Substituenten von $R^1$ und $R^2$ stehen;
$R^5$, $R^6$, $R^7$ und $R^{7a}$ jeweils unabhängig für H, $-CF_3$, $-COR^{10}$, Alkyl oder Aryl stehen, wobei das Alkyl oder Aryl gegebenenfalls mit $-OR^{10}$, $-SR^{10}$, $-S(O)_tR^{15}$, $-NR^{10}COOR^{15}$, $-N(R^{10})_2$, $-NO_2$, $-C(O)R^{10}$, $-OCOR^{10}$, $-OCO_2R^{15}$, $-CO_2R^{10}$, $OPO_3R^{10}$ substituiert ist, oder $R^5$ mit $R^6$ kombiniert ist, um =O oder =S wiederzugeben;
$R^8$ ausgewählt ist aus:

H, 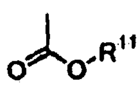 , 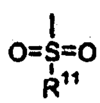 , 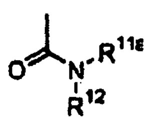 oder 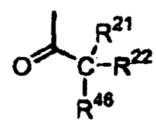 ;

(2.0) (3.0) (4.0) (5.0)

$R^9$ ausgewählt ist aus:

(1) Heteroaryl;
(2) substituiertem Heteroaryl;
(3) Heterocycloalkyl
(4) substituiertem Heterocycloalkyl;
(5) Heterocycloalkylalkyl;
(6) substituiertem Heterocycloalkylalkyl;
(7) Heteroarylalkyl;
(8) substituiertem Heteroarylalkyl;
(9) Heteroarylalkenyl;
(10) substituiertem Heteroarylalkenyl;
(11) Heteroarylalkinyl und
(12) substituiertem Heteroarylalkinyl;

wobei die substituierten $R^9$-Gruppen mit einem oder mehreren Substituenten substituiert sind, die ausgewählt sind aus:

(1) -OH;
(2) $-CO_2R^{14}$;
(3) $-CH_2OR^{14}$,
(4) Halogen;
(5) Alkyl;
(6) Amino;
(7) Trityl;
(8) Heterocycloalkyl
(9) Cycloalkyl;
(10) Arylalkyl;
(11) Heteroaryl;
(12) Heteroarylalkyl und
(13)

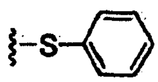

wobei $R^{14}$ unabhängig ausgewählt ist aus H Alkyl, Aryl, Arylalkyl, Heteroaryl und Heteroarylalkyl;
$R^{9a}$ ausgewählt ist aus Alkyl oder Arylalkyl;
$R^{10}$ ausgewählt ist aus H, Alkyl, Aryl oder Arylalkyl;
$R^{11}$ ausgewählt ist aus:

(1) Alkyl;
(2) substituiertem Alkyl;
(3) Aryl;
(4) substituiertem Aryl;
(5) Cycloalkyl;
(6) substituiertem Cycloalkyl;
(7) Heteroaryl;
(8) substituiertem Heteroaryl;
(9) Heterocycloalkyl und
(10) substituiertem Heterocycloalkyl;

wobei die substituierten $R^{11}$-Gruppen 1, 2 oder 3 Substituenten haben, die ausgewählt sind aus:

(1) -OH;
(2) Halogen und
(3) Alkyl;

$R^{11a}$ ausgewählt ist aus:

(1) H;
(2) OH;
(3) Alkyl;
(4) substituiertem Alkyl;
(5) Aryl;
(6) substituiertem Aryl;
(7) Cycloalkyl;
(8) substituiertem Cycloalkyl;
(9) Heteroaryl;
(10) substituiertem Heteroaryl;
(11) Heterocycloalkyl und
(12) substituiertem Heterocycloalkyl;

wobei die substituierten $R^{11a}$-Gruppen einen oder mehrere Substituenten aufweisen, die ausgewählt sind aus:

(1) -OH;
(2) -CN;
(3) $-CF_3$;
(4) Halogen;
(5) Alkyl;
(6) Cycloalkyl;
(7) Heterocycloalkyl
(8) Arylalkyl;
(9) Heteroarylalkyl;
(10) Alkenyl und
(11) Heteroalkenyl;

$R^{12}$ ausgewählt ist aus H oder Alkyl;
$R^{15}$ ausgewählt ist aus Alkyl oder Aryl;
$R^{21}$, $R^{22}$ und $R^{46}$ unabhängig ausgewählt sind aus:

(1) -H;
(2) Alkyl;
(3) Aryl;
(4) substituiertem Aryl, das gegebenenfalls mit einem oder mehreren Substituenten substituiert ist, die ausgewählt sind aus Alkyl, Halogen, $CF_3$ oder OH;
(5) Cycloalkyl;
(6) substituiertem Cycloalkyl, das gegebenenfalls mit einem oder mehreren Substituenten substituiert ist, die ausgewählt sind aus Alkyl, Halogen, $CF_3$ oder OH;
(7) Heteroaryl mit der Formel

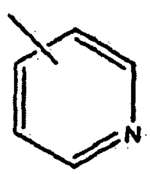
und
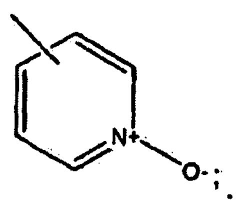

(8) Heterocycloalkyl mit der Formel

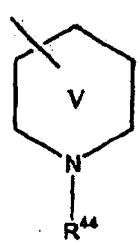

wobei $R^{44}$ ausgewählt ist aus:

(1) -H;
(2) Alkyl;
(3) Alkylcarbonyl;
(4) Alkyloxycarbonyl;
(5) Halogenalkyl und
(6) -C(O)-NH($R^{51}$);

wobei wenn $R^{21}$, $R^{22}$ oder $R^{46}$ das Heterocycloalkyl mit der obigen Formel ist, dann ist Ring V wie folgt:

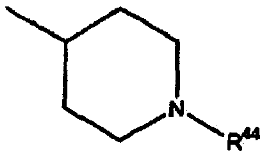 , 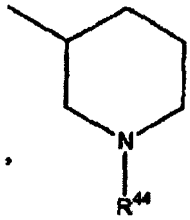 , 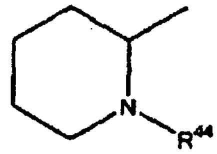

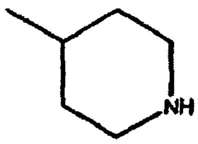 , 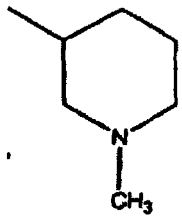 , 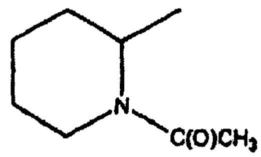 ,

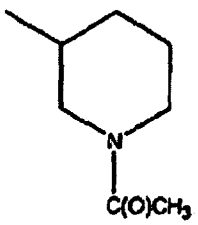 , 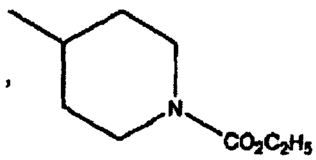 , 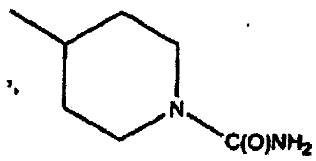

und 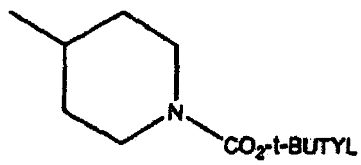

$R^{26}$ ausgewählt ist aus:

(1) -H;
(2) Alkyl;
(3) Alkoxyl;
(4) $-CH_2-CN$;
(5) $R^9$;
(6) $-CH_2CO_2H$;
(7) -C (O) -Alkyl und
(8) $CH_2CO_2$alkyl;

$R^{27}$ ausgewählt ist aus:

(1) -H;
(2) -OH;
(3) Alkyl und
(4) Alkoxy;

$R^{27a}$ ausgewählt ist aus:

(1) Alkyl oder
(2) Alkoxy;

$R^{30}$ bis $R^{33}$ unabhängig ausgewählt ist aus:

(1) -H;
(2) -OH;
(3) =O;
(4) Alkyl;
(5) Aryl und
(6) Arylalkyl;

$R^{50}$ ausgewählt ist aus:

(1) Alkyl;
(2) Heteroaryl;
(3) substituiertem Heteroaryl und
(4) Amino;

wobei die Substituenten an den substituierten $R^{50}$-Gruppen unabhängig ausgewählt sind aus Alkyl, Halogen oder -OH;
$R^{50a}$ ausgewählt ist aus:

(1) Heteroaryl;
(2) substituiertem Heteroaryl und
(3) Amino;

$R^{51}$ ausgewählt ist aus -H oder Alkyl.

**2.** Verbindung nach Anspruch 1 mit der Struktur:

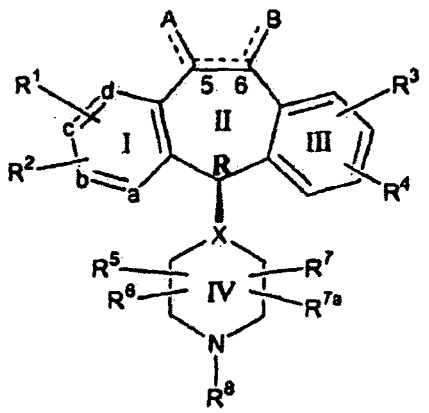

(1.0A)

worin:

X = CH oder N;
B H ist, wenn die optionale Bindung zwischen C-5 und C-6 vorhanden ist, und wenn die optionale Bindung zwischen C-5 und C-6 fehlt, dann ist jedes B H,
oder mit der Struktur:

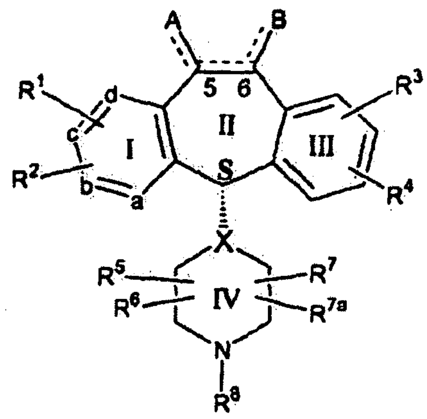

(1.0B)

worin:

X = CH oder N;
A H ist, wenn die optionale Bindung zwischen C-5 und C-6 vorhanden ist, und wenn die optionale Bindung zwischen C-5 und C-6 fehlt, dann jedes A H ist.

**3.** Verbindung nach Anspruch 1, worin
$R^1$ bis $R^4$ jeweils unabhängig ausgewählt sind aus H oder Halogen;
$R^5$ bis $R^7$ H sind;
a N ist und die restlichen b, c und d Kohlenstoff sind, oder a, b, c und d Kohlenstoff sind, und
$R^8$ Gruppe 2.0 oder 4.0 ist.

**4.** Verbindung nach Anspruch 2 mit der Formel (1.0A), wobei
a N ist und die restlichen b, c und d Kohlenstoff sind, und
$R^1$ bis $R^4$ jeweils unabhängig ausgewählt sind aus H, Br oder Cl.

**5.** Verbindung nach Anspruch 1, worin

(1) $R^{11}$ ausgewählt ist aus Alkyl, Cycloalkyl oder substituiertem Cycloalkyl, wobei die substituierten Gruppen mit Halogen, Alkyl oder Amino substituiert sind;
(2) $R^{11a}$ ausgewählt ist aus Alkyl, Aryl, substituiertem Aryl, Cycloalkyl oder substituiertem Cycloalkyl, wobei die substituierten Gruppen mit Halogen, -CN oder $CF_3$ substituiert sind;
(3) $R^{12}$, $R^{21}$ und $R^{22}$ H sind, und
(4) $R^{46}$ ausgewählt ist aus: Aryl, substituiertem Aryl, Heteroaryl mit der Formel:

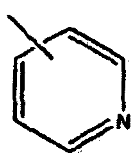

und

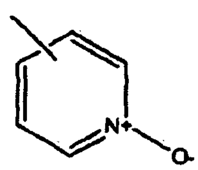

oder
Heterocycloalkyl mit der Formel

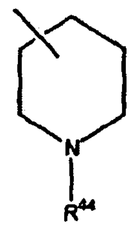

wobei die substituierten Gruppen mit Alkyl, Alkylcarbonyl oder Halogenalkyl substituiert sind;
(5) $R^{44}$ ausgewählt ist aus H oder $-C(O)NH_2$;
(6) $R^8$ ausgewählt ist aus:

(a) Gruppe 2.0, wobei $R^{11}$ ausgewählt ist aus t-Butyl oder Cyclohexyl,
(b) Gruppe 3.0, wobei $R^{11}$ ausgewählt ist aus Methyl oder t-Butyl;
(c) Gruppe 4.0, wobei $R^{12}$ H ist und $R^{11a}$ ausgewählt ist aus t-Butyl, Cyanophenyl, Chlorphenyl, Fluorphenyl oder Cyclohexyl;
(d) Gruppe 5.0, wobei $R^{21}$ und $R^{22}$ H sind und $R^{46}$ ausgewählt ist aus:

(1) Heteroaryl mit der Formel:

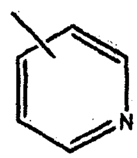

und

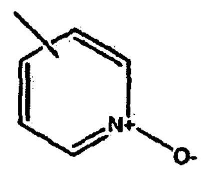

oder
(2) Heterocycloalkyl mit der Formel:

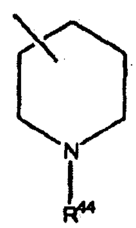

und wobei $R^{44}$ $-C(O)NH_2$ ist.

**6.** Verbindung nach Anspruch 5, bei der $R^8$ Gruppe 4.0 ist.

**7.** Verbindung nach Anspruch 1, bei der einer von A und B H ist und der andere $R^9$ ist.

**8.** Verbindung nach Anspruch 1, bei der $R^9$ ausgewählt ist aus:

(1) Heterocycloalkylalkyl mit der Formel $-(CH_2)_n$-Heterocycloalkyl;
(2) substituiertem Heterocycloalkylalkyl mit der Formel $-(CH_2)_n$-substituiertem Heterocycloalkyl;
(3) Heteroarylalkyl mit der Formel $-(CH_2)_n$-Heteroaryl und
(4) substituiertem Heteroarylalkyl mit der Formel $-(CH_2)_n$-substituiertem Heteroaryl;

wobei n 1, 2 oder 3 ist und die Substituenten der substituierten $R^9$-Gruppen jeweils unabhängig ausgewählt sind aus:

(1) -OH;

(2) $-CO_2R^{14}$;
(3) $-CH_2OR^{14}$,
(4) Halogen;
(5) Alkyl;
(6) Amino;
(7) Trityl;
(8) Heterocycloalkyl
(9) Arylalkyl;
(10) Heteroaryl und
(11) Heteroarylalkyl;

worin $R^{14}$ unabhängig ausgewählt ist aus H oder Alkyl.

**9.** Verbindung nach Anspruch 8, bei der $R^9$

(1) $-(CH_2)_n$-Imidazolyl;
(2) $-(CH_2)_n$-substituiertes Imidazolyl;
(3) $-(CH_2)_n$-Morpholinyl;
(4) $-(CH_2)_n$-substituiertes Morpholinyl;
(5) $-(CH_2)_n$-Piperazinyl oder
(6) $-(CH_2)_n$-substituiertes Piperazinyl ist,

wobei n 1, 2 oder 3 ist.

**10.** Verbindung nach Anspruch 1, bei der die optionale Bindung zwischen C-5 und C-6 vorhanden ist und A H ist und B $R^9$ ist, oder A $R^9$ ist und B H ist; oder die optionale Bindung zwischen C-5 und C-6 fehlt und jedes A H ist, ein B H ist und das andere B $R^9$ ist, oder ein A H ist, das andere A $R^9$ ist und jedes B H ist; $R^1$ bis $R^4$ unabhängig H oder Halogen sind; $R^5$ bis $R^{7a}$ H sind, a N ist und die restlichen b, c und d-Substituenten Kohlenstoff sind; X N oder CH ist und $R^8$ Gruppe 2.0 oder 4.0 ist.

**11.** Verbindung nach Anspruch 10, bei der $R^9$ ausgewählt ist aus:

(1) Heteroaryl;
(2) substituiertem Heteroaryl;
(3) Heterocycloalkyl
(4) substituiertem Heterocycloalkyl;
(5) Heterocycloalkylalkyl;
(6) substituiertem Heterocycloalkylalkyl;
(7) Heteroarylalkyl;
(8) substituiertem Heteroarylalkyl;
(9) Heteroarylalkenyl und
(10) substituiertem Heteroarylalkenyl;

wobei die Substituenten der substituierten $R^9$-Gruppen jeweils unabhängig ausgewählt sind aus:

(1) -OH;
(2) $-CO_2R^{14}$;
(3) $-CH_2OR^{14}$,
(4) Halogen;
(5) Alkyl;
(6) Amino;
(7) Trityl;
(8) Heterocycloalkyl;
(9) Arylalkyl;
(10) Heteroaryl und
(11) Heteroarylalkyl;

worin $R^{14}$ unabhängig ausgewählt ist aus H oder Alkyl.

**12.** Verbindung nach Anspruch 11, bei der $R^9$ ausgewählt ist aus:

(1) Heterocycloalkylalkyl mit der Formel $-(CH_2)_n$-Heterocycloalkyl;
(2) substituiertem Heterocycloalkylalkyl mit der Formel $-(CH_2)_n$-substituiertem Heterocycloalkyl;
(3) Heteroarylalkyl mit der Formel $-(CH_2)_n$-Heteroaryl und
(4) substituiertem Heteroarylalkyl mit der Formel $-(CH_2)_n$-substituiertem Heteroaryl;

wobei die Substituenten der substituierten $R^9$-Gruppen jeweils unabhängig ausgewählt sind aus:

(1) -OH;
(2) $-CO_2R^{14}$;
(3) $-CH_2OR^{14}$,
(4) Halogen;
(5) Alkyl;
(6) Amino;
(7) Trityl;
(8) Heterocycloalkyl;
(9) Arylalkyl;
(10) Heteroaryl und
(11) Heteroarylalkyl.

**13.** Verbindung nach Anspruch 12, bei der $R^8$ Gruppe 4.0 ist und $R^{12}$ H ist und $R^{11a}$ ausgewählt ist aus:

(1) Alkyl;
(2) Aryl;
(3) substituiertem Aryl;
(4) Cycloalkyl und
(5) substituiertem Cycloalkyl;

wobei die Substituenten der substituierten Gruppen ausgewählt sind aus:

(1) Halogen;
(2) -CN oder
(3) $-CF_3$.

**14.** Verbindung nach Anspruch 12, bei der $R^9$

(1) $-(CH_2)_n$-Imidazolyl;
(2) $-(CH_2)_n$-substituiertes Imidazolyl;
(3) $-(CH_2)_n$-Morpholinyl;
(4) $-(CH_2)_n$-substituiertes Morpholinyl;
(5) $-(CH_2)_n$-Piperazinyl oder
(6) $-(CH_2)_n$-substituiertes Piperazinyl ist,

wobei n 1, 2 oder 3 ist.

**15.** Verbindung nach Anspruch 14, bei der die optionale Bindung vorhanden ist.

**16.** Verbindung nach Anspruch 15,bei der $R^8$ 4.0 ist und $R^{12}$ H ist und $R^{11a}$ ausgewählt ist aus:

(1) Alkyl;
(2) Aryl;
(3) substituiertem Aryl;
(4) Cycloalkyl und
(5) substituiertem Cycloalkyl;

wobei die Substituenten der substituierten Gruppen ausgewählt sind aus:

(1) Halogen;
(2) Cyano und
(3) -$CF_3$.

**17.** Verbindung nach Anspruch 16, bei der $R^8$ 4.0 ist, $R^{12}$ H ist und $R^{11a}$ substituiertes Phenyl ist, und wobei der Substituent der substituierten Gruppe ausgewählt ist aus:

(1) -CN oder
(2) -$CF_3$.

**18.** Verbindung nach Anspruch 14, bei der die optionale Bindung fehlt.

**19.** Verbindung nach Anspruch 1, die aus irgendeinem der Beispiele 1 bis 505 ausgewählt ist.

**20.** Verbindung nach Anspruch 1, die ausgewählt ist aus der Gruppe bestehend aus:

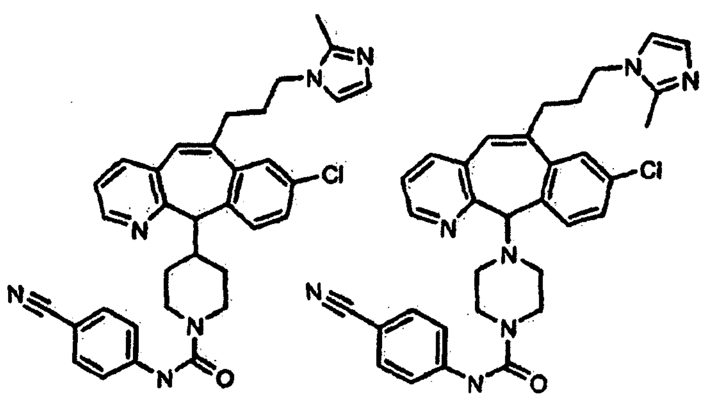

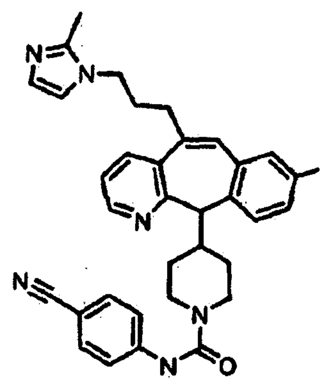

(139) (628) (699)

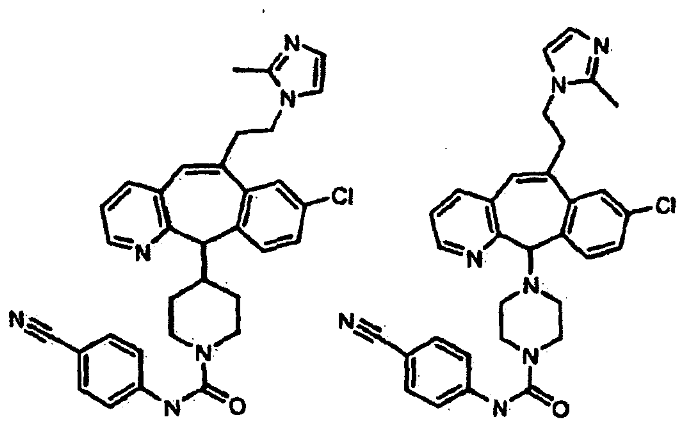

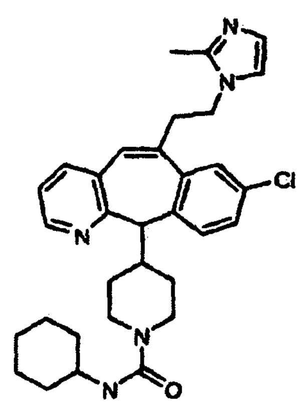

(326) (644) (332)

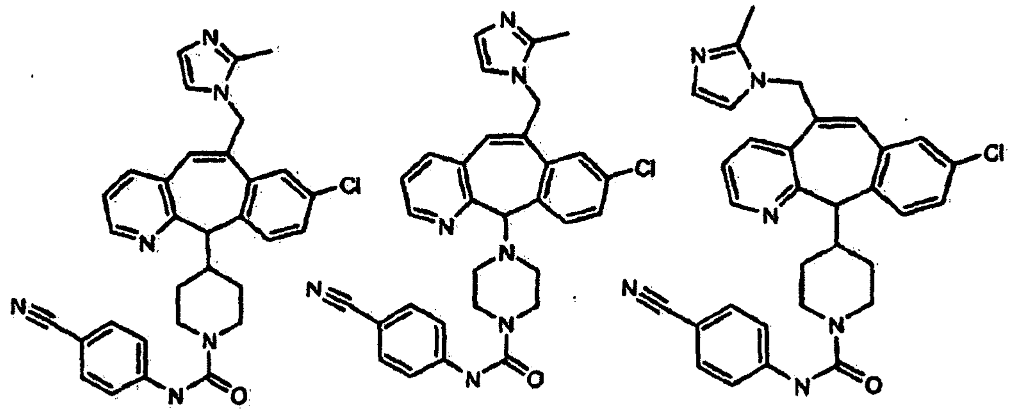

(362a) (372) (230)

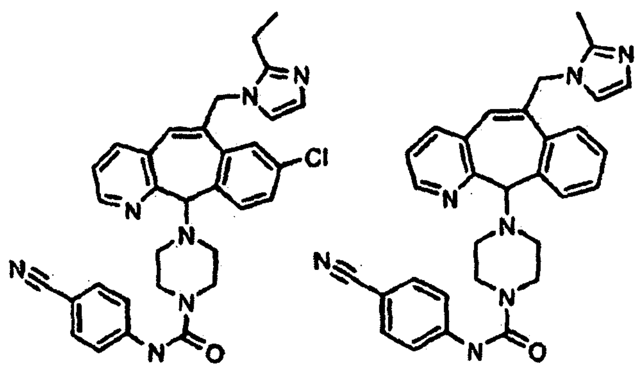

(378) (690)

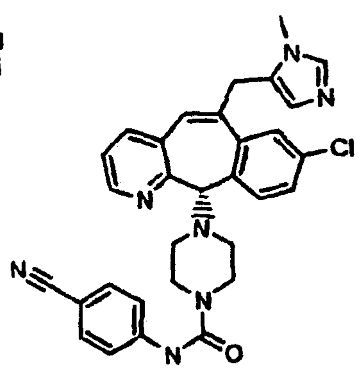

(784)

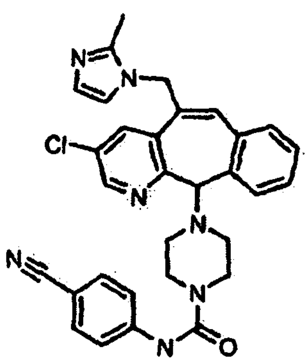

(684)

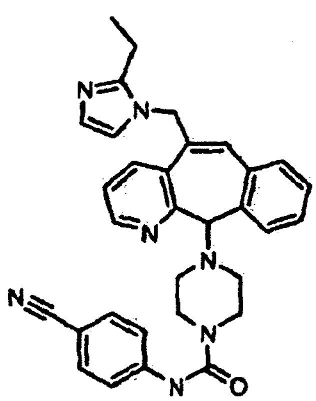

(688)

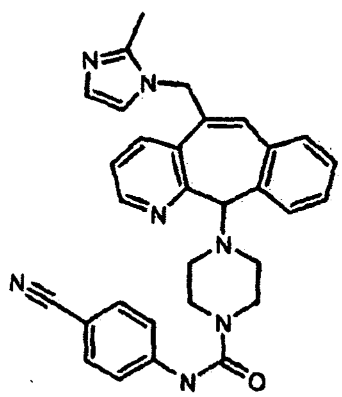

(686)

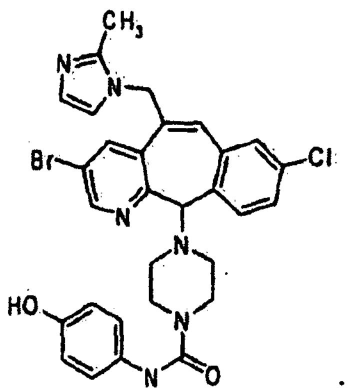

;

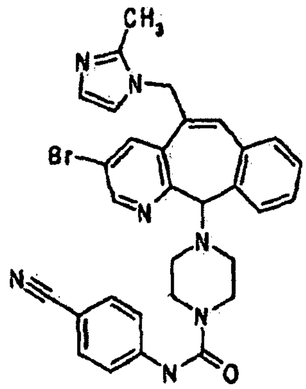

;

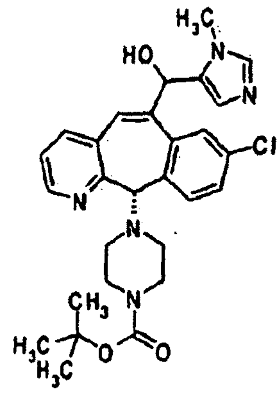

(683.2) (877) (790)

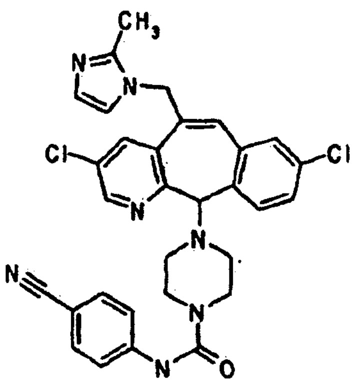

;

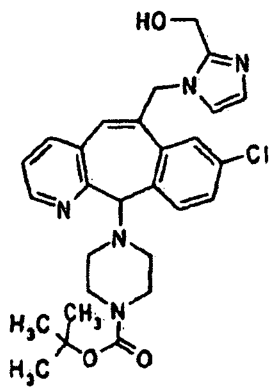

;

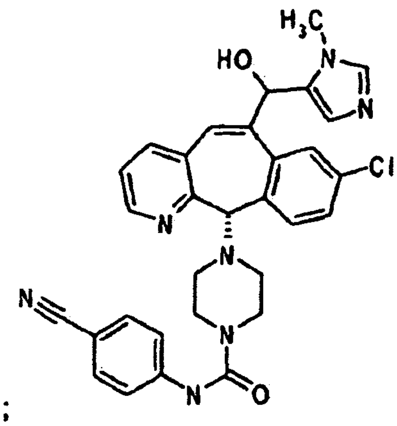

;

(816) (788) (793)

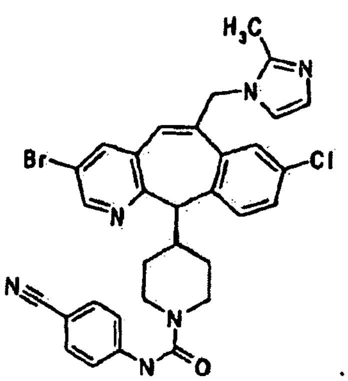

;

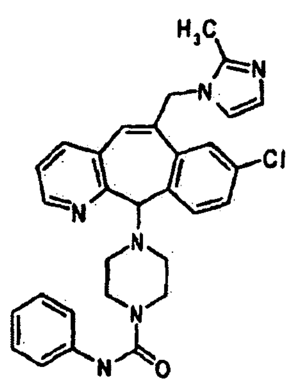

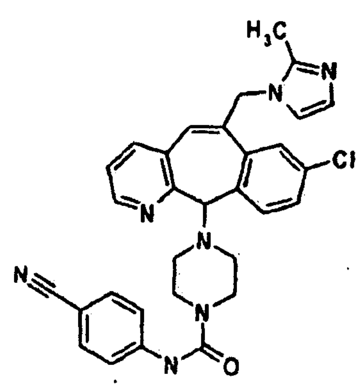

.

(778) (375.1) (372)

und

**21.** Pharmazeutische Zusammensetzung, die eine wirksame Menge einer Verbindung nach einem der Ansprüche 1 bis 20 in Kombination mit einem pharmazeutisch annehmbaren Träger enthält.

**22.** Verwendung einer Verbindung nach einem der Ansprüche 1 bis 20 zur Herstellung eines Medikaments in einer Kombinationstherapie zur Behandlung einer proliferierenden Erkrankung, wobei die Verbindung und mindestens ein chemotherapeutisches Mittel gleichzeitig oder sequentiell verabreicht werden.

**23.** Verwendung nach Anspruch 22, bei der das chemotherapeutische Mittel ein antineoplastisches Mittel ausgewählt aus der Gruppe bestehend aus Uracil-Senfgas, Chlormethin, Cyclophosphamid, Ifosfamid, Melphalan, Chlorambucil, Pipobroman, Triethylenmelamin, Triethylenthiophosphoramin, Busulfan, Carmustin, Lomustin, Streptozocin, Dacarbazin, Temozolomid, Methotrexat, 5-Fluoruracil, Floxuridin, Cytarabin, 6-Mercaptopurin, 6-Thioguanin, Fludarabinphosphat, Pentostatin, Gemcitabin, Vinblastin, Vincristin, Vindesin, Bleomycin, Dactinomycin, Daunorubicin, Doxorubicin, Epirubicin, Idarubicin, Paclitaxel (Taxol), Mithramycin, Deoxycoformycin, Mitomycin-C, L-Asparaginase, Interferonen, Etoposid, Teniposid, 17$\alpha$-Ethinylestradiol, Diethylstilbestrol, Testosteron, Prednison, Fluoxymesteron, Dromostanolonpropionat, Testolacton, Megestrolacetat, Tamoxifen, Methylprednisolon, Methyltestosteron, Prednisolon, Triamcinolon, Chlorotrianisen, Hydroxyprogesteron, Aminoglutethimid, Estramustin, Medroxyprogesteronacetat, Leuprolid, Flutamid, Toremifen, Goserelin, Cisplatin, Carboplatin, Hydroxyharnstoff, Amsacrin, Procarbazin, Mitotan, Mitoxantron, Levamisol, Navelben, CPT-11, Anastrazol, Letrazol, Capecitabin, Reloxafin, Droloxifin und Hexamethylmelamin ist.

**24.** Verwendung nach Anspruch 22, bei der das chemotherapeutische Mittel ein Mikrotubuli beeinflussendes Mittel ausgewählt aus Allocolchicin, Halichondrin B, Colchicin, Colchicinderivativen, Dolastatin 10, Maytansin, Rhizoxin, Paclitaxel, Paclitaxelderivativen, Thiocolchicin, Tritylcystein, Vinblastinsulfat, Vincristinsulfat, Epothilon A, Epothilon, Discodermolid, Estramustin, Nocodazol und MAP4 ist.

**25.** Verwendung einer Verbindung nach einem der Ansprüche 1 bis 20 zur Herstellung eines Medikaments in einer Kombinationstherapie zur Behandlung einer proliferierenden Erkrankung, wobei die Verbindung und mindestens ein Signalübertragungsinhibitor gleichzeitig oder sequentiell verabreicht werden.

**26.** Verwendung nach Anspruch 25, bei der der Signalübertragungsinhibitor ausgewählt ist aus dem bcr/abl-Inhibitor Gleevec, den epidermalen Wachstumsfaktorrezeptorinhibitoren, Iressa, OSI-774, Imclone C225 und Abgenix ABX-EGF und dem her-2/neu-Rezeptorinhibitor Herceptin.

## Revendications

**1.** Composé de formule :

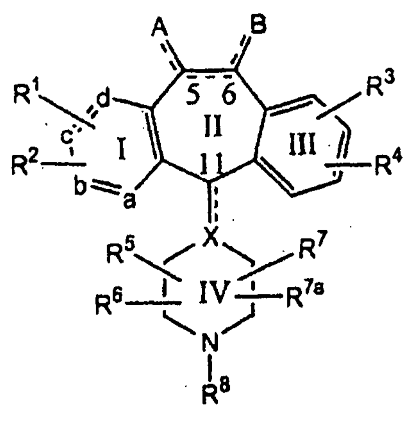

(1.0)

ou sel ou solvat pharmacologiquement admissible d'un tel composé,
dans laquelle formule :

- l'un des symboles a, b, c et d représente un atome d'azote N ou un groupe nitroso $N^+O^-$ et les symboles a, b, c et d restants représentent chacun un atome de carbone, et chacun de ces atomes de carbone porte un

substituant symbolisé par $R^1$ ou $R^2$, qui lui est lié,

- ou bien chacun des symboles a, b, c et d représente un atome de carbone, et chacun de ces atomes de carbone porte un substituant symbolisé par $R^1$ ou $R^2$, qui lui est lié;
- les traits en tiretés (---) représentent des liaisons optionnelles;
- X représente un atome d'azote N ou un groupe CH si la liaison optionnelle est absente, et représente un atome de carbone C si la liaison optionnelle est présente;
- si la liaison optionnelle entre les atomes de carbone 5 et 6 est présente, il n'y a qu'un seul substituant représenté par A et lié à l'atome de carbone 5 et il n'y a qu'un seul substituant représenté par B et lié à l'atome de carbone 6, et A ou B représente autre chose qu'un atome d'hydrogène ;
- si la liaison optionnelle entre les atomes de carbone 5 et 6 n'est pas présente, il y a deux substituants représentés par A et liés à l'atome de carbone 5 et il y a deux substituants représentés par B et liés à l'atome de carbone 6, et au moins l'un des deux substituants représentés par A ou l'un des deux substituants représentés par B est un atome d'hydrogène, et au moins l'un des deux substituants représentés par A ou l'un des deux substituants représentés par B est autre chose qu'un atome d'hydrogène ;
- A et B représentent des entités choisies indépendamment parmi les suivantes :

(1) un atome d'hydrogène H;
ou un groupe symbolisé par
(2)-$R^9$;
(3) -$R^9$-C(O)-$R^9$;
(4) -$R^9$-$CO_2$-$R^{9a}$;
(5) -C(O)NH$R^9$ ;
(6) -C(O)NH-$CH_2$C(O)-$NH_2$ ;
(7) -C(O)NH$R^{26}$ ;
(8) -$(CH_2)_p(R^9)_2$ où les symboles $R^9$ représentent des entités identiques ou différentes;
(9) -$(CH_2)_p$C(O)$R^9$ ;
(10) -$(CH_2)_p$C(O)$R^{27a}$ ;
(11) -$(CH_2)_p$C(O)N$(R^9)_2$ où les symboles $R^9$ représentent des entités identiques ou différentes ;
(12) -$(CH_2)_p$C(O)NH($R^9$) ;
(13) -$(CH_2)_p$NHC(O)$R^{50}$ ;
(14) -$(CH_2)_p$NH$CO_2R^{50}$ ;
(15) -$(CH_2)_p$N[C(O)$R^{27a}]_2$ où les symboles $R^{27a}$ représentent des entités identiques ou différentes ;
(16) -$(CH_2)_p$N$R^{51}$C(O)$R^{27}$ où $R^{51}$ et $R^{27}$ peuvent représenter des entités qui forment, avec les atomes auxquels elles sont liées, un cycle hétérocycloalkyle comportant 5 ou 6 chaînons, sous réserve que, si $R^{51}$ et $R^{27}$ représentent des entités constituant un cycle, $R^{51}$ ne représente pas un atome d'hydrogène ;
(17) -$(CH_2)_p$N$R^{51}$C(O)N$R^{27}$ où $R^{51}$ et $R^{27}$ peuvent représenter des entités qui forment, avec les atomes auxquels elles sont liées, un cycle hétérocycloalkyle comportant 5 ou 6 chaînons, sous réserve que, si $R^{51}$ et $R^{27}$ représentent des entités constituant un cycle, $R^{51}$ ne représente pas un atome d'hydrogène;
(18) -$(CH_2)_p$N$R^{51}$C(O)N$(R^{27a})_2$ où les symboles $R^{27a}$ représentent des entités identiques ou différentes ;
(19) -$(CH_2)_p$NH$SO_2$N$(R^{51})_2$ où les symboles $R^{51}$ représentent des entités identiques ou différentes ;
(20) -$(CH_2)_p$NH$CO_2R^{50}$;
(21) -$(CH_2)_p$$CO_2R^{51}$;
(22) -NH$R^9$;
(23)

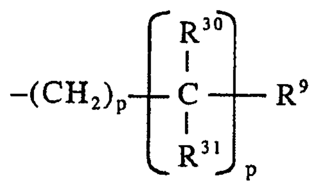

où les symboles $R^{30}$ et $R^{31}$ représentent des entités identiques ou différentes ;
(24)

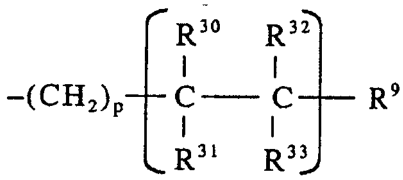

où les symboles $R^{30}$, $R^{31}$, $R^{32}$ et $R^{33}$ représentent des entités identiques ou différentes;

- l'indice p vaut 0, 1, 2, 3 ou 4 ;
- chacun des symboles $R^1$ et $R^2$ représente indépendamment une entité choisie parmi les atomes d'hydrogène et d'halogène, les groupes symbolisés par $-CF_3$, $-OR^{10}$, $-COR^{10}$, $-SR^{10}$, $-S(O)_tR^{15}$ où t représente un nombre qui vaut 0, 1 ou 2, $-N(R^{10})_2$, $-NO_2$, $-OC(O)R^{10}$, $-CO_2R^{10}$, $-OCO_2R^{15}$, -CN, $-NR^{10}COOR^{15}$, $-SR^{15}C(O)OR^{15}$ ou $-SR^{15}N(R^{13})_2$, dans lequel groupe $-SR^{15}N(R^{13})_2$ $R^{15}$ ne représente pas un groupe méthylène $-CH_2-$ et chaque symbole $R^{13}$ représente indépendamment un atome d'hydrogène ou un groupe symbolisé par $-C(O)OR^{15}$, et les groupes benzotriazol-1-yloxy, tétrazol-5-ylthio avec ou sans substituant, alcynyle, alcényle et alkyle, lesquels groupes alkyle et alcényle pouvant en option porter un substituant halogéno ou un substituant symbolisé par $-OR^{10}$ ou $-CO_2R^{10}$ ;
- les symboles $R^3$ et $R^4$ représentent des entités identiques ou différentes et représentent chacun, indépendamment, un atome d'hydrogène ou l'un des substituants mentionnés à propos des symboles $R^1$ et $R^2$ ;
- les symboles $R^5$, $R^6$, $R^7$ et $R^{7a}$ représentent chacun, indépendamment, un atome d'hydrogène, un groupe symbolisé par $-CF_3$ ou $-OR^{10}$, ou un groupe alkyle ou aryle, lequel groupe alkyle ou aryle peut en option porter un substituant symbolisé par $-OR^{10}$, $-SR^{10}$, $-S(O)_tR^{15}$, $-N(R^{10})_2$, $-NR^{10}COOR^{15}$, $-NO_2$, $-C(O)R^{10}$, $-OCOR^{10}$, $-CO_2R^{10}$, $-OCO_2R^{15}$, $-OPO_3R^{10}$, les symboles $R^5$ et $R^6$ pouvant aussi représenter ensemble un substituant oxo =O ou thioxo =S ;
- $R^8$ représente une entité choisie parmi un atome d'hydrogène et les groupes symbolisés par

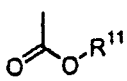 , 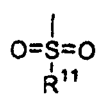 , 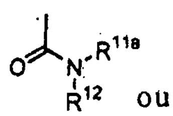 ou 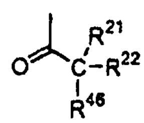

(2.0) (3.0) (4.0) (5.0)

- $R^9$ représente un groupe choisi parmi les suivants :

(1) hétéroaryle,
(2) hétéroaryle à substituant(s),
(3) hétérocycloalkyle,
(4) hétérocycloalkyle à substituant(s),
(5) hétérocycloalkyl-alkyle,
(6) hétérocycloalkyl-alkyle à substituant(s),
(7) hétéroaryl-alkyle,
(8) hétéroaryl-alkyle à substituant(s),
(9) hétéroaryl-alcényle,
(10) hétéroaryl-alcényle à substituant(s),
(11) hétéroaryl-alcynyle,
(12) et hétéroaryl-alcynyle à substituant(s),

parmi lesquels lesdits groupes à substituant(s) représentés par $R^9$ portent un ou plusieurs substituants choisis parmi les suivants :

(1) hydroxy -OH,
(2) substituants symbolisés par $-CO_2R^{14}$,
(3) substituants symbolisés par $-CH_2OR^{14}$,
(4) halogéno,
(5) alkyle,

(6) amino,
(7) trityle,
(8) hétérocycloalkyle,
(9) cycloalkyle,
(10) aryl-alkyle,
(11) hétéroaryle,
(12) hétéroaryl-alkyle,
(13) et phénylthio

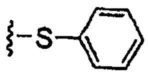

,

où $R^{14}$ représente indépendamment un atome d'hydrogène ou un groupe alkyle, aryle, aryl-alkyle, hétéroaryle ou hétéroaryl-alkyle ;
- $R^{9a}$ représente un groupe alkyle ou aryl-alkyle ;
- $R^{10}$ représente un atome d'hydrogène ou un groupe alkyle, aryle ou aryl-alkyle ;
- $R^{11}$ représente un groupe choisi parmi les suivants :

(1) alkyle,
(2) alkyle à substituant(s),
(3) aryle,
(4) aryle à substituant(s),
(5) cycloalkyle,
(6) cycloalkyle à substituant(s),
(7) hétéroaryle,
(8) hétéroaryle à substituant(s),
(9) hérérocycloalkyle,
(10) et hétérocycloalkyle à substituant(s),

parmi lesquels lesdits groupes à substituant(s) représentés par $R^{11}$ portent un ou plusieurs substituants choisis parmi les suivants :

(1) hydroxy -OH,
(2) halogéno,
(3) et alkyle ;

- $R^{11a}$ représente

(1) un atome d'hydrogène
ou un groupe
(2) hydroxyle -OH,
(3) alkyle,
(4) alkyle à substituant(s),
(5) aryle,
(6) aryle à substituant(s),
(7) cycloalkyle,
(8) cycloalkyle à substituant(s),
(9) hétéroaryle,
(10) hétéroaryle à substituant(s),
(11) hétérocycloalkyle,
(12) ou hétérocycloalkyle à substituant(s),

parmi lesquels lesdits groupes à substituant(s) représentés par $R^{11a}$ portent un ou plusieurs substituants choisis parmi les suivants :

(1) hydroxy -OH,

(2) cyano -CN,
(3) trifluorométhyle $-CF_3$,
(4) halogéno,
(5) alkyle,
(6) cycloalkyle,
(7) hétérocycloalkyle,
(8) aryl-alkyle,
(9) hétéroaryl-alkyle,
(10) alcényle,
(11) et hétéroalcényle ;

- $R^{12}$ représente un atome d'hydrogène ou un groupe alkyle ;
- $R^{15}$ représente un groupe alkyle ou aryle ;
- $R^{21}$, $R^{22}$ et $R^{46}$ représentent chacun, indépendamment, une entité choisie parmi les suivantes :

(1) un atome d'hydrogène,
et les groupes
(2) alkyle,
(3) aryle,
(4) aryle à substituant(s), portant au choix un ou plusieurs substituants choisis parmi les atomes d'halogène et les groupes alkyle, trifluorométhyle et hydroxyle,
(5) cycloalkyle,
(6) cycloalkyle à substituant(s), portant au choix un ou plusieurs substituants choisis parmi les atomes d'halogène et les groupes alkyle, trifluorométhyle et hydroxyle,
(7) hétéroaryle de formule

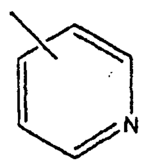 ou 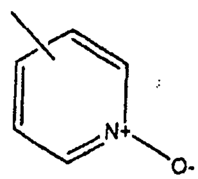 ,

(8) et hétérocycloalkyle de formule

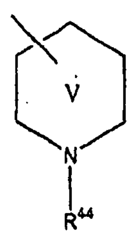

dans laquelle $R^{44}$ représente une entité choisie parmi :

(1) un atome d'hydrogène
les groupes
(2) alkyle,
(3) alkyl-carbonyle,
(4) alcoxy-carbonyle,
(5) et halogénoalkyle,
(6) et les groupes symbolisés par $-C(O)NH(R^{51})$,

étant entendu que, dans le cas où $R^{21}$, $R^{22}$ ou $R^{46}$ représente un groupe hétérocycloalkyle de formule indiquée ci-dessus, le cycle V est choisi parmi les suivants :

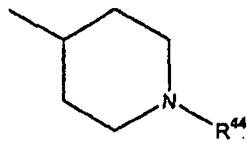, 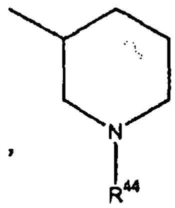, 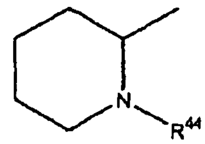

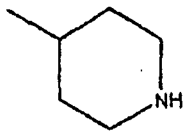, 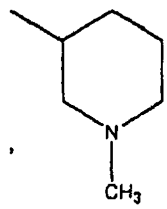, 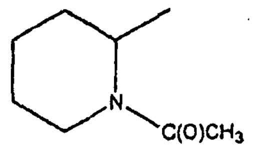,

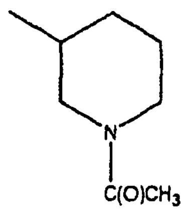, 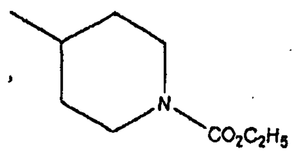, 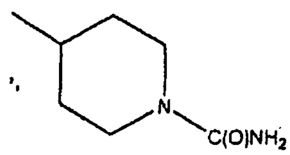

et 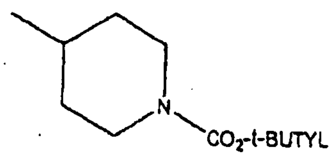 ;

- $R^{26}$ représente une entité choisie parmi:

(1) un atome d'hydrogène,
et les groupes
(2) alkyle,
(3) alcoxy,
(4) cyanométhyle $-CH_2-CN$,
(5) symbolisés par $R^9$,
(6) carboxyméthyle $-CH_2-CO_2H$,
(7) alkyl-carbonyle -C(O)-alkyle,
(8) et alcoxycarbonyl-méthyle $-CH_2-CO_2$-alkyle ;

- $R^{27}$ représente une entité choisie parmi :

(1) un atome d'hydrogène,
et les groupes
(2) hydroxyle -OH,
(3) alkyle,
(4) et alcoxy ;

- $R^{27a}$ représente une entité choisie parmi les groupes:

(1) alkyle,
(2) et alcoxy ;

- les symboles $R^{30}$ à $R^{33}$ représentent chacun, indépendamment, une entité choisie parmi

  (1) un atome d'hydrogène,
  (2) le groupe hydroxyle -OH,
  (3) le substituant oxo =O,
  et les groupes
  (4) alkyle,
  (5) aryle,
  (6) et aryl-alkyle ;

- $R^{50}$ représente une entité choisie parmi les groupes :

  (1) alkyle,
  (2) hétéroaryle,
  (3) hétéroaryle à substituant(s)
  (4) et amino,

où les substituants portés par lesdits groupes à substituant(s) représentés par $R^{50}$ sont choisis parmi les atomes d'halogène et les groupes alkyle et hydroxy -OH ;
- $R^{50a}$ représente une entité choisie parmi les groupes :

  (1) hétéroaryle,
  (2) hétéroaryle à substituant(s),
  (3) et amino ;

- et $R^{51}$ représente un atome d'hydrogène ou un groupe alkyle.

**2.** Composé conforme à la revendication 1, présentant la structure suivante :

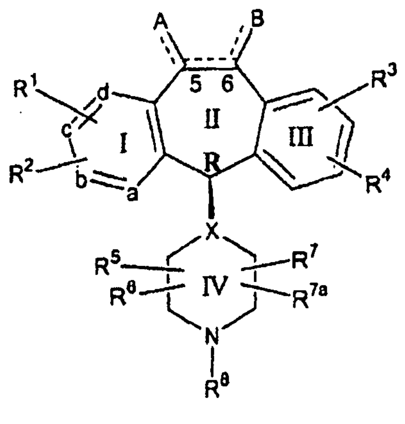

(1.0A)

dans laquelle

- X représente un atome d'azote N ou un groupe CH ;
- et B représente un atome d'hydrogène si la liaison optionnelle entre les atomes de carbone 5 et 6 est présente, mais si la liaison optionnelle entre les atomes de carbone 5 et 6 n'est pas présente, chacune des entités représentées par B est un atome d'hydrogène ;

ou présentant la structure suivante :

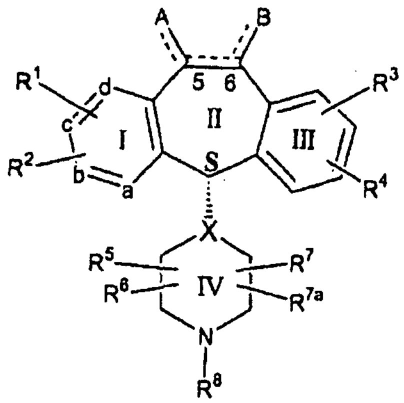

(1.0B)

dans laquelle

- X représente un atome d'azote N ou un groupe CH ;
- et A représente un atome d'hydrogène si la liaison optionnelle entre les atomes de carbone 5 et 6 est présente, mais si la liaison optionnelle entre les atomes de carbone 5 et 6 n'est pas présente, chacune des entités représentées par A est un atome d'hydrogène;

**3.** Composé conforme à la revendication 1, dans lequel:

- les symboles $R^1$ à $R^4$ représentent chacun, indépendamment, un atome d'hydrogène ou d'halogène,
- les symboles $R^5$ à $R^7$ représentent chacun un atome d'hydrogène,
- le symbole a représente un atome d'azote N et les symboles b, c et d restants représentent chacun un atome de carbone, ou bien chacun des symboles a, b, c et d représente un atome de carbone,
- et $R^8$ représente un groupe de formule (2.0) ou (4.0).

**4.** Composé conforme à la revendication 2, de formule (1.0A) dans laquelle :

- le symbole a représente un atome d'azote N et les symboles b, c et d restants représentent chacun un atome de carbone,
- et les symboles $R^1$ à $R^4$ représentent chacun, indépendamment, un atome d'hydrogène, de brome ou de chlore.

**5.** Composé conforme à la revendication 1, dans lequel :

1) $R^{11}$ représente une entité choisie parmi les groupes alkyle, cycloalkyle et cycloalkyle à substituant(s), lesquels groupes à substituant(s) portent un ou des substituant(s) halogéno, alkyle ou amino ;
2) $R^{11a}$ représente une entité choisie parmi les groupes alkyle, aryle, aryle à substituant(s), cycloalkyle et cycloalkyle à substituant(s), lesquels groupes à substituant(s) portent un ou des substituant(s) halogéno, cyano -CN ou trifluorométhyle $-CF_3$;
3) $R^{12}$, $R^{21}$ et $R^{22}$ représentent chacun un atome d'hydrogène;
4) $R^{46}$ représente une entité choisie parmi les groupes aryle, aryle à substituant(s), hétéroaryle de formule

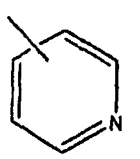

ou

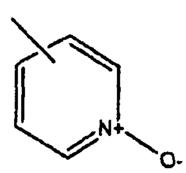

ou hétérocycloalkyle de formule

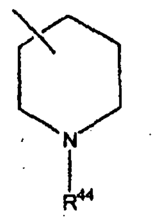

parmi lesquels lesditsgroupes à substituant(s) portent un ou des substituant(s) alkyle, alkyl-carbonyle ou halogénoalkyle ;

5) $R^{44}$ représente un atome d'hydrogène ou un groupe carbamyle $-C(O)NH_2$;

6) et $R^8$ représente une entité choisie parmi :

a) un groupe de formule (2.0) où $R^{11}$ représente un groupe tertiobutyle ou cyclohexyle,

b) un groupe de formule (3.0) où $R^{11}$ représente un groupe méthyle ou tertiobutyle,

c) un groupe de formule (4.0) où $R^{12}$ représente un atome d'hydrogène et $R^{11a}$ représente un groupe tertiobutyle, cyanophényle, chlorophényle, fluorophényle ou cyclohexyle,

d) et un groupe de formule (5.0) où $R^{21}$ et $R^{22}$ représentent chacun un atome d'hydrogène et $R^{46}$ représente un groupe

1) hétéroaryle de formule

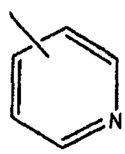

ou

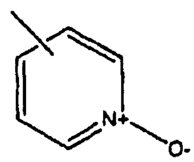

2) ou hétérocycloalkyle de formule

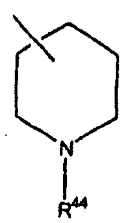

dans laquelle $R^{44}$ représente un groupe carbamyle $-C(O)NH_2$.

**6.** Composé conforme à la revendication 5, dans lequel $R^8$ représente un groupe de formule (4.0).

**7.** Composé conforme à la revendication 1, dans lequel l'un des symboles A et B représente un atome d'hydrogène et l'autre représente un groupe symbolisé par $R^9$.

**8.** Composé conforme à la revendication 1, dans lequel $R^9$ représente une entité choisie parmi les groupes :

(1) hétérocycloalkyl-alkyle, de formule $-(CH_2)_n$-hétérocycloalkyle,

(2) hétérocycloalkyl-alkyle à substituant(s), de formule $-(CH_2)_n$-(hétérocycloalkyle à substituant(s)),

(3) hétéroaryl-alkyle, de formule $-(CH_2)_n$-hétéroaryle,

(4) et hétéroaryl-alkyle à substituant(s), de formule $-(CH_2)_n$-(hétéroaryle à substituant (s)),

où l'indice n vaut 1, 2 ou 3 et les substituants portés par les groupes à substituant(s) mentionnés pour $R^9$ sont

chacun choisis, indépendamment, parmi les suivants :

(1) hydroxy -OH,
(2) substituants symbolisés par $-CO_2R^{14}$,
(3) substituants symbolisés par $-CH_2OR^{14}$,
(4) halogéno,
(5) alkyle,
(6) amino,
(7) tri tyle,
(8) hétérocycloalkyle,
(9) aryl-alkyle,
(10) hétéroaryle,
(11) et hétéroaryl-alkyle,

où $R^{14}$ représente indépendamment un atome d'hydrogène ou un groupe alkyle.

**9.** Composé conforme à la revendication 8, dans lequel $R^9$ représente un groupe de formule :

1) $-(CH_2)_n$-imidazolyle,
2) $-(CH_2)_n$-imidazolyle à substituant(s),
3) $-(CH_2)_n$-morpholinyle,
4) $-(CH_2)_n$-morpholinyle à substituant(s),
5) $-(CH_2)_n$-pipérazinyle,
6) ou $-(CH_2)_n$-ppérazinyle à substituant(s),

où l'indice n vaut 1, 2 ou 3.

**10.** Composé conforme à la revendication 1, dans lequel:

- la liaison optionnelle entre les atomes de carbone 5 et 6 est présente, et A représente un atome d'hydrogène et B représente un substituant symbolisé par $R^9$, ou A représente un substituant symbolisé par $R^9$ et B représente un atome d'hydrogène ;
- ou la liaison optionnelle entre les atomes de carbone 5 et 6 n'est pas présente, et chacun des substituants représentés par A est un atome d'hydrogène et l'un des substituants représentés par B est un atome d'hydrogène et l'autre est un substituant symbolisé par $R^9$, ou l'un des substituants représentés par A est un atome d'hydrogène et l'autre est un substituant symbolisé par $R^9$ et chacun des substituants représentés par B est un atome d'hydrogène ;
- les symboles $R^1$ à $R^4$ représentent chacun, indépendamment, un atome d'hydrogène ou d'halogène ;
- les symboles $R^5$ à $R^{7a}$ représentent chacun un atome d'hydrogène;
- le symbole a représente un atome d'azote N et les symboles b, c et d restants représentent chacun un atome de carbone ;
- X représente un atome d'azote N ou un groupe CH ;
- et $R^8$ représente un groupe de formule (2.0) ou (4.0).

**11.** Composé conforme à la revendication 10, dans lequel $R^9$ représente un groupe choisi parmi les suivants :

(1) hétéroaryle,
(2) hétéroaryle à substituant(s),
(3) hétérocycloalkyle,
(4) hétérocycloalkyle à substituant(s),
(5) hétérocycloalkyl-alkyle,
(6) hétérocycloalkyl-alkyle à substituant(s),
(7) hétéroaryl-alkyle,
(8) hétéroaryl-alkyle à substituant(s),
(9) hétéroaryl-alcényle,
(10) et hétéroaryl-alcényle à substituant(s),

parmi lesquels lesdits groupes à substituant(s) représentés par $R^9$ portent un ou plusieurs substituants choisis parmi

les suivants:

(1) hydroxy -OH,
(2) substituants symbolisés par $-CO_2R^{14}$,
(3) substituants symbolisés par $-CH_2OR^{14}$,
(4) halogéno,
(5) alkyle,
(6) amino,
(7) trityle,
(8) hétérocycloalkyle,
(9) aryl-alkyle,
(10) hétéroaryle,
(11) et hétéroaryl-alkyle,

où $R^{14}$ représente indépendamment un atome d'hydrogène ou un groupe alkyle.

**12.** Composé conforme à la revendication 11, dans lequel $R^9$ représente un groupe choisi parmi les suivants :

(1) hétérocycloalkyl-alkyle, de formule $-(CH_2)_n$-hétérocycloalkyle,
(2) hétérocycloalkyl-alkyle à substituant(s), de formule $-(CH_2)_n$-(hétérocycloalkyle à substituant(s)),
(3) hétéroaryl-alkyle, de formule $-(CH_2)_n$-hétéroaryle,
(4) et hétéroaryl-alkyle à substituant(s), de formule $-(CH_2)_n$-(hétéroaryle à substituant (s)),

où les substituants portés par les groupes à substituant(s) mentionnés pour $R^9$ sont chacun choisis, indépendamment, parmi les suivants :

(1) hydroxy -OH,
(2) substituants symbolisés par $-CO_2R^{14}$,
(3) substituants symbolisés par $-CH_2OR^{14}$,
(4) halogéno,
(5) alkyle,
(6) amino,
(7) trityle,
(8) hétérocycloalkyle,
(9) aryl-alkyle,
(10) hétéroaryle,
(11) et hétéroaryl-alkyle.

**13.** Composé conforme à la revendication 12, dans lequel $R^8$ représente un groupe de formule (4.0), $R^{12}$ représente un atome d'hydrogène et $R^{11a}$ représente un groupe choisi parmi les suivants :

(1) alkyle,
(2) aryle,
(3) aryle à substituant(s),
(4) cycloalkyle,
(5) et cycloalkyle à substituant(s),

où les substituants portés par lesdits groupes à substituant(s) sont choisis parmi les suivants :

(1) halogéno,
(2) cyano -CN,
(3) et trifluorométhyle $-CF_3$.

**14.** Composé conforme à la revendication 12, dans lequel $R^9$ représente un groupe de formule :

(1) $-(CH_2)_n$-imidazolyle,
(2) $-(CH_2)_n$-imidazolyle à substituant(s),
(3) $-(CH_2)_n$-morpholinyle,

(4) $-(CH_2)_n$-morpholinyle à substituant(s),
(5) $-(CH_2)_n$-pipérazinyle,
(6) ou $-(CH_2)_n$-ppérazinyle à substituant(s),

où l'indice n vaut 1, 2 ou 3.

**15.** Composé conforme à la revendication 14, dans lequel la liaison optionnelle est présente.

**16.** Composé conforme à la revendication 15, dans lequel $R^8$ représente un groupe de formule (4.0), $R^{12}$ représente un atome d'hydrogène et $R^{11a}$ représente un groupe choisi parmi les suivants:

(1) alkyle,
(2) aryle,
(3) aryle à substituant(s),
(4) cycloalkyle,
(5) et cycloalkyle à substituant(s),

où les substituants portés par lesdits groupes à substituant(s) sont choisis parmi les suivants :

(1) halogéno,
(2) cyano -CN,
(3) et trifluorométhyle $-CF_3$.

**17.** Composé conforme à la revendication 16, dans lequel $R^8$ représente un groupe de formule (4.0), $R^{12}$ représente un atome d'hydrogène et $R^{11a}$ représente un groupe phényle à substituant(s), où les substituants portés par ledit groupe à substituant(s) sont choisis parmi les suivants : (1) cyano -CN, et (2) trifluorométhyle $-CF_3$.

**18.** Composé conforme à la revendication 14, dans lequel la liaison optionnelle n'est pas présente.

**19.** Composé conforme à la revendication 1, qui est choisi parmi tous les composés décrits dans les exemples 1 à 505.

**20.** Composé conforme à la revendication 1, qui est choisi dans l'ensemble formé par les suivants :

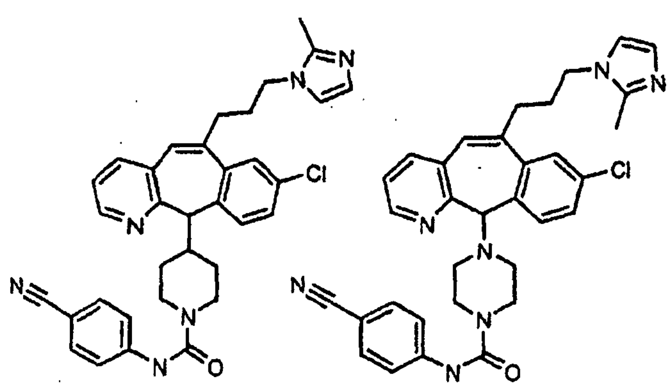

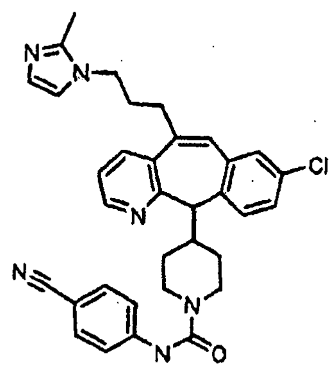

(139) (628) (699)

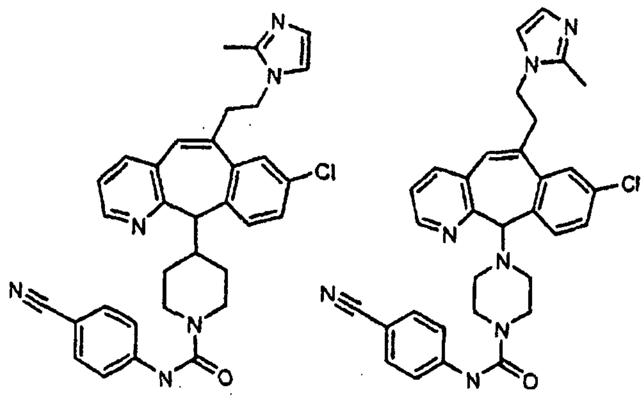

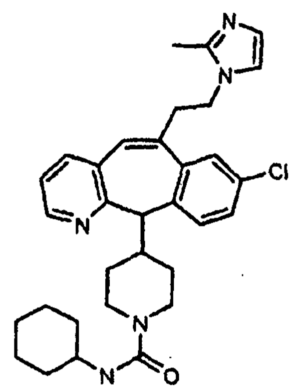

(326) (644) (332)

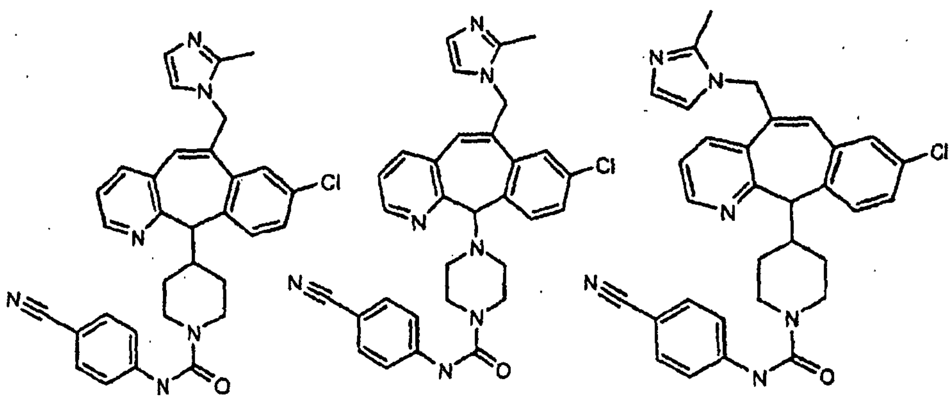

(362a) (372) (230)

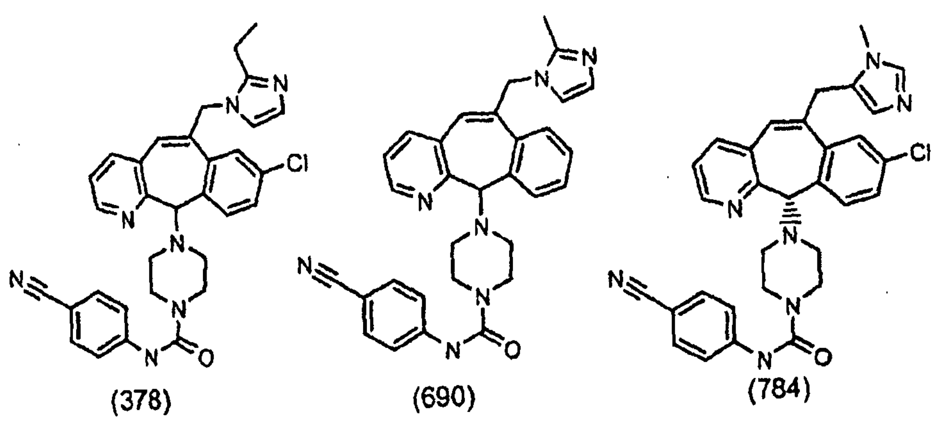

(378) (690) (784)

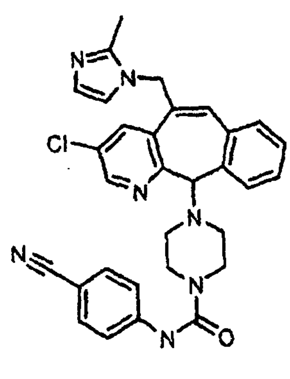

(684)

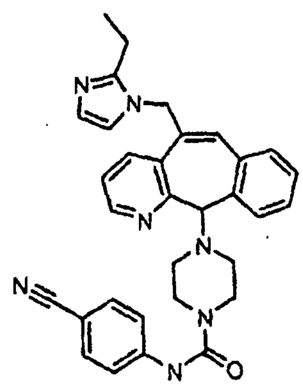

(688)

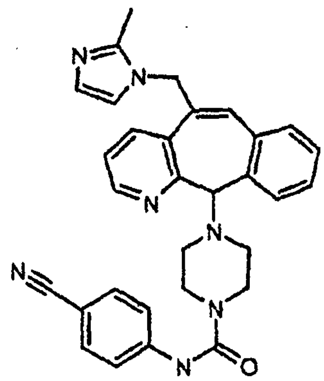

(686)

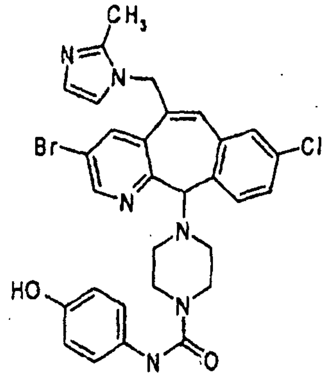

;

(683.2)

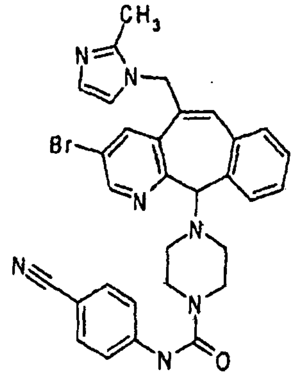

;

(877)

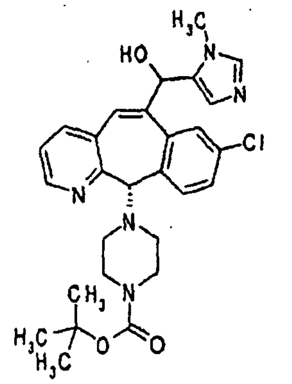

(790)

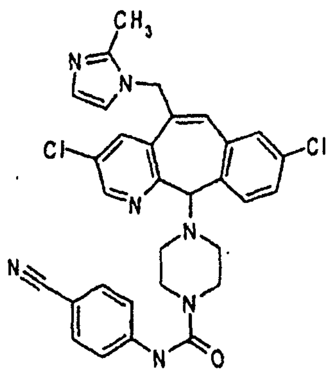

;

(816)

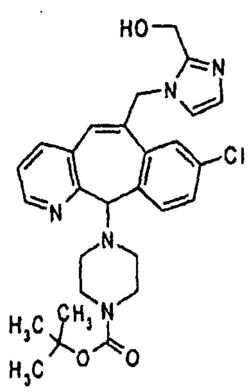

;

(788)

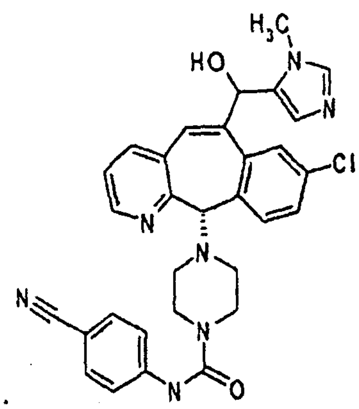

;

(793)

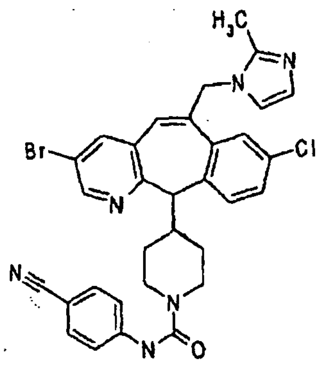 ; 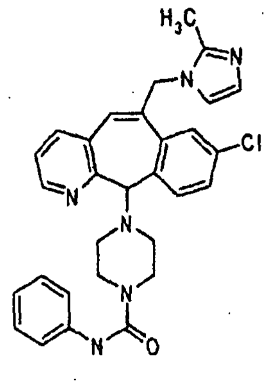 ; et 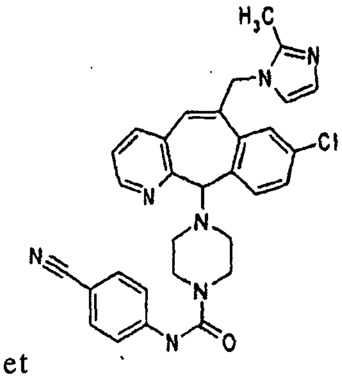 .

(778) (375.1) (372)

**21.** Composition pharmaceutique comprenant, en une quantité efficace, un composé conforme à l'une des revendications 1 à 20, en combinaison avec un véhicule pharmacologiquement admissible.

**22.** Emploi d'un composé conforme à l'une des revendications 1 à 20 en vue de la fabrication d'un médicament conçu pour le traitement d'une maladie proliférative par thérapie combinée, où l'on administrera simultanément ou consécutivement ledit composé et au moins un agent de chimiothérapie.

**23.** Emploi conforme à la revendication 22, ledit agent de chimiothérapie étant un agent antinéoplasique choisi parmi les suivants : moutarde à l'uracile, chlorméthine, cyclophosphamide, ifosfamide, melphalan, chlorambucil, pipobromane, triéthylènemélamine, triéthylènethiophosphoramine, busulfan, carmustine, lomustine, streptozocine, dacarbazine, témozolomide, méthotrexate, 5-fluoro-uracile, floxuridine, cytarabine, 6-mercaptopurine, 6-thioguanine, phosphate de fludarabine, pentostatine, gemcitabine, vinblastine, vincristine, vindésine, bléomycine, dactinomycine, daunorubicine, doxorubicine, épirubicine, idarubicine, paclitaxel (taxol), mithramycine, déoxycoformycine, mitomycine C, L-asparaginase, interférons, étoposide, téniposide, 17$\alpha$-éthynyl-oestradiol, diéthyl-stilbestrol, testostérone, prednisone, fluoxymestérone, propionate de dromostanolone, testolactone, acétate de mégestrol, tamoxifène, méthyl-prednisolone, méthyl-testostérone, prednisolone, triamcinolone, chlorotrianisène, hydroxyprogestérone, aminoglutéthimide, oestramustine, acétate de médroxyprogestérone, leuprolide, flutamide, torémifène, goséréline, cisplatine, carboplatine, hydroxy-urée, amsacrine, procarbazine, mitotane, mitoxantrone, lévamisole, navelbène, CPT-11, anastrazole, létrazole, capécitabine, réloxafine, droloxafine, et hexaméthylmélamine.

**24.** Emploi conforme à la revendication 22, ledit agent de chimiothérapie étant un agent qui agit au niveau des microtubules, choisi parmi les suivants : allocolchicine, halichondrine B, colchicine, dérivés de colchicine, dolastatine 10, maytansine, rhizoxine, paclitaxel, dérivés de paclitaxel, thiocolchicine, trityl-cystéine, sulfate de vinblastine, sulfate de vincristine, épothilone A, épothilone, discodermolide, oestramustine, nocodazole et MAP4.

**25.** Emploi d'un composé conforme à l'une des revendications 1 à 20 en vue de la fabrication d'un médicament conçu pour le traitement d'une maladie proliférative par thérapie combinée, où l'on administrera simultanément ou consécutivement ledit composé et au moins un inhibiteur de transduction de signal.

**26.** Emploi conforme à la revendication 25, ledit inhibiteur de transduction de signal étant choisi parmi l'inhibiteur bcr/abl Gleevec, les inhibiteurs des récepteurs du facteur de croissance épidermique Iressa, OSI-774, Imclone C225 et Abgenix ABX-EGF, et l'inhibiteur du récepteur HER-2/neu Herceptine.